# EUROPEAN PATENT APPLICATION

(11) **EP 4 663 626 A1**
(43) Date of publication of application: **17.12.2025**
(21) Application number: 24752925.8
(22) Date of filing: 08.02.2024
(51) Int. Cl.: C07D 207/22, C07D 211/72, C07D 235/02, A61K 31/40, A61K 31/41, A61P 5/10

(54) **IMINE COMPOUNDS AS WELL AS PREPARATION METHOD THEREFOR AND USE THEREOF**

(30) Priority: 09.02.2023 CN 202310084847; 24.05.2023 CN 202310588306; 10.08.2023 CN 202311005363
(71) Applicant: Bio Genuine (Shanghai) Biotech Co., Ltd, Shanghai 201203 (CN)
(72) Inventor: ZHANG, Peng, Shanghai 201203 (CN); LI, Kun, Shanghai 201203 (CN); YE, Wenwu, Shanghai 201203 (CN); CUI, Xiaofeng, Shanghai 201203 (CN); LV, Hejun, Shanghai 201203 (CN)
(74) Representative: Cabinet Becker et Associés
(86) International application number: PCT/CN2024/077005
(87) International publication number: WO 2024/165071

(57) **Abstract**

The present disclosure relates to imine compounds as well as a preparation method therefor and the use thereof, in particular to compounds as shown in formula I. The compounds can be used for treating medical conditions such as premature delivery.

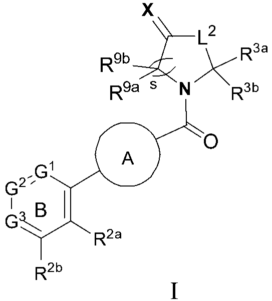

## Description

### Technical Field

The present disclosure belongs to the field of medicine, and specifically relates to an imine compound, and a preparation method therefor and the use thereof.

### Background Art

Oxytocin is a cyclic nonapeptide that exerts its physiological effects by binding to its specific receptors, oxytocin receptors (OTR). It is known that the physiological effects of oxytocin involve various aspects such as social bonding, sexual reproduction, and delivery. The oxytocin receptor is structurally very similar to vasopressin receptors (including V1a, V1b, and V2 receptors), among which the V1a and V2 receptors are primarily expressed peripherally and are responsible for regulating blood pressure and kidney function, respectively. The V1b receptor is primarily expressed in the brain and pituitary gland, where it regulates the release of adrenocorticotropic hormone and β-endorphin.

Studies have shown that oxytocin plays a major role in delivery in mammals, especially in humans. During delivery, the binding of oxytocin to its receptor can induce strong uterine contractions, which facilitate the delivery of the foetus. However, untimely uterine contractions may lead to miscarriage and premature delivery. Downregulating oxytocin or blocking the binding of oxytocin to its receptors can block the contractile effect of oxytocin on the uterus and is an important way to prevent premature delivery.

Atosiban is a peptide-based oxytocin receptor antagonist that has been approved for marketing for the treatment of premature delivery. In addition, several oxytocin receptor antagonists are in the clinical research stage, such as Nolasiban, Cligosiban, and Retosiban.

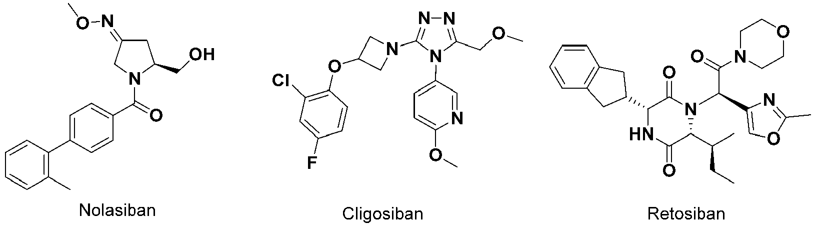

Patent applications WO 2001072705 A1, WO 2002074741 A1, WO 2002102799 A2, WO 2004005249 A1, WO 2004076407 A2, and WO 2015036160 A1 disclose a series of compounds useful as oxytocin receptor antagonists, which can be used for the treatment of sexual dysfunction, hypoactive sexual desire disorder, sexual arousal disorder, orgasm disorder, dyspareunia, premature ejaculation, preterm delivery, delivery complications, appetite and eating disorders, benign prostatic hyperplasia, premature delivery, dysmenorrhea, congestive heart failure, arterial hypertension, liver cirrhosis, renal hypertension, ocular hypertension, obsessive-compulsive disorder, neuropsychiatric disease, etc.

### Summary of the Invention

The present disclosure aims to provide a compound of formula I useful as an oxytocin receptor antagonist. The compound of formula I provided by the present invention has improved OTR antagonistic activity. The compound of formula I provided by the present invention also has lower V1aR antagonistic activity, and exhibits improved OTR/V1a target selectivity.

The present disclosure provides a compound as shown in formula I: 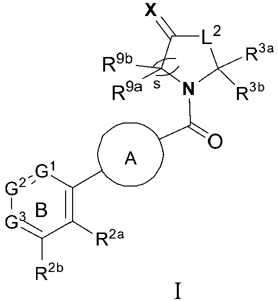
or an isotopic derivative thereof or a pharmaceutically acceptable salt of any of the foregoing;
wherein X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl, -OR^{5a}, -SR^{5a}, -N(R^{5a})(R^{5b}), -C(O)R^{5a}, -OC(O)R^{5a}, -C(O)OR^{5a}, -C(O)N(R^{5a})(R^{5b}), -N(R^{5c})C(O)R^{5a}, -N(R^{5c})C(O)N(R^{5a})(R^{5b}), -S(O)₂R^{5a}, -N(R^{5c})S(O)₂R^{5a}, - S(O)₂N(R^{5a})(R^{5b}), -Si(R^{5a})₃, -Si(R^{5a})2(OR^{5b}), -OSi(R^{5a})₃, -Si(R^{5a})(OR^{5b})₂, - OP(O)(OR^{5a})(OR^{5b}), -P(O)(OR^{5a})(OR^{5b}), -OP(O)(OR^{5a})(R^{5b}), -P(O)(OR^{5a})(R^{5b}), - OP(O)(R^{5a})(R^{5b}) or -P(O)(R^{5a})(R^{5b});
R^{3a} is hydrogen, halogen, cyano, nitro, C₁₋₆ alkyl, C₁₋₆ haloalkyl, C₃₋₈ cycloalkyl, 3- to 8-membered heterocycloalkyl, phenyl, 5- to 6-membered heteroaryl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl or -L¹-R^{3c};
R^{3b} is hydrogen, halogen, cyano, nitro, C₁₋₆ alkyl, C₁₋₆ haloalkyl, C₂₋₆ alkenyl, halogenated C₂₋₆ alkenyl, C₂₋₆ alkynyl, halogenated C₂₋₆ alkynyl, -L¹-R^{3c}, - C(O)OR^{3d}, -C(O)N(R^{3d})(R^{3e}), -S(O)₂R^{3d}, -S(O)₂N(R^{3d})(R^{3e}), -P(O)(OR^{3d})(OR^{3e}), -P(O)(OR^{3d})(R^{3e}), -P(O)(R^{3d})(R^{3e}), C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl, and the C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl optionally forms a fused ring with additional C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl; wherein the C₃₋₈ cycloalkyl, 3- to 8-membered heterocycloalkyl, C₃₋₈ cycloalkenyl, 3- to 8-membered heterocycloalkenyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
alternatively, R^{3a} and R^{3b}, together with the carbon atom to which they are attached, form C₃₋₈ cycloalkyl, 3- to 8-membered heterocycloalkyl, C₃₋₈ cycloalkenyl or 3- to 8-membered heterocycloalkenyl, and the C₃₋₈ cycloalkyl, 3- to 8-membered heterocycloalkyl, C₃₋₈ cycloalkenyl and 3- to 8-membered heterocycloalkenyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
each L¹ is independently -[C(R^{a})(R^{b})]ₙ-; wherein n is 1, 2 or 3; R^{a} and R^{b} are each independently hydrogen, C₁₋₆ alkyl, C₃₋₈ cycloalkyl or 3- to 8-membered heterocycloalkyl; alternatively, R^{a} and R^{b}, together with the carbon atom to which they are attached, form C₃₋₈ cycloalkyl or 3- to 8-membered heterocycloalkyl; wherein the C₁₋₆ alkyl, C₃₋₈ cycloalkyl and 3- to 8-membered heterocycloalkyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
each R^{3c} is independently -OR^{7a}, -SR^{7a}, -N(R^{7a})(R^{7b}), -C(O)R^{7a}, -OC(O)R^{7a}, -C(O)OR^{7a}, -C(O)N(R^{7a})(R^{7b}), -N(R^{7c})C(O)R^{7a}, -N(R^{7c})C(O)N(R^{7a})(R^{7b}), - S(O)₂R^{7a}, -N(R^{7c})S(O)₂R^{7a}, -S(O)₂N(R^{7a})(R^{7b}), -Si(R^{7a})₃, -Si(R^{7a})₂(OR^{7b}), - OSi(R^{7a})₃, -Si(R^{7a})(OR^{7b})₂, -OP(O)(OR^{7a})(OR^{7b}), -P(O)(OR^{7a})(OR^{7b}), - OP(O)(OR^{7a})(R^{7b}), -P(O)(OR^{7a})(R^{7b}), -OP(O)(R^{7a})(R^{7b}) or -P(O)(R^{7a})(R^{7b});
L² is -[C(R^{10a})(R^{10b})]ₜ-, wherein one C(R^{10a})(R^{10b}) moiety is optionally replaced by -O- or -N(R^{10a})-;
s is 1 and t is 1; alternatively, s is 2 and t is 1; alternatively, s is 1 and t is 2;
R^{9a} and R^{9b} are each independently hydrogen, halogen, cyano, nitro, C₁₋₆ alkyl, C₁₋₆ haloalkyl, C₁₋₆ hydroxyalkyl, C₂₋₆ alkenyl, halogenated C₂₋₆ alkenyl, C₂₋₆ alkynyl or halogenated C₂₋₆ alkynyl; alternatively, R^{9a} and R^{9b}, together with the atom to which they are attached, form C₃₋₈ cycloalkyl, 3- to 8-membered heterocycloalkyl, C₃₋₈ cycloalkenyl or 3- to 8-membered heterocycloalkenyl; alternatively, R^{9a} and R^{3a} are joined to form -CH₂- or -CH₂CH₂-;
R^{10a} and R^{10b} are each independently hydrogen, halogen, cyano, C₁₋₆ alkyl, C₁₋₆ haloalkyl, C₁₋₆ hydroxyalkyl, C₂₋₆ alkenyl, halogenated C₂₋₆ alkenyl, C₂₋₆ alkynyl or halogenated C₂₋₆ alkynyl; alternatively, R^{10a} and R^{10b}, together with the carbon atom to which they are attached, form C₃₋₈ cycloalkyl, 3- to 8-membered heterocycloalkyl, C₃₋₈ cycloalkenyl or 3- to 8-membered heterocycloalkenyl; alternatively, R^{10a} and R^{3a}, together with the atom to which they are attached, form C₃₋₈ cycloalkyl, 3- to 8-membered heterocycloalkyl, C₃₋₈ cycloalkenyl or 3- to 8-membered heterocycloalkenyl;
ring A is 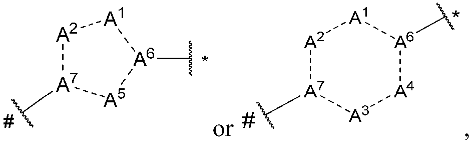 wherein the * end is connected to the carbonyl group in formula I, and the # end is connected to ring B in formula I; each ----bond is independently a single bond or a double bond;
each A¹ is independently CH, C(O), N, NH, O, S, N(R^{4a}) or C(R^{4a});
each A² is independently CH, C(O), N, NH, O, S, N(R^{4b}) or C(R^{4b});
A³ is CH, C(O), N, N(R^{4c}) or C(R^{4c});
each A⁴ is independently CH, C(O), N, N(R^{4d}) or C(R^{4d});
A⁵ is CH, N, O, S, NH, C(R^{4d}) or N(R^{4d});
each A⁶ is independently C or N;
each A⁷ is independently C or N;
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, -N(R^{8c})C(O)N(R^{8a})(R^{8b}), - S(O)₂R^{8a}, -N(R^{8c})S(O)₂R^{8a}, -S(O)₂N(R^{8a})(R^{8b}), -Si(R^{8a})₃, -Si(R^{8a})₂(OR^{8b}), - OSi(R^{8a})₃, -Si(R^{8a})(OR^{8b})₂, -OP(O)(OR^{8a})(OR^{8b}), -P(O)(OR^{8a})(OR^{8b}), - OP(O)(OR^{8a})(R^{8b}), -P(O)(OR^{8a})(R^{8b}), -OP(O)(R^{8a})(R^{8b}) or -P(O)(R^{8a})(R^{8b});
alternatively, R^{4a} and R^{4b}, together with the atom to which they are attached, form C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl; the C₃₋₈ cycloalkyl, 3- to 8-membered heterocycloalkyl, C₃₋₈ cycloalkenyl, 3- to 8-membered heterocycloalkenyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
in ring B, G¹, G² and G³ are each independently N or C(R^{2c});
R^{2a}, R^{2b} and R^{2c} are each independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl, -OR^{6a}, -SR^{6a}, -N(R^{6a})(R^{6b}), -C(O)R^{6a}, -OC(O)R^{6a}, -C(O)OR^{6a}, -C(O)N(R^{6a})(R^{6b}), -N(R^{6c})C(O)R^{6a}, -N(R^{6c})C(O)N(R^{6a})(R^{6b}), - S(O)₂R^{6a}, -N(R^{6c})S(O)₂R^{6a}, -S(O)₂N(R^{6a})(R^{6b}), -Si(R^{6a})₃, -Si(R^{6a})₂(OR^{6b}), - OSi(R^{6a})₃, -S1(R^{6a})(OR^{6b})₂, -OP(O)(OR^{6a})(OR^{6b}), -P(O)(OR^{6a})(OR^{6b}), - OP(O)(OR^{6a})(R^{6b}), -P(O)(OR^{6a})(R^{6b}), -OP(O)(R^{6a})(R^{6b}) or -P(O)(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl; the C₃₋₈ cycloalkyl, 3- to 8-membered heterocycloalkyl, C₃₋₈ cycloalkenyl, 3- to 8-membered heterocycloalkenyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
alternatively, R^{2a} and R^{4b} are joined to form -(CH₂)ₚ-, wherein p is 2, 3 or 4, and wherein one or two CH₂ moieties are optionally replaced by -O- or -NH-;
R^{3d}, R^{3e}, R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl, and the C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl optionally forms a fused ring with additional C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl; wherein the C₁₋₆ alkyl, C₃₋₈ cycloalkyl, 3- to 8-membered heterocycloalkyl, C₃₋₈ cycloalkenyl, 3- to 8-membered heterocycloalkenyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₃₋₈ cycloalkyl, 3- to 8-membered heterocycloalkyl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl, phenyl and 5- to 6-membered heteroaryl;
R^{5c}, R^{6c}, R^{7c} and R^{8c} are each independently hydrogen, C₁₋₆ alkyl, C₁₋₆ haloalkyl, C₁₋₆ hydroxyalkyl, C₂₋₆ alkenyl, halogenated C₂₋₆ alkenyl, C₂₋₆ alkynyl or halogenated C₂₋₆ alkynyl;
in the aforementioned heterocycloalkyl, heterocycloalkenyl and heteroaryl, the number of heteroatoms is independently 1, 2, 3 or 4, and each heteroatom is independently N, O, S, Si, P and Se;
provided that at least one of the following conditions (a), (b), (c), (d), and (e) is satisfied:
   condition (a): neither R^{2a} nor R^{2b} is hydrogen; moreover, when R^{2a} is C₁₋₆ alkyl, R^{3a} is hydrogen and R^{3b} is a group other than -L¹-R^{3c}, then R^{2b} is not C₁₋₆ alkyl;
   condition (b): R^{3a} is C₁₋₆ alkyl or -L¹-R^{3c} and R^{3b} is -L¹-R^{3c}; alternatively, R^{3a} and R^{3b}, together with the carbon atom to which they are attached, form C₃₋₈ cycloalkyl, 3- to 8-membered heterocycloalkyl, C₃₋₈ cycloalkenyl or 3- to 8-membered heterocycloalkenyl, and the C₃₋₈ cycloalkyl, 3- to 8-membered heterocycloalkyl, C₃₋₈ cycloalkenyl and 3- to 8-membered heterocycloalkenyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
   condition (c): R^{3a} is hydrogen, C₁₋₆ alkyl or -L¹-R^{3c}; R^{3b} is -L¹-R^{3c}; ring A is 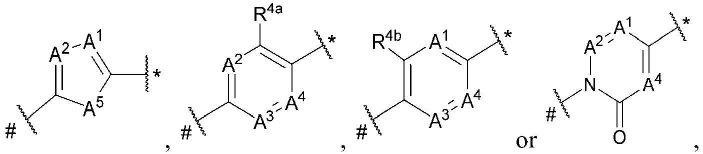 wherein the * end is connected to the carbonyl group in formula I, and the # end is connected to ring B in formula I; moreover, R^{2a} is not hydrogen; wherein each A¹ is independently CH, N or C(R^{4a}); each A² is independently CH, N or C(R^{4b}); each A³ is independently CH, N or C(R^{4c}); each A⁴ is independently CH, N or C(R^{4d}); A⁵ is O, S, NH or N(R^{4d});
   condition (d): R^{3b} is -CH(C₁₋₆ alkyl)-OH, 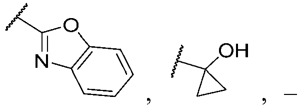 CH₂NHC(O)CH₂OH or -CH₂OCH₂CH₂OH;
   condition (e): R^{9a} and R^{3a} are joined to form -CH₂- or -CH₂CH₂-.

In some embodiments, in the structure of formula I, 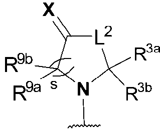 is

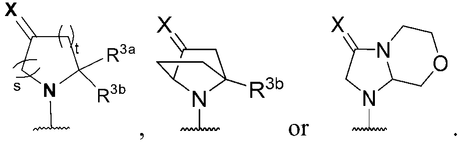

In some embodiments, the compound of formula I has a structure as shown in formula I-1: 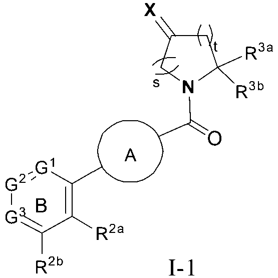
wherein s is 1 and t is 1; alternatively, s is 2 and t is 1; alternatively, s is 1 and t is 2;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{5a}, _ C(O)OR^{5a}, -C(O)N(R^{5a})(R^{5b}), -N(R^{5c})C(O)R^{5a}, -N(R^{5c})C(O)N(R^{5a})(R^{5b}), - S(O)₂R^{5a}, -N(R^{5c})S(O)₂R^{5a} or -S(O)₂N(R^{5a})(R^{5b});
R^{3a} is hydrogen, C₁₋₆ alkyl or -L¹-R^{3c};
R^{3b} is -L¹-R^{3c}, -C(O)OR^{3d}, -C(O)N(R^{3d})(R^{3e}), -S(O)₂R^{3d}, -S(O)₂N(R^{3d})(R^{3e}), C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5-to 6-membered heteroaryl optionally forms a fused ring with additional C₃₋₈ cycloalkyl, 3- to 8-membered heterocycloalkyl, phenyl or 5- to 6-membered heteroaryl; wherein the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
each L¹ is independently -[C(R^{a})(R^{b})]ₙ-; wherein n is 1, 2 or 3; R^{a} and R^{b} are each independently hydrogen, C₁₋₆ alkyl, C₃₋₈ cycloalkyl or 3- to 8-membered heterocycloalkyl; alternatively, R^{a} and R^{b}, together with the carbon atom to which they are attached, form C₃₋₈ cycloalkyl or 3- to 8-membered heterocycloalkyl; wherein the C₁₋₆ alkyl, C₃₋₈ cycloalkyl and 3- to 8-membered heterocycloalkyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
each R^{3c} is independently -OR^{7a}, -SR^{7a}, -N(R^{7a})(R^{7b}), -C(O)R^{7a}, -OC(O)R^{7a}, -C(O)OR^{7a}, -C(O)N(R^{7a})(R^{7b}), -N(R^{7c})C(O)R^{7a}, -N(R^{7c})C(O)N(R^{7a})(R^{7b}), - S(O)₂R^{7a}, -N(R^{7c})S(O)₂R^{7a} or -S(O)₂N(R^{7a})(R^{7b});
ring A is 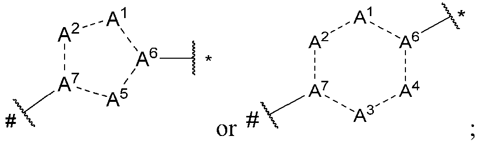 wherein the * end is connected to the carbonyl group in formula I, and the # end is connected to ring B in formula I; each ----bond is independently a single bond or a double bond;
each A¹ is independently CH, C(O), N, NH, O, S, N(R^{4a}) or C(R^{4a});
each A² is independently CH, C(O), N, NH, O, S, N(R^{4b}) or C(R^{4b});
A³ is CH, C(O), N, N(R^{4c}) or C(R^{4c});
each A⁴ is independently CH, C(O), N, N(R^{4d}) or C(R^{4d});
A⁵ is CH, N, O, S, NH, C(R^{4d}) or N(R^{4d});
each A⁶ is independently C or N;
each A⁷ is independently C or N;
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, - N(R^{8c})C(O)N(R^{8a})(R^{8b}), -S(O)₂R^{8a}, -N(R^{8c})S(O)₂R^{8a} or -S(O)₂N(R^{8a})(R^{8b});
alternatively, R^{4a} and R^{4b}, together with the atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy; in ring B, G¹, G² and G³ are each independently N or C(R^{2c});
each R^{2c} is independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
R^{2a} and R^{2b} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
alternatively, R^{2a} and R^{4b} are joined to form -(CH₂)ₚ-, wherein p is 2, 3 or 4, and wherein one or two CH₂ moieties are optionally replaced by -O- or -NH-;
provided that, when R^{2a} is C₁₋₆ alkyl, R^{3a} is hydrogen and R^{3b} is a group other than -L¹-R^{3c}, then R^{2b} is not C₁₋₆ alkyl;
R^{3d}, R^{3e}, R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₃₋₈ cycloalkyl, 3- to 8-membered heterocycloalkyl, phenyl and 5- to 6-membered heteroaryl;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S.

In some embodiments, the compound of formula I has a structure as shown in formula I-1: 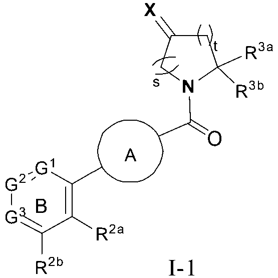
wherein s is 1 and t is 1; alternatively, s is 2 and t is 1; alternatively, s is 1 and t is 2;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{5a}, - C(O)OR^{5a}, -C(O)N(R^{5a})(R^{5b}), -N(R^{5c})C(O)R^{5a}, -N(R^{5c})C(O)N(R^{5a})(R^{5b}), - S(O)₂R^{5a}, -N(R^{5c})S(O)₂R^{5a} or -S(O)₂N(R^{5a})(R^{5b});
R^{3a} is hydrogen, C₁₋₆ alkyl or -L¹-R^{3c};
R^{3b} is -L¹-R^{3c};
each L¹ is independently -[C(R^{a})(R^{b})]ₙ-; wherein n is **1,** 2 or 3; R^{a} and R^{b} are each independently hydrogen, C₁₋₆ alkyl, C₃₋₈ cycloalkyl or 3- to 8-membered heterocycloalkyl; alternatively, R^{a} and R^{b}, together with the carbon atom to which they are attached, form C₃₋₈ cycloalkyl or 3- to 8-membered heterocycloalkyl; wherein the C₁₋₆ alkyl, C₃₋₈ cycloalkyl and 3- to 8-membered heterocycloalkyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
each R^{3c} is independently -OR^{7a}, -SR^{7a}, -N(R^{7a})(R^{7b}), -C(O)R^{7a}, -OC(O)R^{7a}, -C(O)OR^{7a}, -C(O)N(R^{7a})(R^{7b}), -N(R^{7c})C(O)R^{7a}, -N(R^{7c})C(O)N(R^{7a})(R^{7b}), - S(O)₂R^{7a}, -N(R^{7c})S(O)₂R^{7a} or -S(O)₂N(R^{7a})(R^{7b});
ring A is 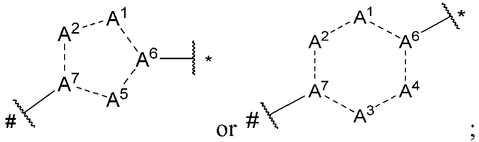 wherein the * end is connected to the carbonyl group in formula **I,** and the # end is connected to ring B in formula I; each ----bond is independently a single bond or a double bond;
each A¹ is independently CH, C(O), N, NH, O, S, N(R^{4a}) or C(R^{4a});
each A² is independently CH, C(O), N, NH, O, S, N(R^{4b}) or C(R^{4b});
A³ is CH, C(O), N, N(R^{4c}) or C(R^{4c});
each A⁴ is independently CH, C(O), N, N(R^{4d}) or C(R^{4d});
A⁵ is CH, N, O, S, NH, C(R^{4d}) or N(R^{4d});
each A⁶ is independently C or N;
each A⁷ is independently C or N;
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, - N(R^{8C})C(O)N(R^{8a})(R^{8b}), -S(O)₂R^{8a}, -N(R^{8c})S(O)₂R^{8a} or -S(O)₂N(R^{8a})(R^{8b});
alternatively, R^{4a} and R^{4b}, together with the atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
in ring B, G¹, G² and G³ are each independently N or C(R^{2c});
each R^{2c} is independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
R^{2a} and R^{2b} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
alternatively, R^{2a} and R^{4b} are joined to form -(CH₂)ₚ-, wherein p is 2, 3 or 4, and wherein one or two CH₂ moieties are optionally replaced by -O- or -NH-;
R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₃₋₈ cycloalkyl, 3- to 8-membered heterocycloalkyl, phenyl and 5- to 6-membered heteroaryl;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S.

In some embodiments, in the definitions of formulae I and I-1, s is 1 and t is 1.

In some embodiments, in the definitions of formulae I and I-1, s is 2 and t is 1.

In some embodiments, in the definitions of formulae I and I-1, s is 1 and t is 2.

In some embodiments, in the definitions of formulae I and I-1, ring A is 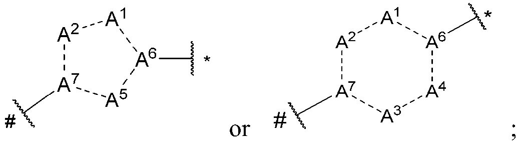 wherein each ----bond is independently a single bond or a double bond, provided that an aromatic ring is formed; each A¹ is independently CH, N, NH, O, S, N(R^{4a}) or C(R^{4a}); each A² is independently CH, N, NH, O, S, N(R^{4b}) or C(R^{4b}); A³ is CH, N, N(R^{4c}) or C(R^{4c}); each A⁴ is independently CH, N, N(R^{4d}) or C(R^{4d}); A⁵ is CH, N, O, S, NH, C(R^{4d}) or N(R^{4d}); each A⁶ is independently C or N; each A⁷ is independently C or N; R^{4a}, R^{4b}, R^{4c} and R^{4d} are as defined in any of the embodiments of the present disclosure.

In some embodiments, the compound of formula I has a structure as shown in formula I-1: 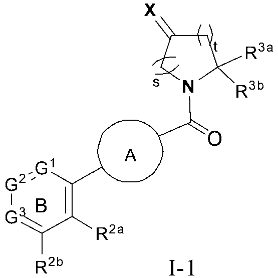
wherein s is 1 and t is 1;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{5a}, _ C(O)OR^{5a}, -C(O)N(R^{5a})(R^{5b}), -N(R^{5c})C(O)R^{5a}, -N(R^{5c})C(O)N(R^{5a})(R^{5b}), - S(O)₂R^{5a}, -N(R^{5c})S(O)₂R^{5a} or -S(O)₂N(R^{5a})(R^{5b});
R^{3a} is hydrogen, C₁₋₆ alkyl or -L¹-R^{3c},
R^{3b} is -L¹-R^{3c};
each L¹ is independently -[C(R^{a})(R^{b})]ₙ-; wherein n is 1, 2 or 3; R^{a} and R^{b} are each independently hydrogen or C₁₋₆ alkyl; alternatively, R^{a} and R^{b}, together with the carbon atom to which they are attached, form C₃₋₈ cycloalkyl or 3- to 8-membered heterocycloalkyl;
each R^{3c} is independently -OR^{7a}, -SR^{7a}, -N(R^{7a})(R^{7b}), -C(O)R^{7a}, -OC(O)R^{7a}, -C(O)OR^{7a}, -C(O)N(R^{7a})(R^{7b}), -N(R^{7c})C(O)R^{7a}, -N(R^{7c})C(O)N(R^{7a})(R^{7b}), - S(O)₂R^{7a}, -N(R^{7c})S(O)₂R^{7a} or -S(O)₂N(R^{7a})(R^{7b});
ring A is 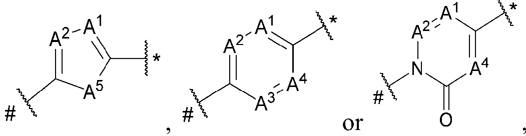 wherein the * end is connected to the carbonyl group in formula I, and the # end is connected to ring B in formula I;
each A¹ is independently CH, N or C(R^{4a});
each A² is independently CH, N or C(R^{4b});
A³ is CH, N or C(R^{4c});
each A⁴ is independently CH, N or C(R^{4a});
A⁵ is O, S, NH or N(R^{4d});
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, - N(R^{8c})C(O)N(R^{8a})(R^{8b}), -S(O)₂R^{8a}, -N(R^{8c})S(O)₂R^{8a} or -S(O)₂N(R^{8a})(R^{8b});
alternatively, R^{4a} and R^{4b}, together with the carbon atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
in ring B, G¹, G² and G³ are each independently N or C(R^{2c});
R^{2a}, R^{2b} and R^{2c} are each independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, -OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5a} , R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S;
provided that at least one of the following conditions (a), (b), (c) and (d) is satisfied:
   condition (a): neither R^{2a} nor R^{2b} is hydrogen;
   condition (b): R^{3a} is not hydrogen;
   condition (c): ring A is 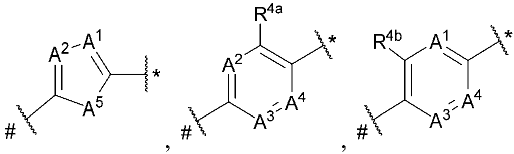 or 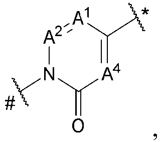 wherein the * end is connected to the carbonyl group in formula I, and the # end is connected to ring B in formula I; moreover, R^{2a} is not hydrogen;
   condition (d): R^{3b} is -CH(C₁₋₆ alkyl)-OH.

In some embodiments, the compound of formula I has a structure as shown in formula I-1: 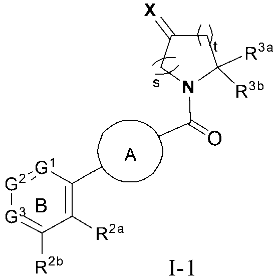
wherein s is 1 and t is 1;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{5a},-C(O)OR^{5a}, -C(O)N(R^{5a})(R^{5b}), -N(R^{5c})C(O)R^{5a}, -N(R^{5c})C(O)N(R^{5a})(R^{5b}), - S(O)₂R^{5a}, -N(R^{5c})S(O)₂R^{5a} or -S(O)₂N(R^{5a})(R^{5b});
R^{3a} is hydrogen, C₁₋₆ alkyl or -L¹-R^{3c};
R^{3b} is -L¹-R^{3c};
L¹ is -[C(R^{a})(R^{b})]ₙ-; wherein n is 1, 2 or 3; R^{a} and R^{b} are each independently hydrogen or C₁₋₆ alkyl; alternatively, R^{a} and R^{b}, together with the carbon atom to which they are attached, form C₃₋₈ cycloalkyl or 3- to 8-membered heterocycloalkyl;
R^{3c} is -OR^{7a}, -SR^{7a}, -N(R^{7a})(R^{7b}), -C(O)R^{7a}, -OC(O)R^{7a}, -C(O)OR^{7a}, - C(O)N(R^{7a})(R^{7b}), -N(R^{7c})C(O)R^{7a}, -N(R^{7c})C(O)N(R^{7a})(R^{7b}), -S(O)2R^{7a}, - N(R^{7c})S(O)₂R^{7a} or -S(O)₂N(R^{7a})(R^{7b});
ring A is 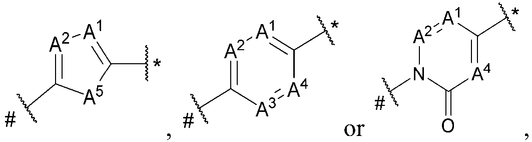 wherein the * end is connected to the carbonyl group in formula I, and the # end is connected to ring B in formula I;
each A¹ is independently CH, N or C(R^{4a});
each A² is independently CH, N or C(R^{4b});
A³ is CH, N or C(R^{4c});
each A⁴ is independently CH, N or C(R^{4d});
A⁵ is O, S, NH or N(R^{4d});
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, - N(R^{8c})C(O)N(R^{8a})(R^{8b}), -S(O)2R^{8a}, -N(R^{8c})S(O)₂R^{8a} or -S(O)₂N(R^{8a})(R^{8b});
alternatively, R^{4a} and R^{4b}, together with the carbon atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy; in ring B, G¹, G² and G³ are each independently N or C(R^{2c});
each R^{2c} is independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
R^{2a} and R^{2b} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S.

In some embodiments, the compound of formula I has a structure as shown in formula I-1: 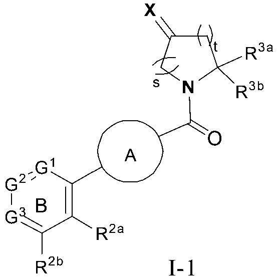
wherein s is 1 and t is 1;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{5a},-C(O)OR^{5a}, -C(O)N(R^{5a})(R^{5b}), -N(R^{5c})C(O)R^{5a}, -N(R^{5c})C(O)N(R^{5a})(R^{5b}), - S(O)₂R^{5a}, -N(R^{5c})S(O)₂R^{5a} or -S(O)₂N(R^{5a})(R^{5b});
R^{3a} is C₁₋₆ alkyl or -L¹-R^{3c};
R^{3b} is -L¹-R^{3c};
each L¹ is independently -[C(R^{a})(R^{b})]ₙ-; wherein n is 1, 2 or 3; R^{a} and R^{b} are each independently hydrogen or C₁₋₆ alkyl; alternatively, R^{a} and R^{b}, together with the carbon atom to which they are attached, form C₃₋₈ cycloalkyl or 3- to 8-membered heterocycloalkyl;
each R^{3c} is independently -OR^{7a}, -SR^{7a}, -N(R^{7a})(R^{7b}), -C(O)R^{7a}, -OC(O)R^{7a}, -C(O)OR^{7a}, -C(O)N(R^{7a})(R^{7b}), -N(R^{7c})C(O)R^{7a}, -N(R^{7c})C(O)N(R^{7a})(R^{7b}), - S(O)₂R^{7a}, -N(R^{7c})S(O)₂R^{7a} or -S(O)₂N(R^{7a})(R^{7b});
ring A is 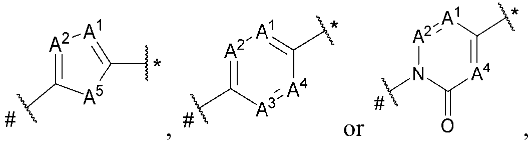 wherein the * end is connected to the carbonyl group in formula **I,** and the # end is connected to ring B in formula I;
each A¹ is independently CH, N or C(R^{4a});
each A² is independently CH, N or C(R^{4b});
A³ is CH, N or C(R^{4c});
each A⁴ is independently CH, N or C(R^{4a});
A¹ is O, S, NH or N(R^{4d});
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, - N(R^{8c})C(O)N(R^{8a})(R^{8b}), -S(O)₂R^{8a}, -N(R^{8c})S(O)₂R^{8a} or -S(O)₂N(R^{8a})(R^{8b});
alternatively, R^{4a} and R^{4b}, together with the carbon atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
in ring B, G¹, G² and G³ are each independently N or C(R^{2c});
R^{2a}, R^{2b} and R^{2c} are each independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, -OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S.

In some embodiments, the compound of formula I has a structure as shown in formula I-1: 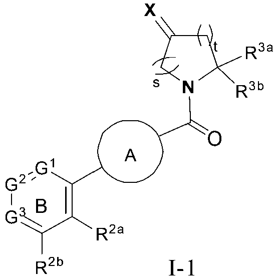
wherein s is 1 and t is 1;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{5a}, - C(O)OR^{5a}, -C(O)N(R^{5a})(R^{5b}), -N(R^{5c})C(O)R^{5a}, -N(R^{5c})C(O)N(R^{5a})(R^{5b}), - S(O)₂R^{5a}, -N(R^{5c})S(O)₂R^{5a} or -S(O)₂N(R^{5a})(R^{5b});
R^{3a} is hydrogen, C₁₋₆ alkyl or -L¹-R^{3c},
R^{3b} is -L¹-R^{3c};
L¹ is -[C(R^{a})(R^{b})]ₙ-; wherein n is 1, 2 or 3; R^{a} and R^{b} are each independently hydrogen or C₁₋₆ alkyl; alternatively, R^{a} and R^{b}, together with the carbon atom to which they are attached, form C₃₋₈ cycloalkyl or 3- to 8-membered heterocycloalkyl;
R^{3c} is -OR^{7a}, -SR^{7a}, -N(R^{7a})(R^{7b}), -C(O)R^{7a}, -OC(O)R^{7a}, -C(O)OR^{7a}, - C(O)N(R^{7a})(R^{7b}), -N(R^{7c})C(O)R^{7a}, -N(R^{7c})C(O)N(R^{7a})(R^{7b}), -S(O)₂R^{7a}, - N(R^{7c})S(O)₂R^{7a} or -S(O)₂N(R^{7a})(R^{7b});
ring A is 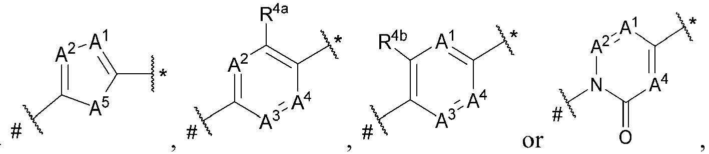 wherein the * end is connected to the carbonyl group in formula I, and the # end is connected to ring B in formula I;
each A¹ is independently CH, N or C(R^{4a});
each A² is independently CH, N or C(R^{4b});
each A³ is independently CH, N or C(R^{4c});
each A⁴ is independently CH, N or C(R^{4d});
A⁵ is O, S, NH or N(R^{4d});
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, - N(R^{8c})C(O)N(R^{8a})(R^{8b}), -S(O)₂R^{8a}, -N(R^{8c})S(O)₂R^{8a} or -S(O)₂N(R^{8a})(R^{8b});
alternatively, R^{4a} and R^{4b}, together with the carbon atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy; in ring B, G¹, G² and G³ are each independently N or C(R^{2c});
R^{2a} is halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, -OC(O)R^{6a}, -C(O)OR^{6a}, -C(O)N(R^{6a})(R^{6b}), -N(R^{6c})C(O)R^{6a}, -N(R^{6c})C(O)N(R^{6a})(R^{6b}), -S(O)₂R^{6a}, -N(R^{6c})S(O)₂R^{6a} or - S(O)₂N(R^{6a})(R^{6b});
R^{2b} and R^{2c} are each independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, -OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S.

In some embodiments, the compound of formula I has a structure as shown in formula 1-1: 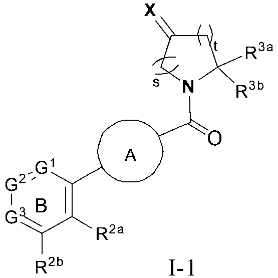
wherein s is 1 and t is 1;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{5a}, - C(O)OR^{5a}, -C(O)N(R^{5a})(R^{5b}), -N(R^{5c})C(O)R^{5a}, -N(R^{5c})C(O)N(R^{5a})(R^{5b}), - S(O)₂R^{5a}, -N(R^{5c})S(O)₂R^{5a} or -S(O)₂N(R^{5a})(R^{5b});
R^{3a} is hydrogen, C₁₋₆ alkyl or -L¹-R^{3c};
R^{3b} is -CH(C₁₋₆ alkyl)-OH;
L¹ is -[C(R^{a})(R^{b})]ₙ-; wherein n is 1, 2 or 3; R^{a} and R^{b} are each independently hydrogen or C₁₋₆ alkyl; alternatively, R^{a} and R^{b}, together with the carbon atom to which they are attached, form C₃₋₈ cycloalkyl or 3- to 8-membered heterocycloalkyl;
R^{3c} is -OR^{7a}, -SR^{7a}, -N(R^{7a})(R^{7b}), -C(O)R^{7a}, -OC(O)R^{7a}, -C(O)OR^{7a}, - C(O)N(R^{7a})(R^{7b}), -N(R^{7c})C(O)R^{7a}, -N(R^{7c})C(O)N(R^{7a})(R^{7b}), -S(O)₂R^{7a}, - N(R^{7c})S(O)₂R^{7a} or -S(O)₂N(R^{7a})(R^{7b});
ring A is 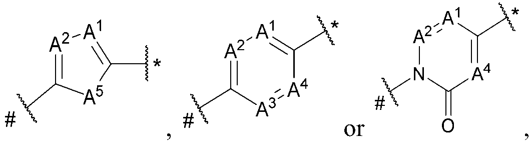 wherein the * end is connected to the carbonyl group in formula I, and the # end is connected to ring B in formula I;
each A¹ is independently CH, N or C(R^{4a});
each A² is independently CH, N or C(R^{4b});
A³ is CH, N or C(R^{4c});
each A⁴ is independently CH, N or C(R^{4d});
A⁵ is O, S, NH or N(R^{4d});
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, - N(R^{8c})C(O)N(R^{8a})(R^{8b}), -S(O)₂R^{8a}, -N(R^{8c})S(O)₂R^{8a} or -S(O)₂N(R^{8a})(R^{8b});
alternatively, R^{4a} and R^{4b}, together with the carbon atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
in ring B, G¹, G² and G³ are each independently N or C(R^{2c});
R^{2a}, R^{2b} and R^{2c} are each independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, -OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S.

In some embodiments, in the definitions of formulae I and I-1, R¹ is C₁₋₆ alkyl.

In some embodiments, in the definitions of formulae I and I-1, R¹ is methyl.

In some embodiments, in the definitions of formulae I and I-1, L¹ is - [CH(R^{a})]ₙ-; wherein n is 1, 2 or 3; R^{a} is hydrogen, C₁₋₆ alkyl or C₃₋₈ cycloalkyl, and the C₁₋₆ alkyl and C₃₋₈ cycloalkyl are optionally substituted with one or more hydroxyl.

In some embodiments, in the definitions of formulae I and I-1, L¹ is - [CH(R^{a})]ₙ-; wherein n is 1, 2 or 3; R^{a} is hydrogen or C₁₋₆ alkyl, and the C₁₋₆ alkyl is optionally substituted with one or more hydroxyl.

In some embodiments, in the definitions of formulae I and I-1, n is 1.

In some embodiments, in the definitions of formulae I and I-1, R^{3c} is -OH.

In some embodiments, in the definitions of formulae I and I-1, R^{3a} is hydrogen or C₁₋₆ alkyl.

In some embodiments, in the definitions of formulae I and I-1, R^{3a} is hydrogen or methyl.

In some embodiments, in the definitions of formulae I and I-1, R^{3a} is hydrogen.

In some embodiments, in the definitions of formulae I and I-1, R^{3a} is C₁₋₆ alkyl, such as methyl.

In some embodiments, in the definitions of formulae I and I-1, R^{3b} is -L¹-R^{3c}, wherein L¹ and R^{3c} are as defined in any of the embodiments of the present disclosure.

In some embodiments, in the definitions of formulae I and I-1, R^{3b} is -CH₂OH, -CH(CH₃)OH, -C(CH₃)₂OH, -CH(OH)CH₂OH, -CH₂CH₂OH or

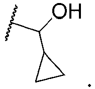

In some embodiments, in the definitions of formulae I and I-1, R^{3b} is 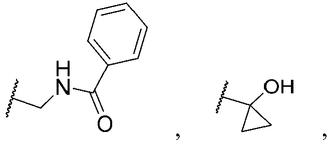 -CH₂NHC(O)CH₃, -CH₂NHC(O)OCH₃, - CH₂NHC(O)CH₂OH or -CH₂OCH₂CH₂OH.

In some embodiments, in the definitions of formulae I and I-1, R^{3b} is -CH₂OH.

In some embodiments, in the definitions of formulae I and I-1, R^{3b} is - CH(CH₃)OH.

In some embodiments, in the definitions of formulae I and I-1, R^{3b} is benzoxazolyl (e.g., 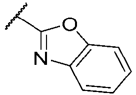 ), -C(O)NHCH₃, -C(O)OCH₃ or

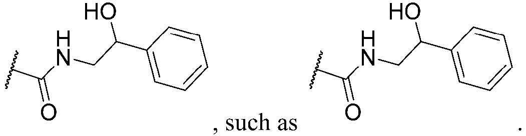

In some embodiments, in the definitions of formulae I and I-1, R^{3a} is hydrogen, and R^{3b} is -CH₂OH.

In some embodiments, in the definitions of formulae I and I-1, R^{3a} is hydrogen, and R^{3b} is -CH(CH₃)OH.

In some embodiments, in the definitions of formulae I and I-1, R^{3a} is methyl, and R^{3b} is -CH₂OH.

In some embodiments, in the definitions of formulae I and I-1, R^{3a} and R^{3b} are joined to form

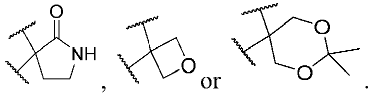

In some embodiments, in the definitions of formulae I and I-1, R^{4c} and R^{4d} are each independently halogen, cyano, C₁₋₆ alkyl, C₁₋₆ haloalkyl, C₁₋₆ alkoxy, -OR^{8a}, -SR^{8a}, -N(R^{8a})(R^{8b}), -C(O)R^{8a}, -C(O)OR^{8a} or -C(O)N(R^{8a})(R^{8b}).

In some embodiments, in the definitions of formulae I and I-1, R^{4a} and R^{4b} are each independently halogen, cyano, C₁₋₆ alkyl, C₁₋₆ haloalkyl, C₁₋₆ alkoxy, -OR^{8a}, -SR^{8a}, -N(R^{8a})(R^{8b}), -C(O)R^{8a}, -C(O)OR^{8a} or -C(O)N(R^{8a})(R^{8b}).

In some embodiments, in the definitions of formulae I and I-1, R^{8a} and R^{8b} are each independently hydrogen, C₁₋₆ alkyl, C₃₋₈ cycloalkyl or 3- to 8-membered heterocycloalkyl, and the C₁₋₆ alkyl is optionally substituted with one or more substituents independently selected from hydroxyl, C₁₋₆ alkyl, C₁₋₆ haloalkyl, C₁₋₆ hydroxyalkyl and C₁₋₆ alkoxy.

In some embodiments, in the definitions of formulae I and I-1, R^{4a} and R^{4b} are each independently halogen, cyano, C₁₋₆ alkyl, C₁₋₆ haloalkyl or C₁₋₆ alkoxy.

In some embodiments, in the definitions of formulae I and I-1, R^{4a} and R^{4b} are each independently methyl, monofluoromethyl, difluoromethyl, trifluoromethyl, methoxy, -OCH₂CH₂OH, -OCH₂CH₂OCH₃, -NHCH₂CH₂OCH₃,

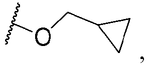

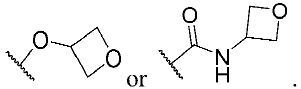

In some embodiments, in the definitions of formulae I and I-1, R^{4a} and R^{4b}, together with the carbon atom to which they are attached, form 3- to 8-membered heterocycloalkyl; the 3- to 8-membered heterocycloalkyl is optionally substituted with one or more of the aforementioned substituents (such as C₁₋₆ alkyl and carbonyl).

In some embodiments, in the definitions of formulae I and I-1, R^{4a} and R^{4b}, together with the carbon atom to which they are attached, form 5- to 6-membered heteroaryl; the 5- to 6-membered heteroaryl is optionally substituted with one or more of the aforementioned substituents (such as C₁₋₆ alkyl and carbonyl).

In some embodiments, in the definitions of formulae I and I-1, R^{4a} and R^{4b}, together with the carbon atom to which they are attached, form 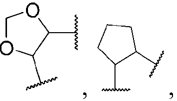 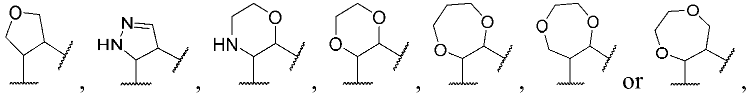 and the aforementioned rings are optionally substituted with one or more of the aforementioned substituents (such as C₁₋₆ alkyl and carbonyl).

In some embodiments, in the definitions of formulae I and I-1, R^{4a} and R^{4b}, together with the carbon atom to which they are attached, form

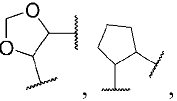

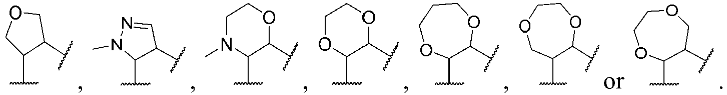

In some embodiments, in the definitions of formulae I and I-1, R^{4a} and R^{4b}, together with the carbon atom to which they are attached, form

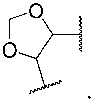

In some embodiments, in the definitions of formulae I and I-1, R^{4a} and R^{4b}, together with the carbon atom to which they are attached, form

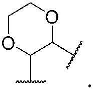

In some embodiments, in the definitions of formulae I and I-1, R^{4c} and R^{4d} are each independently halogen, cyano, C₁₋₆ alkyl, C₁₋₆ haloalkyl or C₁₋₆ alkoxy.

In some embodiments, in the definitions of formulae I and I-1, R^{4c} and R^{4d} are each independently fluorine, chlorine, methyl, monofluoromethyl, difluoromethyl, trifluoromethyl or methoxy, such as fluorine.

In some embodiments, in the definitions of formulae I and I-1, ring A is 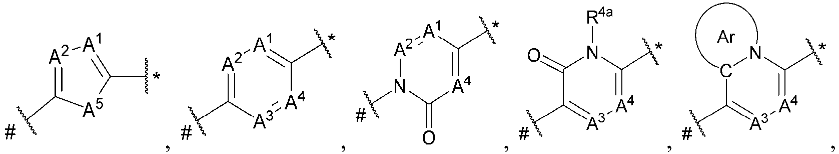 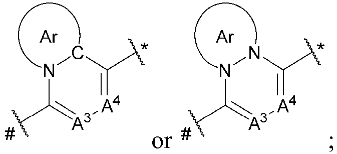 A¹ is CH, N or C(R^{4a}); A² is CH, N or C(R^{4b}); A³ is CH, N or C(R^{4c}); A⁴ is CH, N or C(R^{4d}); A⁵ is O, S, NH or N(R^{4d}); Ar is 3- to 8-membered heterocycloalkyl or 5- to 6-membered heteroaryl, wherein R^{4a}, R^{4b}, R^{4c} and R^{4d} are as defined in any of the embodiments of the present disclosure.

In some embodiments, in the definitions of formulae I and I-1, ring A is 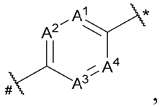 wherein A¹ is CH, N or C(R^{4a}); A² is CH, N or C(R^{4b}); A³ is CH, N or C(R^{4c}); A⁴ is CH, N or C(R^{4d}); R^{4a}, R^{4b}, R^{4c} and R^{4d} are as defined in any of the embodiments of the present disclosure.

In some embodiments, in the definitions of formulae I and I-1, ring A is 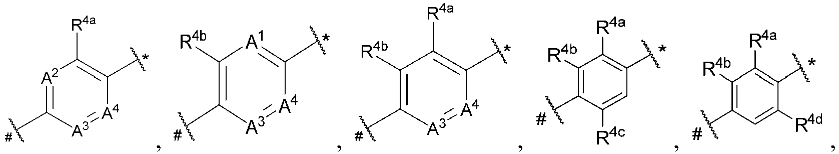 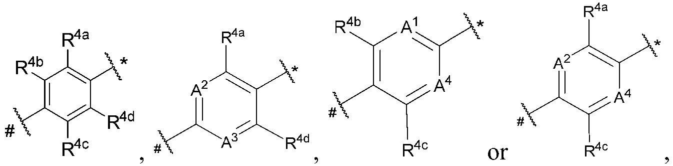 wherein A¹, A², A³ and A⁴ are each independently CH or N; R^{4a}, R^{4b}, R^{4c} and R^{4d} are as defined in any of the embodiments of the present disclosure.

In some embodiments, in the definitions of formulae I and I-1, ring A is 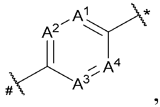 wherein A¹ is CH or C(R^{4a}); A² is CH or C(R^{4b}); A³ is CH or C(R^{4c}); A⁴ is CH or C(R^{4d}); R^{4a}, R^{4b}, R^{4c} and R^{4d} are as defined in any of the embodiments of the present disclosure.

In some embodiments, in the definitions of formulae I and I-1, ring A is 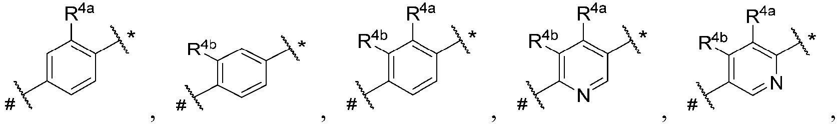 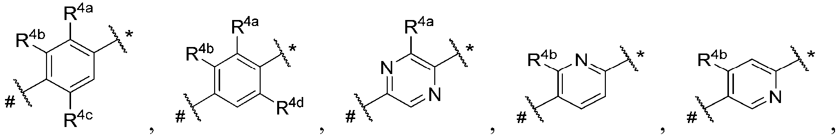 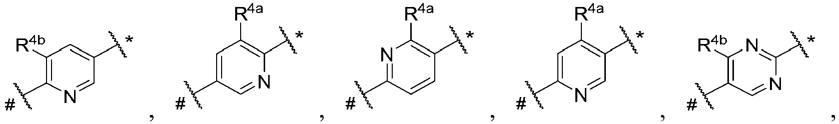 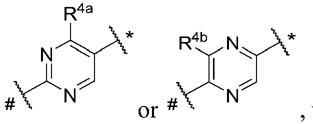 wherein R^{4a}, R^{4b}, R^{4c} and R^{4d} are as defined in any of the embodiments of the present disclosure.

In some embodiments, in the definitions of formulae I and I-1, ring A is 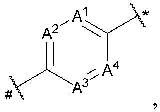 wherein A¹, A², A³ and A⁴ are each independently CH or N.

In some embodiments, in the definitions of formulae I and I-1, ring A is

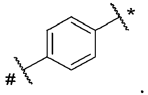

In some embodiments, in the definitions of formulae I and I-1, ring A is 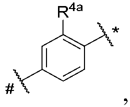 wherein R^{4a} is as defined in any of the embodiments of the present disclosure; for example, ring A may be

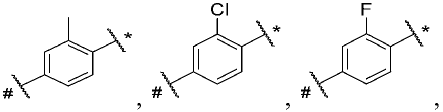

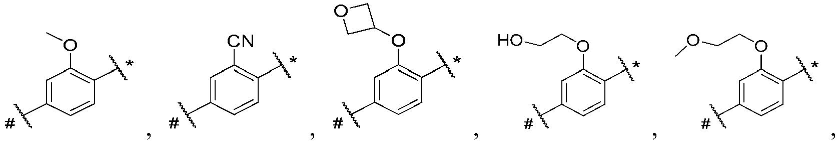

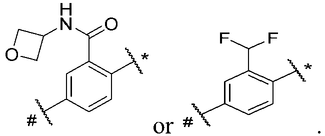

In some embodiments, in the definitions of formulae I and I-1, ring A is 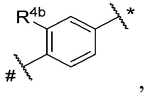 wherein R^{4b} is as defined in any of the embodiments of the present disclosure; for example, ring A may be

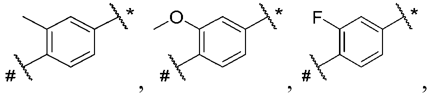

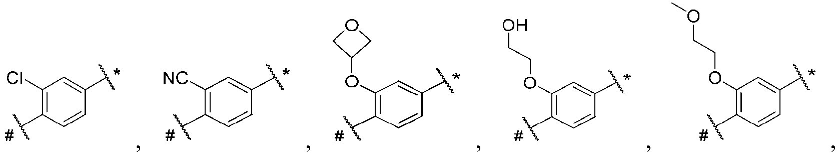

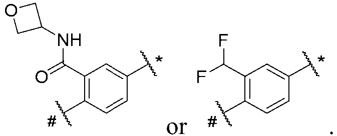

In some embodiments, in the definitions of formulae I and I-1, ring A is 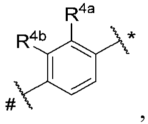 wherein R^{4a} and R^{4b} are as defined in any of the embodiments of the present disclosure; for example, ring A may be 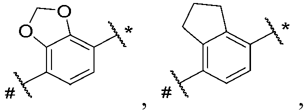 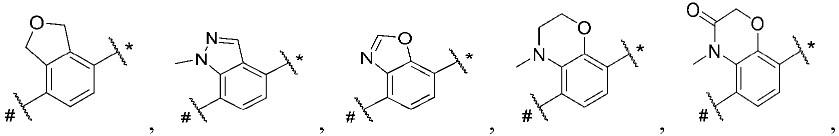 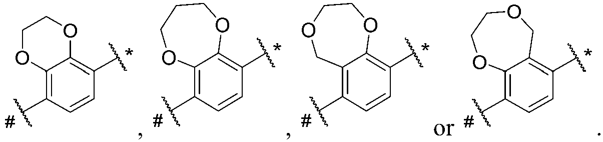 In some embodiments, in the definitions of formulae I and I-1, ring A is 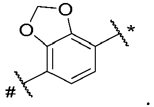 **In** some embodiments, in the definitions of formulae I and I-1, ring A is

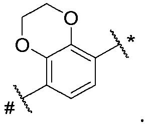

In some embodiments, in the definitions of formulae I and I-1, ring A is

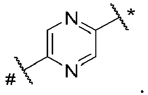

In some embodiments, in the definitions of formulae I and I-1, ring A is 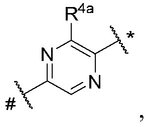 wherein R^{4a} is as defined in any of the embodiments of the present disclosure.

In some embodiments, in the definitions of formulae I and I-1, ring A is 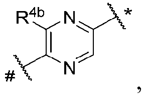 wherein R^{4b} is as defined in any of the embodiments of the present disclosure.

In some embodiments, in the definitions of formulae I and I-1, ring A is 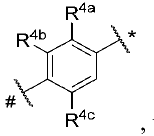 wherein R^{4a}, R^{4b} and R^{4c} are as defined in any of the embodiments of the present disclosure; for example, ring A may be

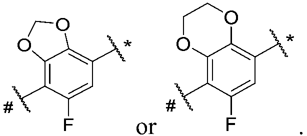

In some embodiments, in the definitions of formulae I and I-1, ring A is 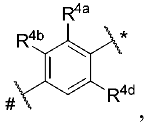 wherein R^{4a}, R^{4b} and R^{4d} are as defined in any of the embodiments of the present disclosure; for example, ring A may be

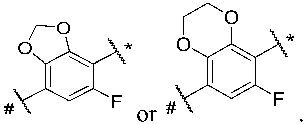

In some embodiments, in the definitions of formulae I and I-1, ring A is 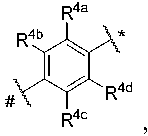 wherein R^{4a}, R^{4b}, R^{4c} and R^{4d} are as defined in any of the embodiments of the present disclosure; for example, ring A may be 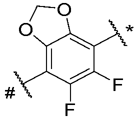 or

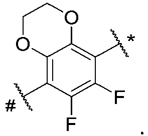

In some embodiments, in the definitions of formulae I and I-1, ring A is 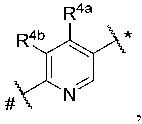 wherein R^{4a} and R^{4b} are as defined in any of the embodiments of the present disclosure; for example, ring A may be

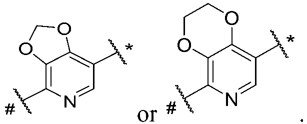

In some embodiments, in the definitions of formulae I and I-1, ring A is 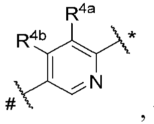 wherein R^{4a} and R^{4b} are as defined in any of the embodiments of the present disclosure; for example, ring A may be

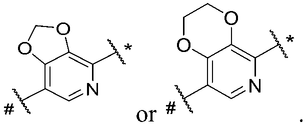

In some embodiments, in the definitions of formulae I and I-1, ring A is

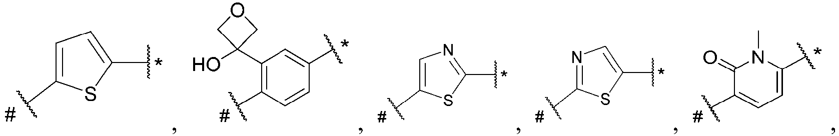

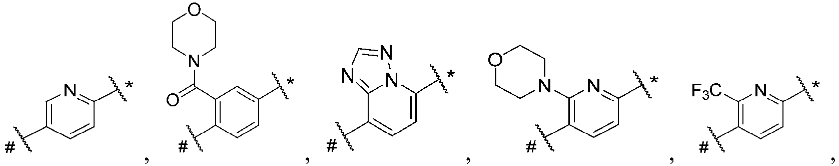

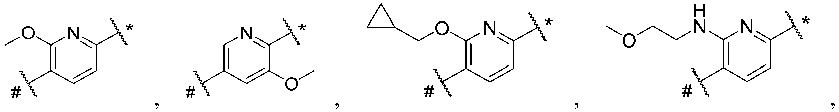

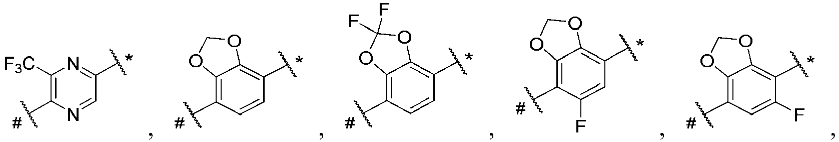

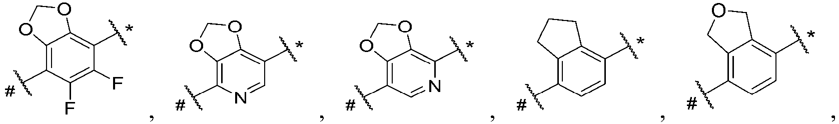

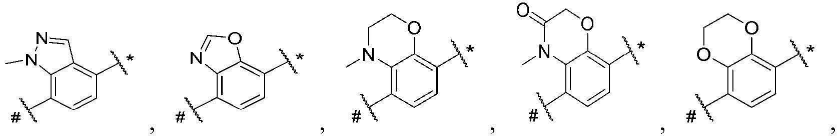

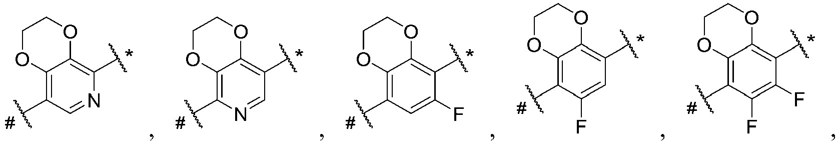

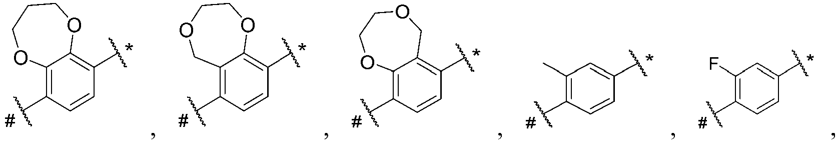

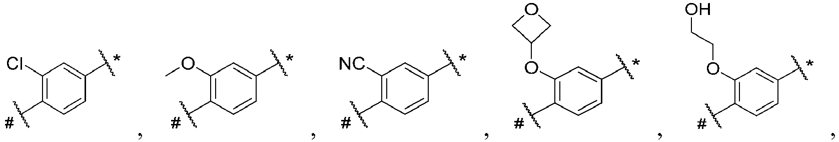

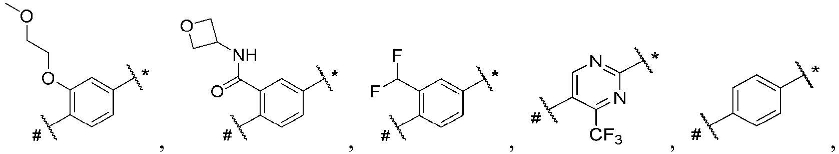

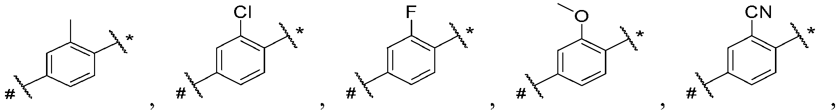

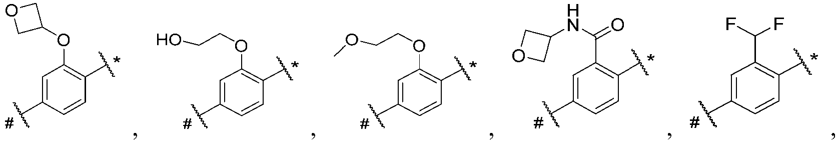

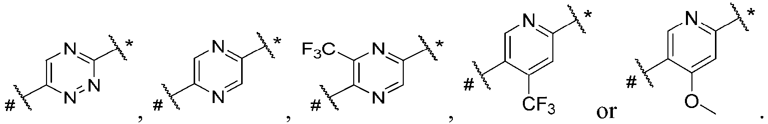

In some embodiments, in the definitions of formulae I and I-1, R^{2c} is hydrogen, halogen or cyano.

In some embodiments, in the definitions of formulae I and I-1, R^{2c} is hydrogen, fluorine or cyano, such as hydrogen or fluorine.

In some embodiments, in the definitions of formulae I and I-1, G¹ is CH or C(F). In some embodiments, in the definitions of formulae I and I-1, G¹ is CH.

In some embodiments, in the definitions of formulae I and I-1, G² is CH or C(F). In some embodiments, in the definitions of formulae I and I-1, G² is CH.

In some embodiments, in the definitions of formulae I and I-1, G³ is CH, C(F), C(CN) or N, such as CH or C(F). In some embodiments, in the definitions of formulae I and I-1, G³ is CH.

In some embodiments, in the definitions of formulae I and I-1, R^{2a} is halogen, cyano, C₁₋₆ alkyl or C₁₋₆ haloalkyl. In some embodiments, in the definitions of formulae I and I-1, R^{2a} is C₁₋₆ alkyl. In some embodiments, R^{2a} substituents, particularly halogen, methyl, etc., may maintain or enhance OTR target activity while reducing V1a activity, thereby improving OTR/V1a selectivity.

In some embodiments, in the definitions of formulae I and I-1, R^{2a} is methyl, monofluoromethyl, difluoromethyl, trifluoromethyl, chlorine or cyano. In some embodiments, in the definitions of formulae I and I-1, R^{2a} is methyl. In some embodiments, in the definitions of formulae I and I-1, R^{2a} is difluoromethyl. In some embodiments, in the definitions of formulae I and I-1, R^{2a} is trifluoromethyl. In some embodiments, in the definitions of formulae I and I-1, R^{2a} is chlorine. In some embodiments, in the definitions of formulae I and I-1, R^{2a} is cyano.

In some embodiments, in the definitions of formulae I and I-1, R^{2b} is hydrogen.

In some embodiments, in the definitions of formulae I and I-1, R^{2b} is C₁₋₆ alkyl, cyano, C₁₋₆ haloalkyl, C₁₋₆ alkoxy or halogen.

In some embodiments, R^{2b} substituents, particularly halogen, alkyl, alkoxy, cyano, etc., may maintain or enhance OTR target activity while reducing V1a activity, thereby improving OTR/V1a selectivity. In some embodiments, in the definitions of formulae I and I-1, R^{2b} is methyl, cyano, monofluoromethyl, difluoromethyl, trifluoromethyl, methoxy or chlorine. In some embodiments, in the definitions of formulae I and I-1, R^{2b} is methyl. In some embodiments, in the definitions of formulae I and I-1, R^{2b} is cyano. In some embodiments, in the definitions of formulae I and I-1, R^{2b} is chlorine. In some embodiments, in the definitions of formulae I and I-1, R^{2b} is trifluoromethyl. In some embodiments, in the definitions of formulae I and I-1, R^{2b} is methoxy. In some embodiments, in the definitions of formulae I and I-1, R^{2b} is difluoromethyl.

In some embodiments, in the definitions of formulae I and I-1, R^{2b} and R^{2a} are defined as any of the following combinations (i) - (x);
(i) R^{2a} is methyl; R^{2b} is methyl;
(ii) R^{2a} is methyl; R^{2b} is chlorine;
(iii) R^{2a} is methyl; R^{2b} is cyano;
(iv) R^{2a} is methyl; R^{2b} is difluoromethyl;
(v) R^{2a} is methyl; R^{2b} is trifluoromethyl;
(vi) R^{2a} is methyl; R^{2b} is methoxy;
(vii) R^{2a} is chlorine; R^{2b} is cyano;
(viii) R^{2a} is cyano; R^{2b} is methyl;
(ix) R^{2a} is difluoromethyl; R^{2b} is cyano;
(x) R^{2a} is trifluoromethyl; R^{2b} is cyano.

In some embodiments, in the definitions of formulae I and I-1, R^{2b} and R^{2a}, together with the carbon atom to which they are attached, form C₃₋₈ cycloalkyl, such as cyclopentyl.

In some embodiments, in the definitions of formulae I and I-1, 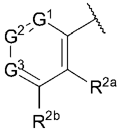 has a structural moiety of

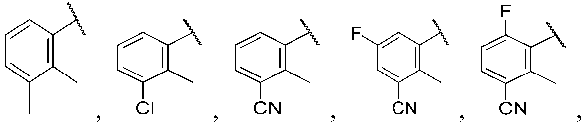

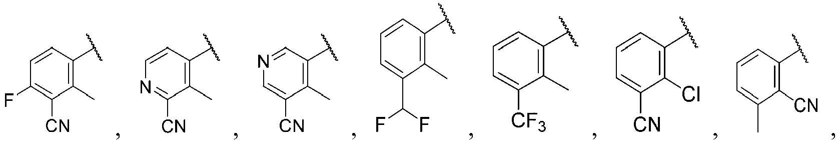

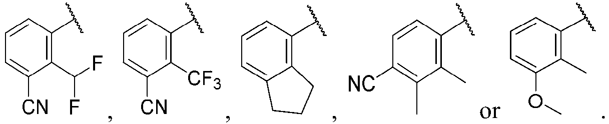

In some embodiments, in the definitions of formulae I and I-1, 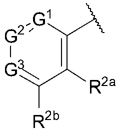 has a structural moiety of 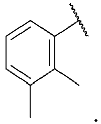 In some embodiments, in the definitions of formulae I and I-1, 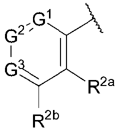 has a structural moiety of 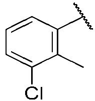 . In some embodiments, in the definitions of formulae I and I-1, 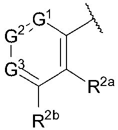 has a structural moiety of 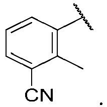 In some embodiments, in the definitions of formulae I and I-1, 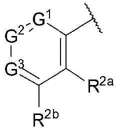 has a structural moiety of 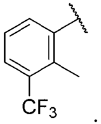 In some embodiments, in the definitions of formulae I and I-1, 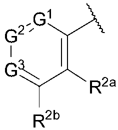 has a structural moiety of 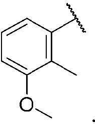 In some embodiments, in the definitions of formulae I and I-1, 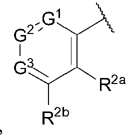 has a structural moiety of 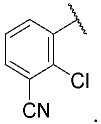 In some embodiments, in the definitions of formulae I and I-1, 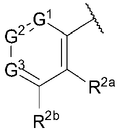 has a structural moiety of

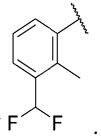

In some embodiments, in the definitions of formulae I and 1-1, 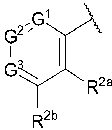 has a structural moiety of

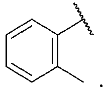

In some embodiments, provided is a compound as shown in formula I, or an isotopic derivative or a pharmaceutically acceptable salt, 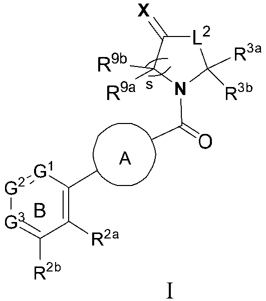
wherein X is N-OR¹;
R¹ is -CH₃;
R^{3a} is selected from hydrogen or C₁₋₆ alkyl;
R^{3b} is -L¹-R^{3c};
L¹ is independently -[C(R^{a})(R^{b})]ₙ-; wherein n is 1; R^{a} and R^{b} are each independently hydrogen or -CH₃;
R^{3c} is independently -OR^{7a}; the R^{7a} is selected from hydrogen or C₁₋₆ alkyl;
L² is -[C(R^{10a})(R^{10b})]ₜ-;
s is 1 and t is 1;
R^{9a}, R^{9b} , R^{10a} and R^{10b} are hydrogen;
ring A is 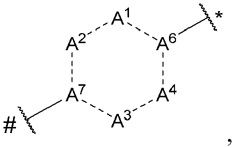 wherein the * end is connected to the carbonyl group in formula I, and the # end is connected to ring B in formula I; each ----bond is independently a single bond or a double bond;
A¹ is CH or C(R^{4a});
A² is CH or C(R^{4b});
A³ is CH;
A⁴ is CH;
each A⁶ is independently C or N;
each A⁷ is independently C or N;
R^{4a} and R^{4b} are each independently H; alternatively, R^{4a} and R^{4b}, together with the atom to which they are attached, form 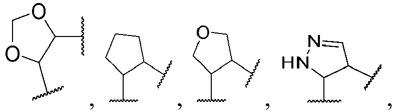 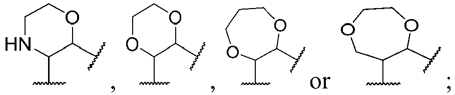
in ring B, G¹, G² and G³ are CH;
R^{2a} is halogen or -CH₃;
R^{2b} is cyano.

In some embodiments, formulae I and I-1 are represented by formula Ia, a stereoisomer thereof formula Ib, or a mixture of formula Ia and formula Ib: 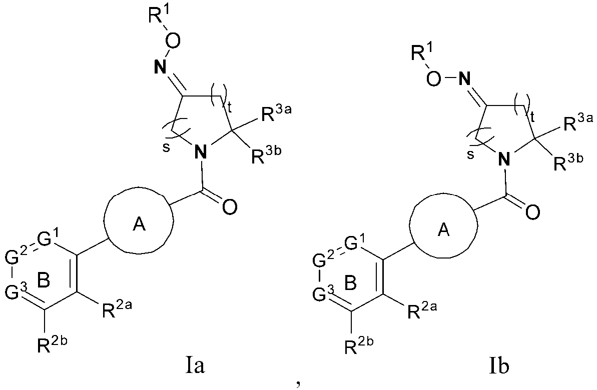 wherein R¹, R^{2a}, R²⁸, R^{3a} , R^{3b}, s, t, G¹, G², G³ and ring A are as defined in formulae I and I-1.

In some embodiments, in formulae I, I-1, Ia and Ib, when the carbon atom to which R^{3a} and R^{3b} are attached is a chiral carbon atom, it may be in the *R* configuration, *S* configuration, or a mixture of both. In some embodiments, in formulae I, I-1, Ia and Ib, when the carbon atom to which R^{3a} and R^{3b} are attached is a chiral carbon atom, it may be in the *S* configuration.

In some embodiments, the compound is any of the following compounds or a mixture thereof (e.g., a mixture of formula Ia and a stereoisomer thereof formula Ib):

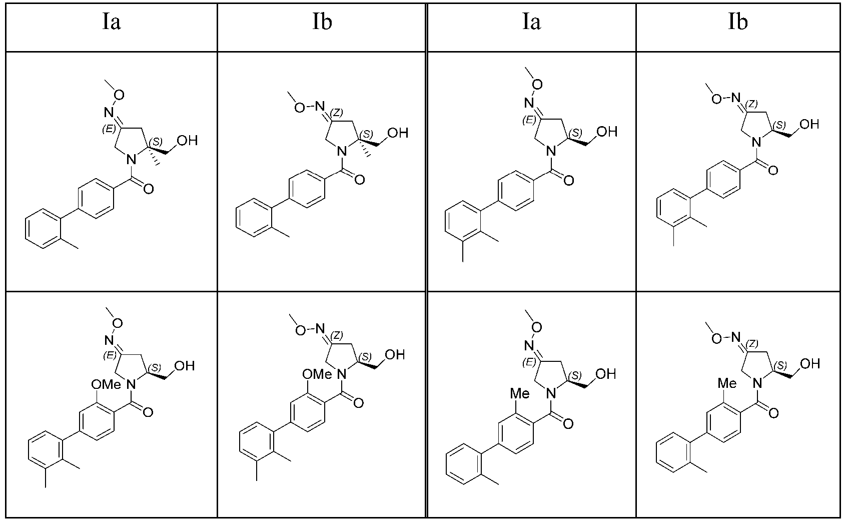

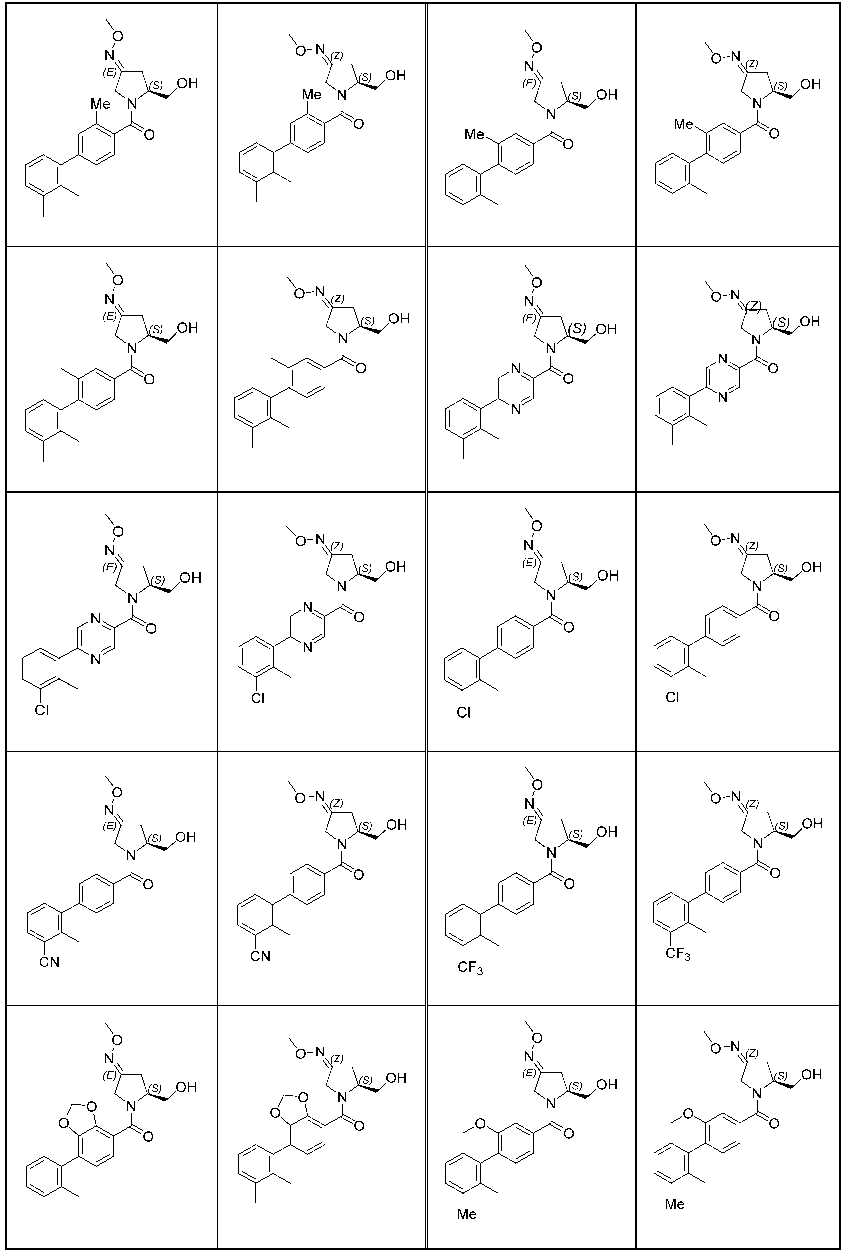

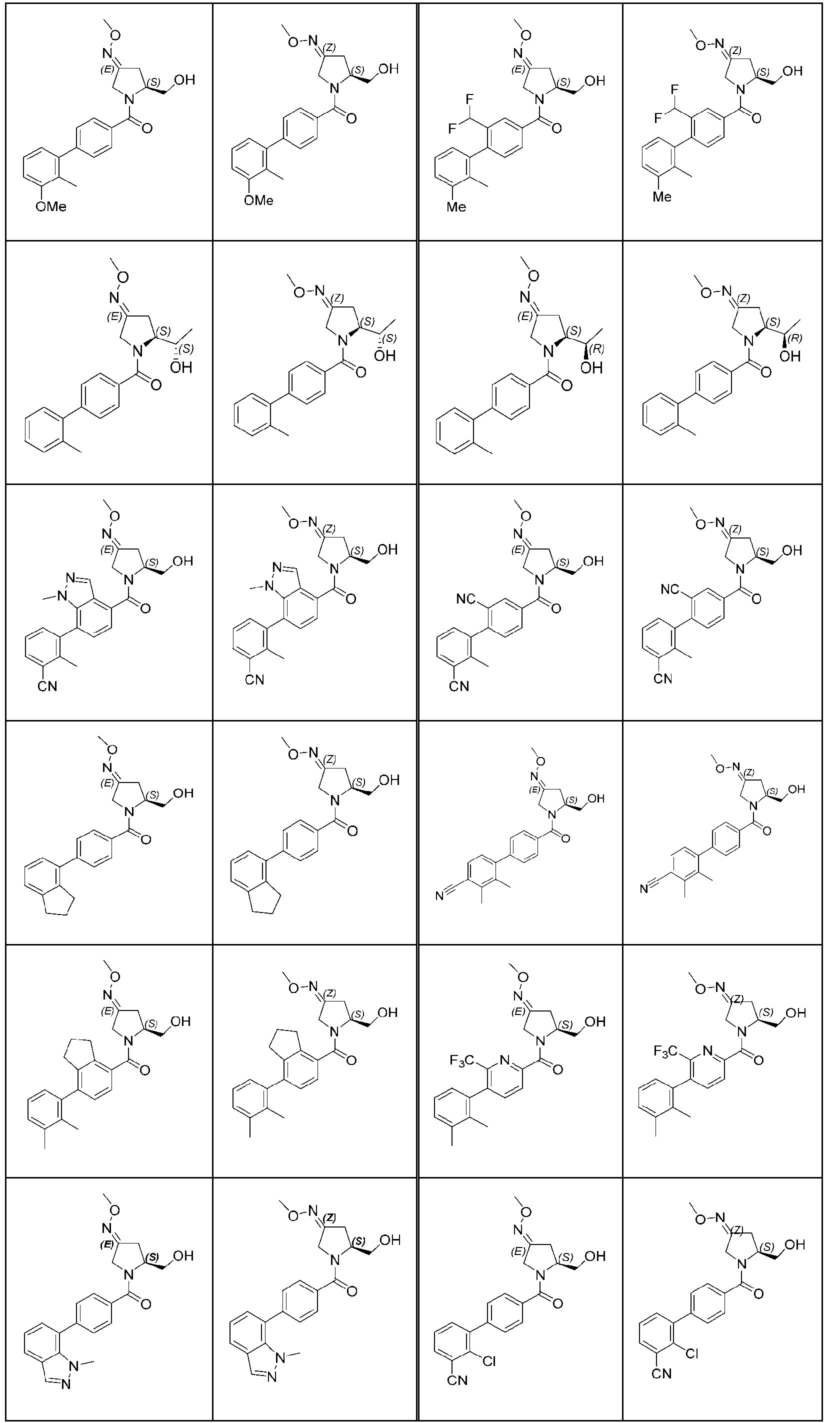

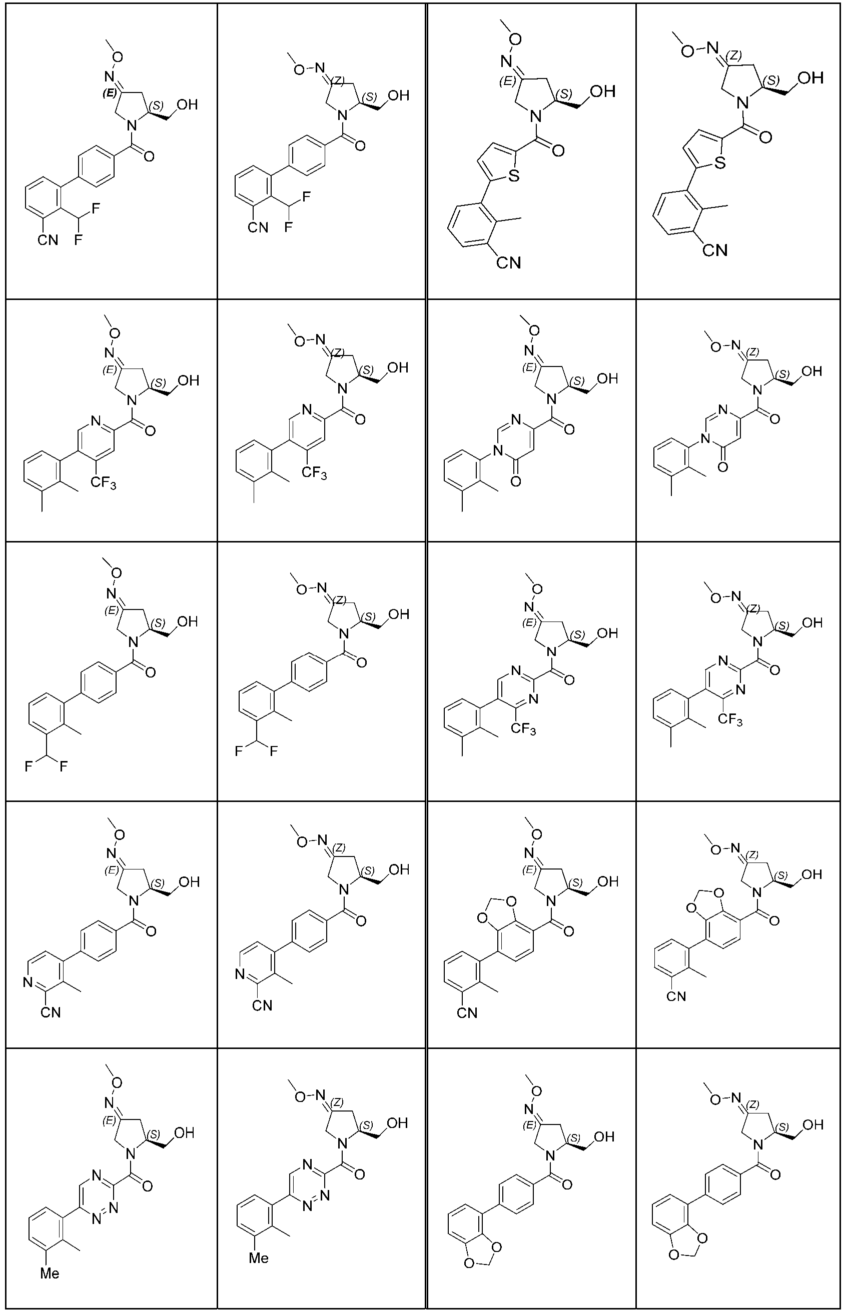

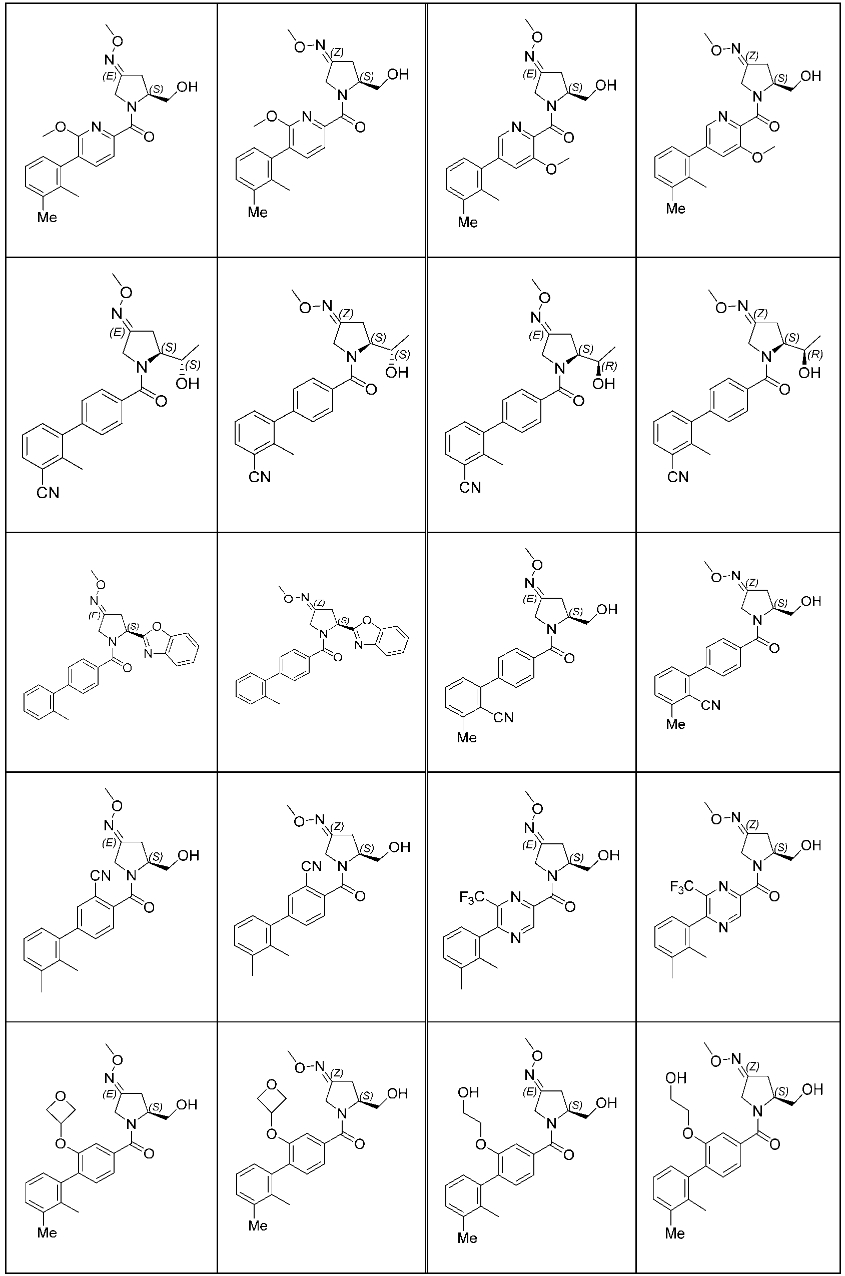

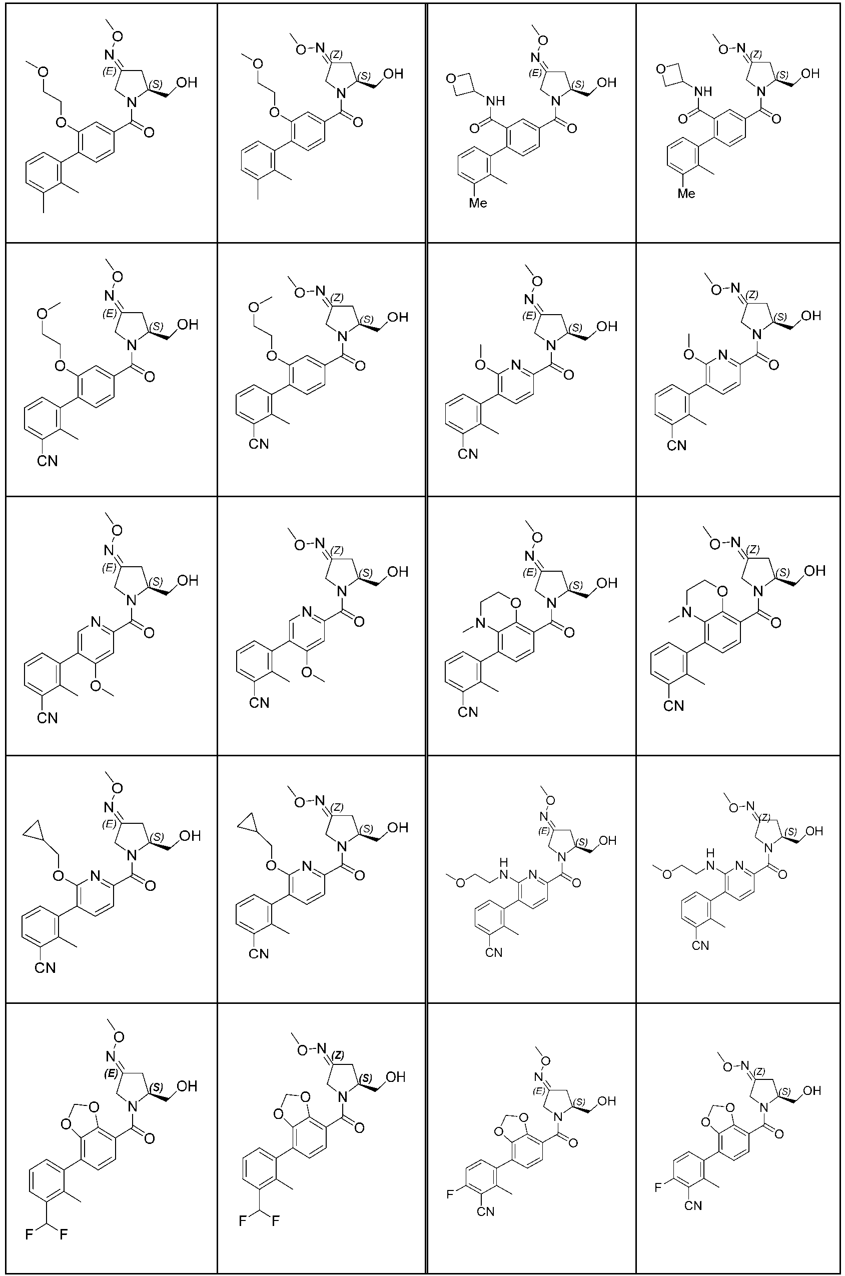

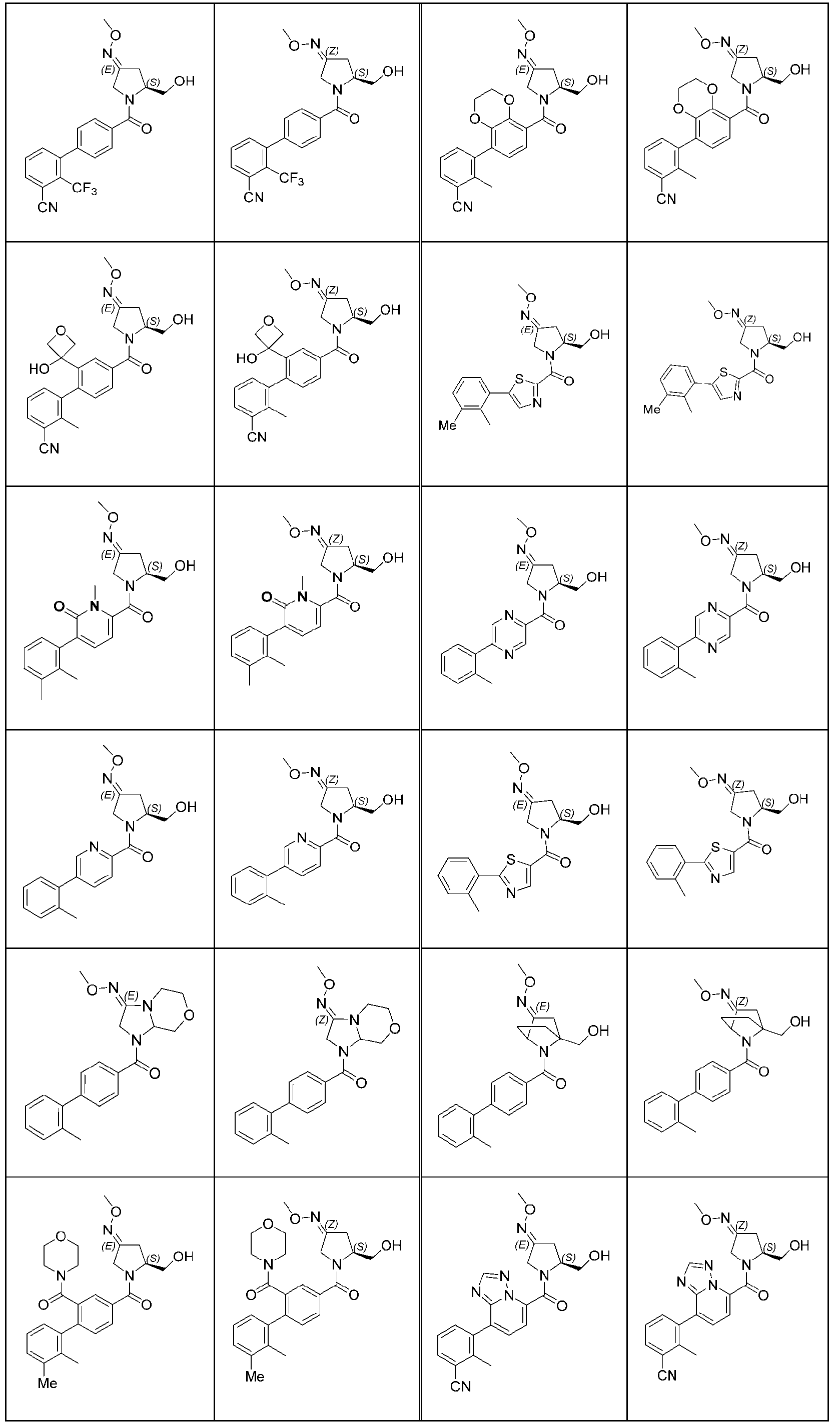

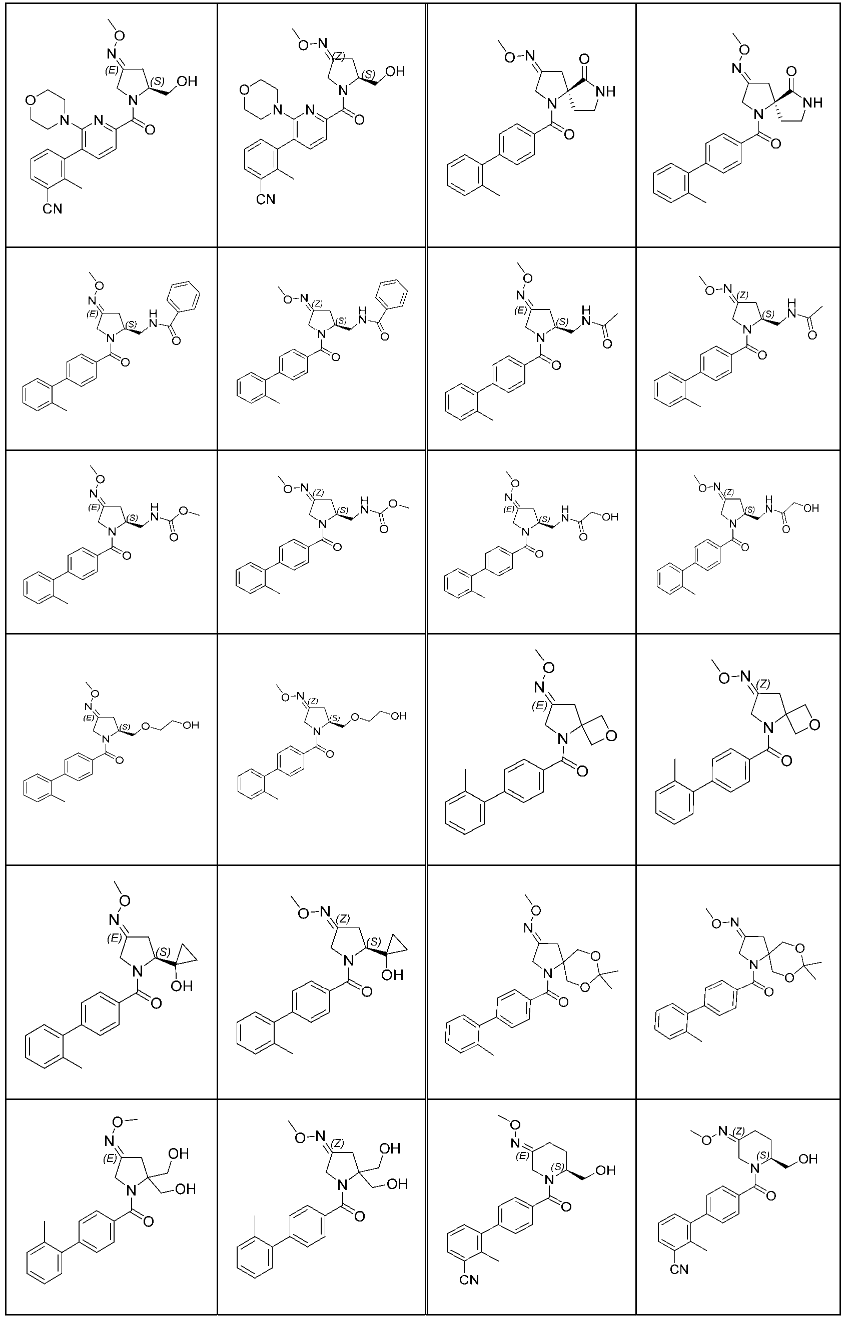

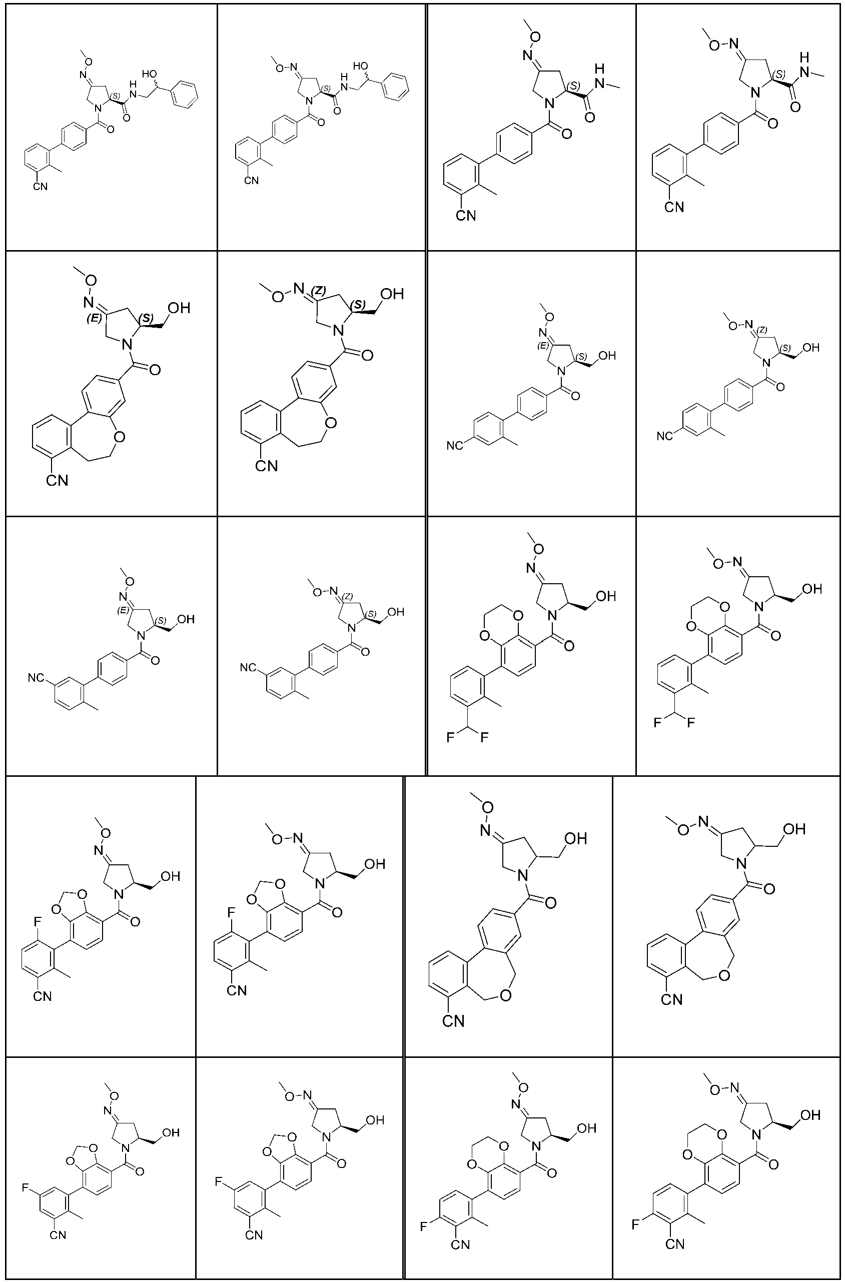

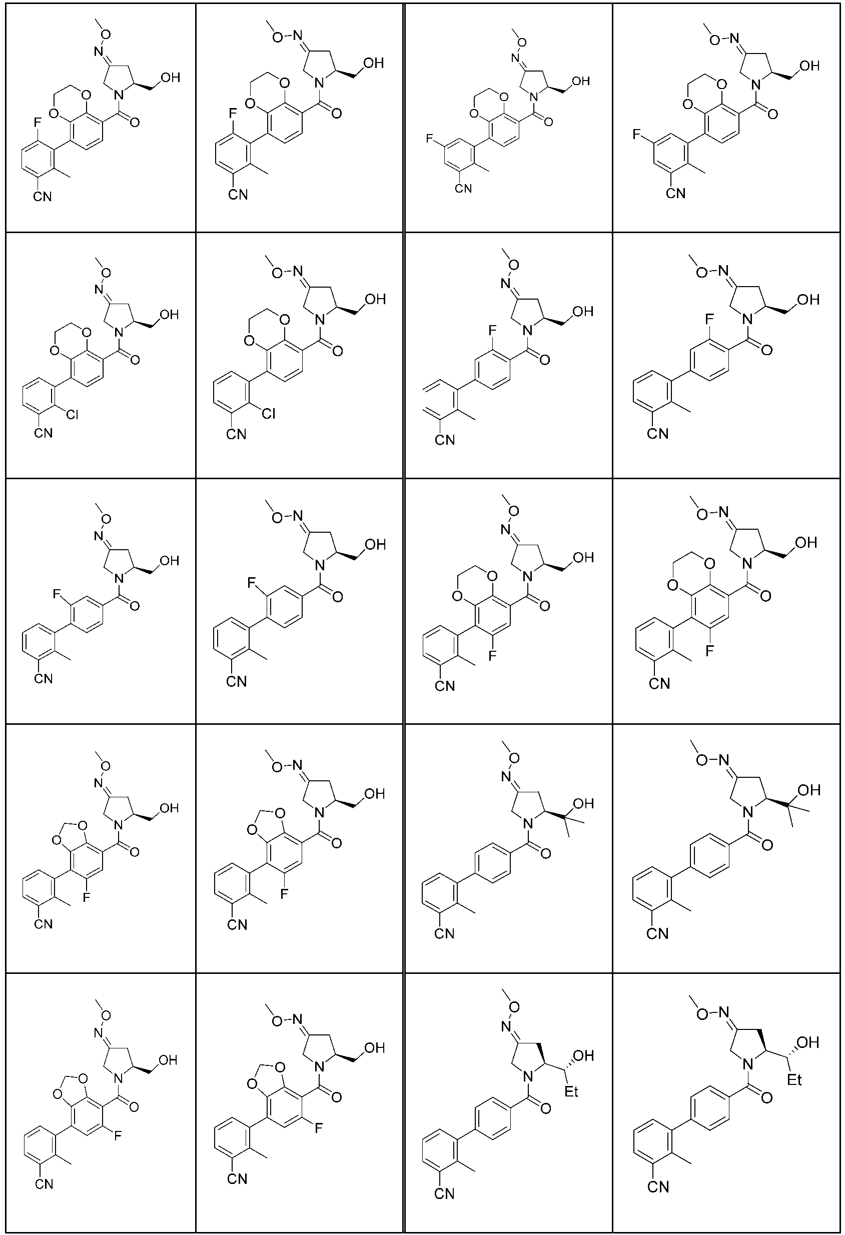

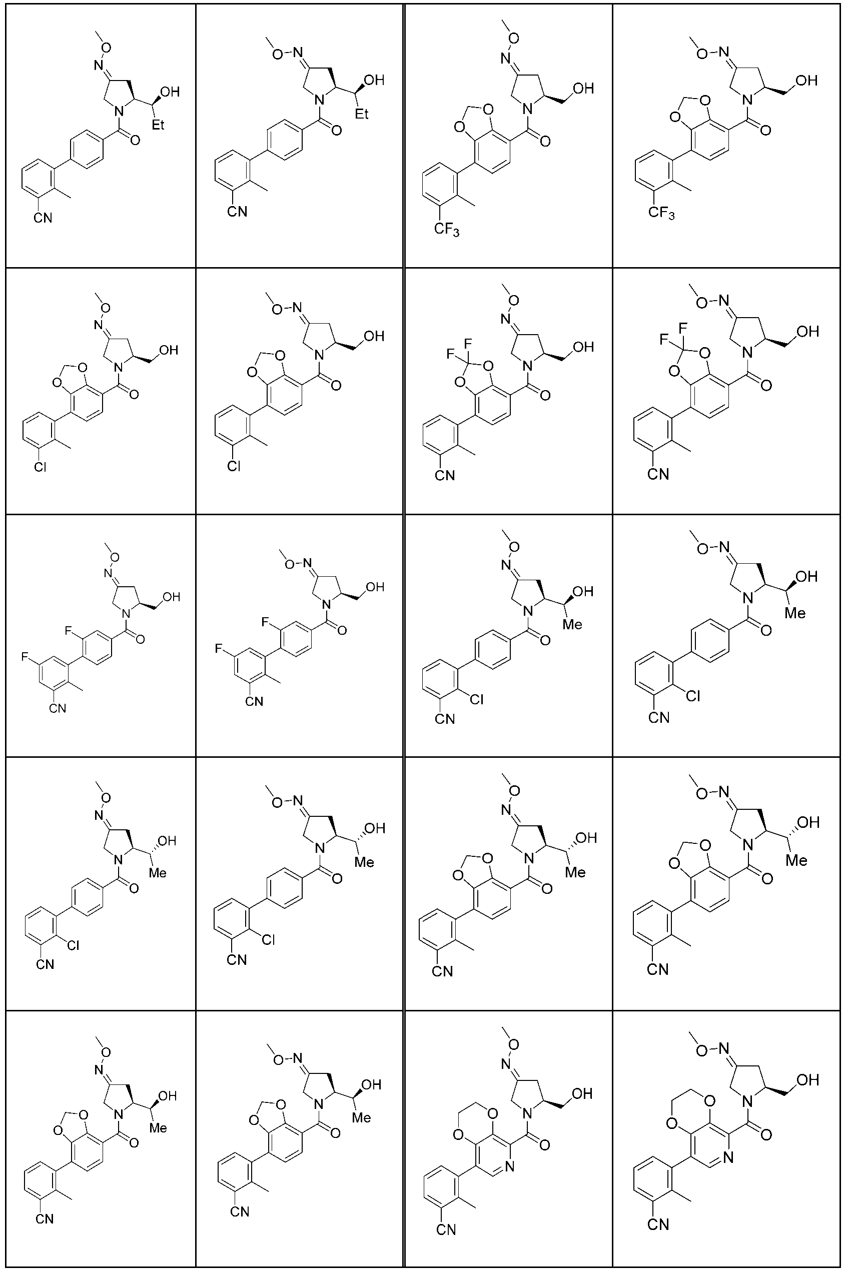

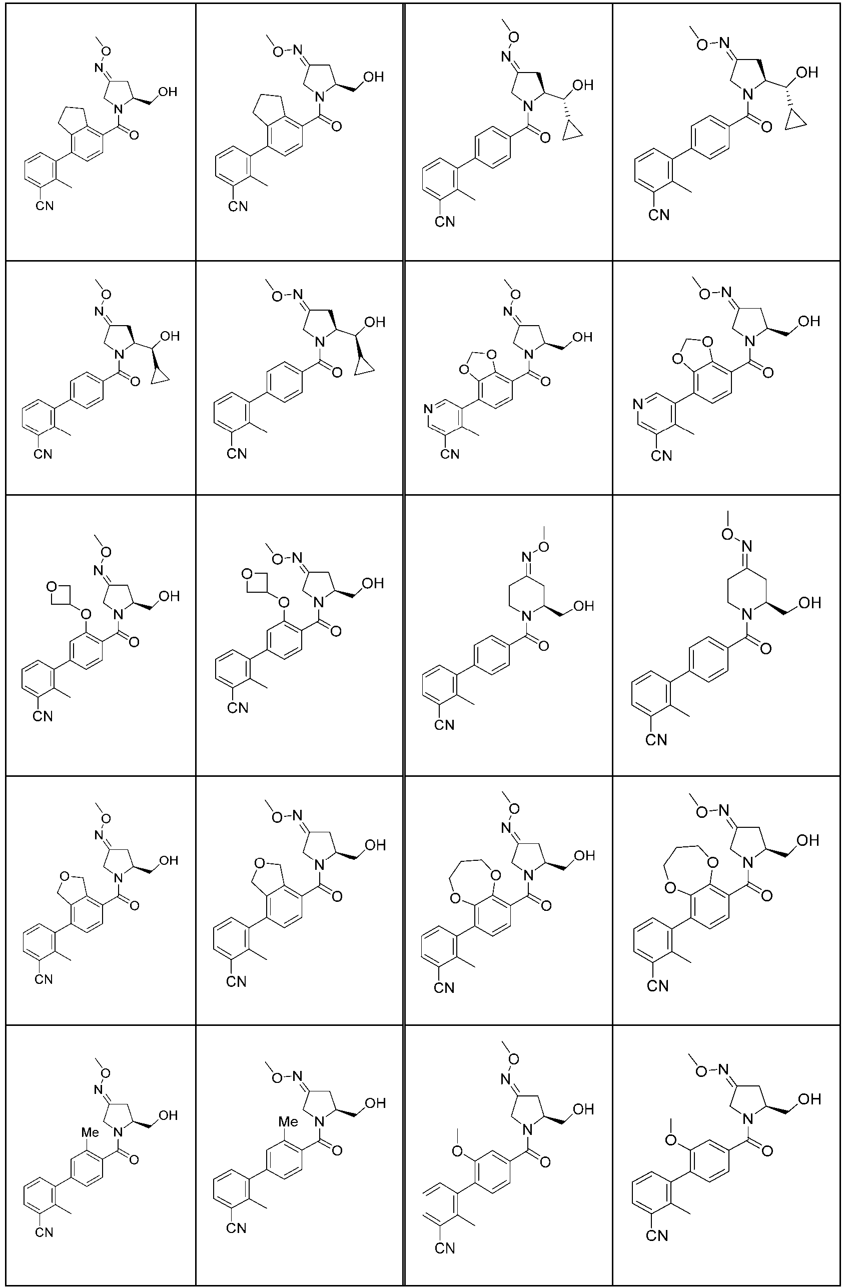

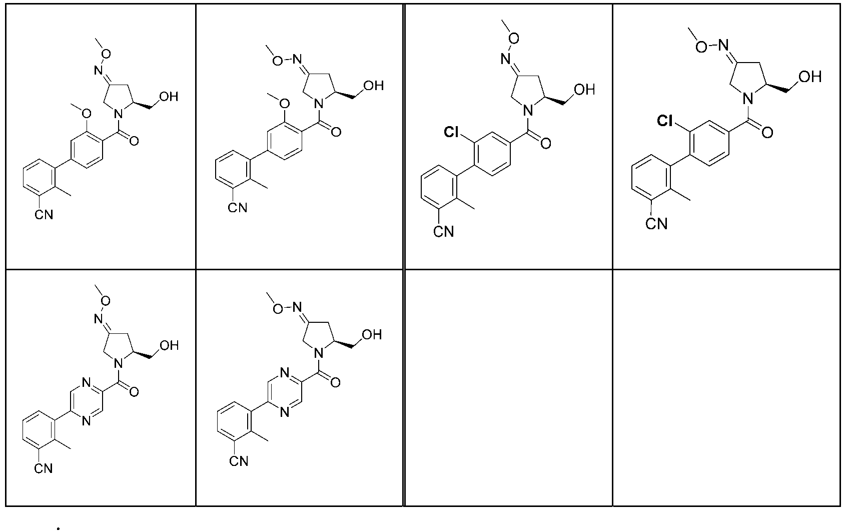

In some embodiments, formulae I and I-1 are either the one with better oxytocin receptor antagonistic activity between formula Ia and a stereoisomer thereof formula Ib, or a mixture of the one with better oxytocin receptor antagonistic activity and the other; wherein the content of the one with better oxytocin receptor antagonistic activity in the mixture is not less than that of the other. The method for measuring oxytocin receptor antagonistic activity is well known in the art, such as the method disclosed in Biological test example 1 herein.

In some embodiments, formulae I and I-1 are either the more polar one between formula Ia and a stereoisomer thereof formula Ib, or a mixture of the more polar one and the less polar one; wherein the content of the more polar one in the mixture is not less than that of the less polar one. In some embodiments, formulae I and I-1 are either the less polar one between formula Ia and a stereoisomer thereof formula Ib, or a mixture of the less polar one and the more polar one; wherein the content of the less polar one in the mixture is not less than that of the more polar one. The relative polarity of two molecules may be determined by experimental methods well known in the art; exemplary methods include silica gel thin-layer chromatography, reversed-phase HPLC, and the like.

In some embodiments, the isotopic derivative has any of the following structures:

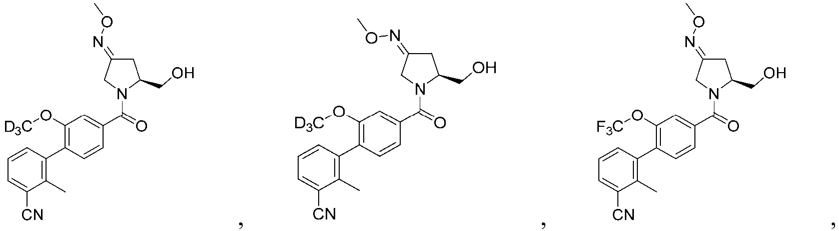

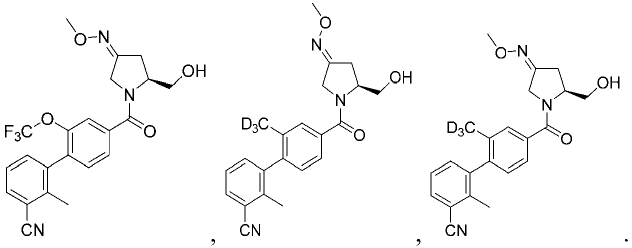

The present disclosure further provides a pharmaceutical composition, comprising the aforementioned compound, or the isotopic derivative or the pharmaceutically acceptable salt, and a pharmaceutical auxiliary material.

The present disclosure further provides the aforementioned compound, or the isotopic derivative or the pharmaceutically acceptable salt, for use as a medicament.

The present disclosure further provides the use of the aforementioned compound, or the isotopic derivative or the pharmaceutically acceptable salt, or the pharmaceutical composition comprising same in the preparation of an oxytocin receptor antagonist.

The present disclosure further provides the use of the aforementioned compound, or the isotopic derivative or the pharmaceutically acceptable salt in the preparation of a medicament for preventing and/or treating a disease or condition for which inhibition of oxytocin is known or demonstrated to produce a beneficial effect.

The present disclosure further provides a method for preventing and/or treating a disease or condition for which inhibition of oxytocin is known or demonstrated to produce a beneficial effect, comprising administering to a subject an effective amount of the compound, or the isotopic derivative or the pharmaceutically acceptable salt.

In some embodiments, the disease or condition is sexual dysfunction, hypoactive sexual desire disorder, sexual arousal disorder, orgasm disorder, dyspareunia, premature ejaculation, preterm delivery, delivery complications, appetite and eating disorders, benign prostatic hyperplasia, premature delivery, dysmenorrhea, congestive heart failure, arterial hypertension, liver cirrhosis, renal hypertension, ocular hypertension, obsessive-compulsive disorder or neuropsychiatric disease.

In some embodiments, the disease or condition is premature delivery.

Without departing from common knowledge in the art, any combination of the aforementioned embodiments or preferred conditions may be employed to obtain various preferred examples of the present disclosure.

The compound as shown in formula I of the present disclosure may be prepared with reference to the methods in the Examples and methods disclosed in the art. An exemplary method is as follows:

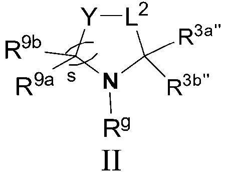

in formula II, Y is CH(OH) or C(=N-OR¹); when Y is CH(OH), it may react with NH₂-OR¹ to form C(=N-OR¹); wherein s, L², R^{9a}, R^{9b} and R¹ are as defined in formula I;
in formula II, R^{g} is 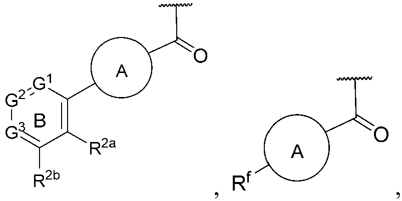 hydrogen or an amino protecting group; when R^{g} is an amino protecting group, the amino protecting group may be removed to form hydrogen; when R^{g} is hydrogen, it may undergo an acylation reaction with 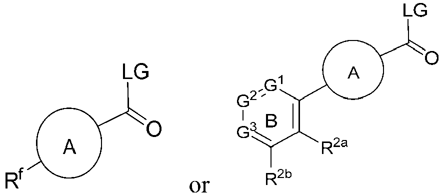 to form 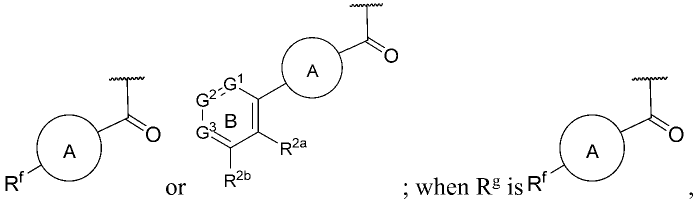 it may couple with 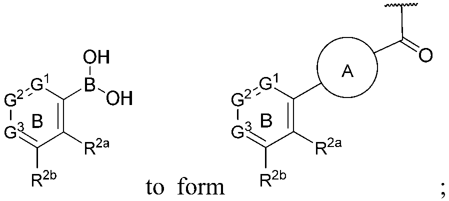 wherein R^{f} may be bromine or iodine; LG may be a leaving group, such as chlorine; ring A, R^{2a}, R^{2b}, G¹, G² and G³ are as defined in formula I;
in formula II, R^{3a"} is R^{3a} or a precursor group that may be converted into R^{3a} by reactions known in the art; for example, when R^{3a"} is hydrogen, a compound of formula II may react with C₁₋₆ alkyl iodide or I-L¹-R^{3c} to form C₁₋₆ alkyl or -L¹-R^{3c}; wherein R^{3a}, L¹ and R^{3c} are as defined in formula I;
in formula II, R^{3b"} is R^{3b} or a precursor group that may be converted into R^{3b} by reactions known in the art; for example, in some embodiments, R^{3b"} may be - COOMe, which may be reduced to -CH₂OH, and -CH₂OH may be further converted into various groups.

### Definition and Description

Unless otherwise stated, the following terms and phrases used herein are intended to have the following meanings. A specific term or phrase should not be considered uncertain or unclear unless specifically defined, but should be understood in its ordinary meaning.

Where any variable (e.g., R) appears more than once in the structure of a compound, its definition in each case is independent. For example, if a group is substituted with 0-2 R, then the group may optionally be substituted with up to two R, and R in each case is independently selected.

The term "substituted" or "substituent" means that a hydrogen atom in a group is replaced by a specified group. When the substitution position is not specified, substitution may occur at any position, provided that only a stable or chemically feasible compound is permitted. Taking the structure 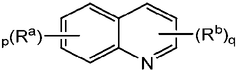 as an example, it indicates that the hydrogen atoms on the benzene ring are substituted with p R^{a}, and the hydrogen atoms on the pyridine ring are substituted with q R^{b}, wherein each R^{a} and R^{b} is independently defined. When describing that a group is substituted with "one or more" of the listed substituents, the number of substituents may be any number that is chemically feasible, for example, the number of substituents may be 1, 2, 3 or 4.

The term "optional" or "optionally" means that the subsequently described event or circumstance may, but not necessarily occur, and that the description includes instances where said event or circumstance occurs and instances where said event or circumstance does not occur. Therefore, the term "optionally substituted" means that the group may or may not be substituted.

The term "halogen" refers to fluorine, chlorine, bromine or iodine.

The term "oxo" refers to =O.

The term "amino" refers to -NH₂.

The term "hydroxyl" refers to -OH.

The term "nitro" refers to -NO₂.

The term "cyano" refers to -CN.

The term "alkyl" refers to a saturated straight or branched monovalent hydrocarbon group. The C₁₋₆ alkyl herein may be C₁, C₂, C₃, C₄, C₅ or C₆ alkyl, and examples thereof include, but are not limited to methyl, ethyl, n-propyl, isopropyl, n-butyl, isobutyl, sec-butyl, and tert-butyl.

The term "alkenyl" refers to a straight or branched monovalent hydrocarbon group containing at least one carbon-carbon double bond. The alkenyl may be attached to other structures via any carbon atom (such as saturated carbon atoms and double-bond carbon atoms) it contains, provided that only a stable or chemically feasible compound is permitted. The C₂₋₆ alkenyl herein may be C₂, C₃, C₄, C₅ or C₆ alkenyl, and examples thereof include, but are not limited to ethenyl, 1-propenyl, and 2-propenyl.

The term "alkynyl" refers to a straight or branched monovalent hydrocarbon group containing at least one carbon-carbon triple bond. The alkynyl may be attached to other structures via any carbon atom (such as saturated carbon atoms and triple-bond carbon atoms) it contains, provided that only a stable or chemically feasible compound is permitted. The C₂₋₆ alkynyl herein may be C₂, C₃, C₄, C₅ or C₆ alkynyl, and examples thereof include, but are not limited to ethynyl.

The term "alkoxy" refers to -O-alkyl, wherein the alkyl is as defined above. The C₁₋₆ alkoxy herein may be C₁, C₂, C₃, C₄, C₅ or C₆ alkoxy, and examples thereof include, but are not limited to methoxy, ethoxy, n-propoxy, isopropoxy, n-butoxy, isobutoxy, sec-butoxy, and tert-butoxy.

The term "haloalkyl" refers to alkyl substituted with one or more halogen, wherein the alkyl and halogen are as defined above. The C₁₋₆ haloalkyl herein may be halogenated C₁, C₂, C₃, C₄, C₅ or C₆ alkyl, and examples thereof include, but are not limited to fluoromethyl (such as monofluoromethyl, difluoromethyl, and trifluoromethyl), fluoroethyl (such as 1-fluoroethyl, 1,1-difluoroethyl, 2,2,2-trifluoroethyl or pentafluoroethyl), and 1-fluoro-2-chloroethyl. In some embodiments, the C₁₋₆ haloalkyl is C₁, C₂, C₃, C₄, C₅ or C₆ fluoroalkyl.

The term "haloalkenyl" refers to alkenyl substituted with one or more halogen, wherein the alkenyl and halogen are as defined above. The halogenated C₂₋₆ alkenyl herein may be halogenated C₂, C₃, C₄, C₅ or C₆ alkenyl, and examples thereof include, but are not limited to 1-fluoroethenyl, 2-fluoroethenyl, and 1-fluoro-2-chloroethenyl. In some embodiments, the halogenated C₂₋₆ alkenyl is fluorinated C₂, C₃, C₄, C₅ or C₆ alkenyl.

The term "haloalkynyl" refers to alkynyl substituted with one or more halogen, wherein the alkynyl and halogen are as defined above. The halogenated C₂₋₆ alkynyl herein may be halogenated C₂, C₃, C₄, C₅ or C₆ alkynyl. In some embodiments, the halogenated C₂₋₆ alkynyl is fluorinated C₂, C₃, C₄, C₅ or C₆ alkynyl.

The term "hydroxyalkyl" refers to alkyl substituted with one or more hydroxyl, wherein the alkyl is as defined above. The C₁₋₆ hydroxyalkyl herein may be C₁, C₂, C₃, C₄, C₅ or C₆ alkyl substituted with one or more hydroxyl, and examples thereof include, but are not limited to hydroxymethyl and hydroxyethyl.

The term "haloalkoxy" refers to alkoxy substituted with one or more halogen, wherein the alkoxy and halogen are as defined above. The halogenated C₁₋₆ alkoxy herein may be halogenated C₁, C₂, C₃, C₄, C₅ or C₆ alkoxy, and examples thereof include, but are not limited to trifluoromethoxy. In some embodiments, the halogenated C₁₋₆ alkoxy is fluorinated C₁, C₂, C₃, C₄, C₅ or C₆ alkoxy.

The term "cycloalkyl" refers to a saturated monocyclic or polycyclic (such as fused, spiro, or bridged) cyclic hydrocarbon group. The C₃₋₈ cycloalkyl herein may be C₃, C₄, C₅, C₆, C₇ or C₈ cycloalkyl, such as C₃ monocyclic cycloalkyl, C₄ monocyclic cycloalkyl, C₅ monocyclic or polycyclic cycloalkyl, C₆ monocyclic or polycyclic cycloalkyl, C₇ monocyclic or polycyclic cycloalkyl, or C₈ monocyclic or polycyclic cycloalkyl, and examples thereof include, but are not limited to cyclopropyl, cyclobutyl, cyclopentyl, and cyclohexyl.

The term "cycloalkenyl" refers to a non-aromatic monocyclic or polycyclic (such as fused, spiro, or bridged) cyclic hydrocarbon group containing at least one carbon-carbon double bond. The cycloalkenyl may be attached to other structures via any atom (such as saturated carbon atoms and double-bond carbon atoms) on the ring, provided that only a stable or chemically feasible compound is permitted. The C₃₋₈ cycloalkenyl herein may be C₃, C₄, C₅, C₆, C₇ or C₈ cycloalkenyl, such as C₃ monocyclic cycloalkenyl, C₄ monocyclic cycloalkenyl, C₅ monocyclic or polycyclic cycloalkenyl, C₆ monocyclic or polycyclic cycloalkenyl, C₇ monocyclic or polycyclic cycloalkenyl, or C₈ monocyclic or polycyclic cycloalkenyl, and examples thereof include, but are not limited to cyclopropenyl, cyclobutenyl, cyclopentenyl, and cyclohexenyl.

The term "heterocycloalkyl" refers to a saturated monocyclic or polycyclic (such as fused, spiro, or bridged) cyclic group formed by carbon atoms and at least one heteroatom. In some embodiments, the heteroatoms in the heterocycloalkyl are independently selected from N, O and S. The heterocycloalkyl may be attached to other structures via any atom (such as carbon atoms and heteroatoms) on the ring, provided that only a stable or chemically feasible compound is permitted. The 3- to 8-membered heterocycloalkyl herein may be 3-, 4-, 5-, 6-, 7- or 8-membered heterocycloalkyl, such as 3-membered monocyclic heterocycloalkyl, 4-membered monocyclic heterocycloalkyl, 5-membered monocyclic or polycyclic heterocycloalkyl, 6-membered monocyclic or polycyclic heterocycloalkyl, 7-membered monocyclic or polycyclic heterocycloalkyl, or 8-membered monocyclic or polycyclic heterocycloalkyl, and examples thereof include, but are not limited to oxetanyl, azetidinyl, tetrahydrofuranyl, tetrahydrothienyl, tetrahydropyrrolyl, piperidyl, piperazinyl, and morpholinyl.

The term "heterocycloalkenyl" refers to a non-aromatic monocyclic or polycyclic (such as fused, spiro or bridged) cyclic group formed by carbon atoms and at least one heteroatom and containing at least one double bond; the atoms attached at both ends of the double bond may be carbon atoms or heteroatoms. In some embodiments, the heteroatoms in the heterocycloalkenyl are independently selected from N, O and S. The heterocycloalkenyl may be attached to other structures via any atom (such as saturated carbon atoms, double-bond carbon atoms and heteroatoms) on the ring, provided that only a stable or chemically feasible compound is permitted. The 3- to 8-membered heterocycloalkenyl herein may be 3-, 4-, 5-, 6-, 7- or 8-membered heterocycloalkenyl, such as 3-membered monocyclic heterocycloalkenyl, 4-membered monocyclic heterocycloalkenyl, 5-membered monocyclic or polycyclic heterocycloalkenyl, 6-membered monocyclic or polycyclic heterocycloalkenyl, 7-membered monocyclic or polycyclic heterocycloalkenyl, or 8-membered monocyclic or polycyclic heterocycloalkenyl, and examples thereof include, but are not limited to dioxolyl, dioxinyl, dihydroimidazolyl, dihydrofuranyl, dihydropyrazolyl, and dihydropyrrolyl.

The term "heteroaryl" refers to an aromatic monocyclic or fused cyclic group formed by carbon atoms and at least one heteroatom. In some embodiments, the heteroatoms in the heteroaryl are independently selected from N, O and S. The heteroaryl may be attached to other structures via any atom (such as carbon atoms and heteroatoms) on the ring, provided that only a stable or chemically feasible compound is permitted. The 5- to 6-membered heteroaryl herein may be 5-membered heteroaryl or 6-membered heteroaryl, and examples thereof include, but are not limited to pyrrolyl (such as pyrrol-1-yl, pyrrol-2-yl, and pyrrol-3-yl), furanyl (such as furan-2-yl and furan-3-yl), thienyl (such as thien-2-yl and thien-3-yl), oxazolyl (such as oxazol-2-yl, oxazol-4-yl, and oxazol-5-yl), isoxazolyl (such as isoxazol-3-yl, isoxazol-4-yl, and isoxazol-5-yl), thiazolyl (such as thiazol-2-yl, thiazol-4-yl, and thiazol-5-yl), isothiazolyl (such as isothiazol-3-yl, isothiazol-4-yl, and isothiazol-5-yl), pyrazolyl (such as pyrazol-1-yl, pyrazol-3-yl, pyrazol-4-yl, and pyrazol-5-yl), imidazolyl (such as imidazol-1-yl, imidazol-2-yl, imidazol-4-yl, and imidazol-5-yl), oxadiazolyl (such as 1,2,3-oxadiazolyl, 1,2,4-oxadiazolyl, and 1,3,4-oxadiazolyl), thiadiazolyl (such as 1,2,3-thiadiazolyl, 1,2,4-thiadiazolyl, and 1,3,4-thiadiazolyl), pyridyl (such as pyridin-2-yl, pyridin-3-yl, and pyridin-4-yl), pyrimidyl (such as pyrimidin-2-yl, pyrimidin-4-yl, pyrimidin-5-yl, and pyrimidin-6-yl), and pyrazinyl (such as pyrazin-2-yl and pyrazin-3-yl).

The term "pharmaceutically acceptable salt" refers to a salt formed by a compound with a relatively non-toxic, pharmaceutically acceptable acid or base. When the compounds of the present disclosure contain relatively acidic functional groups, their base addition salts may be obtained by contacting the free form of such compounds with a sufficient amount of a pharmaceutically acceptable base, either in a pure solution or in a suitable inert solvent. When the compounds of the present disclosure contain relatively basic functional groups, their acid addition salts may be obtained by contacting the free form of such compounds with a sufficient amount of a pharmaceutically acceptable acid, either in a pure solution or in a suitable inert solvent.

The term "isotopic derivative" refers to a derivative formed when atoms in a compound are replaced by their isotopic atoms. Examples of isotopes that may be incorporated into the compounds of the present disclosure include, but are not limited to isotopes of hydrogen, carbon, nitrogen, oxygen, fluorine, sulphur, and chlorine (such as ²H, ³H, ¹³C, ¹⁴C, ¹⁵N, ¹⁸O, ¹⁷O, ¹⁸F, ³⁵S, and ³⁶Cl). Isotopic derivatives may generally be prepared according to the methods described herein by substituting an isotopically labelled reagent for a non-isotopically labelled reagent. In some embodiments, the isotopic derivative is a deuterated compound, for example, a deuterated compound formed by replacing 1, 2, 3, 4, 5, or 6 hydrogen atoms in the compound with deuterium.

The term "treatment" refers to therapeutic therapy. When referring to a specific condition, treatment means: (1) alleviating one or more biological manifestations of a disease or condition; (2) interfering with (a) one or more points in the biological cascade that causes or induces the condition, or (b) one or more biological manifestations of the condition; (3) ameliorating one or more symptoms, effects, or side effects associated with the condition, or one or more symptoms, effects, or side effects associated with the condition or its treatment; or (4) slowing the progression of one or more biological manifestations of the disease or condition.

The term "prevention" refers to a reduction in the risk of acquiring or developing a disease or disorder.

The term "effective amount" refers to an amount of the compound of the present disclosure that is sufficient to effectively treat or prevent the disease or condition described herein when administered to a subject. The effective amount will vary depending on the compound, the condition and its severity, and the age of the patient to be treated, but may be adjusted as needed by those skilled in the art.

The term "subject" refers to any animal, preferably a mammal, most preferably a human, to which a compound or composition is to be or has been administered.

Unless otherwise stated, various isomers or mixtures thereof of the compounds or isotopic derivatives of the present disclosure, such as tautomers, stereoisomers (e.g., geometric isomers or optical isomers) or any mixtures thereof (e.g., racemic mixtures), are all included within the scope of the present invention. Optical isomers may be enantiomers or diastereomers. These stereoisomers may be separated, purified, and enriched by asymmetric synthetic methods or chiral separation methods (including, but not limited to thin-layer chromatography, rotary chromatography, column chromatography, gas chromatography, high-pressure liquid chromatography, etc.), and may also be obtained via chiral resolution by bonding or salt formation with other chiral compounds. In the mixtures of stereoisomers mentioned above (such as mixtures of *E* and *Z* isomers or *R* and *S* isomers), the proportion of each stereoisomer may range from 5%-95% (such as 10%-90%, 20%-80%, 30%-70%, 40%-60%, and 50%).

Various existing forms of the compounds, isotopic derivatives, and pharmaceutically acceptable salts of the present disclosure, including various solid forms and mixtures thereof, such as crystalline forms, amorphous forms, solvates (such as hydrates), or any mixtures thereof, are all included within the scope of the present invention.

### Beneficial Effects

1. The compound of formula I provided by the present invention has improved OTR antagonistic activity.
2. The compound of formula I provided by the present invention also has lower V1aR antagonistic activity, and exhibits improved OTR/V1a target selectivity.
3. The compound of formula I provided by the present invention has improved exposure (AUC), half-life (T_{1/2}) and clearance rate (C1).
4. The compound of formula I provided by the present invention exhibits a lower proportion of distribution in brain tissues after oral administration, thereby reducing the risk of off-target effects caused by OTR antagonism in the brain.

### Detailed Description of Embodiments

The present invention is further described below by way of examples; however, the present invention is not limited to the scope of the described examples. For the experimental methods in which no specific conditions are specified in the following examples, selections are made according to conventional methods and conditions or according to the product instructions.

The starting materials and reagents in the examples are known and commercially available, or may be synthesized according to methods known in the art.

In the following examples, PE represents petroleum ether; EA represents ethyl acetate; DMF represents *N*,*N*-dimethylformamide; DMSO represents dimethylsulfoxide; DCM represents dichloromethane; THF represents tetrahydrofuran; TEA represents triethylamine; Pd(dppf)Cl₂ represents [1,1-bis(diphenylphosphino)ferrocene]palladium(II) dichloride; DIEA and DIPEA represent N,N-diisopropylethylamine; HATU represents 2-(7-azabenzotriazol-1-yl)-*N,N,N',N*'-tetramethyluronium hexafluorophosphate; DAST represents diethylaminosulphur trifluoride; EDCI represents 1-(3-dimethylaminopropyl)-3-ethyl carbodiimide hydrochloride; DMAP represents 4-dimethylaminopyridine; TsOH represents p-toluenesulfonic acid; Pd(PPh₃)₄ represents tetrakis(triphenylphosphine)palladium; Pd(dtbpf)Cl₂ represents 1,1'-bis(di-tert-butylphosphino)ferrocene palladium dichloride; Tf₂O represents trifluoromethanesulfonic anhydride; DPPF represents 1,1'-bis(diphenylphosphino)ferrocene; Boc₂O represents di-tert-butyl dicarbonate; TFA represents trifluoroacetic acid; ACN represents acetonitrile; *t*-BuONO represents tert-butyl nitrite; DPPP represents 1,3-bis(diphenylphosphino)propane; BF₃·Et₂O represents boron trifluoride-diethyl ether complex; t-BuOH represents tert-butanol; DMP represents Dess-Martin oxidant; BTC represents bis(trichloromethyl) carbonate; CDI represents *N,N'*-carbonyldiimidazole; TBSCl represents tertbutyldimethylsilyl chloride; LiHMDS represents lithium bis(trimethylsilyl)amide; DEAD represents diethyl azodicarboxylate; PPTS represents pyridin-1-ium 4-methylbenzenesulfonate; DMK represents acetone; NMP represents N-methylpyrrolidone; Prep-TLC represents preparative thin-layer chromatography; HPLC represents high performance liquid chromatography; rt represents room temperature (20°C-30°C).

### Examples C0020&C0021

### (S, E)-(2-(Hydroxymethyl)-4-(methoxyimino)-2-methylpyrrolidin-1-yl)(2'-methyl-[1,1'-biphenyl]-4-yl)methanone

### (S, Z)-(2-(Hydroxymethyl)-4-(methoxyimino)-2-methylpyrrolidin-1-yl)(2'-methyl-[1,1'-biphenyl]-4-yl)methanone

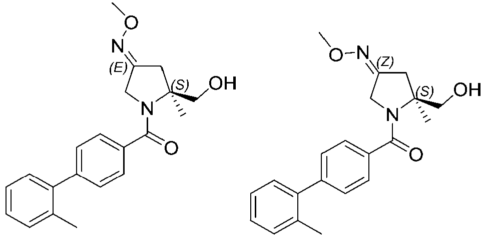

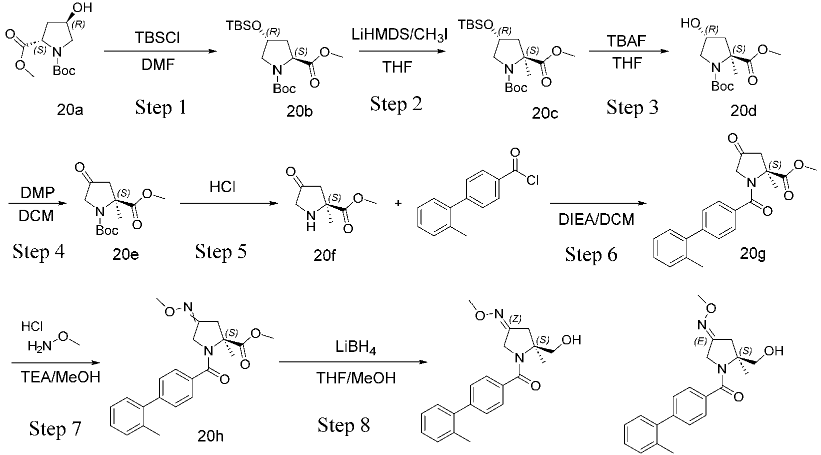

### Step 1

### 1-(Tert-butyl)2-methyl (2S, 4R)-4-((tert-butyldimethylsilyl)oxy)pyrrolidine-1,2-dicarboxylate 20b

*N*-Boc-trans-4-hydroxy-*L*-proline methyl ester 20a (15 g, 61.16 mmol) was dissolved in DMF (200 mL), imidazole (20.82 g, 305.80 mmol) and tertbutyldimethylsilyl chloride (27.65 g, 183.48 mmol) were added, and the mixture was reacted at room temperature for 16 hours. After the reaction was completed, water (100 mL) was added to the reaction solution, and the mixture was extracted with ethyl acetate (100 mL × 3). The organic phase was washed with 1N HCl (100 mL) and saturated sodium chloride aqueous solution (100 mL), respectively, dried over sodium sulphate, and subjected to evaporation to remove the solvent. The residue was purified by silica gel column chromatography (petroleum ether/ethyl acetate = 20:1 to 10:1) to afford 20b (14 g, yield: 60.5%).

MS *m*/*z* (ESI): 260.1 [M+1-Boc]⁺.

### Step 2

### 1-(Tert-butyl)2-methyl (2S,4R)-4-((tert-butyldimethylsilyl)oxy)-2-methylpyrrolidine-1,2-dicarboxylate 20c

20b (14 g, 36.99 mmol) was dissolved in tetrahydrofuran (200 mL), lithium bis(trimethylsilyl)amide (11.14 g, 66.8 mmol) was added dropwise at -15°C, and the mixture was reacted at -15°C for one hour. Subsequently, iodomethane (9.45 g, 66.8 mmol) was added at -15°C. The mixture was reacted at room temperature for 16 hours. After the reaction was completed, saturated ammonium chloride solution (50 mL) was added to the reaction solution, and the mixture was extracted with ethyl acetate (100 mL × 3). The organic phase was washed with saturated sodium chloride aqueous solution (50 mL × 2), dried over sodium sulphate, and subjected to evaporation to remove the solvent. The residue was purified by silica gel column chromatography (petroleum ether/ethyl acetate = 50:1 to 30:1) to afford 20c (2.7 g, yield: 18.6%).

MS *m*/*z* (ESI): 374.1 [M+1]⁺.

### Step 3

### 1-(Tert-butyl)2-methyl (2S,4R)-4-hydroxy-2-methylpyrrolidine-1,2-dicarboxylate 20d

20c (3.2 g, 8.57 mmol) was dissolved in tetrahydrofuran (50 mL), tetrabutylammonium fluoride (3.36 g, 12.86 mmol) was added, and the mixture was reacted at room temperature for 16 hours. After the reaction was completed, the reaction solution was concentrated to remove tetrahydrofuran, and then ethyl acetate (50 mL) and water (50 mL) were added to the residue, and the mixture was extracted with ethyl acetate (30 mL × 2). The organic phase was washed with saturated sodium chloride aqueous solution (30 mL × 2), dried over sodium sulphate, and subjected to evaporation to remove the solvent. The residue was purified by silica gel column chromatography (petroleum ether/ethyl acetate = 10:1 to 5:1) to afford product 20d (1.6 g, yield: 68.4%).

¹H NMR (300 MHz, DMSO-*d*₆) *δ* 5.13-5.10 (m, 1H), 4.29-4.27 (m, 1H), 3.63 (d, *J =* 16.5 Hz, 3H), 3.55-3.50 (m, 1H), 3.34-3.30 (m, 1H), 2.27-2.15 (m, 1H), 1.92-1.82 (m, 1H), 1.58 (d, *J =* 3.9 Hz, 3H), 1.38 (d, *J =* 17.7 Hz, 9H).

### Step 4

### 1-(Tert-butyl)2-methyl (S)-2-methyl-4-oxopyrrolidine-1,2-dicarboxylate 20e

20d (1.6 g, 6.17 mmol) was dissolved in dichloromethane (30 mL), Dess-Martin oxidant (3.92 g, 9.25 mmol) was added, and the mixture was reacted at room temperature for 16 hours. After the reaction was completed, saturated sodium bicarbonate solution (30 mL) was added to the reaction solution, and the mixture was extracted with dichloromethane (30 mL × 2). The organic phase was washed with saturated sodium bicarbonate solution (30 mL) and saturated sodium chloride aqueous solution (30 mL), respectively, dried over sodium sulphate, and subjected to evaporation to remove the solvent. The residue was purified by silica gel column chromatography (petroleum ether/ethyl acetate = 30:1 to 10:1) to afford 20e (1.2 g, yield: 71.8%).

¹H NMR (300 MHz, DMSO-*d*₆) *δ* 3.89 (s, 2H), 3.70 (s, 3H), 3.02-2.87 (m, 1H), 2.78 (d, *J =* 18.9 Hz, 1H), 1.62 (s, 3H), 1.41 (s, 9H) .

### Step 5

### Methyl (S)-2-methyl-4-oxopyrrolidine-2-carboxylate 20f

20e (0.3 g, 1.11 mmol) was dissolved in 4N hydrogen chloride/dioxane (10 mL), and the mixture was reacted at room temperature for 30 minutes. After the reaction was completed, the reaction solution was subjected to evaporation to remove the solvent to afford 20f (0.18 g, yield: 98.22%).

¹H NMR (300 MHz, DMSO-*d*₆) *δ* 10.80 (brs, 1H), 3.95-3.76 (m, 5H), 3.03 (d, *J =* 18.6 Hz, 1H), 2.80-2.75 (d, *J =* 18.6 Hz, 1H), 1.73 (s, 3H).

### Step 6

### Methyl (S)-2-methyl-1-(2'-methyl-[1,1'-biphenyl]-4-carbonyl)-4-oxopyrrolidine-2-carboxylate 20g

20f (0.18 g, 1.09 mmol) was dissolved in dichloromethane (10 mL), 4-(2-methylphenyl)benzoyl chloride (0.38 g, 1.64 mmol) and triethylamine (0.33 g, 3.27 mmol) were added, and the mixture was reacted at room temperature for 16 hours. After the reaction was completed, water (10 mL) was added to the reaction solution, and the mixture was extracted with dichloromethane (10 mL × 2). The organic phase was washed with saturated sodium chloride aqueous solution (10 mL × 2), dried over sodium sulphate, and subjected to evaporation to remove the solvent. The residue was purified by Pre-TLC (petroleum ether/ethyl acetate = 3/1) to afford 20g (0.3 g, yield: 74.5%).

MS *m*/*z* (ESI): 374.1 [M+Na]⁺.

### Step 7

### Methyl (S, EZ)-4-(methoxyimino)-2-methyl-1-(2'-methyl-[1,1'-biphenyl]-4-carbonyl)pyrrolidine-2-carboxylate 20h

20g (0.30 g, 0.81 mmol) was dissolved in methanol (15 mL), methoxyamine hydrochloride (0.23 g, 2.83 mmol) and triethylamine (0.20 g, 2.03 mmol) were added, and the mixture was reacted at 60°C for 16 hours. After the reaction was completed, the reaction solution was concentrated to remove methanol, and ethyl acetate (20 mL) was added to the residue. The mixture was washed with water (10 mL × 2) and once with saturated sodium chloride (10 mL), dried over sodium sulphate, and subjected to evaporation to remove the solvent. The residue was purified by Pre-TLC (petroleum ether/ethyl acetate = 5/1) to afford 20h (0.2 g, yield: 61.6%).

MS *m*/*z* (ESI): 381.1 [M+1]⁺.

### Step 8

### (S, Z)-(2-(Hydroxymethyl)-4-(methoxyimino)-2-methylpyrrolidin-1-yl)(2'-methyl-[1,1'-biphenyl]-4-yl)methanone

### (S, E)-(2-(Hydroxymethyl)-4-(methoxyimino)-2-methylpyrrolidin-1-yl)(2'-methyl-[1,1'-biphenyl]-4-yl)methanone

20h (0.20 g, 0.50 mmol) was dissolved in methanol (2 mL) and tetrahydrofuran (2 mL), and the mixture was cooled to 0°C. Lithium borohydride (0.23 mL, 2.0 mmol) was added under nitrogen protection, and the mixture was reacted at room temperature for 16 hours. Water was added to quench the reaction solution, the reaction solution was concentrated, and the residue was dissolved in dichloromethane and water. After layer separation, the aqueous phase was extracted twice with dichloromethane, and the organic phases were combined, washed once with sodium chloride aqueous solution, dried, filtered, and subjected to rotary evaporation to dryness. The crude product was separated by silica gel Pre-TLC (EA:PE=2:1) to afford the less polar isomer C0021 (Rf=0.5) and the more polar crude isomer C0020 (Rf=0.4). The crude product was further purified by preparative HPLC and lyophilized to afford the more polar C0020 (11.27 mg, yield: 6.3%) and the less polar C0021 (7.80 mg, yield: 4.3%).

C0020: MS *m*/*z* (ESI): 353.1 [M+1]⁺.

¹H NMR (400 MHz, DMSO-*d*₆) *δ* 7.51 (d, *J =* 8.0 Hz, 2H), 7.40 (d, *J =* 8.0 Hz, 2H), 7.33-7.22 (m, 4H), 5.06 (t, *J =* 5.6 Hz, 1H), 4.19-4.04 (m, 3H), 3.70 (s, 3H), 3.51-3.47 (m, 1H), 2.90 (dd, *J =* 16.8, 1.2 Hz, 1H), 2.57-2.53 (m, 1H), 2.25 (s, 3H), 1.50 (s, 3H).

C0021: MS *m*/*z* (ESI): 353.1 [M+1]⁺.

¹H NMR (300 MHz, DMSO-*d*₆) *δ* 7.53 (d, *J =* 8.1 Hz, 2H), 7.41 (d, *J =* 8.1 Hz, 2H), 7.32-7.24 (m, 4H), 5.12 (t, *J =* 5.1 Hz, 1H), 4.22-4.05 (m, 3H), 3.78 (s, 3H), 3.51-3.47 (m, 1H), 3.00 (d, *J* = 18.3 Hz, 1H), 2.56-2.50 (m, 1H), 2.27 (s, 3H), 1.50 (s, 3H).

### Examples C0035&C0036

### (S,E)-(2',3'-Dimethyl-[1,1'-biphenyl]-4-yl)(2-(hydroxymethyl)-4-(methoxyimino)pyrrolidin-1-yl)methanone

### (S,Z)-(2',3'-Dimethyl-[1,1'-biphenyl]-4-yl)(2-(hydroxymethyl)-4-(methoxyimino)pyrrolidin-1-yl)methanone

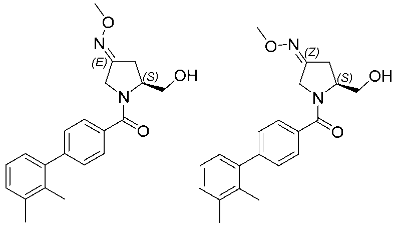

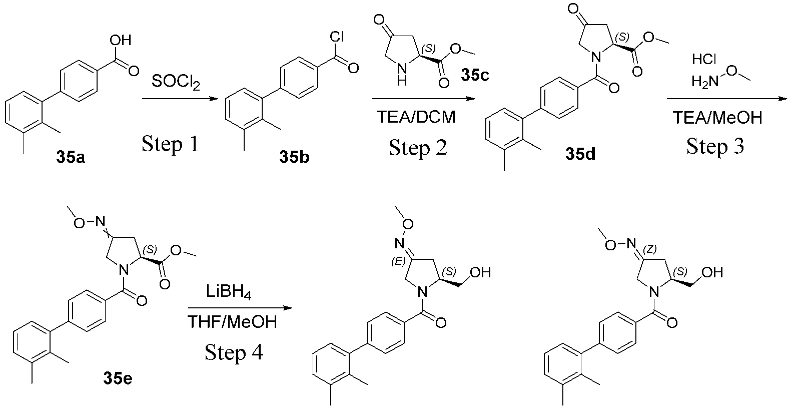

### Step 1

### 2',3'-Dimethyl-[1,1'-biphenyl]-4-carbonyl chloride 35b

35a (1.35 g, 5.37 mmol) was dissolved in thionyl chloride (10 mL), and the mixture was reacted at 80°C for three hours. After the reaction was completed, the reaction solution was directly subjected to rotary evaporation to dryness, to afford crude product 35b (1.31 g, yield: 89.7%) without workup.

### Step 2

### (S)-Methyl 1-(2',3'-dimethyl-[1,1'-biphenyl]-4-carbonyl)-4-oxopyrrolidine-2-carboxylate 35d

35b (1.31 g, 5.35 mmol) was dissolved in dichloromethane (10 mL), triethylamine (2.10 g, 20.79 mmol) and 35c (0.85 g, 5.94 mmol) were added at 0°C, and the mixture was reacted at room temperature for 16 hours. After the reaction was completed, the reaction solution was concentrated to remove dichloromethane, and the residue was purified by silica gel column chromatography (petroleum ether:ethyl acetate = 5:1 to 3:1) to afford product 35d (900 mg, yield: 38.4%).

MS *m*/*z* (ESI): 351.8 [M+1]⁺.

### Step 3

### (S,EZ)-Methyl 1-(2',3'-dimethyl-[1,1'-biphenyl]-4-carbonyl)-4-(methoxyimino)pyrrolidine-2-carboxylate 35e

35d (0.9 g, 2.56 mmol) was dissolved in methanol (10 mL), methoxyamine hydrochloride (0.43 g, 5.12 mmol) and triethylamine (0.65 g, 6.40 mmol) were added at room temperature, and the mixture was reacted at 50°C for 18 hours. After the reaction was completed, the reaction solution was concentrated, and the residue was purified by silica gel column chromatography (petroleum ether:ethyl acetate = 4:1 to 3:1) to afford product 35e (0.9 g, yield: 83.1%).

MS *m*/*z* (ESI): 380.8 [M+1]⁺.

### Step 4

### (S,Z)-(2',3'-Dimethyl-[1,1'-biphenyl]-4-yl)(2-(hydroxymethyl)-4-(methoxyimino)pyrrolidin-1-yl)methanone

### (S,E)-(2',3'-Dimethyl-[1,1'-biphenyl]-4-yl)(2-(hydroxymethyl)-4-(methoxyimino)pyrrolidin-1-yl)methanone

35e (120 mg, 0.30 mmol) was dissolved in methanol (2 mL) and tetrahydrofuran (2 mL), and the mixture was cooled to 0°C. Lithium borohydride (0.32 mL, 0.64 mmol) was added under nitrogen protection, and the mixture was reacted at room temperature for 2 hours. Water was added to quench the reaction solution, the reaction solution was concentrated, and the residue was dissolved in dichloromethane and water. After layer separation, the aqueous phase was extracted twice with dichloromethane, and the organic phases were combined, washed once with sodium chloride aqueous solution, dried, filtered, and subjected to rotary evaporation to dryness. The crude product was separated by silica gel Prep-TLC (EA:PE=2:1) to afford the less polar crude isomer C0035 (Rf=0.5) and the more polar crude isomer C0036 (Rf=0.4). The crude product C0035 was purified by preparative HPLC and lyophilized to afford C0035 (10 mg, yield: 9.3%). The crude product C0036 was purified by preparative HPLC (FA) and lyophilized to afford C0036 (15 mg, yield: 14.1%).

C0035: ESI-MS: *m*/*z=* 353.1 [M+1]⁺.

¹H NMR (400 MHz, CD₃OD-*d*₄) *δ* 7.60 (d, *J =* 7.2 Hz, 2H), 7.40-7.38 (m, 2H), 7.18-7.10 (m, 2H), 7.04-7.02 (m, 1H), 4.76-4.01 (m, 4H), 3.85-3.80 (m, 4H), 3.71-3.57 (m, 1H), 2.88(s, 2H), 2.34 (s, 3H), 2.14 (s, 3H).

C0036: ESI-MS: *m*/*z=* 353.1 [M+1]⁺.

¹H NMR (400 MHz, CD₃OD-*d*₄) *δ* 7.60 (d, *J =* 7.6 Hz, 2H), 7.40-7.38 (m, 2H), 7.16-7.12 (m, 2H), 7.04-7.02 (m, 1H), 4.79-4.10 (m, 3H), 3.87-3.79 (m, 4H), 3.71-3.69 (m, 1H), 3.48-3.39 (m, 1H), 2.95-2.73 (m, 2H), 2.34 (s, 3H), 2.15 (s, 3H).

### Examples C0038&C0038A

### (S,Z)-(2-(Hydroxymethyl)-4-(methoxyimino)pyrrolidin-1-yl)(3-methoxy-2',3'-dimethyl-[1,1'-biphenyl]-4-yl)methanone

### (S,E)-(2-(Hydroxymethyl)-4-(methoxyimino)pyrrolidin-1-yl)(3-methoxy-2',3'-dimethyl-[1,1'-biphenyl]-4-yl)methanone

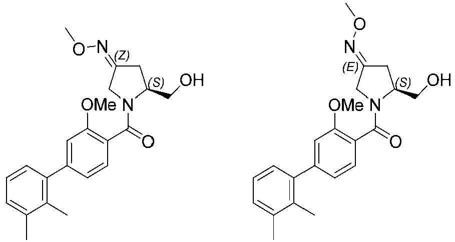

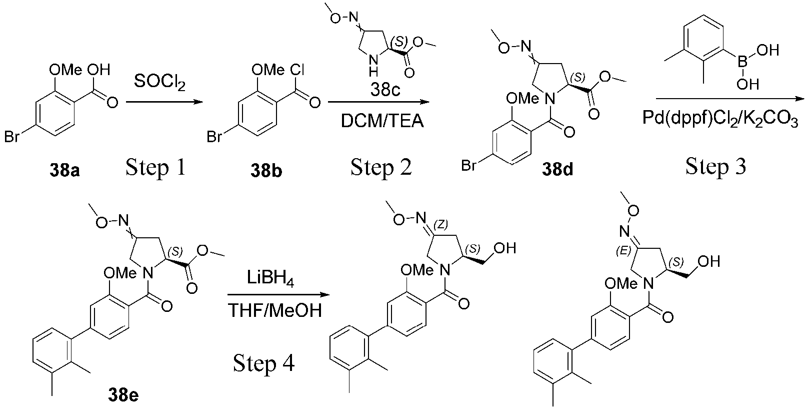

### Step 1

### 4-Bromo-2-methoxybenzoyl chloride 38b

38a (1.2 g, 5.19 mmol) was dissolved in thionyl chloride (10 mL), and the mixture was reacted at 80°C for 3 hours. After the reaction was completed, the reaction solution was directly subjected to rotary evaporation to dryness, to afford crude product 38b (1.5 g) without workup.

MS *m*/*z* (ESI): 246.1 [M+1]⁺.

### Step 2

### Methyl (S,EZ)-1-(4-bromo-2-methoxybenzoyl)-4-(methoxyimino)pyrrolidine-2-carboxylate 38d

To a solution of 38b (1 g, 4.01 mmol) in DCM (20 mL) were added 38c (0.83 g, 4.81 mmol) and triethylamine (2.03 g, 20.05 mmol). After nitrogen purging, the mixture was stirred at room temperature for 20 min. After the reaction was completed, ethyl acetate was added, and the mixture was washed with saturated sodium chloride. The organic phases were combined, dried, and subjected to rotary evaporation to dryness, and the crude product was purified by column chromatography (PE/EA=10:1 to 1:1) to afford 38d (1.2 g, yield: 77.7%).

MS *m*/*z* (ESI): 369.1 [M+1]⁺.

### Step 3

### Methyl (S, EZ)-1-(3-methoxy-2', 3'-dimethyl-[1,1'-biphenyl]-4-carbonyl)-4-(methoxyimino)pyrrolidine-2-carboxylate 38e

To a solution of 38d (200 mg, 0.52 mmol) in dioxane/water (15/1.5 mL) were added (2, 3-dimethylphenyl)boronic acid (94 mg, 0.62 mmol) and 1,1-bis(diphenylphosphino)ferrocene palladium(II) dichloride (38 mg, 52 mmol). After nitrogen purging, the mixture was reacted at 85°C for 2 hours. The reaction solution was cooled and then poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness. The crude product was loaded onto a silica gel column, eluting with a gradient of (PE/EA=10:1 to 1:1). The eluate was collected and subjected to rotary evaporation to afford 38e (150 mg, yield: 66.8%).

MS *m*/*z* (ESI): 410.8 [M+1]⁺.

### Step 4

### (S, Z)-(2-(Hydroxymethyl)-4-(methoxyimino)pyrrolidin-1-yl)(3-methoxy-2', 3'-dimethyl-[1, 1'-biphenyl]-4-yl)methanone

### (S, E)-(2-(Hydroxymethyl)-4-(methoxyimino)pyrrolidin-1-yl)(3-methoxy-2', 3'-dimethyl-[1, 1'-biphenyl]-4-yl)methanone

To a solution of 38e (140 mg, 0.34 mmol) in tetrahydrofuran/methanol (5/5 mL) was slowly added LiBH₄ (7.4 mg, 0.34 mmol) at 0°C under nitrogen protection. The mixture was stirred at room temperature for 1 hour. The reaction solution was cooled and then poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness. The crude product was separated by silica gel Prep-TLC (EA:PE=2:1) to afford the less polar isomer C0038A (Rf=0.5) and the more polar crude isomer C0038 (Rf=0.4). Further purification by preparative HPLC and lyophilization yielded the more polar product C0038 (9.39 mg, yield: 7.2%) and the less polar product C0038A (7.42 mg, yield: 5.7%).

**C0038:** MS *m*/*z* (ESI): 383.1 [M+1]⁺.

¹H NMR (400 MHz, CD₃OD-*d*) *δ* 7.35-7.30 (m, 1H), 7.17-7.02 (m, 3H), 6.98-6.93 (m, 2H), 4.69-4.47 (m, 1H), 4.22-4.15 (m, 1H), 4.04-3.95 (m, 1H), 3.88-3.70 (m, 7H), 3.38-3.35 (m, 1H), 2.96-2.90 (m, 1H), 2.80-2.66 (m, 1H), 2.33 (s, 3H), 2.1 (d, *J* = 2.0 Hz, 3H).

**C0038A:** MS *m*/*z* (ESI): 383.1 [M+1]⁺.

¹H NMR (400 MHz, CD₃OD-*d*) *δ* 7.35-7.32 (m, 1H), 7.17-7.02 (m, 3H), 6.98-6.94 (m, 2H), 4.69-4.51 (m, 1H), 4.20-4.15 (m, 1H), 4.04-3.95 (m, 1H), 3.88-3.70 (m, 7H), 3.38-3.35 (m, 1H), 2.96-2.75 (m, 2H), 2.80-2.66 (m, 1H), 2.33 (s, 3H), 2.17 (s, 3H).

### Examples C0044&C0044A

### (S, Z)-(2',3-Dimethyl-[1,1'-biphenyl]-4-yl)(2-(hydroxymethyl)-4-(methoxyimino)pyrrolidin-1-yl)methanone

### (S, E)-(2',3-Dimethyl-[1,1'-biphenyl]-4-yl)(2-(hydroxymethyl)-4-(methoxyimino)pyrrolidin-1-yl)methanone

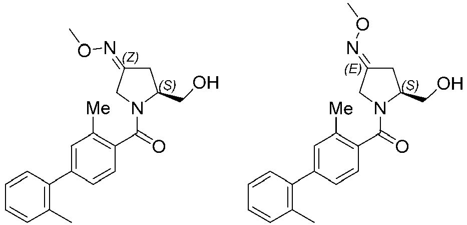

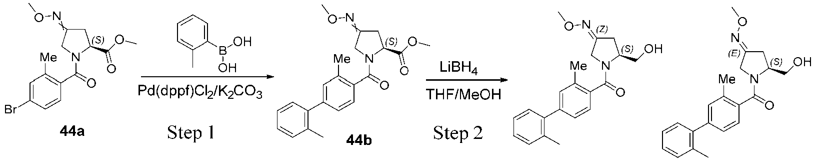

With reference to the synthetic method of compound C0038, the more polar product C0044 (10 mg, yield: 14.20%) and the less polar product C0044A (10 mg, yield: 14.20%) were obtained.

C0044A: MS *m*/*z* (ESI): 353.1 [M+1]⁺.

¹H NMR (400 MHz, CD₃OD-*d*) *δ* 7.35-7.31 (m, 1H), 7.26-7.18 (m, 6H), 4.71-4.55 (m, 1H), 4.19-4.11 (m, 1H), 3.95-3.70 (m, 5H), 3.37-3.36 (m, 1H), 2.92-2.89 (m, 2H), 2.37 (s, 1H), 2.24 (s, 1H).

C0044: MS *m*/*z* (ESI): 353.1 [M+1]⁺.

¹H NMR (400 MHz, CD₃OD-*d*) *δ* 7.36-7.32 (m, 1H), 7.26-7.21 (m, 6H), 4.70-4.51 (m, 1H), 4.21-4.08 (m, 1H), 3.91-3.76 (m, 5H), 3.38-3.37 (m, 1H), 3.01-2.94 (m, 1H), 2.80-2.66 (m, 1H), 2.36 (s, 1H), 2.24 (d, *J =* 2.0 Hz, 1H).

### Examples C0045&C0045A

### (S, Z)-(2-(Hydroxymethyl)-4-(methoxyimino)pyrrolidin-1-yl)(3-methyl-2',3'-dimethyl-[1,1'-biphenyl]-4-yl)methanone

### (S, E)-(2-(Hydroxymethyl)-4-(methoxyimino)pyrrolidin-1-yl)(3-methyl-2',3'-dimethyl-[1,1'-biphenyl]-4-yl)methanone

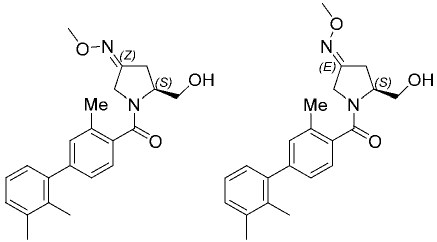

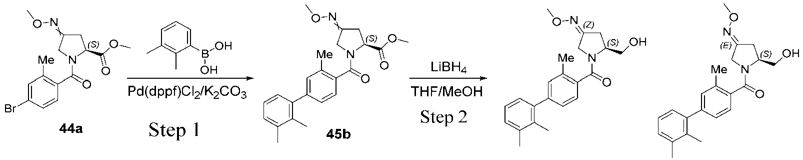

With reference to the synthetic method of compound C0038, the more polar product C0045 (15 mg, yield: 17.0%) and the less polar product C0045A (17 mg, yield: 19.3%) were obtained.

C0045: MS *m*/*z* (ESI): 367.0 [M+1]⁺.

¹H NMR (400 MHz, CD₃OD-*d*) *δ* 7.36-7.31 (m, 1H), 7.21-7.09 (m, 4H), 7.03-6.99 (m, 1H), 4.70-4.51 (m, 1H), 4.26-4.08 (m, 1H), 3.91-3.72 (m, 5H), 3.38 (d, *J* = 4.8 Hz, 1H), 3.01-2.66 (m, 2H), 2.36 (s, 3H), 2.33 (s, 3H), 2.13 (d, *J* = 2.0 Hz, 3H).

C0045A: MS *m*/*z* (ESI): 367.0 [M+1]⁺.

¹H NMR (400 MHz, CD₃OD-*d*) *δ* 7.37-7.32 (m, 1H), 7.21-7.09 (m, 4H), 7.02-6.99 (m, 1H), 4.74-4.55 (m, 1H), 4.19-4.07 (m, 1H), 3.95-3.70 (m, 5H), 3.38-3.34 (m, 1H), 2.91-2.85 (m, 2H), 2.36 (s, 3H), 2.33 (s, 3H), 2.13 (s, 3H).

### Examples C0046&C0046A

### (S, Z)-(2',2-Dimethyl-[1,1'-biphenyl]-4-yl)(2-(hydroxymethyl)-4-(methoxyimino)pyrrolidin-1-yl)methanone

### (S, E)-(2',2-Dimethyl-[1,1'-biphenyl]-4-yl)(2-(hydroxymethyl)-4-(methoxyimino)pyrrolidin-1-yl)methanone

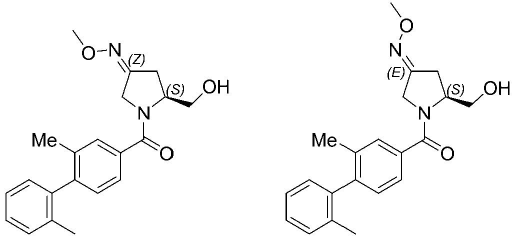

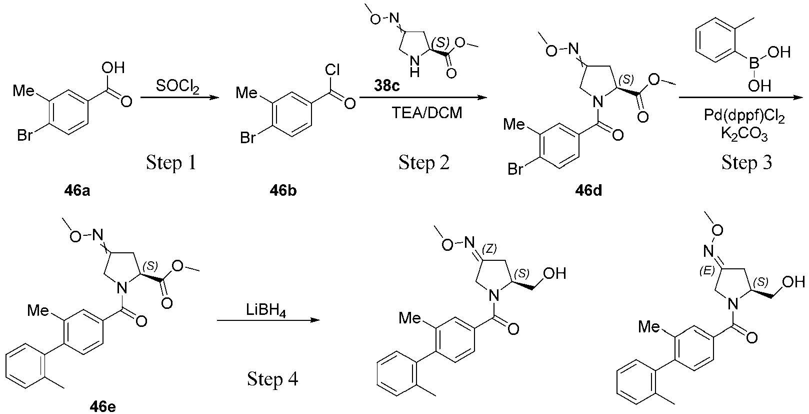

With reference to the synthetic method of compound C0038, the more polar product C0046 (Rf = 0.4, 10.06 mg, yield: 5.8%) and the less polar product C0046A (Rf = 0.5, 5.2 mg, yield: 2.96%) were obtained.

C0046: MS *m*/*z* (ESI): 353.1 [M+1]⁺.

¹H NMR (400 MHz, CD₃OD-*d*) *δ* 7.66-7.52 (m, 1H), 7.48-7.30 (m, 2H), 7.30-7.16 (m, 4H), 7.07 (d, *J =* 6.4 Hz, 1H), 4.74-4.11 (m, 3H), 3.89-3.68 (m, 5H), 3.42-3.35 (m, 1H), 3.11-2.82 (m, 1H), 2.77-2.73 (m, 1H), 2.08 (s, 3H), 2.04 (s, 3H).

C0046A: MS *m*/*z* (ESI): 353.1 [M+1]⁺.

¹H NMR (400 MHz, CD₃OD-*d*) *δ* 7.47 (s, 1H), 7.41 (d, *J =* 7.6 Hz, 1H), 7.30-7.16 (m, 4H), 7.06 (d*, J =* 7.2 Hz, 1H), 4.83-4.06 (m, 3H), 3.96-3.37 (m, 6H), 2.91-2.79 (m, 2H), 2.08 (s, 3H), 2.03 (s, 3H).

### Examples C0047&C0047A

### (S, Z)-(2,2',3'-Trimethyl-[1,1'-biphenyl]-4-yl)(2-(hydroxymethyl)-4-(methoxyimino)pyrrolidin-1-yl)methanone

### (S, E)-(2,2',3'-Trimethyl-[1,1'-biphenyl]-4-yl)(2-(hydroxymethyl)-4-(methoxyimino)pyrrolidin-1-yl)methanone

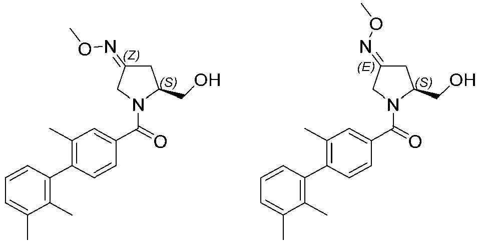

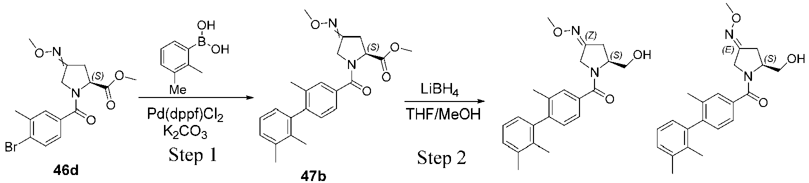

According to the aforementioned synthetic route, with reference to the synthetic method of compound C0038, the more polar product C0047 (34.80 mg, yield: 15.0%) and the less polar product C0047A (30.80 mg, yield: 13.2%) were obtained.

C0047: MS *m*/*z* (ESI): 367.1 [M+1]⁺.

¹H NMR (400 MHz, CD₃OD-*d*) *δ* 7.45 (s, 1H), 7.39 (d, *J =* 7.6 Hz, 1H), 7.18-7.10 (m, 3H), 6.90 (d, *J =* 6.8 Hz, 1H), 4.83-4.10 (m, 3H), 3.87-3.40 (m, 6H), 2.95-2.89 (m, 1H), 2.77-2.65 (m, 1H), 2.33 (s, 3H), 2.07 (s, 3H), 1.96 (s, 3H).

C0047A: MS *m*/*z* (ESI): 367.1 [M+1]⁺.

¹H NMR (400 MHz, CD₃OD-*d*) *δ* 7.46 (s, 1H), 7.40 (d, *J =* 7.6 Hz, 1H), 7.18-7.10 (m, 3H), 6.89 (d*, J =* 7.2 Hz, 1H), 4.80-4.05 (m, 3H), 3.96-3.37 (m, 6H), 2.94-2.81 (m, 2H), 2.33 (s, 3H), 2.07 (s, 3H), 1.95 (s, 3H).

### Examples A0061&A0042

### (S, Z)-(5-(2,3-Dimethylphenyl)pyrazin-2-yl)(2-(hydroxymethyl)-4-(methoxyimino)pyrrolidin-1-yl)methanone

### (S, E)-(5-(2,3-Dimethylphenyl)pyrazin-2-yl)(2-(hydroxymethyl)-4-(methoxyimino)pyrrolidin-1-yl)methanone

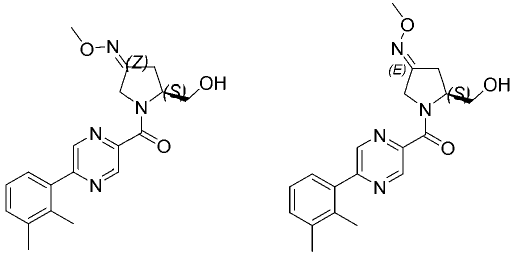

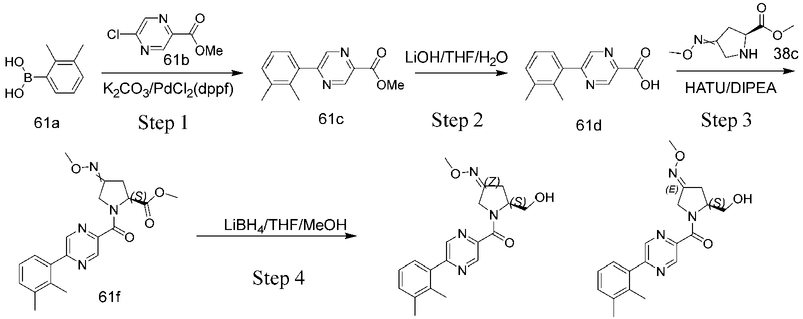

### Step 1

### Methyl 5-(2,3-dimethylphenyl)pyrazine-2-carboxylate 61c

The starting materials 61b (2 g, 11.59 mmol) and 61a (1.91 g, 12.75 mmol) were dissolved in 40 mL of 1,4-dioxane, potassium carbonate (4.81 g, 34.77 mmol) and [1,1'-bis(diphenylphosphino)ferrocene]palladium(II) dichloride (0.42 g, 0.58 mmol) were added, and then the mixture was reacted at 75°C under nitrogen protection for 16 hours. Then, 50 mL of water was added to quench the reaction solution, and the mixture was extracted with ethyl acetate (50 mL × 3). The organic phases were combined, washed with sodium chloride aqueous solution (50 mL × 3), dried over anhydrous sodium sulphate, and filtered, and the filtrate was concentrated under reduced pressure to afford a crude product, which was purified by column chromatography to afford product 61c (1.7 g, white solid, yield: 60.5%).

MS *m*/*z* (ESI): 243[M+1]⁺.

### Step 2

### 5-(2,3-Dimethylphenyl)pyrazine-2-carboxylic acid 61d

The starting material 61c (1.7 g, 7.02 mmol) was dissolved in 5 mL of tetrahydrofuran and 5 mL of water, and then lithium hydroxide (0.50 g, 21.06 mmol) was added, and the reaction solution was reacted at 25°C for 2 hours. Then, 2 M dilute hydrochloric acid was added to adjust the pH to acidic. The mixture was extracted with ethyl acetate (40 mL × 3). The organic phases were combined, washed with sodium chloride aqueous solution (40 mL × 3), dried over anhydrous sodium sulphate, and filtered, and the filtrate was concentrated under reduced pressure to afford product 61d (1.4 g, white solid, yield: 90.5%).

MS *m*/*z* (ESI): 251[M+1]⁺.

### Step 3

### Methyl (S, EZ)-1-(5-(2,3-dimethylphenyl)pyrazine-2-carbonyl)-4-(methoxyimino)pyrrolidine-2-carboxylate 61f

The starting material 61d (1.45 g, 6.35 mmol) was dissolved in 50 mL of DMF, and then 38c (1.20 g, 6.99 mmol) and HATU (3.14 g, 8.25 mmol) were added, followed by slow addition of ethyldiisopropylamine (3.28 g, 25.4 mmol). The reaction solution was reacted at room temperature for 2 hours. Then, 50 mL of water was added, and the mixture was extracted with ethyl acetate (50 mL × 3). The organic phases were combined, washed with sodium chloride aqueous solution (50 mL × 3), dried over anhydrous sodium sulphate, and filtered, and the filtrate was concentrated under reduced pressure to afford a residue, which was purified by silica gel column chromatography to afford product 61f (560 mg, white solid, yield: 23.1%).

MS *m*/*z* (ESI): 383.3[M+1]⁺.

### Step 4

### (S, Z)-(5-(2,3-Dimethylphenyl)pyrazin-2-yl)(2-(hydroxymethyl)-4-(methoxyimino)pyrrolidin-1-yl)methanone

### (S, E)-(5-(2,3-Dimethylphenyl)pyrazin-2-yl)(2-(hydroxymethyl)-4-(methoxyimino)pyrrolidin-1-yl)methanone

The starting material 61f (300 mg, 0.78 mmol) was dissolved in 5 mL of tetrahydrofuran and 5 mL of methanol, and lithium borohydride (0.051 g, 2.34 mmol) was added. The reaction solution was reacted at 25°C for 1 hour. Then, 10 mL of water was added, and the mixture was extracted with ethyl acetate (10 mL × 3). The organic phases were combined, washed with sodium chloride aqueous solution (10 mL × 3), dried over anhydrous sodium sulphate, and filtered, and the filtrate was concentrated under reduced pressure. The crude product was separated by silica gel Prep-TLC (EA:PE=2:1) to afford the less polar isomer A0042 (Rf=0.4) and the more polar crude isomer A0061 (Rf=0.3). Further purification by preparative HPLC and lyophilization yielded the more polar product A0061 (101.55 mg, yield: 35.8%) and the less polar product A0042 (60 mg, yield: 21.3%).

A0061: MS *m*/*z* (ESI): 355.3[M+1]⁺.

¹H NMR (400 MHz, Chloroform-d) *δ* 9.27 (d, *J =* 18.4 Hz, 1H), 8.70-8.60 (m, 1H), 7.29-7.22 (m, 3H), 4.95-4.89 (m, 1H), 4.76 (s, 1H), 3.94-3.82 (m, 5H), 2.95-2.66 (m, 2H), 2.37 (s, 3H), 2.26 (s, 3H).

A0042: MS *m*/*z* (ESI): 355.3[M+1]⁺.

¹H NMR (400 MHz, Chloroform-d) *δ* 9.30-9.25 (m, 1H), 8.69-8.60 (m, 1H), 7.29-7.24 (m, 3H), 4.95-4.89 (m, 1H), 4.77 (d, *J =* 18.4 Hz, 1H), 3.94-3.88 (m, 3H), 3.85-3.60 (m, 2H), 2.95-2.65 (m, 2H), 2.38 (s, 3H), 2.26 (d, *J =* 5.2 Hz, 3H).

### Examples C0041&C0041A

### (S, Z)-(5-(3-Chloro-2-methylphenyl)pyrazin-2-yl)(2-(hydroxymethyl)-4-(methoxyimino)pyrrolidin-1-yl)methanone

### (S, E)-(5-(3-Chloro-2-methylphenyl)pyrazin-2-yl)(2-(hydroxymethyl)-4-(methoxyimino)pyrrolidin-1-yl)methanone

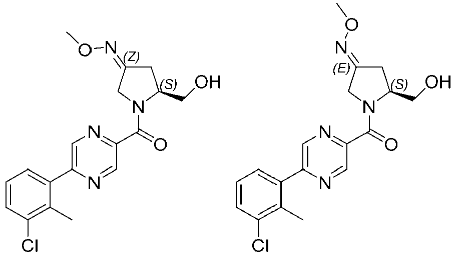

With reference to the method of A0061, by replacing the starting material 61a in Step 1 with 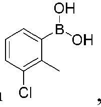 , the more polar product C0041 (23.5 mg, yield: 16.9%) and the less polar product C0041A (5.6 mg, yield: 4.0%) were obtained.

C0041: MS *m*/*z* (ESI): 375.1 [M+1]⁺.

¹H NMR (400 MHz, CD₃OD) *δ* 9.13-9.08 (m, 1H), 8.81-8.76 (m, 1H), 7.55-7.53 (m, 1H), 7.43-7.32 (m, 2H), 5.08-4.78 (m, 1H), 4.70-4.68 (m, 1H), 4.60-4.31 (m, 1H), 3.89-3.82 (m, 3H), 3.86-3.69 (m, 1H), 3.59-3.58 (m, 1H), 3.04-2.89 (m, 1H), 2.78-2.65 (m, 1H), 2.39 (s, 3H).

C0041A: MS *m*/*z* (ESI): 375.0 [M+1]⁺.

¹H NMR (400 MHz, CD₃OD) *δ* 9.11 (dd, *J =* 20, 1.2 Hz, 1H), 8.78 (dd, *J =* 16, 1.2 Hz, 1H), 7.54 (d, *J =* 7.6 Hz, 1H), 7.42 (d, *J =* 7.6 Hz, 1H), 7.36-7.32 (m, 1H), 5.10-4.79 (m, 1H), 4.66-4.63 (m, 1H), 4.63-4.20 (m, 1H), 3.88-3.67 (m, 4H), 3.58-3.56 (m, 1H), 2.96-2.83 (m, 2H), 2.39 (s, 3H).

### Synthesis of intermediate 4

### (S, EZ)-5-(Hydroxymethyl)pyrrolidin-3-one O-methyloxime hydrochloride

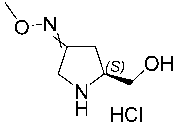

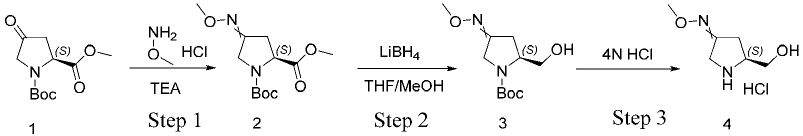

### Step 1

### (S, EZ)-1-(Tert-butyl)-2-methyl 4-(methoxyimino)pyrrolidine-1,2-dicarboxylate 2

Compound 1 (13.5 g, 55.50 mmol) was dissolved in methanol (150 mL), methoxyamine hydrochloride (20.86 g, 249.75 mmol) and triethylamine (19.66 g, 194.25 mmol) were added, and the mixture was reacted at 60°C for 16 hours. After the reaction was completed, the reaction solution was concentrated to remove methanol. Water (50 mL) was added to the reaction solution, and the mixture was extracted with ethyl acetate (100 mL × 3). The organic phase was washed with saturated sodium chloride aqueous solution (50 mL), dried over sodium sulphate, and subjected to evaporation to remove the solvent to afford 2 (14 g, yield: 88.0%), which was directly used in the next reaction.

MS *m*/*z* (ESI): 216.9 [M+1-56]⁺.

### Step 2

### Tert-butyl (S, EZ)-2-(hydroxymethyl)-4-(methoxyimino)pyrrolidine-1-carboxylate 3

Compound 2 (14 g, 48.84 mmol) was dissolved in a mixed solvent of tetrahydrofuran (150 mL) and methanol (150 mL), lithium borohydride (2.13 g, 97.68 mmol) was added dropwise at room temperature, and the mixture was reacted at room temperature for 2 hours. After incomplete consumption of the starting material was observed, lithium borohydride (2.13 g, 97.68 mmol) was added dropwise at room temperature, and the resulting mixture was reacted at room temperature for another 2 hours. After the reaction was completed, water (100 mL) and ethyl acetate (500 mL) were added to the reaction solution, and the mixture was extracted with ethyl acetate (100 mL × 3). The organic phase was washed with saturated sodium chloride aqueous solution (100 mL), dried over sodium sulphate, and subjected to evaporation to remove the solvent. The residue was purified by silica gel column chromatography (petroleum ether/ethyl acetate = 1/1), to afford 3 (10.0 g, yield: 79.6%).

MS *m*/*z* (ESI): 188.9 [M+1]⁺.

### Step 3

### (S, EZ)-5-(Hydroxymethyl)pyrrolidin-3-one O-methyloxime hydrochloride 4

Compound 3 (400 mg, 1.56 mmol) was dissolved in 4N hydrogen chloride dioxane solution (4 mL), and the mixture was reacted at room temperature for 1 hour. After the reaction was completed, the reaction solution was concentrated to remove the solvent to afford a crude product, compound 4 (400 mg), which was directly used in the next reaction.

MS *m*/*z* (ESI): 145.0 [M+1]⁺.

### Examples C0055&C0056

### (S, E)-(3'-Chloro-2'-methyl-[1,1'-biphenyl]-4-yl)(2-(hydroxymethyl)-4-(methoxyimino)pyrrolidin-1-yl)methanone

### (S, Z)-(3'-Chloro-2'-methyl-[1,1'-biphenyl]-4-yl)(2-(hydroxymethyl)-4-(methoxyimino)pyrrolidin-1-yl)methanone

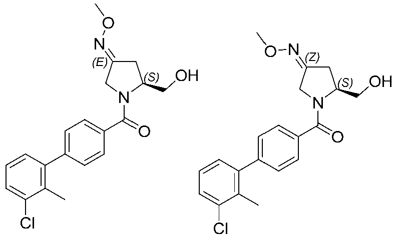

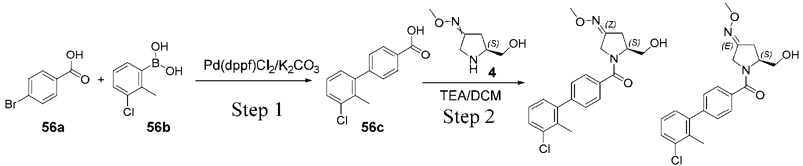

### Step 1

### 3'-Chloro-2'-methyl-[1,1'-biphenyl]-4-carboxylic acid 56c

To a solution of 56a (1.0 g, 4.97 mmol) in 1,4-dioxane/water (18/3 mL) were added 56b (1.10 g, 6.46 mmol), potassium carbonate (1.72 g, 12.42 mmol) and 1,1-bis(diphenylphosphino)ferrocene palladium(II) dichloride (365 mg, 0.5 mmol). After nitrogen purging, the mixture was reacted at 80°C for 3 hours. After the reaction was completed, the reaction solution was concentrated, and the crude product was purified by column chromatography (PE/EA=3:1 to 1:1) to afford 56c (450 mg, yield: 36.8%).

MS *m*/*z* (ESI): 247.0 [M+1]⁺.

### Step 2

### (S, Z)-(3'-Chloro-2'-methyl-[1,1'-biphenyl]-4-yl)(2-(hydroxymethyl)-4-(methoxyimino)pyrrolidin-1-yl)methanone

### (S, E)-(3'-Chloro-2'-methyl-[1,1'-biphenyl]-4-yl)(2-(hydroxymethyl)-4-(methoxyimino)pyrrolidin-1-yl)methanone

56c (150 mg, 0.61 mmol) and intermediate 4 (120 mg) were dissolved in DMF (4 mL). HATU (254 mg, 0.67 mmol) and triethylamine (184 mg, 1.83 mmol) were added at room temperature. After nitrogen purging, the reaction solution was reacted at room temperature for 2 hours. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness, to afford a crude product. The crude product was separated by silica gel Prep-TLC (EA:PE=2:1) to afford the more polar crude isomer C0056 (Rf=0.4) and the less polar crude isomer C0055 (Rf=0.5). The crude product was further purified by preparative HPLC and lyophilized to afford the more polar product C0056 (20.01 mg, yield: 8.8%) and the less polar product C0055 (13.2 mg, yield: 6.0%).

**C0056:** MS *m*/*z* (ESI): 373.1 [M+1]⁺.

¹H NMR (400 MHz, CD₃OD-*d*) *δ* 7.64-7.62 (m, 2H), 7.42-7.39 (m, 3H), 7.23-7.18 (m, 2H), 4.78-4.10 (m, 3H), 3.85-3.36 (m, 5H), 3.07-2.65 (m, 2H), 2.28 (s, 3H).

**C0055:** MS *m*/*z* (ESI): 373.1 [M+1]⁺.

¹H NMR (400 MHz, CD₃OD-*d*) *δ* 7.64-7.62 (m, 1H), 7.42-7.40 (m, 3H), 7.23-7.17 (m, 2H), 4.79-4.09 (m, 3H), 3.86-3.36 (m, 5H), 2.88-2.72 (m, 2H), 2.27 (s, 3H).

### Examples C0057&C0058

### (S, E)-4'-(2-(Hydroxymethyl)-4-(methoxyimino)pyrrolidine-1-carbonyl)-2-methyl-[1,1'-biphenyl]-3-carbonitrile

### (S, Z)-4'-(2-(Hydroxymethyl)-4-(methoxyimino)pyrrolidine-1-carbonyl)-2-methyl-[1,1'-biphenyl]-3-carbonitrile

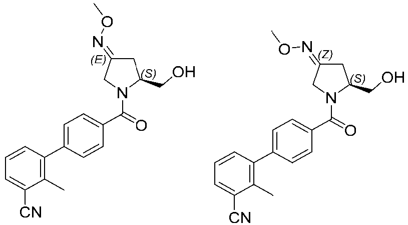

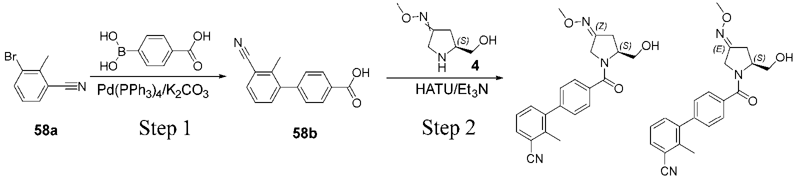

### Step 1

### 3'-Cyano-2'-methyl-[1,1'-biphenyl]-4-carboxylic acid 58b

To a solution of 58a (409.72 mg, 2.09 mmol) in methanol (20 mL) and water (10 mL) were added 4-(dihydroxyboryl)benzoic acid (0.38 g, 2.30 mmol), tetrakis(triphenylphosphine)palladium (0.24 g, 0.21 mmol) and potassium carbonate (0.87 g, 6.27 mmol). After nitrogen purging, the mixture was reacted at 85°C overnight. After the reaction was completed, the reaction solution was subjected to rotary evaporation to remove methanol. The aqueous phase was extracted with ethyl acetate and retained, adjusted to pH 3-4, and re-extracted with ethyl acetate. The organic layers were combined, dried over sodium sulphate, filtered, and subjected to rotary evaporation to afford 58b (400 mg, yield: 72.6%).

MS *m*/*z* (ESI): 238.1 [M+1]⁺.

### Step 2

### (S, Z)-4'-(2-(Hydroxymethyl)-4-(methoxyimino)pyrrolidine-1-carbonyl)-2-methyl-[1,1'-biphenyl]-3-carbonitrile

### (S, E)-4'-(2-(Hydroxymethyl)-4-(methoxyimino)pyrrolidine-1-carbonyl)-2-methyl-[1,1'-biphenyl]-3-carbonitrile

To a solution of 58b (400 mg, 1.69 mmol) in DMF (5 mL) were added intermediate 4 (0.29 g, 2.03 mmol), triethylamine (0.51 g, 5.07 mmol) and HATU (0.96 g, 2.54 mmol). After nitrogen purging, the reaction solution was reacted at room temperature for 2 hours. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness, to afford a crude product. The crude product was separated by silica gel Pre-TLC (EA:PE=5:1) to afford the less polar isomer C0057 (Rf=0.5) and the more polar crude isomer C0058 (Rf=0.4). Further purification by preparative HPLC and lyophilization yielded the less polar product C0057 (34 mg, yield: 5.6%) and the more polar product C0058 (22 mg, yield: 3.6%).

**C0057:** MS *m*/*z* (ESI): 364.1 [M+1]⁺.

¹H NMR (400 MHz, CD₃OD-*d*₄) *δ* 7.72-7.65 (m, 3H), 7.54-7.42 (m, 4H), 4.81-4.74 (m, 1H), 4.41-4.06 (m, 2H), 3.91-3.67 (m, 4H), 3.39-3.36 (m, 1H), 2.88-2.86 (m, 2H), 2.45 (s, 3H).

**C0058:** MS *m*/*z* (ESI): 364.1 [M+1]⁺.

¹H NMR (400 MHz, CD₃OD-*d*₄) *δ* 7.72-7.65 (m, 3H), 7.55-7.42 (m, 4H), 4.76-4.74 (m, 1H), 4.48-4.08 (m, 2H), 3.88-3.67 (m, 4H), 3.39-3.36 (m, 1H), 3.13-2.73 (m, 2H), 2.45 (s, 3H).

### Examples C0059&C0060

### (S,E)-(2-(Hydroxymethyl)-4-(methoxyimino)pyrrolidin-1-yl)(2'-methyl-3'-(trifluoromethyl)-[1,1'-biphenyl]-4-yl)methanone

### (S,Z)-(2-(Hydroxymethyl)-4-(methoxyimino)pyrrolidin-1-yl)(2'-methyl-3'-(trifluoromethyl)-[1,1'-biphenyl]-4-yl)methanone

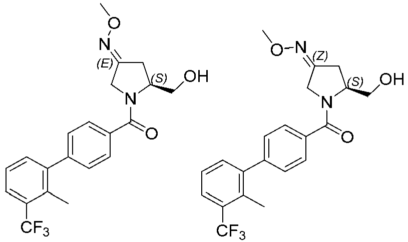

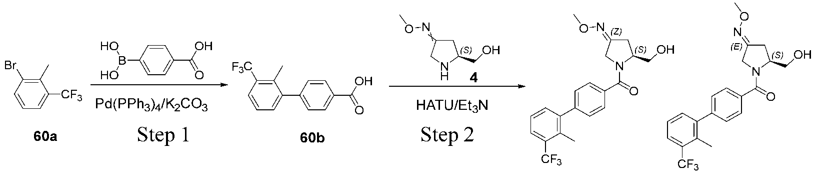

According to the aforementioned synthetic route, with reference to the synthetic method of compound C0057, the less polar isomer C0059 (Rf=0.5) and the more polar crude isomer C0060 (Rf=0.4) were obtained. Further purification by preparative HPLC and lyophilization yielded the less polar product C0059 (36 mg, yield: 9.9%) and the more polar product C0060 (27 mg, yield: 7.5%).

**C0059:** MS *m*/*z* (ESI): 407.1 [M+1]⁺.

¹H NMR (400 MHz, CD₃OD-*d*₄) *δ* 7.71-7.64 (m, 3H), 7.47-7.40 (m, 4H), 4.81-4.75 (m, 1H), 4.43-4.01 (m, 2H), 3.88-3.69 (m, 4H), 3.39-3.33 (m, 1H), 2.97-2.73 (m, 2H), 2.33 (s, 3H).

**C0060:** MS *m*/*z* (ESI): 407.1 [M+1]⁺.

¹H NMR (400 MHz, CD₃OD-*d*₄) *δ* 7.71-7.64 (m, 3H), 7.48-7.40 (m, 4H), 4.76-4.74 (m, 1H), 4.48-4.08 (m, 2H), 3.88-3.67 (m, 4H), 3.39-3.36 (m, 1H), 3.13-2.73 (m, 2H), 2.45 (s, 3H)

### Examples C0061&C0062

### (S, E)-(7-(2,3-Dimethylphenyl)benzo[d][1,3]dioxol-4-yl)(2-(hydroxymethyl)-4-(methoxyimino)pyrrolidin-1-yl)methanone

### (S, Z)-(7-(2,3-Dimethylphenyl)benzo[d][1,3]dioxol-4-yl)(2-(hydroxymethyl)-4-(methoxyimino)pyrrolidin-1-yl)methanone

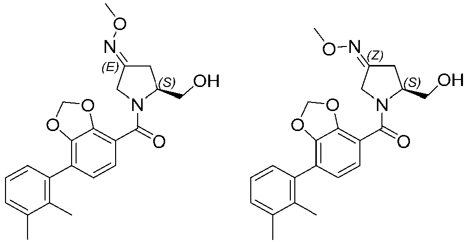

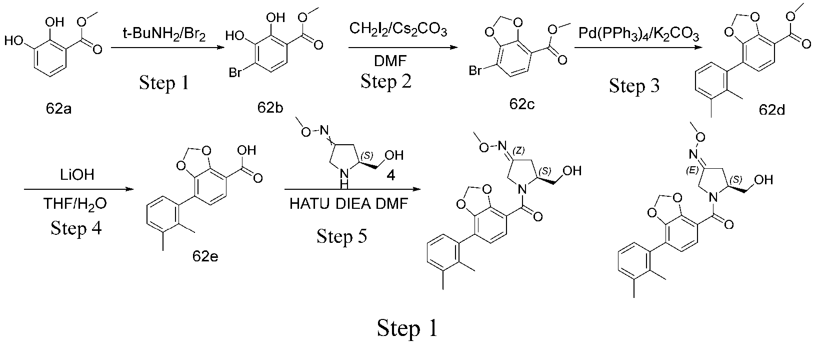

### Methyl 4-bromo-2,3-dihydroxybenzoate 62b

Liquid bromine (5.70 g, 35.69 mmol) was added dropwise to a solution of tert-butylamine (4.35 g, 59.48 mmol) in dichloromethane (10 mL) at -60°C. The reaction mixture was cooled to -78°C, and a solution of 62a (5 g, 29.74 mmol) in dichloromethane (25 mL) was added dropwise to the reaction mixture over 20 minutes. The mixture was stirred at -78°C for 30 minutes and then slowly heated to room temperature. After the reaction was completed, the reaction solution was extracted with ethyl acetate/water, and the organic layers were combined, dried over sodium sulphate, filtered, and subjected to rotary evaporation to afford a crude product. The crude product was purified by reversed-phase column chromatography (acetonitrile:water = 5:95 to 45:55) to afford product 62b (1.3 g, yield: 17.7%). ¹H NMR (400 MHz, CDCl₃) *δ* 11.02 (s, 1H), 7.19 (d*, J =* 8.8Hz, 1H), 6.98 (d*, J =* 8.8Hz, 1H), 5.99 (s, 1H), 3.87 (s, 3H).

### Step 2

### Methyl 7-bromo-benzo[d][1,3]dioxole-4-carboxylate 62c

To a solution of 62b (1.4 g, 5.67 mmol) in DMF (20 mL) was added caesium carbonate (3.69 g, 11.34 mmol), the mixture was stirred for 30 min, and then diiodomethane (2.43 g, 9.07 mmol) was added. After nitrogen purging, the mixture was stirred at 70°C for 12 hours. The reaction solution was extracted with ethyl acetate/water. The organic layers were combined, washed with saturated sodium chloride aqueous solution, combined, dried over sodium sulphate, filtered, and subjected to rotary evaporation to afford a product. The crude product was purified by silica gel preparative Pre-TLC (PE:EA=5:1), to afford product 62c (500 mg, yield: 34.1%).

### Step 3

### Methyl 7-(2,3-dimethylphenyl)benzo[d][1,3]dioxole-4-carboxylate 62d

To a solution of 62c (0.50 g, 1.93 mmol) in dioxane/water (10 mL/1 mL) were added potassium carbonate (0.80 g, 5.79 mmol), tetrakis(triphenylphosphine)palladium (0.21 g, 0.19 mmol) and (2,3-dimethylphenyl)boronic acid (0.33 g, 2.19 mmol). After nitrogen purging, the mixture was stirred at 80°C for 3 hours. The reaction solution was extracted with ethyl acetate/water. The organic layers were combined, washed with saturated sodium chloride aqueous solution, combined, dried over sodium sulphate, filtered, and subjected to rotary evaporation to afford a crude product. The crude product was purified by reversed-phase column chromatography (acetonitrile:water = 5:95 to 70:30) to afford product 62d (0.30 g, yield: 54.7%).

MS *m*/*z* (ESI): 285.0 [M+1]⁺.

### Step 4

### 7-(2,3-Dimethylphenyl)benzo[d][1,3]dioxole-4-carboxylic acid 62e

To a solution of 62d (200 mg, 0.67 mmol) in tetrahydrofuran/water (6 mL/3 mL) was added lithium hydroxide monohydrate (70.2 mg, 1.68 mmol), and the reaction solution was stirred at room temperature for 16 hours. The reaction solution was adjusted to pH approximately 2 with 1N hydrochloric acid aqueous solution and extracted with ethyl acetate/water. The organic layers were combined, washed with saturated sodium chloride aqueous solution, combined, dried over sodium sulphate, filtered, and subjected to rotary evaporation to afford a crude product. The crude product was purified by reversed-phase column chromatography (acetonitrile:water = 5:95 to 50:50) to afford product 62e (150 mg, yield: 78.9%).

MS *m*/*z* (ESI): 270.9 [M+1]⁺.

### Step 5

### (S, E)-(7-(2,3-Dimethylphenyl)benzo[d][1,3]dioxol-4-yl)(2-(hydroxymethyl)-4-(methoxyimino)pyrrolidin-1-yl)methanone

### (S, Z)-(7-(2,3-Dimethylphenyl)benzo[d][1,3]dioxol-4-yl)(2-(hydroxymethyl)-4-(methoxyimino)pyrrolidin-1-yl)methanone

To a solution of 62e (120 mg, 0.42 mmol) in DMF (4 mL) were added intermediate 4 (91 mg, 0.63 mmol), triethylamine (108 mg, 0.84 mmol) and HATU (239 mg, 0.63 mmol), and the reaction solution was reacted at room temperature for 16 hours. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness, to afford a crude product. The crude product was separated by silica gel Pre-TLC (EA:PE=2:1) to afford the less polar isomer C0061 (Rf=0.6) and the more polar isomer C0062 (Rf=0.55). Further purification by preparative HPLC and lyophilization yielded the less polar product C0061 (6.02 mg, yield: 3.4%) and the more polar product C0062 (6.1 mg, yield: 3.4%).

C0061: MS *m*/*z* (ESI): 397.1 [M+1]⁺.

¹H NMR (400 MHz, CD₃OD) *δ* 7.18-7.01 (m, 4H), 6.83 (d, *J =* 8.0 Hz, 1H), 6.07-6.02 (m, 2H), 4.84-4.40 (m, 2H), 4.20-4.14 (m, 1H), 3.87-3.39 (m, 5H), 2.95-2.81 (m, 2H), 2.32 (s, 3H), 2.13 (s, 3H).

C0062: MS *m*/*z* (ESI): 397.1 [M+1]⁺.

¹H NMR (400 MHz, CD₃OD) *δ* 7.18-7.00 (m, 4H), 6.83 (d, *J =* 8.4 Hz, 1H), 6.08-6.01 (m, 2H), 4.84-4.33 (m, 2H), 4.25-4.16 (m, 1H), 3.87-3.41 (m, 5H), 3.02-2.89 (m, 1H), 2.79-2.66 (m, 1H), 2.32 (s, 3H), 2.14 (s, 3H).

### Examples C0063&C0064

### (S, E)-(2-(Hydroxymethyl)-4-(methoxyimino)pyrrolidin-1-yl)(2-methoxy-2',3'-dimethyl-[1,1'-biphenyl]-4-yl)methanone

### (S, Z)-(2-(Hydroxymethyl)-4-(methoxyimino)pyrrolidin-1-yl)(2-methoxy-2',3'-dimethyl-[1,1'-biphenyl]-4-yl)methanone

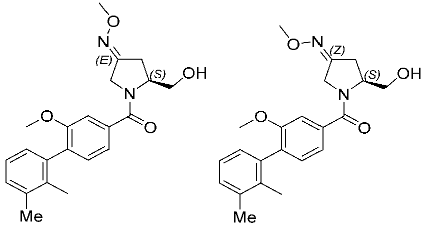

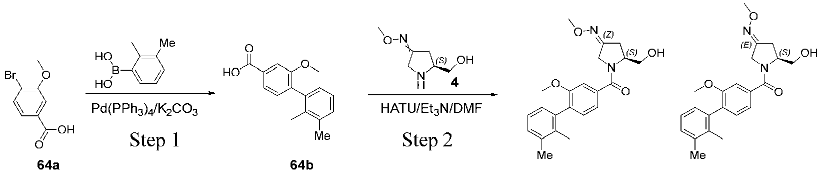

According to the aforementioned synthetic route, with reference to the synthetic method of compound C0056, the less polar isomer C0063 (Rf=0.5) and the more polar crude isomer C0064 (Rf=0.4) were obtained. Further purification by preparative HPLC and lyophilization yielded the less polar product C0063 (26 mg, yield: 5.0%) and the more polar product C0064 (28 mg, yield: 5.4%).

**C0063:** MS *m*/*z* (ESI): 383.1 [M+1]⁺.

¹H NMR (400 MHz, CD₃OD-*d*₄) *δ* 7.22-7.06 (m, 5H), 6.93 (d, *J =* 7.2 Hz, 1H), 4.78-4.77 (m, 1H), 4.41-4.13 (m, 2H), 3.90-3.81 (m, 4H), 3.69-3.40 (m, 1H), 2.89-2.87 (m, 2H), 2.31 (s, 3H), 1.98 (s, 3H).

**C0064:** MS *m*/*z* (ESI): 383.1 [M+1]⁺.

¹H NMR (400 MHz, CD₃OD-*d*₄) *δ* 7.21-7.06 (m, 5H), 6.93 (d, *J =* 6.4 Hz, 1H), 4.78-4.49 (m, 1H), 4.43-4.11 (m, 2H), 3.90-3.80 (m, 4H), 3.72-3.43 (m, 1H), 2.99-2.67 (m, 2H), 2.31 (s, 3H), 1.99 (s, 3H).

### Examples C0065&C0066

### (S, E)-(2-(Hydroxymethyl)-4-(methoxyimino)pyrrolidin-1-yl)(3'-methoxy-2'-methyl-[1,1'-biphenyl]-4-yl)methanone

### (S, Z)-(2-(Hydroxymethyl)-4-(methoxyimino)pyrrolidin-1-yl)(3'-methoxy-2'-methyl-[1,1'-biphenyl]-4-yl)methanone

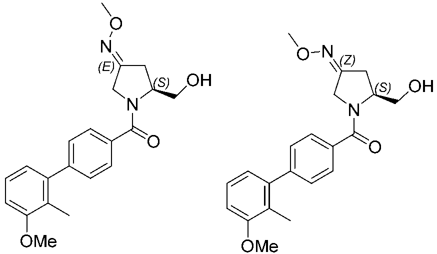

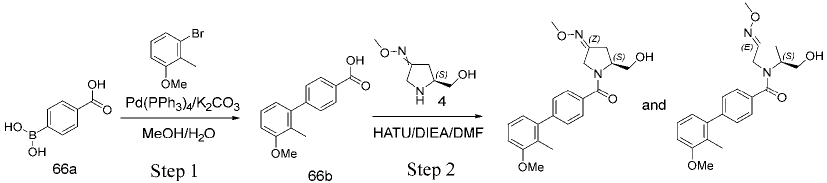

### Step 1

### 3'-Methoxy-2'-methyl-[1,1'-biphenyl]-4-carboxylic acid 66b

1-Bromo-3-methoxy-2-methylbenzene (500 mg, 2.49 mmol), 66a (496 mg, 2.99 mmol), potassium carbonate (1.03 g, 7.47 mmol) and tetrakis(triphenylphosphine)palladium (288 mg, 0.25 mmol) were dissolved in a mixed solution of methanol (20 mL) and water (10 mL). The mixture was purged three times with nitrogen, heated to 80°C under nitrogen protection, and stirred for 17 hours. After the reaction was completed, the reaction solution was subjected to rotary evaporation to remove methanol, and filtered. The filtrate was extracted with a small amount of ethyl acetate, and the aqueous phase was retained, adjusted to pH 3-4, and extracted twice with a mixed solution of toluene/tetrahydrofuran (1/1). The organic layers were combined, dried over sodium sulphate, and concentrated to afford 66b (470 mg, yield: 78.0%).

MS *m*/*z* (ESI): 241.0 [M+1]⁺.

### Step 2

### (S, Z)-(2-(Hydroxymethyl)-4-(methoxyimino)pyrrolidin-1-yl)(3'-methoxy-2'-methyl-[1,1'-biphenyl]-4-yl)methanone

### (S, E)-(2-(Hydroxymethyl)-4-(methoxyimino)pyrrolidin-1-yl)(3'-methoxy-2'-methyl-[1,1'-biphenyl]-4-yl)methanone

Under nitrogen protection, to a solution of intermediate 4 (180 mg, 1 mmol) in DMF (5 mL) were added HATU (570 mg, 1.5 mmol), diisopropylethylamine (517 mg, 4 mmol) and 66b (242 mg, 1 mmol), and the reaction solution was reacted at room temperature overnight. Water (50 mL) was added to quench the reaction solution, and the reaction solution was extracted with ethyl acetate (50 mL × 3). The organic layers were combined, washed with saturated sodium chloride aqueous solution (50 mL × 3), combined, dried over anhydrous sodium sulphate, filtered, and concentrated. The crude product was purified by silica gel Pre-TLC (PE:EA=1:1) to afford the less polar product C0065 (15 mg, yield: 4.1%, Rf=0.3) and the more polar product C0066 (20 mg, yield: 14.6%, Rf=0.2).

**C0065:** MS *m*/*z* (ESI): 369.1 [M+1]⁺.

¹H NMR (400 MHz, CD₃OD) *δ* 7.60 (d, *J =* 7.6 Hz, 2H), 7.39 (d, *J =* 8.0 Hz, 2H), 7.21 (t*, J =* 8.0, 7.6 Hz, 1H), 6.94 (d, *J =* 8.0 Hz, 1H), 6.81 (d, *J =* 7.6 Hz, 1H), 4.77-4.41 (m, 1H), 4.38-4.07 (m, 2H), 3.86-3.68 (m, 8H), 3.41-3.31 (m, 1H), 2.88-2.86 (m, 2H), 2.08 (s, 3H).

**C0066:** MS *m*/*z* (ESI): 369.1 [M+1]⁺.

¹H NMR (400 MHz, CD₃OD) *δ* 7.59 (d, *J =* 7.2 Hz, 2H), 7.39 (d, *J =* 8.4 Hz, 2H), 7.21 (t, *J* = 8.0 Hz, 1H), 6.94 (d, *J* = 8.0 Hz, 1H), 6.82 (d, *J =* 7.2 Hz, 1H), 4.76-4.53 (m, 1H), 4.53-4.09 (m, 2H), 3.86-3.68 (m, 7H), 3.38-3.31 (m, 1H), 2.98-2.73 (m, 2H), 2.08 (s, 3H).

### Examples A0134&A0145

### (S,Z)-(2-(Difluoromethyl)-2', 3'-dimethyl-[1,1'-biphenyl]-4-yl)(2-(hydroxymethyl)-4-(methoxyimino)pyrrolidin-1-yl)methanone

### (S,E)-(2-(Difluoromethyl)-2', 3'-dimethyl-[1,1'-biphenyl]-4-yl)(2-(hydroxymethyl)-4-(methoxyimino)pyrrolidin-1-yl)methanone

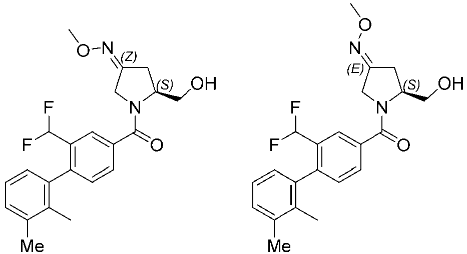

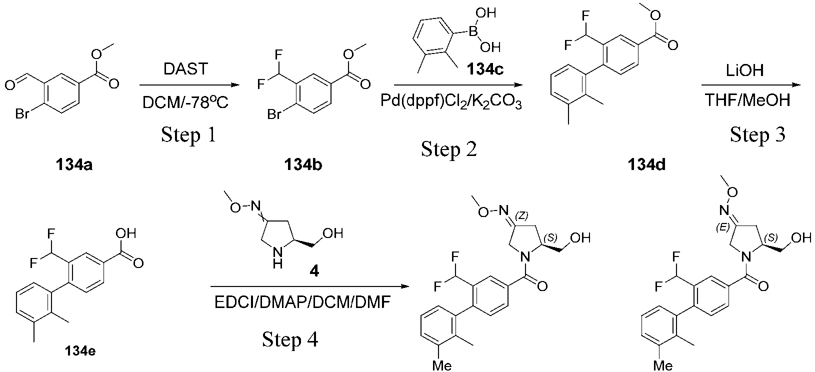

### Step 1

### Methyl 4-bromo-3-difluoromethylbenzoate 134b

134a (500 mg, 2.06 mmol) was dissolved in DCM (5 mL), and the mixture was cooled to 0°C. Diethylaminosulphur trifluoride (0.50 g, 3.09 mmol) was added, and then the mixture was reacted at room temperature for 16 hours. The reaction solution was poured into saturated sodium carbonate solution and extracted with dichloromethane. The organic phase was dried over anhydrous sodium sulphate and concentrated, and the crude product was purified by column chromatography (petroleum ether/ethyl acetate = 30:1), to afford **134b** (400 mg, yield: 73.4%).

MS *m*/*z* (ESI): 265 [M+1]⁺.

### Step 2

### Methyl 2-(difluoromethyl)-2',3'-dimethyl-[1,1'-biphenyl]-4-carboxylate 134d

With reference to the synthetic method of compound A0061, **134d** (200 mg, yield: 45.6%) was obtained.

MS *m*/*z* (ESI): 291 [M+1]⁺.

### Step 3

### 2-(Difluoromethyl)-2',3'-dimethyl-[1,1'-biphenyl]-4-carboxylic acid 134e

With reference to the synthetic method of compound A0061, crude **134e** (0.2 g, yield: 94.4%) was obtained.

MS *m*/*z* (ESI): 277 [M+1]⁺.

### Step 4

### (S,Z)-(2-(Difluoromethyl)-2', 3'-dimethyl-[1,1'-biphenyl]-4-yl)(2-(hydroxymethyl)-4-(methoxyimino)pyrrolidin-1-yl)methanone

### (S,E)-(2-(Difluoromethyl)-2', 3'-dimethyl-[1,1'-biphenyl]-4-yl)(2-(hydroxymethyl)-4-(methoxyimino)pyrrolidin-1-yl)methanone

134e (100 mg, 0.42 mmol) and intermediate 4 (0.061 g, 0.36 mmol) were dissolved in DCM (1.7 mL) and DMF (4 mL), and then DMAP (0.10 g, 0.84 mmol) and EDCI (0.081 g, 0.42 mmol) were slowly added. The reaction solution was stirred at room temperature for 16 hours. The reaction solution was diluted with dichloromethane, and washed with sodium chloride aqueous solution and 1N hydrochloric acid, successively. The organic phase was dried over anhydrous sodium sulphate and concentrated, and the crude product was purified by silica gel Prep-TLC (EA:PE=5:1) to afford the more polar crude product **A0134** (Rf=0.4) and the less polar crude product **A0145** (Rf=0.5). Further purification by preparative HPLC and lyophilization yielded the more polar **A0134** (5.37 mg, yield: 3.7%) and the less polar **A0145** (6.82 mg, yield: 4.6%).

**A0134:** MS *m*/*z* (ESI): 403.0 [M+1]⁺.

¹H NMR (400 MHz, Chloroform-*d*) *δ* 7.91-7.89 (m, 1H), 7.68-7.61 (m, 1H), 7.29-7.22 (m, 2H), 7.20-7.13 (m, 1H), 6.99-6.95 (m, 1H), 6.47-6.12 (m, 1H), 4.89-4.88 (m, 1H), 4.42-4.16 (m, 2H), 3.97-3.76 (m, 5H), 3.00-2.90 (m, 1H), 2.71-2.66 (m, 1H), 2.34 (s, 3H), 1.96 (s, 3H).

**A0145:** MS *m*/*z* (ESI): 403.0 [M+1]⁺.

¹H NMR (400 MHz, Chloroform-d) *δ* 7.88 (s, 1H), 7.64 (d, *J =* 8.0 Hz, 1H), 7.28-7.21 (m, 2H), 7.20-7.14 (m, 1H), 6.98-6.85 (m, 1H), 6.44-6.16 (m, 1H), 4.89 (brs, 1H), 4.40-4.18 (m, 2H), 3.94-3.78 (m, 5H), 2.95-2.69 (m, 2H), 2.33 (s, 3H), 1.93 (s, 3H).

### Examples C0067&C0068

### (S, Z, RS)-(2-(1-Hydroxyethyl)-4-(methoxyimino)pyrrolidin-1-yl)(2'-methyl-[1,1'-biphenyl]-4-yl)methanone

### (S, E, RS)-(2-(1-Hydroxyethyl)-4-(methoxyimino)pyrrolidin-1-yl)(2'-methyl-[1,1'-biphenyl]-4-yl)methanone

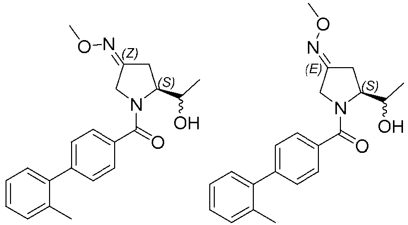

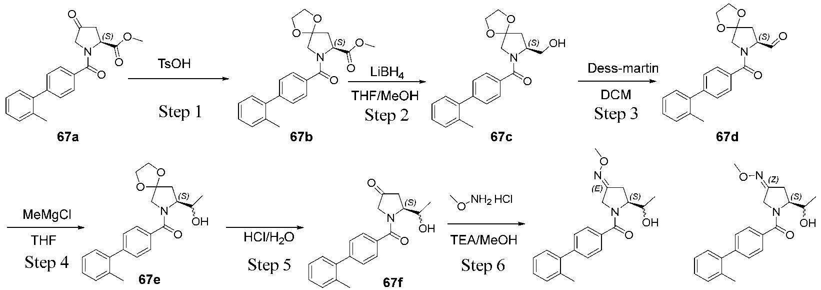

### Step 1

### Methyl (S)-7-(2'-methyl-[1,1'-biphenyl]-4-carbonyl)-1,4-dioxo-7-azaspiro[4.4]nonane-8-carboxylate 67b

67a (800 mg, 2.37 mmol, prepared with reference to the method of 20g) was dissolved in toluene (7 mL), ethylene glycol (221 mg, 3.56 mmol) and p-toluenesulfonic acid hydrate (90 mg, 0.47 mmol) were added to the reaction solution, and the mixture was reacted at 120°C for 16 hours. After the reaction was completed, the reaction solution was subjected to rotary evaporation to remove the solvent, and the residue was purified by Prep-TLC (PE/EA=6/1) to afford product 67b (500 mg, yield: 55.3%).

MS *m*/*z* (ESI): 382.0 [M+1]⁺.

### Step 2

### (S)-(8-(Hydroxymethyl)-1,4-dioxo-7-azaspiro[4.4]nonan-7-yl)(2'-methyl-[1,1'-biphenyl]-4-yl)methanone 67c

67b (160 mg, 0.42 mmol) was dissolved in methanol (5 mL) and tetrahydrofuran (5 mL), and lithium borohydride tetrahydrofuran solution (2 mol/L, 0.42 mL) was added dropwise at -10°C. After the dropwise addition was completed, the reaction solution was heated to room temperature and stirred for 3 hours. After the reaction was completed, water was added to quench the reaction solution, the reaction solution was concentrated, and the residue was dissolved in dichloromethane and water. After layer separation, the aqueous phase was extracted twice with dichloromethane, and the organic phases were combined, washed once with sodium chloride aqueous solution, dried, filtered, and subjected to rotary evaporation to dryness. The crude product was loaded onto a silica gel column, eluting with a gradient of (3:1 to 1:1 PE/EA). The eluate was collected and concentrated to afford product 67c (100 mg, yield: 67.4%).

MS *m*/*z* (ESI): 354.0 [M+1]⁺.

### Step 3

### (S)-7-(2'-Methyl-[1,1'-biphenyl]-4-carbonyl)-1,4-dioxo-7-azaspiro[4.4]nonane-8-carbaldehyde 67d

67c (450 mg, 1.27 mmol) was dissolved in dichloromethane (3 mL), Dess-Martin oxidant (539 mg, 0.14 mmol) was added to the reaction solution, and the mixture was reacted at room temperature for 16 hours. After the reaction was completed, dichloromethane and water were added, and the mixture was extracted twice with dichloromethane. The organic phases were combined, subjected to rotary evaporation to remove the solvent, washed once with sodium chloride aqueous solution, dried, filtered, and subjected to rotary evaporation to dryness. The crude product was loaded onto a silica gel column, eluting with a gradient of (2:1 to 1:1 PE/EA). The eluate was collected and concentrated to afford product 67d (350 mg, yield: 78.2%).

MS *m*/*z* (ESI): 352.0 [M+1]⁺.

### Step 4

### (S, RS)-(8-(1-Hydroxyethyl)-1,4-dioxo-7-azaspiro[4.4]nonan-7-yl)(2'-methyl-[1,1'-biphenyl]-4-yl)methanone 67e

67d (320 mg, 0.91 mmol) was dissolved in tetrahydrofuran (5 mL), 4.5 mL of 3M methylmagnesium chloride tetrahydrofuran solution was added at -78°C, and the mixture was heated to room temperature and stirred for 20 minutes. After the reaction was completed, water was added to the reaction solution to quench the reaction solution, and the reaction solution was extracted three times with water and ethyl acetate. The organic phase was collected, washed once with sodium chloride aqueous solution, dried over anhydrous sodium sulphate, filtered, and subjected to rotary evaporation to remove the solvent to afford product 67e (180 mg, yield: 53.8%).

MS *m*/*z* (ESI): 368.0 [M+1]⁺.

### Step 5

### (S, RS)-5-(1-Hydroxyethyl)-1-(2'-methyl-[1,1'-biphenyl]-4-carbonyl)pyrrolidin-3-one 67f

67e (130 mg, 0.35 mmol) was dissolved in hydrochloric acid (2 mL) and water (20 mL), and the mixture was reacted at 100°C for 1 hour. After the reaction was completed, the reaction solution adjusted to neutral, and extracted three times with water and ethyl acetate. The organic phase was collected, washed once with sodium chloride aqueous solution, dried over anhydrous sodium sulphate, and filtered. The crude product was loaded onto a silica gel column, eluting with a gradient of (1:1 to 1:2 PE/EA). The eluate was collected and concentrated to afford product 67f (75 mg, yield: 65.5%).

MS *m*/*z* (ESI): 324.0 [M+1]⁺.

### Step 6

### (S, E, RS)-(2-(1-Hydroxyethyl)-4-(methoxyimino)pyrrolidin-1-yl)(2'-methyl-[1,1'-biphenyl]-4-yl)methanone

### (S, Z, RS)-(2-(1-Hydroxyethyl)-4-(methoxyimino)pyrrolidin-1-yl)(2'-methyl-[1,1'-biphenyl]-4-yl)methanone

67f (100 mg, 0.31 mmol) was dissolved in methanol (5 mL), methoxyamine hydrochloride (26 mg, 0.31 mmol) and triethylamine (78 mg, 0.78 mmol) were added at room temperature, and the mixture was reacted at 50°C for 18 hours. The reaction solution was subjected to rotary evaporation to remove methanol, and extracted three times with dichloromethane and water. The organic phases were combined, washed once with sodium chloride aqueous solution, dried, filtered, and subjected to rotary evaporation to dryness. The crude product was purified by silica gel Prep-TLC (PE:EA=1:3), to afford the less polar crude product C0067 (Rf=0.7) and the more polar crude product C0068 (Rf=0.5). Further purification by preparative HPLC and lyophilization yielded the less polar product C0067 (3.94 mg, yield: 3.6%) and the more polar product C0068 (8.96 mg, yield: 8.2%).

C0067: MS *m*/*z* (ESI): 353.1[M+1]⁺.

¹H NMR (400 MHz, CD₃OD) *δ* 7.64-7.62 (m, 2H), 7.43 (d, *J =* 8.0 Hz, 2H), 7.28-7.19 (m, 4H), 4.64-4.41 (m, 2H), 4.25-4.05 (m, 2H), 3.85-3.80 (m, 3H), 2.98-2.94 (m, 1H), 2.76-2.69 (m, 1H), 2.25 (s, 3H), 1.29-1.21 (m, 3H).

C0068: MS *m*/*z* (ESI): 353.1[M+1]⁺.

### Examples A0167&A0213

### 3-(4-[(2S,4Z)-2-(Hydroxymethyl)-4-(methoxyimino)pyrrolidine-1-carbonyl]-1-methyl-1H-indazol-7-yl)-2-methylbenzonitrile

### 3-(4-[(2S,4E)-2-(Hydroxymethyl)-4-(methoxyimino)pyrrolidine-1-carbonyl]-1-methyl-1H-indazol-7-yl)-2-methylbenzonitrile

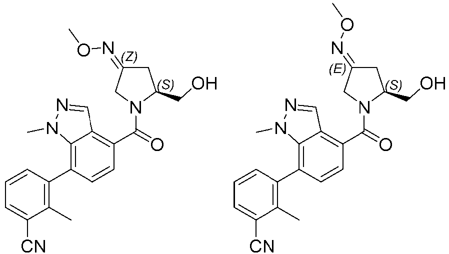

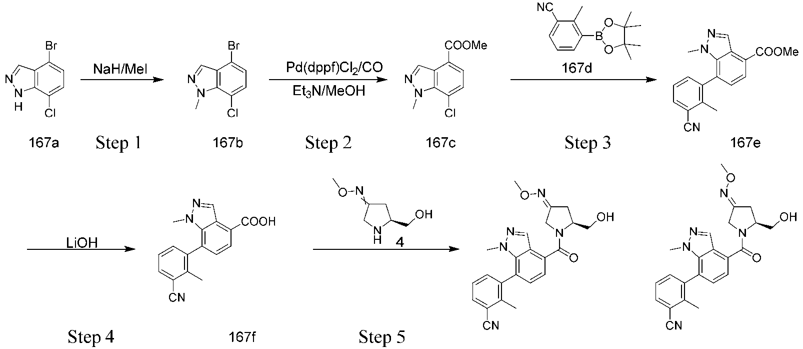

### Step 1

### 4-Bromo-7-chloro-1-methyl-1H-indazole 167b

167a (4 g, 17.28 mmol) was dissolved in DMF (30 mL), sodium hydride (1.38 g, 34.56 mmol) was added at 0°C, and the mixture was reacted for 40 minutes. Iodomethane (2.94 g, 20.74 mmol) was added, and the mixture was heated to 20°C and reacted for 1 hour. The reaction solution was quenched with saturated ammonium chloride solution (150 mL), and extracted with ethyl acetate (50 mL × 3). The organic phases were combined, washed with saturated sodium chloride solution (100 mL × 2), dried, mixed with adsorbent, and purified by silica gel column chromatography (PE:EA=76:24) to afford product 167b (1.67 g, yield: 39.4%).

MS *m*/*z* (ESI): 244.9[M+1]⁺.

### Step 2

### Methyl 7-chloro-1-methyl-1H-indazole-4-carboxylate 167c

167b (1.40 g, 5.70 mmol), Pd(dppf)Cl₂ (0.42 g, 0.57 mmol), and triethylamine (1.73 g, 17.1 mmol) were dissolved in methanol (15 mL), and the mixture was reacted at 60°C under a CO atmosphere for 5 hours. The reaction solution was directly concentrated, mixed with adsorbent, and purified by silica gel column chromatography (PE:EA=3:1) to afford 167c (0.73 g, yield: 56.9%).

MS *m*/*z* (ESI): 224.9[M+1] ⁺.

### Step 3

### Methyl 7-(3-cyano-2-methylphenyl)-1-methyl-1H-indazole-4-carboxylate 167e

167c (0.230 g, 1.02 mmol), 167d (0.25 g, 1.02 mmol), potassium carbonate (0.28 g, 2.04 mmol), and Pd(dtbpf)Cl₂ (0.066 g, 0.10 mmol) were dissolved in 1,4-dioxane (4 mL) and water (1 mL), and the mixture was reacted at 100°C under an argon atmosphere for 16 hours. The reaction solution was directly concentrated, mixed with adsorbent, and purified by silica gel column chromatography (PE:EA=3:1) to afford 167e (0.30 g, yield: 95.9%).

MS *m*/*z* (ESI): 306.0[M+1] ⁺.

### Step 4

### 7-(3-Cyano-2-methylphenyl)-1-methyl-1H-indazole-4-carboxylic acid 167f

167e (0.10 g, 0.33 mmol) and lithium hydroxide (0.042 g, 0.99 mmol) were dissolved in methanol (0.5 mL), tetrahydrofuran (0.5 mL), and water (0.5 mL), and the mixture was reacted at 40°C for 2 hours. 3 mL of water was added to adjust the pH to 2-3, and the mixture was extracted with ethyl acetate (50 mL × 3), dried, and concentrated to afford 167f (0.07 g, yield: 73.4%).

MS *m*/*z* (ESI): 292.1[M+1]⁺.

### Step 5

### 3-(4-[(2S,4Z)-2-(Hydroxymethyl)-4-(methoxyimino)pyrrolidine-1-carbonyl]-1-methyl-1H-indazol-7-yl)-2-methylbenzonitrile

### 3-(4-[(2S,4E)-2-(Hydroxymethyl)-4-(methoxyimino)pyrrolidine-1-carbonyl]-1-methyl-1H-indazol-7-yl)-2-methylbenzonitrile

167f (0.07 g, 0.24 mmol), 4 (0.035 g, 0.24 mmol), and DMAP (0.059 g, 0.48 mmol) were dissolved in DMF (3 mL)/DCM (2 mL), EDCI (0.055 g, 0.29 mmol) was added at 15°C, and the mixture was reacted for 16 hours. The reaction solution was diluted with ethyl acetate, and washed with sodium chloride aqueous solution and 1N hydrochloric acid, successively. The organic phase was dried over anhydrous sodium sulphate and concentrated, and the residue was purified by silica gel Pre-TLC (PE:EA=1:3) to afford the less polar crude isomer A0213 (Rf=0.4) and the more polar crude isomer A0167 (Rf=0.35). Further purification by preparative HPLC and lyophilization yielded the more polar product A0167 (2.14 mg, yield: 2.05%) and the less polar product A0213 (2.03 mg, yield: 1.97%).

A0167: MS *m*/*z* (ESI): 418.3 [M+1]⁺.

¹H NMR (400 MHz, Chloroform-*d*) *δ* 8.11 (s, 1H), 7.76 (d, *J =* 7.6 Hz, 1H), 7.55-7.52 (m, 1H), 7.43 (m, 1H), 7.30 (m, 1H), 7.15 (dd, *J =* 7.2, 4.0 Hz, 1H), 4.97 (brs, 1H), 4.34-4.30 (m, 1H), 4.20-3.93 (m, 2H), 3.90-3.75 (m, 4H), 3.51 (d, *J =* 4.0 Hz, 3H), 3.03-2.76 (m, 2H), 2.72-2.63 (m, 1H), 2.30 (s, 3H).

A0213: MS *m*/*z* (ESI): 418.3 [M+1]⁺.

¹H NMR (400 MHz, Chloroform-d) *δ* 8.12 (s, 1H), 7.76 (d, *J* = 7.6 Hz, 1H), 7.52-7.50 (m, 1H), 7.43 (m, 1H), 7.30 (m, 1H), 7.15 (d, *J =* 7.2 Hz, 1H), 4.97 (brs, 1H), 4.35-4.27 (m, 1H), 4.20-4.00 (m, 1H), 3.87-3.82 (m, 4H), 3.53 (d, *J =* 4.4 Hz, 3H), 3.22-3.10 (m, 1H), 3.05-2.88 (m, 1H), 2.80-2.70 (m, 1H), 2.29 (t, *J =* 4.0 Hz, 3H).

### Examples A0173&A0181

### 2-(3-Cyano-2-methylphenyl)-5-[(2S, 4Z)-2-(hydroxymethyl)-4-(methoxyimino)pyrrolidine-1-carbonyl]benzonitrile

### 2-(3-Cyano-2-methylphenyl)-5-[(2S, 4E)-2-(hydroxymethyl)-4-(methoxyimino)pyrrolidine-1-carbonyl]benzonitrile

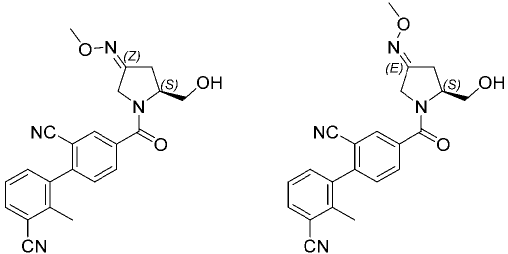

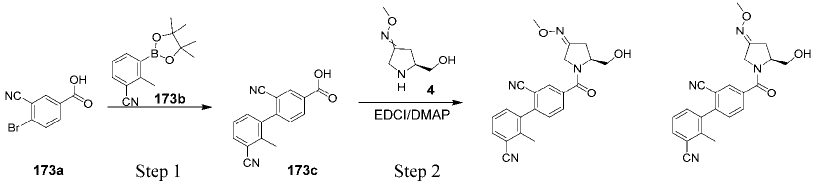

### Step 1

### 3-Cyano-4-(3-cyano-2-methylphenyl)benzoic acid 173c

173a (0.25 g, 1.11 mmol), 173b (0.27 g, 1.11 mmol), Pd(dppf)Cl₂ (0.081 g, 0.11 mmol), and potassium carbonate (0.31 g, 2.22 mmol) were dissolved in 1,4-dioxane (1 mL) and water (0.2 mL), and the mixture was reacted at 100°C under argon protection for 4 hours. The reaction solution was filtered, concentrated, dissolved in ethyl acetate (20 mL), washed with 1N hydrochloric acid, dried, and concentrated to afford product 173c (350 mg, crude).

MS *m*/*z* (ESI): 263.0[M+1]⁺.

### Step 2

### 2-(3-Cyano-2-methylphenyl)-5-[(2S, 4Z)-2-(hydroxymethyl)-4-(methoxyimino)pyrrolidine-1-carbonyl]benzonitrile

### 2-(3-Cyano-2-methylphenyl)-5-[(2S, 4E)-2-(hydroxymethyl)-4-(methoxyimino)pyrrolidine-1-carbonyl]benzonitrile

173c (0.20 g, 0.76 mmol), 4 (0.19 g, 0.99 mmol), and DMAP (0.19 g, 1.52 mmol) were dissolved in DMF (1 mL) and DCM (2 mL), EDCI (0.19 g, 0.99 mmol) was added at 15°C, and the mixture was reacted for 16 hours. The reaction solution was diluted with ethyl acetate, and washed with sodium chloride aqueous solution and 1N hydrochloric acid, successively. The organic phase was dried over anhydrous sodium sulphate and concentrated, and the residue was purified by silica gel Pre-TLC (PE:EA=1:3) to afford the less polar crude isomer A0181 (Rf=0.4) and the more polar crude isomer A0173 (Rf=0.35). Further purification by preparative HPLC and lyophilization yielded the more polar product A0173 (36.03 mg, yield: 11.6%) and the less polar product A0181 (25.85 mg, yield: 7.25%).

A0173: MS *m*/*z* (ESI): 389.1 [M+1]⁺.

¹H NMR (400 MHz, Chloroform-*d*) *δ* 7.98 (d, *J =* 17.2 Hz, 1H), 7.85 (d, *J* = 6.0 Hz, 1H), 7.75 (d, *J =* 7.6 Hz, 1H), 7.45-7.41 (m, 3H), 4.89 (brs, 1H), 4.41-4.37 (m, 1H), 4.19-4.10 (m, 1H), 4.00-3.92 (m, 1H), 3.87 (d, *J=* 4.0 Hz, 3H), 3.83-3.72 (m, 1H), 3.02-2.89 (m, 1H), 2.85-2.69 (m, 1H), 2.41 (d, *J =* 6.8Hz, 3H).

A0181: MS *m*/*z* (ESI): 389.1 [M+1]⁺.

¹H NMR (400 MHz, Chloroform-*d*) *δ* 7.96 (d, *J =* 17.2 Hz, 1H), 7.84 (d, *J* = 7.6 Hz, 1H), 7.76-7.74 (m, 1H), 7.45-7.41 (m, 3H), 4.89 (brs, 1H), 4.41-4.37 (m, 1H), 4.19-4.10 (m, 1H), 4.00-3.89 (m, 1H), 3.88 (d, *J =* 4.0 Hz, 3H), 3.82-3.72 (m, 1H), 2.99-2.89 (m, 1H), 2.80-2.69 (m, 1H), 2.41 (d, *J =* 6.4 Hz, 3H).

### Examples A0183&A0184

### 3-(4-[(2S, 4Z)-2-(Hydroxymethyl)-4-(methoxyimino)pyrrolidine-1-carbonyl]-2-(3-hydroxyoxetan-3-yl)phenyl)-2-methylbenzonitrile

### 3-(4-[(2S, 4E)-2-(Hydroxymethyl)-4-(methoxyimino)pyrrolidine-1-carbonyl]-2-(3-hydroxyoxetan-3-yl)phenyl)-2-methylbenzonitrile

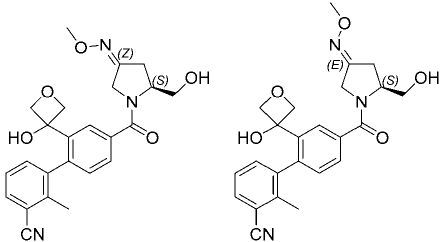

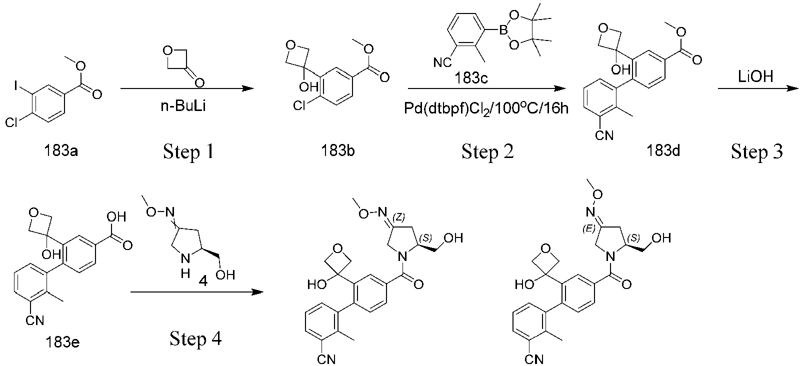

### Step 1

### Methyl 4-chloro-3-(3-hydroxyoxetan-3-yl)benzoate 183b

183a (2 g, 6.75 mmol) and oxetan-3-one (1.46 g, 20.25 mmol) were dissolved in THF (10 mL), and n-butyllithium (0.56 g, 8.78 mmol) was added dropwise at - 60°C to -70°C. After the dropwise addition was completed, the mixture was reacted for 30-60 minutes. Saturated ammonium chloride (50 mL) was added to quench the reaction solution, and the reaction solution was extracted with ethyl acetate (50 mL × 3), dried, concentrated, mixed with adsorbent, and purified by silica gel column chromatography to afford 183b (0.55 g, yield: 33.6%).

MS *m*/*z* (ESI): 225.0[M-17+1]⁺. According to Step 2 to Step 4 in the aforementioned synthetic route, with reference to the synthetic method of compound A0167, the less polar crude isomer A0184 (Rf=0.4) and the more polar crude isomer A0183 (Rf=0.35) were obtained. Further purification by preparative HPLC and lyophilization yielded the more polar product A0183 (15.58 mg, yield: 15.6%) and the less polar product A0184 (18.67 mg, yield: 18.0%).

A0183: MS *m*/*z* (ESI): 436.0 [M+1]⁺.

¹H NMR (400 MHz, Chloroform-d) *δ* 7.67 (d, *J =* 7.6 Hz, 1H), 7.52-7.45 (m, 2H), 7.41 (s, 1H), 7.34-7.26 (m, 1H), 7.19-7.16 (m, 1H), 5.04-4.87 (m, 2H), 4.55-4.50 (m, 1H), 4.48-4.10 (m, 3H), 3.98-3.86 (m, 5H), 3.80-3.52 (m, 2H), 2.96-2.59 (m, 2H), 2.34 (d, *J =* 8.8 Hz, 3H).

A0184: MS *m*/*z* (ESI): 436.0 [M+1]⁺.

¹H NMR (400 MHz, Chloroform-d) *δ* 7.66 (d, *J =* 6.8 Hz, 1H), 7.52-7.47 (m, 2H), 7.41 (s, 1H), 7.34-7.26 (m, 1H), 7.18 (d, *J =* 8.0 Hz, 1H), 5.04-4.86 (m, 2H), 4.54-4.22 (m, 5H), 4.00-3.83 (m, 5H), 3.80-3.72 (m, 1H), 2.98-2.87 (m, 1H), 2.69-2.60 (m, 1H), 2.34 (d, *J =* 8.8 Hz, 3H).

### Examples C0050&C0051

### (S, E)-(4-(2,3-Dihydro-1H-inden-4-yl)phenyl)(2-(hydroxymethyl)-4-(methoxyimino)pyrrolidin-1-yl)methanone

### (S, Z)-(4-(2,3-Dihydro-1H-inden-4-yl)phenyl)(2-(hydroxymethyl)-4-(methoxyimino)pyrrolidin-1-yl)methanone

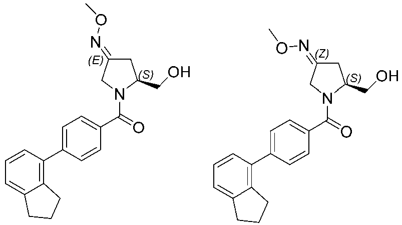

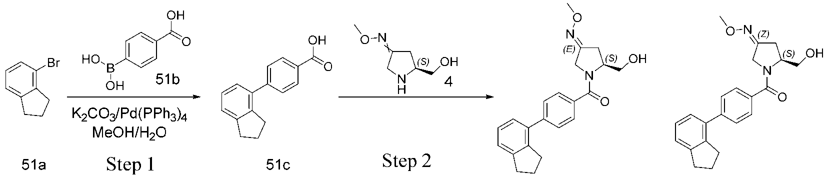

According to the aforementioned synthetic route, with reference to the synthetic method of compound C0055, the less polar isomer C0050 (Rf=0.6) and the more polar crude isomer C0051 (Rf=0.4) were obtained. Further purification by preparative HPLC and lyophilization yielded the less polar product C0050 (29.1 mg, yield: 11.2%) and the more polar product C0051 (26.2 mg, yield: 10.0%).

C0050: MS *m*/*z* (ESI): 365.1 [M+1]⁺.

¹H NMR (400 MHz, CD₃OD-*d*₄) *δ* 7.62-7.53 (m, 4H), 7.25-7.16 (m, 3H), 4.83-3.68 (m, 7H), 3.31-3.30 (m, 1H), 2.99-2.86 (m, 4H), 2.88-2.86 (m, 2H), 2.08-2.01 (m, 2H).

C0051: MS *m*/*z* (ESI): 365.1 [M+1]⁺.

¹H NMR (400 MHz, CD₃OD-*d*₄) *δ* 7.62-7.55 (m, 4H), 7.25-7.16 (m, 3H), 4.76-4.09 (m, 3H), 3.84-3.38 (m, 5H), 2.99-2.75 (m, 6H), 2.08-2.01 (m, 2H).

### Examples C0074&C0075 (S,E)-4'-(2-(hydroxymethyl)-4-(methoxymino)pyrrolidine-1-carbonyl)-2,3- dimethyl-[1,1'-biphenyl]-4-carbonitrile (S,Z)-4'-(2-(hydroxymethyl)-4-(methoxymino)pyrrolidine-1-carbonyl)-2,3- dimethyl-[1,1'-biphenyl]-4-carbonitrile

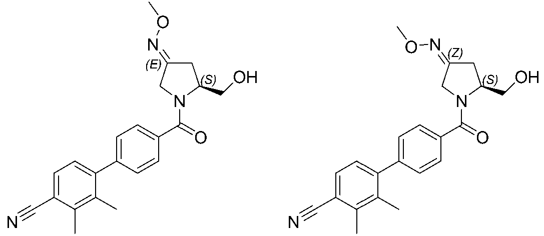

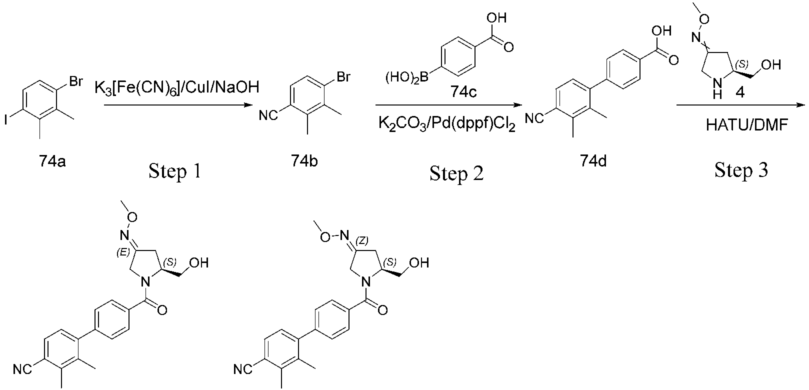

### Step 1

### 4-Bromo-2,3-dimethylbenzonitrile 74b

74a (2 g, 6.43 mmol) was dissolved in ethylene glycol (30 mL), potassium ferrocyanide (2.12 g, 6.43 mmol), sodium hydroxide (0.51 g, 12.86 mmol) and cuprous iodide (0.12 g, 0.64 mmol) were added, and the reaction solution was stirred at 140°C for 18 hours. After the reaction was completed, the reaction solution was poured into water and extracted with ethyl acetate. The organic phases were combined, dried, and subjected to rotary evaporation to dryness, and the crude product was purified by column chromatography (PE/EA=10:1) to afford 74b (0.9 g, yield: 66.6%).

MS *m*/*z* (ESI): 210.0 [M+1]⁺.

According to Step 2 to Step 3 in the aforementioned synthetic route, with reference to the synthetic method of compound C0065, the less polar crude product C0074 (Rf=0.4) and the more polar crude product C0075 (Rf=0.3) were obtained. Further purification by preparative HPLC and lyophilization yielded the less polar product C0074 (11 mg, yield: 7.3%) and the more polar product C0075 (40 mg, yield: 26.7%).

C0074: MS *m*/*z* (ESI): 378.1 [M+1]⁺.

¹H NMR (400 MHz, CD₃OD-*d*₄) *δ* 7.65 (d, *J =* 7.2 Hz, 2H), 7.57 (d, *J =* 8.0 Hz, 1H), 7.41 (d, *J =* 8.4 Hz, 2H), 7.22 (d, *J =* 8.0 Hz, 1H), 4.82-4.36 (m, 2H), 4.11-3.66 (m, 6H), 2.88-2.86 (m, 2H), 2.58 (s, 3H), 2.21 (s, 3H).

C0075: ESI-MS: *m*/*z*= 378.1 [M+1]⁺.

¹H NMR (400 MHz, CD₃OD-*d*₄) *δ* 7.64 (d, *J =* 7.6 Hz, 2H), 7.57 (d, *J =* 8.0 Hz, 1H), 7.42 (d, *J =* 8.0 Hz, 2H), 7.23 (d, *J =* 8.4 Hz, 1H), 4.83-4.75 (m, 1H), 4.42-4.06 (m, 2H), 3.88-3.62 (m, 4H), 3.35-3.11 (m, 1H), 3.30-2.63 (m, 2H), 2.56 (s, 3H), 2.22 (s, 3H).

### Examples C0082&C0083

### (S,E)-(7-(2,3-Dimethylphenyl)-2,3-dihydro-1H-inden-4-yl)(2-(hydroxymethyl)-4-(methoxyimino)pyrrolidin-1-yl)methanone

### (S,Z)-(7-(2,3-Dimethylphenyl)-2,3-dihydro-1H-inden-4-yl)(2-(hydroxymethyl)-4-(methoxyimino)pyrrolidin-1-yl)methanone

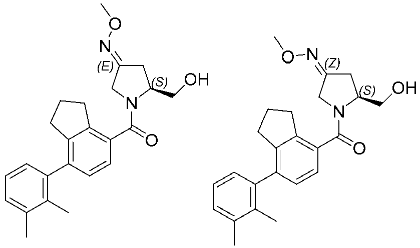

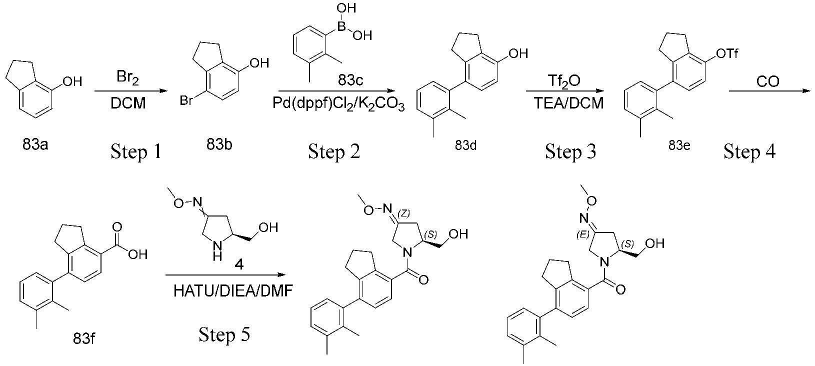

### Step 1

### 7-Bromo-2,3-dihydro-1H-inden-4-ol 83b

83a (1.0 g, 7.45 mmol) was dissolved in dichloromethane (20 mL), liquid bromine Br₂ (1.43 g, 8.94 mmol) was added dropwise in an ice bath under nitrogen protection, and the mixture was reacted at room temperature for 2 hours. After the reaction was completed, the reaction solution was poured into sodium thiosulphate solution (10%). The organic phase was collected and washed twice with saturated sodium bicarbonate solution. The organic layers were combined, washed with saturated sodium chloride aqueous solution, combined, dried over anhydrous sodium sulphate, filtered, and concentrated to afford a crude product, which was separated by column chromatography (PE:EA=10:1 to 5:1) to afford 83b (1.1 g, yield: 63.7%).

MS *m*/*z* (ESI): 212.8 [M+1]⁺.

### Step 2

### 7-(2,3-Dimethylphenyl)-2,3-dihydro-1H-inden-4-ol 83d

To a solution of 83b (1.0 g, 4.41 mmol) in dioxane/water (20/4 mL) were added 83c (860 mg, 5.73 mmol) and potassium carbonate (1.52 g, 11.03 mmol), and 1,1-bis(diphenylphosphino)ferrocene palladium(II) dichloride (323 mg, 0.44 mmol) was added at room temperature under nitrogen protection. After nitrogen purging, the mixture was reacted at 80°C for 3 hours. After the reaction was completed, the reaction solution was concentrated, and the crude product was separated by column chromatography (PE:EA=6:1 to 4:1) to afford 83d (1.2 g, crude).

MS *m*/*z* (ESI): 239.0 [M+1]⁺.

### Step 3

### 7-(2,3-Dimethylphenyl)-2,3-dihydro-1H-inden-4-yl trifluoromethanesulfonate 83e

83d (1.2 g, 4.73 mmol) was dissolved in dichloromethane (30 mL), triethylamine (1.44 g, 14.19 mmol) and trifluoromethanesulfonic anhydride (2.0 g, 7.10 mmol) were successively added at room temperature, and the mixture was reacted at room temperature for 16 hours. After the reaction was completed, the reaction solution was concentrated, and the crude product was separated by column chromatography (PE:EA=1:0 to 5:1) to afford 83e (1.1 g, yield: 60.8%).

MS *m*/*z* (ESI): 271.0 [M+1]⁺.

### Step 4

### 7-(2,3-Dimethylphenyl)-2,3-dihydro-1H-indene-4-carboxylic acid 83f

83e (700 mg, 1.85 mmol) was dissolved in acetonitrile/water (14/2 mL), and 1,3-bis(diphenylphosphino)propane (76 mg, 0.18 mmol), triethylamine (370 mg, 3.66 mmol) and palladium acetate (43 mg, 0.19 mmol) were added at room temperature. The mixture was purged three times with carbon monoxide, pressurized to 3 MPa, and reacted at 80°C for 16 hours. After the reaction was completed, the reaction solution was concentrated, and the crude product was separated by C18 reversed-phase silica gel column chromatography to afford 83f (230 mg, yield: 45.7%).

MS *m*/*z* (ESI): 267.0 [M+1]⁺.

### Step 5

### (S,Z)-(7-(2,3-Dimethylphenyl)-2,3-dihydro-1H-inden-4-yl)(2-(hydroxymethyl)-4-(methoxyimino)pyrrolidin-1-yl)methanone

### (S,E)-(7-(2,3-Dimethylphenyl)-2,3-dihydro-1H-inden-4-yl)(2-(hydroxymethyl)-4-(methoxyimino)pyrrolidin-1-yl)methanone

To a solution of 83f (110 mg, 0.41 mmol) in DMF were added 4 (80.0 mg, 0.55 mmol), HATU (170 mg, 0.45 mmol) and *N,N*-diisopropylethylamine (160 mg, 1.42 mmol), and the mixture was reacted at room temperature for 2 hours. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness, to afford a crude product. The crude product was purified by silica gel Prep-TLC (PE:EA=1:2) to afford the less polar crude product C0082 (Rf=0.4) and the more polar product C0083 (Rf=0.3). Further purification by preparative HPLC and lyophilization yielded the less polar product C0082 (10.55 mg, yield: 6.5%) and the more polar product C0083 (14.05 mg, yield: 8.6%).

C0082: MS *m*/*z* (ESI): 393.2 [M+1]⁺.

¹H NMR (400 MHz, CD₃OD-*d*₄) *δ* 7.25 (d, *J =* 7.6 Hz, 1H), 7.16-7.08 (m, 2H), 7.03 (d, *J =* 7.6 Hz, 1H), 6.93 (d, *J =* 6.4 Hz, 1H), 4.73-4.49 (m, 1H), 4.21-3.31 (m, 7H), 3.09-2.83 (m, 4H), 2.69-2.55 (m, 2H), 2.32 (s, 3H), 2.16-2.00 (m, 5H).

C0083: MS *m*/*z* (ESI): 393.2 [M+1]⁺.

¹H NMR (400 MHz, CD₃OD-*d*₄) *δ* 7.25 (d, *J =* 7.2 Hz, 1H), 7.16-7.09 (m, 2H), 7.04 (d, *J* = 7.6 Hz, 1H), 6.95 (m, 1H), 4.71-4.58 (m, 1H), 4.25-3.73 (m, 6H), 3.36-3.31 (m, 1H), 3.09-2.83 (m, 3H), 2.80-2.55 (m, 3H), 2.32 (s, 3H), 2.15-2.01 (m, 5H).

### Examples C0084&C0085

### (S, E)-(5-(2,3-Dimethylphenyl)-6-(trifluoromethyl)pyridin-2-yl)(2-(hydroxymethyl)-4-(methoxyimino)pyrrolidin-1-yl)methanone

### (S, Z)-(5-(2,3-Dimethylphenyl)-6-(trifluoromethyl)pyridin-2-yl)(2-(hydroxymethyl)-4-(methoxyimino)pyrrolidin-1-yl)methanone

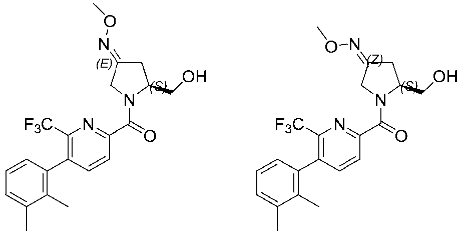

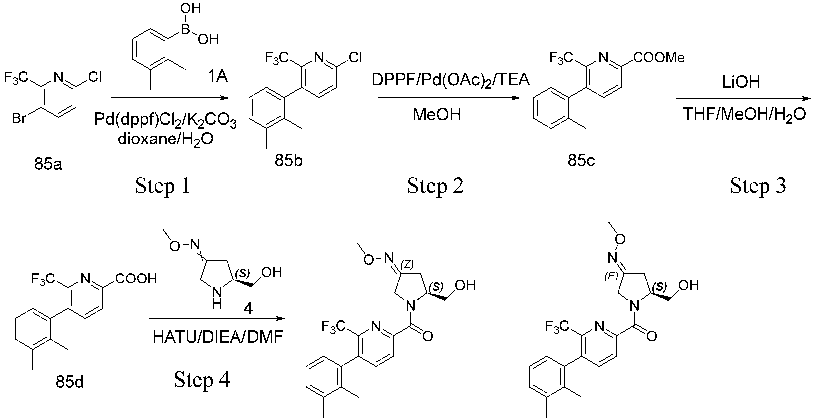

### Step 1

### 6-Chloro-3-(2,3-dimethylphenyl)-2-(trifluoromethyl)pyridine 85b

To a solution of 85a (500 mg, 1.92 mmol) in 1,4-dioxane (20 mL) and water (2 mL) were added 1A (720 mg, 4.80 mmol), potassium carbonate (663 mg, 4.80 mmol) and Pd(dppf)Cl₂ (78.40 mg, 0.096 mmol). After nitrogen purging, the mixture was reacted at 80°C for 3 hours. After the reaction was completed, water (20 mL) was added to the reaction solution, and the mixture was extracted with ethyl acetate (30 mL × 3). The organic phases were combined, washed with saturated sodium chloride aqueous solution (30 mL), dried over sodium sulphate, and subjected to evaporation to remove the solvent. The residue was purified by C18 reversed-phase column chromatography (eluting with a gradient of acetonitrile:water = 5:95 to 70:30) to afford product 85b (300 mg, yield: 51.9%).

MS *m*/*z* (ESI): 285.9 [M+1]⁺.

### Step 2

### Methyl 5-(2,3-dimethylphenyl)-6-(trifluoromethyl)picolinate 85c

To a solution of 85b (200 mg, 1.92 mmol) in methanol (15 mL) were added triethylamine (203.4 mg, 2.01 mmol), palladium acetate (30.1 mg, 0.13 mmol) and 1,1'-bis(diphenylphosphino)ferrocene (74.3 mg, 0.13 mmol), and the reaction solution was purged with a carbon monoxide balloon and reacted at 70°C for 16 hours. After the reaction was completed, the reaction solution was concentrated to remove methanol, water (20 mL) was added to the residue, and the mixture was extracted with ethyl acetate (30 mL × 3). The organic phases were combined, washed with saturated sodium chloride aqueous solution (50 mL), dried over sodium sulphate, and subjected to evaporation to remove the solvent. The residue was purified by silica gel Pre-TLC (PE/EA=4:1, Rf=0.5) to afford product 85c (150 mg, yield: 69.3%).

MS *m*/*z* (ESI): 309.9 [M+1]⁺.

According to Step 3 to Step 4 in the aforementioned synthetic route, with reference to the synthetic method of compound C0061, the less polar crude isomer C0084 (Rf=0.6) and the more polar crude isomer C0084 (Rf=0.55) were obtained. Further purification by preparative HPLC and lyophilization yielded the less polar product C0084 (38.0 mg, yield: 12.7%) and the more polar product C0085 (26.0 mg, yield: 18.6%).

C0084: MS *m*/*z* (ESI): 422.1 [M+1]⁺.

¹H NMR (DMSO-*d*₆, 400 MHz) *δ* 8.18-8.09 (m, 1H), 7.98 (d, *J =* 8.0 Hz, 1H), 7.27 (d, *J =* 7.6 Hz, 1H), 7.15 (t, *J =* 7.6 Hz, 1H), 7.00 (m, *J =* 8.0 Hz, 1H), 5.03-4.93 (m, 1H), 4.78-4.65 (m, 1H), 4.60-4.58 (m, 1H), 4.52-4.11(m, 1H), 3.81-3.80 (m, 3H), 3.64-3.39 (m, 2H), 3.30-2.66 (m, 2H), 2.30 (s, 3H), 1.91 (d, *J =* 6.0 Hz, 3H).

C0085: MS *m*/*z* (ESI): 422.1 [M+1]⁺.

¹H NMR (DMSO-*d*₆, 400 MHz) *δ* 8.16-8.11 (m, 1H), 8.00-7.97 (m, 1H), 7.28-7.26 (m, 1H), 7.19-7.16 (m, 1H), 7.03-6.90 (m, 1H), 5.03-4.93 (m, 1H), 4.80-4.77 (m, 1H), 4.63-4.12 (m, 3H), 3.83-3.75 (m, 3H), 3.62-3.45 (m, 2H), 2.99-2.59 (m, 2H), 2.30 (s, 3H), 2.31-1.89 (m, 3H).

### Examples A0203&A0217

### 2-Chloro-3-(4-[(2S, 4Z)-2-(hydroxymethyl)-4-(methoxyimino)pyrrolidine-1-carbonyl]phenyl)benzonitrile

### 2-Chloro-3-(4-[(2S, 4E)-2-(hydroxymethyl)-4-(methoxyimino)pyrrolidine-1-carbonyl]phenyl)benzonitrile

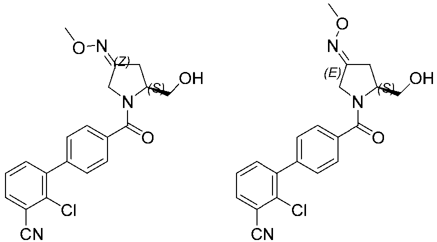

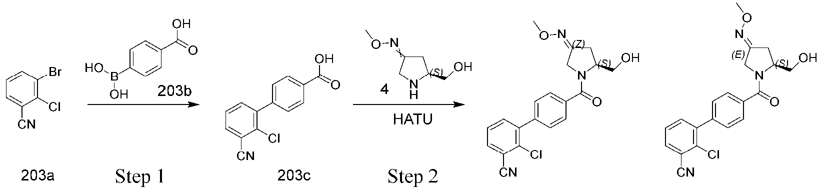

### Step 1

### 4-(2-Chloro-3-cyanophenyl)benzoic acid 203c

203a (0.3 g, 1.39 mmol), 203b (0.28 g, 1.67 mmol), Pd(dppf)Cl₂ (0.051 g, 0.069 mmol), and potassium carbonate (0.38 g, 2.78 mmol) were dissolved in 1,4-dioxane (3 mL), and the mixture was reacted at 100°C for 16 hours. The reaction solution was diluted with 10 mL of ethyl acetate, filtered, concentrated, re-dissolved in 10 mL of ethyl acetate, washed with 1N hydrochloric acid, and extracted with ethyl acetate. The organic phases were combined, dried, and concentrated to afford 203c (150 mg, yield: 42.0%).

MS *m*/*z* (ESI): 257.9[M+1]⁺.

### Step 2

### 2-Chloro-3-(4-[(2S, 4Z)-2-(hydroxymethyl)-4-(methoxyimino)pyrrolidine-1-carbonyl]phenyl)benzonitrile

### 2-Chloro-3-(4-[(2S, 4E)-2-(hydroxymethyl)-4-(methoxyimino)pyrrolidine-1-carbonyl]phenyl)benzonitrile

203c (0.10 g, 0.39 mmol), 4 (0.056 g, 0.39 mmol), and DIPEA (0.15 g, 1.17 mmol) were dissolved in DMF (2 mL), HATU (0.18 g, 0.47 mmol) was added at 20°C, and the mixture was reacted for 16 hours. The reaction solution was diluted with ethyl acetate, and washed with sodium chloride aqueous solution and 1N hydrochloric acid, successively. The organic phase was dried over anhydrous sodium sulphate and concentrated, and the residue was purified by silica gel Pre-TLC (PE:EA=1:3) to afford the less polar crude isomer A0217 (Rf=0.4) and the more polar crude isomer A0203 (Rf=0.35). Further purification by preparative

HPLC and lyophilization yielded the more polar product A0203 (5.44 mg, yield: 3.32%) and the less polar product A0217 (3.64 mg, yield: 1.99%).

A0203: MS *m*/*z* (ESI): 384.1 [M+1]⁺.

¹H NMR (400 MHz, Chloroform-d) *δ* 7.72 (d, *J =* 6.0 Hz, 1H), 7.63-7.57 (m, 3H), 7.52-7.48 (m, 3H), 4.89 (brs, 1H), 4.42-4.18 (m, 2H), 3.98-3.77 (m, 5H), 2.99-2.92 (m, 2H), 2.65 (d, *J =* 17.2 Hz, 1H).

A0217: MS *m*/*z* (ESI): 384.1 [M+1]⁺.

¹H NMR (400 MHz, Chloroform-*d*) *δ* 7.72 (d, *J =* 6.0 Hz, 1H), 7.65-7.55 (m, 3H), 7.52-7.43 (m, 3H), 4.91 (brs, 1H), 4.55-4.17 (m, 2H), 3.98-3.75 (m, 5H), 3.24-2.89 (m, 3H).

### Examples A0204&A0218

### 2-(Difluoromethyl)-3-(4-[(2S, 4Z)-2-(hydroxymethyl)-4-(methoxyimino)pyrrolidine-1-carbonyl]phenyl)benzonitrile

### 2-(Difluoromethyl)-3-(4-[(2S, 4E)-2-(hydroxymethyl)-4-(methoxyimino)pyrrolidine-1-carbonyl]phenyl)benzonitrile

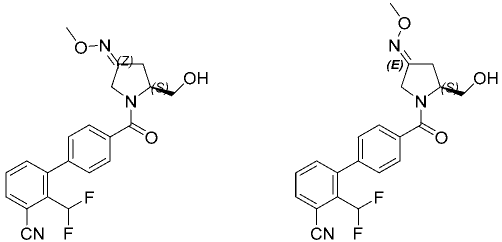

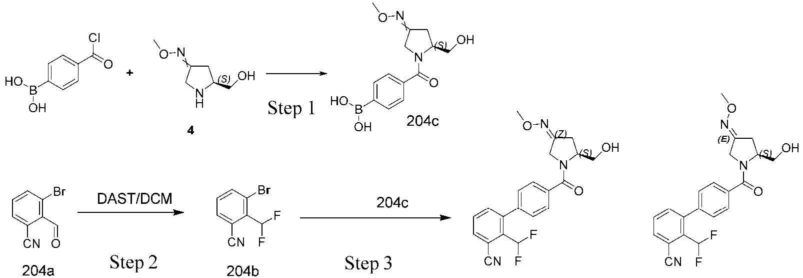

### Step 1

To a solution of 4 (2.00 g, 13.88 mmol) in dichloromethane (20 mL) were added 4-formylchlorophenylboronic acid (2.21 g, 12.00 mmol) and triethylamine (3.51 g, 34.7 mmol) at 0°C, and the mixture was reacted at room temperature for 1 hour. After the reaction was completed, the reaction solution was filtered, and the filtrate was washed with water (20 mL). The organic layer was washed with saturated sodium chloride aqueous solution, dried over anhydrous sodium sulphate, filtered, and subjected to rotary evaporation to afford a crude product. The crude product was purified by normal-phase silica gel column chromatography (PE:EA=1:3) to afford product 204c (2.81 g, yield: 80%).

### Step 2

### 3-Bromo-2-(difluoromethyl)benzonitrile 204b

204a (0.13 g, 0.62 mmol) was dissolved in DCM (1 mL), DAST (0.15 g, 0.93 mmol) was added at 15°C, and the mixture was reacted for 16 hours. 3 mL of saturated sodium bicarbonate was added to quench the reaction solution, and the reaction solution was extracted with dichloromethane. The organic phases were combined, dried, and concentrated to afford 204b (130 mg, crude).

MS *m*/*z* (ESI): 231.8[M+1]⁺.

### Step 3

### 2-(Difluoromethyl)-3-(4-[(2S, 4Z)-2-(hydroxymethyl)-4-(methoxyimino)pyrrolidine-1-carbonyl]phenyl)benzonitrile

### 2-(Difluoromethyl)-3-(4-[(2S, 4E)-2-(hydroxymethyl)-4-(methoxyimino)pyrrolidine-1-carbonyl]phenyl)benzonitrile

204b (0.065 g, 0.28 mmol), 204c (0.082 g, 0.28 mmol), Pd(dppf)Cl₂ (0.02 g, 0.028 mmol), and potassium carbonate (0.077 g, 0.56 mmol) were dissolved in 1,4-dioxane (2 mL) and water (1 mL), and the mixture was reacted at 100°C for 16 hours. The reaction solution was diluted with ethyl acetate, and washed with sodium chloride aqueous solution and 1N hydrochloric acid, successively. The organic phase was dried over anhydrous sodium sulphate and concentrated, and the residue was purified by silica gel Pre-TLC (PE:EA=1:3) to afford the less polar crude isomer A0218 (Rf=0.4) and the more polar crude isomer A0204 (Rf=0.35). Further purification by preparative HPLC and lyophilization yielded the more polar product A0204 (28.55 mg, yield: 24.8%) and the less polar product A0218 (23.95 mg, yield: 21.0%).

A0204: MS *m*/*z* (ESI): 400.3 [M+1]⁺.

¹H NMR (400 MHz, Chloroform-*d*) *δ* 7.92-7.84 (m, 1H), 7.72-7.58 (m, 4H), 7.60-7.33 (m, 2H), 6.66 (td, *J* = 53.2, 6.4 Hz, 1H), 4.88 (brs, 1H), 4.42-4.12 (m, 2H), 4.03-3.74 (m, 5H), 2.99-2.93 (m, 2H), 2.69-2.65 (m, 1H).

A0218: MS *m*/*z* (ESI): 400.3 [M+1]⁺.

¹H NMR (400 MHz, Chloroform-d) *δ* 7.87 (d, *J =* 7.2 Hz, 1H), 7.66-7.63 (m, 3H), 7.57 (d, *J =* 8.0 Hz, 1H), 7.40 (d, *J =* 7.6 Hz, 2H), 6.65 (t, *J =* 53.2 Hz, 1H), 4.90 (brs, 1H), 4.38-4.13 (m, 2H), 3.93-3.72 (m, 5H), 2.97-2.90 (m, 2H), 2.80-2.65 (m, 1H).

### Examples A0212&A0221

### 3-(5-[(2S, 4Z)-2-(Hydroxymethyl)-4-(methoxyimino)pyrrolidine-1-carbonyl]thien-2-yl)-2-methylbenzonitrile

### 3-(5-[(2S, 4E)-2-(Hydroxymethyl)-4-(methoxyimino)pyrrolidine-1-carbonyl]thien-2-yl)-2-methylbenzonitrile

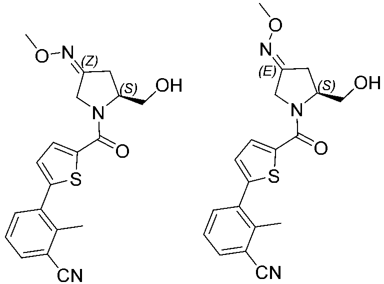

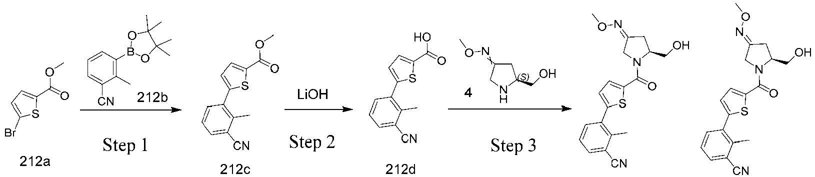

According to the aforementioned synthetic route, with reference to the synthetic method of compound A0167, the less polar crude isomer A0221 (Rf=0.6) and the more polar crude isomer A0212 (Rf=0.55) were obtained. Further purification by preparative HPLC and lyophilization yielded the more polar product A0212 (9.55 mg, yield: 6.16%) and the less polar product A0221 (9.00 mg, yield: 5.61%).

A0212: MS *m*/*z* (ESI): 370.0 [M+1]⁺.

¹H NMR (400 MHz, Chloroform-*d*) *δ* 7.66 (d, *J =* 7.6 Hz, 1H), 7.63-7.58 (m, 2H), 7.35 (t, *J =* 7.6 Hz, 1H), 7.07 (d, *J =* 3.6 Hz, 1H), 4.90 (brs, 1H), 4.68-4.53 (m, 2H), 3.92-3.84 (m, 4H), 3.80-3.75 (m, 1H), 2.98-2.89 (m, 1H), 2.82-65 (m, 2H), 2.62 (s, 3H).

A0221: MS *m*/*z* (ESI): 370.0 [M+1]⁺.

¹H NMR (400 MHz, Chloroform-d) *δ* 7.66 (d, *J =* 8.0 Hz, 1H), 7.59 (d, *J =* 7.6 Hz, 1H), 7.53 (d, *J* = 3.6 Hz, 1H), 7.35-7.32 (m, 1H), 7.05 (d, *J =* 3.6 Hz, 1H), 4.98-4.88 (m, 1H), 4.69 (d, *J =* 15.2 Hz, 1H), 4.60-4.48 (m, 1H), 3.95-3.87 (m, 4H), 3.80-3.72 (m, 1H), 2.91-2.84 (m, 1H), 2.80-2.73 (m, 1H), 2.61 (s, 3H).

### Examples C0070&C0071

### (S, E)-(5-(2,3-Dimethylphenyl)-4-(trifluoromethyl)pyridin-2-yl)(2-(hydroxymethyl)-4-(methoxyimino)pyrrolidin-1-yl)methanone

### (S, Z)-(5-(2,3-Dimethylphenyl)-4-(trifluoromethyl)pyridin-2-yl)(2-(hydroxymethyl)-4-(methoxyimino)pyrrolidin-1-yl)methanone

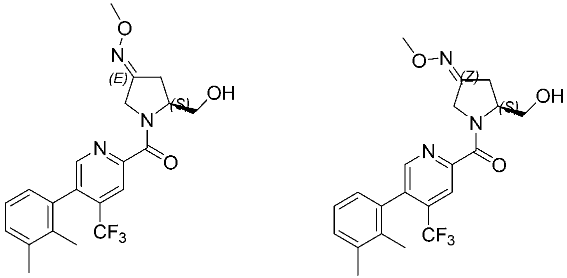

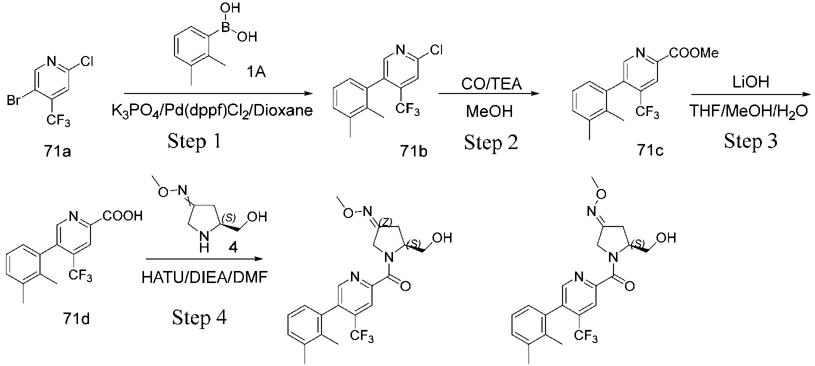

According to the aforementioned synthetic route, with reference to the synthetic method of compound C0084, the less polar crude isomer C0070 (Rf=0.6) and the more polar crude isomer C0071 (Rf=0.55) were obtained. Further purification by preparative HPLC and lyophilization yielded the less polar product C0070 (30.0 mg, yield: 21.4%) and the more polar product C0071 (19.0 mg, yield: 13.6%).

C0070: MS *m*/*z* (ESI): 422.0 [M+1]⁺.

¹H NMR (400 MHz, DMSO-*d*₆) *δ* 8.63-8.59 (m, 1H), 8.15-8.09 (m, 1H), 7.29 (d, *J =* 7.6 Hz, 1H), 7.20-7.17 (m, 1H), 7.04-7.01 (m, 1H), 5.03-4.93 (m, 1H), 4.94-4.67 (m, 1H), 4.52-4.12 (m, 2H), 3.82-3.78 (m, 3H), 3.65-3.36 (m, 2H), 2.83-2.66 (m, 2H), 2.31 (s, 3H), 1.91-1.89 (m, 3H).

C0071: MS *m*/*z* (ESI): 422.1 [M+1]⁺.

¹H NMR (DMSO-*d*₆, 400 MHz) *δ* 8.65-8.59 (m, 1H), 8.14-8.09 (m, 1H), 7.28 (d, *J* = 7.6 Hz, 1H), 7.20-7.17 (m, 1H), 7.05-7.01 (m, 1H), 4.99-4.87 (m, 1H), 4.80-4.64 (m, 1H), 4.54-4.13 (m, 2H), 3.82-3.75 (m, 3H), 3.01-2.67 (m, 2H), 2.63-2.59 (m, 1H), 2.31 (s, 3H), 1.91-1.89 (m, 3H).

### Examples C0076&C0077

### (S, E)-3-(2,3-Dimethylphenyl)-6-(2-(hydroxymethyl)-4-(methoxyimino)pyrrolidine-1-carbonyl)pyrimidin-4(3H)-one

### (S, Z)-3-(2,3-Dimethylphenyl)-6-(2-(hydroxymethyl)-4-(methoxyimino)pyrrolidine-1-carbonyl)pyrimidin-4(3H)-one

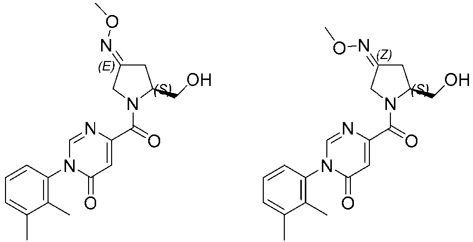

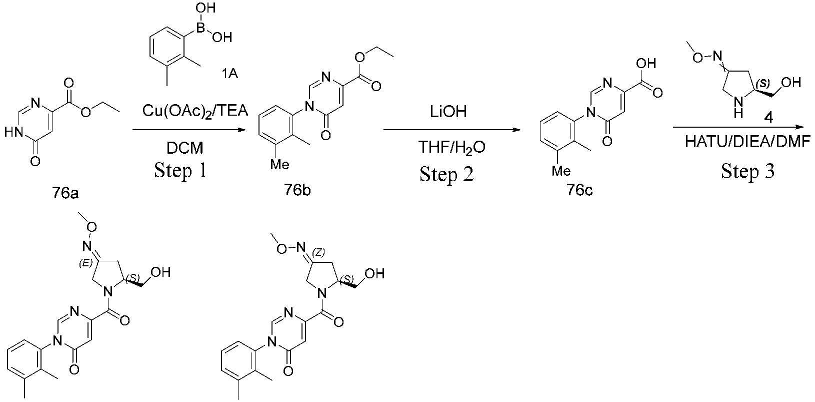

### Step 1

### Ethyl 1-(2,3-dimethylphenyl)-6-oxo-1,6-dihydropyrimidine-4-carboxylate 76b

76a (500 mg, 2.97 mmol) was dissolved in dichloromethane (30 mL), and 1A (535 mg, 3.56 mmol), copper acetate monohydrate (1.19 g, 5.94 mmol), pyridine (1.17 g, 14.85 mmol), triethylamine (0.60 g, 5.94 mmol), zeolite (1 g) and 1,10-phenanthroline (0.54 g, 2.97 mmol) were added. After nitrogen purging, the mixture was reacted at room temperature for 17 hours. After the reaction was completed, the reaction solution was filtered to remove the insoluble solid, and the filtrate was subjected to rotary evaporation to remove the solvent. Water (20 mL) was added, and the mixture was extracted with ethyl acetate (30 mL × 3). The organic phases were combined, washed with saturated sodium chloride aqueous solution (30 mL), dried over sodium sulphate, and subjected to evaporation to remove the solvent. The residue was purified by C18 reversed-phase column chromatography (eluting with a gradient of acetonitrile:water = 5:95 to 70:30) to afford product 76b (60 mg, yield: 7.4%).

MS *m*/*z* (ESI): 273.0 [M+1]⁺.

According to Step 2 to Step 3 in the aforementioned synthetic route, with reference to the synthetic method of compound C0061, the less polar crude isomer C0076 (Rf=0.6) and the more polar crude isomer C0077 (Rf=0.55) were obtained. Further purification by preparative HPLC and lyophilization yielded the less polar product C0076 (1.9 mg, yield: 2.5%) and the more polar product C0077 (4.9 mg, yield: 6.5%).

C0076: MS *m*/*z* (ESI): 371.1 [M+1]⁺.

¹H NMR (400 MHz, CD₃OD-*d*₄) *δ* 8.38 (d, *J =* 12.0 Hz, 1H), 7.36 (d, *J* = 7.6 Hz, 1H), 7.31-7.29 (m, 1H), 7.17-7.15 (m, 1H), 6.91 (s, 1H), 4.70-4.50 (m, 3H), 3.87-3.85 (m, 3H), 3.66-3.48 (m, 2H), 2.86-2.77 (m, 2H), 2.38 (s, 3H), 2.04 (s, 3H).

C0077: MS *m*/*z* (ESI): 371.1 [M+1]⁺.

¹H NMR (400 MHz, CD₃OD-*d*₄) *δ* 8.38 (d, *J =* 12.8 Hz, 1H), 7.35 (m, 1H), 7.31-7.29 (m, 1H), 7.17-7.15 (m, 1H), 6.91 (s, 1H), 4.68-4.54 (m, 3H), 3.88-3.82 (m, 3H), 3.69-3.60 (m, 2H), 3.03-2.63 (m, 2H), 2.38 (s, 3H), 2.04 (s, 3H).

### Examples C0078&C0079

### (S, E)-(3'-(Difluoromethyl)-2'-methyl-[1,1'-biphenyl]-4-yl)(2-(hydroxymethyl)-4-(methoxyimino)pyrrolidin-1-yl)methanone

### (S, Z)-(3'-(Difluoromethyl)-2'-methyl-[1,1'-biphenyl]-4-yl)(2-(hydroxymethyl)-4-(methoxyimino)pyrrolidin-1-yl)methanone

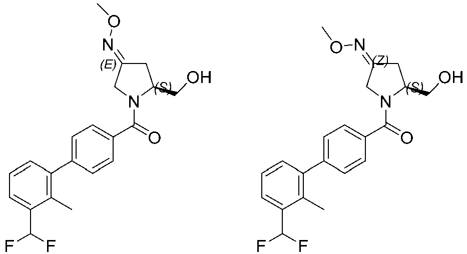

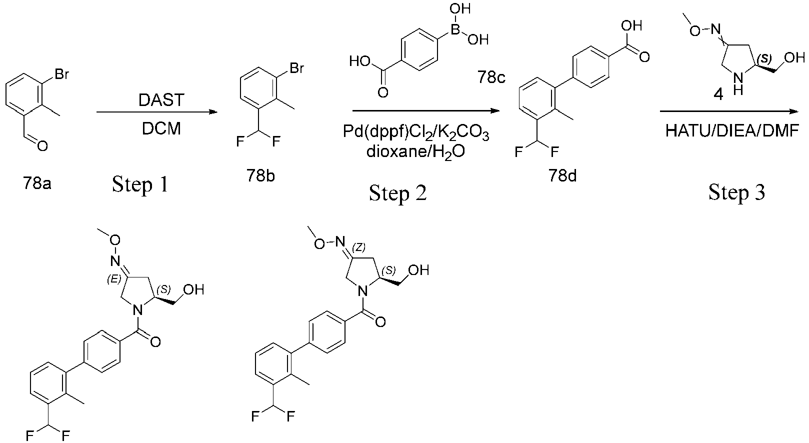

### Step 1

### 1-Bromo-3-(difluoromethyl)-2-methylbenzene 78b

78a (1 g, 5.02 mmol) was dissolved in dichloromethane (20 mL), diethylaminosulphur trifluoride (0.95 g, 5.89 mmol) was added dropwise to the reaction solution at 0°C under nitrogen protection, and the mixture was reacted at room temperature for 16 hours. After the reaction was completed, water (20 mL) was added to the reaction solution, and the mixture was extracted with dichloromethane (30 mL × 3). The organic phases were combined, washed with saturated sodium chloride aqueous solution (30 mL), dried over sodium sulphate, and subjected to evaporation to remove the solvent. After rotary evaporation to dryness, product 78b (500 mg, yield: 44.1%) was obtained.

According to Step 2 to Step 3 in the aforementioned synthetic route, with reference to the synthetic method of compound C0065, the less polar crude isomer C0078 (Rf=0.6) and the more polar crude isomer C0079 (Rf=0.5) were obtained. Further purification by preparative HPLC and lyophilization yielded the less polar product C0078 (29.0 mg, yield: 14.5%) and the more polar product C0079 (57.6 mg, yield: 28.8%).

C0078: MS *m*/*z* (ESI): 389.1 [M+1]⁺.

¹H NMR (400 MHz, CD₃OD-*d*₄) *δ* 7.65-7.55 (m, 3H), 7.42-7.36 (m, 4H), 7.12-6.84 (t, *J =* 54.8 Hz, 1H), 4.76-4.08 (m, 3H), 3.84 (s, 3H), 3.69-3.35 (m, 2H), 2.88-2.87 (m, 2H), 2.29 (s, 3H).

C0079: MS *m*/*z* (ESI): 389.1 [M+1]⁺.

¹H NMR (400 MHz, CD₃OD-*d*₄) *δ* 7.65-7.55 (m, 3H), 7.43-7.37 (m, 4H), 7.12-6.85 (t, *J =* 54.8 Hz, 1H), 4.77-4.09 (m, 3H), 3.88-3.80 (m, 3H), 3.73-3.37 (m, 2H), 2.98-2.76 (m, 2H), 2.30 (s, 3H).

### Examples C0086&C0087

### (S, E)-(5-(2,3-Dimethylphenyl)-4-(trifluoromethyl)pyrimidin-2-yl)(2-(hydroxymethyl)-4-(methoxyimino)pyrrolidin-1-yl)methanone

### (S, Z)-(5-(2,3-Dimethylphenyl)-4-(trifluoromethyl)pyrimidin-2-yl)(2-(hydroxymethyl)-4-(methoxyimino)pyrrolidin-1-yl)methanone

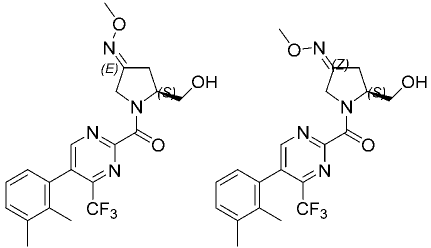

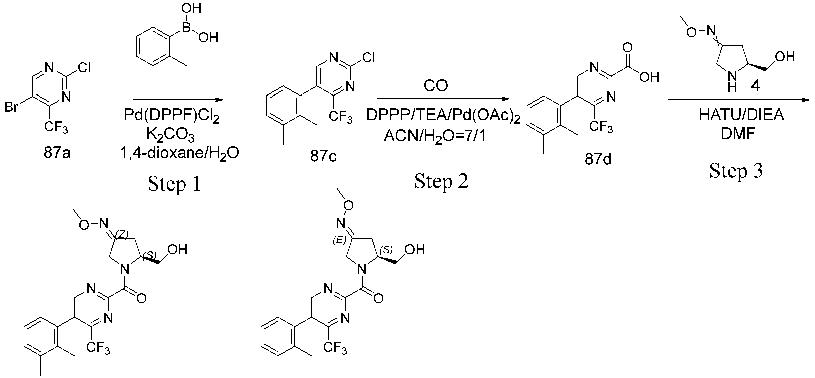

### Step 1

### 2-Chloro-5-(2,3-dimethylphenyl)-4-(trifluoromethyl)pyrimidine 87c

To a solution of 87a (500 mg, 1.91 mmol) in 1,4-dioxane (10 mL) were added (2,3-dimethylphenyl)boronic acid (290 mg, 1.93 mmol), potassium carbonate (580 mg, 4.19 mmol) and Pd(dppf)Cl₂ (140 mg, 0.19 mmol). After nitrogen purging, the mixture was reacted at 80°C for 2 hours. After the reaction was completed, water (20 mL) was added to the reaction solution, and the mixture was extracted with ethyl acetate (30 mL × 3). The organic phases were combined, washed with saturated sodium chloride aqueous solution (30 mL), dried over sodium sulphate, and subjected to evaporation to remove the solvent. The residue was purified by normal-phase silica gel column chromatography (eluting with a gradient of petroleum ether:ethyl acetate = 5:1 to 3:1) to afford product 87c (320 mg, yield: 57.2%).

MS *m*/*z* (ESI): 286.9 [M+1]⁺.

### Step 2

### 5-(2,3-Dimethylphenyl)-4-(trifluoromethyl)pyrimidine-2-carboxylic acid 87d

To a solution of 87c (260 mg, 0.89 mmol) in acetonitrile (7 mL) and water (1 mL) were added 1,3-bis(diphenylphosphino)propane (37 mg, 0.09 mmol), triethylamine (185 mg, 1.83 mmol) and palladium acetate (20 mg, 0.089 mmol) at room temperature. The mixture was purged three times with carbon monoxide, pressurized to 4 MPa, and reacted at 80°C for 4 hours. After the reaction was completed, the reaction solution was concentrated, and the crude product was separated by C18 reversed-phase silica gel column chromatography (eluting with a gradient of acetonitrile: water = 30:70 to 75:25) to afford 87d (170 mg, yield: 63.3%).

MS *m*/*z* (ESI): 296.9 [M+1]⁺.

### Step 3

### (S, Z)-(5-(2,3-Dimethylphenyl)-4-(trifluoromethyl)pyrimidin-2-yl)(2-(hydroxymethyl)-4-(methoxyimino)pyrrolidin-1-yl)methanone

### (S, E)-(5-(2,3-Dimethylphenyl)-4-(trifluoromethyl)pyrimidin-2-yl)(2-(hydroxymethyl)-4-(methoxyimino)pyrrolidin-1-yl)methanone

To a solution of 87d (170 mg, 0.52 mmol) in DMF (5 mL) were added 4 (120 mg, 0.75 mmol), HATU (220 mg, 0.58 mmol) and *N,N*-diisopropylethylamine (200 mg, 1.55 mmol) at room temperature, and the mixture was reacted at room temperature for 2 hours. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness. The residue was purified by silica gel Pre-TLC (PE:EA=2:5) to afford the less polar crude isomer C0086 (Rf=0.6) and the more polar crude isomer C0087 (Rf=0.55). Further purification by preparative HPLC and lyophilization yielded the less polar product C0086 (16.89 mg, yield: 7.73%) and the more polar product C0087 (20.29 mg, yield: 9.29%).

C0086: MS *m*/*z* (ESI): 423.1 [M+1]⁺.

¹H NMR (400 MHz, CD₃OD-*d*₄) *δ* 8.98-8.95 (m, 1H), 7.31 (d, *J =* 8.0 Hz, 1H), 7.20 (t, *J* = 7.6 Hz, 1H), 7.04 (d, *J =* 7.2 Hz, 1H), 4.81-4.26 (m, 3H), 3.92-3.73 (m, 4H), 3.69-3.51 (m, 1H), 2.98-2.89 (m, 2H), 2.36 (s, 3H), 2.00 (s, 3H).

C0087: MS *m*/*z* (ESI): 423.1 [M+1]⁺.

¹H NMR (400 MHz, CD₃OD-*d*₄) *δ* 8.97 (d, *J* = 9.2 Hz, 1H), 7.31 (d, *J* = 7.6 Hz, 1H), 7.20 (t, *J =* 7.6 Hz, 1H), 7.06-7.03 (m, 1H), 4.83-4.30 (m, 3H), 3.89-3.73 (m, 4H), 3.64-3.53 (m, 1H), 3.03-2.94 (m, 1H), 2.82-2.64 (m, 1H), 2.36 (s, 3H), 2.01 (s, 3H).

### Examples C0090&C0091

### (S, E)-4-(4-(2-(Hydroxymethyl)-4-(methoxyimino)pyrrolidine-1-carbonyl)phenyl)-3-methylpicolinonitrile

### (S, Z)-4-(4-(2-(Hydroxymethyl)-4-(methoxyimino)pyrrolidine-1-carbonyl)phenyl)-3-methylpicolinonitrile

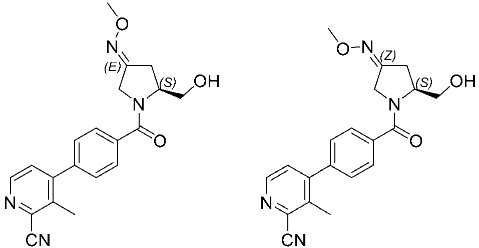

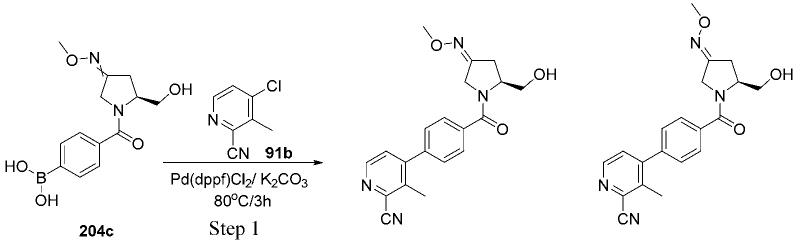

### Step 1

### (S, Z)-4-(4-(2-(Hydroxymethyl)-4-(methoxyimino)pyrrolidine-1-carbonyl)phenyl)-3-methylpicolinonitrile

### (S, E)-4-(4-(2-(Hydroxymethyl)-4-(methoxyimino)pyrrolidine-1-carbonyl)phenyl)-3-methylpicolinonitrile

204c (150 mg, 0.50 mmol), 91b (88.5 mg, 0.58 mmol) and potassium carbonate (175 mg, 1.27 mmol) were dissolved in 1,4-dioxane (5 mL) and water (1 mL), Pd(dppf)Cl₂ (38 mg, 0.052 mmol) was added at room temperature under nitrogen protection, and the mixture was reacted at 80°C for 3 hours. After the reaction was completed, water (10 mL) was added, and the mixture was extracted with ethyl acetate (20 mL × 3). The organic phases were combined, washed with saturated sodium chloride aqueous solution (10 mL × 3), dried over sodium sulphate, and filtered, and the filtrate was collected and concentrated. The residue was purified by silica gel Pre-TLC (DCM:MeOH=10:1) to afford the less polar isomer C0090 (Rf=0.65, 17.5 mg, yield: 8.55%) and the more polar crude isomer C0091 (Rf=0.60). The more polar crude isomer was further purified by preparative HPLC and lyophilized to afford C0091 (23.68 mg, yield: 12.8%).

C0090: MS *m*/*z* (ESI): 365.1 [M+1]⁺.

¹H NMR (400 MHz, CD₃OD-*d*₄) *δ* 8.58 (*d, J =* 4.8 Hz, 1H), 7.70 (*d, J =* 7.6 Hz, 2H), 7.55-7.51 (m, 3H), 4.76-4.06 (m, 3H), 3.95-3.76 (m, 4H), 3.73-3.36 (m, 1H), 2.90-2.81 (m, 2H), 2.50 (s, 3H).

C0091: MS *m*/*z* (ESI): 365.1 [M+1]⁺.

¹H NMR (400 MHz, CD₃OD-*d*₄) *δ* 8.58 (d, *J =* 4.8 Hz, 1H), 7.70 *(d, J=* 7.6 Hz, 2H), 7.56-7.52 (m, 3H), 4.76-4.06 (m, 3H), 3.90-3.37 (m, 5H), 3.05-2.90 (m, 1H), 2.77-2.55 (m, 1H), 2.50 (s, 3H).

### Examples C0092&C0093

### (S, E)-4-(4-(2-(Hydroxymethyl)-4-(methoxyimino)pyrrolidine-1-carbonyl)phenyl)-3-methylpicolinonitrile

### (S, Z)-4-(4-(2-(Hydroxymethyl)-4-(methoxyimino)pyrrolidine-1-carbonyl)phenyl)-3-methylpicolinonitrile

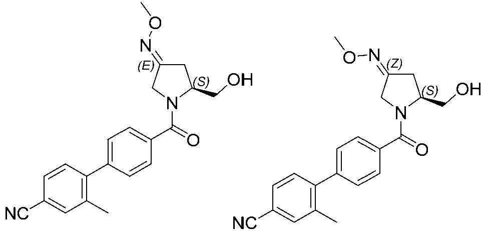

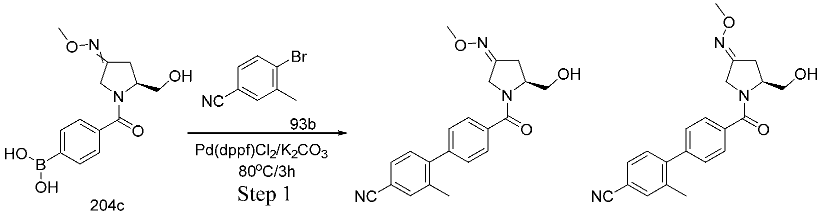

### Step 1

### (S, Z)-4-(4-(2-(Hydroxymethyl)-4-(methoxyimino)pyrrolidine-1-carbonyl)phenyl)-3-methylpicolinonitrile

### (S, E)-4-(4-(2-(Hydroxymethyl)-4-(methoxyimino)pyrrolidine-1-carbonyl)phenyl)-3-methylpicolinonitrile

204c (150 mg, 0.51 mmol), 93b (100 mg, 0.51 mmol) and potassium carbonate (175 mg, 1.27 mmol) were dissolved in 1,4-dioxane (5 mL) and water (1 mL), Pd(dppf)Cl₂ (38 mg, 0.052 mmol) was added at room temperature under nitrogen protection, and the mixture was reacted at 80°C for 3 hours. After the reaction was completed, water (10 mL) was added, and the mixture was extracted with ethyl acetate (20 mL × 3). The organic phases were combined, washed with saturated sodium chloride aqueous solution (20 mL × 3), dried over sodium sulphate, and filtered, and the filtrate was collected and concentrated. The residue was purified by silica gel Pre-TLC (EA:PE=3:1) to afford the less polar isomer C0092 (Rf=0.60, 26.23 mg, yield: 13.6%) and the more polar crude isomer C0093 (Rf=0.55). The more polar crude isomer was further purified by preparative HPLC and lyophilized to afford C0093 (36.68 mg, yield: 19.6%).

C0092: MS *m*/*z* (ESI): 364.1 [M+1]⁺.

¹H NMR (400 MHz, CD₃OD-*d*₄) *δ* 7.68-7.61 (m, 4H), 7.47-7.41 (m, 3H), 4.76-4.04 (m, 3H), 3.93-3.36 (m, 5H), 2.92-2.81 (m, 2H), 2.31 (s, 3H).

C0093: MS *m*/*z* (ESI): 364.1 [M+1]+.

¹H NMR (400 MHz, CD₃OD-*d*₄) *δ* 7.6-7.61 (m, 4H), 7.46 (d, *J =* 8.0 Hz, 2H), 7.41 (d, *J* = 7.6 Hz, 1H), 4.75-4.08 (m, 3H), 3.88-3.38 (m, 5H), 2.96-2.90 (m, 1H), 2.77-2.63 (m, 1H), 2.31 (s, 3H).

### Examples C0094&C0095

### (S, E)- 4'-(2-(Hydroxymethyl)-4-(methoxyimino)pyrrolidine-1-carbonyl)-6-methyl-[1,1'-biphenyl]-3-carbonitrile

### (S, Z)- 4'-(2-(Hydroxymethyl)-4-(methoxyimino)pyrrolidine-1-carbonyl)-6-methyl-[1,1'-biphenyl]-3-carbonitrile

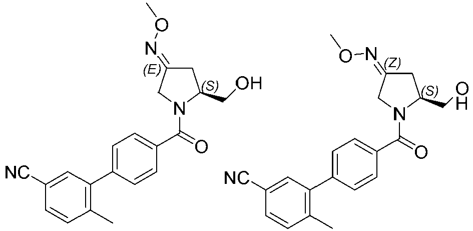

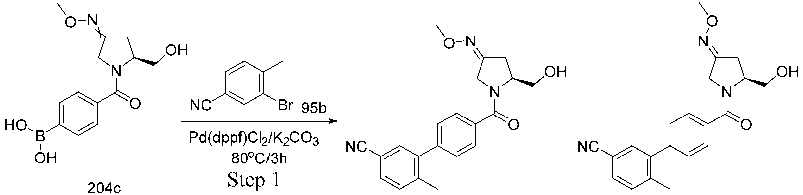

### Step 1

### (S, Z)- 4'-(2-(Hydroxymethyl)-4-(methoxyimino)pyrrolidine-1-carbonyl)-6-methyl-[1,1'-biphenyl]-3-carbonitrile

### (S, E)- 4'-(2-(Hydroxymethyl)-4-(methoxyimino)pyrrolidine-1-carbonyl)-6-methyl-[1,1'-biphenyl]-3-carbonitrile

204c (150 mg, 0.51 mmol), 95b (100 mg, 0.51 mmol) and potassium carbonate (175 mg, 1.27 mmol) were dissolved in 1,4-dioxane (5 mL) and water (1 mL), Pd(dppf)Cl₂ (38 mg, 0.052 mmol) was added at room temperature under nitrogen protection, and the mixture was reacted at 80°C for 3 hours. After the reaction was completed, water (10 mL) was added, and the mixture was extracted with ethyl acetate (20 mL × 3). The organic phases were combined, washed with saturated sodium chloride aqueous solution (20 mL × 3), dried over sodium sulphate, and filtered, and the filtrate was collected and concentrated. The residue was purified by silica gel Pre-TLC (EA:PE=3:1) to afford the less polar isomer C0094 (Rf=0.60, 30.22 mg, yield: 16.07%) and the more polar crude isomer C0095 (Rf=0.55). The more polar crude isomer was further purified by preparative HPLC and lyophilized to afford C0095 (46.49 mg, yield: 26.82%).

C0094: MS *m*/*z* (ESI): 364.1 [M+1]⁺.

¹H NMR (400 MHz, CD₃OD-*d*₄) *δ* 7.67-7.59 (m, 4H), 7.51-7.44 (m, 3H), 4.77-4.07 (m, 3H), 3.94-3.38 (m, 5H), 2.91-2.82 (m, 2H), 2.33 (s, 3H).

C0095: MS *m*/*z* (ESI): 364.1 [M+1]⁺.

¹H NMR (400 MHz, CD₃OD-*d*₄) *δ* 7.63-7.60 (m, 4H), 7.51-7.44 (m, 3H), 4.76-4.08 (m, 3H), 3.88-3.38 (m, 5H), 3.03-2.90 (m, 1H), 2.77-2.63 (m, 1H), 2.34 (s, 3H).

### Examples C0096&C0097

### (S, E)-(5-(2,3-Dimethylphenyl)thiazol-2-yl)(2-(hydroxymethyl)-4-(methoxyimino)pyrrolidin-1-yl)methanone

### (S, Z)-(5-(2,3-Dimethylphenyl)thiazol-2-yl)(2-(hydroxymethyl)-4-(methoxyimino)pyrrolidin-1-yl)methanone

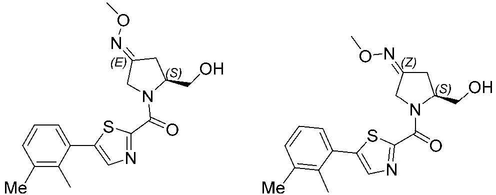

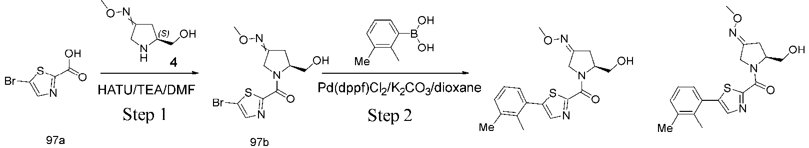

### Step 1

### (S, EZ)-(5-Bromothiazol-2-yl)(2-(hydroxymethyl)-4-(methoxyimino)pyrrolidin-1-yl)methanone 97b

To a solution of 97a (450 mg, 2.16 mmol) in DMF (9 mL) were added 4 (373.69 mg, 2.59 mmol), HATU (0.99 g, 2.59 mmol) and TEA (0.66 g, 6.48 mmol). After nitrogen purging, the mixture was reacted at room temperature for 30 minutes. The reaction solution was extracted with ethyl acetate/water. The organic layers were combined, washed with saturated sodium chloride solution, combined, dried over anhydrous sodium sulphate, filtered, and subjected to rotary evaporation to afford a crude product. The crude product was purified by reversed-phase column chromatography (H₂O:ACN=95:5 to 50:50), to afford product 97b (450 mg, yield: 56.0%).

MS *m*/*z* (ESI): 333.8, 335.8 [M+1]⁺.

### Step 2

### (S, E)-(5-(2,3-Dimethylphenyl)thiazol-2-yl)(2-(hydroxymethyl)-4-(methoxyimino)pyrrolidin-1-yl)methanone

### (S, Z)-(5-(2,3-Dimethylphenyl)thiazol-2-yl)(2-(hydroxymethyl)-4-(methoxyimino)pyrrolidin-1-yl)methanone

To a solution of 97b (200 mg, 0.60 mmol) in 1,4-dioxane (10 mL) and water (1 mL) were added (2,3-dimethylphenyl)boronic acid (0.11 g, 0.72 mmol), potassium carbonate (0.25 g, 1.80 mmol) and Pd(dppf)Cl₂ (0.049 g, 0.060 mmol). After nitrogen purging, the reaction solution was reacted at 95°C for 1 hour. The reaction solution was extracted with ethyl acetate/water. The organic layers were combined, washed with saturated sodium chloride solution, combined, dried over anhydrous sodium sulphate, filtered, and subjected to rotary evaporation to afford a crude product. The crude product was purified by silica gel Pre-TLC (PE:EA=1:5) to afford the less polar isomer C0096 (Rf=0.6, 21.0 mg, yield: 9.6%) and the more polar isomer C0097 (Rf=0.5, 55.0 mg, yield: 25.1%).

C0096: MS *m*/*z* (ESI): 360.1 [M+1]⁺.

¹H NMR (400 MHz, CD₃OD-*d*₄) *δ* 7.85 (d, *J =* 10.4 Hz, 1H), 7.26-7.13 (m, 3H), 5.66-5.15 (m, 1H), 4.73-4.69 (m, 1H), 4.59-4.19 (m, 1H), 3.90-3.64 (m, 5H), 3.05-2.82 (m, 2H), 2.35 (s, 3H), 2.28 (s, 3H).

C0097: MS *m*/*z* (ESI): 360.1 [M+1]⁺.

¹H NMR (400 MHz, CD₃OD-*d*₄) *δ* 7.85 (d*, J =* 10.4 Hz, 1H), 7.26-7.13 (m, 3H), 5.66-5.09 (m, 1H), 4.180-4.69 (m, 1H), 4.59-4.26 (m, 1H), 3.90-3.64 (m, 5H), 3.05-2.73 (m, 2H), 2.35 (s, 3H), 2.28 (s, 3H).

### Examples C0098&C0099

### 3-(7-[(2S, 4E)-2-(Hydroxymethyl)-4-(methoxyimino)pyrrolidine-1-carbonyl]-2H-1,3-benzodioxolan-4-yl)-2-methylbenzonitrile

### 3-(7-[(2S, 4Z)-2-(Hydroxymethyl)-4-(methoxyimino)pyrrolidine-1-carbonyl]-2H-1,3-benzodioxolan-4-yl)-2-methylbenzonitrile

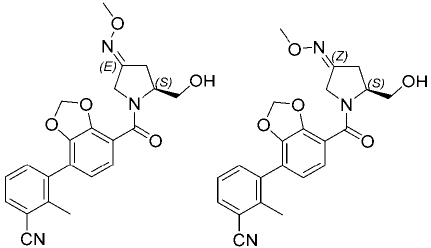

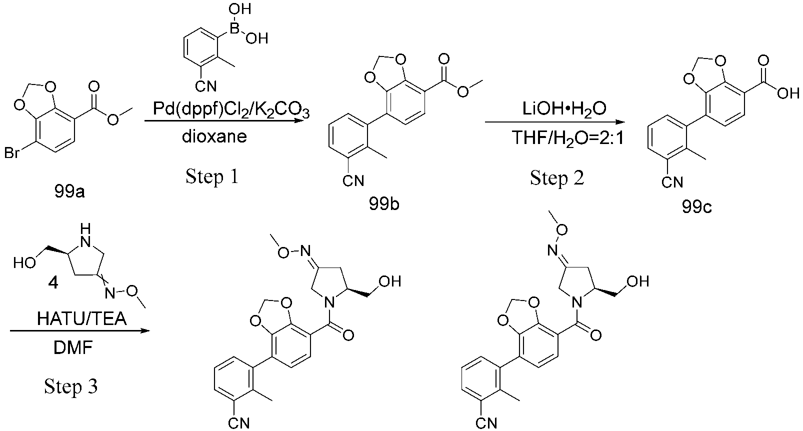

### Step 1

### Methyl 7-(3-cyano-2-methylphenyl)benzo[d][1,3]dioxolane-4-carboxylate 99b

To a solution of 99a (200 mg, 0.77 mmol) in dioxane (5 mL) and water (0.5 mL) were added (3-cyano-2-methylphenyl)boronic acid (140 mg, 0.87 mmol), potassium carbonate (210 mg, 1.52 mmol) and Pd(dppf)Cl₂ (63 mg, 0.077 mmol). After nitrogen purging, the mixture was reacted at 90°C for 3 hours. The reaction solution was filtered, subjected to rotary evaporation to dryness, and purified by silica gel Prep-TLC (PE:EA=5:1) to afford product 99b (150 mg, yield: 65.8%).

MS *m*/*z* (ESI): 296.1 [M+1]⁺.

### Step 2

### 7-(3-Cyano-2-methylphenyl)benzo[d][1,3]dioxolane-4-carboxylic acid 99c

To a solution of 99b (160 mg, 0.54 mmol) in THF (8 mL) and water (4 mL) was added LiOH (90.63 mg, 2.16 mmol), and the mixture was stirred at room temperature overnight. The reaction solution was filtered with a Buchner funnel, and the filtrate was extracted with EA after methanol was removed. The aqueous phase was collected, adjusted to pH 1-3 with hydrochloric acid, and extracted with EA. The organic layers were combined, washed with saturated sodium chloride aqueous solution, combined, dried over anhydrous sodium sulphate, filtered, and subjected to rotary evaporation to afford product 99c (100 mg, yield: 65.6%).

### Step 3

### 3-(7-[(2S, 4Z)-2-(Hydroxymethyl)-4-(methoxyimino)pyrrolidine-1-carbonyl]-2H-1,3-benzodioxolan-4-yl)-2-methylbenzonitrile

### 3-(7-[(2S, 4E)-2-(Hydroxymethyl)-4-(methoxyimino)pyrrolidine-1-carbonyl]-2H-1,3-benzodioxolan-4-yl)-2-methylbenzonitrile

To a solution of 4 (74 mg, 0.52 mmol) in DMF (3 mL) were added 99c (120 mg, 0.69 mmol), triethylamine (270 mg, 2.07 mmol) and HATU (390 mg, 0.43 mmol). After nitrogen purging, the reaction solution was reacted at room temperature for 2 hours. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness, to afford a crude product. The crude product was separated by silica gel Pre-TLC (EA:PE=5:1) to afford the less polar isomer C0098 (Rf=0.6) and the more polar crude isomer C0099 (Rf=0.5). The more polar crude isomer was purified by preparative HPLC and lyophilized to afford the more polar product C0099 (14.0 mg, yield: 7.97%).

C0098: MS *m*/*z* (ESI): 408.1 [M+1]⁺.

¹H NMR (400 MHz, CD₃OD-*d*₄) *δ* 7.72 (d, *J =* 7.2 Hz, 1H), 7.58 (d, *J =* 7.6 Hz, 1H), 7.43 (t, *J* = 8.0 Hz, 1H), 7.06 (d, *J =* 8.4 Hz, 1H), 6.88 (d, *J* = 8.0 Hz, 1H), 6.16-6.06 (m, 2H), 4.72-4.13 (m, 3H), 3.87-3.68 (m, 4H), 3.39-3.38 (m, 1H), 2.90-2.81 (m, 2H), 2.45 (s, 3H).

C0099: MS *m*/*z* (ESI): 408.1 [M+1]⁺.

¹H NMR (400 MHz, CD₃OD-*d*₄) *δ* 7.72 (d, *J =* 8.0 Hz, 1H), 7.58 (dd, *J =* 7.6, 2.8 Hz, 1H), 7.43 (t, *J =* 7.6 Hz, 1H), 7.07 (d, *J =* 8.4 Hz, 1H), 6.88 (d, *J =* 8.0 Hz, 1H), 6.10 (dd, *J =* 19.6, 5.2 Hz, 2H), 4.72-4.15 (m, 3H), 3.88-3.69 (m, 4H), 3.39-3.38 (m, 1H), 3.03-2.90 (m, 1H), 2.79-2.65 (m, 1H), 2.46 (s, 3H).

### Examples C0102&C0103

### (S,E)-3-(2,3-Dimethylphenyl)-6-(2-(hydroxymethyl)-4-(methoxyimino)pyrrolidine-1-carbonyl)-1-methylpyridin-2(1H)-one

### (S,Z)-3-(2,3-Dimethylphenyl)-6-(2-(hydroxymethyl)-4-(methoxyimino)pyrrolidine-1-carbonyl)-1-methylpyridin-2(1H)-one

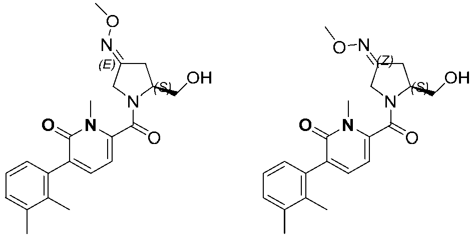

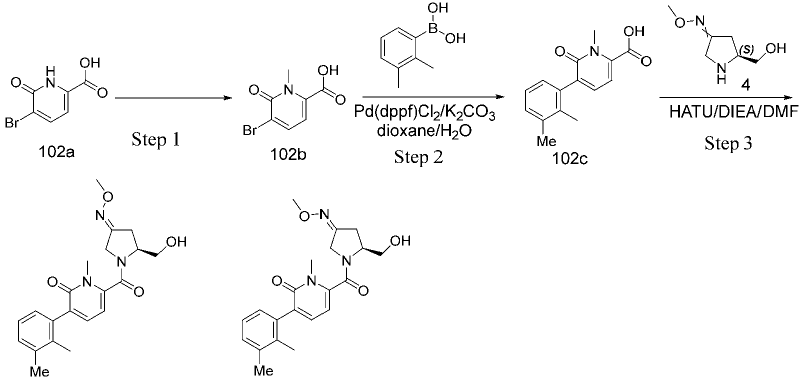

### Step 1

### 5-Bromo-1-methyl-6-oxo-1,6-dihydropyridine-2-carboxylic acid 102b

102a (50 mg, 0.23 mmol) was dissolved in a mixed solution of water (7 mL) and methanol (1 mL), iodomethane (326 mg, 2.30 mmol) and potassium hydroxide (43 mg, 0.76 mmol) were added, and the mixture was reacted at 110°C under microwave for 4 hours. After the reaction was completed, the reaction solution was concentrated to remove methanol, and extracted with ethyl acetate (20 mL × 3). The organic phases were combined, washed with saturated sodium chloride aqueous solution (30 mL), dried over sodium sulphate, and subjected to evaporation to remove the solvent. After rotary evaporation to dryness, product 102b (33 mg, yield: 62.0%) was obtained.

### Step 2

### 5-(2,3-Dimethylphenyl)-1-methyl-6-oxo-1,6-dihydropyridine-2-carboxylic acid 102c

To a mixed solution of 102b (700 mg, 3.02 mmol) in 1,4-dioxane (30 mL) and water (3 mL) were added (2,3-dimethylphenyl)boronic acid (453 mg, 3.02 mmol), potassium carbonate (1252 mg, 9.06 mmol) and Pd(dppf)Cl₂ (221 mg, 0.30 mmol). After nitrogen purging, the mixture was reacted at 105°C for 16 hours. After the reaction was completed, water (20 mL) was added to the reaction solution, and the mixture was adjusted to pH 3-4 with dilute hydrochloric acid and extracted with ethyl acetate (30 mL × 3). The organic phases were combined, dried over anhydrous sodium sulphate, and subjected to evaporation to remove the solvent. After rotary evaporation to dryness, product 102c (720 mg, yield: 92.8%) was obtained.

MS *m*/*z* (ESI): 258.0 [M+1]⁺.

### Step 3

### (S,E)-3-(2,3-Dimethylphenyl)-6-(2-(hydroxymethyl)-4-(methoxyimino)pyrrolidine-1-carbonyl)-1-methylpyridin-2(1H)-one

### (S,Z)-3-(2,3-Dimethylphenyl)-6-(2-(hydroxymethyl)-4-(methoxyimino)pyrrolidine-1-carbonyl)-1-methylpyridin-2(1H)-one

To a solution of 102c (700 mg, 2.72 mmol) in DMF were added 4 (392 mg, 2.72 mmol), HATU (1551 mg, 4.08 mmol) and *N,N*-diisopropylethylamine (879 mg, 6.80 mmol), and the reaction solution was reacted at room temperature for 16 hours. Water (20 mL) was added to the reaction solution, and the mixture was extracted with ethyl acetate (30 mL × 3). The organic phases were combined, washed with saturated sodium chloride aqueous solution (10 mL), dried over sodium sulphate, and subjected to evaporation to remove the solvent. The residue was purified by silica gel Pre-TLC (PE:EA=1:3) to afford the less polar crude isomer C0102 (Rf=0.5) and the more polar crude isomer C0103 (Rf=0.4). Further purification by preparative HPLC and lyophilization yielded the less polar product C0102 (30.0 mg, yield: 2.8%) and the more polar product C0103 (50.0 mg, yield: 4.7%).

C0102: MS *m*/*z* (ESI): 384.1 [M+1]⁺.

¹H NMR (400 MHz, CD₃OD-*d*₄) *δ* 7.44-7.40 (m, 1H), 7.17-7.09 (m, 2H), 6.99-6.96 (m, 1H), 6.59-6.56 (m, 1H), 4.70-4.15 (m, 4H), 3.88-3.85 (m, 3H), 3.68-3.65 (m, 1H), 3.56 (s, 3H), 3.48-3.38 (m, 1H), 2.93-2.91 (m, 2H), 2.32 (s, 3H), 2.07 (s, 3H).

C0103: MS *m*/*z* (ESI): 384.1 [M+1]⁺.

¹H NMR (400 MHz, CD₃OD-*d*₄) *δ* 7.44-7.40 (m, 1H), 7.17-7.10 (m, 2H), 7.00-7.98 (m, 1H), 6.59-6.56 (m, 1H), 4.68-3.91 (m, 4H), 3.89 (s, 3H), 3.71-3.70 (m, 1H), 3.55 (s, 3H), 3.47-3.31 (m, 1H), 3.13-2.80 (m, 2H), 2.32 (s, 3H), 2.08 (s, 3H).

### Examples C0108&C0109

### (S, E)-(6-(2,3-Dimethylphenyl)-1,2,4-triazin-3-yl)(2-(hydroxymethyl)-4-(methoxyimino)pyrrolidin-1-yl)methanone

### (S, Z)-(6-(2,3-Dimethylphenyl)-1,2,4-triazin-3-yl)(2-(hydroxymethyl)-4-(methoxyimino)pyrrolidin-1-yl)methanone

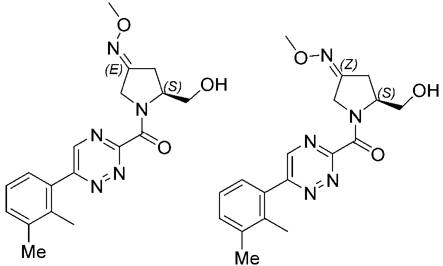

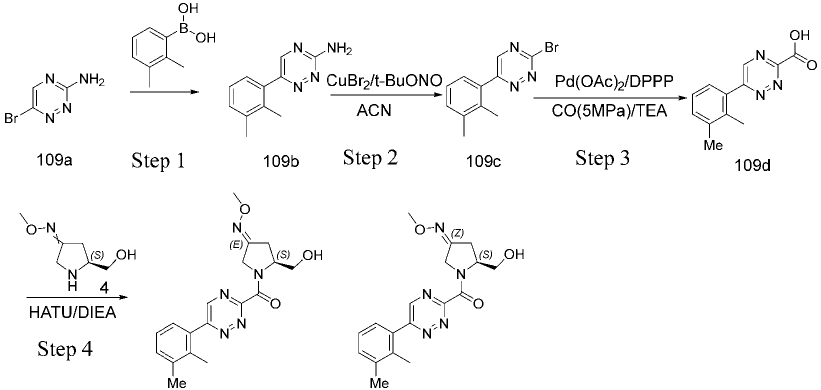

### Step 1

### 6-(2,3-Dimethylphenyl)-1,2,4-triazin-3-amine 109b

109a (3.7 g, 21.14 mmol), (2,3-dimethylphenyl)boronic acid (4.12 g, 27.48 mmol), Pd(dtbpf)Cl₂ (219 mg, 0.34 mmol) and potassium carbonate (7.3 g, 52.85 mmol) were added to a mixed solvent of 1,4-dioxane (100 mL) and water (10 mL). The mixture was purged three times with nitrogen, and stirred at 25°C under nitrogen protection for 17 hours. The reaction solution was diluted with ethyl acetate (200 mL), washed with saturated sodium chloride aqueous solution (200 mL), dried, and concentrated. The concentrate was purified by flash silica gel column chromatography (PE:EA=3:1) to afford 109b (4.0 g, yield: 94.5%) as a yellow solid.

MS *m*/*z* (ESI): 201.0 [M+1]⁺.

### Step 2

### 3-Bromo-6-(2,3-dimethylphenyl)-1,2,4-triazine 109c

109b (4.0 g, 19.98 mmol) and copper bromide (8.9 g, 40 mmol) were added to acetonitrile (500 mL), tert-butyl nitrite (3.09 g, 29.97 mmol) was slowly added dropwise, and the mixture was heated to 30°C and stirred for 1 hour. The reaction solution was filtered and concentrated, and the concentrate was purified by flash column chromatography (PE:EA=1:1), to afford 109c (2.05 g, yield: 38.8%) as a yellow oil.

MS *m*/*z* (ESI): 263.8,265.8 [M+1]⁺.

¹H NMR (400 MHz, DMSO-*d*₆) *δ* 8.92 (s, 1H), 7.39-7.34 (m, 2H), 7.30 (d, *J =* 7.2 Hz, 1H), 2.34 (s, 3H), 2.21 (s, 3H).

### Step 3

### 6-(2,3-Dimethylphenyl)-1,2,4-triazine-3-carboxylic acid 109d

109c (2.0 g, 7.57 mmol), palladium acetate (170 mg, 0.76 mmol), 1,3-bis(diphenylphosphino)propane (624 mg, 1.51 mmol) and triethylamine (1.68 g, 16.65 mmol) were dissolved in a mixed solvent of acetonitrile (100 mL) and water (10 mL). The system was purged three times with CO, pressurized to 5MPa in an autoclave, and stirred at 70°C for 5 hours. The reaction solution was concentrated, and the residue was purified by C18 reversed-phase column chromatography (acetonitrile:water = 10:90) to afford 109d (980 mg, yield: 56.4%) as a yellow solid.

MS *m*/*z* (ESI): 228.1 [M-1] ⁺.

### Step 4

### (S, Z)-(6-(2,3-Dimethylphenyl)-1,2,4-triazin-3-yl)(2-(hydroxymethyl)-4-(methoxyimino)pyrrolidin-1-yl)methanone

### (S, E)-(6-(2,3-Dimethylphenyl)-1,2,4-triazin-3-yl)(2-(hydroxymethyl)-4-(methoxyimino)pyrrolidin-1-yl)methanone

109d (500 mg, 2.18 mmol), 4 (393.75 mg, 2.18 mmol), HATU (1.24 g, 3.27 mmol) and N,N-diisopropylethylamine (845 mg, 6.54 mmol) were dissolved in DMF (20 mL), and the mixture was stirred at room temperature for 2 hours. The reaction solution was diluted with ethyl acetate (100 mL), washed with saturated sodium chloride aqueous solution (100 mL × 4), dried over anhydrous sodium sulphate, and concentrated. The residue was purified by silica gel Pre-TLC (PE:EA=1:3) to afford the less polar crude isomer C0108 (Rf=0.6) and the more polar crude isomer C0109 (Rf=0.5). Further purification by preparative HPLC and lyophilization yielded the less polar product C0108 (46 mg, yield: 5.9%) and the more polar product C0109 (43 mg, yield: 5.5%).

C0108: MS *m*/*z* (ESI): 356.1 [M+1]⁺.

¹H NMR (400 MHz, DMSO-*d*₆) *δ* 9.13-9.11 (m, 1H), 7.41-7.39 (m, 2H), 7.33-7.29 (m, 1H), 5.09-4.92 (m, 1H), 4.66-4.43 (m, 1H), 4.53-4.18 (m, 1H), 4.34 (s, 1H), 3.83-3.78 (m, 3H), 3.71-3.57 (m, 1H), 3.39-3.27 (m, 1H), 2.88-2.71 (m, 2H), 2.36 (s, 3H), 2.25-2.24 (m, 3H).

C0109: MS *m*/*z* (ESI): 356.1 [M+1]⁺.

¹H NMR (400 MHz, DMSO-*d*₆) *δ* 9.13-9.12 (d, *J =* 1.6 Hz, 1H), 7.41-7.39 (m, 2H), 7.33-7.29 (m, 1H), 5.09-4.92 (m, 1H), 4.65-4.17 (m, 3H), 3.83-3.74 (m, 3H), 3.66-3.61 (m, 1H), 3.39-3.31 (m, 1H), 3.03-2.90 (m, 1H), 2.71-2.54 (m, 1H), 2.36 (s, 3H), 2.25-2.24 (m, 3H).

### Examples C0014&C0015

### (S, E)-(2-(Hydroxymethyl)-4-(methoxyimino)pyrrolidin-1-yl)(5-(o-tolyl)pyridin-2-yl)methanone

### (S, Z)-(2-(Hydroxymethyl)-4-(methoxyimino)pyrrolidin-1-yl)(5-(o-tolyl)pyridin-2-yl)methanone

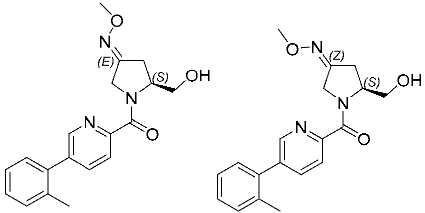

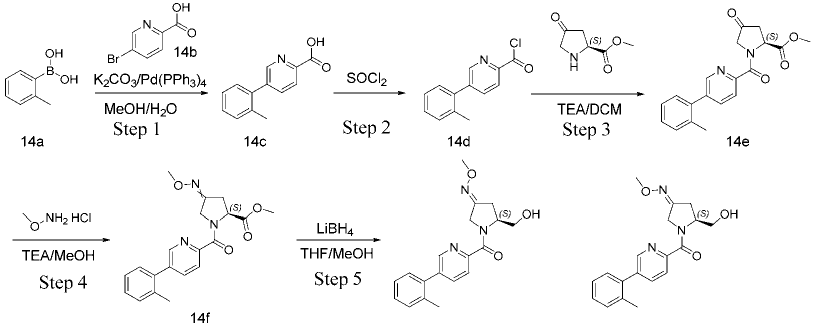

### Step 1

### 5-(o-Tolyl)picolinic acid 14c

14a (0.74 g, 5.44 mmol) and 14b (1 g, 4.95 mmol) were dissolved in methanol (7 mL) and water (14 mL), potassium carbonate (2.26 g, 16.32 mmol) and tetrakis(triphenylphosphine)palladium (63 mg, 0.054 mmol) were added at room temperature under nitrogen protection, and the mixture was reacted at 80°C under nitrogen protection for 16 hours. After the reaction was completed, the reaction solution was concentrated to remove methanol. Water (10 mL) was added to the reaction solution, and the mixture was adjusted to pH 3 with 6M hydrochloric acid, and extracted with toluene/tetrahydrofuran. The organic phase was dried over sodium sulphate, and subjected to evaporation to remove the solvent to afford 14c (0.7 g, yield: 58.5%).

MS *m*/*z* (ESI): 214.1 [M+1]⁺.

### Step 2

### 5-(o-Tolyl)picolinoyl chloride 14d

14c (0.7 g, 3.28 mmol) was dissolved in thionyl chloride (10 mL), and the mixture was reacted at 80°C for 2 hours. After the reaction was completed, the reaction solution was concentrated to afford crude 14d (0.43 g, yield: 50.8%).

### Step 3

### Methyl (S)-4-oxo-1-(5-(o-tolyl)picolinoyl)pyrrolidine-2-carboxylate 14e

Methyl (S)-4-oxopyrrolidine-2-carboxylate (0.3 g, 1.47 mmol) was dissolved in dichloromethane (10 mL), triethylamine (0.52 g, 5.14 mmol) and 14d (0.43 g, 1.76 mmol) were added at 0°C, and the mixture was reacted at room temperature for 16 hours. After the reaction was completed, the reaction solution was concentrated to remove dichloromethane, and the residue was purified by silica gel column chromatography (eluting with a gradient of petroleum ether:ethyl acetate = 5:1 to 3:1) to afford product 14e (230 mg, yield: 46.3%).

MS *m*/*z* (ESI): 339.0 [M+1]⁺.

### Step 4

### Methyl (S,EZ)-4-(methoxyimino)-1-(5-(o-tolyl)picolinoyl)pyrrolidine-2-carboxylate 14f

14e (0.5 g, 1.40 mmol) was dissolved in methanol (5 mL), methoxyamine hydrochloride (230 mg, 2.80 mmol) and triethylamine (350 mg, 3.5 mmol) were added at room temperature, and the mixture was reacted at 50°C for 18 hours. After the reaction was completed, the reaction solution was concentrated, and the residue was purified by silica gel column chromatography (eluting with a gradient of petroleum ether:ethyl acetate = 4:1 to 3:1) to afford 14f (230 mg, yield: 40.1%).

MS *m*/*z* (ESI): 368.1 [M+1]⁺.

### Step 5

### (S, Z)-(2-(Hydroxymethyl)-4-(methoxyimino)pyrrolidin-1-yl)(5-(o-tolyl)pyridin-2-yl)methanone

### (S, E)-(2-(Hydroxymethyl)-4-(methoxyimino)pyrrolidin-1-yl)(5-(o-tolyl)pyridin-2-yl)methanone

14f (230 mg, 0.59 mmol) was dissolved in methanol (10 mL) and tetrahydrofuran (10 mL), and the mixture was cooled to 0°C. Lithium borohydride (0.62 mL, 1.23 mmol) was added under nitrogen protection, and the mixture was reacted at room temperature for 2 hours. Water was added to quench the reaction solution, the reaction solution was concentrated, and the residue was dissolved in dichloromethane and water. After layer separation, the aqueous phase was extracted twice with dichloromethane, and the organic phases were combined, washed once with sodium chloride aqueous solution, dried, filtered, and subjected to rotary evaporation to dryness. The crude product was separated by silica gel Prep-TLC (EA:PE=2:1) to afford the more polar isomer C0015 (Rf=0.4, 66.09 mg, yield: 32.2%) and the less polar isomer C0014 (Rf=0.5, 48.22 mg, yield: 23.0%).

C0014: MS *m*/*z* (ESI): 340.1[M+1]⁺.

¹H NMR (400 MHz, DMSO-*d*₆) *δ* 8.61-8.58 (m, 1H), 7.98-7.81 (m, 2H), 7.37-7.30 (m, 4H), 4.90-4.62 (m, 1H), 4.58-4.52 (m, 1H), 4.47-4.10 (m, 2H), 3.82-3.75 (m, 3H), 3.62-3.52 (m, 1H), 3.40-3.34 (m, 1H), 2.83-2.62 (m, 2H), 2.27 (s, 3H).

C0015: MS *m*/*z* (ESI): 340.1[M+1]⁺.

¹H NMR (400 MHz, DMSO-*d*₆) *δ* 8.64-8.58 (m, 1H), 7.98-7.84 (m, 2H), 7.37-7.30 (m, 4H), 4.98-4.95 (m, 1H), 4.89-4.59 (m, 1H), 4.54-4.52 (m, 1H), 4.48-4.10 (m, 1H), 3.82-3.75 (m, 3H), 3.57-3.55 (m, 1H), 3.42-3.33 (m, 1H), 2.99-2.81 (m, 1H), 2.66-2.57 (m, 1H), 2.27 (s, 3H).

Examples C0012&C0013

### (S, E)-(2-(Hydroxymethyl)-4-(methoxyimino)pyrrolidin-1-yl)(5-(o-tolyl)pyrazin-2-yl)methanone

### (S, Z)-(2-(Hydroxymethyl)-4-(methoxyimino)pyrrolidin-1-yl)(5-(o-tolyl)pyrazin-2-yl)methanone

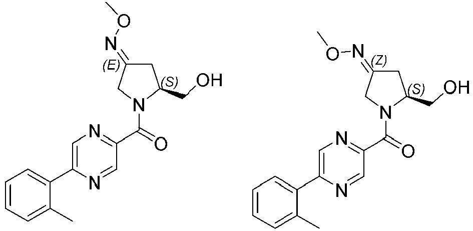

With reference to Examples C0014&C0015, 14b was replaced with 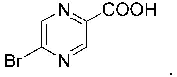 The crude product was separated by silica gel Prep-TLC (EA:PE=2:1) to afford the more polar isomer C0013 (Rf=0.4, 98.17 mg, yield: 24.3%) and the less polar isomer C0012 (Rf=0.5, 75.55 mg, yield: 19.0%).

C0012: MS *m*/*z* (ESI): 341.0 [M+1]⁺.

¹HNMR (400 MHz, DMSO-*d*₆) *δ* 9.07-9.01 (m, 1H), 8.87-8.84 (m, 1H), 7.55-7.53 (m, 1H), 7.43-7.37 (m, 3H), 5.00-4.95 (m, 1H), 4.82-4.64 (m, 1H), 4.55-4.53 (m, 1H), 4.50-4.12 (m, 1H), 3.82 -3.78 (m, 3H), 3.63-3.56 (m, 1H), 3.37 (m, 1H), 2.85-2.67 (m, 2H), 2.39 (s, 3H).

C0013: MS *m*/*z* (ESI): 341.0 [M+1]⁺.

¹H NMR (400 MHz, DMSO-*d*₆) *δ* 9.09-9.02 (m, 1H), 8.90-8.84 (m, 1H), 7.56-7.54 (m, 1H), 7.43-7.37 (m, 3H), 4.83-4.62 (m, 1H), 4.56-4.55 (m, 1H), 4.50-4.12 (m, 1H), 4.50-4.12 (m, 1H), 3.82 -3.76 (m, 3H), 3.61-3.53 (m, 1H), 3.40-3.38 (m, 1H), 3.01-2.55 (m, 2H), 2.39 (s, 3H).

### Examples C0029&C0030

### (S, E)-(2-(Hydroxymethyl)-4-(methoxyimino)pyrrolidin-1-yl)(2-(o-tolyl)thiazol-5-yl)methanone

### (S, Z)-(2-(Hydroxymethyl)-4-(methoxyimino)pyrrolidin-1-yl)(2-(o-tolyl)thiazol-5-yl)methanone

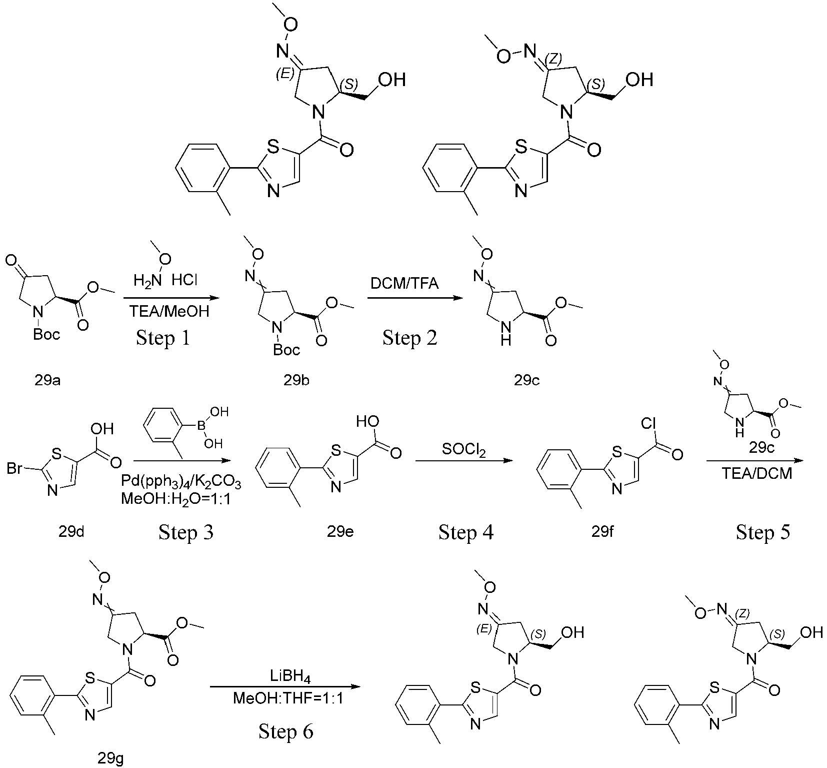

### Step 1

### 1-(Tert-butyl) 2-methyl (S, EZ)-4-(methoxyimino)pyrrolidine-1,2-dicarboxylate 29b

To a solution of 29a (5.0 g, 20.55 mmol) in methanol (50 mL) were added methoxyamine hydrochloride (1.16 g, 24.66 mmol) and triethylamine (4.57 g, 45.21 mmol). After nitrogen purging, the mixture was reacted at 60°C overnight. The reaction solution was directly subjected to rotary evaporation to dryness to afford a crude product, which was purified by silica gel column chromatography (PE:EA=5:1) to afford 29b (3.2 g, yield: 56.0%).

MS *m*/*z* (ESI): 173.0 [M+1-56]⁺.

### Step 2

### Methyl (S, EZ)-4-(methoxyimino)pyrrolidine-2-carboxylate 29c

To a solution of 29b (3.2 g, 11.75 mmol) in DCM (8 mL) was added TFA (1.34 g, 11.75 mmol), and the reaction solution was stirred at room temperature for 20 min. After the reaction was completed, the reaction solution was directly subjected to rotary evaporation to dryness, to afford crude product 29c (2.0 g, yield: 89.0%).

MS *m*/*z* (ESI): 217 [M+1-56]⁺.

According to Step 3 to Step 4 in the aforementioned synthetic route, with reference to the synthetic method of compound C0014, crude 2-(o-tolyl)thiazole-5-carbonyl chloride was obtained.

### Step 5

### Methyl (2S,4EZ)-4-(methoxyimino)-1-(2-(2-methylphenyl)-1,3-thiazole-5-carbonyl)pyrrolidine-2-carboxylate 29g

To a solution of 29f (400 mg, 1.68 mmol) and 29c (433.89 mg, 2.52 mmol) in DCM (10 mL) was added TEA (0.68 g, 6.72 mmol), and the mixture was stirred at room temperature for 1 hour. After the reaction was completed, the reaction solution was subjected to rotary evaporation to dryness to afford a crude product. The crude product was purified by preparative chromatography (ACN:H₂O=5:95 to 45:55) to afford 29g (400 mg, yield: 62.4%).

MS *m*/*z* (ESI): 374.1 [M+1]⁺.

### Step 6

### (S, E)-(2-(Hydroxymethyl)-4-(methoxyimino)pyrrolidin-1-yl)(2-(o-tolyl)thiazol-5-yl)methanone

### (S, Z)-(2-(Hydroxymethyl)-4-(methoxyimino)pyrrolidin-1-yl)(2-(o-tolyl)thiazol-5-yl)methanone

To a solution of 29g (390 mg, 1.04 mmol) in THF (5 mL) and methanol (5 mL) was slowly added LiBH₄ (0.023 g, 1.04 mmol) at 0°C, and the mixture was stirred at room temperature for 1 hour. After the reaction was completed, water (10 mL) was added to the mixture, and the resulting mixture was extracted with ethyl acetate (20 mL × 3). The organic phases were combined, washed with saturated sodium chloride aqueous solution (10 mL × 3), dried over anhydrous sodium sulphate, filtered, and subjected to rotary evaporation to afford a crude product. The residue was purified by silica gel Pre-TLC (PE:EA=1:3) to afford the less polar crude isomer C0029 (Rf=0.5) and the more polar crude isomer C0030 (Rf=0.4). Further chiral resolution and purification yielded the less polar product C0029 (15.9 mg, yield: 4.41%) and the more polar product C0030 (21.4 mg, yield: 5.87%).

C0029: MS *m*/*z* (ESI): 346.1 [M+1]⁺.

¹H NMR (400 MHz, Chloroform-*d*) *δ* 8.26 (s, 1H), 7.76 (d, *J =* 7.2 Hz, 1H), 7.39-7.27 (m, 3H), 4.87-4.56 (m, 3H), 3.91-3.71 (m, 5H), 2.85-2.83 (m, 2H), 2.60 (s, 3H).

C0030: MS *m*/*z* (ESI): 346.1 [M+1]⁺.

¹H NMR (400 MHz, Chloroform-*d*) *δ* 8.29 (s, 1H), 7.77 (d, *J =* 6.8 Hz, 1H), 7.39-7.27 (m, 3H), 4.87-4.60 (m, 3H), 3.91-3.75 (m, 5H), 2.98-2.91 (m, 1H), 2.75-2.71 (m, 1H), 2.61 (s, 3H).

### Example C0033

### (EZ)-(3-(Methoxyimino)hexahydro-1H-imidazo[2,1-c][1,4]oxazin-1-yl)(2'-methyl-[1, 1'-biphenyl]-4-yl)methanone

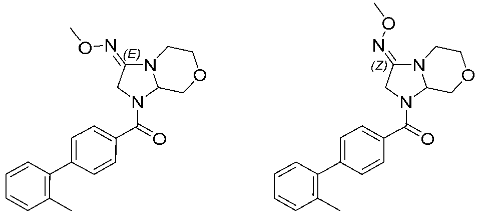

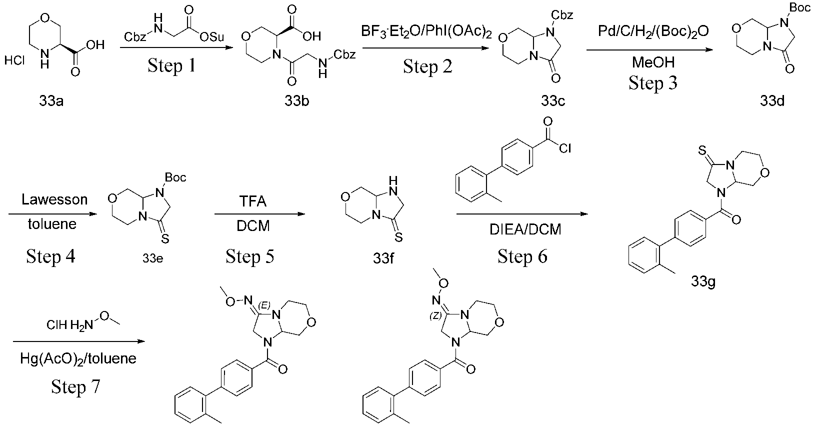

### Step 1

### (S)-4-(((Benzyloxy)carbonyl)glycyl)morpholine-3-carboxylic acid 33b

33a (4.67 g, 15.25 mmol), CbzOSU (7 g, 22.8 mmol) and triethylamine (2.31 g, 22.88 mmol) were dissolved in DMF (20 mL), and the mixture was heated to 80°C and stirred for 17 hours. The reaction solution was cooled, concentrated, purified by C18 reversed-phase column chromatography (acetonitrile/water=50/50), and concentrated to afford 33b (3.06 g, yield: 62.2%) as a white solid.

MS *m*/*z* (ESI): 323.1 [M+H]⁺.

### Step 2

Benzyl 3-oxohexahydro-1*H*-imidazo[2,1-*c*][1,4]oxazine-1-carboxylate 33c 33b (2 g, 6.21 mmol) and iodobenzene diacetate (4.00 g, 12.42 mmol) were dissolved in dichloromethane (100 mL), and the mixture was cooled to 0°C. Boron trifluoride-diethyl ether (1.76 g, 12.42 mmol) was slowly added dropwise, and the mixture was reacted at room temperature for 24 hours. Sodium bicarbonate aqueous solution (100 mL) was added to quench the reaction solution, and the reaction solution was separated by partitioning. The aqueous phase was extracted twice with (100 mL), and the organic phases were combined, dried over anhydrous sodium sulphate, filtered, concentrated, and purified by C18 reversed-phase column chromatography (water:acetonitrile = 60:40) to afford 33c (1.2 g, yield: 70.0%) as a colourless oil.

MS *m*/*z* (ESI): 277.1 [M+H]⁺.

### Step 3

### Tert-butyl 3-oxohexahydro-1H-imidazo[2,1-c][1,4]oxazine-1-carboxylate 33d

33c (0.2 g, 0.72 mmol) was dissolved in methanol (10 mL), and di-tert-butyl dicarbonate (0.2 g, 0.94 mmol) and palladium on carbon (10%, 39 mg) were added under nitrogen. The mixture was purged three times with hydrogen, and stirred at room temperature for 16 hours. After the reaction was completed, the reaction solution was filtered to remove palladium on carbon, and concentrated to remove methanol to afford crude product 33d (0.1 g, yield: 57.0%).

MS *m*/*z* (ESI): 243.1 [M+H]⁺.

### Step 4

### Tert-butyl 3-thiohexahydro-1H-imidazo[2,1-c][1,4]oxazine-1-carboxylate 33e

33d (0.8 g, 3.30 mmol) was dissolved in toluene (15 mL), Lawesson's reagent (1.33 g, 3.30 mmol) was added, and the mixture was heated to 50°C and reacted for half an hour. After the reaction was completed, the reaction solution was filtered to remove solid impurities, subjected to rotary evaporation to dryness to remove the solvent, and purified by reversed-phase preparative chromatography to afford 33e (0.6 g, yield: 62.6%).

MS *m*/*z* (ESI): 259.0 [M+H]⁺.

### Step 5

### Tetrahydro-1H-imidazo[2,1-c][1,4]oxazine-3(2H)-thione 33f

33e (0.68 g, 2.63 mmol) was dissolved in dichloromethane (9 mL), trifluoroacetic acid (3 mL) was added, and the mixture was reacted at room temperature for 3 hours. After the reaction was completed, the reaction solution was subjected to evaporation to remove the solvent to afford 33f (0.4 g, yield: 96.0%).

MS *m*/*z* (ESI): 159.0 [M+H]⁺.

### Step 6

### (2'-Methyl-[1, 1'-biphenyl]-4-yl)(3-thiohexahydro-1H-imidazo[2,1-c][1,4]oxazin-1-yl)methanone 33g

33f (0.7 g, 4.42 mmol) was dissolved in dichloromethane (10 mL), 2'-methyl-[1, 1'-biphenyl]-4-carbonyl chloride (1.02 g, 4.42 mmol) and triethylamine (1.34 g, 13.26 mmol) were added, and the mixture was reacted at room temperature for 16 hours. After the reaction was completed, water (10 mL) was added to the reaction solution, and the mixture was extracted with dichloromethane (10 mL × 2). The organic phase was washed with saturated sodium chloride aqueous solution (10 mL × 2), dried over sodium sulphate, and subjected to evaporation to remove the solvent. The residue was purified by Pre-TLC (petroleum ether:ethyl acetate = 5:1) to afford 33g (1 g, yield: 56.4%).

MS *m*/*z* (ESI): 353.1 [M+H]⁺.

### Step 7

### (EZ)-(3-(Methoxyimino)hexahydro-1H-imidazo[2,1-c][1,4]oxazin-1-yl)(2'-methyl-[1, 1'-biphenyl]-4-yl)methanone

33g (0.2 g, 0.57 mmol) was dissolved in toluene (5 mL), methoxyamine hydrochloride (0.19 g, 2.28 mmol) and mercury acetate (0.36 g, 1.14 mmol) were added, and the mixture was reacted at 100°C for 2 hours. After the reaction was completed, ethyl acetate (20 mL) was added to the reaction solution. The mixture was washed with water (10 mL × 2) and once with saturated sodium chloride (10 mL), dried over sodium sulphate, and subjected to evaporation to remove the solvent. The crude product was purified by preparative HPLC (HCOOH) and lyophilized to afford C0033 (80 mg, yield: 38.6%).

MS *m*/*z* (ESI): 366.1 [M+H]⁺.

¹H NMR (400 MHz, DMSO-*d*₆) *δ* 7.72-7.62 (m, 2H), 7.46-7.44 (m, 2H), 7.29-7.22 (m, 4H), 5.46-5.34 (m, 1H), 4.67-4.60 (m, 1H), 4.39-4.25 (m, 1H), 3.79-3.72 (m, 2H), 3.64 (s, 3H), 3.52-3.43 (m, 1H), 3.25-3.15 (m, 1H), 2.25 (s, 3H).

### Examples C0048&C0049

### (S, E)-(4-(Benzo[d][1,3]dioxolan-4-yl)phenyl)(2-(hydroxymethyl)-4-(methoxyimino)pyrrolidin-1-yl)methanone

### (S, Z)-(4-(Benzo[d][1,3]dioxolan-4-yl)phenyl)(2-(hydroxymethyl)-4-(methoxyimino)pyrrolidin-1-yl)methanone

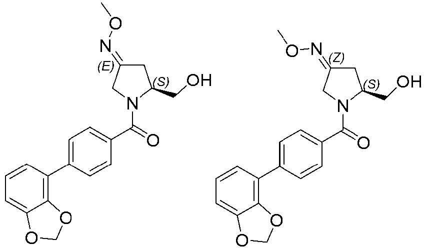

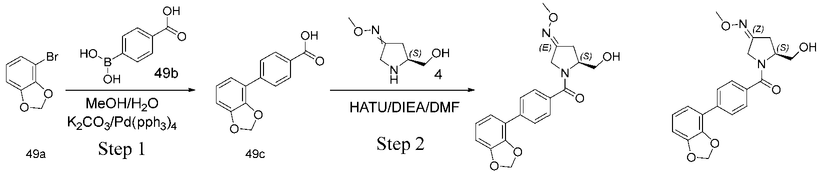

According to the aforementioned synthetic route, with reference to the synthetic method of compound A0203, the less polar isomer C0048 (Rf=0.4) and the more polar crude isomer C0049 (Rf=0.3) were obtained. The crude product was further purified by preparative HPLC and lyophilized to afford the less polar product C0048 (39 mg, yield: 15.3%) and the more polar product C0049 (30 mg, yield: 11.8%).

C0048: MS *m*/*z* (ESI): 369.1 [M+1]⁺.

¹H NMR (400 MHz, CD₃OD-*d*₄) *δ* 7.87-7.85 (m, 2H), 7.61 (d, *J =* 7.6 Hz, 2H), 7.13 (d, *J =* 7.6 Hz, 1H), 6.97-6.93 (m, 1H), 6.85 (dd, *J =* 8.0, 1.2 Hz, 1H), 6.02 (s, 2H), 4.80-4.08 (m, 3H), 4.18-3.65 (m, 5H), 2.87-2.85 (m, 2H).

C0049: MS *m*/*z* (ESI): 369.1 [M+1]⁺.

¹H NMR (400 MHz, CD₃OD-*d*₄) *δ* 7.85 (d, *J =* 8.4 Hz, 2H), 7.61 (d, *J =* 7.6 Hz, 2H), 7.13 (d, *J =* 8.4 Hz, 1H), 6.97-6.93 (m, 1H), 6.85 (dd, *J =* 8.0, 0.8 Hz, 1H), 6.02 (s, 2H), 4.79-4.36 (m, 2H), 4.35-3.69 (m, 6H), 2.98-2.67 (m, 2H).

### Examples C0080&C0081

### (S, E)-(5-(2,3-Dimethylphenyl)-3-methoxypyridin-2-yl)(2-(hydroxymethyl)-4-(methoxyimino)pyrrolidin-1-yl)methanone

### (S, Z)-(5-(2,3-Dimethylphenyl)-3-methoxypyridin-2-yl)(2-(hydroxymethyl)-4-(methoxyimino)pyrrolidin-1-yl)methanone

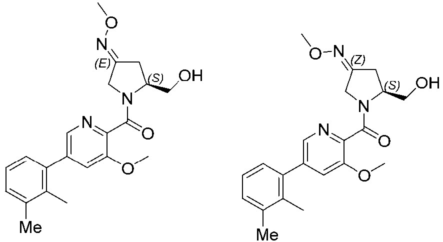

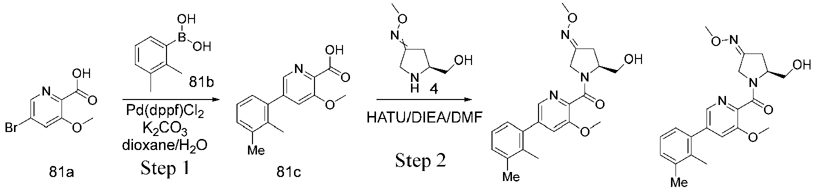

According to the aforementioned synthetic route, with reference to the synthetic method of compound C0102, the less polar isomer C0080 (Rf=0.4) and the more polar crude isomer C0081 (Rf=0.3) were obtained. Further purification by preparative HPLC and lyophilization yielded the less polar product C0080 (19.2 mg, yield: 4.29%) and the more polar product C0081 (22.69 mg, yield: 5.07%).

C0080: MS *m*/*z* (ESI): 384.1 [M+1]⁺.

¹H NMR (400 MHz, CD₃OD-*d*₄) *δ* 8.11-8.09 (m, 1H), 7.54-7.52 (m, 1H), 7.24-7.10 (m, 3H), 4.80-4.55 (m, 1H), 4.19-4.15 (m, 1H), 3.91-3.90 (m, 4H), 3.88-3.83 (m, 3H), 3.71-3.40 (m, 2H), 2.98-2.82 (m, 2H), 2.36 (s, 3H), 2.18 (s, 3H).

C0081: MS *m*/*z* (ESI): 384.1 [M+1]⁺.

¹H NMR (400 MHz, CD₃OD-*d*₄) *δ* 8.11-8.09 (m, 1H), 7.54-7.52 (m, 1H), 7.24-7.08 (m, 3H), 4.69-4.50 (m, 1H), 4.26-3.99 (m, 2H), 3.93-3.92 (m, 3H), 3.89-3.70 (m, 4H), 3.43-3.41 (m, 1H), 2.98-2.66 (m, 2H), 2.36 (s, 3H), 2.18 (s, 3H).

### Examples C0072&C0073

### (S, E)-(5-(2,3-Dimethylphenyl)-6-methoxypyridin-2-yl)(2-(hydroxymethyl)-4-(methoxyimino)pyrrolidin-1-yl)methanone

### (S, Z)-(5-(2,3-Dimethylphenyl)-6-methoxypyridin-2-yl)(2-(hydroxymethyl)-4-(methoxyimino)pyrrolidin-1-yl)methanone

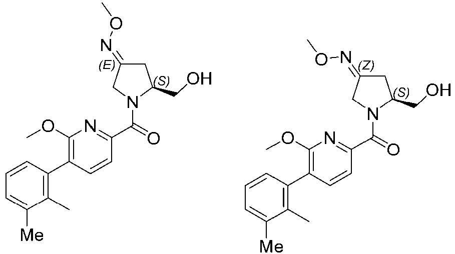

With reference to the method of C0080&C0081, by replacing the starting material 81a in Step 1 with 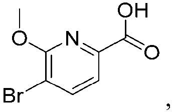 the aforementioned compound was obtained.

The crude product was separated by silica gel Prep-TLC (PE:EA=1:5) to afford the less polar crude product C0072 (Rf=0.4) and the pure more polar product C0073 (Rf=0.3, 20 mg, yield: 18.7%). The less polar crude isomer was further purified by preparative HPLC and lyophilized to afford the less polar product C0072 (34.9 mg, yield: 9.72%).

C0072: MS *m*/*z* (ESI): 384.1 [M+1]⁺.

¹H NMR (400 MHz, CD₃OD-*d*₄) *δ* 7.64-7.18 (m, 2H), 7.16-7.10 (m, 2H), 6.95 (d, *J* = 7.2 Hz, 2H), 4.81-4.73 (m, 2H), 4.60-4.19 (m, 1H), 3.93-3.58 (m, 8H), 3.02-2.77 (m, 2H), 2.32 (s, 3H), 2.00 (s, 3H).

C0073: MS *m*/*z* (ESI): 384.1 [M+1]⁺.

¹H NMR (400 MHz, CD₃OD-*d*₄) *δ* 7.64-7.54 (m, 2H), 7.18-7.10 (m, 2H), 6.97-6.95 (m, 1H), 4.83-4.76 (m, 2H), 4.56-4.24 (m, 1H), 3.90-3.60 (m, 8H), 2.99-2.32 (m, 2H), 2.32 (s, 3H), 2.00 (s, 3H).

### Examples C0110&C0111&C0112&C0113

### (S, S, E)-4'-(2-(1-Hydroxyethyl)-4-(methoxyimino)pyrrolidine-1-carbonyl)-2-methyl-[1,1'-biphenyl]-3-carbonitrile

### (S, S, Z)-4'-(2-(1-Hydroxyethyl)-4-(methoxyimino)pyrrolidine-1-carbonyl)-2-methyl-[1,1'-biphenyl]-3-carbonitrile

### (S, R, Z)-4'-(2-(1-Hydroxyethyl)-4-(methoxyimino)pyrrolidine-1-carbonyl)-2-methyl-[1,1'-biphenyl]-3-carbonitrile

### (S, R, E)-4'-(2-(1-Hydroxyethyl)-4-(methoxyimino)pyrrolidine-1-carbonyl)-2-methyl-[1,1'-biphenyl]-3-carbonitrile

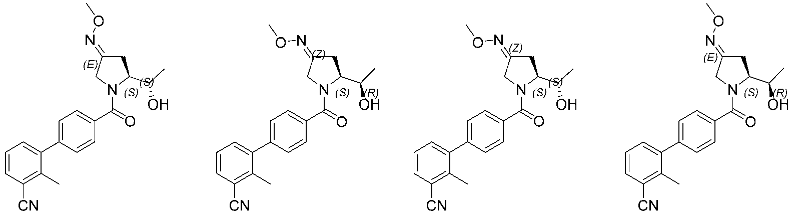

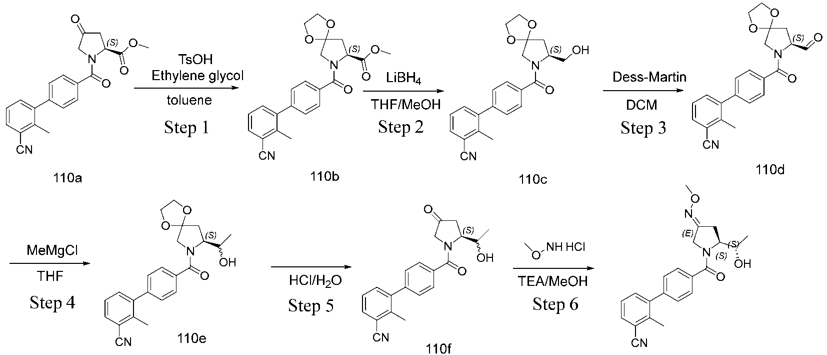

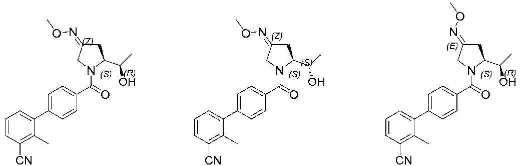

### Step 1

### Methyl (S)-7-(3'-cyano-2'-methyl-[1,1'-biphenyl]-4-carbonyl)-1,4-dioxo-7-azaspiro[4.4]nonane-8-carboxylate 110b

110a (1.8 g, 4.97 mmol, prepared with reference to the method of 35d in Examples C0035&C0036) was dissolved in toluene (15 mL), ethylene glycol (463 mg, 7.46 mmol) and p-toluenesulfonic acid hydrate (331 mg, 1.74 mmol) were added to the reaction solution, and the mixture was reacted at 120°C for 16 hours. After the reaction was completed, the reaction solution was subjected to rotary evaporation to remove the solvent, and the residue was purified by column chromatography (PE/EA=6/1) to afford product 110b (500 mg, yield: 24.8%).

MS *m*/*z* (ESI): 406.8 [M+1]⁺.

### Step 2

### (S)-4'-(8-(Hydroxymethyl)-1,4-dioxo-7-azaspiro[4.4]nonane-7-carbonyl)-2-methyl-[1,1'-biphenyl]-3-carbonitrile 110c

110b (6 g, 14.76 mmol) was dissolved in methanol (100 mL) and tetrahydrofuran (100 mL), and lithium borohydride tetrahydrofuran solution (2 M, 14.8 mL) was added dropwise at -10°C. After the dropwise addition was completed, the reaction solution was heated to room temperature and stirred for 3 hours. After the reaction was completed, water was added to quench the reaction solution, the reaction solution was concentrated, and the residue was dissolved in dichloromethane and water. After layer separation, the aqueous phase was extracted twice with dichloromethane, and the organic phases were combined, washed with sodium chloride aqueous solution, dried, filtered, and subjected to rotary evaporation to dryness. The crude product was loaded onto a silica gel column, eluting with a gradient of (PE/EA=3:1 to 1:1). The eluate was collected and concentrated to afford product 110c (4 g, yield: 71.6%).

MS *m*/*z* (ESI): 378.9 [M+1]⁺.

### Step 3

### (S)-4'-(8-Formyl-1,4-dioxo-7-azaspiro[4.4]nonane-7-carbonyl)-2-methyl-[1,1'-biphenyl]-3-carbonitrile 110d

110c (4.2 g, 11.10 mmol) was dissolved in dichloromethane (200 mL), Dess-Martin oxidant (4.71 g, 11.1 mmol) was added to the reaction solution, and the mixture was reacted at room temperature for 16 hours. After the reaction was completed, dichloromethane and water were added, and the mixture was extracted twice with dichloromethane. The organic phases were combined, subjected to rotary evaporation to remove the solvent, washed once with sodium chloride aqueous solution, dried, filtered, and subjected to rotary evaporation to dryness. The crude product was loaded onto a silica gel column, eluting with a gradient of (PE/EA=2:1 to 1:1). The eluate was collected and concentrated to afford product 110d (3.5 g, yield: 83.8%).

MS *m*/*z* (ESI): 376.9 [M+1]⁺.

### Step 4

### (S)-4'-(8-(1-Hydroxyethyl)-1,4-dioxo-7-azaspiro[4.4]nonane-7-carbonyl)-2-methyl-[1,1'-biphenyl]-3-carbonitrile 110e

110d (3.5 g, 9.30 mmol) was dissolved in tetrahydrofuran (125 mL), 24.7 mL of 3M methylmagnesium chloride tetrahydrofuran solution was added at -78°C, and the mixture was heated to room temperature and stirred for 20 minutes. After the reaction was completed, water was added to the reaction solution to quench the reaction solution, and the reaction solution was extracted three times with water and ethyl acetate. The organic phase was collected, washed once with sodium chloride aqueous solution, dried over anhydrous sodium sulphate, filtered, and subjected to rotary evaporation to remove the solvent to afford product 110e (1.5 g, yield: 41.1%).

MS *m*/*z* (ESI): 392.9 [M+1]⁺.

### Step 5

### (S)-4'-(2-(1-Hydroxyethyl)-4-oxopyrrolidine-1-carbonyl)-2-methyl-[1,1'-biphenyl]-3-carbonitrile 110f

110e (50 mg, 0.13 mmol) was dissolved in hydrochloric acid (2 mL) and water (20 mL), and the mixture was reacted at 105°C for 20 minutes. After the reaction was completed, the reaction solution adjusted to neutral, and extracted three times with water and ethyl acetate. The organic phase was collected, washed once with sodium chloride aqueous solution, dried over anhydrous sodium sulphate, and filtered. The crude product was loaded onto a silica gel column, eluting with a gradient of (PE/EA=1:1 to 1:2). The eluate was collected and concentrated to afford product 110f (28 mg, yield: 63.0%).

MS *m*/*z* (ESI): 348.9 [M+1]⁺.

### Step 6

### (S, S, E)-4'-(2-(1-Hydroxyethyl)-4-(methoxyimino)pyrrolidine-1-carbonyl)-2-methyl-[1,1'-biphenyl]-3-carbonitrile

### (S, R, E)-4'-(2-(1-Hydroxyethyl)-4-(methoxyimino)pyrrolidine-1-carbonyl)-2-methyl-[1,1'-biphenyl]-3-carbonitrile

### (S, S, Z)-4'-(2-(1-Hydroxyethyl)-4-(methoxyimino)pyrrolidine-1-carbonyl)-2-methyl-[1,1'-biphenyl]-3-carbonitrile

### (S, R, Z)-4'-(2-(1-Hydroxyethyl)-4-(methoxyimino)pyrrolidine-1-carbonyl)-2-methyl-[1,1'-biphenyl]-3-carbonitrile

110f (400 mg, 1.15 mmol) was dissolved in methanol (20 mL), methoxyamine hydrochloride (144 mg, 1.72 mmol) and triethylamine (349 mg, 3.45 mmol) were added at room temperature, and the mixture was reacted at 50°C for 18 hours. The reaction solution was subjected to rotary evaporation to remove methanol, and extracted three times with dichloromethane and water. The organic phases were combined, washed once with sodium chloride aqueous solution, dried, filtered, and subjected to rotary evaporation to dryness. The residue was purified by silica gel Pre-TLC (PE:EA=1:2) to afford the less polar crude isomer (Rf=0.6) and the more polar crude isomer (Rf=0.5). Further chiral resolution yielded the first-eluting peak of the less polar product, C0110 (30 mg, yield: 6.9%, retention time: 5.85 min), the second-eluting peak of the less polar product, C0111 (52 mg, yield: 12.0%, retention time: 7.98 min) (chiral analysis method: instrument: high-throughput semi-preparative chromatograph GX-281; chromatographic column: Daicel CHIRALPAK ^{®} IA-3.0 cm column; mobile phase: n-hexane: ethanol = 40:60; column temperature: 30°C; flow rate: 25 ml/min; detection wavelength: 254 nm);
the first-eluting peak of the more polar product, C0112 (72 mg, yield: 12.6%, retention time: 6.59 min), and the second-eluting peak of the more polar product, C0113 (47 mg, yield: 10.9%, retention time: 8.42 min) (chiral analysis method: instrument: high-throughput semi-preparative chromatograph GX-281; chromatographic column: Daicel CHIRALPAK ^{®} IC-3.0 cm column; mobile phase: n-hexane: ethanol = 40: 60; column temperature: 30°C; flow rate: 25 ml/min; detection wavelength: 254 nm).

C0110: MS *m*/*z* (ESI): 378.1 [M+1]⁺.

¹H NMR (400 MHz, CD₃OD) *δ* 7.72-7.67 (m, 3H), 7.55-7.53 (m, 1H), 7.46-7.42 (m, 3H), 4.45-4.02 (m, 4H), 3.83 (s, 3H), 2.93-2.88 (m, 2H), 2.45 (s, 3H), 1.24-1.22 (m, 3H).

C0111: MS *m*/*z* (ESI): 378.1 [M+1]⁺.

¹H NMR (400 MHz, CD₃OD) *δ* 7.72-7.66 (m, 3H), 7.55-7.53 (m, 1H), 7.46-7.42 (m, 3H), 4.61-4.07 (m, 4H), 3.86-3.83 (m, 3H), 2.99-2.94 (m, 1H), 2.73-2.68 (m, 1H), 2.45 (s, 3H), 1.22-1.21 (m, 3H).

C0112: MS *m*/*z* (ESI): 378.1 [M+1]⁺.

¹H NMR (400 MHz, CD₃OD) *δ* 7.72-7.66 (m, 3H), 7.55-7.54 (m, 1H), 7.46-7.42 (m, 3H), 4.62-4.40 (m, 2H), 4.11-4.06 (m, 2H), 3.87-3.78 (m, 3H), 2.84-2.78 (m, 2H), 2.45 (s, 3H), 1.29-1.23 (m, 3H).

C0113: MS *m*/*z* (ESI): 378.1 [M+1]⁺.

¹H NMR (400 MHz, CD₃OD) *δ* 7.72-7.67 (m, 3H), 7.56-7.54 (m, 1H), 7.46-7.42 (m, 3H), 4.47-4.03 (m, 4H), 3.87-3.78 (m, 3H), 2.87-2.69 (m, 2H), 2.45 (s, 3H), 1.17-1.16 (m, 3H).

### Example A0086

### (S, EZ)-(2-(Benzo[d]oxazol-2-yl)-4-(methoxyimino)pyrrolidin-1-yl)(2'-methyl-[1, 1'-biphenyl]-4-yl)methanone

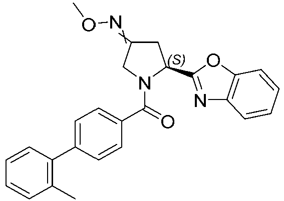

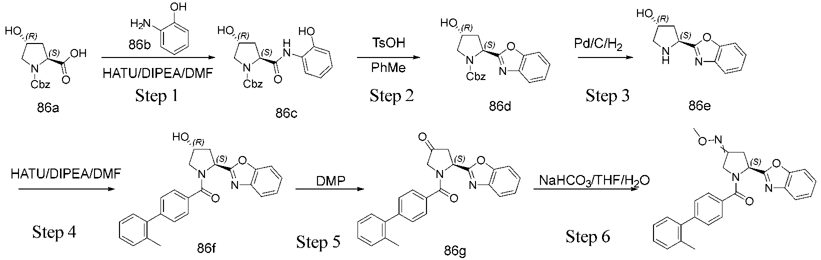

### Step 1

### (2S, 4R)-Benzyl 4-hydroxy-2-((2-hydroxyphenyl)carbamoyl)pyrrolidine-1-carboxylate 86c

86a (9 g, 33.93 mmol) and ethyldiisopropylamine (17.54 g, 135.72 mmol) were dissolved in 100 mL of DMF, and then HATU (16.77 g, 44.11 mmol) was added, and the mixture was reacted for 5 minutes. 86b (4.44 g, 40.72 mmol) was added, and then the reaction solution was reacted at room temperature for 16 hours. Then, 100 mL of water was added, and the mixture was extracted with ethyl acetate (100 mL × 3). The organic phases were combined, washed with sodium chloride aqueous solution (50 mL × 3), dried over anhydrous sodium sulphate, and filtered, and the filtrate was concentrated under reduced pressure to afford a residue, which was purified by silica gel column chromatography to afford crude product 86c (5 g, yield: 41.3%).

MS *m*/*z* (ESI): 357.2 [M+1]⁺.

### Step 2

### Benzyl (2S, 4R)-2-(benzo[d]oxazol-2-yl)-4-hydroxypyrrolidine-1-carboxylate 86d

86c (5.3 g, 14.87 mmol) was dissolved in 50 mL of toluene, and then toluene-4-sulfonic acid (0.51 g, 2.97 mmol) was added, and the reaction solution was reacted at 110°C under reflux for 16 hours. Then, 100 mL of water was added, and the mixture was extracted with ethyl acetate (100 mL × 3). The organic phases were combined, washed with sodium chloride aqueous solution (50 mL × 3), dried over anhydrous sodium sulphate, and filtered, and the filtrate was concentrated under reduced pressure to afford a residue, which was purified by silica gel column chromatography to afford product 86d (184 mg, yield: 3.66%).

MS *m*/*z* (ESI): 339.2 [M+1]⁺.

### Step 3

### (3R, 5S)-5-(Benzo[d]oxazol-2-yl)pyrrolidin-3-ol 86e

The starting material 86d (270 mg, 0.8 mmol) was dissolved in 5 mL of methanol, and then wet palladium on carbon (0.085 g, 0.8 mmol) was added. After H₂ purging, the reaction solution was reacted at 25°C for 16 hours. The reaction solution was filtered, and the filter cake was rinsed twice with methanol. The filtrate was then concentrated to afford crude product 86e (140 mg).

MS *m*/*z* (ESI): 205.2 [M+1]⁺.

### Step 4

### ((2S, 4R)-2-(Benzo[d]oxazol-2-yl)-4-hydroxypyrrolidin-1-yl)(2'-methyl-[1, 1'-biphenyl]-4-yl)methanone 86f

86e (140 mg, 0.69 mmol) and 4-(2-methylphenyl)benzoic acid (0.18 g, 0.83 mmol) were dissolved in 5 mL of DMF, and then HATU (0.34 g, 0.90 mmol) and ethyldiisopropylamine (0.36 g, 2.76 mmol) were added, and the reaction solution was reacted at room temperature for 2 hours. Then, 10 mL of water was added, and the mixture was extracted with ethyl acetate (10 mL × 3). The organic phases were combined, washed with sodium chloride aqueous solution (10 mL × 3), dried over anhydrous sodium sulphate, and filtered, and the filtrate was concentrated under reduced pressure to afford a residue, which was purified by silica gel column chromatography to afford product 86f (140 mg, yield: 51.3%).

MS *m*/*z* (ESI): 399.2 [M+1]⁺.

### Step 5

### ((S)-5-(Benzo[d]oxazol-2-yl)-1-(2'-methyl-[1, 1'-biphenyl]-4-carbonyl)pyrrolidin-3-one 86g

The starting material 86f (260 mg, 0.65 mmol) was dissolved in 5 mL of dichloromethane, and then Dess-Martin reagent (0.55 g, 1.3 mmol) was added, and the reaction solution was reacted at 25°C for 16 hours. 20 mL of saturated sodium bicarbonate solution was then added to quench the reaction solution. The reaction solution was extracted with dichloromethane (10 mL × 3). The organic phases were combined, washed with sodium chloride aqueous solution (10 mL × 3), dried over anhydrous sodium sulphate, and filtered, and the filtrate was concentrated under reduced pressure to afford product 86g (250 mg, yield: 96.6%).

MS *m*/*z* (ESI): 397.2 [M+1]⁺.

### Step 6

### (S, EZ)-(2-(Benzo[d]oxazol-2-yl)-4-(methoxyimino)pyrrolidin-1-yl)(2'-methyl-[1, 1'-biphenyl]-4-yl)methanone

86g (270 mg, 0.68 mmol) and methoxyamine hydrochloride (0.17 g, 2.04 mmol) were dissolved in 2 mL of tetrahydrofuran and 2 mL of water, and then sodium bicarbonate (0.17 g, 2.04 mmol) was added, and the reaction solution was reacted at 25°C for 5 hours. Then, 10 mL of water was added, and the mixture was extracted with ethyl acetate (10 mL × 3). The organic phases were combined, washed with sodium chloride aqueous solution (10 mL × 3), dried over anhydrous sodium sulphate, and filtered, and the filtrate was concentrated under reduced pressure to afford a crude product, which was separated and purified by preparative chromatography to afford product A0086 (55.67 mg, yield: 96.6%).

MS *m*/*z* (ESI): 426.0 [M+1]⁺.

¹H NMR (400 MHz, Chloroform-*d*) *δ* 7.78-7.62 (m, 2H), 7.56-7.38 (m, 1H), 7.37-7.26 (m, 6H), 7.23-7.12 (m, 3H), 6.16 (s, 1H), 4.68-4.33 (m, 2H), 3.86 (s, 3H), 3.34-3.11 (m, 2H), 2.30-2.25 (m, 2H).

### Examples A0087&A0101

### [(3Z)-3-(Methoxyimino)-7-(4-(2-methylphenyl)benzoyl)-7-azabicyclo[2.2.1]heptan-1-yl]methanone

### [(3E)-3-(Methoxyimino)-7-(4-(2-methylphenyl)benzoyl)-7-azabicyclo[2.2.1]heptan-1-yl]methanone

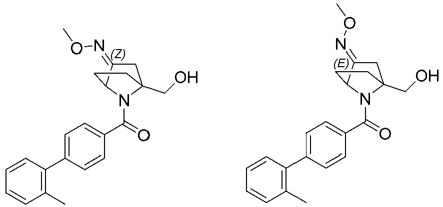

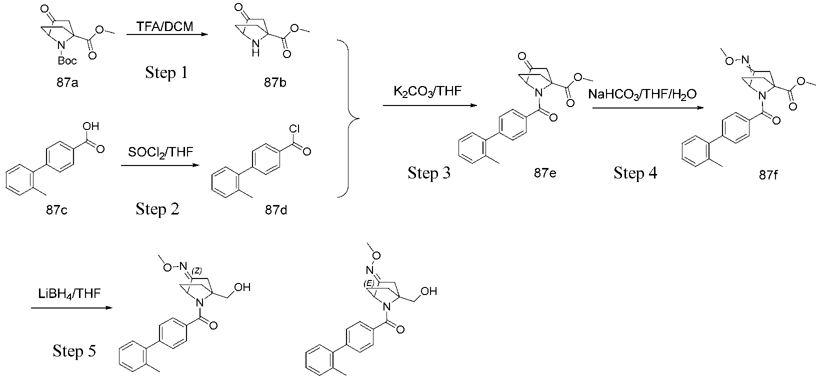

### Step 1

### Methyl 3-oxo-7-azabicyclo[2.2.1]heptane-1-carboxylate 87d

87a (20 mg, 0.074 mmol) was dissolved in 5 mL of dichloromethane, and then trifluoroacetic acid (4.59 g, 40.26 mmol) was added, and the reaction solution was reacted at 25°C for 30 min. Then, the reaction solution was concentrated under reduced pressure, to afford crude product 87b (125 mg).

MS *m*/*z* (ESI): 170.0 [M+1]⁺.

### Step 3

### Methyl 7-(2'-methyl-[1,1'-biphenyl]-4-carbonyl)-3-oxo-7-azabicyclo[2.2.1]heptane-1-carboxylate 87e

87d (125 mg, 0.74 mmol) and 87b (0.20 g, 0.89 mmol) were dissolved in 5 mL of tetrahydrofuran solution, potassium carbonate (0.31 g, 2.22 mmol) was added, and then the reaction solution was reacted at 25°C for 6 hours. Then, 10 mL of water was added, and the mixture was extracted with ethyl acetate (10 mL × 3). The organic phases were combined, washed with sodium chloride aqueous solution (10 mL × 3), dried over anhydrous sodium sulphate, and filtered, and the filtrate was concentrated under reduced pressure to afford crude product 87e (268 mg).

MS *m*/*z* (ESI): 364.3 [M+1]⁺.

### Step 4

### Methyl (EZ)-3-(methoxyimino)-7-(2'-methyl-[1,1'-biphenyl]-4-carbonyl)-7-azabicyclo[2.2.1]heptane-1-carboxylate 87f

87e (200 mg, 0.55 mmol) was dissolved in 2 mL of tetrahydrofuran and 2 mL of water, and then methoxyamine hydrochloride (0.18 g, 2.2 mmol) and sodium bicarbonate (0.14 g, 1.65 mmol) were added, and the reaction solution was reacted at 25°C for 16 hours. Then, 10 mL of water was added, and the mixture was extracted with ethyl acetate (10 mL × 3). The organic phases were combined, washed with sodium chloride aqueous solution (10 mL × 3), dried over anhydrous sodium sulphate, filtered, and concentrated to afford product 87f (299 mg).

MS *m*/*z* (ESI): 364.2[M+1]⁺.

### Step 5

### [(3Z)-3-(Methoxyimino)-7-(4-(2-methylphenyl)benzoyl)-7-azabicyclo[2.2.1]heptan-1-yl]methanone

### [(3E)-3-(Methoxyimino)-7-(4-(2-methylphenyl)benzoyl)-7-azabicyclo[2.2.1]heptan-1-yl]methanone

87f (100 mg, 0.25 mmol) was dissolved in 2 mL of tetrahydrofuran solution, and the mixture was purged with nitrogen. Lithium borohydride (8.2 mg, 0.38 mmol) was then added, and the reaction solution was reacted at 25°C for 16 hours. Then, 5 mL of water was added, and the mixture was extracted with ethyl acetate (5 mL × 3). The organic phases were combined, washed with sodium chloride aqueous solution (5 mL × 3), dried over anhydrous sodium sulphate, filtered, and concentrated to afford a crude product. The crude product was separated by silica gel Prep-TLC (EA:PE=2:1) to afford the less polar isomer A0101 (Rf=0.4) and the more polar crude isomer A0087 (Rf=0.3). Further purification by preparative HPLC and lyophilization yielded the more polar product, and the residue thus obtained was purified by preparative chromatography to afford the more polar product A0087 (3.3 mg, yield: 3.5%) and the less polar product A0101 (2.94 mg, yield: 3.15%).

A0087: MS *m*/*z* (ESI): 365.0 [M+1]⁺.

¹H NMR (400 MHz, Chloroform-*d*) *δ* 7.54 (d, *J =* 8.4 Hz, 2H), 7.39-7.37 (m, 2H), 7.30-7.26 (m, 2H), 7.25-7.22 (m, 2H), 5.39-5.30 (m, 1H), 4.70 (d, *J =* 4.4 Hz, 1H), 4.11-4.05 (m, 2H), 3.84 (s, 3H), 3.01-2.93 (m, 1H), 2.36-2.25 (m, 1H), 2.27 (s, 3H), 2.18-2.13 (m, 2H), 1.84-1.76 (m, 1H), 1.69-1.63 (m, 1H).

A0101: MS *m*/*z* (ESI): 365.0 [M+1]⁺.

¹H NMR (400 MHz, Chloroform-*d*) *δ* 7.55-7.53 (m, 2H), 7.41-7.25 (m, 6H), 5.38-5.30 (m, 2H), 4.72 (s, 1H), 4.17-4.01 (m, 1H), 3.86 (s, 3H), 3.02-2.97 (m, 1H), 2.40-2.22 (m, 7H), 2.20-2.12 (m, 1H).

### Examples A0130& A0144

### (S, Z)-4'-(2-(Hydroxymethyl)-4-(methoxyimino)pyrrolidine-1-carbonyl)-3-methyl-[1,1'-biphenyl]-2-carbonitrile

### (S, E)-4'-(2-(Hydroxymethyl)-4-(methoxyimino)pyrrolidine-1-carbonyl)-3-methyl-[1,1'-biphenyl]-2-carbonitrile

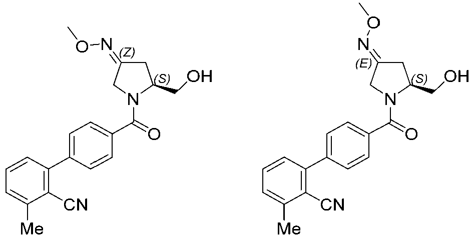

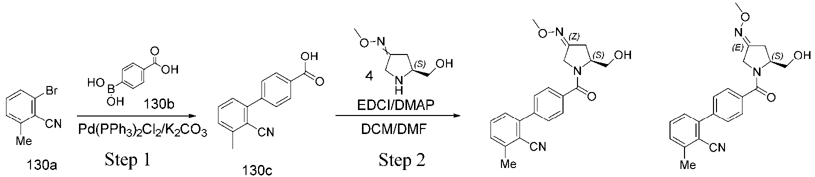

### Step 1

### 2'-Cyano-3'-methyl-[1,1'-biphenyl]-4-carboxylic acid 130c

130a (200 mg, 1.02 mmol), 130b (0.17 g, 1.02 mmol), bis(triphenylphosphine)palladium dichloride (0.072 g, 0.10 mmol), and potassium carbonate (0.70 g, 5.1 mmol) were mixed in 1,4-dioxane (3 mL) and water (0.5 mL) under argon protection, and the reaction solution was stirred at 100°C for 2 hours. Ethyl acetate was added to the reaction solution, and the aqueous phase was adjusted to pH = 4-5 with 1N hydrochloric acid to precipitate a solid. The mixture was filtered, and the obtained solid was dried to afford 130c (120 mg, yield: 44.6%).

MS *m*/*z* (ESI): 238 [M+1]⁺.

### Step 2

### (S, Z)-4'-(2-(Hydroxymethyl)-4-(methoxyimino)pyrrolidine-1-carbonyl)-3-methyl-[1,1'-biphenyl]-2-carbonitrile

### (S, E)-4'-(2-(Hydroxymethyl)-4-(methoxyimino)pyrrolidine-1-carbonyl)-3-methyl-[1,1'-biphenyl]-2-carbonitrile

130c (100 mg, 0.42 mmol) and 4 (0.061 g, 0.36 mmol) were dissolved in DCM (1.7 mL) and DMF (4 mL), and then 4-dimethylaminopyridine (0.10 g, 0.84 mmol) and EDCI (0.081 g, 0.42 mmol) were slowly added. The reaction solution was stirred at room temperature for 16 hours. The reaction solution was concentrated to remove dichloromethane, diluted with 20 mL of water, and extracted with ethyl acetate (20 mL × 3). The organic phases were combined, washed with sodium chloride aqueous solution (20 mL × 3), dried over anhydrous sodium sulphate, and filtered, and the filtrate was concentrated under reduced pressure. The crude product was separated by silica gel Prep-TLC (EA:PE=1:3) to afford the less polar isomer A0144 (Rf=0.4) and the more polar isomer A0130 (Rf=0.3). Further purification by preparative HPLC and lyophilization yielded the more polar product A0130 (11.53 mg, yield: 7.15%) and the less polar product A0144 (9.14 mg, yield: 5.76%).

A0130: MS *m*/*z* (ESI): 364.0 [M+1]⁺.

¹H NMR (400 MHz, Chloroform-d) *δ* 7.64-7.58 (m, 4H), 7.53 (t, *J =* 7.6 Hz, 1H), 7.36-7.29 (m, 2H), 4.92-4.82 (m, 1H), 4.43-4.17 (m, 2H), 3.95-3.75 (m, 5H), 2.98-2.91 (m, 1H), 2.70-2.63 (m, 4H).

A0144: MS *m*/*z* (ESI): 364.0 [M+1]⁺.

¹H NMR (400 MHz, Chloroform-d) *δ* 7.64-7.61 (m, 4H), 7.53 (t, *J =* 7.6 Hz, 1H), 7.34 (d, *J =* 7.6 Hz, 1H), 7.29 (d, *J =* 7.6 Hz, 1H), 4.92-4.81 (m, 1H), 4.47-4.16 (m, 2H), 4.00-3.72 (m, 5H), 2.99-2.68 (m, 2H), 2.62 (s, 3H).

### Examples A0131&A0153

### 5-(2, 3-Dimethylphenyl)-2-[(2S, 4Z)-2-(hydroxymethyl)-4-(methoxyimino)pyrrolidine-1-carbonyl]benzonitrile

### 5-(2, 3-Dimethylphenyl)-2-[(2S, 4E)-2-(hydroxymethyl)-4-(methoxyimino)pyrrolidine-1-carbonyl]benzonitrile

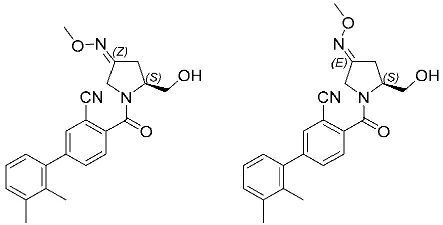

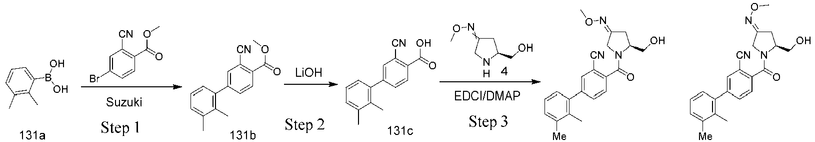

### Step 1

### Methyl 2-cyano-4-(2,3-dimethylphenyl)benzoate 131b

131a (0.200 g, 1.33 mmol), methyl 4-bromo-2-cyanobenzoate (0.30 g, 1.26 mmol), Pd(dppf)Cl₂ (0.11 g, 0.13 mmol), and potassium carbonate (0.37 g, 2.66 mmol) were dissolved in 1,4-dioxane (4 mL), and the mixture was reacted at 100°C under argon protection for 12 hours. The reaction solution was directly concentrated and purified by column chromatography to afford 131b (0.300 g, yield: 67.8%).

MS *m*/*z* (ESI): 266.0 [M+1]⁺.

### Step 2

### 2-Cyano-4-(2,3-dimethylphenyl)benzoic acid 131c

131b (0.200 g, 0.75 mmol) and lithium hydroxide (0.094 g, 2.25 mmol) were dissolved in methanol (1 mL), water (1 mL) and tetrahydrofuran (1 mL), and the mixture was reacted at 40°C for 1-2 hours. The reaction solution was adjusted to pH=3-4 with 1M hydrochloric acid, and extracted with ethyl acetate (10 mL × 3). The organic phases were combined, dried, and concentrated to afford crude 131c (0.210 g).

MS *m*/*z* (ESI): 252.1 [M+1]⁺.

### Step 3

### 5-(2, 3-Dimethylphenyl)-2-[(2S, 4Z)-2-(hydroxymethyl)-4-(methoxyimino)pyrrolidine-1-carbonyl]benzonitrile

### 5-(2, 3-Dimethylphenyl)-2-[(2S, 4E)-2-(hydroxymethyl)-4-(methoxyimino)pyrrolidine-1-carbonyl]benzonitrile

131c (0.080 g, 0.32 mmol), 4 (0.082 g, 0.32 mmol), EDCI (0.074 g, 0.38 mmol), and 4-dimethylaminopyridine (0.078 g, 0.64 mmol) were dissolved in *N,N-*dimethylformamide (0.7 mL) and dichloromethane (0.3 mL), and the mixture was reacted at 15°C for 2-12 hours. 10 mL of water was added, and the mixture was extracted with dichloromethane (10 mL × 3). The organic phases were combined, washed with sodium chloride aqueous solution (10 mL × 3), dried over anhydrous sodium sulphate, and filtered, and the filtrate was concentrated under reduced pressure. The crude product was separated by silica gel Prep-TLC (PE:EA=1:2) to afford the less polar isomer A0153 (Rf=0.25) and the more polar isomer A0131 (Rf=0.2). Further purification by preparative HPLC and lyophilization yielded the more polar product A0131 (9.52 mg, yield: 7.32%) and A0153 (4.51 mg, yield: 3.68%).

A0131: MS *m*/*z* (ESI): 378.3 [M+1]⁺.

¹H NMR (400 MHz, Chloroform-d) *δ* 7.67-7.56 (m, 3H), 7.25-7.17 (m, 2H), 7.04 (d, *J =* 7.2 Hz, 1H), 4.87-4.83 (m, 1H), 4.37-4.33 (m, 1H), 4.28-3.75 (m, 6H), 3.28-3.12 (m, 1H), 3.15-2.95 (m, 1H), 2.84-2.80 (m, 1H), 2.36 (s, 3H), 2.15 (s, 3H).

A0153: MS *m*/*z* (ESI): 378.3 [M+1]⁺.

¹H NMR (400 MHz, Chloroform-*d*) *δ* 7.66-7.56 (m, 3H), 7.25-7.17 (m, 2H), 7.04-6.95 (m, 1H), 4.89-4.83 (m, 1H), 4.39-4.33 (m, 1H), 4.20-3.70 (m, 6H), 3.30-3.12 (m, 1H), 3.02-2.83 (m, 2H), 2.36 (s, 3H), 2.12 (s, 3H).

### Examples A0137&A0154

### [(2S, 4Z)-1-(5-(2,3-Dimethylphenyl)-6-(trifluoromethyl)pyrazine-2-carbonyl)-4-(methoxyimino)pyrrolidin-2-yl]methanone

### [(2S, 4E)-1-(5-(2,3-Dimethylphenyl)-6-(trifluoromethyl)pyrazine-2-carbonyl)-4-(methoxyimino)pyrrolidin-2-yl]methanone

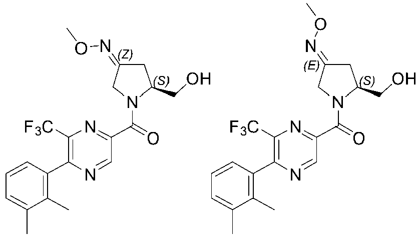

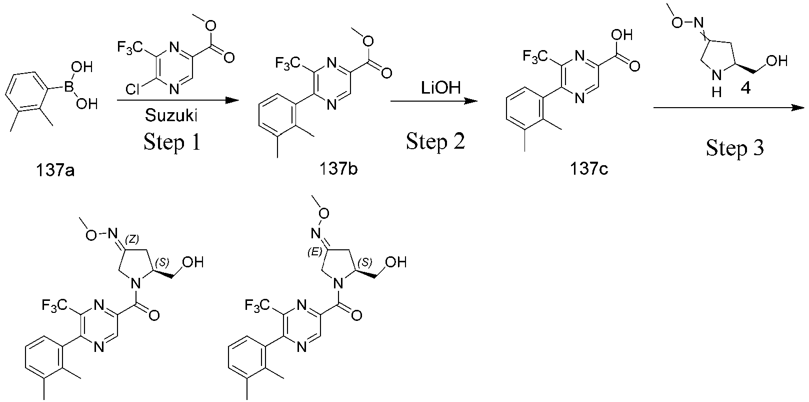

According to the aforementioned synthetic route, with reference to the synthetic method of compound A0131, the more polar product A0154 (15.93 mg, yield: 11.8%) and A0137 (4.71 mg, yield: 3.48%) were obtained.

A0137: MS *m*/*z* (ESI): 423.3 [M+1]⁺.

¹H NMR (400 MHz, Chloroform-*d*) *δ* 9.48 (d, *J =* 16.4 Hz, 1H), 7.33-7.26 (m, 1H), 7.22-7.20 (m, 1H), 7.03-7.01 (m, 1H), 5.14-4.32 (m, 3H), 3.93-3.80 (m, 5H), 3.03-2.73 (m, 2H), 2.45 (s, 1H), 2.35 (s, 3H), 1.98 (s, 3H).

A0154: MS *m*/*z* (ESI): 423.3 [M+1]⁺.

¹H NMR (400 MHz, Chloroform-*d*) *δ* 9.49 (d, *J =* 7.2 Hz, 1H), 7.32-7.26 (m, 1H), 7.22-7.15 (m, 1H), 7.02-7.00 (m, 1H), 5.20-4.25 (m, 3H), 3.99-3.78 (m, 5H), 3.03-2.83 (m, 2H), 2.35 (s, 3H), 1.97 (s, 3H).

### Examples A0147&A0170

### (S, Z)-(2', 3'-Dimethyl-2-(oxetan-3-yloxy)-[1, 1'-biphenyl]-4-yl)(2-(hydroxymethyl)-4-(methoxyimino)pyrrolidin-1-yl)methanone

### (S, E)-(2', 3'-Dimethyl-2-(oxetan-3-yloxy)-[1, 1'-biphenyl]-4-yl)(2-(hydroxymethyl)-4-(methoxyimino)pyrrolidin-1-yl)methanone

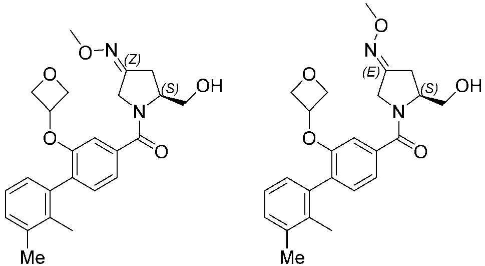

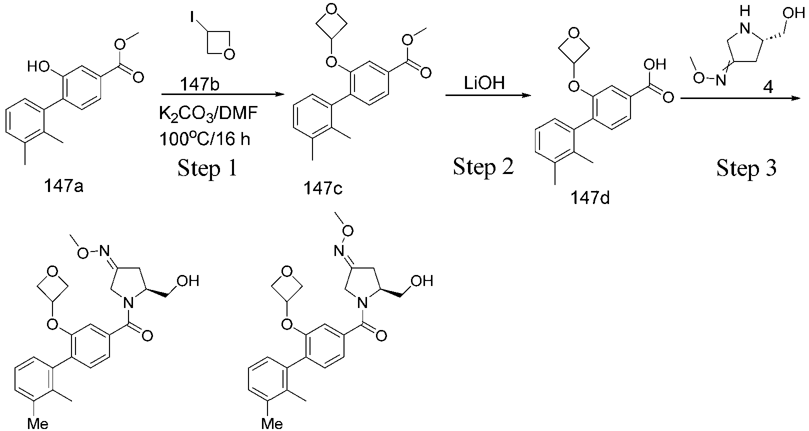

With reference to the synthetic method of Step 1 (131b) in Examples A0131&A0153, by replacing the starting material methyl 4-bromo-2-cyanobenzoate with methyl 4-bromo-3-hydroxybenzoate, 147a was obtained.

### Step 1

### Methyl 2', 3'-dimethyl-2-(oxetan-3-yloxy)-[1, 1'-biphenyl]-4-carboxylate 147c

147a (300 mg, 1.17 mmol), 147b (0.22 g, 1.17 mmol), and potassium carbonate (0.49 g, 3.51 mmol) were mixed in DMF (3 mL) under argon protection. The reaction solution was reacted at 100°C for 16 hours. Ethyl acetate was added to the reaction solution, and the mixture was washed with water. The organic phase was dried over anhydrous sodium sulphate and concentrated, and the crude product was purified by column chromatography (ethyl acetate:petroleum ether = 1:5) to afford 147c (290 mg, yield: 47.6%).

MS *m*/*z* (ESI): 313.0 [M+1]⁺.

### Step 2

### 2', 3'-Dimethyl-2-(oxetan-3-yloxy)-[1, 1'-biphenyl]-4-carboxylic acid 147d

147c (290 mg, 0.56 mmol) was dissolved in methanol (3 mL), THF (3 mL) and water (3 mL), lithium hydroxide (0.067 g, 2.80 mmol) was added, and the reaction solution was stirred at 40°C for 2 hours. The reaction solution was adjusted to pH 4-5 with 1N hydrochloric acid, and extracted with ethyl acetate. The organic phase was dried over anhydrous sodium sulphate and concentrated to afford crude 147d (260 mg).

MS *m*/*z* (ESI): 299.0 [M+1]⁺.

### Step 3

### (S, Z)-(2', 3'-Dimethyl-2-(oxetan-3-yloxy)-[1, 1'-biphenyl]-4-yl)(2-(hydroxymethyl)-4-(methoxyimino)pyrrolidin-1-yl)methanone

### (S, E)-(2', 3'-Dimethyl-2-(oxetan-3-yloxy)-[1, 1'-biphenyl]-4-yl)(2-(hydroxymethyl)-4-(methoxyimino)pyrrolidin-1-yl)methanone

147d (260 mg, 0.52 mmol) and 4 (0.061 g, 0.36 mmol) were dissolved in DCM (3 mL) and DMF (7 mL), and then 4-dimethylaminopyridine (0.13 g, 1.04 mmol) and EDCI (0.10 g, 0.52 mmol) were slowly added. The reaction solution was stirred at room temperature for 16 hours. The reaction solution was concentrated to remove dichloromethane, diluted with 30 mL of water, and extracted with ethyl acetate (30 mL × 3). The organic phases were combined, washed with sodium chloride aqueous solution (30 mL × 3), dried over anhydrous sodium sulphate, and filtered, and the filtrate was concentrated under reduced pressure. The crude product was separated by silica gel Prep-TLC (EA:PE=2:1) to afford the less polar isomer A0170 (Rf=0.3) and the more polar isomer A0147 (Rf=0.2). Further purification by preparative HPLC and lyophilization yielded the more polar product A0147 (60.71 mg, yield: 27.1%) and the less polar product A0170 (58.91 mg, yield: 26.0%).

A0147: MS *m*/*z* (ESI): 425.0 [M+1]⁺.

¹H NMR (400 MHz, DMSO-*d*₆) *δ* 7.21-7.12 (m, 4H), 7.02-6.97 (m, 1H), 6.80-6.76 (m, 1H), 5.32-5.25 (m, 1H), 5.10-4.93 (m, 1H), 4.90-4.80 (m, 2H), 4.62-4.52 (m, 1H), 4.53-4.27 (m, 3H), 4.15-3.92 (m, 1H), 3.87-3.72 (m, 3H), 3.62-3.48 (m, 1H), 3.28-3.15 (m, 1H), 2.98-2.72 (m, 1H), 2.68-2.58 (m, 1H), 2.30 (s, 3H), 2.02 (s, 3H).

A0170: MS *m*/*z* (ESI): 425.0 [M+1]⁺.

¹H NMR (400 MHz, DMSO-d6) δ 7.20-7.15 (m, 4H), 7.01-6.99 (m, 1H), 6.85-6.72 (m, 1H), 5.45-5.23 (m, 1H), 5.08-4.94 (s, 1H), 4.91-4.82 (m, 2H), 4.65-3.92 (m, 5H), 3.88-3.72 (m, 3H), 3.68-3.45 (m, 1H), 3.30-3.15 (m, 1H), 2.82-2.65 (m, 2H), 2.30 (s, 3H), 2.02 (s, 3H).

### Examples A0149&A0171

### (S, Z)-(2-(Hydroxymethyl)-4-(methoxyimino)pyrrolidin-1-yl)(2-(2-methoxyethoxy)-2', 3'-dimethyl-[1,1'-biphenyl]-4-yl)methanone

### (S, E)-(2-(Hydroxymethyl)-4-(methoxyimino)pyrrolidin-1-yl)(2-(2-methoxyethoxy)-2', 3'-dimethyl-[1,1'-biphenyl]-4-yl)methanone

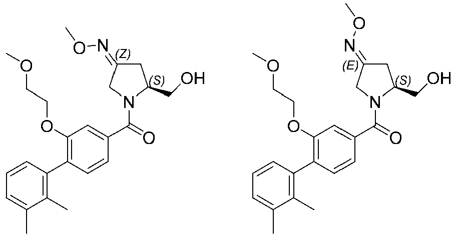

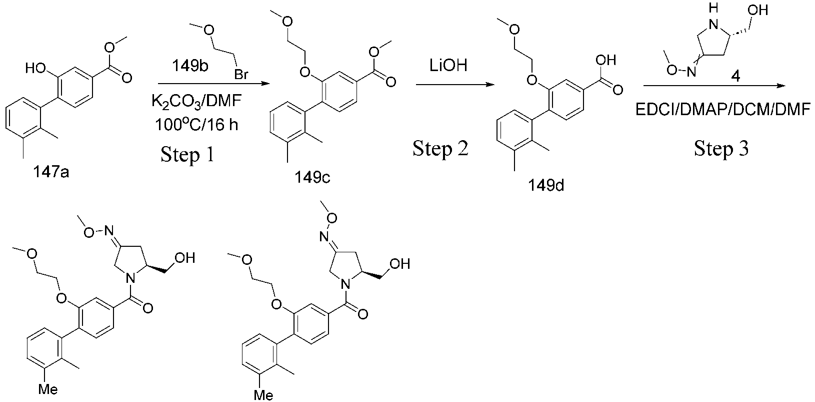

According to the aforementioned synthetic route, with reference to the synthetic method of compound A0147, the less polar isomer A0171 (Rf=0.35) and the more polar isomer A0149 (Rf=0.3) were obtained. Further purification by preparative HPLC and lyophilization yielded the more polar product A0149 (38.84 mg, yield: 9.05%) and the less polar product A0171 (42.95 mg, yield: 9.99%).

A0149: MS *m*/*z* (ESI): 427.1 [M+1]⁺.

¹H NMR (400 MHz, DMSO-*d*₆) *δ* 7.25-7.09 (m, 5H), 6.97-6.96 (m, 1H), 5.03-4.99 (m, 1H), 4.62-4.52 (m, 1H), 4.42-4.30 (m, 1H), 4.20-3.93 (m, 3H), 3.85-3.70 (m, 3H), 3.62-3.48 (m, 3H), 3.30-3.22 (m, 1H), 3.15 (s, 3H), 3.01-2.79 (m, 1H), 2.70-2.55 (m, 1H), 2.27 (s, 3H), 1.97 (s, 3H).

A0171: MS *m*/*z* (ESI): 427.1 [M+1]⁺.

¹H NMR (400 MHz, DMSO-*d*₆) *δ* 7.36-7.07 (m, 5H), 7.00-6.95 (m, 1H), 5.05-4.95 (m, 1H), 4.61-4.20 (m, 2H), 4.12-3.92 (m, 3H), 3.85-3.72 (m, 3H), 3.65-3.45 (m, 3H), 3.31-3.22 (m, 1H), 3.14 (s, 3H), 2.82-2.70 (m, 2H), 2.27 (s, 3H), 1.94 (s, 3H).

### Examples A0148&A0169

### (S, Z)-(2-(2-Hydroxyethoxy)-2',3'-dimethyl-[1,1'-biphenyl]-4-yl)(2-(hydroxymethyl)-4-(methoxyimino)pyrrolidin-1-yl)methanone

### (S, E)-(2-(2-Hydroxyethoxy)-2',3'-dimethyl-[1,1'-biphenyl]-4-yl)(2-(hydroxymethyl)-4-(methoxyimino)pyrrolidin-1-yl)methanone

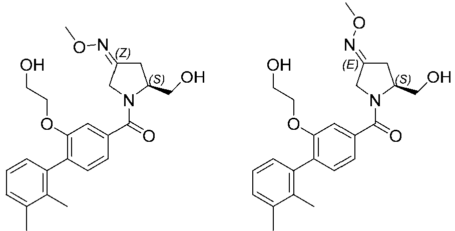

With reference to Examples A0149&A0171, 149b was replaced with 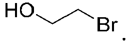 The crude product was separated by silica gel Prep-TLC (EA:PE=2:1) to afford the less polar isomer A0169 (Rf=0.25) and the more polar isomer A0148 (Rf=0.2). Further purification by preparative HPLC and lyophilization yielded the more polar product A0148 (61.98 mg, yield: 15.87%) and the less polar product A0169 (60.43 mg, yield: 15.39%).

A0148: MS *m*/*z* (ESI): 413.0 [M+1]⁺.

¹H NMR (400 MHz, DMSO-*d*₆) *δ* 7.22-7.09 (m, 5H), 6.98-6.96 (m, 1H), 5.08-4.95 (m, 1H), 4.78-4.72 (m, 1H), 4.62-4.12 (m, 2H), 4.10-3.92 (m, 3H), 3.75 (d, *J =* 34.0 Hz, 3H), 3.62-3.52 (m, 3H), 3.30-3.20 (m, 1H), 3.02-2.78 (m, 1H), 2.68-2.58 (m, 1H), 2.24 (s, 3H), 1.98 (s, 3H).

A0169: MS *m*/*z* (ESI): 413.0 [M+1]⁺.

¹H NMR (400 MHz, DMSO-*d*₆) *δ* 7.21-7.09 (m, 5H), 6.97 (d, *J =* 6.8 Hz, 1H), 5.05-4.97 (m, 1H), 4.76-4.72 (m, 1H), 4.63-4.14 (m, 2H), 4.10-3.92 (m, 3H), 3.78 (s, 3H), 3.76-3.45 (m, 3H), 3.30-3.21 (m, 1H), 2.82-2.69 (m, 2H), 2.28 (s, 3H), 1.98 (s, 3H).

### Examples A0157&A0179

### (S, Z)-4-(2-(Hydroxymethyl)-4-(methoxyimino)pyrrolidine-1-carbonyl)-2', 3'-dimethyl-N-(oxetan-3-yl)-[1,1'-biphenyl]-2-carboxamide

### (S, E)-4-(2-(Hydroxymethyl)-4-(methoxyimino)pyrrolidine-1-carbonyl)-2', 3'-dimethyl-N-(oxetan-3-yl)-[1,1'-biphenyl]-2-carboxamide

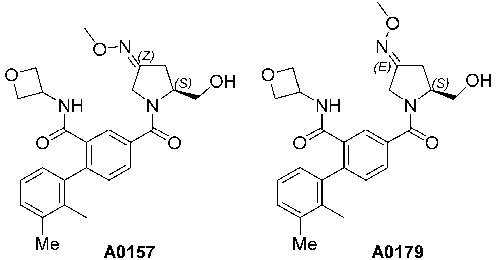

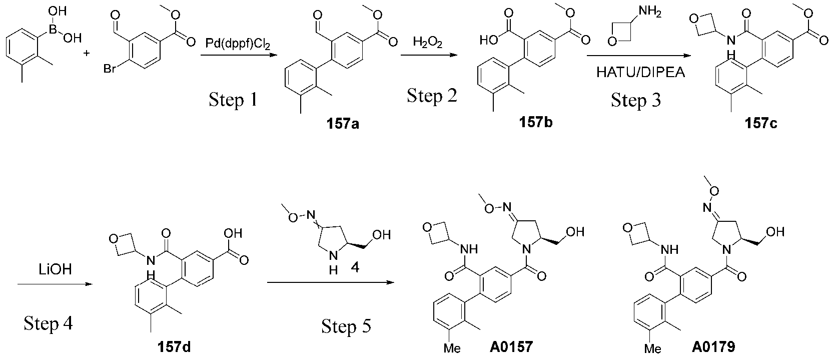

### Step 1

### Methyl 4-(2,3-dimethylphenyl)-3-formylbenzoate 157a

Methyl 4-bromo-3-formylbenzoate (3 g, 12.34 mmol), 2,3-dimethylphenylboronic acid (2.78 g, 18.51 mmol), [1,1-bis(diphenylphosphino)ferrocene]palladium(II) dichloride (0.90 g, 1.23 mmol), and potassium carbonate (5.12 g, 37.02 mmol) were mixed in 1,4-dioxane (30 mL) and water (5 mL) under argon protection. The reaction solution was stirred at 100°C for 16 hours. The reaction solution was diluted with ethyl acetate, and washed with saturated sodium chloride aqueous solution and saturated sodium bicarbonate, successively. The organic phase was dried over anhydrous sodium sulphate and concentrated, and the crude product was purified by column chromatography (petroleum ether/ethyl acetate = 5:1) to afford product 157a (2.0 g, yield: 54.35%). MS *m*/*z* (ESI): 269.2 [M+1]⁺.

### Step 2

### Methyl 4-(2,3-dimethylphenyl)-3-carboxybenzoate 157b

The starting material methyl 4-(2,3-dimethylphenyl)-3-formylbenzoate 157a (1 g, 3.73 mmol, purity: 100%) was dissolved in acetonitrile (10 mL) and water (10 mL), and then sodium dihydrogen phosphate monohydrate (1.54 g, 11.19 mmol), hydrogen peroxide (0.13 g, 3.73 mmol) and sodium chlorite (1.86 g, 20.52 mmol) were added. The mixture was reacted at room temperature for 16 hours. 20 ml of water was added, and the mixture was extracted with ethyl acetate (30 mL × 3). The organic phases were combined, washed with sodium chloride aqueous solution (30 mL × 3), dried over anhydrous sodium sulphate, and filtered, and the filtrate was concentrated under reduced pressure to afford a residue, which was purified by silica gel column chromatography to afford product methyl 4-(2,3-dimethylphenyl)-3-carboxybenzoate 157b (650 mg, yield: 61.34%). MS *m*/*z* (ESI): 285.3 [M+1]⁺.

### Step 3

### Methyl 4-(2,3-dimethylphenyl)-3-[(oxetan-3-yl)carbamoyl]benzoate 157c

157a (100 mg, 0.35 mmol) was dissolved in DMF (2 mL), and then HATU (0.16 g, 0.42 mmol) and ethyldiisopropylamine (0.18 g, 1.4 mmol) were added, and the mixture was reacted at room temperature for 30 minutes. 3-Oxetanamine (0.031 g, 0.42 mmol) was then added, and the reaction solution was reacted at room temperature for 5 hours. Then, 20 mL of water was added, and the mixture was extracted with ethyl acetate (20 mL × 2). The organic phases were combined, washed with sodium chloride aqueous solution (10 mL × 2), dried over anhydrous sodium sulphate, and filtered, and the filtrate was concentrated under reduced pressure to afford a residue, which was purified by silica gel column chromatography to afford product 157c (104 mg, yield: 87.1%).

MS *m*/*z* (ESI): 340.0 [M+1]⁺.

### Step 4

### 2', 3'-Dimethyl-2-(oxetan-3-ylcarbamoyl)-[1,1'-biphenyl]-4-carboxylic acid 157d

157c (104 mg, 0.31 mmol) was dissolved in THF (2 mL), methanol (2 mL) and water (1 mL), and then lithium hydroxide hydrate (0.039 g, 0.93 mmol) was added, and the reaction solution was reacted at room temperature for 16 hours. Then, 20 mL of water was added, and the mixture was extracted with ethyl acetate (20 mL × 2). The organic phases were combined, washed with sodium chloride aqueous solution (10 mL × 3), dried over anhydrous sodium sulphate, and filtered, and the filtrate was concentrated under reduced pressure to afford a residue, which was purified by silica gel column chromatography to afford product 157d (70 mg, yield: 70.0%).

MS *m*/*z* (ESI): 326.2 [M+1]⁺.

### Step 5

### (S, Z)-4-(2-(Hydroxymethyl)-4-(methoxyimino)pyrrolidine-1-carbonyl)-2', 3'-dimethyl-N-(oxetan-3-yl)-[1,1'-biphenyl]-2-carboxamide

### (S, E)-4-(2-(Hydroxymethyl)-4-(methoxyimino)pyrrolidine-1-carbonyl)-2', 3'-dimethyl-N-(oxetan-3-yl)-[1,1'-biphenyl]-2-carboxamide

157d (75 mg, 0.23 mmol) and 4 (0.036 g, 0.25 mmol) were dissolved in dichloromethane (7 mL) and DMF (3 mL), and then EDCI (0.044 g, 0.23 mmol) and 4-dimethylaminopyridine (0.056 g, 0.46 mmol) were added, and the reaction solution was reacted at room temperature for 16 hours. 10 mL of water was added, and the mixture was extracted with dichloromethane (10 mL × 3). The organic phases were combined, washed with sodium chloride aqueous solution (10 mL × 3), dried over anhydrous sodium sulphate, and filtered, and the filtrate was concentrated under reduced pressure. The crude product was separated by silica gel Prep-TLC (PE:EA=1:2) to afford the less polar isomer A0179 (Rf=0.25) and the more polar isomer A0157 (Rf=0.2). Further purification by preparative HPLC and lyophilization yielded the more polar product A0157 (4.08 mg, yield: 3.69%) and the less polar product A0179 (0.72 mg, yield: 0.59%).

A0157: MS *m*/*z* (ESI): 452.0 [M+1]⁺.

¹H NMR (400 MHz, Chloroform-*d*) *δ* 8.11-8.09 (m, 1H), 7.72-7.65 (m, 1H), 7.35-7.26 (m, 2H), 7.25-7.22 (m, 1H), 7.13-7.05 (m, 1H), 6.06-5.88 (m, 1H), 5.00-4.82 (m, 1H), 4.74-4.69 (m, 2H), 4.42-4.11 (m, 2H), 3.98-3.74 (m, 8H), 2.98-2.91 (m, 1H), 2.70-2.65 (m, 1H), 2.35 (d, *J =* 3.2 Hz, 3H), 2.07-1.98 (m, 3H).

A0179: MS *m*/*z* (ESI): 452.0 [M+1]⁺.

¹H NMR (400 MHz, Chloroform-*d*) *δ* 8.10-8.09 (m, 1H), 7.70-7.62 (m, 1H), 7.35-7.27 (m, 2H), 7.25-7.22 (m, 1H), 7.10-7.05 (m, 1H), 5.82-5.75 (m, 1H), 5.00-4.82 (m, 1H), 4.73-4.68 (m, 2H), 4.42-4.11 (m, 2H), 3.98-3.75 (m, 8H), 2.99-2.88 (m, 1H), 2.72-2.58 (m, 1H), 2.40-2.28 (m, 3H), 2.07-1.98 (m, 3H).

### Examples A0158&A0180

### (2S, 4Z)-(2', 3'-Dimethyl-2-(morpholine-4-carbonyl)-[1, 1'-biphenyl]-4-yl)(2-(hydroxymethyl)-4-(methoxyimino)pyrrolidin-1-yl)methanone

### (2S, 4E)-(2', 3'-Dimethyl-2-(morpholine-4-carbonyl)-[1, 1'-biphenyl]-4-yl)(2-(hydroxymethyl)-4-(methoxyimino)pyrrolidin-1-yl)methanone

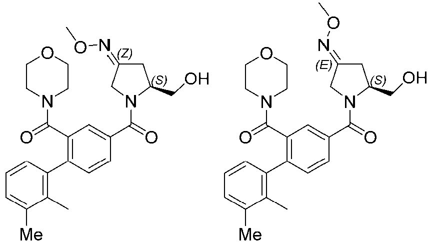

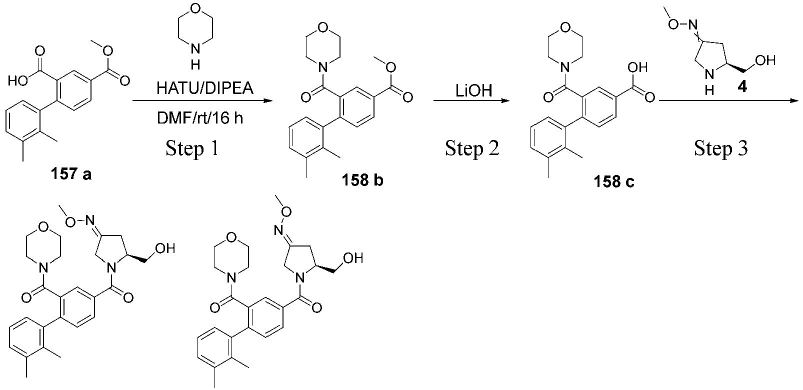

According to the aforementioned synthetic route, with reference to the synthetic method of compound A0157, the less polar isomer A0180 (Rf=0.25) and the more polar isomer A0158 (Rf=0.2) were obtained. Further separation and purification by preparative chromatography yielded the more polar product A0158 (10.74 mg, yield: 6.17%) and the less polar product A0180 (9.01 mg, yield: 5.29%).

A0158: MS *m*/*z* (ESI): 465.9[M+1]⁺.

¹H NMR (400 MHz, Chloroform-*d*) *δ* 7.65-7.52 (m, 2H), 7.37-7.33 (m, 1H), 7.22-7.12 (m, 3H), 4.98-4.87 (m, 1H), 4.49-4.27 (m, 2H), 3.95-3.72 (m, 5H), 3.67-3.25 (m, 4H), 3.12-2.58 (m, 6H), 2.40-2.33 (m, 3H), 2.15-2.03 (m, 3H).

A0180: MS *m*/*z* (ESI): 465.9[M+1]⁺.

¹H NMR (400 MHz, Chloroform-*d*) *δ* 7.64-7.51 (m, 2H), 7.38-7.32 (m, 1H), 7.19-7.13 (m, 3H), 4.92-4.83 (m, 1H), 4.42-4.17 (m, 2H), 3.98-3.80 (m, 5H), 3.72-3.22 (m, 4H), 3.18-2.50 (m, 6H), 2.38-2.30 (m, 3H), 2.15-2.02 (m, 3H).

### Examples A0191&A0199

### (S, Z)-4'-(2-(Hydroxymethyl)-4-(methoxyimino)pyrrolidine-1-carbonyl)-2'-(2-methoxyethoxy)-2-methyl-[1, 1'-biphenyl]-3-carbonitrile

### (S, E)-4'-(2-(Hydroxymethyl)-4-(methoxyimino)pyrrolidine-1-carbonyl)-2'-(2-methoxyethoxy)-2-methyl-[1, 1'-biphenyl]-3-carbonitrile

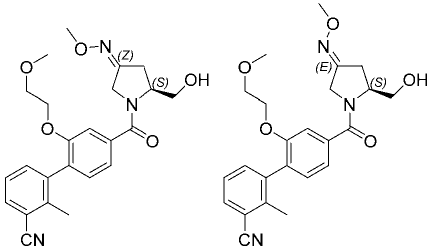

With reference to Examples A0149&A0171, 149a was replaced with 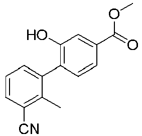 (prepared with reference to the method of 147a). The crude product was separated by silica gel Prep-TLC (PE:EA=1:3) to afford the less polar isomer A0199 (Rf=0.25) and the more polar isomer A0191 (Rf=0.2). Further purification by preparative HPLC and lyophilization yielded the more polar product A0191 (12.1 mg, yield: 2.76%) and the less polar product A0199 (9.17 mg, yield: 2.14%).

A0191: MS *m*/*z* (ESI): 438.0 [M+1]⁺.

¹H NMR (400 MHz, Chloroform-d) *δ* 7.64-7.62 (m, 1H), 7.41-7.39 (m, 1H), 7.34-7.26 (m, 1H), 7.18-7.14 (m, 3H), 4.92-4.82 (m, 1H), 4.48-4.05 (m, 4H), 3.98-3.78 (m, 5H), 3.60-3.57 (m, 2H), 3.26 (s, 3H), 2.98-2.92 (m, 1H), 2.67-2.63 (m, 1H), 2.35 (s, 3H).

A0199: MS *m*/*z* (ESI): 438.0 [M+1]⁺.

¹H NMR (400 MHz, Chloroform-d) *δ* 7.64-7.61 (m, 1H), 7.38-7.36 (m, 1H), 7.34-7.26 (m, 1H), 7.17-7.14 (m, 3H), 4.92-4.82 (m, 1H), 4.52-4.05 (m, 4H), 3.98-3.68 (m, 5H), 3.59-3.57 (m, 2H), 3.26 (s, 3H), 2.98-2.55 (m, 2H), 2.35 (s, 3H).

### Examples A0215&A0222

### 3-(6-[(2S, 4Z)-2-(Hydroxymethyl)-4-(methoxyimino)pyrrolidine-1-carbonyl]-2-methoxypyridin-3-yl)-2-methylbenzonitrile

### 3-(6-[(2S, 4E)-2-(Hydroxymethyl)-4-(methoxyimino)pyrrolidine-1-carbonyl]-2-methoxypyridin-3-yl)-2-methylbenzonitrile

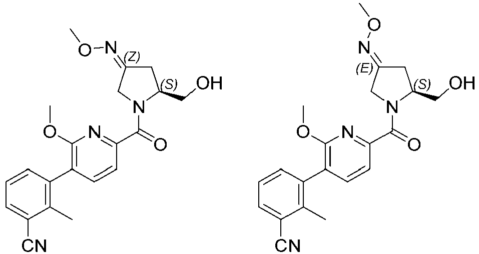

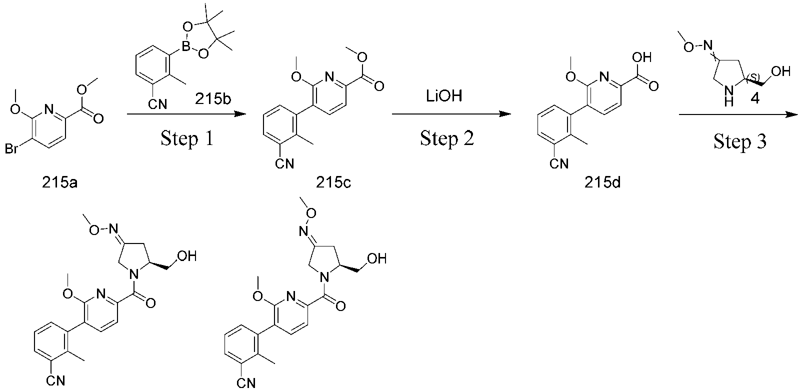

According to the aforementioned synthetic route, with reference to the synthetic method of compound C0061, the less polar isomer A0222 (Rf=0.25) and the more polar isomer A0215 (Rf=0.2) were obtained. Further purification by preparative HPLC and lyophilization yielded the more polar product A0215 (8.93 mg, yield: 6.56%) and the less polar product A0222 (7.91 mg, yield: 5.68%).

A0215: MS *m*/*z* (ESI): 395.0 [M+1]⁺.

¹H NMR (400 MHz, Chloroform-d) *δ* 7.73-7.57 (m, 3H), 7.38-7.26 (m, 2H), 5.20-4.75 (m, 3H), 4.02-3.72 (m, 8H), 2.97-2.91 (m, 1H), 2.69-2.62 (m, 1H), 2.35 (s, 3H).

A0222: MS *m*/*z* (ESI): 395.0 [M+1]⁺.

¹H NMR (400 MHz, Chloroform-*d*) *δ* 7.73-7.66 (m, 2H), 7.60-7.57 (m, 1H), 7.40-7.34 (m, 2H), 5.20-4.75 (m, 3H), 4.00-3.72 (m, 8H), 2.99-2.63 (m, 2H), 2.35 (s, 3H).

### Examples A0216&A0223

### 3-(6-[(2S, 4Z)-2-(Hydroxymethyl)-4-(methoxyimino)pyrrolidine-1-carbonyl]-4-methoxypyridin-3-yl)-2-methylbenzonitrile

### 3-(6-[(2S, 4E)-2-(Hydroxymethyl)-4-(methoxyimino)pyrrolidine-1-carbonyl]-4-methoxypyridin-3-yl)-2-methylbenzonitrile

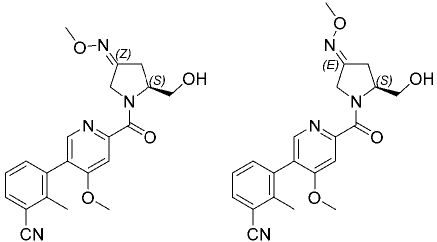

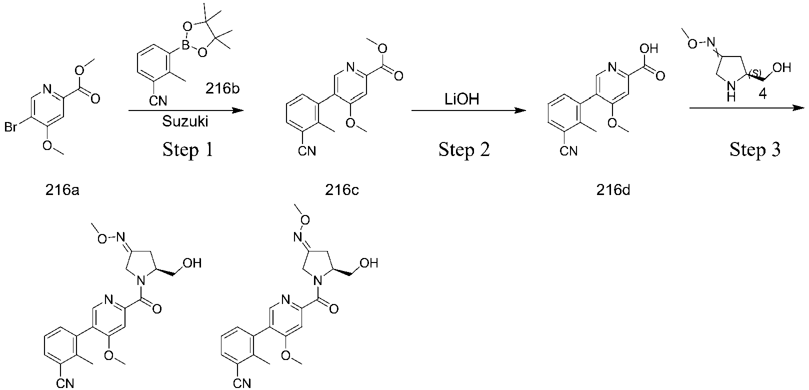

According to the aforementioned synthetic route, with reference to the synthetic method of compound C0061, the more polar product A0216 (5.53 mg, yield: 4.71%) and the less polar product A0223 (3.47 mg, yield: 2.68%) were obtained.

A0216: MS *m*/*z* (ESI): 395.0 [M+1]⁺.

¹H NMR (400 MHz, Chloroform-d) *δ* 8.26-8.20 (m, 1H), 7.70-7.52 (m, 2H), 7.39-7.35 (m, 2H), 4.95-4.71 (m, 2H), 4.26-4.21 (m, 1H), 3.93-3.90 (m, 6H), 3.80-3.72 (m, 1H), 3.65-3.55 (m, 1H), 2.96-2.82 (m, 1H), 2.70-2.60 (m, 1H), 2.34 (s, 3H).

A0223: MS *m*/*z* (ESI): 395.0 [M+1]⁺.

¹H NMR (400 MHz, Chloroform-*d*) *δ* 8.22-8.19 (m, 1H), 7.72-7.52 (m, 2H), 7.39-7.37 (m, 2H), 4.95-4.88 (m, 2H), 4.88-4.72 (m, 1H), 4.20-4.11 (m, 1H), 3.99-3.85 (m, 6H), 3.80-3.68 (m, 1H), 3.60-3.50 (m, 1H), 2.98-2.65 (m, 2H), 2.33 (s, 3H).

### Examples A0163&A0193

### (S, Z)-3-(8-(2-(Hydroxymethyl)-4-(methoxyimino)pyrrolidine-1-carbonyl)-4-methyl-3-oxo-3,4-dihydro-2H-benzo[b][1,4]oxazin-5-yl)-2-methylbenzonitrile

### (S, E)-3-(8-(2-(Hydroxymethyl)-4-(methoxyimino)pyrrolidine-1-carbonyl)-4-methyl-3-oxo-3,4-dihydro-2H-benzo[b][1,4]oxazin-5-yl)-2-methylbenzonitrile

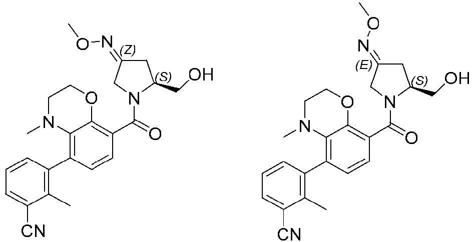

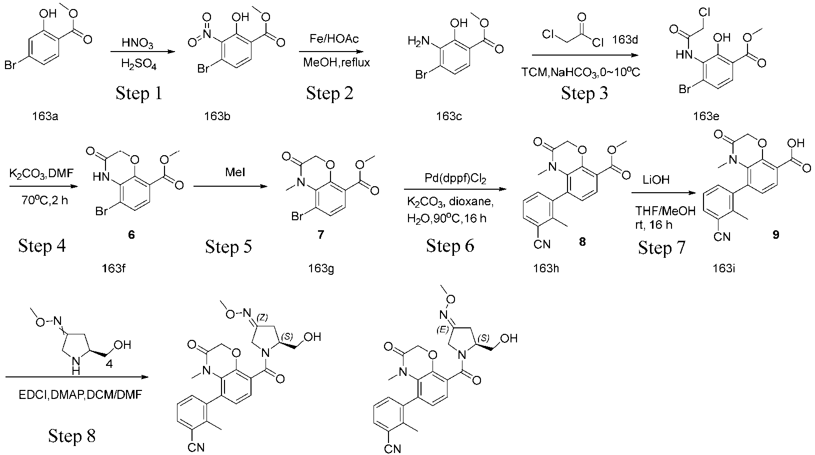

### Step 1

### Methyl 4-bromo-2-hydroxy-3-nitrobenzoate 163b

163a (12 g, 51.94 mmol) was dissolved in concentrated sulphuric acid (30 mL), and the mixture was cooled to 0°C. Concentrated nitric acid (3.6 g, 57.13mmol) was slowly added dropwise, and the reaction solution was reacted at 0°C for 1 hour. The reaction solution was poured into ice water, and the precipitated solid was collected. The filtrate was extracted with ethyl acetate, and the solids were combined and purified by column chromatography (petroleum ether/ethyl acetate = 5:1), to afford 163b (3.0 g, yield: 18.83%).

MS *m*/*z* (ESI): 275.9 [M+1]⁺.

### Step 2

### Methyl 3-amino-4-bromo-2-hydroxybenzoate 163c

Under argon protection, 163b (2.5 g, 9.06 mmol) was dissolved in methanol (10 mL) and acetic acid (10 mL), iron powder (2.53g, 45.3 mmol) was added, and the reaction solution was refluxed for 2 hours. Water was added to the reaction solution, and the mixture was neutralized with saturated sodium bicarbonate, and extracted with ethyl acetate. The organic phase was dried over anhydrous sodium sulphate and concentrated, and the crude product was purified by column chromatography (petroleum ether/ethyl acetate = 4:1), to afford 163c (1 g, yield: 44.87%).

MS *m*/*z* (ESI): 246.0 [M+1]⁺.

### Step 3

### Methyl 4-bromo-3-(2-chloroacetamido)-2-hydroxybenzoate 163e

163c (200 mg, 0.81 mmol) was dissolved in chloroform (5 mL), saturated sodium bicarbonate aqueous solution (0.34 g, 4.05 mmol) was added, and 163d (0.11 g, 0.97 mmol) was slowly added dropwise while controlling the temperature at 0°C-10°C. After the dropwise addition was completed, the reaction solution was stirred at room temperature for 4 hours. Dichloromethane was added to the reaction solution, and the mixture was washed with water. The organic phase was dried over anhydrous sodium sulphate and concentrated, and the crude product was purified by column chromatography (dichloromethane/methanol = 10:1), to afford 163e (150 mg, yield: 57.22%).

MS *m*/*z* (ESI): 322.0 [M+1]⁺.

### Step 4

### Methyl 5-bromo-3-oxo-3,4-dihydro-2H-benzo[b][1,4]oxazine-8-carboxylate 163f

163e (150 mg, 0.47 mmol) was dissolved in DMF (8 mL), potassium carbonate (0.26 g, 1.88 mmol) was added, and the reaction solution was reacted at 70°C for 2 hours. Ethyl acetate was added to the reaction solution, and the mixture was washed with water. The organic phase was dried over anhydrous sodium sulphate and concentrated, to afford 163f (100 mg, yield: 67.65%).

MS *m*/*z* (ESI): 286.0 [M+1]⁺.

### Step 5

### Methyl 5-bromo-4-methyl-3-oxo-3,4-dihydro-2H-benzo[b][1,4]oxazine-8-carboxylate 163g

163f(100 mg, 0.47 mmol) was dissolved in DMF (5 mL), potassium carbonate (0.13 g, 0.94 mmol) and iodomethane (133 mg, 0.94 mmol) were added, and the reaction solution was reacted at room temperature for 2 hours. Ethyl acetate was added to the reaction solution, and the mixture was washed with water. The organic phase was dried over anhydrous sodium sulphate and concentrated, to afford 163g (100 mg, yield: 95.3%).

MS *m*/*z* (ESI): 300.0 [M+1]⁺.

According to Step 6 to Step 8 in the aforementioned synthetic route, with reference to the synthetic method of compound A0134, the less polar isomer A0193 (Rf=0.25) and the more polar isomer A0163 (Rf=0.2) were obtained. Further purification by preparative HPLC and lyophilization yielded the more polar product A0163 (7.67 mg, yield: 5.08%) and the less polar product A0193 (30.57 mg, yield: 21.64%).

A0163: MS *m*/*z* (ESI): 449.0 [M+1]⁺.

¹H NMR (400 MHz, Chloroform-d) *δ* 7.69 (dd, *J =* 7.6, 1.2 Hz, 2H), 7.53-7.36 (m, 2H), 7.15 (dd, *J =* 8.0, 1.2 Hz, 1H), 6.96-6.92 (m, 1H), 4.83-4.75 (m, 1H), 4.70-4.56 (m, 2H), 4.30-4.18 (m, 1H), 4.14-4.05 (m, 1H), 3.97-3.91 (m, 1H), 3.84 (s, 3H), 3.81-3.76 (m, 1H), 3.01-2.95 (m, 1H), 2.73-2.62 (m, 4H), 2.44-2.27 (m, 3H).

A0193: MS *m*/*z* (ESI): 449.0 [M+1]⁺.

¹H NMR (400 MHz, Chloroform-*d*) *δ* 7.70 (dd, J = 7.5, 1.2 Hz, 1H), 7.50-7.36 (m, 2H), 7.16 (dd, *J =* 8.0, 1.2 Hz, 1H), 6.97-6.91 (m, 1H), 4.82-4.76 (m, 1H), 4.70-4.55 (m, 2H), 4.30-4.02 (m, 2H), 3.98-3.92 (m, 1H), 3.86 (s, 3H), 3.82-3.77 (m, 1H), 2.98-2.82 (m, 1H), 2.78-2.70 (m, 1H), 2.64 (s, 3H), 2.43-2.26 (m, 3H).

### Examples A0162&A0206

### (S, Z)-3-(8-(2-Hydroxymethyl)-4-(methoxyimino)pyrrolidine-1-carbonyl)-4-methyl-3,4-dihydro- 2H-benzo[b][1,4]oxazin-5-yl)-2-methylbenzonitrile

### (S, E)-3-(8-(2-Hydroxymethyl)-4-(methoxyimino)pyrrolidine-1-carbonyl)-4-methyl-3,4-dihydro- 2H-benzo[b][1,4]oxazin-5-yl)-2-methylbenzonitrile

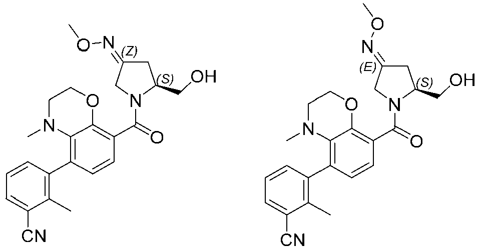

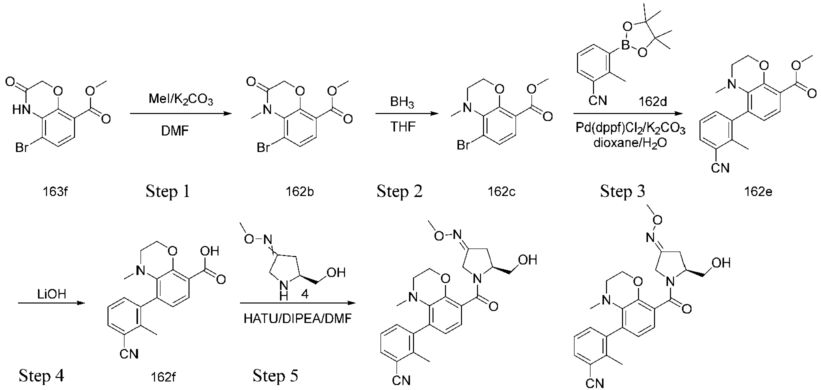

### Step 1

### Methyl 5-bromo-4-methyl-3-oxo-3,4-dihydro- 2H-benzo[b][1,4]oxazine-8-carboxylate 162b

163f(300 mg, 1.05 mmol) was dissolved in DMF (10 mL), potassium carbonate (440 mg, 3.15 mmol) and iodomethane (227 mg, 1.57 mmol) were added, and the reaction solution was reacted at room temperature for 2 hours. Ethyl acetate was added to the reaction solution, and the mixture was washed with water. The organic phase was dried over anhydrous sodium sulphate and concentrated, to afford paleyellow crude product 162b (200 mg, yield: 57.20%).

MS *m*/*z* (ESI): 300.1 [M+1]⁺.

### Step 2

### Methyl 5-bromo-4-methyl-3,4-dihydro- 2H-benzo[b][1,4]oxazine-8-carboxylate 162c

162b (200 mg, 0.67 mmol) was dissolved in THF (10 mL), boranetetrahydrofuran complex (1.01 mL, 1.01 mmol) was added, and the reaction solution was stirred at room temperature for 8 hours. Methanol was added to the reaction solution to quench the reaction solution, and the mixture was concentrated to dryness, to afford crude 162c (180 mg).

MS *m*/*z* (ESI): 286.1 [M+1]⁺.

According to Step 3 to Step 5 in the aforementioned synthetic route, with reference to the synthetic method of compound A0134, the more polar product A0162 (14.54 mg, yield: 9.86%) and the less polar product A0206 (12.12 mg, yield: 8.27%) were obtained.

A0162: MS *m*/*z* (ESI): 435.0 [M+1]⁺.

¹H NMR (400 MHz, Chloroform-*d*) *δ* 7.71-7.61 (m, 1H), 7.59-7.46 (m, 1H), 7.43-7.27 (m, 1H), 7.03-6.94 (m, 1H), 6.76-6.66 (m, 1H), 4.78-4.72 (m, 1H), 4.32-4.02 (m, 4H), 3.87 (d, *J* = 21.2 Hz, 3H), 3.80-3.72 (m, 1H), 3.28-3.19 (m, 2H), 3.04-2.91 (m, 1H), 2.76-2.59 (m, 1H), 2.50-2.18 (m, 7H).

A0206: MS *m*/*z* (ESI): 435.0 [M+1]⁺.

¹H NMR (400 MHz, Chloroform-*d*) *δ* 7.67-7.63 (m, 2H), 7.39-7.34 (m, 1H), 7.05-6.97 (m, 1H), 6.75-6.71 (m, 1H), 4.81-4.76 (m, 1H), 4.35-3.98 (m, 4H), 3.88 (d, *J =* 18.4 Hz, 3H), 3.80-3.70 (m, 1H), 3.32-3.16 (m, 2H), 3.01-2.94 (m, 1H), 2.75-2.62 (m, 1H), 2.46-2.28 (m, 7H).

### Examples A0188&A0230

### (S, Z)-3-(2-(Cyclopropylmethoxy)-6-(2-(hydroxymethyl)-4-(methoxyimino)pyrrolidine-1-carbonyl)pyridin-3-yl)-2-methylbenzonitrile

### (S, E)-3-(2-(Cyclopropylmethoxy)-6-(2-(hydroxymethyl)-4-(methoxyimino)pyrrolidine-1-carbonyl)pyridin-3-yl)-2-methylbenzonitrile

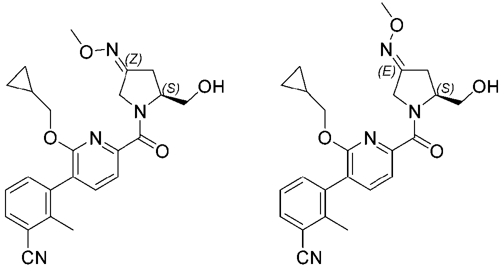

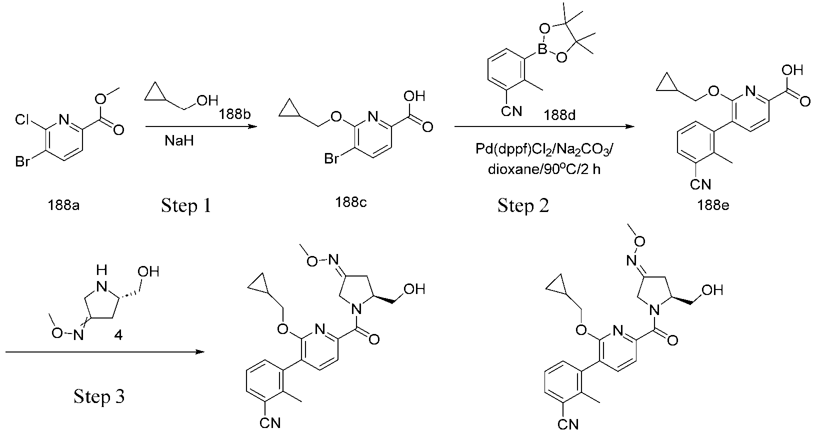

### Step 1

### 5-Bromo-6-(cyclopropylmethoxy)picolinic acid 188c

188b (6.67 g, 87.6 mmol) was slowly added to NaH (0.12 g, 5 mmol) at room temperature, and the mixture was stirred for 10 minutes. 188a (250 mg, 1.0 mmol) was then added, and the reaction solution was reacted at 100°C for 16 hours. The reaction solution was concentrated under reduced pressure, and water (5 mL) was added. The mixture was adjusted to pH 3-4 with 2N hydrochloric acid, and extracted with ethyl acetate (20 mL × 3). The organic phases were combined, dried over anhydrous sodium sulphate, and concentrated, to afford crude 188c (200 mg, yield: 70.03%).

MS *m*/*z* (ESI): 272.0 [M+1]⁺.

According to Step 2 to Step 3 in the aforementioned synthetic route, with reference to the synthetic method of compound C0102, the less polar isomer A0230 (Rf=0.25) and the more polar isomer A0188 (Rf=0.2) were obtained. Further purification by preparative HPLC and lyophilization yielded the more polar product A0188 (3.52 mg, yield: 1.96%) and the less polar product A0230 (7.95 mg, yield: 3.90%).

A0188: MS *m*/*z* (ESI): 435.0 [M+1]⁺.

¹H NMR (400 MHz, Chloroform-*d*) *δ* 7.85-7.65 (m, 2H), 7.62-7.56 (m, 1H), 7.42 -7.32 (m, 2H), 4.97-4.72 (m, 3H), 4.30-4.18 (m, 2H), 3.94-3.70 (m, 5H), 3.10-3.04 (m, 1H), 2.99-2.88 (m, 1H), 2.70-2.58 (m, 1H), 2.38 (s, 3H), 0.90-0.85 (m, 1H), 0.60-0.50 (m, 2H), 0.30-0.20 (m, 2H).

A0230 :MS *m*/*z* (ESI): 435.0 [M+1]⁺.

¹H NMR (400 MHz, Chloroform-*d*) *δ* 7.72-7.53 (m, 3H), 7.40-7.36 (m, 2H), 4.72 -4.54 (m, 1H), 4.23-4.02 (m, 2H), 3.93-3.78 (m, 3H), 3.32-3.18 (m, 4H), 2.95-2.53 (m, 1H), 2.39 (s, 3H), 1.90-1.83 (m, 1H), 1.20-1.12 (m, 1H), 0.60-0.50 (m, 2H), 0.30-0.20 (m, 2H).

### Examples A0190&A0208

### (S, Z)-3-(5-(2-(Hydroxymethyl)-4-(methoxyimino)pyrrolidine-1-carbonyl)-[1,2,4]triazolo[1,5-a]pyridin-8-yl)-2-methylbenzonitrile

### (S, E)-3-(5-(2-(Hydroxymethyl)-4-(methoxyimino)pyrrolidine-1-carbonyl)-[1,2,4]triazolo[1,5-a]pyridin-8-yl)-2-methylbenzonitrile

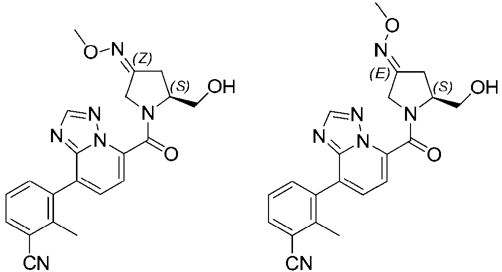

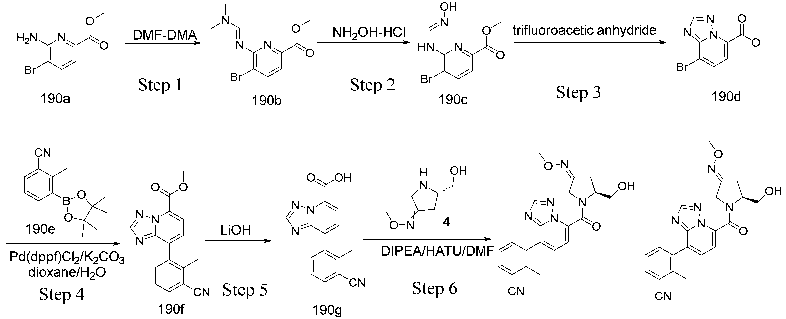

### Step 1

Methyl 5-bromo-6-((dimethylamino)methylene)amino)picolinate 190b 190a (1 g, 4.33 mmol) and DMF-DMA (1.03 g, 8.66 mmol) were dissolved in toluene (20 mL), and the reaction solution was reacted at 110°C for 4 hours. The reaction solution was concentrated to afford red-brown crude product 190b (1.0 g).

MS *m*/*z* (ESI): 287.9 [M+1]⁺.

### Step 2

### Methyl 5-bromo-6-(N'-hydroxycarbamimidoyl)picolinate 190c

190b (1.2 g, 4.19 mmol), hydroxylamine hydrochloride (0.58 g, 8.38 mmol), and sodium acetate (0.69 g, 8.38 mmol) were mixed in ethanol (12 mL) under argon protection, and the reaction solution was stirred at 50°C for 4 hours. The reaction solution was cooled to room temperature to precipitate a white solid, which was filtered to afford crude product 190c (1 g).

MS *m*/*z* (ESI): 273.9 [M+1]⁺.

### Step 3

### Methyl 8-bromo-[1,2,4]triazolo[1,5-a]pyridine-5-carboxylate 190d

190c (1 g, 3.65 mmol) was dissolved in THF (10 mL) at room temperature, trifluoroacetic anhydride (2.30 g, 10.95 mmol) was slowly added dropwise, and then the reaction solution was heated to 75°C and stirred for 3 hours. After the reaction was completed, saturated sodium bicarbonate solution (10 mL) was added at 0°C to quench the reaction solution, and the mixture was extracted with ethyl acetate (20 mL × 3). The organic phases were combined, dried over anhydrous sodium sulphate, and concentrated, and the crude product was purified by column chromatography (petroleum ether/ethyl acetate = 5:1), to afford 190d (420 mg, yield: 44.95%).

MS *m*/*z* (ESI): 255.9 [M+1]⁺.

According to Step 4 to Step 6 in the aforementioned synthetic route, with reference to the synthetic method of compound A0212, the less polar isomer A0208 (Rf=0.25) and the more polar isomer A0190 (Rf=0.2) were obtained. Further purification by preparative HPLC and lyophilization yielded the more polar product A0190 (29.99 mg, yield: 17.26%) and the less polar product A0208 (28.31 mg, yield: 16.29%).

A0190: MS *m*/*z* (ESI): 405.0 [M+1]⁺.

¹H NMR (400 MHz, Chloroform-*d*) *δ* 8.44 (s, 1H), 7.78-7.76 (m, 1H), 7.61-7.40 (m, 3H), 7.36-7.31 (m, 1H), 4.89-4.82 (m, 1H), 4.41-4.20 (m, 2H), 4.05-3.82 (m, 5H), 3.75-3.65 (m, 1H), 3.09-3.00 (m, 1H), 2.97-2.82 (m, 1H), 2.50-2.40 (m, 3H).

A0208: MS *m*/*z* (ESI): 405.0 [M+1]⁺.

¹H NMR (400 MHz, Chloroform-*d*) *δ* 8.38 (s, 1H), 7.71-7.68 (m, 1H), 7.55-7.33 (m, 3H), 7.28-7.19 (m, 1H), 4.82-4.78 (m, 1H), 4.36-4.12 (m, 2H), 3.88-3.82 (m, 1H), 3.77 (s, 3H), 3.65-3.57 (m, 1H), 3.27-3.20 (m, 1H), 3.00-2.75 (m, 2H), 2.35 (s, 3H).

### Examples A0197& A0197A

### (S, Z)-3-(6-(2-(Hydroxymethyl)-4-(methoxyimino)pyrrolidine-1-carbonyl)-2-((2-methoxyethyl)amino)pyridin-3-yl)-2-methylbenzonitrile

### (S, E)-3-(6-(2-(Hydroxymethyl)-4-(methoxyimino)pyrrolidine-1-carbonyl)-2-((2-methoxyethyl)amino)pyridin-3-yl)-2-methylbenzonitrile

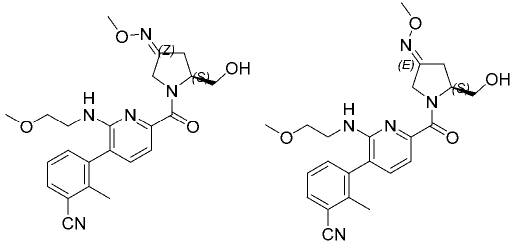

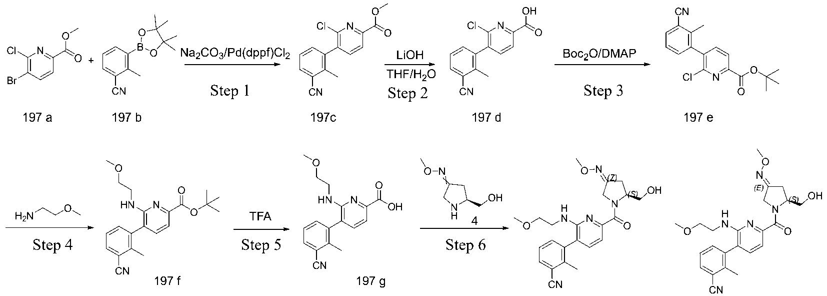

### Step 1

### Methyl 6-chloro-5-(3-cyano-2-methylphenyl)picolinate 197c

The starting materials 197a (3 g, 11.98 mmol) and 197b (3.49 g, 14.38 mmol) were dissolved in dioxane (50 mL) and water (5 mL), and then sodium carbonate (3.81 g, 35.94 mmol) and dichloro[1,1-bis(di-tert-butylphosphino)ferrocene]palladium(II) (0.39 g, 0.60 mmol) were added. After nitrogen purging, the reaction solution was reacted at 100°C for 2 hours. Then, 50 mL of water was added, and the mixture was extracted with ethyl acetate (50 mL × 3). The organic phases were combined, washed with sodium chloride aqueous solution (50 mL × 3), dried over anhydrous sodium sulphate, and filtered, and the filtrate was concentrated under reduced pressure to afford a crude product, which was purified by column chromatography to afford product 197c (3.04 g, yield: 88.53%).

MS *m*/*z* (ESI): 287.2 [M+1]⁺.

### Step 2

### 6-Chloro-5-(3-cyano-2-methylphenyl)picolinic acid 197d

The starting material 197c (3 g, 10.46 mmol) was dissolved in tetrahydrofuran (30 mL) and water (15 mL), and then lithium hydroxide hydrate (2.19 g, 52.30 mmol) was added, and the reaction solution was reacted at room temperature for 16 hours. Then, 50 mL of water was added, and the mixture was extracted with ethyl acetate (50 mL × 3). The organic phases were combined, washed with sodium chloride aqueous solution (50 mL × 3), dried over anhydrous sodium sulphate, and filtered, and the filtrate was concentrated under reduced pressure to afford a residue, which was purified by silica gel column chromatography to afford product 197d (2.4 g, yield: 84.11 %).

MS *m*/*z* (ESI): 273.0 [M+1]⁺.

### Step 3

### Tert-butyl 6-chloro-5-(3-cyano-2-methylphenyl)pyridine-2-carboxylate 197e

The starting material 197d (1.5 g, 5.50 mmol) and tert-butyloxycarbonyl anhydride (3.00 g, 13.75 mmol) were dissolved in tert-butanol (30 mL), and then DMAP (0.34 g, 2.75 mmol) was added, and the reaction solution was reacted at 80°C for 16 hours. Then, the reaction solution was concentrated under reduced pressure to afford a residue, which was purified by silica gel column chromatography to afford product 197e (1.3 g, yield: 71.88%).

MS *m*/*z* (ESI): 329.2 [M+1]⁺.

### Step 4

### Tert-butyl 5-(3-cyano-2-methylphenyl)-6-[(2-methoxyethyl)amino]pyridine-2-carboxylate 197f

The starting material 197e (200 mg, 0.61 mmol) and 2-methoxyethan-1-amine (0.14 g, 1.83 mmol) were dissolved in DME (5 mL), and then palladium acetate (0.014 g, 0.061 mmol), 2-dicyclohexylphosphino-2',6'-diisopropoxybiphenyl (0.057 g, 0.12 mmol), and caesium carbonate (0.60 g, 1.83 mmol) were added, and the reaction solution was reacted at 100°C for 16 hours. Then, 10 mL of water was added, and the mixture was extracted with ethyl acetate (10 mL × 3). The organic phases were combined, washed with sodium chloride aqueous solution (10 mL × 3), dried over anhydrous sodium sulphate, and filtered, and the filtrate was concentrated under reduced pressure to afford product 197f (20 mg, yield: 8.95%).

MS *m*/*z* (ESI): 368.0 [M+1]⁺.

### Step 5

### 5-(3-Cyano-2-methylphenyl)-6-[(2-methoxyethyl)amino]pyridine-2-carboxylic acid 197g

The starting material 197f (20 mg, 0.054 mmol) was dissolved in dichloromethane (1 mL), and then trifluoroacetic acid (0.38 g, 3.30 mmol) was added, and the reaction solution was reacted at room temperature for 2 hours. Then, the reaction solution was concentrated under reduced pressure, to afford product 197g (15 mg, yield: 88.51%).

MS *m*/*z* (ESI): 312.0 [M+1]⁺.

### Step 6

### (S, Z)-3-(6-(2-(Hydroxymethyl)-4-(methoxyimino)pyrrolidine-1-carbonyl)-2-((2-methoxyethyl)amino)pyridin-3-yl)-2-methylbenzonitrile

### (S, E)-3-(6-(2-(Hydroxymethyl)-4-(methoxyimino)pyrrolidine-1-carbonyl)-2-((2-methoxyethyl)amino)pyridin-3-yl)-2-methylbenzonitrile

The starting material 197g (15 mg, 0.048 mmol) and 4 (0.014 g, 0.096 mmol) were dissolved in dichloromethane (1 mL) and DMF (0.3 mL), and then EDCI (0.0092 g, 0.048 mmol) and DMAP (0.012 g, 0.096 mmol) were added, and the reaction solution was reacted at room temperature for 16 hours. Then, 10 mL of water was added, and the mixture was extracted with ethyl acetate (5 mL × 3). The organic phases were combined, washed with sodium chloride aqueous solution (5 mL × 3), dried over anhydrous sodium sulphate, and filtered, and the filtrate was concentrated under reduced pressure to afford a crude product, which was separated by silica gel Prep-TLC (EA:PE=2:1) to afford the more polar isomer A0197 (Rf=0.25) and the less polar isomer A0197A (Rf=0.3). Further purification by preparative HPLC and lyophilization yielded the more polar product A0197 (4.25 mg, yield: 20.16%) and a trace amount of the less polar product A0197A.

A0197: MS *m*/*z* (ESI): 438.0 [M+1]⁺.

¹H NMR (400 MHz, Chloroform-*d*) *δ* 7.75-7.69 (m, 1H), 7.53-7.23 (m, 4H), 4.88-4.68 (m, 1H), 3.90-3.78 (m, 4H), 3.75-3.35 (m, 6H), 3.30-3.25 (m, 5H), 2.97-2.83 (m, 1H), 2.69-2.49 (m, 1H), 2.35 (s, 3H).

A0197A: MS *m*/*z* (ESI): 438.0 [M+1]⁺.

### Examples A0198&A0231

### (S, Z)-3-(6-(2-(Hydroxymethyl)-4-(methoxyimino)pyrrolidine-1-carbonyl)-2-morpholinopyridin-3-yl)-2-methylbenzonitrile

### (S, E)-3-(6-(2-(Hydroxymethyl)-4-(methoxyimino)pyrrolidine-1-carbonyl)-2-morpholinopyridin-3-yl)-2-methylbenzonitrile

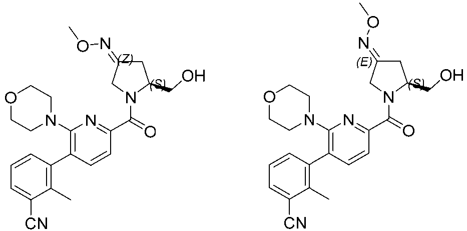

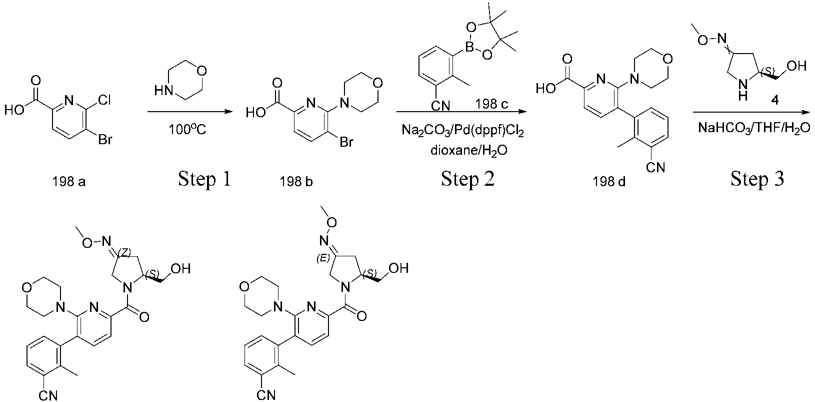

### Step 1

### 5-Bromo-6-(morpholin-4-yl)pyridine-2-carboxylic acid 198b

198a (190 mg, 0.80 mmol) was mixed with morpholine (0.5 mL, 4 mmol). The reaction solution was then reacted at 120°C for 16 hours. Then, the reaction solution was concentrated under reduced pressure, to afford product 198b (135 mg, yield: 58.52%).

MS *m*/*z* (ESI): 287.1 [M+1]⁺.

### Step 2

### 5-(3-Cyano-2-methylphenyl)-6-(morpholin-4-yl)pyridine-2-carboxylic acid 198d

The starting materials 198b (110 mg, 0.38 mmol) and 198c (0.12 g, 0.49 mmol) were dissolved in dioxane (5 mL) and water (0.5 mL), and then sodium carbonate (0.12 g, 1.14 mmol) and dichloro[1,1-bis(di-tert-butylphosphino)ferrocene]palladium(II) (24.77 mg, 0.038 mmol) were added. After nitrogen purging, the reaction solution was reacted at 100°C for 2 hours. Then, the reaction solution was concentrated under reduced pressure to afford a crude product, which was purified by column chromatography to afford product 198d (120 mg, yield: 96.86%).

### Step 3

### (S, Z)-3-(6-(2-(Hydroxymethyl)-4-(methoxyimino)pyrrolidine-1-carbonyl)-2-morpholinopyridin-3-yl)-2-methylbenzonitrile

### (S, E)-3-(6-(2-(Hydroxymethyl)-4-(methoxyimino)pyrrolidine-1-carbonyl)-2-morpholinopyridin-3-yl)-2-methylbenzonitrile

The starting material 197d (105 mg, 0.31 mmol) was dissolved in tetrahydrofuran (2 mL), a solution of sodium bicarbonate (0.26 g, 3.1 mmol) in water (2 mL) was added, and the mixture was reacted for 10 minutes. 4 (0.054 g, 0.37 mmol) was added dropwise at 0°C, and then the reaction solution was reacted at 25°C for 2 hours. The reaction solution was diluted with 5 mL of ethyl acetate, washed twice with water, dried and concentrated. The crude product was separated by silica gel Prep-TLC (EA:PE=2:1) to afford the less polar isomer A0231 (Rf=0.25) and the more polar isomer A0198 (Rf=0.2). Further purification by preparative HPLC and lyophilization yielded the more polar product A0198 (1.58 mg, yield: 1.14%) and the less polar product A0231 (1.8 mg, yield: 1.30%).

A0198: MS *m*/*z* (ESI): 450.0 [M+1]⁺.

¹H NMR (400 MHz, Chloroform-d) *δ* 7.68-7.37 (m, 5H), 5.00-4.50 (m, 4H), 3.98-3.81 (m, 4H), 3.57-3.48 (m, 5H), 3.10-2.63 (m, 5H), 2.42 (s, 3H).

A0231: MS *m*/*z* (ESI): 450.0 [M+1]⁺.

¹H NMR (400 MHz, Chloroform-*d*) *δ* 8.06-7.37 (m, 5H), 5.00-3.75 (m, 8H), 3.56-3.30 (m, 3H), 3.28-3.00 (m, 2H), 3.22-2.60 (m, 5H), 2.42 (s, 3H).

### Common intermediate A

### (S)-(7-(2-(Hydroxymethyl)-4-(methoxyimino)pyrrolidine-1-carbonyl)benzo[d][1,3]dioxolan-4-yl)boronic acid

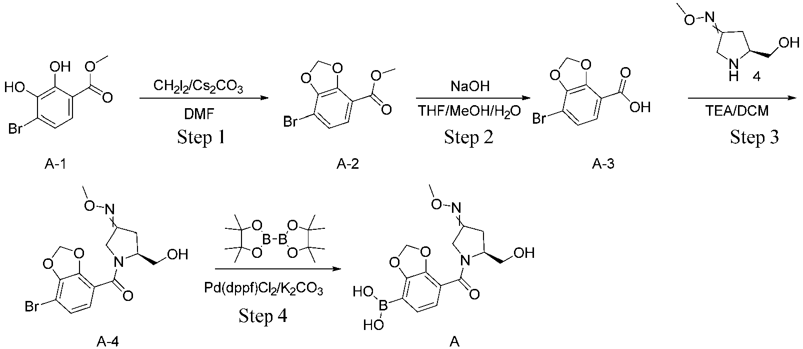

### Step 1

### Methyl 7-bromobenzo[d][1,3]dioxolane-4-carboxylate A-2

To a solution of A-1 (6.40 g, 25.91 mmol) in N,N-dimethylformamide (150 mL) were added diiodomethane (9.02 g, 33.68 mmol) and caesium carbonate (16.88 g, 51.82 mmol). After nitrogen purging, the mixture was reacted at 70°C for 3 hours. After the reaction was completed, the reaction solution was filtered to remove caesium carbonate, and the filter cake was washed with ethyl acetate. Water (50 mL) was added to the filtrate, and the mixture was extracted with ethyl acetate (100 mL × 2). The organic phases were combined, washed with saturated sodium chloride aqueous solution (50 mL × 2), dried over sodium sulphate, and subjected to evaporation to remove the solvent. The residue was purified by C18 reversed-phase column chromatography (eluting with a gradient of acetonitrile:water = 5:95 to 50:50) to afford product A-2 (2.2 g, yield: 32.78%).

MS *m*/*z* (ESI): 260.0 [M+1]⁺.

### Step 2

### 7-Bromobenzo[d][1,3]dioxole-4-carboxylic acid A-3

To a solution of A-2 (950 mg, 3.67 mmol) in tetrahydrofuran (10 mL)/methanol (5 mL)/water (5 mL) was added sodium hydroxide (734 mg, 18.35 mmol), and the reaction solution was reacted at room temperature for 3 hours. After the reaction was completed, the reaction solution was concentrated to remove methanol, and ethyl acetate (100 mL) was added to the residue. The mixture was adjusted to pH 3 with 1N HCl, and the mixture was extracted with ethyl acetate (50 mL × 2). The organic phases were combined, washed with saturated sodium chloride aqueous solution (50 mL), dried over sodium sulphate, and subjected to evaporation to remove the solvent to afford product A-3 (800 mg, yield: 89.03%).

MS *m*/*z* (ESI): 244.0 [M-1]⁺.

### Step 3

### (S,EZ) -(7-Bromobenzo[d][1,3]dioxol-4-yl)(2-(hydroxymethyl)-4-(methoxyimino)pyrrolidin-1-yl)methanone A-4

To a solution of A-3 (1.7 g, 6.49 mmol) in N,N-dimethylformamide (50 mL) were added triethylamine (2.69 g, 20.82 mmol), 4 (1.5 g, 8.33 mmol) and HATU (3.17 g, 8.33 mmol), and the reaction solution was reacted at room temperature for 90 minutes. After the reaction was completed, water (50 mL) was added to the reaction solution, and the mixture was extracted with ethyl acetate (50 mL × 2). The organic phase was washed with saturated sodium chloride aqueous solution (30 mL), dried over anhydrous sodium sulphate, and subjected to evaporation to remove the solvent. The residue was purified by C18 reversed-phase column chromatography (eluting with a gradient of acetonitrile:water=5:95 to 60:40) to afford product A-4 (2.38 g, yield: 92.42%).

MS *m*/*z* (ESI): 372.2 [M+1]⁺.

### Step 4

### (S,EZ)-(7-(2-(Hydroxymethyl)-4-(methoxyimino)pyrrolidine-1-carbonyl)benzo[d][1,3]dioxol-4-yl)boronic acid A

A-4 (1.60 g, 4.31 mmol), bis(pinacolato)diboron (1.97 g, 7.76 mmol) and potassium acetate (1.27 g, 12.93 mmol) were dissolved in 1,4-dioxane (30 mL). After nitrogen purging, Pd(dppf)Cl₂ (320 mg, 0.44 mmol) was added at room temperature, and the mixture was reacted at 80°C for 4 hours. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness. The residue was purified by C18 reversed-phase silica gel column chromatography (eluting with a gradient of acetonitrile:water = 15:85 to 60:40) to afford A (650 mg, yield: 44.87%).

MS *m*/*z* (ESI): 337.0 [M+1]⁺.

### Examples C0117&C0118

### (S, E)-(7-(3-(Difluoromethyl)-2-methylphenyl)benzo[d] [1,3]dioxolan-4-yl)(2-(hydroxymethyl)-4-(methoxyimino)pyrrolidin-1-yl)methanone

### (S, Z)-(7-(3-(Difluoromethyl)-2-methylphenyl)benzo[d] [1,3]dioxolan-4-yl)(2-(hydroxymethyl)-4-(methoxyimino)pyrrolidin-1-yl)methanone

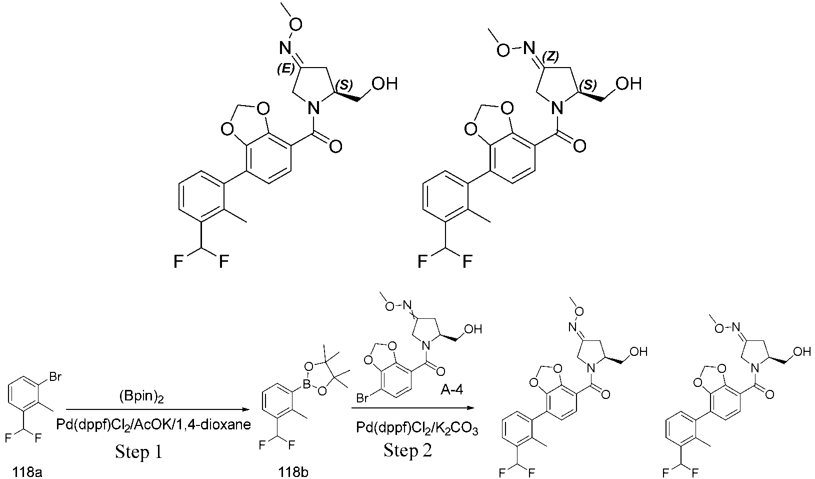

### Step 1

### 3-Difluoromethyl-2-methylphenylboronic acid pinacol ester 118b

To a solution of 118a (150 mg, 0.68 mmol) in 1,4-dioxane (8 mL) were added bis(pinacolato)diboron (0.35 g, 1.36 mmol), potassium carbonate (0.20 g, 2.04 mmol) and Pd(dppf)Cl₂ (0.050 g, 0.068 mmol). After nitrogen purging, the mixture was reacted at 75°C for 3 hours. Upon complete depletion of starting materials as monitored by TLC, the mixture was directly used in the next step without workup.

### Step 2

### (S, E)-(7-(3-(Difluoromethyl)-2-methylphenyl)benzo[d] [1,3]dioxolan-4-yl)(2-(hydroxymethyl)-4-(methoxyimino)pyrrolidin-1-yl)methanone

### (S, Z)-(7-(3-(Difluoromethyl)-2-methylphenyl)benzo[d] [1,3]dioxolan-4-yl)(2-(hydroxymethyl)-4-(methoxyimino)pyrrolidin-1-yl)methanone

To the reaction solution of 118b were added A-4 (200 mg, 0.54 mmol), Pd(dppf)Cl₂ (0.044 g, 0.054 mmol, potassium carbonate (0.22 g, 1.62 mmol) and water (1 mL). After nitrogen purging, the reaction solution was reacted at 95°C for 2 hours. Water (10 mL) was added to the reaction solution, and the mixture was extracted with ethyl acetate (20 mL × 3). The organic phases were combined, washed with saturated sodium chloride aqueous solution (10 mL × 3), dried over sodium sulphate, and subjected to evaporation to remove the solvent. The residue was purified by silica gel Prep-TLC (PE:EA=1:5) to afford the less polar crude isomer C0117 (Rf=0.6) and the more polar crude isomer C0118 (Rf=0.55). Further purification by preparative HPLC and lyophilization yielded the less polar product C0117 (2.0 mg, yield: 0.86%) and the more polar product C0118 (2.0 mg, yield: 0.86%).

C0117: MS *m*/*z* (ESI): 433.1 [M+1]⁺.

¹H NMR (400 MHz, CD₃OD-*d*₄) *δ* 7.57-7.56 (m, 1H), 7.41-7.34 (m, 2H), 7.11-6.84 (m, 3H), 6.09-6.04 (m, 2H), 4.72-4.14 (m, 3H), 3.87-3.31 (m, 5H), 2.95-2.30 (m, 2H), 2.23 (s, 3H).

C0118: MS *m*/*z* (ESI): 433.1 [M+1]⁺.

¹H NMR (400 MHz, CD₃OD-*d*₄) *δ* 7.57-7.56 (m, 1H), 7.40-7.34 (m, 2H), 7.11-6.84 (m, 3H), 6.10-6.05 (m, 2H), 4.72-4.16 (m, 3H), 3.87-3.31 (m, 5H), 3.12-2.66 (m, 2H), 2.30 (s, 3H).

### Examples C0120&C0121

### (S, Z)-(2-(Hydroxymethyl)-4-(methoxyimino)pyrrolidin-1-yl)(4-(1-methyl-1H-indazol-7-yl)phenyl)methanone

### (S, E)-(2-(Hydroxymethyl)-4-(methoxyimino)pyrrolidin-1-yl)(4-(1-methyl-1H-indazol-7-yl)phenyl)methanone

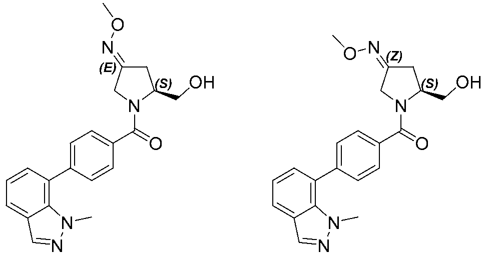

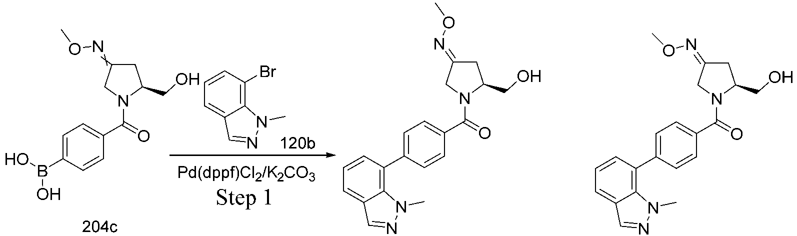

### Step 1

### (S, Z)-(2-(Hydroxymethyl)-4-(methoxyimino)pyrrolidin-1-yl)(4-(1-methy1-1H-indazol-7-yl)phenyl)methanone

### (S, E)-(2-(Hydroxymethyl)-4-(methoxyimino)pyrrolidin-1-yl)(4-(1-methyl-1H-indazol-7-yl)phenyl)methanone

According to the aforementioned synthetic route, with reference to the synthetic method of compound A0204, the less polar isomer C0120 (20 mg, yield: 31.9%) and the more polar isomer C0121 (22 mg, yield: 35.1%) were obtained.

C0120: MS *m*/*z* (ESI): 379.1 [M+1]⁺.

¹H NMR (400 MHz, CD₃OD-*d*₄) *δ* 8.08 (s, 1H), 7.81-7.79 (dd, *J =* 8.0, 0.8 Hz, 1H), 7.70-7.69 (m, 2H), 7.59-7.57 (m, 2H), 7.28-7.20 (m, 2H), 4.77-4.71 (m, 8H), 3.63 (s, 3H), 2.93-2.86 (m, 2H).

C0121: MS *m*/*z* (ESI): 379.1 [M+1]⁺.

¹H NMR (400 MHz, CD₃OD-*d*₄) *δ* 8.08 (s, 1H), 7.81-7.79 (dd, *J =* 8.0, 0.8 Hz, 1H), 7.70-7.68 (m, 2H), 7.59-7.57 (m, 2H), 7.27-7.20 (m, 2H), 4.78-3.79 (m, 8H), 3.63 (s, 3H), 3.35-3.30 (m, 1H), 2.97-2.65 (m, 2H).

### Examples C0122&C0123

### (S, E)-6-Fluoro-3-(7-(2-(hydroxymethyl)-4-(methoxyimino)pyrrolidine-1-carbonyl)benzo[d][1,3]dioxolan-4-yl)-2-methylbenzonitrile

### (S, Z)-6-Fluoro-3-(7-(2-(hydroxymethyl)-4-(methoxyiimino)pyrrolidine-1-carbonyl)benzo[d][1,3]dioxolan-4-yl)-2-methylbenzonitrile

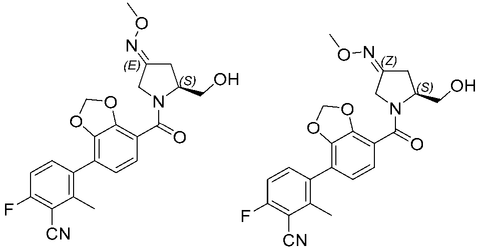

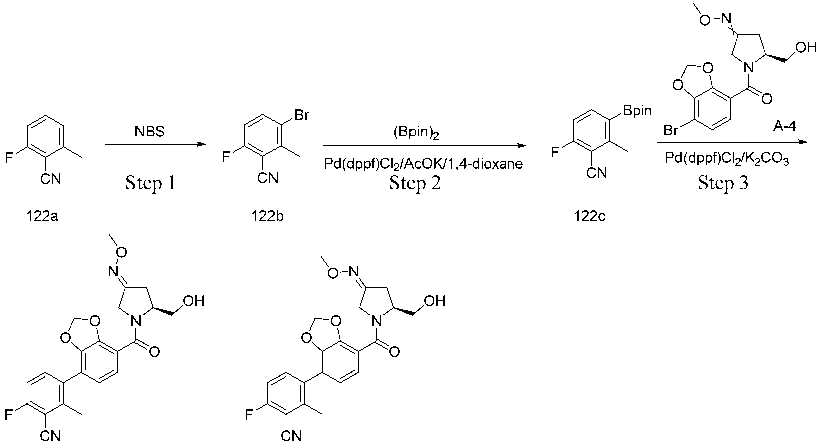

### Step 1

122a (1 g, 7.40 mmol) was dissolved in trifluoromethanesulfonic acid (15 mL), NBS (1.32 g, 7.40 mmol) was slowly added at 0°C, and the mixture was allowed to warm to room temperature and stirred for 1 hour. The reaction solution was poured into water and extracted three times with dichloromethane. The organic phase was collected, dried over anhydrous sodium sulphate, filtered, and concentrated to afford product 122b (1.45 g, yield: 91.55%).

According to Step 2 to Step 3 in the aforementioned synthetic route, with reference to the synthetic method of compound C0117, the less polar crude isomer C0122 (Rf=0.4) and the more polar crude isomer C0123 (Rf=0.3) were obtained. Further purification by preparative HPLC and lyophilization yielded the less polar isomer C0122 (5 mg, yield: 1.8%) and the more polar isomer C0123 (10 mg, yield: 3.5%).

C0122: MS *m*/*z* (ESI): 426.1 [M+1]⁺.

¹H NMR (400 MHz, CD₃OD-*d*₄) *δ* 7.63-7.58 (m, 1H), 7.27 (t, *J =* 8.4 Hz, 1H), 7.06 (d, *J =* 8.4 Hz, 1H), 6.87 (d, *J =* 8.4 Hz, 1H), 6.12-6.07 (m, 2H), 4.74-3.70 (m, 8H), 2.90-2.85 (m, 2H), 2.46 (s, 3H).

C0123: MS *m*/*z* (ESI): 426.1 [M+1]⁺.

¹H NMR (400 MHz, CD₃OD-*d*₄) *δ* 7.64-7.60 (m, 1H), 7.27 (t, *J =* 9.2 Hz, 1H), 7.06 (d, *J =* 8.4 Hz, 1H), 6.87 (d, *J =* 8.0 Hz, 1H), 6.13-6.07 (m, 2H), 4.71-4.14 (m, 4H), 3.88-3.69 (m, 4H), 3.13-2.65 (m, 2H), 2.47 (s, 3H).

### Examples C0126&C0127

### (S, E)-4'-(2-(Hydroxymethyl)-4-(methoxyimino)pyrrolidine-1-carbonyl)-2-(trifluoromethyl)-[1,1'-biphenyl]-3-carbonitrile

### (S, Z)-4'-(2-(Hydroxymethyl)-4-(methoxyimino)pyrrolidine-1-carbonyl)-2-(trifluoromethyl)-[1,1'-biphenyl]-3-carbonitrile

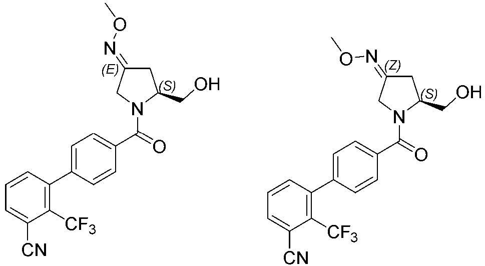

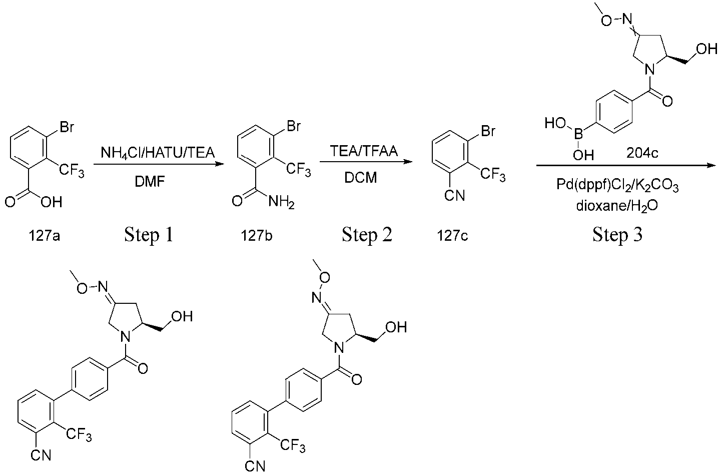

### Step 1

### 3-Bromo-2-(trifluoromethyl)benzamide 127b

127a (500 mg, 1.86 mmol) and HATU (780 mg, 2.05 mmol) were dissolved in DMF (8 mL), triethylamine (750 mg, 7.44 mmol) and ammonium chloride (200 mg, 3.72 mmol) were added at room temperature, and the mixture was reacted at room temperature for 2 hours. After the reaction was completed, the reaction solution was filtered, and the filtrate was purified by C18 reversed-phase column chromatography (eluting with a gradient of acetonitrile:water = 15:85 to 60:40) to afford product 127b (350 mg, yield: 70.26%).

MS *m*/*z* (ESI): 268.8 [M+1]⁺.

### Step 2

### 3-Bromo-2-(trifluoromethyl)benzonitrile 127c

127b (330 mg, 1.23 mmol) was dissolved in DMF (8 mL), triethylamine (190 mg, 1.88 mmol) was added at room temperature, trifluoroacetic anhydride (390 mg, 1.86 mmol) was added dropwise at 0°C under nitrogen protection, and the mixture was reacted at room temperature for 4 hours. After the reaction was completed, the reaction solution was concentrated, and the residue was purified by C18 reversed-phase column chromatography (eluting with a gradient of acetonitrile:water = 20:80 to 70:30) to afford product 127c (150 mg, yield: 48.73%).

MS *m*/*z* (ESI): 250.1 [M+1]⁺.

### Step 3

### (S, Z)-4'-(2-(Hydroxymethyl)-4-(methoxyimino)pyrrolidine-1-carbonyl)-2-(trifluoromethyl)-[1,1'-biphenyl]-3-carbonitrile

### (S, E)-4'-(2-(Hydroxymethyl)-4-(methoxyimino)pyrrolidine-1-carbonyl)-2-(trifluoromethyl)-[1,1'-biphenyl]-3-carbonitrile

204c (120 mg, 0.41 mmol), 127c (100 mg, 0.40 mmol) and potassium carbonate (145 mg, 1.05 mmol) were dissolved in 1,4-dioxane (4 mL) and water (0.8 mL), Pd(dppf)Cl₂ (30 mg, 0.041 mmol) was added at room temperature under nitrogen protection, and the mixture was reacted at 80°C for 2 hours. After the reaction was completed, water (10 mL) was added, and the mixture was extracted with ethyl acetate (20 mL × 3). The organic phases were combined, washed with saturated sodium chloride aqueous solution (20 mL × 3), dried over sodium sulphate, and filtered, and the filtrate was collected and concentrated. The residue was purified by silica gel Prep-TLC (PE:EA=1:4) to afford the less polar isomer C0126 (Rf=0.6, 26.32 mg, yield: 13.53%) and the more polar isomer C0127 (Rf=0.50, 46.55 mg, yield: 25.67%).

C0127: MS *m*/*z* (ESI): 418.0 [M+1]⁺.

¹H NMR (400 MHz, CD₃OD-*d*₄) *δ* 8.04 (d, *J =* 7.6 Hz, 1H), 7.85 (t*, J =* 7.6 Hz, 1H), 7.71 (d, *J =* 7.2 Hz, 1H), 7.64 (d, *J =* 7.6 Hz, 2H), 7.44 (d, *J =* 8.0 Hz, 2H), 4.76-4.03 (m, 3H), 3.90-3.38 (m, 5H), 3.07-2.84 (m, 1H), 2.77-2.63 (m, 1H).

C0126: MS *m*/*z* (ESI): 418.0 [M+1]⁺.

¹H NMR (400 MHz, CD₃OD-*d*₄) *δ* 8.04 (d, *J =* 7.6 Hz, 1H), 7.85 (t, *J =* 7.6 Hz, 1H), 7.70 (d, *J =* 8.0 Hz, 1H), 7.64 (d, *J =* 7.6 Hz, 2H), 7.43 (d, *J =* 8.0 Hz, 2H), 4.83-4.00 (m, 3H), 3.95-3.36 (m, 5H), 2.98-2.77 (m, 2H).

### Examples C0132&C0133

### (S,E)-3-(8-(2-(Hydroxymethyl)-4-(methoxyimino)pyrrolidine-1-carbonyl)-2,3-dihydrobenzo[b][1,4]dioxin-5-yl)-2-methylbenzonitrile

### (S,Z)-3-(8-(2-(Hydroxymethyl)-4-(methoxyimino)pyrrolidine-1-carbonyl)-2,3-dihydrobenzo[b][1,4]dioxin-5-yl)-2-methylbenzonitrile

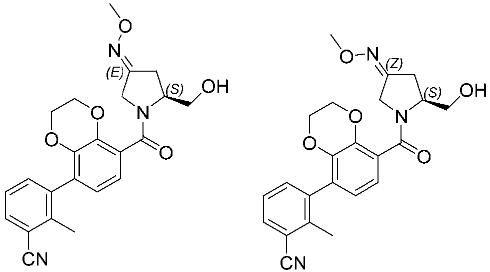

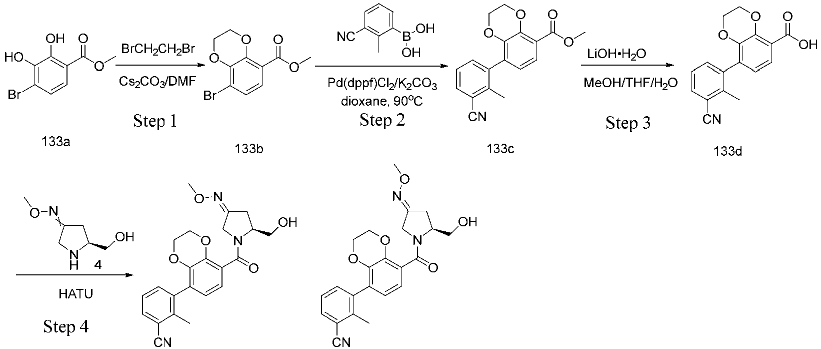

According to the aforementioned synthetic route, with reference to the synthetic method of compound C0061, the more polar product C0133 (54 mg, yield: 12.2%) and the less polar product C0132 (38 mg, yield: 8.6%) were obtained.

C0132: MS *m*/*z* (ESI): 422.1 [M+1]⁺.

¹H NMR (300 MHz, DMSO-*d*₆) *δ* 7.84-7.82 (m, 1H), 7.56-7.45 (m, 2H), 6.96-6.90 (m, 1H), 6.81 (dd, *J =* 7.8, 2.7 Hz, 1H), 5.01-4.98 (m, 1H), 4.51-4.28 (m, 5H), 4.08-3.96 (m, 2H), 3.84-3.80 (m, 3H), 3.59-3.58 (m, 1H), 3.27-3.26 (m, 1H), 2.87-2.74 (m, 2H), 2.33 (s, 3H).

C0133: MS *m*/*z* (ESI): 422.1 [M+1]⁺.

¹H NMR (400 MHz, DMSO-*d*₆) *δ* 7.80 (d, *J =* 7.6 Hz, 1H), 7.55-7.42 (m, 2H), 6.92-6.89 (m, 1H), 6.78 (dd, *J =* 6.4, 1.6 Hz, 1H), 4.97-4.93 (m, 1H), 4.48-4.24 (m, 5H), 4.09-3.86 (m, 2H), 3.81-3.73 (m, 3H), 3.55-3.51 (m, 1H), 3.27-3.15 (m, 1H), 2.91-2.84 (m, 1H), 2.69-2.56 (m, 1H), 2.30 (s, 3H).

### Examples C0039&C0040

### (S, Z)-3-(Methoxyimino)-1-(2'-methyl-[1,1'-biphenyl]-4-carbonyl)-1,7-diazaspiro[4.4]nonan-6-one

### (S, E)-3-(Methoxyimino)-1-(2'-methyl-[1,1'-biphenyl]-4-carbonyl)-1,7-diazaspiro[4.4]nonan-6-one

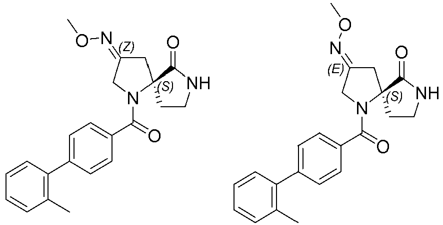

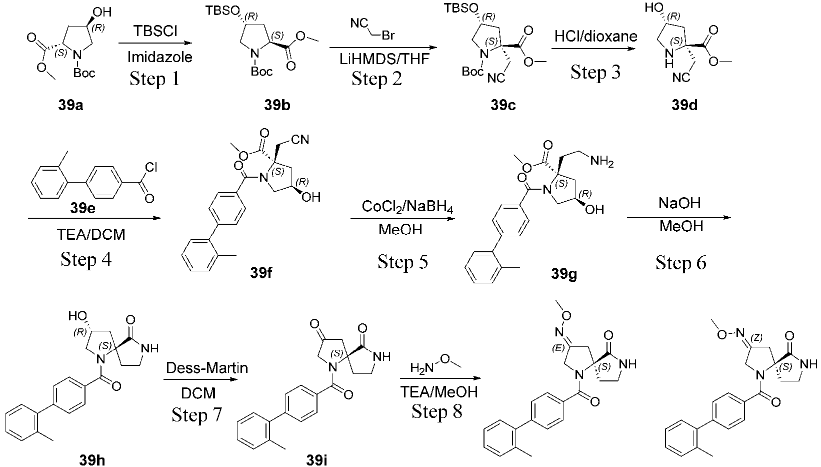

### Step 1

### 1-(Tert-butyl)2-methyl (2S, 4R)-4-((tert-butyldimethylsilyl)oxy)-2-pyrrolidine-1,2-dicarboxylate 39b

39a (10 g, 40.77 mmol), tert-butyldimethylsilyl chloride (12.29 g, 81.54 mmol) and imidazole (6.94 g, 101.93 mmol) were dissolved in DMF (50 mL). After nitrogen purging, the mixture was stirred at room temperature overnight. The reaction solution was cooled and then poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness. The crude product was loaded onto a silica gel column, eluting with a gradient of (PE/EA=10:1). The eluate was collected and subjected to rotary evaporation to afford 39b (13.5 g, yield: 87.5%).

¹H NMR (400 MHz, CDCl₃) *δ* 4.43-4.31 (m, 2H), 3.74-3.72 (m, 3H), 3.62-3.59 (m, 1H), 3.42-3.30 (m, 1H), 2.19-2.15 (m, 1H), 2.03-1.98 (m, 1H), 1.46-1.41 (m, 9H), 0.92 (s, 6H), 0.059 (s, 6H).

### Step 2

### 1-(Tert-butyl)2-methyl (2S, 4R)-4-((tert-butyldimethylsilyl)oxy)-2-(cyanomethyl)pyrrolidine-1,2-dicarboxylate 39c

To a solution of 39b (7.9 g, 21.97 mmol) and potassium iodide (0.36 g, 2.20 mmol) in THF (100 mL) was added dropwise LiHMDS (7.35 g, 43.94 mmol) at 0°C under nitrogen protection, and the mixture was stirred for 20 minutes and cooled to -78°C. 2-Bromoacetonitrile (5.27 g, 43.94 mmol) was added dropwise at -78°C. After the dropwise addition was completed, the reaction solution was gradually heated to room temperature, and reacted at room temperature overnight. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness. The crude product was loaded onto a silica gel column, eluting with a gradient of (PE/EA=50:1 to 5:1). The eluate was collected and subjected to rotary evaporation to afford 39c (2.0 g, yield: 20.5%).

¹H NMR (400 MHz, CDCl₃) *δ* 4.45-4.07 (m, 1H), 3.63-3.53 (m, 3H), 3.26-3.12 (m, 2H), 2.95-2.91 (m, 1H), 2.37-2.28 (m, 1H), 2.11-2.02 (m, 1H), 1.31-1.24 (m, 9H), 0.79-0.76 (m, 9H), 0.01-0.00 (m, 6H).

### Step 3

### Methyl (2S, 4R)-2-(cyanomethyl)-4-hydroxypyrrolidine-2-carboxylate 39d

39c (2.0 g, 5.02 mmol) was dissolved in hydrogen chloride dioxane solution (10 mL). The mixture was reacted at room temperature for 1 hour. After the reaction was completed, the reaction solution was directly subjected to rotary evaporation to dryness, to afford crude 39d (1.0 g).

MS *m*/*z* (ESI): 185.1 [M+1]⁺.

### Step 4

### Methyl (2S, 4R)-2-(cyanomethyl)-4-hydroxy-1-(4-(2-methylphenyl)benzoyl)pyrrolidine-2-carboxylate 39f

To a solution of 39d (1 g, 5.43 mmol) and 39e (1.88 g, 8.14 mmol) in DCM (20 mL) was added triethylamine (1.65 g, 16.29 mmol). After nitrogen purging, the reaction solution was stirred at room temperature for 20 minutes. The reaction solution was extracted with dichloromethane/water. The organic layers were combined, washed with saturated NaCl solution, combined, dried over Na₂SO₄, filtered, and subjected to rotary evaporation. The crude product was loaded onto a silica gel column, eluting with a gradient of (PE/EA=10:1 to 1:1). The eluate was collected and subjected to rotary evaporation to dryness, to afford product 39f (900 mg, yield: 39.4%).

MS *m*/*z* (ESI): 379.0 [M+1]⁺.

### Step 5

### Methyl (2S, 4R)-2-(2-aminoethyl)-4-hydroxy-1-(2'-methyl-[1,1'-biphenyl]-4-carbonyl)pyrrolidine-2-carboxylate 39g

Under nitrogen protection, to a solution of 39f (500 mg, 1.32 mmol) in methanol (20 mL) and water (0.2 mL) were added cobalt chloride (1.88 g, 7.92 mmol) and NaBH₄ (0.20 g, 5.28 mmol), and the mixture was reacted at -20°C to 0°C for 4 hours. After the reaction was completed, water was added to quench the reaction solution, and the reaction solution was filtered with a Buchner funnel. The reaction residue was subjected to rotary evaporation to dryness at 45°C to afford crude product 39g (500 mg).

MS *m*/*z* (ESI): 383.0 [M+1]⁺.

### Step 6

### (3R, 5S)-3-Hydroxy-1-(4-(2-methylphenyl)benzoyl)-1,7-diazaspiro[4.4]nonan-6-one 39h

Under nitrogen protection, to a solution of 39g (500 mg, 1.31 mmol) in methanol (30 mL) was added sodium hydroxide (0.42 g, 10.48 mmol), and the mixture was stirred at room temperature for 3 hours. After the reaction was completed, the reaction solution was extracted with ethyl acetate/water. The organic layers were combined, washed with saturated NaCl solution, combined, dried over Na₂SO₄, filtered and subjected to rotary evaporation to afford a crude product, which was purified by preparative thin-layer chromatography (dichloromethane:methanol = 10:1) to afford 39h (220 mg, yield: 38.4%).

MS m/z (ESI): 351.0 [M+1]⁺.

### Step 7

### 1-(4-(2-Methylphenyl)benzoyl)-1,7-diazaspiro[4.4]nonane-3,6-dione 39i

To a solution of 39h (200 mg, 0.57 mmol) in DCM (10 mL) was added DMP (290.11 mg, 0.68 mmol) under nitrogen protection. The reaction solution was reacted at room temperature overnight. After the reaction was completed, the reaction solution was extracted with ethyl acetate/water. The organic layers were combined, washed with saturated NaCl solution, combined, dried over Na₂SO₄, filtered and subjected to rotary evaporation to afford a crude product, which was purified by preparative thin-layer chromatography (EA:MeOH=20:1) to afford product 39i (110 mg, yield: 52.5%).

MS *m*/*z* (ESI): 348.8 [M+1]⁺.

### Step 8

### (S, E)-3-(Methoxyimino)-1-(2'-methyl-[1,1'-biphenyl]-4-carbonyl)-1,7-diazaspiro[4.4]nonan-6-one

### (S, Z)-3-(Methoxyimino)-1-(2'-methyl-[1,1'-biphenyl]-4-carbonyl)-1,7-diazaspiro[4.4]nonan-6-one

To a solution of 39i (110 mg, 0.32 mmol) in methanol (15 mL) were added methoxyamine (0.080 g, 0.96 mmol) and triethylamine (0.097 g, 0.96 mmol). After nitrogen purging, the mixture was reacted at room temperature overnight. After the reaction was completed, the reaction solution was extracted with ethyl acetate/water. The organic layers were combined, washed with saturated NaCl solution, combined, dried over Na₂SO₄, filtered and subjected to rotary evaporation to afford a crude product. The crude product was separated by silica gel Pre-TLC (EA:PE=2:1) to afford the less polar isomer C0039 (Rf=0.5) and the more polar crude isomer C0040 (Rf=0.4). The less polar crude isomer was purified by preparative HPLC and lyophilized to afford the less polar product C0039 (28.2 mg, yield: 23.5%). The more polar crude isomer C0040 (Rf=0.4) was purified by preparative HPLC and lyophilized to afford the more polar product C0040 (36.1 mg, yield: 30.2%).

C0039: MS *m*/*z* (ESI): 378.1 [M+1]⁺.

¹H NMR (400 MHz, CD₃OD-*d*) *δ* 7.61 (d, *J =* 8.0 Hz, 2H), 7.42 (d, *J =* 8.0 Hz, 2H), 7.30-7.19 (m, 4H), 4.39-4.30 (m, 2H), 3.84 (s, 3H), 3.59-3.55 (m, 1H), 3.45-3.39 (m, 1H), 3.05-3.00 (m, 2H), 2.95-2.87 (m, 1H), 2.28-2.23 (m, 4H).

C0040: MS m/z (ESI): 378.1 [M+1]⁺.

¹H NMR (400 MHz, CD₃OD-*d*) *δ* 7.62 (d, *J =* 8.4 Hz, 2H), 7.42 (d, *J =* 8.0 Hz, 2H), 7.29-7.19 (m, 4H), 4.41-4.31 (m, 2H), 3.81 (s, 3H), 3.59-3.55 (m, 1H), 3.46-3.39 (m, 1H), 3.05-2.92 (m, 2H), 2.88-2.85 (m, 1H), 2.30-2.27 (m, 4H).

### Examples C0009&C0010

### (S, Z)-N-((4-(Methoxyimino)-1-(2'-methyl-[1,1'-biphenyl]-4-carbonyl)pyrrolidin-2-yl)methyl)benzamide

### (S, E)-N-((4-(Methoxyimino)-1-(2'-methyl-[1,1'-biphenyl]-4-carbonyl)pyrrolidin-2-yl)methyl)benzamide

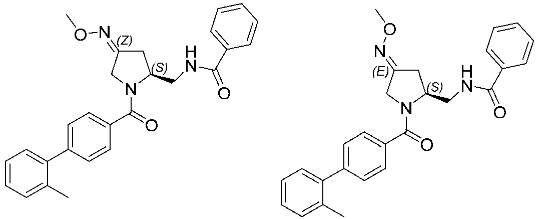

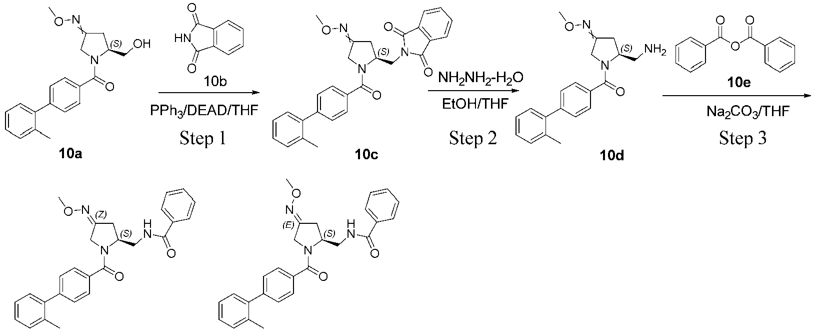

### Step 1

### (S, EZ)-2-((4-(Methoxyimino)-1-(2'-methyl-[1,1'-biphenyl]-4-carbonyl)pyrrolidin-2-yl)methyl)isoindole-1,3-dione 10b

10a (500 mg, 1.48 mmol) and isoindole-1,3-dione 10b (290 mg, 1.97 mmol) were dissolved in tetrahydrofuran (12 mL), triphenylphosphine (390 mg, 1.48 mmol) and diethyl azodicarboxylate (260 mg, 1.48 mmol) were successively added at room temperature, and the mixture was reacted at room temperature for 16 hours. After the reaction was completed, the reaction solution was concentrated, and the concentrate was dissolved in dichloromethane and water. The organic phase was separated, and the aqueous phase was extracted with dichloromethane (50 mL × 3). The organic phases were combined, washed with saturated sodium chloride aqueous solution (70 mL × 3), dried, and subjected to rotary evaporation to dryness, to afford a crude product. The crude product was purified by column chromatography (PE:EA=1:1 to pure EA) to afford 10c (630 mg, yield: 82.08%).

MS *m*/*z* (ESI): 468.1 [M+1]⁺.

### Step 2

### (S, EZ)-(2-(Aminomethyl)-4-(methoxyimino)pyrrolidin-1-yl)(2'-methyl-[1,1'-biphenyl]-4-yl)methanone 10d

10c (620 mg, 1.19 mmol) was dissolved in ethanol (7 mL) and tetrahydrofuran (7 mL), and hydrazine hydrate (240 mg, 4.79 mmol) was added at room temperature. The mixture was reacted at room temperature for 4 hours. After the reaction was completed, the reaction solution was concentrated, and the concentrate was dissolved in dichloromethane and water. The mixture was adjusted to pH 7-8, the organic phase was separated, and the aqueous phase was extracted with dichloromethane (50 mL × 3). The organic phases were combined, washed with saturated sodium chloride aqueous solution (70 mL × 2), dried, and subjected to rotary evaporation to dryness, to afford a crude product, which was purified by reversed-phase column chromatography (H₂O/ACN=1: 2) to afford 10d (190 mg, yield: 44.8%).

MS *m*/*z* (ESI): 338.1 [M+1]⁺.

### Step 3

### (S, Z)-N-((4-(Methoxyimino)-1-(2'-methyl-[1,1'-biphenyl]-4-carbonyl)pyrrolidin-2-yl)methyl)benzamide

### (S, E)-N-((4-(Methoxyimino)-1-(2'-methyl-[1,1'-biphenyl]-4-carbonyl)pyrrolidin-2-yl)methyl)benzamide

10d (60 mg, 0.17 mmol) and benzoic anhydride (45 mg, 1.12 mmol) were dissolved in ethyl acetate (2 mL), saturated sodium carbonate solution (0.5 mL) was added, and the mixture was reacted at room temperature for 16 hours. After the reaction was completed, the reaction solution was poured into a mixed solvent of ethyl acetate and water. After layer separation, the aqueous phase was extracted with ethyl acetate (50 mL × 3), and the organic phases were combined, washed with sodium chloride aqueous solution (70 mL × 2), dried, filtered, and subjected to rotary evaporation to dryness. The crude product was purified by silica gel TLC plate (PE:EA=1:3) and lyophilized to afford the more polar isomer C0010 (Rf=0.6, 33.19 mg, yield: 43.6%) and the less polar isomer C0009 (Rf=0.7, 13.51 mg, yield: 16.8%).

C0010: MS *m*/*z* (ESI): 342.1 [M+1]⁺.

¹H NMR (400 MHz, CD₃OD-*d*) *δ* 8.69-8.57 (m, 1H), 7.79-7.73 (m, 2H), 7.61-7.50 (m, 3H), 7.48-7.22 (m, 8H), 4.86 (t, *J =* 3.2 Hz, 1H), 4.39-4.35 (m, 1H), 4.14-3.93 (m, 1H), 3.81-3.62 (m, 3H), 3.61-3.23 (m, 2H), 3.14-2.75 (m, 1H), 2.67-2.63 (m, 1H), 2.24 (s, 3H).

C0009: MS *m*/*z* (ESI): 342.1 [M+1]⁺.

¹H NMR (400 MHz, CD₃OD-*d*) *δ* 8.69-8.57 (m, 1H), 7.79 (m, 2H), 7.60-7.50 (m, 3H), 7.49-7.22 (m, 8H), 4.89-4.80 (m, 1H), 4.47-4.35 (m, 1H), 4.22-3.97 (m, 1H), 3.90-3.75 (s, 3H), 3.51-3.22 (m, 2H), 2.83-2.76 (m, 2H), 2.24 (s, 3H).

Examples C0003&C0006

### (S, E)-N-((4-(Methoxyimino)-1-(2'-methyl-[1,1'-biphenyl]-4-carbonyl)pyrrolidin-2-yl)methyl)acetamide

### (S, Z)-N-((4-(Methoxyimino)-1-(2'-methyl-[1,1'-biphenyl]-4-carbonyl)pyrrolidin-2-yl)methyl)acetamide

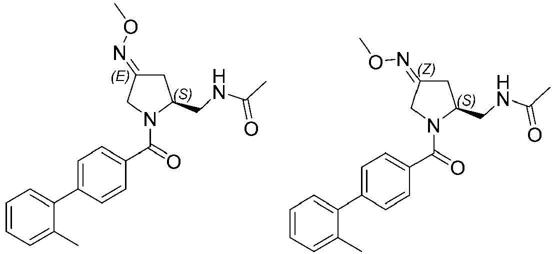

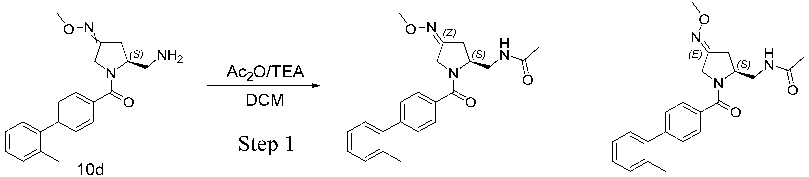

### Step 1

### (S, Z)-N-((4-(Methoxyimino)-1-(2'-methyl-[1,1'-biphenyl-4-carbonyl)pyrrolidin-2-yl)methyl)acetamide

### (S, E)-N-((4-(Methoxyimino)-1-(2'-methyl-[1,1'-biphenyl]-4-carbonyl)pyrrolidin-2-yl)methyl)acetamide

The EZ mixture 10d (60 mg, 0.18 mmol) and triethylamine (20.04 mg, 0.20 mmol) were dissolved in dichloromethane (10 mL), and the mixture was cooled to 0°C. Acetic anhydride (20.21 mg, 0.20 mmol) was slowly added dropwise, and the mixture was stirred at room temperature for 17 hours. Saturated sodium bicarbonate (10 mL) was added to quench the reaction solution, and the reaction solution was separated by partitioning, dried, and concentrated. The residue was purified by silica gel preparative thin-layer chromatography (petroleum ether/ethyl acetate = 1/1.5), to afford the more polar product C0003 (Rf=0.4, 15 mg, yield: 21.7%) and the less polar product C0006 (Rf=0.5, 12 mg, yield: 17.6%).

C0003: MS *m*/*z* (ESI): 380.1 [M+1]⁺.

¹H NMR (400 MHz, DMSO-*d₆*) *δ* 8.07-7.98 (m, 1H), 7.61-7.52 (m, 2H), 7.43 (d, *J* = 7.6 Hz, 2H), 7.33-7.25 (m, 4H), 4.69-4.28 (m, 1H), 4.38-4.35 (m, 1H), 4.11-3.91 (m, 1H), 3.82-3.69 (m, 3H), 3.38-3.25 (m, 1H), 2.97-2.77 (m, 2H), 2.54-2.41 (m, 1H), 2.25 (s, 3H), 1.80-1.65 (m, 3H).

C0006: MS *m*/*z* (ESI): 380.1 [M+1]⁺.

¹H NMR (400 MHz, CD₃OD) *δ* 7.88-7.83 (m, 1H), 7.64-7.62 (m, 1H), 7.45-7.41 (m, 2H), 7.30-7.21 (m, 4H), 4.93-4.52 (m, 1H), 4.40-4.07 (m, 2H), 3.86-3.75 (m, 3H), 3.73-3.47 (m, 2H), 3.04-2.73 (m, 2H), 2.28-2.26 (m, 3H), 2.22-1.85 (m, 3H).

### Examples C0004&C0007

### Methyl (S, E)-((4-(methoxyimino)-1-(2'-methyl-[1,1'-biphenyl]-4-carbonyl)pyrrolidin-2-yl)methyl)carbamate

### Methyl (S, Z)-((4-(methoxyimino)-1-(2'-methyl-[1,1'-biphenyl]-4-carbonyl)pyrrolidin-2-yl)methyl)carbamate

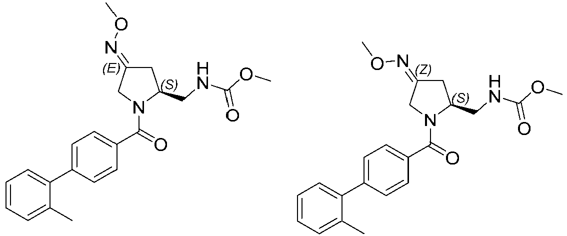

With reference to Examples C0003&C0006, the starting material acetic anhydride was replaced with methyl chloroformate. The crude product was separated by silica gel Prep-TLC (PE:EA=1:3) to afford the more polar product C0004 (Rf=0.3, 10 mg, yield: 10.2%) and the less polar product C0007 (Rf=0.4, 12 mg, yield: 12.5%).

C0004: MS *m*/*z* (ESI): 396.1 [M+1]⁺.

¹H NMR (400 MHz, DMSO-*d₆*) *δ* 7.62-7.53 (m, 2H), 7.43-7.39 (m, 3H), 7.33-7.23 (m, 4H), 4.75-4.68 (m, 1H), 4.38-4.27 (m, 1H), 4.05-3.89 (m, 1H), 3.82-3.72 (m, 3H), 3.54-3.40 (m, 3H), 3.24-2.85 (m, 2H), 2.84-2.67 (m, 1H), 2.56-2.49 (m, 1H), 2.25 (s, 3H).

C0007: MS *m*/*z* (ESI): 396.1 [M+1]⁺.

¹H NMR (400 MHz, DMSO-*d₆*) *δ* 7.86-7.21 (m, 9H), 4.71-4.32 (m, 2H), 4.12-3.98 (m, 1H), 3.77-3.69 (m, 3H), 3.62-3.54 (m, 3H), 3.41-3.26 (m, 2H), 2.84-2.65 (m, 2H), 2.25-2.23 (m, 3H).

Examples C0005&C0008

### (S, E)-2-Hydroxy-N-((4-(methoxyimino)-1-(2'-methyl-[1,1'-biphenyl]-4-carbonyl)pyrrolidin-2-yl)methyl)acetamide

### (S, Z)-2-Hydroxy-N-((4-(methoxyimino)-1-(2'-methyl-[1,1'-biphenyl]-4-carbonyl)pyrrolidin-2-yl)methyl)acetamide

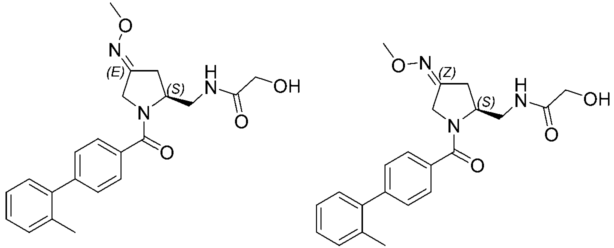

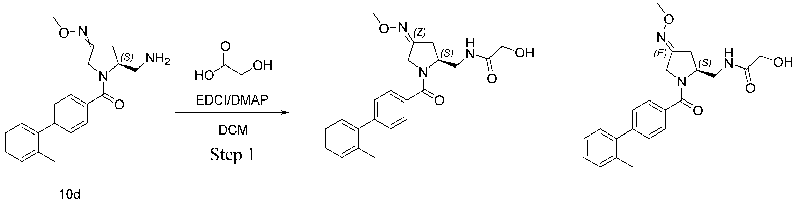

### Step 1

### (S, Z)-2-Hydroxy-N-((4-(methoxyimino)-1-(2'-methyl-[1,1'-biphenyl]-4-carbonyl)pyrrolidin-2-yl)methyl)acetamide

### (S, E)-2-Hydroxy-N-((4-(methoxyimino)-1-(2'-methyl-[1,1'-biphenyl]-4-carbonyl)pyrrolidin-2-yl)methyl)acetamide

The *EZ* mixture 10d (96 mg, 0.28 mmol), 2-hydroxyacetic acid (21.3 mg, 0.28 mmol) and 4-dimethylaminopyridine (10.3 mg, 0.084 mmol) were dissolved in dichloromethane (3 mL), EDCI (59.0 mg, 0.31 mmol) was added, and the mixture was stirred at room temperature for 17 hours. Saturated sodium bicarbonate (10 mL) was added to quench the reaction solution, and the reaction solution was separated by partitioning, dried, and concentrated. The crude product was purified by silica gel Prep-TLC (DCM:EA=1:3), to afford the more polar product C0005 (Rf=0.3, 25.0 mg, yield: 21.2%) and the less polar product C0008 (Rf=0.4, 8 mg, yield: 6.7%).

C0005: MS *m*/*z* (ESI): 396.1 [M+1]⁺.

¹H NMR (400 MHz, DMSO-*d₆*) *δ* 8.04-7.96 (m, 1H), 7.59 (d, *J =* 7.2 Hz, 2H), 7.42 (d, *J* = 7.2 Hz, 2H), 7.31-7.24 (m, 4H), 5.43 (t, *J =* 5.2 Hz, 1H), 4.80-4.75 (m, 1H), 4.36-4.32 (m, 1H), 4.10-3.91 (m, 1H), 3.81-3.63 (m, 5H), 3.39-3.32 (m, 1H), 3.09-2.76 (m, 2H), 2.59-2.55 (m, 1H), 2.25 (s, 3H).

C0008: MS *m*/*z* (ESI): 396.1 [M+1]⁺.

¹H NMR (400 MHz, CD₃OD) *δ* 7.63 (d, *J =* 8.4 Hz, 2H), 7.43 (d, *J =* 8.0 Hz, 2H), 7.31-7.21 (m, 4H), 5.00-4.85 (m, 1H), 4.70-4.39 (m, 1H), 4.25-4.07 (m, 1H), 3.98-3.85 (m, 5H), 3.58-3.42 (m, 2H), 2.90-2.80 (m, 2H), 2.27 (s, 3H).

### Examples C0017&C0018

### (S, Z)-(2-((2-Hydroxyethoxy)methyl)-4-(methoxyimino)pyrrolidin-1-yl)(2'-methyl-[1,1'-biphenyl]-4-yl)methanone

### (S, E)-(2-((2-Hydroxyethoxy)methyl)-4-(methoxyimino)pyrrolidin-1-yl)(2'-methyl-[1,1'-biphenyl]-4-yl)methanone

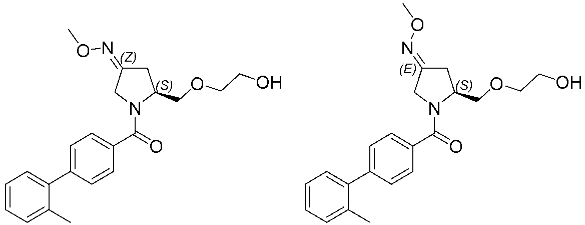

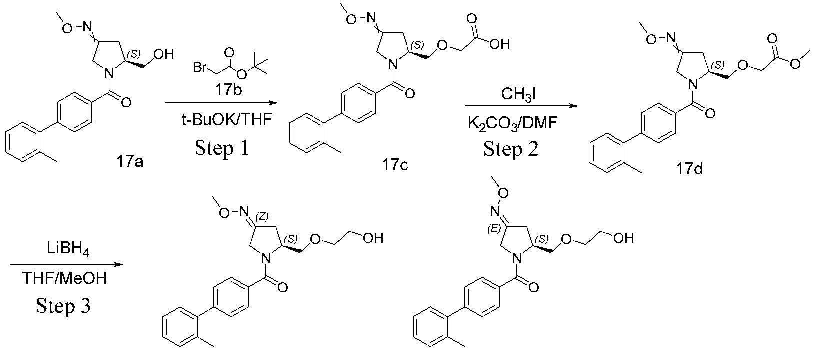

### Step 1

### (S, EZ)-2-((4-(Methoxyimino)-1-(2'-methyl-[1,1'-biphenyl]-4-carbonyl)pyrrolidin-2-yl)methoxy)acetic acid 17c

Compounds 17a (500 mg, 1.36 mmol) and 17b (340 mg, 1.71 mmol) were dissolved in tetrahydrofuran (15 mL), and the mixture was cooled to 0°C. Potassium tert-butoxide (460 mg, 4.02 mmol) was added under nitrogen protection, and the mixture was reacted at 35°C for 16 hours. After the reaction was completed, water was added to quench the reaction solution, and the reaction solution was concentrated. Methanol was added to the residue, and the mixture was filtered. The filtrate was purified by C18 reversed-phase column chromatography (H₂O/ACN=95:5 to 40:60) to afford 17c (260 mg, yield: 43.5%).

MS *m*/*z* (ESI): 397.1 [M+1]⁺.

### Step 2

### Methyl (S, EZ)-2-((4-(methoxyimino)-1-(2'-methyl-[1,1'-biphenyl]-4-carbonyl)pyrrolidin-2-yl)methoxy)acetate 17d

29c (230 mg, 0.55 mmol) was dissolved in DMF (5 mL), potassium carbonate (230 mg, 1.66 mmol) and iodomethane (120 mg, 0.84 mmol) were successively added at room temperature, and the mixture was reacted at room temperature for 16 hours. After the reaction was completed, the reaction solution was purified by C18 reversed-phase column chromatography (H₂O/ACN=95:5 to 20:80) to afford 17d (150 mg, yield: 63.0%).

MS *m*/*z* (ESI): 411.0 [M+1]⁺.

### Step 3

### (S, Z)-(2-((2-Hydroxyethoxy)methyl)-4-(methoxyimino)pyrrolidin-1-yl)(2'-methyl-[1,1'-biphenyl]-4-yl)methanone

### (S, E)-(2-((2-Hydroxyethoxy)methyl)-4-(methoxyimino)pyrrolidin-1-yl)(2'-methyl-[1,1'-biphenyl]-4-yl)methanone

17d (135 mg, 0.30 mmol) was dissolved in THF (1.5 mL) and methanol (1.5 mL), and the mixture was cooled to 0°C. Lithium borohydride (0.23 mL, 0.46 mmol) was added under nitrogen protection, and the mixture was reacted at room temperature for 1.5 hours. Water was added to quench the reaction solution, the reaction solution was concentrated, and the residue was dissolved in dichloromethane and water. After layer separation, the aqueous phase was extracted twice with dichloromethane, and the organic phases were combined, washed once with sodium chloride aqueous solution, dried, filtered, and subjected to rotary evaporation to dryness. The crude product was separated by silica gel Prep-TLC (EA:PE=2:1) to afford the more polar isomer C0018 (Rf=0.4) and the less polar isomer C0017 (Rf=0.5). Further purification by preparative HPLC and lyophilization yielded the more polar product C0018 (7.77 mg, yield: 6.86%) and the less polar product C0017 (5.46 mg, yield: 4.6%).

C0018: MS *m*/*z* (ESI): 383.1 [M+1]⁺.

¹H NMR (400 MHz, CD₃OD-*d*) *δ* 7.61 (dd, *J =* 6.4, 1.6 Hz, 2H), 7.42 (dd, *J* = 6.4 ,1.6 Hz, 2H), 7.28-7.22 (m, 4H), 4.61-4.42 (m, 2H), 4.32-4.09 (m, 1H), 3.93-3.77 (m, 4H), 3.68-3.52 (m, 4H), 3.49-3.37 (m, 1H), 3.18-2.74 (m, 2H), 2.26 (s, 3H).

C0017: MS *m*/*z* (ESI): 383.1 [M+1]⁺.

¹H NMR (400 MHz, CD₃OD-*d*) *δ* 7.61 (dd, *J =* 6.4, 1.6 Hz, 2H), 7.42 (dd, *J* = 6.4, 1.6 Hz, 2H), 7.28-7.21 (m, 4H), 4.67-4.38 (m, 2H), 4.32-4.09 (m, 1H), 3.93-3.77 (m, 4H), 3.68-3.52 (m, 4H), 3.49-3.37 (m, 1H), 2.90-2.82 (m, 2H), 2.25 (s, 3H).

### Examples A0041&A0112

### (Z)-(7-(Methoxyimino)-2-oxa-5-azaspiro[3.4]octan-5-yl)(2'-methyl-[1, 1'-biphenyl]-4-yl)methanone

### (E)-(7-(Methoxyimino)-2-oxa-5-azaspiro[3.4]octan-5-yl)(2'-methyl-[1, 1'-biphenyl]-4-yl)methanone

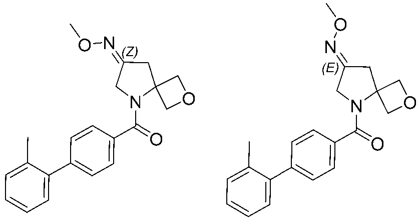

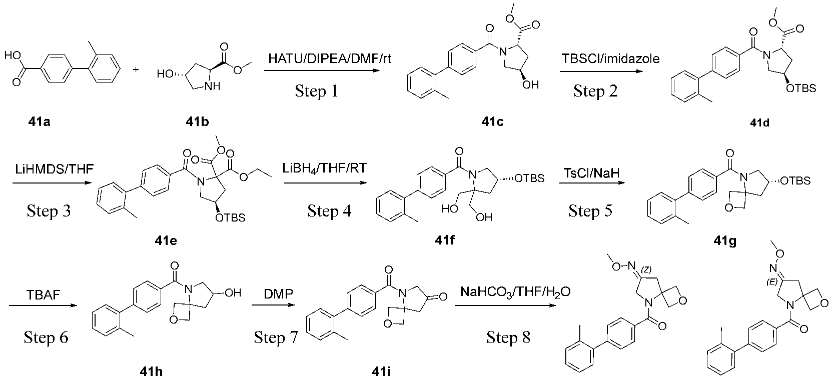

### Step 1

### Methyl (2S, 4R)-4-hydroxy-1-(2'-methyl-[1, 1'-biphenyl]-4-carbonyl)pyrrolidine-2-carboxylate 41c

The starting materials 41a (3 g, 14.13 mmol) and 41b (2.46 g, 16.96 mmol) were dissolved in 50 mL of DMF, and then HATU (6.98 g, 18.37 mmol) and ethyldiisopropylamine (7.30 g, 56.52 mmol) were added, and the reaction solution was reacted at room temperature for 3 hours. Saturated ammonium chloride solution was then added to quench the reaction solution. The mixture was extracted with ethyl acetate. The organic phases were combined, washed with sodium chloride aqueous solution, dried over anhydrous sodium sulphate, and filtered, and the filtrate was concentrated under reduced pressure to afford a residue, which was purified by silica gel column chromatography to afford product 41c (4.5 g, colourless oil, yield: 93.8%).

MS *m*/*z* (ESI): 340.1 [M+1]⁺.

### Step 2

### Methyl (2S, 4R)-4-((tert-butyldimethylsilyl)oxy)-1-(2'-methyl-[1, 1'-biphenyl]-4-carbonyl)pyrrolidine-2-carboxylate 41d

The starting material 41c (4.5 g, 13.26 mmol) and imidazole (1.81 g, 26.52 mmol) were dissolved in 100 mL of DMF, and then tert-butyldimethylsilyl chloride (2.44 g, 16.18 mmol) was added. The reaction solution was reacted at room temperature for 16 hours. Sodium bicarbonate solution was then added to quench the reaction solution. The mixture was extracted with ethyl acetate. The organic phases were combined, washed with sodium chloride aqueous solution, dried over anhydrous sodium sulphate, and filtered, and the filtrate was concentrated under reduced pressure to afford a residue, which was purified by silica gel column chromatography to afford product 41d (4.2 g, white solid, yield: 69.8%).

MS *m*/*z* (ESI): 454.2 [M+1]⁺.

### Step 3

### (4R)-2-Ethyl-2-methyl-4-((tert-butyldimethylsilyl)oxy)-1-(2'-methyl-[1, 1'-biphenyl]-4-carbonyl)pyrrolidine-2,2-dicarboxylate 41e

The starting material 41d (5.1 g, 11.24 mmol) was dissolved in 50 mL of tetrahydrofuran. After nitrogen purging, the reaction system was cooled to -70°C, and then lithium di(propan-2-yl)azanide (1.81 g, 16.86 mmol) was added, and the mixture was reacted for 1.5 hours. Ethyl chloroformate (2.44 g, 22.48 mmol) was added, and the reaction system was reacted at -70°C for 16 hours. 100 mL of saturated ammonium chloride was then added in an ice bath to quench the reaction solution. The mixture was extracted with ethyl acetate (50 mL × 3). The organic phases were combined, washed with sodium chloride aqueous solution (50 mL × 3), dried over anhydrous sodium sulphate, and filtered, and the filtrate was concentrated under reduced pressure to afford a residue, which was purified by silica gel column chromatography to afford product 41e (5.3 g, colourless oil, yield: 89.7%).

MS *m*/*z* (ESI): 526.1 [M+1]⁺.

### Step 4

### (R)-(4-((Tert-butyldimethylsilyl)oxy)-2,2-bis(hydroxymethyl)pyrrolidin-1-yl)(2'-methyl-[1, 1'-biphenyl]-4-yl)methanone 41f

The starting material 41e (5 g, 9.51 mmol) was dissolved in 40 mL of tetrahydrofuran, and then lithium borohydride (414.26 mg, 19.02 mmol) was added, and the reaction solution was reacted at room temperature for 16 hours. Then, 100 mL of water was added to the reaction solution to quench the reaction solution, and the mixture was extracted twice with ethyl acetate and washed with saturated sodium chloride aqueous solution. The organic phase was collected, dried over anhydrous sodium sulphate, filtered, and concentrated to afford a crude product, which was purified by column chromatography to afford product 41f(1.2 g, white solid, yield: 27.7%).

MS *m*/*z* (ESI): 456.4[M+1]⁺.

### Step 5

### (R)-(7-((Tert-butyldimethylsilyl)oxy)-2-oxa-5-azaspiro[3.4]octan-5-yl)(2'-methyl-[1, 1'-biphenyl]-4-yl)methanone 41g

The starting material 41f (450 mg, 0.99 mmol) was dissolved in 10 mL of tetrahydrofuran. After nitrogen purging, 60% sodium hydride (118.80 mg, 4.95 mmol) was slowly added in an ice bath, and the mixture was stirred in an ice bath for 15 minutes. p-Toluenesulfonyl chloride (0.19 g, 0.99 mmol) was then added, and the reaction solution was maintained in an ice bath and reacted for 2 hours. Subsequently, the reaction solution was slowly heated to room temperature and reacted overnight. Then, 20 mL of water was added, and the mixture was extracted with ethyl acetate (20 mL × 3). The organic phases were combined, washed with sodium chloride aqueous solution (20 mL × 3), dried over anhydrous sodium sulphate, and filtered, and the filtrate was concentrated under reduced pressure to afford a crude product, which was separated and purified by preparative chromatography to afford product 41g (38 mg, white solid, yield: 8.8%).

MS *m*/*z* (ESI): 438.1[M+1]⁺.

### Step 6

### (7-Hydroxy-2-oxa-5-azaspiro[3.4]octan-5-yl)(2'-methyl-[1, 1'-biphenyl]-4-yl)methanone 41h

The starting material 41g (35 mg, 0.080 mmol) was dissolved in 1 mL of tetrahydrofuran, and then tetrabutylammonium fluoride (0.10 g, 0.40 mmol) was added, and the reaction solution was reacted at 25°C for 2 hours. Then, 5 mL of water was added, and the mixture was extracted with ethyl acetate (5 mL × 3). The organic phases were combined, washed with sodium chloride aqueous solution (5 mL × 3), dried over anhydrous sodium sulphate, and filtered, and the filtrate was concentrated under reduced pressure to afford a residue, which was purified by silica gel column chromatography to afford product 41h (24 mg, white solid, yield: 92.8%).

MS *m*/*z* (ESI): 324.2[M+1]⁺.

### Step 7

### 5-(2'-Methyl-[1, 1'-biphenyl]-4-carbonyl)-2-oxa-5-azaspiro[3.4]octan-7-one 41i

The starting material 41h (24 mg, 0.074 mmol) was dissolved in 2 mL of dichloromethane, and then Dess-Martin reagent (0.063 g, 0.15 mmol) was added, and the reaction solution was reacted at 25°C for 16 hours. Then, 5 mL of water was added, and the mixture was extracted with dichloromethane (5 mL × 3). The organic phases were combined, washed with sodium chloride aqueous solution (5 mL × 3), dried over anhydrous sodium sulphate, and filtered, and the filtrate was concentrated under reduced pressure to afford product 41i (23 mg, white solid, yield: 96.4%).

MS *m*/*z* (ESI): 322.0 [M+1]⁺.

### Step 8

### (Z)-(7-(Methoxyimino)-2-oxa-5-azaspiro[3.4]octan-5-yl)(2'-methyl-[1, 1'-biphenyl]-4-yl)methanone

### (E)-(7-(Methoxyimino)-2-oxa-5-azaspiro[3.4]octan-5-yl)(2'-methyl-[1, 1'-biphenyl]-4-yl)methanone

The starting material 41i (24 mg, 0.066 mmol) was dissolved in 0.5 mL of tetrahydrofuran and 0.5 mL of water, and then methoxyammonium chloride (0.019 g, 0.22 mmol) and sodium bicarbonate (0.14 g, 1.65 mmol) were added, and the reaction solution was reacted at 25°C for 16 hours. Then, 5 mL of water was added to the reaction system, and the mixture was extracted three times with ethyl acetate. The organic phases were combined, washed twice with saturated sodium chloride aqueous solution, dried over anhydrous sodium sulphate, filtered, and concentrated to afford a crude product. The crude product was separated by silica gel Prep-TLC (EA:PE=3:1) to afford the less polar product A0041 (Rf=0.4) and the more polar product A0112 (Rf=0.3). Further purification by HPLC yielded the less polar product A0041 (3.16 mg, yield: 12.1%) and the more polar product A0112 (4.93 mg, yield: 18.8%).

A0041: MS *m*/*z* (ESI): 351.3 [M+1]⁺.

¹H NMR (400 MHz, Chloroform-d) *δ* 7.55-7.53 (m, 2H), 7.40-7.38 (m, 2H), 7.30-7.24 (m, 4H), 5.76-5.71 (m, 2H), 4.55 (d, *J* = 5.6 Hz, 2H), 4.19 (d, *J =* 14.8 Hz, 2H), 3.86 (d, *J =* 11.2 Hz, 3H), 3.28 (d, *J* = 31.2 Hz, 2H), 2.25 (d, *J =* 10.0 Hz, 3H).

A0112: MS *m*/*z* (ESI): 351.3 [M+1]⁺.

¹H NMR (400 MHz, Chloroform-d) *δ* 7.55-7.53 (m, 2H), 7.40-7.38 (m, 2H), 7.30-7.24 (m, 4H), 5.76-5.71 (m, 2H), 4.55 (d, *J* = 6.4 Hz, 2H), 4.17 (d, *J* = 32.4 Hz, 2H), 3.89-3.84 (m, 3H), 3.30 (d, *J =* 31.2 Hz, 2H), 2.27 (d, *J* = 9.6 Hz, 3H).

### Example A0066

### (S, EZ)-(2-(1-Hydroxycyclopropyl)-4-(methoxyimino)pyrrolidin-1-yl)(2'-methyl-[1,1'-biphenyl]-4-yl)methanone A0066

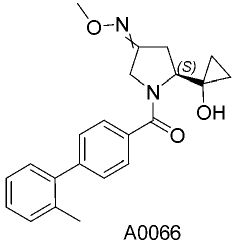

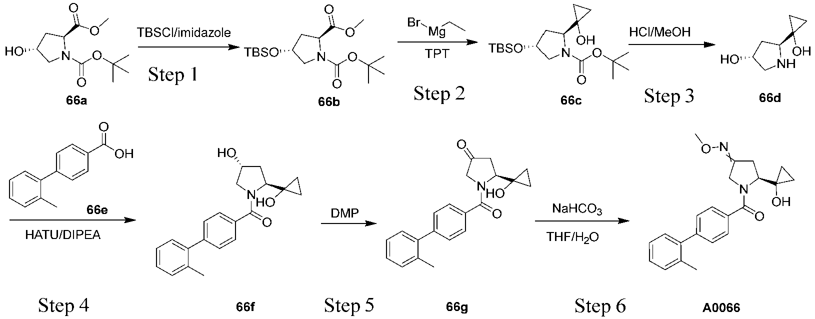

### Step 1

### (2S, 4R)-1-Tert-butyl-2-methyl-4-((tert-butyldimethylsilyl)oxy)pyrrolidine-1,2-dicarboxylate 66b

The starting material 66a (10 g, 40.77 mmol) and imidazole (5.55 g, 81.54 mmol) were dissolved in 100 mL of DMF, and then tert-butyldimethylsilyl chloride (7.50 g, 49.74 mmol) was added. The reaction solution was reacted at room temperature for 16 hours. Saturated sodium bicarbonate solution was then added to quench the reaction solution, and the mixture was extracted with ethyl acetate. The organic phases were combined, washed with sodium chloride aqueous solution, dried over anhydrous sodium sulphate, and filtered, and the filtrate was concentrated under reduced pressure to afford a residue, which was purified by silica gel column chromatography to afford product 66b (14 g, colourless oil, yield: 95.5%).

MS *m*/*z* (ESI): 360.0 [M+1]⁺.

### Step 2

### Tert-butyl (2S, 4R)-4-((tert-butyldimethylsilyl)oxy)-2-(1-hydroxycyclopropyl)pyrrolidine-1-carboxylate 66c

66b (2 g, 5.56 mmol) and titanium tetraisopropoxide (0.32 g, 1.11 mmol) were dissolved in 15 mL of tetrahydrofuran, and ethylmagnesium bromide (3.70 g, 27.80 mmol) was added dropwise at 0°C (via syringe pump at a rate of 40 mL/h for a total volume of 27.8 mL). 10 minutes after the dropwise addition was completed, the ice bath was removed, and the mixture was reacted at room temperature (25°C) for 24 hours. The reaction solution was slowly poured into an ice-cold saturated ammonium chloride solution, stirred for half an hour, extracted three times with ethyl acetate, concentrated, and purified by column chromatography to afford product 66c (0.730 g, yellow solid, yield: 18%).

MS *m*/*z* (ESI): 258.2 [M+1]⁺.

### Step 3

### Tert-butyl (2S, 4R)-4-hydroxy-2-(1-hydroxycyclopropyl)pyrrolidine-1-carboxylate 66d

The starting material 66c (1.09 g, 3.05 mmol) was dissolved in 10.89 mL of hydrogen chloride methanol solution, and the reaction solution was reacted at 50°C for 30 minutes. Then, the mixture was concentrated to remove the solvent to afford crude product 66d (440 mg, black oil).

MS *m*/*z* (ESI): 144.3 [M+1]⁺.

### Step 4

### ((2S, 4R)-4-Hydroxy-2-(1-hydroxycyclopropyl)pyrrolidin-1-yl)(2'-methyl-[1, 1'-biphenyl]-4-yl)methanone 66f

The starting materials 66d (440 mg, 3.07 mmol) and 66e (0.78 g, 3.68 mmol) were dissolved in 20 mL of DMF, and then HATU (1.52 g, 3.99 mmol) and ethyldiisopropylamine (1.59 g, 12.28 mmol) were added, and the reaction solution was reacted at room temperature for 16 hours. Then, 20 mL of water was added, and the mixture was extracted three times with ethyl acetate. The organic phases were combined, dried over anhydrous sodium sulphate, and filtered, and the filtrate was concentrated under reduced pressure to afford a residue, which was purified by silica gel column chromatography to afford product 66f (600 mg).

MS *m*/*z* (ESI): 338.3 [M+1]⁺.

### Step 5

### (S)-5-(1-Hydroxycyclopropyl)-1-(2'-methyl-[1, 1'-biphenyl]-4-carbonyl)pyrrolidin-3-one 66g

66f (300 mg, 0.89 mmol) was dissolved in 5 mL of dichloromethane, and then Dess-Martin reagent (0.75 g, 1.78 mmol) was added, and the reaction solution was reacted at 25°C for 16 hours. 10 mL of saturated sodium bicarbonate solution was added to the reaction solution to quench the reaction solution, and the mixture was extracted twice with ethyl acetate and washed with saturated sodium chloride aqueous solution. The organic phase was collected, dried over anhydrous sodium sulphate, filtered, and concentrated to afford a crude product, which was purified by column chromatography to afford product 66g (210 mg, colourless oil, yield: 70.4%).

MS *m*/*z* (ESI): 336.0 [M+1]⁺.

### Step 6

### (S, EZ)-(2-(1-Hydroxycyclopropyl)-4-(methoxyimino)pyrrolidin-1-yl)(2'-methyl-[1,1'-biphenyl]-4-yl)methanone A0066

The starting material 66g (100 mg, 0.30 mmol) and methoxyammonium chloride (0.07 g, 0.90 mmol) were dissolved in 1 mL of tetrahydrofuran and 1 mL of water, and then sodium bicarbonate (0.076 g, 0.90 mmol) was added, and the reaction solution was reacted at 25°C for 16 hours. 5 mL of water was added to the reaction system, and the mixture was extracted three times with ethyl acetate. The organic phases were combined, dried over anhydrous sodium sulphate, filtered, and concentrated to afford a crude product, which was purified by column chromatography to afford product A0066 (100 mg, yield: 92.0%).

MS *m*/*z* (ESI): 365.1 [M+1]⁺.

¹H NMR (400 MHz, Chloroform-d) *δ* 7.58 (d, *J =* 7.6 Hz, 2H), 7.41 (d, *J* = 6.8 Hz, 2H), 7.30-7.18 (m, 4H), 5.30-5.20 (m, 1H), 4.42-4.30 (m, 2H), 3.90-3.85 (m, 3H), 2.99-2.67 (m, 4H), 2.28 (d, *J* = 8.8 Hz, 3H), 1.18-1.14 (m, 2H).

### Example A0082

### (EZ)-(3-(Methoxyimino)-8,8-dimethyl-7,9-dioxa-1-azaspiro[4.5]decan-1-yl)(2'-methyl-[1, 1'-biphenyl]-4-yl)methanone A0082

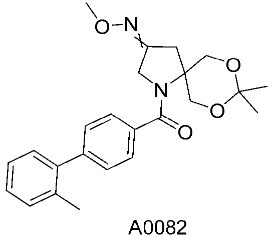

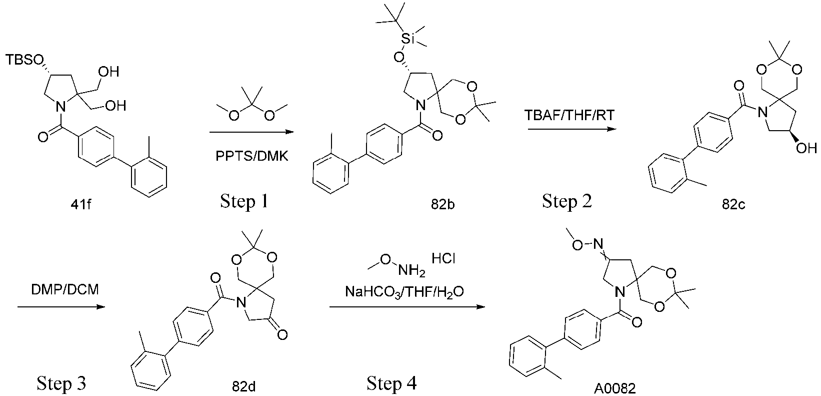

### Step 1

### (R)-(3-((Tert-butyldimethylsilyl)oxy)-8,8-dimethyl-7,9-dioxa-1-azaspiro[4.5]decan-1-yl)(2'-methyl-[1, 1'-biphenyl]-4-yl)methanone 82b

The starting material 41f (370 mg, 0.81 mmol) was dissolved in 20 mL of acetone, and then 2,2-dimethoxypropane (0.42 g, 4.05 mmol) and pyridin-1-ium 4-methylbenzenesulfonate (40.71 mg, 0.16 mmol) were added, and the reaction solution was reacted at room temperature for 16 hours. Then, 20 mL of water was added to the reaction solution to quench the reaction solution, and the mixture was extracted twice with ethyl acetate and washed with saturated sodium chloride aqueous solution. The organic phase was collected, dried over anhydrous sodium sulphate, filtered, and concentrated to afford a crude product, which was purified by column chromatography to afford product 82b (303 mg, white solid, yield: 75.3%).

MS *m*/*z* (ESI): 438.2 [M+1]⁺.

### Step 2

### (R)-(3-Hydroxy-8,8-dimethyl-7,9-dioxa-1-azaspiro[4.5]decan-1-yl)(2'-methyl-[1, 1'-biphenyl]-4-yl)methanone 82c

The starting material 82b (240 mg, 0.48 mmol) was dissolved in 10 mL of tetrahydrofuran, and the mixture was purged with nitrogen. Tetrabutylammonium fluoride (0.26 mL, 0.96 mmol) was then added, and the reaction system was reacted at room temperature for 5 hours and reacted at 45°C for 1 hour. Then, 10 mL of water was added, and the mixture was extracted with ethyl acetate (10 mL × 3). The organic phases were combined, washed with sodium chloride aqueous solution (10 mL × 3), dried over anhydrous sodium sulphate, and filtered, and the filtrate was concentrated under reduced pressure to afford a crude product, which was purified by silica gel column chromatography to afford product 82c (170 mg, white solid, yield: 92.0%).

MS *m*/*z* (ESI): 382.1[M+1]⁺.

### Step 3

### 8,8-Dimethyl-1-(2'-methyl-[1, 1'-biphenyl]-4-carbonyl)-7,9-dioxa-1-azaspiro[4.5]decan-3-one 82d

The starting material 82c (170 mg, 0.45 mmol) was dissolved in 5 mL of dichloromethane, and then Dess-Martin reagent (0.38 g, 0.90 mmol) was added, and the reaction solution was reacted at 25°C for 5 hours. 10 mL of water was added to the reaction solution, and the mixture was extracted twice with dichloromethane and washed with saturated sodium chloride aqueous solution. The organic phase was collected, dried over anhydrous sodium sulphate, filtered, and concentrated to afford a crude product, which was purified by column chromatography to afford product 82d (177 mg, white solid, yield: 100%).

MS *m*/*z* (ESI): 380.1[M+1]⁺.

### Step 4

### (EZ)-(3-(Methoxyimino)-8,8-dimethyl-7,9-dioxa-1-azaspiro[4.5]decan-1-yl)(2'-methyl-[1, 1'-biphenyl]-4-yl)methanone A0082

The starting material 82d (147 mg, 0.39 mmol) and methoxyamine hydrochloride (0.098 g, 1.17 mmol) were dissolved in 1 mL of tetrahydrofuran and 1 mL of water, and then sodium bicarbonate (0.098 g, 1.17 mmol) was added, and the reaction solution was reacted at 25°C for 16 hours. 5 mL of water was added to the reaction system, and the mixture was extracted three times with ethyl acetate. The organic phases were combined, dried over anhydrous sodium sulphate, filtered, and concentrated to afford a crude product, which was purified by column chromatography to afford product A0082 (100 mg, white solid, yield: 63.2%).

MS *m*/*z* (ESI): 351.2 [M+1]⁺.

¹H NMR (400 MHz, CD₃OD) *δ* 7.53 (dd, *J* = 8.0, 1.6 Hz, 2H), 7.43 (d, *J* = 7.6 Hz, 2H), 7.30-7.21 (m, 4H), 5.12 (s, 2H), 4.25 (d, *J =* 14.8 Hz, 2H), 3.84 (d, *J* = 22.0 Hz, 3H), 3.53 (t, *J =* 10.8 Hz, 2H), 3.16 (d, *J* = 16.4 Hz, 2H), 2.27 (d, *J* = 2.8 Hz, 3H), 1.64 (s, 3H), 1.40 (s, 3H).

### Examples A0040&A0083

### (E)-(2,2-Bis(hydroxymethyl)-4-(methoxyimino)pyrrolidin-1-yl)(2'-methyl-[1, 1'-biphenyl]-4-yl)methanone

### (Z)-(2,2-Bis(hydroxymethyl)-4-(methoxyimino)pyrrolidin-1-yl)(2'-methyl-[1, 1'-biphenyl]-4-yl)methanone

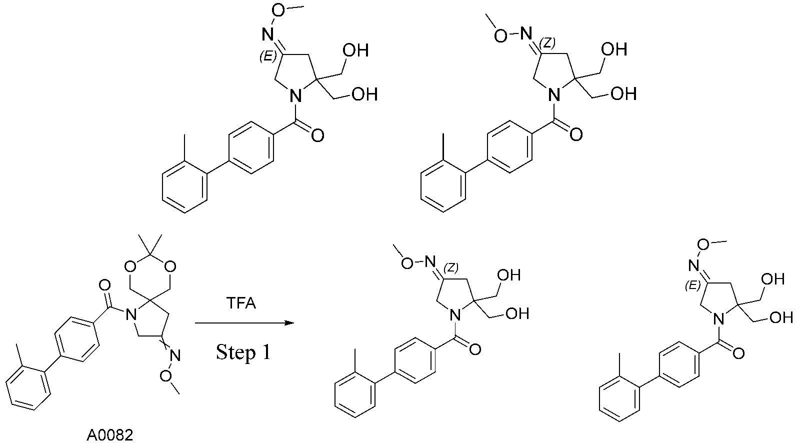

### Step 1

### (Z)-(2,2-Bis(hydroxymethyl)-4-(methoxyimino)pyrrolidin-1-yl)(2'-methyl-[1, 1'-biphenyl]-4-yl)methanone

### (E)-(2,2-Bis(hydroxymethyl)-4-(methoxyimino)pyrrolidin-1-yl)(2'-methyl-[1, 1'-biphenyl]-4-yl)methanone

The starting material A0082 (40 mg, 0.098 mmol) was dissolved in 0.5 mL of dichloromethane, and then trifluoroacetic acid was added, and the mixture was reacted at 25°C for 1 hour. Then, the reaction solution was concentrated to remove the solvent to afford a crude product. The crude product was dissolved in dichloromethane and washed twice with saturated sodium bicarbonate solution. The organic phase was collected, dried over anhydrous sodium sulphate, filtered, and concentrated to afford a crude product, which was separated and purified by HPLC to afford the more polar product A0040 (retention time: 1.835 min, 5.61 mg, yield: 14.3%) and the less polar product A0083 (retention time: 1.843 min, 3.53 mg, yield: 8.9%). HPLC conditions:
Instrument: Shimadzu preparative liquid chromatograph SIL-10AP
Chromatographic column: Welch Xtimate C18, 21.2*250 mm, 10 um
Mobile phase: A: 0.05% NH₃.H₂O in water; B: acetonitrile
Column temperature: room temperature
Flow rate: 20 ml/min
Detection wavelength: 210 nm

A0040: MS *m*/*z* (ESI): 369.3 [M+1]⁺.

¹H NMR (400 MHz, Chloroform-d) *δ* 7.46 (d, *J =* 8.4 Hz, 2H), 7.40 (dd, *J* = 8.4, 2.0 Hz, 2H), 7.30-7.25 (m, 4H), 4.26 (s, 2H), 4.08 (d, *J* = 12.0 Hz, 2H), 3.85-3.79 (m, 5H), 2.73 (s, 2H), 2.28 (s, 3H).

A0083: MS *m*/*z* (ESI): 369.3 [M+1]⁺.

¹H NMR (400 MHz, Chloroform-d) *δ* 7.52-7.38 (m, 4H), 7.30-7.19 (m, 4H), 4.26 (s, 2H), 4.10-4.06 (m, 2H), 3.88-3.80 (m, 5H), 2.75 (d, *J* = 14.8 Hz, 2H), 2.26 (d, *J* = 10.4 Hz, 3H).

### Examples A0225&A0234

### (S, Z)-4'-(2-(Hydroxymethyl)-5-(methoxyimino)piperidine-1-carbonyl)-2-methyl-[1,1'-biphenyl]-3-carbonitrile

### (S, E)-4'-(2-(Hydroxymethyl)-5-(methoxyimino)piperidine-1-carbonyl)-2-methyl-[1,1'-biphenyl]-3-carbonitrile

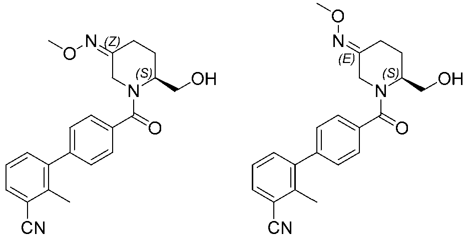

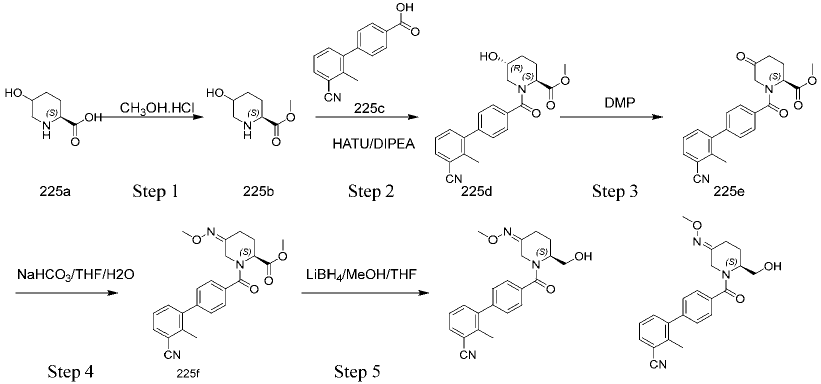

### Step 1

### Methyl (2S)-5-hydroxypiperidine-2-carboxylate 225b

225a (500 mg, 3.44 mmol) was dissolved in hydrogen chloride methanol solution (5 mL), and the reaction solution was reacted at 65°C for 2 hours. The reaction solution was concentrated to afford crude 225b (530 mg, yield: 96.66%).

MS *m*/*z* (ESI): 160.1 [M+1]⁺.

### Step 2

### Methyl (2S, 5R)-1-(3'-cyano-2'-methyl-[1,1'-biphenyl]-4-carbonyl)-5-hydroxypiperidine-2-carboxylate 225d

225c (500 mg, 2.11 mmol) and 225b (400 mg, 2.53 mmol) were dissolved in DMF (10 mL). HATU (1.6 g, 4.22 mmol) and ethyldiisopropylamine (1.09 g, 8.44 mmol) were then added, and the reaction solution was reacted at room temperature for 16 hours. Ethyl acetate was added to the reaction solution, and the mixture was washed with saturated sodium chloride aqueous solution. The organic phase was dried over anhydrous sodium sulphate and concentrated, and the crude product was purified by column chromatography (petroleum ether/ethyl acetate = 2:1), to afford 225d (750 mg, yield: 94.04%).

MS *m*/*z* (ESI): 379.2 [M+1]⁺.

### Step 3

### Methyl (S)-1-(3'-cyano-2'-methyl-[1,1'-biphenyl]-4-carbonyl)-5-oxopiperidine-2-carboxylate 225e

225d (860 mg, 2.27 mmol) was dissolved in dichloromethane (20 mL), and then Dess-Martin reagent (1.93 g, 4.54 mmol) was added, and the reaction solution was stirred at 25°C for 16 hours. Then, the reaction solution was concentrated under reduced pressure to afford a crude product, which was purified by column chromatography to afford 225e (330 mg, yield: 31.63%).

MS *m*/*z* (ESI): 377.2 [M+1]⁺.

### Step 4

### Methyl (S, Z)-1-(3'-cyano-2'-methyl-[1,1'-biphenyl]-4-carbonyl)-5-(methoxyimino)piperidine-2-carboxylate 225f

225e (500 mg, 1.33 mmol) was dissolved in tetrahydrofuran (10 mL) and water (0.5 mL), and then methoxyammonium chloride (0.33 g, 3.99 mmol) and sodium bicarbonate (0.34 g, 3.99 mmol) were added, and the reaction solution was stirred at 25°C for 16 hours. Then, 20 mL of water was added to the reaction system, and the mixture was extracted with ethyl acetate, washed with saturated sodium chloride aqueous solution, dried over anhydrous sodium sulphate, filtered, and concentrated to afford crude product 225f (300 mg, yield: 55.7%).

MS *m*/*z* (ESI): 406 [M+1]⁺.

### Step 5

### (S, Z)-4'-(2-(Hydroxymethyl)-5-(methoxyimino)piperidine-1-carbonyl)-2-methyl-[1,1'-biphenyl]-3-carbonitrile

### (S, E)-4'-(2-(Hydroxymethyl)-5-(methoxyimino)piperidine-1-carbonyl)-2-methyl-[1,1'-biphenyl]-3-carbonitrile

225f (300 mg, 0.74 mmol) was dissolved in tetrahydrofuran (2 mL) and methanol (2 mL), and then lithium borohydride (24.18 mg, 1.11 mmol) was added, and the reaction solution was stirred at room temperature for 16 hours. The reaction solution was diluted with ethyl acetate, and washed with sodium chloride aqueous solution. The organic phase was dried over anhydrous sodium sulphate and concentrated, and the crude product was separated by silica gel Prep-TLC (EA:PE=2:1) to afford the less polar isomer A0234 (Rf=0.25) and the more polar isomer A0225 (Rf=0.2). Further purification by preparative HPLC and lyophilization yielded the more polar product A0225 (20.06 mg, yield: 6.89%) and the less polar product A0234 (18.79 mg, yield: 6.34%).

A0225: MS *m*/*z* (ESI): 378.0 [M+1]⁺.

¹H NMR (400 MHz, Chloroform-*d*) *δ* 7.64 (d, *J* = 4.0 Hz, 1H), 7.57-7.42 (m, 3H), 7.39-7.30 (m, 3H), 5.35-5.32 (m, 1H), 4.65-4.48 (m, 6H), 4.25-4.05 (m, 1H), 3.56-3.22 (m, 5H), 3.29-3.06 (m, 1H), 2.91-2.73 (m, 1H).

A0234: MS *m*/*z* (ESI): 378.0 [M+1]⁺.

¹H NMR (400 MHz, Chloroform-*d*) *δ* 7.64 (d, *J* = 8.0 Hz, 1H), 7.53 (d, *J* = 8.0 Hz, 2H), 7.47-7.43 (m, 1H), 7.34-7.25 (m, 3H), 4.65-4.58 (m, 1H), 4.29-4.22 (m, 1H), 3.99-3.95 (m, 1H), 3.93-3.72 (m, 5H), 3.55-3.49 (m, 1H), 2.81-2.77 (m, 1H), 2.45 (s, 3H), 2.08-2.02 (m, 1H), 1.81-1.76 (m, 1H).

### Examples A0229&A0233

### (2S, E)-1-(3'-Cyano-2'-methyl-[1,1'-biphenyl]-4-carbonyl)-N-(2-hydroxy-2-phenethyl)-4-(methoxyimino)pyrrolidine-2-carboxamide

### (2S, Z)-1-(3'-Cyano-2'-methyl-[1,1'-biphenyl]-4-carbonyl)-N-(2-hydroxy-2-phenethyl)-4-(methoxyimino)pyrrolidine-2-carboxamide

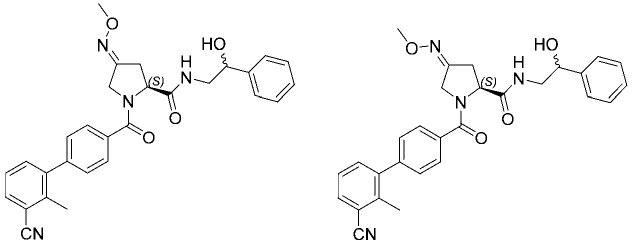

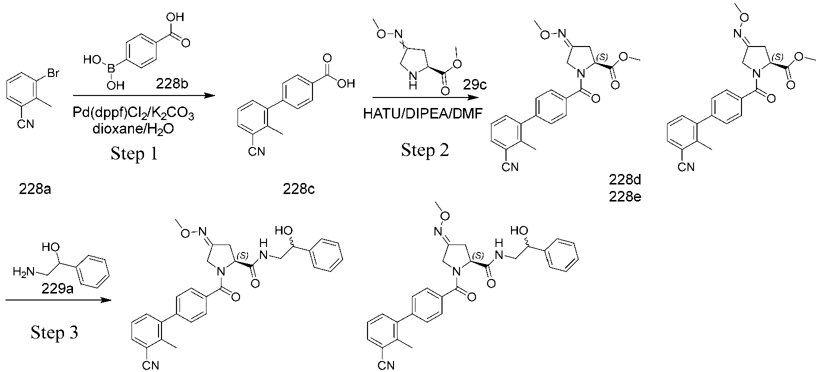

According to Step 1 to Step 2 in the aforementioned synthetic route, with reference to the synthetic method of compound C0057, the more polar product 228d (Rf=0.3, 75 mg, yield: 15.15%) and the less polar product 228e (Rf=0.4, 80 mg, yield: 16.16%) were obtained.

MS *m*/*z* (ESI): 392.2 [M+1]⁺.

### Step 3

### (2S, Z)-1-(3 '-Cyano-2'-methyl-[1,1'-biphenyl]-4-carbonyl)-N-(2-hydroxy-2-phenethyl)-4-(methoxyimino)pyrrolidine-2-carboxamide

### (2S, E)-1-(3'-Cyano-2'-methyl-[1,1 '-biphenyl]-4-carbonyl)-N-(2-hydroxy-2-phenethyl)-4-(methoxyimino)pyrrolidine-2-carboxamide

The more polar 228d (50 mg, 0.13 mmol) from the previous step and 229a (0.089 g, 0.65 mmol) were dissolved in acetonitrile (1 mL), and the mixture was reacted at 65°C for 16 hours. The reaction solution was purified by preparative HPLC and lyophilized to afford product A0229 (10.02 mg, yield: 15.33%).

A0229: MS *m*/*z* (ESI): 497.0 [M+1]⁺.

¹H NMR (400 MHz, Chloroform-*d*) *δ* 7.66 (d, *J =* 8.0 Hz, 1H), 7.58-7.52 (m, 2H), 7.45-7.30 (m, 9H), 7.26-7.23 (m, 1H), 5.23-5.14 (m, 1H), 4.91-4.82 (m, 1H), 4.38-4.18 (m, 2H), 3.88 (s, 3H), 3.82-3.64 (m, 1H), 3.51-3.30 (m, 2H), 2.94-2.82 (m, 1H), 2.48-2.41 (m, 3H).

The less polar 228e (55 mg, 0.14 mmol) from the previous step and 229a (0.19 g, 1.40 mmol) were dissolved in acetonitrile (1 mL), and the mixture was reacted at 70°C for 8 hours. The reaction solution was purified by preparative HPLC and lyophilized to afford product A0233 (27.94 mg, yield: 38.87%).

A0233: MS *m*/*z* (ESI): 497.0 [M+1]⁺.

¹H NMR (400 MHz, Chloroform-*d*) *δ* 7.67 (d, *J* = 8.0 Hz, 1H), 7.58-7.52 (m, 2H), 7.45-7.29 (m, 9H), 7.26-7.13 (m, 1H), 5.23-5.13 (m, 1H), 4.90-4.87 (m, 1H), 4.43-4.20 (m, 2H), 3.87 (s, 3H), 3.82-3.62 (m, 1H), 3.49-3.18 (m, 2H), 2.98-2.83 (m, 1H), 2.45 (d, *J* = 8.4 Hz, 3H).

### Examples A0228&A0232

### (S, Z)-1-(3'-Cyano-2'-methyl-[1,1'-biphenyl]-4-carbonyl)-4-(methoxyimino)-N-methylpyrrolidine-2-carboxamide

### (S, E)-1-(3'-Cyano-2'-methyl-[1,1'-biphenyl]-4-carbonyl)-4-(methoxyimino)-N-methylpyrrolidine-2-carboxamide

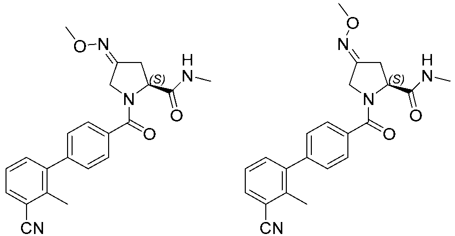

With reference to Examples A0229&A0233, by replacing the starting material 229a with methylamine ethanol solution, A0228&A0232 were obtained, respectively.

A0228: MS *m*/*z* (ESI): 391.0 [M+1]⁺.

¹H NMR (400 MHz, Chloroform-*d*) *δ* 7.67-7.59 (m, 3H), 7.41-7.35 (m, 4H), 6.86-6.82 (m, 1H), 5.23-5.15 (m, 1H), 4.32 (dd, *J =* 44.8, 15.2 Hz, 2H), 3.86 (d, *J =* 8.8 Hz, 3H), 3.47-3.42 (m, 1H), 2.89-2.82 (m, 4H), 2.46 (d, *J* = 8.4 Hz, 3H).

A0232: MS *m*/*z* (ESI): 391.0 [M+1]⁺.

¹H NMR (400 MHz, Chloroform-*d*) *δ* 7.67-7.61 (m, 3H), 7.45-7.34 (m, 4H), 6.84-6.76 (m, 1H), 5.19 (d, *J* = 7.6 Hz, 1H), 4.34 (dd, *J* = 44.8, 15.6 Hz, 2H), 3.89-3.80 (m, 3H), 3.40-3.26 (m, 1H), 2.90-2.83 (m, 4H), 2.46 (d, *J* = 8.8 Hz, 3H).

### Examples C0145&C0146

### (S, E)-3-(2-(Hydroxymethyl)-4-(methoxyimino)pyrrolidine-1-carbonyl)-6,7-dihydrodibenzo[b,d]oxepine-8-carbonitrile

### (S, Z)-3-(2-(Hydroxymethyl)-4-(methoxyimino)pyrrolidine-1-carbonyl)-6,7-dihydrodibenzo[b,d]oxepine-8-carbonitrile

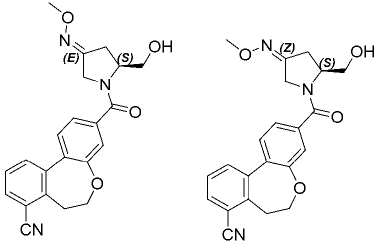

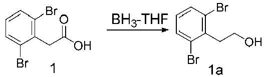

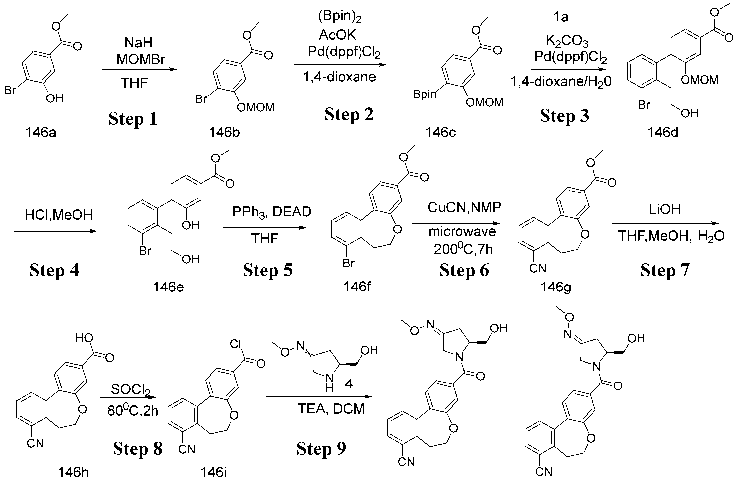

### Intermediate 1a

### 2-(2,6-Dibromophenyl)ethan-1-ol

1 (5 g, 17.01 mmol) was dissolved in tetrahydrofuran (50 mL), and the mixture was purged three times with nitrogen. Borane tetrahydrofuran solution (1 M, 25.5 mL) was slowly added dropwise at 0°C. After the dropwise addition was completed, the reaction solution was heated to 80°C and reacted for 1 hour. After the reaction was completed as monitored by LCMS, the reaction flask was cooled in ice water, water and methanol were added to quench the reaction solution, and the mixture was subjected to rotary evaporation to remove a portion of the solvent, and extracted three times with ethyl acetate. The organic phase was collected and subjected to rotary evaporation to remove the solvent to afford crude product 1a (4.2 g, yield: 88.19%).

MS *m*/*z* (ESI): 303.8 [M+Na]⁺

### Step 1

### Methyl 4-bromo-3-(methoxymethoxy)benzoate 146b

To a solution of 146a (4 g, 17.31 mmol) in tetrahydrofuran (40 mL) was added NaH (1.73 g, 43.27 mmol), and the mixture was stirred for 30 minutes at 0°C under nitrogen protection. Subsequently, bromomethyl methyl ether (3.24 g, 25.96 mmol) was added, and the reaction solution was heated to room temperature and stirred for another 30 minutes. After the reaction was completed, the reaction solution was poured into ice water and extracted three times with ethyl acetate. The organic phase was collected, anhydrous sodium sulphate was added, and the mixture was filtered and concentrated. The residue was purified by silica gel Pre-TLC (PE/EA=10:1, Rf=0.5) to afford product 146b (4.6 g, yield: 96.6%).

MS *m*/*z* (ESI): 275.1 [M+1]⁺.

### Step 2

### Methyl 3-(methoxymethoxy)-4-(4,4,5,5-tetramethyl-1,3,2-dioxaborolan-2-yl)benzoate 146c

To a solution of 146b (4.6 g, 16.72 mmol) in 1,4-dioxane (50 mL) were added bis(pinacolato)diboron (5.94 g, 23.41 mmol), potassium acetate (3.28 mg, 33.44 mmol) and 1,1-bis(diphenylphosphino)ferrocene palladium(II) dichloride (1.22 g, 1.67 mmol). After nitrogen purging, the mixture was reacted at 90°C for 3 hours. After the reaction was completed, the reaction solution was poured into water and extracted three times with ethyl acetate. The organic phases were combined, washed with saturated sodium chloride aqueous solution, dried over anhydrous sodium sulphate, and subjected to evaporation to remove the solvent. The residue was purified by silica gel Pre-TLC (PE/EA=10:1, Rf=0.5) to afford product 146c (4.8 g, yield: 89.11%).

MS *m*/*z* (ESI): 323.0 [M+1]⁺.

### Step 3

### Methyl 3'-bromo-2'-(2-hydroxyethyl)-2-(methoxymethoxy)-[1,1'-biphenyl]-4-carboxylate 146d

To a solution of 146c (4.8 g, 14.90 mmol) in 1,4-dioxane (42 mL) and water (7 mL) were added 1a (4.59 g, 16.39 mmol), potassium carbonate (4.12 g, 29.80 mmol) and 1,1-bis(diphenylphosphino)ferrocene palladium(II) dichloride (1.09 g, 1.49 mmol). After nitrogen purging, the mixture was reacted at 80°C for 3 hours. After the reaction was completed, the reaction solution was poured into water and extracted three times with ethyl acetate. The organic phases were combined, washed with saturated sodium chloride aqueous solution, dried over anhydrous sodium sulphate, and subjected to evaporation to remove the solvent. The residue was purified by silica gel Pre-TLC (PE/EA=6:1, Rf=0.6) to afford product 146d (1.8 g, yield: 30.57%).

MS *m*/*z* (ESI): 416.8 [M+Na]⁺.

### Step 4

### Methyl 3'-bromo-2-hydroxy-2'-(2-hydroxyethyl)-[1,1'-biphenyl]-4-carboxylate 146e

146d (2 g, 5.06 mmol) was dissolved in 4M methanol hydrogen chloride solution (20 mL), and the mixture was reacted at room temperature for 2 hours. After the reaction was completed, the reaction solution was concentrated under reduced pressure, and the resulting residue was adjusted to pH = 7 with saturated sodium bicarbonate aqueous solution and extracted twice with ethyl acetate. The organic phases were combined, washed with saturated sodium chloride aqueous solution, dried over anhydrous sodium sulphate, and filtered, and the filtrate was concentrated under reduced pressure to afford product 146e (1.3 g, yield: 73.0%).

MS *m*/*z* (ESI): 350.9 [M+1]⁺.

### Step 5

### Methyl 8-bromo-6,7-dihydrodibenzo[b, d]oxirane-3-carboxylate 146f

To a solution of 146e (1.3 g, 3.70 mmol) in tetrahydrofuran (80 mL) was added triphenylphosphine (1.36 g, 5.18 mmol), diisopropyl azodicarboxylate (897.81 mg, 4.44 mmol) was slowly added dropwise under nitrogen protection, and the mixture was reacted at room temperature for 16 hours. After the reaction was completed, the reaction solution was poured into ice water and extracted three times with ethyl acetate. The organic phase was collected, anhydrous sodium sulphate was added, and the mixture was filtered and concentrated. The crude product was purified by normal-phase silica gel column chromatography (PE/EA=8:1 to 1:1) to afford product 146f (670 mg, yield: 54.3%).

MS *m*/*z* (ESI): 332.8 [M+1]⁺.

### Step 6

### Methyl 8-cyano-6,7-dihydrodibenzo[b, d]oxepine-3-carboxylate 146g

To a solution of 146f (650 mg, 1.95 mmol) in *N*-methylpyrrolidone (12 mL) was added cuprous cyanide (180 mg, 2.01 mmol), and the mixture was heated to 200°C under microwave under nitrogen protection and reacted for 7 hours. After the reaction was completed, the reaction solution was poured into ice water and extracted three times with ethyl acetate. The organic phase was collected, anhydrous sodium sulphate was added, and the mixture was filtered and concentrated to afford a crude product, which was purified by normal-phase silica gel column chromatography (PE/EA=8:1 to 3:1) to afford product 146g (350 mg, yield: 64.24%).

MS *m*/*z* (ESI): 280.0 [M+1]⁺.

### Step 7

### 8-Cyano-6,7-dihydrodibenzo[b, d]oxepine-3-carboxylic acid 146h

To a solution of 146g (350 mg, 1.13 mmol, 90%) in tetrahydrofuran/methanol/water (6 mL/6 mL/4 mL) was added sodium hydroxide (226 mg, 5.65 mmol), and the mixture was reacted at room temperature for 16 hours. After the reaction was completed, the reaction solution was poured into ice water, adjusted to pH 4-5 with dilute hydrochloric acid, and extracted three times with ethyl acetate. The organic phase was collected, anhydrous sodium sulphate was added, and the mixture was filtered and concentrated to afford crude 146h (300 mg, yield: 90.25%).

MS *m*/*z* (ESI): 266.0 [M+1]⁺.

### Step 8

### 8-Cyano-6,7-dihydrodibenzo[[b, d]oxepine-3-carbonyl chloride 146i

146h (300 mg, 1.13 mmol, 90%) was dissolved in thionyl chloride (6 mL), and the mixture was heated to 80°C and reacted for 2 hours. After the reaction was completed, the reaction solution was concentrated to afford crude 146i (300 mg, yield: 93.5%), which was directly used in the next reaction.

### Step 9

### (S, Z)-3-(2-(Hydroxymethyl)-4-(methoxyimino)pyrrolidine-1-carbonyl)-6,7-dihydrodibenzo[b,d]oxepine-8-carbonitrile

### (S, E)-3-(2-(Hydroxymethyl)-4-(methoxyimino)pyrrolidine-1-carbonyl)-6,7-dihydrodibenzo[b,d]oxepine-8-carbonitrile

To a solution of 146i (300 mg, 0.95 mmol, 90%) in DCM (10 mL) were added 4 (240 mg, 1.50 mmol, 90%) and triethylamine (480 mg, 4.75 mmol), and the reaction solution was reacted at room temperature for 16 hours. Water (50 mL) was added to the reaction solution, and the mixture was extracted with ethyl acetate (40 mL × 3). The organic phases were combined, washed with saturated sodium chloride aqueous solution (70 mL × 3), dried over sodium sulphate, and subjected to evaporation to remove the solvent. The residue was purified by silica gel Pre-TLC (PE:EA=1:5) to afford the less polar isomer C0145 (Rf=0.6, 43.40 mg, yield: 10.39%) and the more polar isomer C0146 (Rf=0.55, 51.12 mg, yield: 12.01%).
C0145: MS *m*/*z* (ESI): 392.1 [M+1]⁺
¹H NMR (400 MHz, CD₃OD-*d*₄) *δ* 7.78-7.76 (m, 2H), 7.60-7.58 (m, 2H), 7.48 (d, *J* = 6.4 Hz, 1H), 7.35 (s, 1H), 4.87-4.07 (m, 5H), 3.90-3.38 (m, 5H), 3.12-2.86 (m, 4H).
C0146: MS *m*/*z* (ESI): 392.1 [M+1]⁺
¹H NMR (400 MHz, CD₃OD-*d*₄) *δ* 7.80-7.77 (m, 2H), 7.67-7.57 (m, 2H), 7.48 (d, *J* = 7.2 Hz, 1H), 7.35 (s, 1H), 4.76-4.05 (m, 5H), 3.91-3.38 (m, 5H), 3.14-2.63 (m, 4H).

### Examples C0136&C0137

### (S,E)-(8-(3-(Difluoromethyl)-2-methylphenyl)-2,3-dihydrobenzo[b][1,4]dioxin-5-yl)(2-(hydroxymethyl)-4-(methoxyimino)pyrrolidin-1-yl)methanone

### (S,Z)-(8-(3-(Difluoromethyl)-2-methylphenyl)-2,3-dihydrobenzo[b][1,4]dioxin-5-yl)(2-(hydroxymethyl)-4-(methoxyimino)pyrrolidin-1-yl)methanone

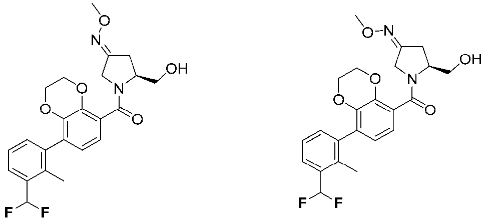

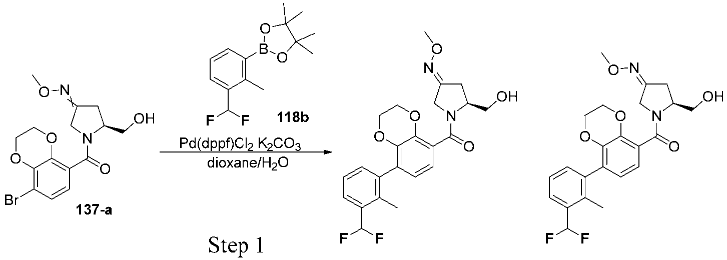

### Step 1

### (S,E)-(8-(3-(Difluoromethyl)-2-methylphenyl)-2,3-dihydrobenzo[b][1,4]dioxin-5-yl)(2-(hydroxymethyl)-4-(methoxyimino)pyrrolidin-1-yl)methanone

### (S,Z)-(8-(3-(Difluoromethyl)-2-methylphenyl)-2,3-dihydrobenzo[b][1,4]dioxin-5-yl)(2-(hydroxymethyl)-4-(methoxyimino)pyrrolidin-1-yl)methanone

With reference to the synthetic route of common intermediate A-4, by replacing diiodomethane with dibromoethane, compound 137-a was obtained. 137-a (235 mg, 0.61 mmol), 118b (245.32 mg, 0.92 mmol), Pd(dtbpf)Cl₂ (39.76 mg, 0.061 mmol) and potassium carbonate (129.31 mg, 1.22 mmol) were dissolved in a mixed solution of 1,4-dioxane (5 mL) and water (0.5 mL). After nitrogen purging, the mixture was heated to 65°C and reacted for 2 hours. After the reaction was completed, water (10 mL) was added to the reaction solution, and the mixture was extracted with ethyl acetate (20 mL × 3). The organic phases were combined, washed with saturated sodium chloride aqueous solution (10 mL × 3), dried over sodium sulphate, and subjected to evaporation to remove the solvent. The residue was purified by silica gel Pre-TLC (PE:EA=1:5) to afford the less polar isomer (Rf=0.6) C0136 (80.05 mg, yield: 26.01%) and the more polar isomer (Rf=0.55) (C0137) (72.45 mg, yield: 24.59%).
**C0136:** MS *m*/*z* (ESI): 447.1 [M+1]⁺, Rt= 3.372 min
¹H NMR (CD₃OD-*d*₄, 400 MHz) δ 7.52 (d, *J* = 7.2 Hz, 1H), 7.38-7.22 (m, 2H), 7.13-6.74 (m, 3H), 4.70-3.96 (m, 7H), 3.93-3.66 (m, 4H), 3.47-3.37 (m, 1H), 3.04-2.80 (m, 2H), 2.20 (s, 3H).
**C0137:** MS *m*/*z* (ESI): 447.1 [M+1]⁺, Rt= 3.298 min
¹H NMR (CD₃OD-*d*₄, 400 MHz) δ 7.52 (d, *J* = 7.2 Hz, 1H), 7.34-7.25 (m, 2H), 7.09-6.78 (m, 3H), 4.71-3.98 (m, 7H), 3.88-3.71 (m, 4H), 3.45-3.39 (m, 1H), 2.97-2.67 (m, 2H).

### Examples C0138&C0139

### 4-Fluoro-3-(7-((S,E)-2-(hydroxymethyl)-4-(methoxyimino)pyrrolidine-1-carbonyl)benzo[d][1,3]dioxol-4-yl)-2-methylbenzonitrile

### 4-Fluoro-3-(7-((S,Z)-2-(hydroxymethyl)-4-(methoxyimino)pyrrolidine-1-carbonyl)benzo[d][1,3]dioxol-4-yl)-2-methylbenzonitrile

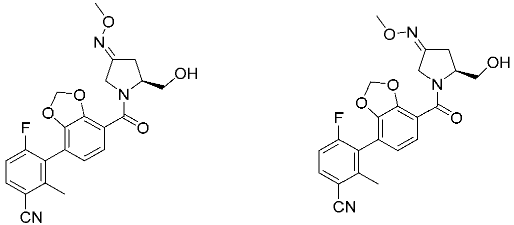

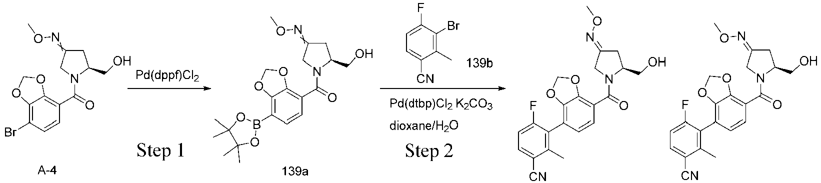

### Step 1

### (S,EZ)-(7-(2-(Hydroxymethyl)-4-(methoxyimino)pyrrolidine-1-carbonyl)benzo[d][1,3]dioxol-4-yl)boronic acid 139a

A-4 (1.0 g, 2.69 mmol), bis(pinacolato)diboron (1.37 g, 5.38 mmol) and potassium acetate (527 mg, 5.38 mmol) were dissolved in 1,4-dioxane (20 mL). After nitrogen purging, Pd(dppf)Cl₂ (219 mg, 0.27 mmol) was added at room temperature, and the mixture was reacted at 80°C for 3 hours. After the reaction was completed, the reaction solution was poured into a mixed solvent of ethyl acetate and water. After layer separation, the aqueous phase was extracted again, and the organic phases were combined, washed once with sodium chloride aqueous solution, dried, filtered, and subjected to rotary evaporation to dryness. The residue was purified by silica gel column chromatography (eluting with a gradient of 10:1 to 3:1 petroleum ether:ethyl acetate) to afford 139a (620 mg, yield: 55%). MS *m*/*z* (ESI): 419.2 [M+1]⁺.

### Step 2

### 4-Fluoro-3-(7-((S,E)-2-(hydroxymethyl)-4-(methoxyimino)pyrrolidine-1-carbonyl)benzo[d][1,3]dioxol-4-yl)-2-methylbenzonitrile

### 4-Fluoro-3-(7-((S,Z)-2-(hydroxymethyl)-4-(methoxyimino)pyrrolidine-1-carbonyl)benzo[d][1,3]dioxol-4-yl)-2-methylbenzonitrile

139a (200 mg, 0.54 mmol), 139b (139 mg, 0.65 mmol), Pd(dtbpf)Cl₂ (35.19 mg, 0.061 mmol) and potassium carbonate (223.90 mg, 1.62 mmol) were dissolved in a mixed solution of 1,4-dioxane (20 mL) and water (2 mL). After nitrogen purging, the mixture was heated to 65°C and reacted for 2 hours. After the reaction was completed, water (20 mL) was added to the reaction solution, and the mixture was extracted with ethyl acetate (30 mL × 3). The organic phases were combined, washed with saturated sodium chloride aqueous solution (20 mL × 3), dried over sodium sulphate, and subjected to evaporation to remove the solvent. The residue was purified by silica gel Pre-TLC (PE:EA=1:5) to afford the less polar isomer C0138 (42 mg, yield: 18.34%, Rf=0.6) and the more polar isomer C0139 (44 mg, yield: 19.21%, Rf=0.5).
**C0138:** MS *m*/*z* (ESI): 426.0 [M+1]⁺, Rt= 3.052 min
¹H NMR (CD₃OD-*d*₄, 400 MHz) δ 7.80 (dd, *J* = 8.8, 5.2 Hz, 1H), 7.24 (t, *J* = 8.8 Hz, 1H), 7.08 (d, *J* = 8.0 Hz, 1H), 6.86 (d, *J* = 8.0 Hz, 1H), 6.13-6.05 (m, 2H), 4.83-4.14 (m, 3H), 3.87-3.68 (m, 4H), 3.41-3.40 (m, 1H), 2.90-2.86 (m, 2H), 2.41-2.38 (m, 3H).
**C0139:** MS *m*/*z* (ESI): 426.0 [M+1]⁺, Rt= 3.000 min
¹H NMR (CD₃OD-*d*₄, 400 MHz) δ 7.80 (dd, *J* = 8.8, 5.2 Hz, 1H), 7.24 (t, *J* = 8.8 Hz, 1H), 7.08 (d, *J* = 8.0 Hz, 1H), 6.86 (d, *J* = 8.0 Hz, 1H), 6.13-6.05 (m, 2H), 4.83-4.16 (m, 3H), 3.88-3.69 (m, 4H), 3.41-3.40 (m, 1H), 3.26-2.42 (m, 2H), 2.42-2.40 (m, 3H).

### Example C0142

### (Z/E)-9-(2-(Hydroxymethyl)-4-(methoxyimino)pyrrolidine-1-carbonyl)-5,7-dihydrodibenzo[c,e]oxepine-4-carbonitrile

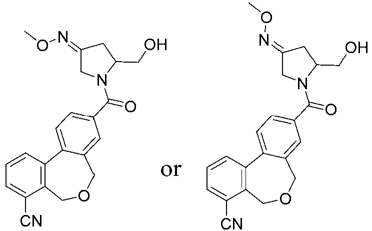

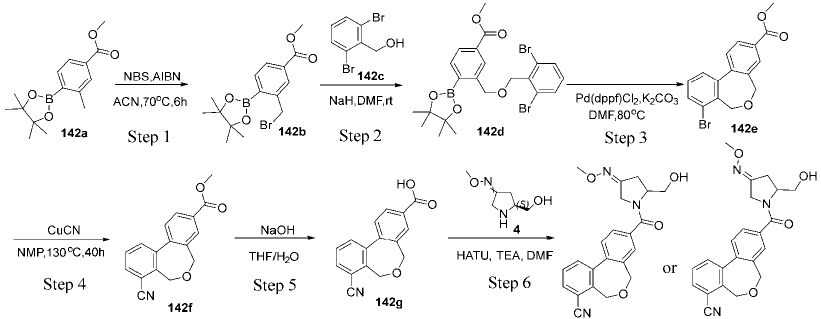

### Step 1

### Methyl 3-(bromomethyl)-4-(4,4,5,5-tetramethyl-1,3,2-dioxaborolan-2-yl)benzoate 142b

142a (4.0 g, 14.49 mmol) was dissolved in acetonitrile (80 mL), NBS (2.58 g, 14.49 mmol) and 2,2'-azobis(2-methylpropionitrile) (23.8 g, 0.14 mmol) were added at room temperature, and the reaction solution was stirred at 70°C for 6 hours. After the reaction was completed, the reaction solution was concentrated, and the residue was loaded onto a silica gel column, eluting with a gradient of (20:1 to 15:1 PE/EA). The eluate was collected to afford product 142b (4.8 g, yield: 85.87%).

MS *m*/*z* (ESI): 354.8 [M+1]⁺.

### Step 2

### Methyl 3-(((2,6-dibromobenzyl)oxy)methyl)-4-(4,4,5,5-tetramethyl-1,3,2-dioxaborolan-2-yl)benzoate 142d

142c (1.6 g, 6.02 mmol) was dissolved in DMF (40 mL), sodium hydride (360 mg, 9 mmol, purity: 60%) was added at 0°C under nitrogen protection, and the mixture was stirred for 0.5 h. 142b (2.8 g, 7.49 mmol) was added, and the mixture was stirred at room temperature for 16 hours. After the reaction was completed, water was added to quench the reaction solution, and the reaction solution was poured into water/ethyl acetate (100 mL/100 mL). The organic phase was separated, washed with saturated sodium chloride aqueous solution (100mL × 3), collected, dried over anhydrous sodium sulphate, and filtered, and the filtrate was collected and concentrated to afford a crude product. The crude product was purified by normal-phase silica gel column chromatography (PE:EA=20:1-12:1) to afford 142d (1.0 g, yield: 24.71%).

MS *m*/*z* (ESI): 539.6 [M+1]⁺.

### Step 3

### Methyl 8-bromo-5,7-dihydrodibenzo[c,e]oxepine-3-carboxylate 142e

142d (1.0 g, 1.85 mmol) and potassium carbonate (645 mg, 4.67 mmol) were dissolved in DMF (20 mL), and Pd(dppf)Cl₂ (136 mg, 0.19 mmol) was added at room temperature under nitrogen protection. The reaction solution was stirred at 80°C under nitrogen protection for 2.0 hours. The reaction solution was poured into a mixed solvent of ethyl acetate and water (100 mL/100 mL). The organic phase was separated, and the aqueous phase was extracted with ethyl acetate (2×100 mL). The organic layers were combined, washed with saturated NaCl solution (3×500 mL), combined, dried over anhydrous sodium sulphate, filtered, and concentrated to afford a crude product, which was loaded onto a 2-mm TLC plate, eluting with a gradient of (4:1 PE/EA) to afford product 142e (250 mg, yield: 40.52%). MS *m*/*z* (ESI): 333.0 [M+1]⁺.

### Step 4

### Methyl 8-cyano-5,7-dihydrodibenzo[c,e]oxepine-3-carboxylate 142f

142e (250 mg, 0.75 mmol) was dissolved in NMP (6 mL), cuprous cyanide (75 mg, 0.84 mmol) was added at room temperature, and the mixture was reacted at 130°C for 40 hours. The reaction solution was filtered, and the filtrate was loaded onto C18 reversed-phase silica gel column, eluting with a gradient of (ACN/H₂O = 25/75 to 80/20). The eluate was collected to afford 142f (100 mg, yield: 47.72%). MS *m*/*z* (ESI): 279.9 [M+1]⁺.

### Step 5

### 8-Cyano-5,7-dihydrodibenzo[c,e]oxepine-3-carboxylic acid 142g

142f (95 mg, 0.34 mmol) and sodium hydroxide (55 mg, 1.38 mmol) were dissolved in a mixed solution of tetrahydrofuran (4 mL) and water (4 mL), and the mixture was stirred at room temperature for 16 hours. The reaction solution was concentrated, and the residue was dissolved in a mixed solution of ethyl acetate and water. The layers were separated, and the aqueous phase was adjusted to pH 3-4 with dilute hydrochloric acid and extracted with ethyl acetate (3×50 mL). The organic layers were combined, washed with saturated NaCl solution (3×70 mL), dried over anhydrous sodium sulphate, filtered, and concentrated to afford 142g (80 mg, yield: 88.66%).

MS *m*/*z* (ESI): 266.0 [M+1]⁺.

### Step 6

### (Z/E)-9-(2-(Hydroxymethyl)-4-(methoxyimino)pyrrolidine-1-carbonyl)-5,7-dihydrodibenzo[c,e]oxepine-4-carbonitrile C0142

142g (60 mg, 0.23 mmol) and HATU (96 mg, 0.25 mmol) were dissolved in DMF (2 mL), triethylamine (0.12 g, 1.15 mmol) and 4 (60 mg, 0.37 mmol) were added at room temperature, and the mixture was stirred at room temperature for 2 hours. Water (50 mL) was added to the reaction solution, and the mixture was extracted with ethyl acetate (40 mL × 3). The organic phases were combined, washed with saturated sodium chloride aqueous solution (70 mL × 3), dried over sodium sulphate, and filtered, and the filtrate was collected and concentrated. The residue was purified by silica gel Pre-TLC (PE:EA =1:5) to afford the more polar isomer (Rf=0.55) C0142 (10.38 mg, yield: 11.01%).
**C0142:** MS *m*/*z* (ESI): 392.1 [M+1]⁺, Rt= 2.739 min
¹H NMR (CD₃OD-*d*₄, 400 MHz) δ 7.96 (d, *J* = 7.6 Hz, 1H), 7.89 (dd, *J* = 8.0, 1.2 Hz, 1H), 7.75-7.70 (m, 4H), 4.78-4.07 (m, 7H), 3.89-3.36 (m, 5H), 3.04-2.90 (m, 1H), 2.78-2.61 (m, 1H).

### Examples C0143&C0144

### (S,E)-5-Fluoro-3-(7-(2-(hydroxymethyl)-4-(methoxyimino)pyrrolidine-1-carbonyl)benzo[d][1,3]dioxol-4-yl)-2-methylbenzonitrile

### (S,Z)-5-Fluoro-3-(7-(2-(hydroxymethyl)-4-(methoxyimino)pyrrolidine-1-carbonyl)benzo[d][1,3]dioxol-4-yl)-2-methylbenzonitrile

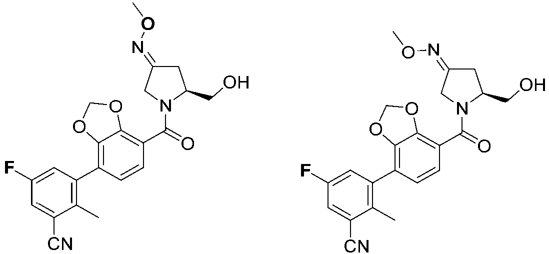

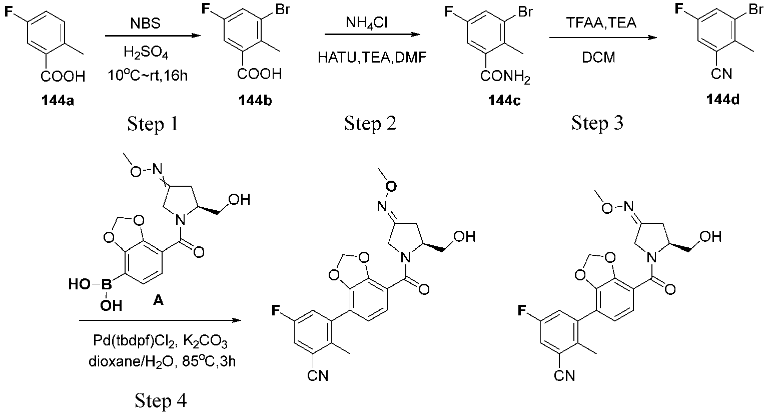

### Step 1

### 3-Bromo-5-fluoro-2-methylbenzoic acid 144b

144a (10 g, 64.88 mmol) was dissolved in concentrated sulphuric acid (80 mL), NBS (11.55 g, 64.88 mmol) was added portionwise at 0°C, and the reaction solution was stirred at 0°C for 3h, and then heated to room temperature and stirred for 16h. After the reaction was completed, the reaction solution was added dropwise to ice water, and ethyl acetate (150 mL) was added. The organic phase was separated, and the aqueous phase was extracted with ethyl acetate (3 × 80 mL). The organic layers were combined, washed with saturated NaCl solution (3 × 100 mL), combined, dried over Na₂SO₄, filtered, and concentrated. The crude product was purified by silica gel column chromatography, eluting with a gradient of (16:1 to 10:1 dichloromethane: methanol) to afford 144b (6.2 g, yield: 22.56%, purity: 50%).

MS *m*/*z* (ESI): 230.9 [M-1]⁺.

### Step 2

### 3-Bromo-5-fluoro-2-methylbenzamide 144c

144b (6.2 g, 13.30 mmol, 50%) and HATU (11.13 g, 29.26 mmol) were dissolved in DMF (60 mL), triethylamine (10.77 g, 106.4 mmol) and ammonium chloride (2.56 g, 47.88 mmol) were added at room temperature, and the mixture was reacted at room temperature overnight. The reaction solution was filtered, and the filtrate was purified by C18 reversed-phase column chromatography (ACN:H₂O=15:85 to 60:40) to afford 144c (2.0 g, yield: 64.79%).

MS *m*/*z* (ESI): 231.8 [M+1]⁺.

### Step 3

### 3-Bromo-5-fluoro-2-methylbenzonitrile 144d

144c (2 g, 7.93 mmol) was dissolved in dichloromethane (50 mL), triethylamine (1.20 g, 11.89 mmol) was added at room temperature under nitrogen protection, trifluoroacetic anhydride (2.50 g, 11.89 mmol) was added dropwise at 0°C, and the mixture was stirred at room temperature for 16 h. The reaction solution was concentrated, and the residue was dissolved in a mixed solution of ethyl acetate and water. The organic phase was collected, and the aqueous phase was extracted with ethyl acetate (3 × 50 mL). The organic layers were combined, washed with saturated NaCl solution (3 × 70 mL), combined, dried over Na₂SO₄, and filtered, and the filtrate was collected and concentrated. The residue was loaded onto a silica gel column, eluting with a gradient of (10:1 PE/EA). The eluate was collected and concentrated to afford 144d (1.4 g, yield: 75.89%).

### Step 4

### (S,E)-5-Fluoro-3-(7-(2-(hydroxymethyl)-4-(methoxyimino)pyrrolidine-1-carbonyl)benzo[d][1,3]dioxol-4-yl)-2-methylbenzonitrile

### (S,Z)-5-Fluoro-3-(7-(2-(hydroxymethyl)-4-(methoxyimino)pyrrolidine-1-carbonyl)benzo[d][1,3]dioxol-4-yl)-2-methylbenzonitrile

A (200 mg, 0.60 mmol), 144d (150 mg, 0.70 mmol) and potassium carbonate (250 mg, 1.80 mmol) were dissolved in 1,4-dioxane (5 mL) and water (1 mL), and Pd(dtbpf)Cl₂ (39 mg, 0.06 mmol) was added at room temperature under nitrogen protection. After nitrogen purging, the mixture was reacted at 85°C for 3 h. Water (50 mL) was added to the reaction solution, and the mixture was extracted with ethyl acetate (40 mL × 3). The organic phases were combined, washed with saturated sodium chloride aqueous solution (70 mL × 3), dried over sodium sulphate, and subjected to evaporation to remove the solvent. The residue was purified by silica gel Pre-TLC (PE:EA=1:5) to afford the less polar isomer (Rf=0.6) C0143 (43.32 mg, yield: 15.16%) and the more polar isomer (Rf=0.55) C0144 (60.88 mg, yield: 23.02%).
**C0143:** MS *m*/*z* (ESI): 426.0 [M+1]⁺, Rt= 3.552 min
¹H NMR (CD₃OD-*d*₄, 400 MHz) δ 7.56 (dd, *J* = 8.0, 2.8 Hz, 1H), 7.42-7.39 (m, 1H), 7.07 (d, *J* = 8.4 Hz, 1H), 6.89 (d, *J* = 8.4 Hz, 1H), 6.18-6.08 (m, 2H), 4.76-4.11 (m, 3H), 3.89-3.38 (m, 5H), 2.97-2.82 (m, 2H), 2.42 (s, 3H).
**C0144:** MS *m*/*z* (ESI): 440.0 [M+1]⁺, Rt= 2.370 min
¹H NMR (CD₃OD-*d*₄, 400 MHz) δ 7.56 (dd, *J* = 8.0, 2.4 Hz, 1H), 7.42-7.38 (m, 1H), 7.07 (d, *J =* 8.0 Hz, 1H), 6.89 (d, *J* = 8.4 Hz, 1H), 6.14-6.08 (m, 2H), 4.78-4.11 (m, 3H), 3.88-3.38 (m, 5H), 3.03-2.89 (m, 1H), 2.81-2.58 (m, 1H), 2.42 (s, 3H).

### Examples C0147&C0148

### (S,Z)-6-Fluoro-3-(8-(2-(hydroxymethyl)-4-(methoxyimino)pyrrolidine-1-carbonyl)-2,3-dihydrobenzo[b][1,4]dioxin-5-yl)-2-methylbenzonitrile

### (S,E)-6-Fluoro-3-(8-(2-(hydroxymethyl)-4-(methoxyimino)pyrrolidine-1-carbonyl)-2,3-dihydrobenzo[b][1,4]dioxin-5-yl)-2-methylbenzonitrile

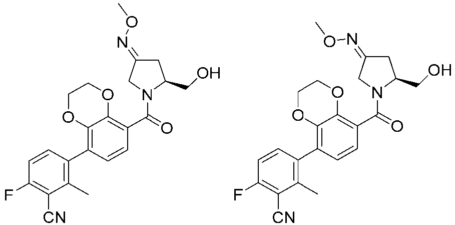

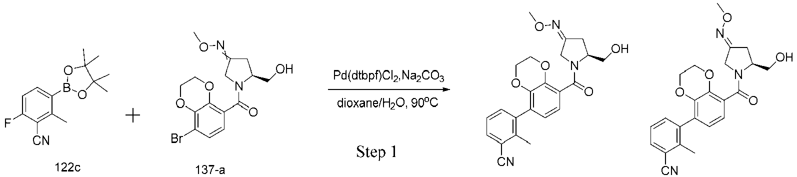

### Step 1

### (S,Z)-6-Fluoro-3-(8-(2-(hydroxymethyl)-4-(methoxyimino)pyrrolidine-1-carbonyl)-2,3-dihydrobenzo[b][1,4]dioxin-5-yl)-2-methylbenzonitrile

### (S,E)-6-Fluoro-3-(8-(2-(hydroxymethyl)-4-(methoxyimino)pyrrolidine-1-carbonyl)-2,3-dihydrobenzo[b][1,4]dioxin-5-yl)-2-methylbenzonitrile

137-a (250 mg, 0.65 mmol), 122c (255 mg, 0.98 mmol), Pd(dtbpf)Cl₂ (42 mg, 0.065 mmol) and sodium carbonate (138 mg, 1.3 mmol) were dissolved in a mixed solution of 1,4-dioxane (50 mL) and water (5 mL), and the mixture was purged three times with nitrogen, heated to 65°C under nitrogen protection and reacted for 2 hours. Water (50 mL) was added to the reaction solution, and the mixture was extracted with ethyl acetate (50 mL × 2). The organic phases were combined, washed with saturated sodium chloride aqueous solution (150 mL × 2), dried over sodium sulphate, and subjected to evaporation to remove the solvent. The residue was purified by silica gel Pre-TLC (EA) to afford the less polar isomer C0147 (Rf=0.6) (71 mg, yield: 23.79%) and the more polar isomer C0148 (Rf=0.5) (81 mg, yield: 27.71%).
**C0147:** MS *m*/*z* (ESI): 440.0 [M+1]⁺, Rt= 3.146 min
¹H NMR (400 MHz, DMSO-*d*₆) *δ* 7.60-7.56 (m, 1H), 7.41 (t, *J* = 8.8 Hz, 1H), 6.93-6.88 (m, 1H), 6.79-6.76 (m, 1H), 4.95-4.92 (m, 1H), 4.49-4.25 (m, 5H), 4.09-3.87 (m, 2H), 3.81-3.77 (m, 3H), 3.55-3.54 (m, 1H), 3.23-3.21 (m,1H), 2.79-2.67 (m, 2H), 2.31 (s, 3H).
**C0148:** MS *m*/*z* (ESI): 440.0 [M+1]⁺, Rt= 3.097 min
¹H NMR (400 MHz, DMSO-*d*₆) *δ* 7.61-7.57 (m, 1H), 7.41 (t, *J* = 8.8 Hz, 1H), 6.92-6.89 (m, 1H), 6.79-6.77 (m, 1H), 4.97-4.92 (m, 1H), 4.47-4.25 (m, 5H), 4.08-3.86 (m, 2H), 3.81-3.73 (m, 3H), 3.57-3.54 (m, 1H), 3.26-3.18 (m,1H), 2.90-2.84 (m, 1H), 2.67-2.55 (m, 1H), 2.31 (s, 3H).

### Examples C0149&C0150

### 4-Fluoro-3-(8-((S,E)-2-(hydroxymethyl)-4-(methoxyimino)pyrrolidine-1-carbonyl)-2,3-dihydrobenzo[b][1,4]dioxin-5-yl)-2-methylbenzonitrile

4-Fluoro-3-(8-((*S,Z*)-2-(hydroxymethyl)-4-(methoxyimino)pyrrolidine-1-carbonyl)-2,3-dihydrobenzo[*b*][1,4]dioxin-5-yl)-2-methylbenzonitrile

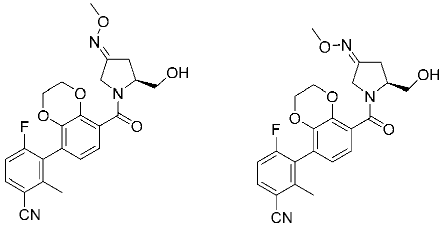

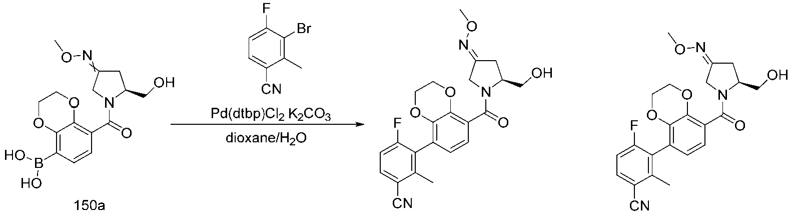

### Step 1

### 4-Fluoro-3-(8-((S,E)-2-(hydroxymethyl)-4-(methoxyimino)pyrrolidine-1-carbonyl)-2,3-dihydrobenzo[b][1,4]dioxin-5-yl)-2-methylbenzonitrile

### 4-Fluoro-3-(8-((S,Z)-2-(hydroxymethyl)-4-(methoxyimino)pyrrolidine-1-carbonyl)-2,3-dihydrobenzo[b][1,4]dioxin-5-yl)-2-methylbenzonitrile

With reference to the synthetic route of common intermediate A, by replacing diiodomethane with dibromoethane, compound 150a was obtained. To a solution of 150a (207.49 mg, 0.48 mmol) in 1,4-dioxane (15 mL) and water (1.5 mL) were added 3-bromo-4-fluoro-2-methylbenzonitrile (123.28 mg, 0.58 mmol), Pd(dtbpf)Cl₂ (31.28 mg, 0.048 mmol) and potassium carbonate (132.68 mg, 0.96 mmol). After nitrogen purging, the mixture was reacted at 65°C for 3 h. Water (10 mL) was added to the reaction solution, and the mixture was extracted with ethyl acetate (20 mL × 3). The organic phases were combined, washed with saturated sodium chloride aqueous solution (10 mL × 3), dried over sodium sulphate, and subjected to evaporation to remove the solvent. The residue was purified by silica gel Pre-TLC (PE:EA=1:3) to afford the less polar isomer (Rf=0.6) C0149 (34 mg, yield: 15.69%) and the more polar crude isomer (Rf=0.5). The more polar crude isomer was purified by preparative HPLC and lyophilized to afford the more polar product C0150 (34 mg, yield: 15.69%).
**C0149:** MS *m*/*z* (ESI): 439.44 [M+1]⁺, Rt= 3.011 min
¹H NMR (CD₃OD-*d*₄, 400 MHz) δ 7.75 (dd, *J* = 8.4, 5.2 Hz, 1H), 7.20 (t, *J =* 8.6 Hz, 1H), 7.08-6.91 (m, 1H), 6.82 (d, *J* = 7.6 Hz, 1H), 4.66-4.48 (m, 1H), 4.34-4.11 (m, 5H), 4.08-3.70 (m, 5H), 3.42-3.41 (m, 1H), 2.98-2.78 (m, 2H), 2.32 (s, 3H).
**C0150:** MS *m*/*z* (ESI): 439.44 [M+1]⁺, Rt= 2.959 min
¹H NMR (CD₃OD-*d*₄, 400 MHz) δ 7.75 (dd, *J* = 8.4, 5.2 Hz, 1H), 7.21 (t, *J* = 8.6 Hz, 1H), 7.01-6.97 (m, 1H), 6.82 (d, *J* = 7.6 Hz, 1H), 4.66-4.46 (m, 1H), 4.37-4.11 (m, 5H), 4.08-3.70 (m, 5H), 3.42-3.41 (m, 1H), 2.98-2.67 (m, 2H), 2.32 (s, 3H).

### Examples C0151&C0152

### (S,E)-5-Fluoro-3-(8-(2-(hydroxymethyl)-4-(methoxyimino)pyrrolidine-1-carbonyl)-2,3-dihydrobenzo[b][1,4]dioxin-5-yl)-2-methylbenzonitrile

### (S,Z)-5-Fluoro-3-(8-(2-(hydroxymethyl)-4-(methoxyimino)pyrrolidine-1-carbonyl)-2,3-dihydrobenzo[b][1,4]dioxin-5-yl)-2-methylbenzonitrile

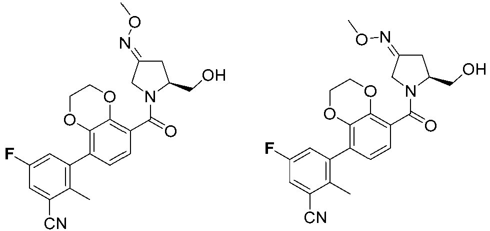

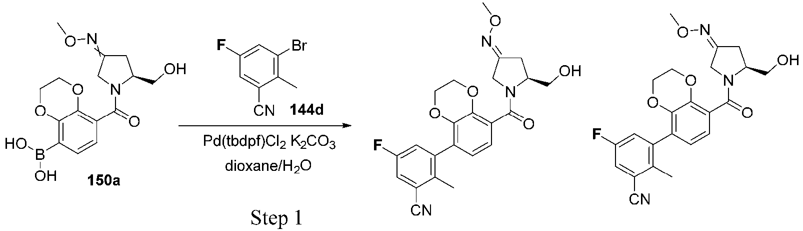

### Step 1

### (S,E)-5-Fluoro-3-(8-(2-(hydroxymethyl)-4-(methoxyimino)pyrrolidine-1-carbonyl)-2,3-dihydrobenzo[b][1,4] dioxin-5-yl)-2-methylbenzonitrile

### (S,Z)-5-Fluoro-3-(8-(2-(hydroxymethyl)-4-(methoxyimino)pyrrolidine-1-carbonyl)-2,3-dihydrobenzo[b][1,4] dioxin-5-yl)-2-methylbenzonitrile

150a (700 mg, 2.00 mmol), 144d (450 mg, 2.10 mmol) and potassium carbonate (830 mg, 6.01 mmol) were dissolved in 1,4-dioxane (15 mL) and water (3 mL), and Pd(dtbpf)Cl₂ (130 mg, 0.20 mmol) was added at room temperature under nitrogen protection. After nitrogen purging, the mixture was reacted at 80°C for 3 h. Water (30 mL) was added to the reaction solution, and the mixture was extracted with ethyl acetate (20 mL × 3). The organic phases were combined, washed with saturated sodium chloride aqueous solution (40 mL × 3), dried over sodium sulphate, and subjected to evaporation to remove the solvent. The residue was purified by silica gel Pre-TLC (PE:EA=1:5) to afford the less polar isomer (Rf=0.6) C0151 (112 mg, yield: 11.69%) and the more polar crude isomer (Rf=0.55). The more polar crude isomer was further purified by silica gel Pre-TLC (PE:EA=1:5) to afford C0152 (104 mg, yield: 11.43%).
**C0151:** MS *m*/*z* (ESI): 440.1 [M+1]⁺, Rt= 2.481 min
¹H NMR (CD₃OD-*d*₄, 400 MHz) δ 7.51 (dd, *J =* 8.0, 2.4 Hz, 1H), 7.31-7.25 (m, 1H), 7.03-6.93 (m, 1H), 6.82 (d, *J* = 8.0 Hz, 1H), 4.71-3.97 (m, 7H), 3.89-3.38 (m, 5H), 2.99-2.76 (m, 2H), 2.31 (s, 3H).
**C0152:** MS *m*/*z* (ESI): 440.0 [M+1]⁺, Rt= 2.370 min
¹H NMR (CD₃OD-*d*₄, 400 MHz) δ 7.51 (dd, *J =* 8.0, 2.8 Hz, 1H), 7.32-7.26 (m, 1H), 6.99-6.95 (m, 1H), 6.82 (d, *J* = 7.6 Hz, 1H), 4.64-4.017 (m, 7H), 3.89-3.38 (m, 5H), 2.93-2.66 (m, 2H), 2.32 (s, 3H).

### Examples C0153&C0154

### (S,E)-2-Chloro-3-(8-(2-(hydroxymethyl)-4-(methoxyimino)pyrrolidine-1-carbonyl)-2,3-dihydrobenzo[b][1,4]dioxin-5-yl)benzonitrile

### (S,Z)-2-Chloro-3-(8-(2-(hydroxymethyl)-4-(methoxyimino)pyrrolidine-1-carbonyl)-2,3-dihydrobenzo[b][1,4]dioxin-5-yl)benzonitrile

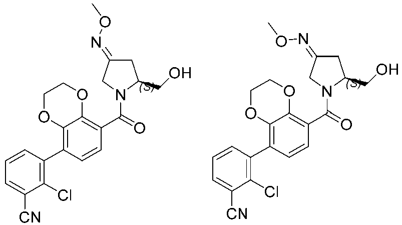

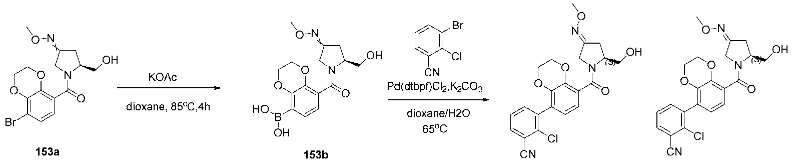

### Step 1

### (S)-(8-(2-(Hydroxymethyl)-4-(methoxyimino)pyrrolidine-1-carbonyl)-2,3-dihydrobenzo[b][1,4]dioxin-5-yl)boronic acid 153b

To a solution of 153a (350 mg, 0.86 mmol) in dioxane (10 mL) were added bis(pinacolato)diboron (436.8 mg, 1.72 mmol), potassium acetate (253.2 mg, 2.58 mmol) and 1,1'-bis(diphenylphosphino)ferrocene palladium dichloride (26.9 mg, 0.086 mmol). After nitrogen purging, the reaction solution was reacted at 85°C for 10 hours. After the reaction was completed, the reaction solution was filtered, and the filter cake was washed with dioxane (4 mL). The organic phases were combined, and the residue was directly used in the next step.

MS *m*/*z* (ESI): 350.8 [M+1]⁺.

### Step 2

### (S,E)-2-Chloro-3-(8-(2-(hydroxymethyl)-4-(methoxyimino)pyrrolidine-1-carbonyl)-2,3-dihydrobenzo[b][1,4]dioxin-5-yl)benzonitrile

### (S,Z)-2-Chloro-3-(8-(2-(hydroxymethyl)-4-(methoxyimino)pyrrolidine-1-carbonyl)-2,3-dihydrobenzo[b][1,4]dioxin-5-yl)benzonitrile

3-Bromo-2-chlorobenzonitrile (130 mg, 0.61 mmol) was dissolved in a solution of 153b in dioxane/water (15 mL/2 mL), and potassium carbonate (250 mg, 1.83 mmol) and [1,1'-bis(di-tert-butylphosphino)ferrocene]palladium dichloride were added to the solution. After nitrogen purging, the reaction solution was reacted at 65°C for 3 hours. After the reaction was completed, water (30 mL) was added to the reaction solution, and the mixture was extracted with ethyl acetate (20 mL × 3). The organic phases were combined, washed with saturated sodium chloride aqueous solution (20 mL × 3), dried over sodium sulphate, and subjected to evaporation to remove the solvent. The residue was purified by silica gel Pre-TLC (PE:EA=1:2) to afford the less polar isomer C0153 (48.80 mg, yield: 13.54%) (Rf=0.6) and the more polar crude isomer C0154 (Rf=0.55). Further purification by HPLC yielded the more polar isomer C0154 (23.93 mg, yield: 8.99%).

C0153: MS *m*/*z* (ESI): 442.0 [M+1]⁺.

¹H NMR (CD₃OD-*d*₄, 400 MHz) *δ* 7.83-7.81 (m, 1H), 7.65-7.61 (m, 1H), 7.58-7.52 (m, 1H), 7.00-6.96(m, 1H), 6.87-6.85 (m, 1H), 4.70-4.00 (m, 7H), 3.88-3.83 (m, 3H), 3.83-3.67 (m, 1H), 2.99-2.76 (m, 3H).

C0154: MS *m*/*z* (ESI): 442.0 [M+1]⁺.

¹H NMR (CD₃OD-*d*₄, 400 MHz) *δ* 7.83-7.81 (m, 1H), 7.67-7.61 (m, 2H), 7.55-7.51 (m, 2H), 7.00-6.95(m, 1H), 6.87-6.85 (m, 1H), 4.68-4.05 (m, 7H), 3.88-3.80 (m, 3H), 3.77-3.70 (m, 1H), 3.47-3.40 (m, 1H), 2.93-2.47 (m, 2H).

### Examples C0155&C0156

### (S,Z)-3'-Fluoro-4'-(2-(hydroxymethyl)-4-(methoxyimino)pyrrolidine-1-carbonyl)-2-methyl-[1,1'-biphenyl]-3-carbonitrile

### (S,E)-3'-Fluoro-4'-(2-(hydroxymethyl)-4-(methoxyimino)pyrrolidine-1-carbonyl)-2-methyl-[1,1'-biphenyl]-3-carbonitrile

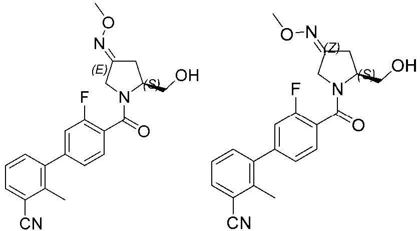

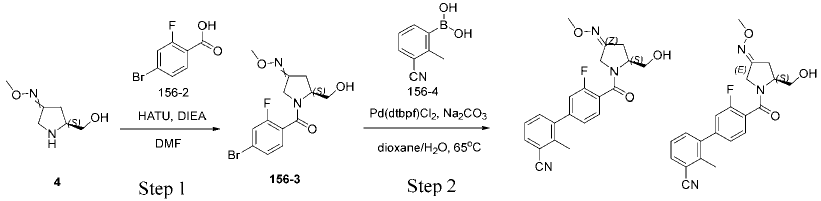

### Step 1

### (S)-(4-Bromo-2-fluorophenyl)(2-(hydroxymethyl)-4-(methoxyimino)pyrrolidin-1-yl)methanone 156-3

156-2 (450 mg, 2.05 mmol), 4 (370 mg, 2.05 mmol) and HATU (1.17 g, 3.07 mmol) were dissolved in DMF (5 mL), diisopropylethylamine (1.06 g, 8.2 mmol) was added, and the mixture was stirred at room temperature for 2 hours. The reaction solution was directly purified by C18 reversed-phase column chromatography (acetonitrile:water = 40: 60) and concentrated to afford 156-3 (364 mg, yield: 51.33%) as a yellow oil.

MS *m*/*z* (ESI): 344.8, 346.8 [M+1]⁺.

### Step 2

### (S,Z)-3'-Fluoro-4'-(2-(hydroxymethyl)-4-(methoxyimino)pyrrolidine-1-carbonyl)-2-methyl-[1,1'-biphenyl]-3-carbonitrile

### (S,E)-3'-Fluoro-4'-(2-(hydroxymethyl)-4-(methoxyimino)pyrrolidine-1-carbonyl)-2-methyl-[1,1'-biphenyl]-3-carbonitrile

156-3 (362 mg, 1.05 mmol), 156-4 (219 mg, 1.37 mmol, purity: 100%), Pd(dtbpf)Cl₂ (68 mg, 0.11 mmol) and sodium carbonate (334 mg, 3.15 mmol) were dissolved in 1,4-dioxane (10 mL) and water (1 mL), and the mixture was purged three times with nitrogen, heated to 65°C under nitrogen protection and stirred for 5 hours. Water (20 mL) was added to the reaction solution, and the mixture was extracted with ethyl acetate (20 mL × 3). The organic phases were combined, washed with saturated sodium chloride aqueous solution (30 mL), dried over sodium sulphate, and subjected to evaporation to remove the solvent. The residue was purified by silica gel Pre-TLC (PE:EA=1:3) to afford the less polar isomer C0155 (80 mg, yield: 19.83%, Rf=0.6) and the more polar isomer C0156 (100 mg, yield: 24.74%, Rf=0.55).
**C0155:** MS *m*/*z* (ESI): 382.1 [M+1]⁺, Rt= 3.113 min
¹H NMR (400 MHz, DMSO-*d*₆) *δ* 7.86 (dd, *J=* 8.0, 1.2 Hz, 1H), 7.62-7.55 (m, 2H), 7.50 (t, *J =* 7.6 Hz, 1H), 7.41 (dd, *J* = 10.0, 3.2 Hz, 1H), 7.30 (dd, *J* = 8.0, 1.6 Hz, 1H), 5.01 (t, *J* = 5.6 Hz, 1H), 4.56-4.41 (m, 1H), 4.16-4.06 (m, 1H), 3.97-3.91 (m, 1H), 3.82-3.77 (m, 3H), 3.64-3.51 (m, 1H), 3.23-3.21 (m, 1H), 2.80-2.75 (m, 2H), 2.43 (s, 3H).
**C0156:** MS *m*/*z* (ESI): 382.1 [M+1]⁺, Rt= 3.072 min
¹H NMR (400 MHz, DMSO-*d*₆) *δ* 7.86 (dd, *J* = 7.6, 0.8 Hz, 1H), 7.64-7.56 (m, 2H), 7.50 (t, *J =* 7.6 Hz, 1H), 7.41 (d, *J =* 10.4 Hz, 1H), 7.33-7.29 (m, 1H), 5.01 (t, *J* = 5.6 Hz, 1H), 4.56-4.36 (m, 1H), 4.17-4.05 (m, 1H), 3.94-3.90 (m, 1H), 3.82-3.72 (m, 3H), 3.61-3.53 (m, 1H), 3.24-3.22 (m, 1H), 2.97-2.87 (m, 1H), 2.69-2.54 (m, 1H), 2.43 (s, 3H).

### Examples C0157&C0158

### (S,E)-2'-Fluoro-4'-(2-(hydroxymethyl)-4-(methoxyimino)pyrrolidine-1-carbonyl)-2-methyl-[1,1'-biphenyl]-3-carbonitrile

### (S,Z)-2'-Fluoro-4'-(2-(hydroxymethyl)-4-(methoxyimino)pyrrolidine-1-carbonyl)-2-methyl-[1,1'-biphenyl]-3-carbonitrile

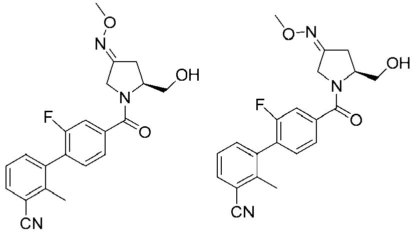

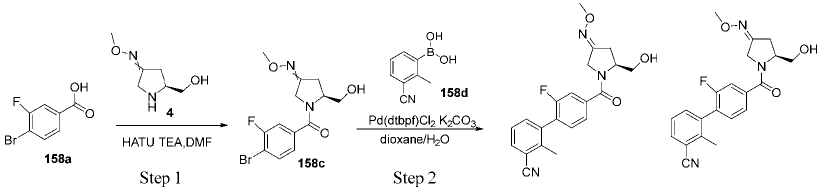

According to the aforementioned synthetic route, with reference to the synthetic method of compound C0155, the less polar isomer (Rf=0.6) C0157 (75.02 mg, yield: 30.17%) and the more polar crude isomer (Rf=0.55) were obtained. The crude product was further purified by Pre-HPLC to afford C0158 (87.47 mg, yield: 35.07%).
**C0157:** MS *m*/*z* (ESI): 382.1 [M+1]⁺, Rt= 3.191 min
¹H NMR (CD₃OD-*d*₄, 400 MHz) δ 7.76 (d, *J* = 7.6 Hz, 1H), 7.55-7.40 (m, 5H), 4.77-4.04 (m, 3H), 3.90-3.38 (m, 5H), 2.94-2.72 (m, 2H), 2.38 (S, 3H).
**C0158:** MS *m*/*z* (ESI): 382.1 [M+1]⁺, Rt= 3.179 min
¹H NMR (CD₃OD-*d*₄, 400 MHz) δ 7.76 (d, *J* = 7.6 Hz, 1H), 7.56-7.40 (m, 5H), 4.78-4.08 (m, 3H), 3.91-3.39 (m, 5H), 3.10-2.85 (m, 1H), 2.80-2.59 (m, 1H), 2.39 (S, 3H).

### Examples C0159&C0160

### 3-(6-Fluoro-8-((S,E)-2-(hydroxymethyl)-4-(methoxyimino)pyrrolidine-1-carbonyl)-2,3-dihydrobenzo[b][1,4] dioxin-5-yl)-2-methylbenzonitrile

### 3-(6-Fluoro-8-((S,Z)-2-(hydroxymethyl)-4-(methoxyimino)pyrrolidine-1-carbonyl)-2,3-dihydrobenzo[b][1,4] dioxin-5-yl)-2-methylbenzonitrile

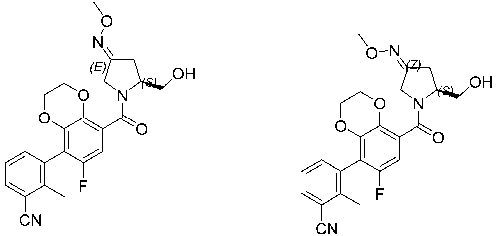

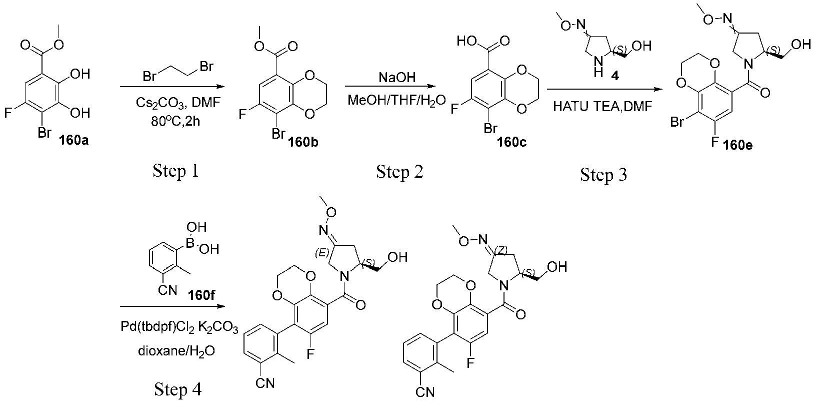

### Step 1

### Methyl 8-bromo-7-fluoro-2,3-dihydrobenzo[b][1,4]dioxine-5-carboxylate 160b

To a solution of 160a (100 mg, 2.64 mmol) in DMF (10 mL) were added caesium carbonate (1.38 g, 4.22 mmol) and 1,2-dibromoethane (0.55 g, 2.90 mmol), and the mixture was stirred at 80°C for 2 h. The reaction solution was extracted with ethyl acetate/water (3×50/30 mL). The organic layers were combined, washed with saturated NaCl solution (4 * 80 mL), combined, dried over anhydrous sodium sulphate, and filtered, and the filtrate was collected and concentrated to afford a crude product. The crude product was purified by C18 reversed-phase column chromatography (ACN:H₂O=25:75 to 80:20) to afford 160b (430 mg, yield: 55.93%).

MS *m*/*z* (ESI): 291.8 [M+1]⁺.

### Step 2

### 8-Bromo-7-fluoro-2,3-dihydrobenzo[b][1,4]dioxine-5-carboxylic acid 160c

To a solution of 160b (430 mg, 1.48 mmol) in THF (3 mL), methanol (3 mL) and water (6 mL) was added sodium hydroxide (296 mg, 7.4 mmol), and the mixture was stirred at room temperature for 16 h. The reaction solution was subjected to rotary evaporation to remove most of the solvent and extracted with a small amount of ethyl acetate. The aqueous phase was adjusted to pH 5-4 with 2M hydrochloric acid and extracted with ethyl acetate (3 * 50 mL). The organic phases were combined, dried over anhydrous sodium sulphate, and filtered, and the filtrate was collected and concentrated to afford product 160c (330 mg, yield: 80.63%).

MS *m*/*z* (ESI): 276.8 [M-1]⁺.

### Step 3

### (S)-(8-Bromo-7-fluoro-2,3-dihydrobenzo[b][1,4]dioxin-5-yl)(2-(hydroxymethyl)-4-(methoxyimino)pyrrolidin-1-yl)methanone 160e

To a solution of 160c (330 mg, 1.19 mmol) in DMF (6 mL) were added 4 (250 mg, 1.73 mmol), HATU (500 mg, 1.31 mmol) and triethylamine (500 mg, 4.94 mmol), and the mixture was reacted at room temperature overnight. The reaction solution was purified by C18 reversed-phase column chromatography (ACN:H₂O=25:75 to 80:20) to afford 160e (200 mg, yield: 41.64%).

MS *m*/*z* (ESI): 403.0 [M+1]⁺.

### Step 4

### 3-(6-Fluoro-8-((S,E)-2-(hydroxymethyl)-4-(methoxyimino)pyrrolidine-1-carbonyl)-2,3-dihydrobenzo[b][1,4]dioxin-5-yl)-2-methylbenzonitrile

### 3-(6-Fluoro-8-((S,Z)-2-(hydroxymethyl)-4-(methoxyimino)pyrrolidine-1-carbonyl)-2,3-dihydrobenzo[b][1,4]dioxin-5-yl)-2-methylbenzonitrile

To a solution of 160e (200 mg, 0.50 mmol) in 1,4-dioxane (5 mL) and water (1 mL) were added 160f (100 mg, 0.62 mmol), Pd(dtbpf)Cl₂ (30 mg, 0.051 mmol) and potassium carbonate (200 mg, 1.45 mmol). After nitrogen purging, the mixture was reacted at 80°C for 3 h. Water (30 mL) was added to the reaction solution, and the mixture was extracted with ethyl acetate (20 mL × 3). The organic phases were combined, washed with saturated sodium chloride aqueous solution (40 mL × 3), dried over sodium sulphate, and subjected to evaporation to remove the solvent. The residue was purified by silica gel Pre-TLC (PE:EA=1:5) to afford the less polar isomer (Rf=0.6) C0159 (40 mg, yield: 13.86%) and the more polar crude isomer (Rf=0.55) C0160 (42.05 mg, yield: 16.63%).
**C0159:** MS *m*/*z* (ESI): 440.0 [M+1]⁺, Rt= 3.150 min
¹H NMR (CD₃OD-*d*₄, 400 MHz) *δ* 7.88 (d, *J =* 7.6 Hz, 1H), 7.60-7.48 (m, 2H), 6.92-6.87 (m, 1H), 4.99-4.96 (m, 1H), 4.53-4.25 (m, 5H), 4.23-3.85 (m, 2H), 3.83-3.56 (m, 4H), 3.28-2.68 (m, 3H), 2.31 (s, 3H).
**C0160:** MS *m*/*z* (ESI): 440.0 [M+1]⁺, Rt= 3.179 min
¹H NMR (CD₃OD-*d*₄, 400 MHz) *δ* 7.72 (d, *J =* 7.6 Hz, 1H), 7.50-7.40 (m, 2H), 6.86-6.81 (m, 1H), 4.60-4.45 (m, 1H), 4.37-4.03 (m, 6H), 3.89-3.42 (m, 5H), 3.01-2.90 (m, 1H), 2.82-2.63 (m, 1H), 2.34 (s, 3H).

### Examples C0161&C0162

### 3-(5-Fluoro-7-((S,E)-2-(hydroxymethyl)-4-(methoxyimino)pyrrolidine-1-carbonyl)benzo[d][1,3]dioxol-4-yl)-2-methylbenzonitrile

### 3-(5-Fluoro-7-((S,Z)-2-(hydroxymethyl)-4-(methoxyimino)pyrrolidine-1-carbonyl)benzo[d][1,3]dioxol-4-yl)-2-methylbenzonitrile

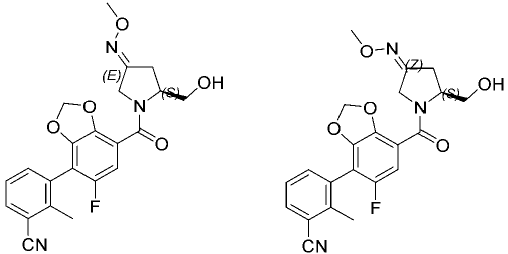

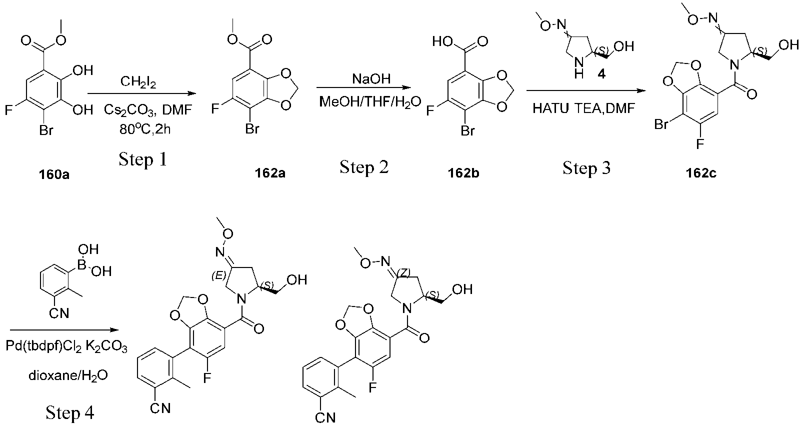

According to the aforementioned synthetic route, with reference to the synthetic method of compound C0159, the less polar isomer (Rf=0.6) (C0161) (20 mg, yield: 4.18%) and the more polar crude isomer (Rf=0.55) C0162 (32.06 mg, yield: 14.60%) were obtained.
**C0161:** MS *m*/*z* (ESI): 426.0 [M+1]⁺, Rt= 3.310 min
¹H NMR (CD₃OD-*d*₄, 400 MHz) *δ* 7.76 (dd, *J* = 8.0, 1.2 Hz, 1H), 7.62-7.59 (m, 1H), 7.45 (t, *J =* 7.6 Hz, 1H), 6.88-6.84 (m, 1H), 6.15-6.10 (m, 2H), 4.69-3.41 (m, 8H), 3.07-2.75 (m, 2H), 2.43 (s, 3H).
**C0162:** MS *m*/*z* (ESI): 426.0 [M+1]⁺, Rt= 3.106 min
¹H NMR (CD₃OD-*d*₄, 400 MHz) *δ* 7.77 (d, *J =* 7.6 Hz, 1H), 7.62-7.58 (m, 1H), 7.45 (t, *J* = 7.6 Hz, 1H), 6.86 (d, *J =* 10.4 Hz, 1H), 6.15-6.08 (m, 2H), 4.68-4.19 (m, 3H), 3.88-3.41 (m, 5H), 3.03-2.90 (m, 1H), 2.79-2.63 (m, 1H), 2.43 (s, 3H).

### Examples C0163&C0164

### (S,E)-4'-(2-(2-Hydroxypropan-2-yl)-4-(methoxyimino)pyrrolidine-1-carbonyl)-2-methyl-[1,1'-biphenyl]-3-carbonitrile

### (S,Z)-4'-(2-(2-Hydroxypropan-2-yl)-4-(methoxyimino)pyrrolidine-1-carbonyl)-2-methyl-[1,1'-biphenyl]-3-carbonitrile

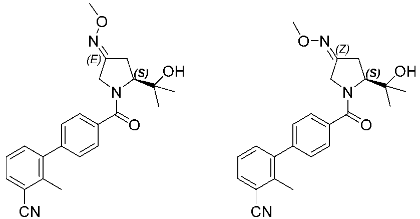

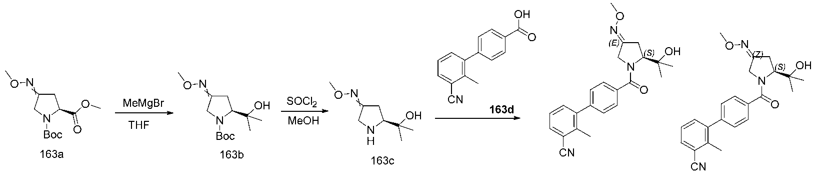

### Step 1

Tert-butyl (*S*)-2-(2-hydroxypropan-2-yl)-4-(methoxyimino)pyrrolidine-1-carboxylate 163b

To a solution of 163a (1.0 g, 3.49 mmol) in tetrahydrofuran (10 mL) was added dropwise a solution of methylmagnesium bromide in tetrahydrofuran (3.5 mL, 10.47 mmol) in an ice bath. After the dropwise addition was completed, the reaction solution was stirred at room temperature for 2 hours. After the reaction was completed, water (20 mL) was added to the reaction solution, and the mixture was extracted with ethyl acetate (20 mL × 3). The organic phases were combined and washed with saturated sodium chloride aqueous solution (10 mL × 3) to afford product 163b (700 mg, yield: 69.99%).

MS *m*/*z* (ESI): 181.0 [M+1-56]⁺

### Step 2

### (S)-5-(2-Hydroxypropan-2-yl)pyrrolidin-3-oneO-methyloxime 163c

Thionyl chloride (1.4 mL) was added dropwise to methanol (9.5 mL), and the reaction solution was stirred at room temperature for 15 minutes. Then, the aforementioned solution was added to 163b (350 mg, 1.22 mmol), and the reaction solution was reacted at room temperature for 30 minutes. After the reaction was completed, the reaction solution was concentrated to remove methanol, to afford crude product 163c (300 mg), which was directly used in the next step.

MS *m*/*z* (ESI): 173.0 [M+1]⁺

### Step 3

### (S,E)-4'-(2-(2-Hydroxypropan-2-yl)-4-(methoxyimino)pyrrolidine-1-carbonyl)-2-methyl-[1,1'-biphenyl]-3-carbonitrile

### (S,Z)-4'-(2-(2-Hydroxypropan-2-yl)-4-(methoxyimino)pyrrolidine-1-carbonyl)-2-methyl-[1,1'-biphenyl]-3-carbonitrile

To a solution of 163c (300 mg) in DMF (10 mL) were added 163d (290 mg, 1.22 mmol), HATU (600 mg, 1.3 mmol) and triethylamine (370 mg, 3.66 mmol), and the reaction solution was reacted at room temperature for 2 hours. Water (10 mL) was added to the reaction solution, and the mixture was extracted with ethyl acetate (20 mL × 3). The organic phases were combined, washed with saturated sodium chloride aqueous solution (10 mL × 3), dried over sodium sulphate, and subjected to evaporation to remove the solvent. The residue was purified by silica gel Pre-TLC (PE:EA=1:2) to afford the less polar isomer C0163 (Rf=0.6) and the more polar crude isomer C0164 (Rf=0.55). Further purification by HPLC yielded the more polar product C0164 (26.0 mg, yield: 18.6%).

C0163: MS *m*/*z* (ESI): 392.1 [M+1]⁺.

¹H NMR (CD₃OD-*d*₄, 400 MHz) *δ* 7.72-7.68 (m, 3H), 7.55-7.53 (m, 1H), 7.46-7.42 (m, 3H), 4.81-4.79 (m, 1H), 4.51-4.49 (m, 1H), 4.05-4.03 (m, 1H), 3.81-3.76 (m, 3H),3.06-3.01 (m, 1H),2.89-2.61(m, 1H), 2.45 (s, 3H), 1.31-1.27 (m, 6H).

C0164: MS *m*/*z* (ESI): 392.1 [M+1]⁺.

¹H NMR (CD3OD, 400 MHz) *δ* 7.72-7.68 (m, 3H), 7.56-7.54 (m, 1H), 7.46-7.42 (m, 3H), 4.79-4.77 (m, 1H), 4.51-4.47 (m, 1H), 4.05-4.01 (m, 1H), 3.76 (s, 3H), 2.89-2.85 (m, 1H),2.79-2.72(m, 1H), 2.45 (s, 3H), 1.30-1.29 (m, 6H).

### Examples C0165&C0166

### (S,Z)-3-(6-Fluoro-7-(2-(hydroxymethyl)-4-(methoxyimino)pyrrolidine-1-carbonyl)benzo[d][1,3]dioxol-4-yl)-2-methylbenzonitrile

### (S,E)-3-(6-Fluoro-7-(2-(hydroxymethyl)-4-(methoxyimino)pyrrolidine-1-carbonyl)benzo[d][1,3]dioxol-4-yl)-2-methylbenzonitrile

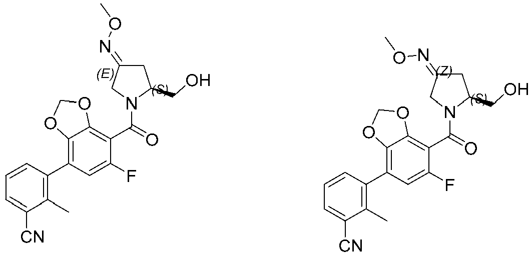

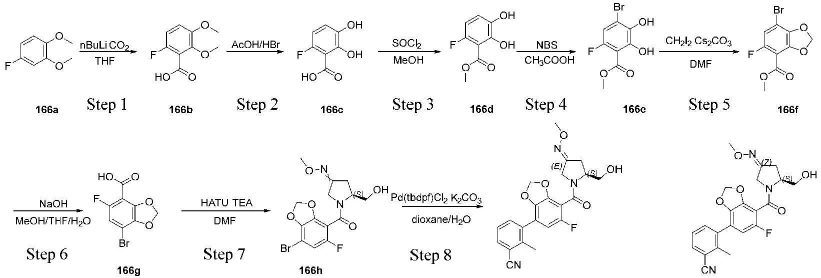

### Step 1

### 6-Fluoro-2,3-dimethoxybenzoic acid 166b

To a solution of 166a (10 g, 64.04 mmol) in THF (150 mL) was added dropwise n-butyllithium (4.5 g, 70.44 mmol) at -78°C, and the reaction solution was stirred for 1 h and then poured into dry ice tetrahydrofuran solution. After the reaction was completed, water was slowly added to the reaction solution to quench the reaction solution, and the reaction solution was adjusted to pH 2-3 with 1M HCl aqueous solution and extracted with ethyl acetate. The organic layers were combined, washed with saturated NaCl solution, combined, dried over anhydrous sodium sulphate, filtered, and subjected to rotary evaporation to afford a crude product. The crude product was slurried with PE/EA to afford product 166b (8 g, yield: 62.41%).

MS *m*/*z* (ESI): 199.1 [M-1]⁺.

### Step 2

### 6-Fluoro-2,3-dihydroxybenzoic acid 166c

166b (8 g, 39.97 mmol) was dissolved in hydrobromic acid acetic acid solution, and the mixture was reacted at 130°C for 2 h. After the reaction was completed, the reaction solution was subjected to rotary evaporation to afford product 166c (6.5 g, yield: 94.49%).

MS *m*/*z* (ESI): 171.1 [M-1]⁺.

### Step 3

### Methyl 6-fluoro-2,3-dihydroxybenzoate 166d

To a solution of 166c (6.5 g, 37.77 mmol) in methanol (100 mL) was added dropwise thionyl chloride (6.74 g, 56.66 mmol) at 0°C. After the dropwise addition was completed, the reaction solution was heated to 75°C and reacted for 24 h. The reaction solution was directly subjected to rotary evaporation to dryness and extracted with ethyl acetate/water (3 * 50/30 mL). The organic layers were combined, washed with saturated NaCl solution (3 * 20 mL), combined, dried over anhydrous sodium sulphate, filtered, and subjected to rotary evaporation to afford a crude product, which was purified by reversed-phase flash column chromatography (ACN:H₂O=5:95 to 55:45) to afford product 166d (5.4 g, yield: 76.82%).

MS *m*/*z* (ESI): 185.1 [M-1]⁺.

### Step 4

### Methyl 4-bromo-6-fluoro-2,3-dihydroxybenzoate 166e

To a solution of 166d (5.4 g, 29.01 mmol) in acetic acid (60 mL) was added NBS (5.16 g, 29.01 mmol), and the mixture was reacted at 70°C overnight. The reaction solution was subjected to rotary evaporation to dryness to afford a crude product, which was purified by reversed-phase flash column chromatography (ACN:H₂O=5:95 to 45:55) to afford product 166e (3 g, yield: 39.02%).

MS *m*/*z* (ESI): 264.8, 266.8 [M+1]⁺.

According to Step 5 to Step 8 in the aforementioned synthetic route, with reference to the synthetic method of compound C0159, the less polar isomer (Rf=0.6) (C0165) (90 mg, yield: 19.85%) and the more polar crude isomer (Rf=0.55) were obtained. The more polar crude isomer was subjected to chiral resolution and lyophilization to afford the more polar product C0166 (54 mg, yield: 13.32%).
**C0165:** MS *m*/*z* (ESI): 426.1 [M+1]⁺, Rt= 3.186 min
¹H NMR (CD₃OD-*d*₄, 400 MHz) δ 7.75-7.71 (m, 1H), 7.59-7.51 (m, 1H), 7.46-7.42 (m, 1H), 6.85-6.67 (m, 1H), 6.17-6.10 (m, 2H), 4.69-4.14 (m, 3H), 3.88-3.70 (m, 4H), 3.44-3.42 (m, 1H), 2.95-2.47 (m, 2H), 2.46-2.37 (m, 3H).
**C0166:** MS *m*/*z* (ESI): 426.1 [M+1]⁺, Rt= 3.123 min
¹H NMR (CD₃OD-*d*₄, 400 MHz) δ 7.74 (d, *J* = 7.6 Hz, 1H), 7.58 (t, *J* = 8.0 Hz, 1H), 7.44 (t, *J* = 8.0 Hz, 1H), 6.69 (dd, *J* = 10.8, 3.2 Hz, 1H), 6.14-6.10 (m, 2H), 4.67-4.14 (m, 3H), 3.88-3.73 (m, 4H), 3.44-3.43 (m, 1H), 2.99-2.93 (m, 1H), 2.82-2.65 (m, 1H), 2.48 (d, *J* = 2.0 Hz, 3H).

### Examples C0167&C0168

### 3-(4-[(2S,4E)-2-(Hydroxymethyl)-4-(methoxyimino)pyrrolidine-1-carbonyl]-2-(methyl-d₃oxy)phenyl)-2-methylbenzonitrile

### 3-(4-[(2S,4Z)-2-(Hydroxymethyl)-4-(methoxyimino)pyrrolidine-1-carbonyl]-2-(methyl-d₃oxy)phenyl)-2-methylbenzonitrile

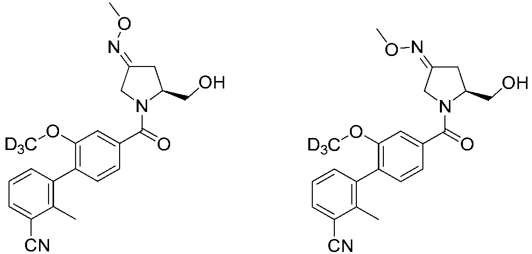

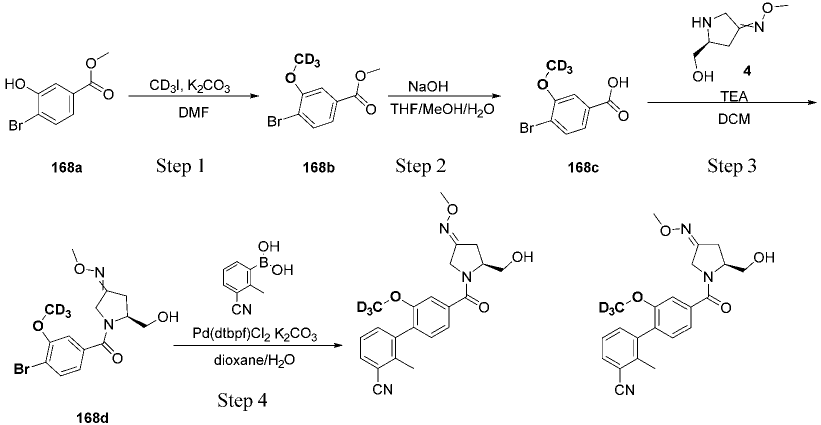

### Step 1

### Methyl 4-bromo-3-(methyl-d₃oxy)benzoate 168b

To a solution of 4-bromo-3-hydroxybenzoate 168a (3 g, 12.98 mmol) in DMF (30 mL) were added potassium carbonate (3.59 g, 25.96 mmol) and deuterated iodomethane (2.82 g, 19.47 mmol), and the reaction solution was reacted at room temperature for 8 h. After the reaction was completed, the reaction solution was extracted with ethyl acetate/water (3×10/10 mL). The organic layers were combined, washed with saturated NaCl solution (3×10 mL), combined, dried over anhydrous sodium sulphate, filtered, and subjected to rotary evaporation to afford a crude product. The crude product was purified by silica gel column chromatography (PE:EA=5:1) to afford product 168b (3 g, yield: 93.13%).

MS *m*/*z* (ESI): 247.9, 249.9 [M+1]⁺.

According to Step 2 to Step 4 in the aforementioned synthetic route, with reference to the synthetic method of compound C0159, the less polar isomer (Rf=0.65) C0167 (180 mg, yield: 22.15%) and the more polar crude isomer (Rf=0.55) were obtained. The more polar isomer was purified by preparative HPLC and lyophilized to afford the more polar product C0168 (130 mg, yield: 23.57%).
**C0167:** MS *m*/*z* (ESI): 397.1 [M+1]⁺, Rt= 3.056 min
¹H NMR (CD₃OD-*d*₄, 400 MHz) δ 7.67 (d, *J* = 6.4 Hz, 1H), 7.42-7.37 (m, 2H), 7.27-7.22 (m, 3H), 4.76-4.12 (m, 3H), 3.84-3.40 (m, 5H), 2.88-2.78 (m, 2H), 2.29 (s, 3H).
**C0168:** MS *m*/*z* (ESI): 397.1 [M+1]⁺, Rt= 3.071 min
¹H NMR (CD₃OD-*d*₄, 400 MHz) δ 7.67 (d, *J* = 6.4 Hz, 1H), 7.43-7.37 (m, 2H), 7.26-7.22 (m, 3H), 4.75-4.10 (m, 3H), 3.88-3.70 (m, 4H), 3.42-3.30 (m, 1H), 2.97-2.73 (m, 2H), 2.30 (s, 3H).

### Examples C0169&C0170

### (S, E)-4'-(2-(Hydroxymethyl)-4-(methoxyimino)pyrrolidine-1-carbonyl)-2-methyl-2'-(trifluoromethoxy)-[1,1'-biphenyl]-3-carbonitrile

### (S, Z)-4'-(2-(Hydroxymethyl)-4-(methoxyimino)pyrrolidine-1-carbonyl)-2-methyl-2'-(trifluoromethoxy)-[1,1'-biphenyl]-3-carbonitrile

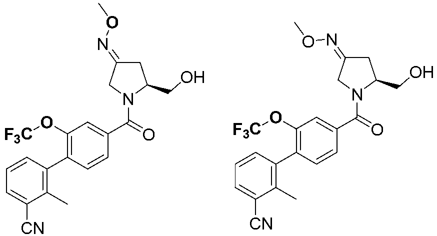

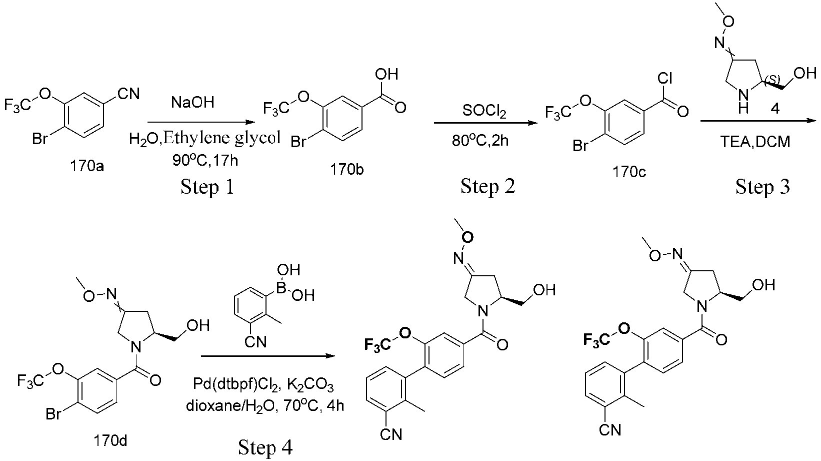

### Step 1

### 4-Bromo-3-(trifluoromethoxy)benzoic acid 170b

170a (600 mg, 2.26 mmol) was dissolved in ethylene glycol (10 mL), a solution of sodium hydroxide (366 mg, 9.15 mmol) in water (5 mL) was added at room temperature, and the reaction solution was stirred at 90°C for 16 h. The reaction solution was concentrated, diluted with water (25 mL), and extracted with ethyl acetate (30 mL). The aqueous phase was adjusted to pH 4-5 with dilute hydrochloric acid (2 mol/L), extracted with ethyl acetate (50 mL * 3), dried over anhydrous sodium sulphate, and filtered, and the filtrate was collected and concentrated to afford 170b (400 mg, yield: 62.22%).

MS *m*/*z* (ESI): 284.8 [M+1]⁺.

### Step 2

### 4-Bromo-3-(trifluoromethoxy)benzoyl chloride 170c

170b (400 mg, 1.40 mmol) was dissolved in thionyl chloride (10 mL), and the mixture was reacted at 80°C for 2 h. After the reaction was completed, the reaction solution was concentrated, dichloromethane was added to the residue, and the mixture was concentrated again to afford 170c (400 mg, yield: 93.92%). The crude product was directly used in the next reaction. The detected product was the methyl ester due to methanol used for sample delivery.

MS *m*/*z* (ESI): 300.0 [M+1]⁺.

### Step 3

### (S,EZ)-(4-Bromo-3-(trifluoromethoxy)phenyl)(2-(hydroxymethyl)-4-(methoxyimino)pyrrolidin-1-yl)methanone 170d

4 (300 mg, 2.08 mmol) and triethylamine (670 mg, 6.62 mmol) were dissolved in dichloromethane (12 mL), 170c (400 mg, 1.32 mmol) was added at room temperature under nitrogen protection, and the mixture was reacted at room temperature for 2 h. The reaction solution was concentrated, and the crude product was purified by normal-phase silica gel column chromatography (PE:EA=3:1 to 1:1) to afford 170d (500.0 mg, yield: 84.87%).

MS *m*/*z* (ESI): 410.8 [M+1]⁺.

### Step 4

### (S, E) -4'-(2-(Hydroxymethyl)-4-(methoxyimino)pyrrolidine-1-carbonyl)-2-methyl-2'-(trifluoromethoxy)-[1,1'-biphenyl]-3-carbonitrile

### (S, Z)-4'-(2-(Hydroxymethyl)-4-(methoxyimino)pyrrolidine-1-carbonyl)-2-methyl-2'-(trifluoromethoxy)-[1,1'-biphenyl]-3-carbonitrile

170d (220 mg, 0.54 mmol), 3-cyano-2-methylphenylboronic acid (100 mg, 1.15 mmol) and potassium carbonate (220 mg, 1.59 mmol) were dissolved in 1,4-dioxane (6 mL) and water (1 mL), Pd(dtbpf)Cl₂ (38 mg, 0.011 mmol) was added at room temperature under nitrogen protection, and the mixture was stirred at 70°C for 4 h. Water (50 mL) was added to the reaction solution, and the mixture was extracted with ethyl acetate (40 mL × 3). The organic phases were combined, washed with saturated sodium chloride aqueous solution (70 mL×3), dried over sodium sulphate, and filtered, and the filtrate was collected and subjected to evaporation to remove the solvent. The residue was purified by silica gel Pre-TLC (PE:EA=1:5) to afford the less polar crude isomer C0169 (Rf=0.6) and the more polar crude isomer C0170 (Rf=0.55). Further purification by preparative HPLC and lyophilization yielded the less polar product C0169 (43.0 mg, yield: 17.92%) and the more polar product C0170 (45.0 mg, yield: 18.78%).

C0169: MS *m*/*z* (ESI): 448.0 [M+1]⁺, Rt= 3.420 min.

¹H NMR (400 MHz, CD₃OD-*d*₄) *δ* 7.77 (d, *J* = 6.4 Hz, 1H), 7.68-7.66 (m, 2H), 7.52-7.47 (m, 3H), 4.77-4.07 (m, 3H), 3.98-3.37 (m, 5H), 2.88 (brs, 2H), 2.33 (s, 3H).

C0170: MS *m*/*z* (ESI): 448.0 [M+1]⁺, Rt= 3.410 min.

¹H NMR (400 MHz, CD₃OD-*d*₄) *δ* 7.77 (d, *J* = 7.2 Hz, 1H), 7.68-7.66 (m, 2H), 7.53-7.45 (m, 3H), 4.76-4.07 (m, 3H), 3.94-3.35 (m, 5H), 3.07-2.91 (m, 1H), 2.77-2.58 (m, 1H), 2.33 (s, 3H).

### Examples C0171&C0172

### (S,E)-4'-(2-(1-Hydroxypropyl)-4-(methoxyimino)pyrrolidine-1-carbonyl)-2-methyl-[1,1'-biphenyl]-3-carbonitrile

### (S,Z)-4'-(2-(1-Hydroxypropyl)-4-(methoxyimino)pyrrolidine-1-carbonyl)-2-methyl-[1,1'-biphenyl]-3-carbonitrile

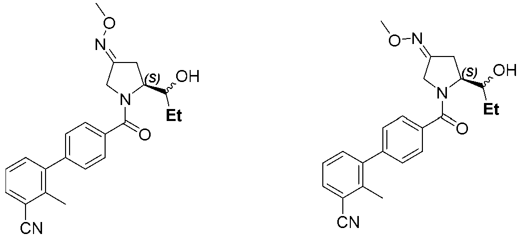

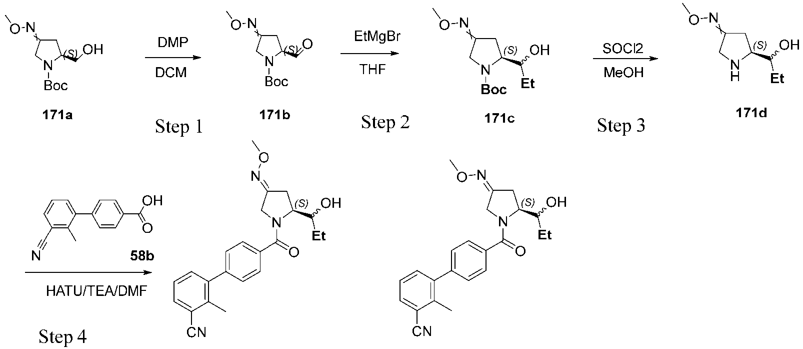

### Step 1

### Tert-butyl (S,EZ)-2-formyl-4-(methoxyimino)pyrrolidine-1-carboxylate 171b

To a solution of 171a (2.0 g, 7.78 mmol) in dichloromethane (30 mL) was added DMP (3.96 g, 9.34 mmol). After nitrogen purging, the mixture was reacted at room temperature for 16 hours. After the reaction was completed, the reaction solution was filtered, and the filter cake was washed with dichloromethane (200 mL). The filtrate was washed with saturated sodium bicarbonate (50 mL) and saturated sodium chloride aqueous solution (30 mL × 2), respectively, dried over sodium sulphate, and subjected to evaporation to remove the solvent. The residue was purified by silica gel column chromatography (eluting with a gradient of petroleum ether:ethyl acetate = 5:1 to 2:1) to afford product 171b (1.0 g, yield: 50.4%).

MS *m*/*z* (ESI): 187.0 [M+1-56]⁺.

### Step 2

### Tert-butyl (S,EZ)-2-(1-hydroxypropyl)-4-(methoxyimino)pyrrolidine-1-carboxylate 171c

To a solution of 171b (1.0 g, 3.92 mmol) in tetrahydrofuran (40 mL) was added dropwise a solution of ethylmagnesium bromide in tetrahydrofuran (12.5 mL, 12.54 mmol) at -78°C, and the reaction solution was reacted at -78°C for 2 hours and then stirred at room temperature for 6 hours. After the reaction was completed, water (30 mL) was added to the reaction solution, and the mixture was extracted with ethyl acetate (30 mL × 3). The organic phases were combined and washed with saturated sodium chloride aqueous solution (10 mL × 2). The residue was purified by silica gel column chromatography (eluting with a gradient of petroleum ether:ethyl acetate = 10:1 to 3:1) to afford product 171c (500 mg, yield: 44.48%).

MS *m*/*z* (ESI): 217.0 [M+1-56]⁺.

### Step 3

### (S,EZ)-5-(1-Hydroxypropyl)pyrrolidin-3-oneO-methyloxime 171d

Thionyl chloride (1.5 mL) was added dropwise to methanol (10 mL), and the reaction solution was stirred at room temperature for 15 minutes. Then, the aforementioned solution was added to 171c (500 mg, 1.74 mmol), and the reaction solution was reacted at room temperature for 1 hour. After the reaction was completed, the reaction solution was concentrated to remove methanol, to afford crude product 171d (500 mg), which was directly used in the next step.

MS *m*/*z* (ESI): 173.0 [M+1]⁺.

### Step 4

### (S,E)-4'-(2-(1-Hydroxypropyl)-4-(methoxyimino)pyrrolidine-1-carbonyl)-2-methyl-[1,1'-biphenyl]-3-carbonitrile

### (S,Z)-4'-(2-(1-Hydroxypropyl)-4-(methoxyimino)pyrrolidine-1-carbonyl)-2-methyl-[1,1'-biphenyl]-3-carbonitrile

To a solution of 171d (500 mg) in DMF (10 mL) were added 58b (412.8 mg, 1.74 mmol), HATU (860.1 mg, 2.26 mmol) and triethylamine (528 mg, 5.22 mmol), and the reaction solution was reacted at room temperature for 2 hours. Water (10 mL) was added to the reaction solution, and the mixture was extracted with ethyl acetate (20 mL × 3). The organic phases were combined, washed with saturated sodium chloride aqueous solution (10 mL × 3), dried over sodium sulphate, and subjected to evaporation to remove the solvent. The residue was purified by silica gel Pre-TLC (PE:EA=1:2) to afford the less polar crude isomer C0171 (Rf=0.6) and the more polar crude isomer C0172 (Rf=0.55). Further purification by preparative HPLC and lyophilization yielded the less polar product C0171 (43.10 mg, yield: 12.7%) and the more polar product C0172 (37.01 mg, yield: 5.4%).

C0171: MS *m*/*z* (ESI): 392.1 [M+1]⁺.

¹H NMR (DMSO-*d*₆, 400 MHz) *δ* 7.72-7.67 (m, 3H), 7.54-7.52 (m, 1H), 7.45-7.42 (m, 3H), 4.71-3.47 (m, 7H), 3.12-2.60 (m, 2H), 2.44 (s, 3H),1.67-1.06(m, 2H), 1.04-0.58 (m, 3H).

C0172: MS *m*/*z* (ESI): 392.1 [M+1]⁺.

¹H NMR (DMSO-*d*₆, 400 MHz) *δ* 7.72-7.67 (m, 3H), 7.55-7.53 (m, 1H), 7.45-7.41 (m, 3H), 4.68-3.64 (m, 7H), 2.91-2.65 (m, 2H), 2.45 (s, 3H),1.67-1.24(m, 2H), 1.06-0.57 (m, 3H).

### Examples C0173&C0174

### (S,E)-(2-(Hydroxymethyl)-4-(methoxyimino)pyrrolidin-1-yl)(7-(2-methyl-3-(trifluoromethyl)phenyl)benzo[d][1,3]dioxol-4-yl)methanone

### (S,Z)-(2-(Hydroxymethyl)-4-(methoxyimino)pyrrolidin-1-yl)(7-(2-methyl-3-(trifluoromethyl)phenyl)benzo[d][1,3]dioxol-4-yl)methanone

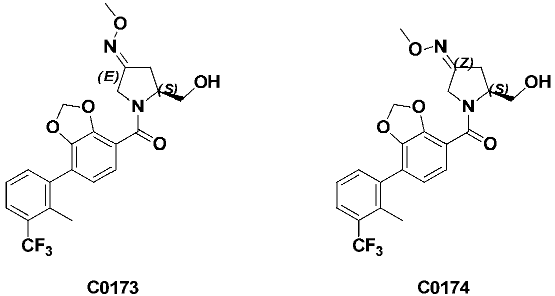

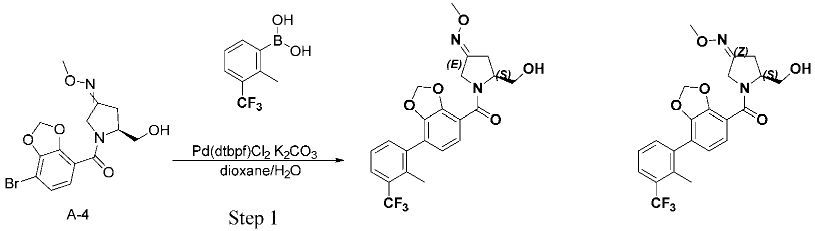

### Step 1

### (S,E)-(2-(Hydroxymethyl)-4-(methoxyimino)pyrrolidin-1-yl)(7-(2-methyl-3-(trifluoromethyl)phenyl)benzo[d][1,3]dioxol-4-yl)methanone

### (S,Z)-(2-(Hydroxymethyl)-4-(methoxyimino)pyrrolidin-1-yl)(7-(2-methyl-3-(trifluoromethyl)phenyl)benzo[d][1,3]dioxol-4-yl)methanone

A-4 (200 mg, 0.54 mmol), 3-trifluoromethyl-2-methylphenylboronic acid (132.16 mg, 0.65 mmol), Pd(dtbpf)Cl₂ (35.19 mg, 0.061 mmol) and potassium carbonate (223.90 mg, 1.62 mmol) were dissolved in a mixed solution of 1,4-dioxane (20 mL) and water (2 mL). After nitrogen purging, the mixture was heated to 65°C and reacted for 2 hours. After the reaction was completed, water (20 mL) was added to the reaction solution, and the mixture was extracted with ethyl acetate (30 mL × 3). The organic phases were combined, washed with saturated sodium chloride aqueous solution (20 mL × 3), dried over sodium sulphate, and subjected to evaporation to remove the solvent. The residue was purified by silica gel Pre-TLC (PE:EA=1:5) to afford the less polar isomer C0173 (42 mg, yield: 14.71%) (Rf=0.6) and the more polar crude isomer. The more polar crude isomer was purified by preparative HPLC and lyophilized to afford the more polar product C0174 (42 mg, yield: 17.23%).
**C0173:** MS *m*/*z* (ESI): 451.1 [M+1]⁺, Rt= 3.732 min
¹H NMR (CD₃OD-*d*₄, 400 MHz) δ 7.70 (d, *J =* 7.6 Hz, 1H), 7.50 (d, *J* = 8.4 Hz, 1H), 7.42 (t, *J =* 8.0 Hz, 1H), 7.06 (d, *J =* 8.0 Hz, 2H), 6.87 (dd, *J =* 8.0, 4.4 Hz, 1H), 6.15-6.05 (m, 2H), 4.72-4.14 (m, 3H), 3.99-3.68 (m, 4H), 3.47-3.31(m, 1H), 2.95-2.81 (m, 2H), 2.35 (s, 3H).
**C0174:** MS *m*/*z* (ESI): 451.1 [M+1]⁺, Rt= 3.671 min
¹H NMR (CD₃OD-*d*₄, 400 MHz) δ 7.70 (d, *J =* 7.6 Hz, 1H), 7.50 (d, *J =* 8.0 Hz, 1H), 7.42 (t, *J =* 8.0 Hz, 1H), 7.05 (dd, *J =* 8.4, 3.2 Hz, 1H), 6.87 (dd, *J* = 8.0, 4.4 Hz, 1H), 6.08 (dd, *J =* 19.2, 5.2 Hz, 1H), 4.70-4.16 (m, 3H), 3.88-3.69 (m, 4H), 3.41-3.40 (m, 1H), 2.96-2.66 (m, 2H), 2.35 (s, 3H).

### Examples C0175&C0176

### (S,E)-(7-(3-Chloro-2-methylphenyl)benzo[d][1,3]dioxol-4-yl)(2-(hydroxymethyl)-4-(methoxyimino)pyrrolidin-1-yl)methanone

### (S,Z)-(7-(3-Chloro-2-methylphenyl)benzo[d][1,3]dioxol-4-yl)(2-(hydroxymethyl)-4-(methoxyimino)pyrrolidin-1-yl)methanone

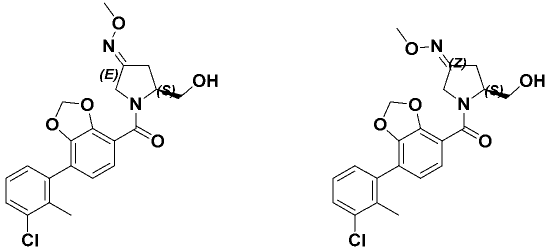

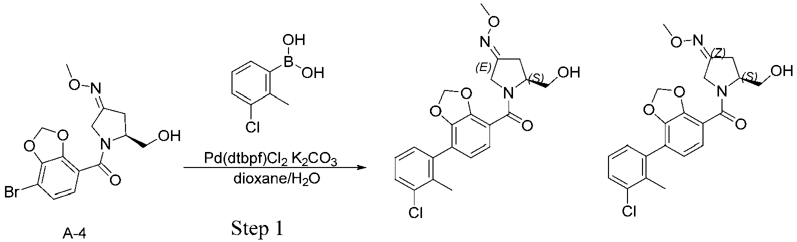

### Step 1

### (S,E)-(7-(3-Chloro-2-methylphenyl)benzo[d][1,3]dioxol-4-yl)(2-(hydroxymethyl)-4-(methoxyimino)pyrrolidin-1-yl)methanone

### (S,Z)-(7-(3-Chloro-2-methylphenyl)benzo[d][1,3]dioxol-4-yl)(2-(hydroxymethyl)-4-(methoxyimino)pyrrolidin-1-yl)methanone

A-4 (200 mg, 0.54 mmol), 3-chloro-2-methylphenylboronic acid (110.42 mg, 0.65 mmol), Pd(dtbpf)Cl₂ (35.19 mg, 0.061 mmol) and potassium carbonate (223.90 mg, 1.62 mmol) were dissolved in a mixed solution of 1,4-dioxane (20 mL) and water (2 mL). After nitrogen purging, the mixture was heated to 65°C and reacted for 2 hours. After the reaction was completed, water (20 mL) was added to the reaction solution, and the mixture was extracted with ethyl acetate (30 mL × 3). The organic phases were combined, washed with saturated sodium chloride aqueous solution (20 mL × 3), dried over sodium sulphate, and subjected to evaporation to remove the solvent. The residue was purified by silica gel Pre-TLC (PE:EA=1:5) to afford the less polar isomer C0175 (52 mg, yield: 17.74%) (Rf=0.6) and the more polar crude isomer. The more polar crude isomer was purified by preparative HPLC and lyophilized to afford the more polar product C0176 (40 mg, yield: 17.73%).
**C0175:** MS *m*/*z* (ESI): 417.0 [M+1]⁺, Rt= 3.666 min
¹H NMR (CD₃OD-*d*₄, 400 MHz) δ 7.42-7.40 (m, 1H), 7.25-7.20 (m, 2H), 7.04 (dd, *J* = 8.0, 2.8 Hz, 1H), 6.86 (dd, *J* = 8.0, 2.8 Hz, 1H), 6.09-6.04 (m, 2H), 4.70-4.13 (m, 3H), 3.87-3.67 (m, 4H), 3.40 (brs, 1H), 2.88-2.81 (m, 2H), 2.28 (s, 3H).
**C0176:** MS *m*/*z* (ESI): 417.0 [M+1]⁺, Rt= 3.596 min
¹H NMR (CD₃OD-*d*₄, 400 MHz) δ 7.42-7.40 (m, 1H), 7.25-7.20 (m, 2H), 7.03 (d, *J* = 8.0 Hz, 1H), 6.86 (d, *J* = 8.0 Hz, 1H), 6.08 (dd, *J* = 19.2, 5.2 Hz, 2H), 4.70-4.15 (m, 3H), 3.88-3.71 (m, 4H), 3.41-3.39 (m, 1H), 2.96-2.79 (m, 2H), 2.28 (s, 3H).

### Examples C0177&C0178

### (S,E)-3-(2,2-Difluoro-7-(2-(hydroxymethyl)-4-(methoxyimino)pyrrolidine-1-carbonyl)benzo[d][1,3]dioxol-4-yl)-2-methylbenzonitrile

### (S,Z)-3-(2,2-Difluoro-7-(2-(hydroxymethyl)-4-(methoxyimino)pyrrolidine-1-carbonyl)benzo[d][1,3]dioxol-4-yl)-2-methylbenzonitrile

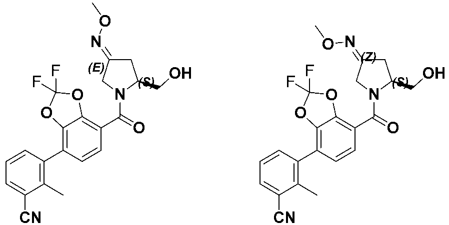

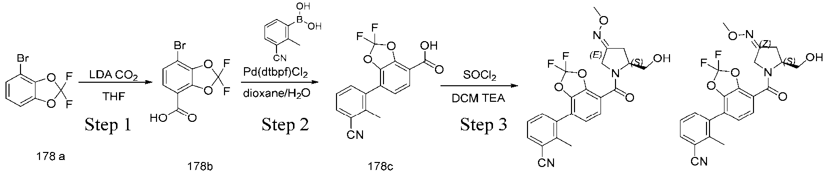

### Step 1

### 7-Bromo-2,2-difluorobenzo[d][1,3]dioxole-4-carboxylic acid 178b

To a solution of n-butyllithium (0.92 g, 14.35 mmol) in THF (25 mL) were successively added diisopropylamine (1.45 g, 14.35 mmol) and 4-bromo-2,2-difluoro-2H-1,3-benzodioxole 178a (3.4 g, 14.35 mmol) at -78°C, and the mixture was reacted at -78°C for 2 h and then poured into dry ice. After the reaction was completed, the reaction solution was directly subjected to rotary evaporation to dryness, to afford crude product 178b (3.6 g, yield: 71.44%).

MS *m*/*z* (ESI): 280.8, 282.8 [M-1]⁻.

### Step 2

### 7-Bromo-2,2-difluorobenzo[d][1,3]dioxole-4-carboxylic acid 178c

To a solution of 178b (1.5 g, 5.34 mmol) in 1,4-dioxane (100 mL) and water (10 mL) were added (3-cyano-2-methylphenyl)boronic acid (1.03 g, 6.41 mmol), potassium carbonate (1.48 g, 10.68 mmol) and Pd(dppf)Cl₂ (31.46 mg, 0.043 mmol), and the mixture was reacted at 65°C for 3 h. The reaction solution was subjected to rotary evaporation to remove most of the solvent, adjusted to pH 3-4 with 6M hydrochloric acid aqueous solution, and extracted with tetrahydrofuran/toluene (5:1). The organic phase was collected, dried, filtered, and subjected to rotary evaporation to afford a crude product. The crude product was slurried with PE/EA and acetonitrile, successively, to afford product 178c (510 mg, yield: 30.12%). MS *m*/*z* (ESI): 316.0 [M-1^{]-}.

### Step 3

### (S,E)-3-(2,2-Difluoro-7-(2-(hydroxymethyl)-4-(methoxyimino)pyrrolidine-1-carbonyl)benzo[d][1,3]dioxol-4-yl) -2-methylbenzonitrile

### (S,Z)-3-(2,2-Difluoro-7-(2-(hydroxymethyl)-4-(methoxyimino)pyrrolidine-1-carbonyl)benzo[d][1,3]dioxol-4-yl) -2-methylbenzonitrile

178c (510 mg, 1.61 mmol) was dissolved in thionyl chloride(10 mL), and the mixture was heated to 75°C for 2 h and subjected to rotary evaporation to dryness. The residue was added to a solution of [(2S,4EZ)-4-(methoxyimino)pyrrolidin-2-yl]methanol (348.17 mg, 2.42 mmol) and triethylamine (488.75 mg, 4.83 mmol) in dichloromethane (5 mL), and the mixture was reacted at room temperature for 2 h. After the reaction was completed, water (10 mL) was added to the reaction solution, and the mixture was extracted with ethyl acetate (20 mL × 3). The organic phases were combined, washed with saturated sodium chloride aqueous solution (10 mL × 3), dried over sodium sulphate, and subjected to evaporation to remove the solvent. The residue was purified by silica gel Pre-TLC (PE:EA=1:5) to afford the less polar isomer C0177 (20 mg, yield: 2.81%) (Rf=0.5) and the more polar crude isomer (Rf=0.45). The more polar crude isomer was purified by preparative HPLC and lyophilized to afford the more polar product C0178 (15.5 mg, yield: 2.17%).
**C0177:** MS *m*/*z* (ESI): 444.0 [M+1]⁺, Rt= 4.350 min
¹H NMR (CD₃OD-*d*₄, 400 MHz) δ 7.81-7.79 (m, 1H), 7.64-7.61 (m, 1H), 7.52-7.48 (m, 1H), 7.43-7.40 (m, 1H), 7.24-7.21 (m, 1H), 4.74-4.14 (m, 3H), 3.89-3.71 (m, 4H), 3.48-3.39 (m, 1H), 3.12-2.60 (m, 2H), 2.46 (s, 3H).
**C0178:** MS *m*/*z* (ESI): 444.0 [M+1]⁺, Rt= 3.430 min
¹H NMR (CD₃OD-*d*₄, 400 MHz) δ 7.81-7.80 (m, 1H), 7.64-7.61 (m, 1H), 7.52-7.48 (m, 1H), 7.43-7.40 (m, 1H), 7.25-7.22 (m, 1H), 4.74-4.14 (m, 3H), 3.89-3.71 (m, 4H), 3.46-3.37 (m, 1H), 2.96-2.76 (m, 2H), 2.46 (s, 3H).

### Examples C0179&C0180

### (S,E) -2',5-Difluoro-4'-(2-(hydroxymethyl)-4-(methoxyimino)pyrrolidine-1-carbonyl)-2-methyl-[1,1'-biphenyl]-3-carbonitrile

### (S,Z)-2',5-Difluoro-4'-(2-(hydroxymethyl)-4-(methoxyimino)pyrrolidine-1-carbonyl)-2-methyl-[1,1'-biphenyl]-3-carbonitrile

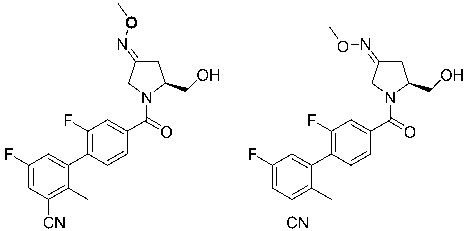

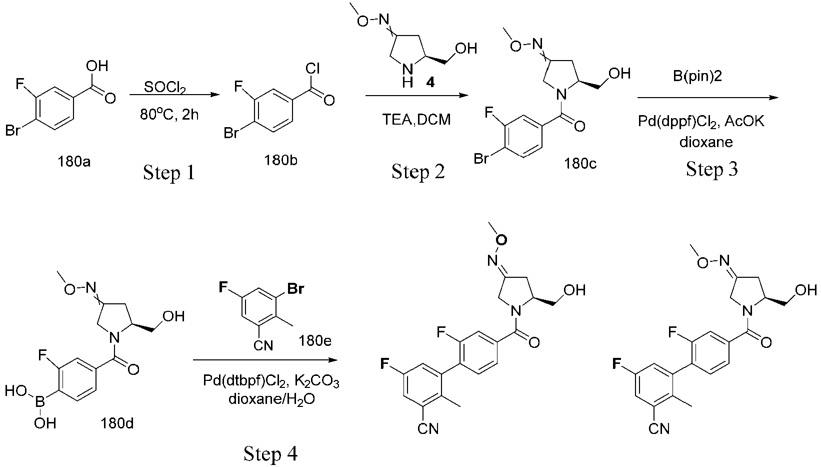

### Step 1

### 4-Bromo-3-fluorobenzoyl chloride 180b

180a (350 mg, 1.60 mmol) was dissolved in thionyl chloride (7 mL), and the mixture was reacted at 80°C for 2 h. After the reaction was completed, the reaction solution was concentrated, dichloromethane was added to the residue, and the mixture was concentrated again to afford 180b (400 mg, yield: 94.87%). The crude product was directly used in the next reaction. The detected product was the methyl ester due to methanol used for sample delivery.

MS *m*/*z* (ESI): 233.0 [M+1]⁺ (sample was quenched with methanol for monitoring).

### Step 2

### (S,EZ)-(4-Bromo-3-fluorophenyl)(2-(hydroxymethyl)-4-(methoxyimino)pyrrolidin-1-yl)methanone 180c

4 (350 mg, 2.18 mmol) and triethylamine (770 mg, 7.60 mmol) were dissolved in dichloromethane (10 mL), 180b (400 mg, 1.52 mmol) was added at room temperature under nitrogen protection, and the mixture was reacted at room temperature for 2 h. The reaction solution was filtered, and the filtrate was collected and concentrated. The residue was separated by column chromatography (PE:EA=1:0 to 1:1) to afford product 180c (320 mg, yield: 61.15%).

MS *m*/*z* (ESI): 345.9 [M+1]⁺.

According to Step 3 to Step 4 in the aforementioned synthetic route, with reference to the synthetic method of compound C0138, the less polar crude isomer C0179 (Rf=0.6) and the more polar crude isomer C0180 (Rf=0.55) were obtained. Further purification by preparative HPLC and lyophilization yielded the less polar product C0179 (20.0 mg, yield: 9.48%) and the more polar product C0180 (21.24 mg, yield: 10.26%).

C0179: MS *m*/*z* (ESI): 400.0 [M+1]⁺, Rt= 3.294 min.

¹H NMR (400 MHz, CD₃OD-*d*₄) *δ* 7.60 (dd, *J =* 8.0 Hz, 2.8 Hz, 1H), 7.51-7.38 (m, 4H), 4.76-4.11 (m, 3H), 3.84-3.36 (m, 5H), 2.87 (brs, 2H), 2.35 (s, 3H).

C0180: MS *m*/*z* (ESI): 400.1 [M+1]⁺, Rt= 3.238 min.

¹H NMR (400 MHz, CD₃OD-*d*₄) *δ* 7.60 (dd, *J =* 8.0 Hz, 2.8 Hz, 1H), 7.51-7.39 (m, 4H), 4.74-4.09 (m, 3H), 3.88-3.39 (m, 5H), 3.06-2.90 (m, 1H), 2.77-2.59 (m, 1H), 2.35 (s, 3H).

### Examples C0181&C0182

### (S, E)-2-Chloro-4'-(2-(1-hydroxyethyl)-4-(methoxyimino)pyrrolidine-1-carbonyl)-[1,1'-biphenyl]-3-carbonitrile

### (S, Z)-2-Chloro-4'-(2-(1-hydroxyethyl)-4-(methoxyimino)pyrrolidine-1-carbonyl)-[1,1'-biphenyl]-3-carbonitrile

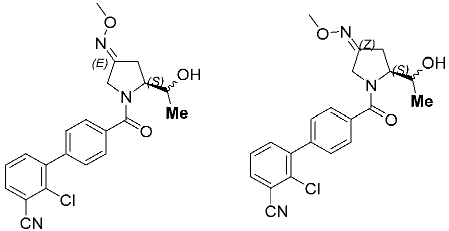

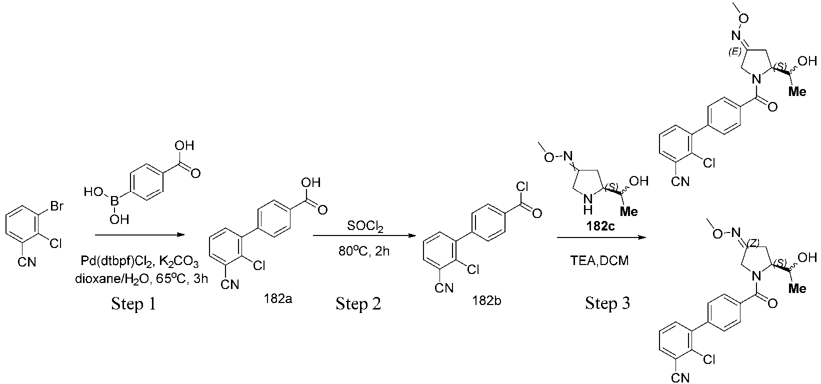

### Step 1

### 2'-Chloro-3'-cyano-[1,1'-biphenyl]-4-carboxylic acid 182a

3-Bromo-2-chlorobenzonitrile (1.4 g, 6.47 mmol), 4-carboxyphenylboronic acid (1.17 g, 7.05 mmol) and potassium carbonate (2.24 mg, 16.18 mmol) were dissolved in 1,4-dioxane (21 mL) and water (3 mL), Pd(dtbpf)Cl₂ (420 mg, 0.64 mmol) was added at room temperature under nitrogen protection, and the mixture was stirred at 65°C for 3 h. The reaction solution was concentrated, water (60 mL) was added, and the mixture was extracted with ethyl acetate (45 mL × 2). The aqueous phase was adjusted to pH about 4 with dilute hydrochloric acid (2 N) and filtered, and the filter cake was rinsed with water (30 mL × 3). The solid was collected, slurried with acetonitrile, filtered to collect solid, and dried to afford 182a (1.0 g, yield: 60.00%).

MS *m*/*z* (ESI): 257.9 [M+1]⁺.

### Step 2

### 4-Bromo-3-fluorobenzoyl chloride 182b

182a (600 mg, 2.32 mmol) was dissolved in thionyl chloride (10 mL), and the mixture was reacted at 80°C for 2 h. After the reaction was completed, the reaction solution was concentrated, dichloromethane was added to the residue, and the mixture was concentrated again to afford 182b (600 mg, yield: 88.50%). The crude product was directly used in the next reaction. The detected product was the methyl ester due to methanol used for sample delivery.

MS *m*/*z* (ESI): 271.9 [M+1]⁺.

### Step 3

### (S,E) -2-Chloro-4'-(2-(1-hydroxyethyl)-4-(methoxyimino)pyrrolidine-1-carbonyl)-[1,1'-biphenyl]-3-carbonitrile

### (S,Z) -2-Chloro-4'-(2-(1-hydroxyethyl)-4-(methoxyimino)pyrrolidine-1-carbonyl)-[1,1'-biphenyl]-3-carbonitrile

182c (400 mg, 2.28 mmol, with reference to the preparation method of 171d, by replacing ethylmagnesium bromide with methylmagnesium bromide, compound 182c was obtained) and triethylamine (1.0 g, 9.88 mmol) were dissolved in dichloromethane (12 mL), 182b (600 mg, 1.96 mmol) was added at room temperature under nitrogen protection, and the mixture was reacted at room temperature for 2 h. The reaction solution was filtered, and the filtrate was collected and concentrated. The residue was purified by silica gel Pre-TLC (PE:EA=1:3) to afford a crude product. Further purification by preparative HPLC and lyophilization yielded the less polar product C0181 (139.05 mg, yield: 17.83%) and the more polar product C0182 (156.34 mg, yield: 20.05%).

C0181: MS *m*/*z* (ESI): 398.1 [M+1]⁺, Rt= 3.200 min.

¹H NMR (400 MHz, CD₃OD-*d*₄) *δ* 7.85 (dd, *J =* 7.6 Hz, 1.6 Hz, 1H), 7.73-7.68 (m, 3H), 7.60-7.55 (m, 3H), 4.62-4.20 (m, 3H), 4.06-4.02 (m, 1H), 3.82 (s, 3H), 2.99-2.68 (m, 2H), 1.22-1.20 (m, 3H).

C0182: MS *m*/*z* (ESI): 398.1 [M+1]⁺, Rt= 3.137 min.

¹H NMR (400 MHz, CD₃OD-*d*₄) *δ* 7.85 (dd, *J =* 8.0 Hz, 1.6 Hz, 1H), 7.74-7.67 (m, 3H), 7.60-7.55 (m, 3H), 4.62-4.20 (m, 3H), 4.05-3.77 (m, 4H), 2.88-2.74 (m, 2H), 1.24-1.22 (m, 3H).

### Examples C0184&C0185&C0186

### (S,E)-3-(7-(2-((R)-1-Hydroxyethyl)-4-(methoxyimino)pyrrolidine-1-carbonyl)benzo[d][1,3]dioxol-4-yl)-2-methylbenzonitrile

### (S,E)-3-(7-(2-((S)-1-Hydroxyethyl)-4-(methoxyimino)pyrrolidine-1-carbonyl)benzo[d][1,3]dioxol-4-yl)-2-methylbenzonitrile

### (S,Z)-3-(7-(2-((R)-1-Hydroxyethyl)-4-(methoxyimino)pyrrolidine-1-carbonyl)benzo[d][1,3]dioxol-4-yl)-2-methylbenzonitrile

### (S,Z)-3-(7-(2-((S)-1-Hydroxyethyl)-4-(methoxyimino)pyrrolidine-1-carbonyl)benzo[d][1,3]dioxol-4-yl)-2-methylbenzonitrile

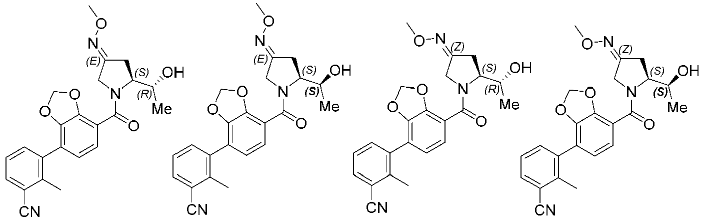

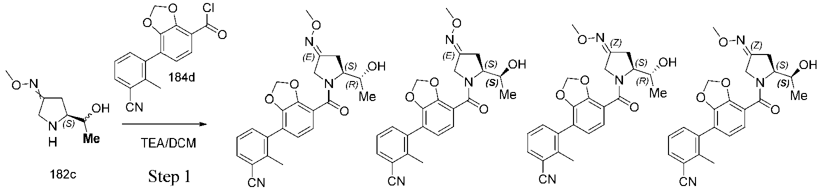

### Step 1

To a solution of 182c (700 mg) in dichloromethane (20 mL) were added 184d (700 mg) and triethylamine (528 mg, 5.22 mmol), and the reaction solution was reacted at room temperature for 2 hours. Water (30 mL) was added to the reaction solution, and the mixture was extracted with ethyl acetate (50 mL × 3). The organic phases were combined, washed with saturated sodium chloride aqueous solution (20 mL × 3), dried over sodium sulphate, and subjected to evaporation to remove the solvent. The residue was purified by silica gel Pre-TLC (PE:EA=1:2) to afford the less polar crude isomers C0184 and C0185 (Rf=0.6) and the more polar crude isomer C0186 (Rf=0.55). Further purification by HPLC yielded the less polar isomers C0184 (10.26 mg, yield: 1.01%) and C0185 (13.81 mg, yield: 1.38%) and the more polar product C0186 (104.15 mg, yield: 5.52%). C0184 (RT = 10.5 min) was the more polar peak in prep-HPLC among the two less polar isomers on TLC, whereas C0185 (RT = 10.9 min) was the less polar peak in prep-HPLC among the two less polar isomers on TLC (C0184 and C0185 completely overlapped on TLC and were indistinguishable). HPLC conditions: chromatographic column: Waters xbrige C18 SN.325|3831918909, 19*150 mm, 5 um, mobile phase: A: 0.1% ammonium bicarbonate in water, B: acetonitrile, gradient variation of B: 35% B from 0 to 3 minutes, 35% to 50% B from 3 to 12 minutes, 98% B from 12 to 16 minutes; column temperature: room temperature, flow rate: 15 ml/min, detection wavelength: 214 nm.

C0186, RT=11.0 min; HPLC conditions: chromatographic column: Waters xbrige C18 SN.315|3818611211, 19*150 mm, 5 um, mobile phase: A: 0.1% ammonium bicarbonate in water, B: acetonitrile, gradient variation of B: 30% B from 0 to 3 minutes, 30% to 55% B from 3 to 12 minutes, 98% B from 12 to 16 minutes; column temperature: room temperature; flow rate: 15 ml/min, detection wavelength: 214 nm.

C0184: MS *m*/*z* (ESI): 422.1 [M+1]⁺.

¹H NMR (DMSO-*d*₆, 400 MHz) *δ* 7.86-7.84 (m, 1H), 7.63-7.61 (m, 1H), 7.49 (t, *J =* 8.0 Hz, 1H), 7.01 (d, *J =* 8.4 Hz, 1H), 6.87 (d, *J =* 8.0 Hz, 1H), 6.16-6.12(m, 2H), 4.91 (d, *J =* 4.8 Hz, 1H), 4.67-3.93 (m, 4H), 3.81-3.76 (m, 3H), 2.82-2.64 (m, 2H), 2.40 (s, 3H), 1.03 (d, *J =* 6.4 Hz, 2H), 0.86-0.79 (m, 1H).

C0185: MS *m*/*z* (ESI): 422.1 [M+1]⁺.

¹H NMR (DMSO-*d*₆, 400 MHz) *δ* 7.85 (dd, *J =* 7.6, 1.2 Hz, 1H), 7.62 (dd, *J* = 7.6, 1.2 Hz, 1H), 7.49 (t, *J =* 8.0 Hz, 1H), 7.01 (dd, *J =* 8.0, 4.4 Hz, 1H), 6.90-6.86 (m, 1H), 6.14-6.10 (m, 2H), 4.96-4.89 (m,1H), 4.43-4.28 (m, 2H),4.03-3.95 (m, 2H), 3.81-3.76 (m, 3H), 2.85-2.65 (m, 2H), 2.40-2.38 (m, 3H), 1.07 (d, *J* = 6.4 Hz, 2H), 0.82-0.80 (m, 1H).

C0186: MS *m*/*z* (ESI): 422.1 [M+1]⁺.

¹H NMR (DMSO-*d*₆, 400 MHz) *δ* 7.86 (d, *J =* 7.2 Hz, 1H), 7.64 (t, *J =* 7.2 Hz, 1H), 7.49 (t, *J =* 8.0 Hz, 1H), 7.04-7.00 (m, 1H), 6.89 (d, *J =* 8.0 Hz, 1H), 6.17-6.09 (m, 2H), 4.91-4.89 (m,1H), 4.65-4.25 (m, 2H),4.12-3.93 (m, 2H), 3.80-3.69 (m, 3H), 2.87-2.58 (m, 2H), 2.41-2.38 (m, 3H), 1.09-1.03 (m, 2H), 0.83 (d, *J* = 6.4 Hz, 1H).

### Examples C0187&C0188

### (S,E)-4'-(2-(Hydroxymethyl)-4-(methoxyimino)pyrrolidine-1-carbonyl)-2-methyl-2'-(methyl-d₃)-[1,1'-biphenyl]-3-carbonitrile

### (S,Z)-4'-(2-(Hydroxymethyl)-4-(methoxyimino)pyrrolidine-1-carbonyl)-2-methyl-2'-(methyl-d₃)-[1,1'-biphenyl]-3-carbonitrile

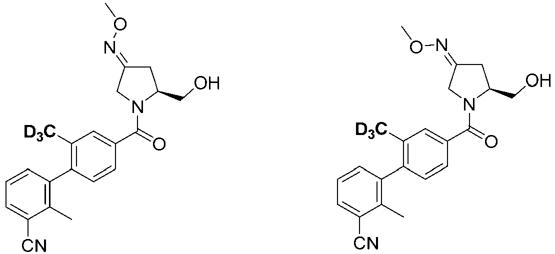

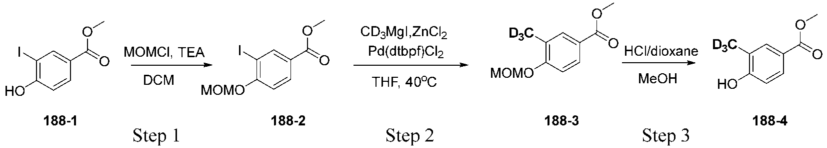

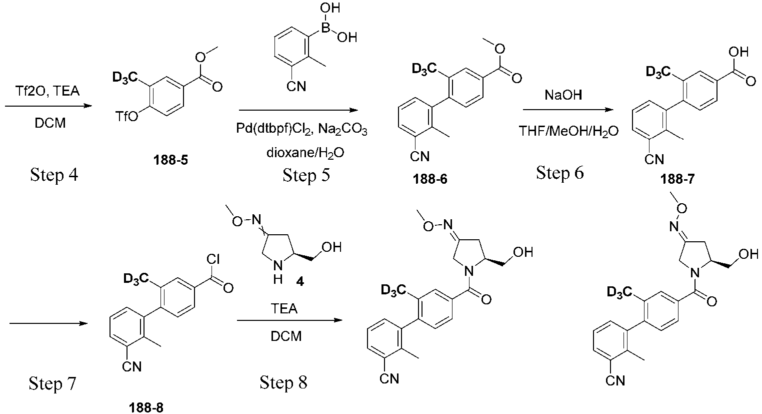

### Step 1

### Methyl 3-iodo-4-(methoxymethoxy)benzoate 188-2

188-1 (2.0 g, 7.19 mmol) and triethylamine (1.46 g, 14.38 mmol) were dissolved in dichloromethane (30 mL), chloromethyl methyl ether (1.36 g, 14.38 mmol) was added dropwise at 0°C under nitrogen protection, and the mixture was stirred at room temperature for 2 hours. Water (50 mL) was added to the reaction solution, and the mixture was extracted with dichloromethane (3 × 50 mL). The organic layers were combined, washed with saturated sodium chloride aqueous solution (3 × 60 mL), combined, dried over anhydrous sodium sulphate, filtered, and concentrated, and the crude product was purified by silica gel column chromatography (PE/EA=3:1). The eluate was collected and concentrated to afford 188-2 (2.0 g, yield: 77.69%).

MS *m*/*z* (ESI): 322.9 [M+1]⁺.

### Step 2

### Methyl 4-(methoxymethoxy)-3-(methyl-d₃)benzoate 188-3

Deuterated methylmagnesium iodide (39.74 mL, 39.74 mmol; 1M in diethyl ether) was dissolved in THF (200 mL), and the mixture was cooled to 0°C. 0.7M solution of zinc chloride (2.7 g, 19.87 mmol) in tetrahydrofuran was slowly added dropwise, and after the addition was completed, the mixture was stirred at room temperature for 5 hours. 188-2 (2.0 g, 6.21 mmol) and Pd(dtbpf)Cl₂ (405 mg, 0.62 mmol) were added, and the mixture was stirred at room temperature overnight. Saturated ammonium chloride solution (200 mL) was added to quench the reaction solution, and the reaction solution was extracted with ethyl acetate (200 mL × 3). The organic phases were combined, washed with saturated sodium chloride aqueous solution (200 mL), dried over anhydrous sodium sulphate, and concentrated. The residue was purified by silica gel column chromatography (PE:EA=5:1) to afford 188-3 (1.3 g, yield: 98.18%) as a brown oil.

MS *m*/*z* (ESI): 214.0 [M+1]⁺.

### Step 3

### Methyl 4-hydroxy-3-(methyl-d₃)benzoate 188-4

188-3 (1.3 g, 6.10 mmol) was dissolved in methanol (30 mL), 4M HCl/dioxane solution (30 mL) was added, and the mixture was stirred at room temperature for 2 hours. The reaction solution was concentrated, and the residue was purified by normal-phase silica gel column chromatography (PE:EA=5:1) to afford 188-4 (1.0 g, yield: 96.95%) as a brown solid.

MS *m*/*z* (ESI): 170.0 [M+1]⁺.

### Step 4

### Methyl 3-(methyl-d₃)-4-(((trifluoromethyl)sulfonyl)oxy)benzoate 188-5

188-4 (1.0 g, 5.91 mmol) and triethylamine (1.79 g, 17.73 mmol) were dissolved in dichloromethane (10 mL), trifluoromethanesulfonic anhydride (3.33 g, 11.82 mmol) was added, and the mixture was stirred at room temperature for 2 hours. The reaction solution was diluted with dichloromethane (50 mL), washed with saturated sodium chloride aqueous solution (50 mL × 2), dried, and concentrated. The residue was purified by silica gel column chromatography (PE:EA=10:1) to afford 188-5 (1.4 g, yield: 78.63%) as a yellow oil.

MS *m*/*z* (ESI): 302.2 [M+1]⁺.

¹H NMR (400 MHz, DMSO-*d*₆) *δ* 8.06 (d, *J =* 2.4 Hz, 1H), 7.96 (dd, *J =* 8.4, 2.4 Hz, 1H), 7.55 (d, *J =* 8.4 Hz, 1H), 3.88 (s, 3H).

### Step 5

### Methyl 3'-cyano-2'-methyl-2-(methyl-d₃)-[1,1'-biphenyl]-4-carboxylate 188-6

(3-Cyano-2-methylphenyl)boronic acid (1.08 g, 6.72 mmol), 188-5 (1.35 g, 4.48 mmol), Pd(dtbpf)Cl₂ (292 mg, 0.45 mmol), and sodium carbonate (1.42 g, 13.44 mmol) were dissolved in 1,4-dioxane (50 mL) and water (5 mL). The mixture was purged three times with nitrogen and stirred at 65°C for 3 hours under nitrogen protection. The reaction solution was diluted with ethyl acetate (100 mL), washed with saturated sodium chloride aqueous solution (100 mL × 2), dried over anhydrous sodium sulphate, and concentrated. The residue was purified by silica gel column chromatography (PE:EA=10:1) to afford 188-6 (600 mg, yield: 49.90%) as a yellow oil.

MS *m*/*z* (ESI): 269.0 [M+1]⁺.

### Step 6

### 3'-Cyano-2'-methyl-2-(methyl-d₃)-[1,1'-biphenyl]-4-carboxylic acid 188-7

188-6 (550 mg, 2.05 mmol) was dissolved in a mixed solvent of tetrahydrofuran (30 mL), methanol (10 mL) and water (10 mL), sodium hydroxide (410 mg, 10.25 mmol) was added, and the mixture was stirred at room temperature for 2 hours. The reaction solution was concentrated, and the aqueous phase was adjusted to pH 2 with 1M hydrochloric acid and extracted with ethyl acetate (50 mL × 3). The organic phases were combined, dried over anhydrous sodium sulphate, and concentrated, to afford 188-7 (470 mg, yield: 90.17%) as a yellow solid.

MS *m*/*z* (ESI): 255.0 [M+1]⁺.

¹H NMR (400 MHz, DMSO-*d*₆) *δ* 12.89 (brs, 1H), 7.92 (d, *J =* 1.6 Hz, 1H), 7.86-7.83 (m, 2H), 7.50-7.44 (m, 2H), 7.23 (d, *J =* 8.0 Hz, 1H), 2.17 (s, 3H).

### Step 7

### 3'-Cyano-2'-methyl-2-(methyl-d₃)-[1,1'-biphenyl]-4-formyl chloride 188-8

188-7 (470 mg, 1.85 mmol) was dissolved in thionyl chloride (10 mL), and the mixture was heated to 85°C and stirred for 2 hours. The reaction solution was concentrated to afford 188-8 (500 mg, yield: 99.19%) as a yellow solid.

MS *m*/*z* (ESI): 269.0 [M+1]⁺ (sample was quenched with methanol for monitoring).

### Step 8

### (S,E)-4'-(2-(Hydroxymethyl)-4-(methoxyimino)pyrrolidine-1-carbonyl)-2-methyl-2'-(methyl-d₃)-[1,1'-biphenyl]-3-carbonitrile

### (S,Z)-4'-(2-(Hydroxymethyl)-4-(methoxyimino)pyrrolidine-1-carbonyl)-2-methyl-2'-(methyl-d₃)-[1,1'-biphenyl]-3-carbonitrile

188-8 (500 mg, 1.83 mmol), 4 (496 mg, 2.75 mmol), and triethylamine (741 mg, 7.32 mmol) were dissolved in dichloromethane (20 mL), and the mixture was stirred at room temperature for 3 hours. The reaction solution was washed with saturated sodium chloride aqueous solution (20 mL), dried over anhydrous sodium sulphate, and concentrated, and the residue was purified by silica gel PTLC (EA) to afford the less polar isomer C0187 (88 mg, yield: 11.40%, Rf = 0.6) and the more polar crude isomer (Rf = 0.55). The more polar isomer was purified by HPLC (NH₄HCO₃) to afford C0188 (109 mg, yield: 15.45%).
**C0187:** MS *m*/*z* (ESI): 381.1 [M+1]⁺, Rt= 3.222 min
¹H NMR (400 MHz, DMSO-*d*₆) *δ* 7.85-7.83 (m, 1H), 7.49-7.42 (m, 4H), 7.17 (d, *J* = 8.0 Hz, 1H), 4.98 (t, *J =* 5.6 Hz, 1H), 4.61-4.01 (m, 3H), 3.78 -3.51 (m, 4H), 3.31-3.22 (m, 1H), 2.75-2.74 (m, 2H), 2.19 (s, 3H).
**C0188:** MS *m*/*z* (ESI): 381.1 [M+1]⁺, Rt= 3.223 min
¹H NMR (400 MHz, DMSO-*d*₆) *δ* 7.85-7.82 (m, 1H), 7.48-7.41 (m, 4H), 7.18 (d, *J =* 8.0 Hz, 1H), 4.96 (brs, 1H), 4.60-3.97 (m, 3H), 3.81-3.54 (m, 4H), 3.31-3.25 (m, 1H), 2.95-2.81 (m, 1H), 2.67-2.56 (m, 1H), 2.19 (s, 3H).

### Examples C0189&C0190

### (S,E)-3-(5-(2-(Hydroxymethyl)-4-(methoxyimino)pyrrolidine-1-carbonyl)-2,3-dihydro-[1,4]dioxino[2,3-c]pyridin-8-yl)-2-methylbenzonitrile

### (S,Z)-3-(5-(2-(Hydroxymethyl)-4-(methoxyimino)pyrrolidine-1-carbonyl)-2,3-dihydro-[1,4]dioxino[2,3-c]pyridin-8-yl)-2-methylbenzonitrile

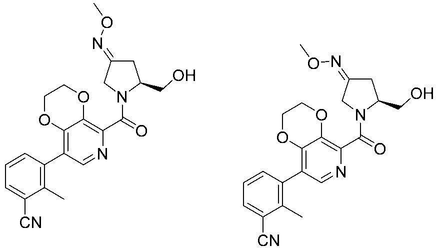

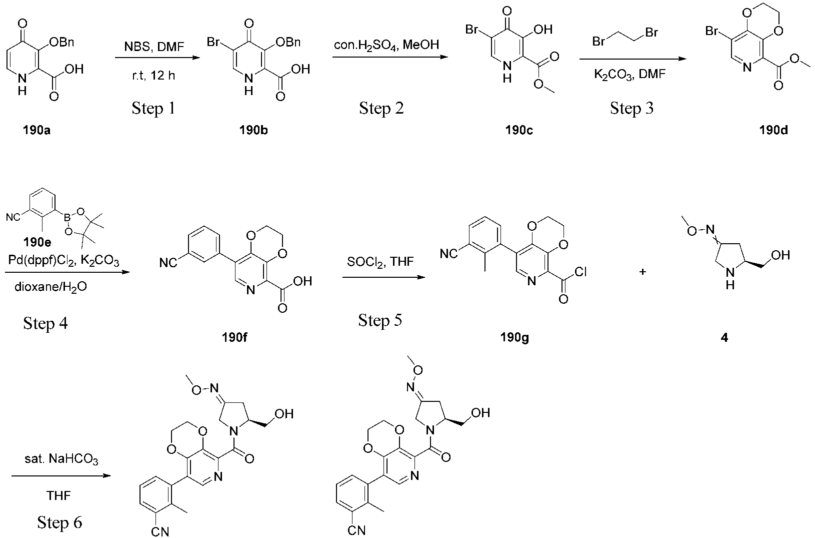

### Step 1

### 3-(Benzyloxy)-5-bromo-4-oxo-1,4-dihydropyridine-2-carboxylic acid 190b

190a (4.5 g, 18.35mmol) was dissolved in DMF (45 mL), NBS (3.59 g, 20.19 mmol) was added portionwise, and the mixture was reacted at 20°C for 16 hours. Water (200 mL) was added to quench the reaction solution, and the reaction solution was extracted with ethyl acetate (100 mL × 3). The organic phases were combined, washed with saturated sodium chloride aqueous solution (20 mL × 3), dried over sodium sulphate, and subjected to evaporation to remove the solvent to afford the crude product 190b (3.5 g, yield: 58.8%).

MS *m*/*z* (ESI): 323.9 [M+1]⁺.

### Step 2

### Methyl 5-bromo-3-hydroxy-4-oxo-1,4-dihydropyridine-2-carboxylate 190c

190b (400 mg, 1.23 mmol) was dissolved in methanol (10 mL), concentrated sulphuric acid (920 mg, 9.38 mmol) was slowly added, and the mixture was reacted at 80°C for 16 hours. The reaction solution was concentrated, and the residue was purified by reversed-phase column chromatography (water:acetonitrile=80: 20) to afford 190c (200 mg, yield: 65.6%).

MS *m*/*z* (ESI): 247.8 [M+1]⁺.

### Step 3

### Methyl 8-bromo-2,3-dihydro-[1,4]dioxino[2,3-c]pyridine-5-carboxylate 190d

190c (200 mg, 0.81 mmol) was dissolved in DMF (10 mL), potassium carbonate (340 mg, 2.43 mmol) and 1,2-dibromoethane (180 mg, 0.97 mmol) were added, and the mixture was reacted at 90°C for 5 hours. Water (10 mL) was added to quench the reaction solution, and the reaction solution was extracted with ethyl acetate (10 mL × 2). The organic phases were combined, washed with saturated sodium chloride aqueous solution (10 mL × 3), dried over sodium sulphate, filtered, and concentrated. The crude product was purified by preparative TLC (EA/PA=1/1) to afford 190d (100 mg, yield: 45.2%).

MS *m*/*z* (ESI): 275.9 [M+1]⁺.

### Step 4

### 8-(3-Cyano-2-methylphenyl)-2,3-dihydro-[1,4]dioxino[2,3-c]pyridine-5-carboxylic acid 190f

190d (80 mg, 0.29 mmol), 190e (110 mg, 0.43 mmol) and potassium carbonate (100 mg, 0.72 mmol) were dissolved in 1,4-dioxane (4 mL) and water (0.5 mL), and Pd(dppf)Cl₂ (11 mg, 0.05 mmol) was added at room temperature under nitrogen protection. After nitrogen purging, the mixture was reacted at 90°C for 2 hours. Water (10 mL) was added to quench the reaction solution, and the reaction solution was extracted with ethyl acetate (10 mL × 3). The organic phases were combined, washed with saturated sodium chloride aqueous solution (10 mL), dried over sodium sulphate, filtered, and concentrated. The crude product was purified by preparative TLC (EA/PA=1/1) to afford 190f(60 mg, yield: 69.4%).

MS *m*/*z* (ESI): 297.2 [M+1]⁺.

### Step 5

### 8-(3-Cyano-2-methylphenyl)-2,3-dihydro-[1,4]dioxino[2,3-c]pyridine-5-carbonyl chloride 190g

190f (60 mg, 0.2 mmol) was dissolved in THF (2 mL), thionyl chloride (0.15 mL) and a drop of DMF were added, and the mixture was reacted at 60°C for 2 hours. After the reaction was completed, the reaction solution was concentrated to afford crude 190g (64 mg, yield: 100%), which was directly used in the next reaction.

### Step 6

### (S,E)-3-(5-(2-(Hydroxymethyl)-4-(methoxyimino)pyrrolidine-1-carbonyl)-2,3-dihydro-[1,4]dioxino[2,3-c]pyridin-8-yl)-2-methylbenzonitrile

### (S,Z)-3-(5-(2-(Hydroxymethyl)-4-(methoxyimino)pyrrolidine-1-carbonyl)-2,3-dihydro-[1,4]dioxino[2,3-c]pyridin-8-yl)-2-methylbenzonitrile

4 (43 mg, 0.3 mmol) was dissolved in THF (4 mL), saturated sodium bicarbonate solution (1 mL) was added, followed by 190g (64 mg, 0.2 mmol), and the mixture was reacted at 25°C for 2 hours. Water (10 mL) was added to quench the reaction solution, and the reaction solution was extracted with dichloromethane (10 mL × 2). The organic phases were combined, washed with saturated sodium chloride aqueous solution (10 mL), dried over sodium sulphate, filtered, and concentrated. The residue was purified by silica gel Pre-TLC (PE:EA=1:3) to afford the less polar isomer C0189 (Rf=0.6, 8.4 mg, yield: 9.76%) and the more polar isomer C0190 (Rf=0.55, 9.6 mg, yield: 11.9%).
**C0189:** MS *m*/*z* (ESI): 423.0 [M+1]⁺, Rt= 2.693 min
¹H NMR (400 MHz, CDCl₃) *δ* 7.94-7.89 (m, 1H), 7.70-7.68 (m, 1H), 7.39-7.38 (m, 2H), 4.92-4.77 (m, 1H), 4.44-4.32 (m, 5H), 4.16-4.04 (m, 2H), 3.90-3.85 (m, 3H), 3.76-3.50 (m, 2H), 2.99-2.64 (m, 2H), 2.38 (s, 3H).
**C0190:** MS *m*/*z* (ESI): 423.0 [M+1]⁺, Rt= 2.656 min
¹H NMR (400 MHz, CDCl₃) *δ* 7.94-7.89 (m, 1H), 7.70-7.69 (m, 1H), 7.45-7.36 (m, 2H), 4.89-4.76 (m, 1H), 4.47-4.37 (m, 5H), 4.21-4.01 (m, 2H), 3.91-3.85 (m, 3H), 3.79-3.51 (m, 2H), 3.01-2.70 (m, 2H), 2.41 (s, 3H).

### Examples C0191&C0192

### (S,E)-3-(7-(2-(Hydroxymethyl)-4-(methoxyimino)pyrrolidine-1-carbonyl)-2,3-dihydro-1H-inden-4-yl)-2-methylbenzonitrile

### (S,Z)-3-(7-(2-(Hydroxymethyl)-4-(methoxyimino)pyrrolidine-1-carbonyl)-2,3-dihydro-1H-inden-4-yl)-2-methylbenzonitrile

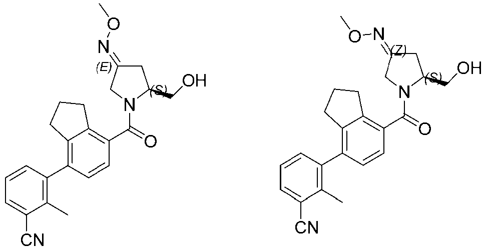

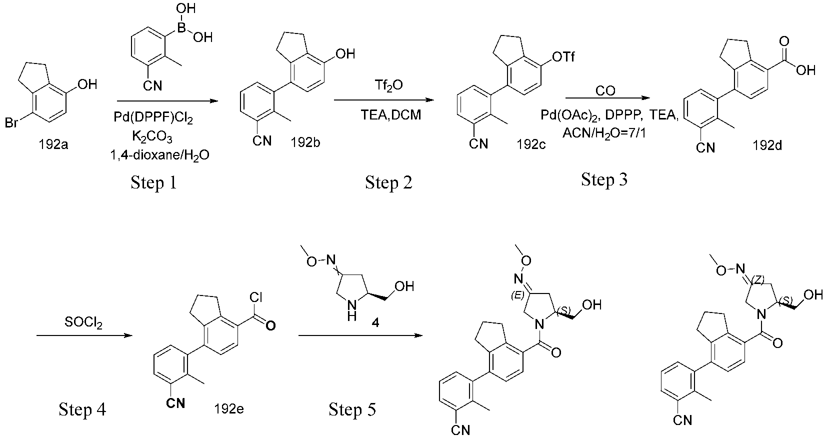

According to Step 1 to Step 3 in the aforementioned synthetic route, with reference to the synthetic method of compound C0082, product 192d (650 mg, yield: 59.59%) was obtained.

MS m/z (ESI): 277.8 [M+1]⁺

### Step 4

### 7-(3-Cyano-2-methylphenyl)-2,3-dihydro-1H-indene-4-carbonyl chloride 192e

192d (350 mg, 1.26 mmol) was dissolved in thionyl chloride (6 mL), and the mixture was reacted at 80°C for 2 h. After the reaction was completed, the reaction solution was concentrated, dichloromethane was added to the residue, and the mixture was concentrated again to afford 192e (350 mg, yield: 93.77%). The crude product was directly used in the next reaction. The detected product was the methyl ester due to methanol used for sample delivery.

MS m/z (ESI): 292.0 [M+1]⁺

### Step 5

### (S,E)-3-(7-(2-(Hydroxymethyl)-4-(methoxyimino)pyrrolidine-1-carbonyl)-2,3-dihydro-1H-inden-4-yl)-2-methylbenzonitrile

### (S,Z)-3-(7-(2-(Hydroxymethyl)-4-(methoxyimino)pyrrolidine-1-carbonyl)-2,3-dihydro-1H-inden-4-yl)-2-methylbenzonitrile

4 (250 mg, 1.56 mmol) and triethylamine (600 mg, 5.90 mmol) were dissolved in dichloromethane (10 mL), 192e (350 mg, 1.18 mmol) was added at room temperature under nitrogen protection, and the mixture was reacted at room temperature for 2 h. The reaction solution was filtered, and the filtrate was collected and concentrated to afford a crude product. The crude product was purified by preparative silica gel thin-layer chromatography (PE:EA=1:3) to afford the less polar crude product (Rf=0.6) and the more polar product (Rf=0.55). Further purification by preparative HPLC and lyophilization yielded the less polar product C0191 (75.0 mg, yield: 15.36%) and the more polar product C0192 (105.22 mg, yield: 21.99%).
C0191: MS m/z (ESI): 404.1 [M+1]⁺, Rt= 3.577 min
¹H NMR (400 MHz, CD₃OD-*d*₄) *δ* 7.70 (dd, *J =* 6.8 Hz, 2.0 Hz, 1H), 7.50-7.39 (m, 2H), 7.30 (d, *J* = 7.6 Hz, 1H), 7.08 (d, *J =* 7.6 Hz, 1H), 4.73-4.50 (m, 1H), 4.20-3.34 (m, 7H), 3.10-2.83 (m, 4H), 2.73-2.52 (m, 2H), 2.30 (s, 3H), 2.13-2.05 (m, 2H).
C0192: MS m/z (ESI): 404.1 [M+1]⁺, Rt= 3.519 min
¹H NMR (400 MHz, CD₃OD-*d*₄) δ 7.70 (dd, *J =* 7.2 Hz, 2.0 Hz, 1H), 7.48-7.40 (m, 2H), 7.32-7.27 (m, 1H), 7.08 (d, *J =* 7.6 Hz, 1H), 4.72-4.47 (m, 1H), 4.25-3.35 (m, 7H), 3.09-2.86 (m, 3H), 2.79-2.54 (m, 3H), 2.31 (s, 3H), 2.13-2.01 (m, 2H).

### Examples C0193&C0194&C0195

### 4'-((2S,E)-2-((R)-Cyclopropyl(hydroxyl)methyl)-4-(methoxyimino)pyrrolidine-1-carbonyl)-2-methyl-[1,1'-biphenyl]-3-carbonitrile

### 4'-((2S,E)-2-((S)-Cyclopropyl(hydroxyl)methyl)-4-(methoxyimino)pyrrolidine-1-carbonyl)-2-methyl-[1,1'-biphenyl]-3-carbonitrile

### 4'-((2S,Z)-2-((R)-Cyclopropyl(hydroxyl)methyl)-4-(methoxyimino)pyrrolidine-1-carbonyl)-2-methyl-[1,1'-biphenyl]-3-carbonitrile

### 4'-((2S,Z)-2-((S)-Cyclopropyl(hydroxyl)methyl)-4-(methoxyimino)pyrrolidine-1-carbonyl)-2-methyl-[1,1'-biphenyl]-3-carbonitrile

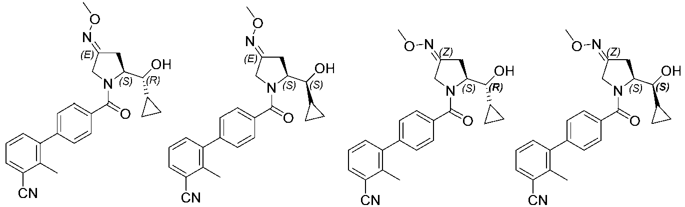

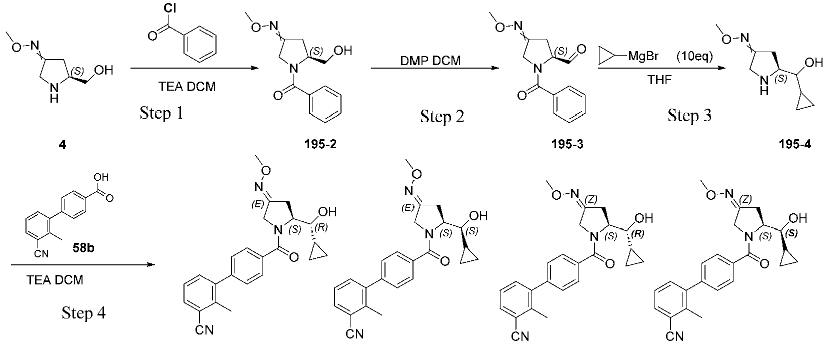

### Step 1

### [(2S,4EZ)-1-Benzoyl-4-(methoxyimino)pyrrolidin-2-yl]methanol 195-2

To a solution of 4 (1 g, 6.94 mmol) in dichloromethane (20 mL) were added benzoyl chloride (1.3 g, 9.25 mmol) and triethylamine (3.51 g, 34.7 mmol) at 0°C, and the mixture was reacted at room temperature for 1 hour. After the reaction was completed, the reaction solution was filtered, and the filtrate was washed with water (20 mL). The organic layer was washed with saturated sodium chloride aqueous solution, dried over anhydrous sodium sulphate, filtered, and subjected to rotary evaporation to afford a crude product. The crude product was purified by normal-phase silica gel column chromatography (PE:EA=1:3) to afford product 195-2 (920 mg, yield: 53.42%).

MS *m*/*z* (ESI): 249.0 [M+1]⁺.

### Step 2

### (2S,4EZ)-1-Benzoyl-4-(methoxyimino)pyrrolidine-2-carbaldehyde 195-3

To a solution of 195-2 (900 mg, 3.62 mmol) in DCM (20 mL) was added Dess-Martin (2.00 g, 4.71 mmol), and the mixture was reacted at room temperature for 2 hours. The reaction solution was extracted with ethyl acetate/water (3×50/30 mL). The organic layers were combined, washed with saturated sodium chloride aqueous solution (3 × 20 mL), dried over anhydrous sodium sulphate, filtered, and subjected to rotary evaporation to afford a crude product. The crude product was purified by reversed-phase column chromatography (ACN:H₂O=5:95 to 45:55), to afford product 195-3 (450 mg, yield: 50.41%).

MS *m*/*z* (ESI): 247.0 [M+1]⁺.

### Step 3

### (5S,EZ)-5-((RS)-Cyclopropyl(hydroxyl)methyl)pyrrolidin-3-oneO-methyloxime 195-4

195-3 (417 mg, 1.69 mmol) was dissolved in tetrahydrofuran (10 mL), and the mixture was cooled to 0°C. Cyclopropylmagnesium bromide tetrahydrofuran solution (16.9 mL, 16.9 mmol) was slowly added dropwise, and the mixture was stirred at room temperature for 5 hours. The reaction solution was cooled to 0°C, and 4M HCl/dioxane solution (5 mL) was slowly added to neutralize the reaction solution. After the addition was completed, the mixture was concentrated at 30°C, which was directly used in the next reaction.

MS *m*/*z* (ESI): 184.9 [M+1]⁺.

### Step 4

### 4'-((2S,E)-2-((R)-Cyclopropyl(hydroxyl)methyl)-4-(methoxyimino)pyrrolidine-1-carbonyl)-2-methyl-[1,1'-biphenyl]-3-carbonitrile

### 4'-((2S,E)-2-((S)-Cyclopropyl(hydroxyl)methyl)-4-(methoxyimino)pyrrolidine-1-carbonyl)-2-methyl-[1,1'-biphenyl]-3-carbonitrile

### 4'-((2S,Z)-2-((R)-Cyclopropyl(hydroxyl)methyl)-4-(methoxyimino)pyrrolidine-1-carbonyl)-2-methyl-[1,1'-biphenyl]-3-carbonitrile

### 4'-((2S,Z)-2-((S)-Cyclopropyl(hydroxyl)methyl)-4-(methoxyimino)pyrrolidine-1-carbonyl)-2-methyl-[1,1'-biphenyl]-3-carbonitrile

4-(3-Cyano-2-methylphenyl)benzoic acid 58b (400 mg, 1.69 mmol) was dissolved in thionyl chloride (10 mL), and the mixture was heated to 80°C and stirred for 3 hours. The reaction solution was concentrated, and the residue was dissolved in dichloromethane (10 mL). The mixture was slowly added dropwise to a solution of 195-4 (300 mg, 1.63 mmol) and triethylamine (660 mg, 6.52 mmol) in dichloromethane (20 mL), and the resulting mixture was stirred at room temperature for 2 hours. The reaction solution was washed with saturated sodium chloride aqueous solution (20 mL), dried over anhydrous sodium sulphate, and concentrated. The residue was purified by silica gel Prep-TLC (EA) to afford a crude product, which was purified by prep-HPLC (chromatographic column: Waters xbrige C18 SN.325|3831918909, 19*150 mm, 5 um, mobile phase: A: 0.1% ammonium bicarbonate in water, B: acetonitrile, gradient variation of B: 35% B from 0 to 3 minutes, 35% to 50% B from 3 to 15 minutes, 98% B from 15 to 20 minutes; column temperature: room temperature, flow rate: 15 ml/min, detection wavelength: 214 nm) to afford three isomers: C0193 (13 mg, yield: 1.97%, collected as the fourth peak, RT: 15.0 min), C0194 (25 mg, yield:3.80%, collected as the second and third peaks, RT: 13.8 min), and C0195 (12 mg, yield: 1.82%, collected as the first peak, RT: 13.1 min).
**C0193:** MS *m*/*z* (ESI): 404.1 [M+1]⁺
¹H NMR (400 MHz, DMSO-*d*₆) *δ* 7.84 (d, *J =* 7.6 Hz, 1H), 7.63-7.44(m, 6H), 5.05-5.03 (m, 1H), 4.69-4.23 (m, 2H), 4.11-3.93 (m, 1H), 3.81-3.76 (m, 3H), 3.26-3.16 (m, 1H), 3.00-2.67(m, 2H), 2.41 (s, 3H), 0.87-0.51 (m, 1H), 0.44-0.14 (m, 4H).
**C0194:** MS *m*/*z* (ESI): 404.1 [M+1]⁺
¹H NMR (400 MHz, DMSO-*d*₆) *δ* 7.84 (d, *J =* 7.2 Hz, 1H), 7.64-7.44(m, 6H), 5.05-4.97 (m, 1H), 4.83-3.93 (m, 3H), 3.80-3.71 (m, 3H), 3.18-3.14 (m, 1H), 3.00-2.67(m, 2H), 2.41 (s, 3H), 0.89-0.52 (m, 1H), 0.44-0.18 (m, 4H).
**C0195:** MS *m*/*z* (ESI): 404.1 [M+1]⁺
¹H NMR (400 MHz, DMSO-*d*₆) *δ* 7.84 (d, *J =* 6.8 Hz, 1H), 7.63-7.46(m, 6H), 4.97-4.96 (m, 1H), 4.81-4.31 (m, 2H), 4.11-3.96 (m, 1H), 3.80-3.71 (m, 3H), 3.20-3.19 (m, 1H), 3.02-2.67(m, 2H), 2.41 (s, 3H), 0.83-0.55 (m, 1H), 0.46-0.11 (m, 4H).

### Examples C0196&C0197

### (S,E)-5-(7-(2-(Hydroxymethyl)-4-(methoxyimino)pyrrolidine-1-carbonyl)benzo[d][1,3]dioxol-4-yl)-4-methylnicotinonitrile

### (S,Z)-5-(7-(2-(Hydroxymethyl)-4-(methoxyimino)pyrrolidine-1-carbonyl)benzo[d][1,3]dioxol-4-yl)-4-methylnicotinonitrile

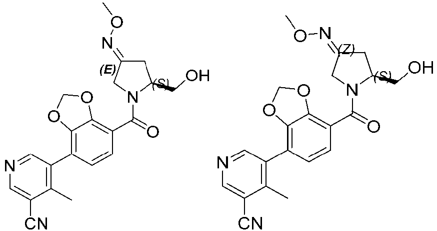

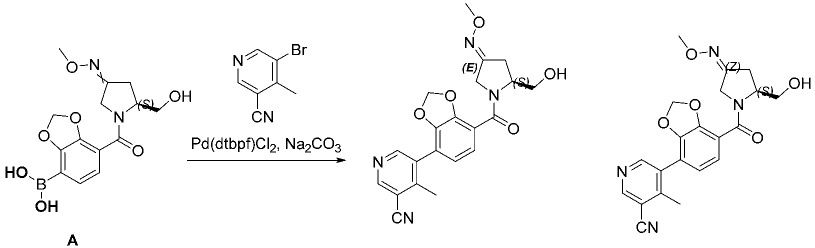

### Step 1

### (S,E)-5-(7-(2-(Hydroxymethyl)-4-(methoxyimino)pyrrolidine-1-carbonyl)benzo[d][1,3]dioxol-4-yl)-4-methylnicotinonitrile

### (S,Z)-5-(7-(2-(Hydroxymethyl)-4-(methoxyimino)pyrrolidine-1-carbonyl)benzo[d][1,3]dioxol-4-yl)-4-methylnicotinonitrile

A (200 mg, 0.60 mmol), 3-bromo-4-methylnicotinonitrile (154 mg, 0.78 mmol), Pd(dtbpf)Cl₂ (39 mg, 0.060 mmol) and sodium carbonate (190 mg, 1.80 mmol) were dissolved in 1,4-dioxane (10 mL) and water (1 mL). The mixture was purged three times with nitrogen and stirred at 85°C for 3 hours. Water (20 mL) was added to the reaction solution, and the mixture was extracted with ethyl acetate (20 mL × 2). The organic phases were combined, washed with saturated sodium chloride aqueous solution (70 mL), dried over anhydrous sodium sulphate, and subjected to evaporation to remove the solvent. The residue was purified by silica gel Pre-TLC (PE:EA=1:5) to afford the less polar crude isomer C0196 (Rf=0.5) and the more polar crude isomer C0197 (Rf=0.4). The crude product C0196 was purified by HPLC (NH₄HCO₃) to afford C0196 (30.1 mg, yield: 12.35%), and the crude product C0197 was purified by HPLC (NH₄HCO₃) to afford C0197 (41 mg, yield: 16.84%).
**C0196:** MS *m*/*z* (ESI): 409.1 [M+1]⁺
¹H NMR (400 MHz, DMSO-*d*₆) *δ* 8.99 (s, 1H), 8.74 (s, 1H), 7.04 (d, *J =* 8.0 Hz, 1H), 6.96 (d, *J=* 8.0 Hz, 1H), 6.17-6.12 (m, 2H), 4.98 (d, *J=* 5.6 Hz, 1H), 4.57-4.16 (m, 2H), 4.09-4.05 (m, 1H), 3.81-3.77 (m, 3H), 3.55-3.52 (m, 1H), 3.31-3.24 (m, 1H), 2.77-2.75 (m, 2H), 2.45 (s, 3H).
**C0197:** MS *m*/*z* (ESI): 409.1 [M+1]⁺
¹H NMR (400 MHz, DMSO-*d*₆) δ 8.99 (s, 1H), 8.76-8.74 (m, 1H), 7.05 (d, *J =* 8.0 Hz, 1H), 6.97 (d, *J =* 8.0 Hz, 1H), 6.17-6.12 (m, 2H), 4.98 (d, *J =* 5.6 Hz, 1H), 4.55-4.02 (m, 3H), 3.81-3.74 (m, 3H), 3.55-3.53 (m, 1H), 3.27-3.22 (m, 1H), 2.94-2.84 (m, 1H), 2.67-2.56 (m, 1H), 2.45 (s, 3H).

### Examples A0210&A0220

### (S,Z)-4'-(2-(Hydroxymethyl)-4-(methoxyimino)pyrrolidine-1-carbonyl)-2-methyl-3-(oxetan-3-yloxy)-[1,1'-biphenyl]-3-carbonitrile

### (S,E)-4'-(2-(Hydroxymethyl)-4-(methoxyimino)pyrrolidine-1-carbonyl)-2-methyl-3-(oxetan-3-yloxy)-[1,1'-biphenyl]-3-carbonitrile

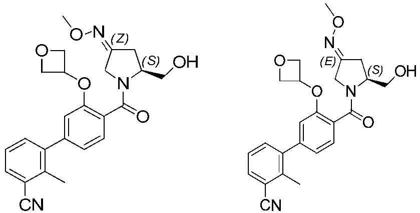

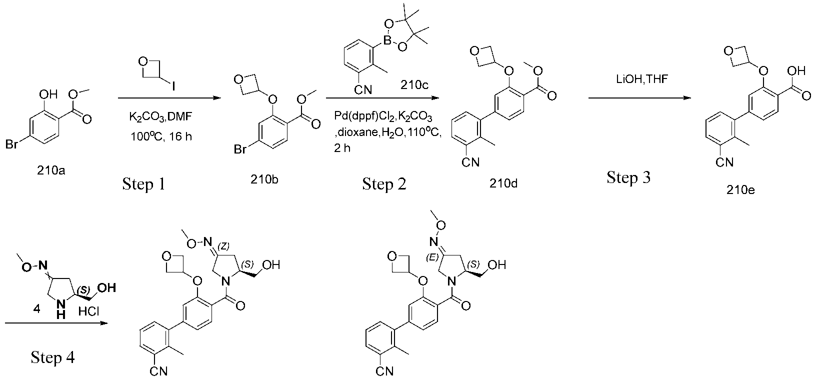

### Step 1

### Methyl 4-bromo-2-(oxetan-3-yloxy)benzoate 210b

210a (300 mg, 1.30mmol), 3-iodooxetane (0.24 g, 1.3 mmol), and potassium carbonate (0.54 g, 3.90 mmol) were mixed in DMF (4 mL). The reaction solution was stirred at 100°C for 16 hours. The reaction solution was extracted with ethyl acetate (20 mL x 3). The organic phases were combined, dried over anhydrous sodium sulphate, and concentrated, and the crude product was purified by column chromatography (petroleum ether/ethyl acetate = 5:1), to afford product 210b (270 mg, yield: 72.42%).

MS *m*/*z* (ESI): 287.1 [M+1]⁺.

According to Step 2 to Step 4 in the aforementioned synthetic route, with reference to the synthetic method of compound C0061, the less polar isomer A0220 (Rf=0.25) and the more polar isomer A0210 (Rf=0.2) were obtained. Further purification by preparative HPLC and lyophilization yielded the more polar product A0210 (20.09 mg, yield: 9.54%) and the less polar product A0220 (10.95 mg, yield: 5.13%).

A0210: MS *m*/*z* (ESI): 436.0 [M+1]⁺.

¹H NMR (400 MHz, Chloroform-d) *δ* 7.68-7.63 (m, 1H), 7.45-7.33 (m, 3H), 7.02-6.97 (m, 1H), 6.38-6.35 (m, 1H), 5.28-5.21 (m, 1H), 4.97-4.92 (m, 2H), 4.85-4.72 (m, 3H), 4.32-4.20 (m, 1H), 4.15-4.08 (m, 1H), 3.98-3.78 (m, 5H), 3.03-2.95 (m, 1H), 2.73-2.67 (m, 1H), 2.44 (d, *J =* 6.4 Hz, 3H).

A0220: MS *m*/*z* (ESI): 436.0 [M+1]⁺.

¹H NMR (400 MHz, Chloroform-d) δ 7.60-7.58 (m, 1H), 7.46-7.28 (m, 3H), 6.93-6.89 (m, 1H), 6.35-6.24 (m, 1H), 5.26-5.21 (m, 1H), 4.97-4.95 (m, 2H), 4.76-4.73 (m, 3H), 4.38-4.21 (m, 1H), 4.13-4.08 (m, 1H), 3.97-3.78 (m, 5H), 3.03-2.97 (m, 1H), 2.72-2.66 (m, 1H), 2.44 (d, *J =* 6.4 Hz, 3H).

### Examples A0226&A0249

### (S,E)-4'-(2-(Hydroxymethyl)-4-(methoxyimino)piperidine-1-carbonyl)-2-methyl-[1,1'-biphenyl]-3-carbonitrile

### (S,Z)-4'-(2-(Hydroxymethyl)-4-(methoxyimino)piperidine-1-carbonyl)-2-methyl-[1,1'-biphenyl]-3-carbonitrile

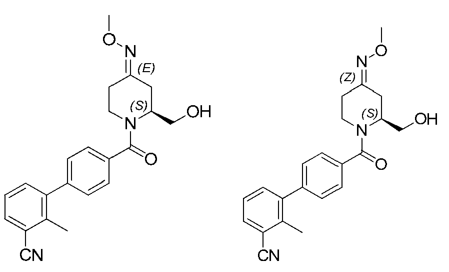

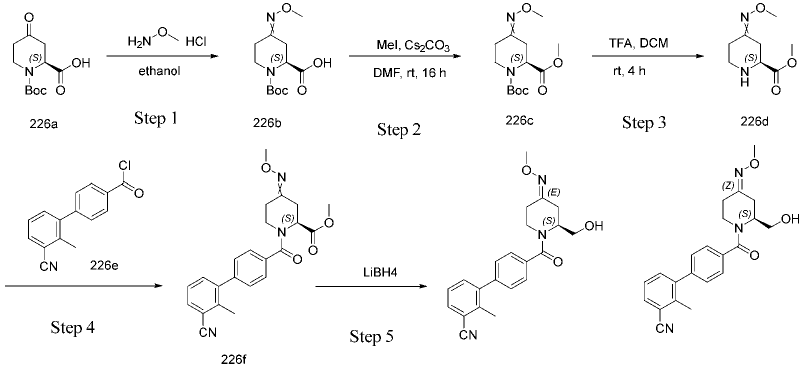

### Step 1

### (S,ZE)-1-(Tert-butyloxycarbonyl)-4-(methoxyimino)piperidine-2-carboxylic acid 226b

226a (400 mg, 1.64 mmol), methoxyamine hydrochloride (0.15 g, 1.80 mmol), and TEA (0.18 g, 1.80 mmol) were mixed in ethanol (10 mL), and the reaction solution was stirred at room temperature for 16 hours. The reaction solution was stirred at 100°C for 16 hours. The reaction solution was extracted with ethyl acetate (20 mL x 3). The organic phases were combined, dried over anhydrous sodium sulphate, and concentrated, and the crude product was purified by column chromatography (petroleum ether/ethyl acetate = 2:1), to afford product 226b (160 mg, yield: 35.73%).

MS *m*/*z* (ESI): 273.1 [M+1]⁺.

### Step 2

### (S,ZE)-1-Tert-butyl-2-methyl-4-(methoxyimino)piperidine-1,2-dicarboxylate 226c

226b (400 mg, 1.47 mmol) and potassium carbonate (0.61 g, 4.41 mmol) were mixed in DMF (8 mL), iodomethane (0.42 g, 0.18 mmol) was added, and the reaction solution was stirred at room temperature for 16 hours. Ethyl acetate was added to the reaction solution, and the mixture was washed with saturated sodium bicarbonate and water. The organic phase was dried over anhydrous sodium sulphate and concentrated, and the crude product was purified by column chromatography (petroleum ether/ethyl acetate = 2:1), to afford 226c (200 mg, yield: 47.55%).

MS *m*/*z* (ESI): 287.1 [M+1]⁺.

### Step 3

### Methyl (S,ZE)-4-(methoxyimino)piperidine-2-carboxylate 226d

226c (200 mg, 0.53 mmol) was dissolved in dichloromethane (3 mL), TFA (1 mL) was added, and the reaction solution was stirred at room temperature for 2 hours. The reaction solution was concentrated to afford crude 226d (130 mg).

MS *m*/*z* (ESI): 187.1 [M+1]⁺

### Step 4

### (S, ZE)-Methyl-1-(3'-cyano-2'-methyl-[1,1'-biphenyl]-4-carbonyl)-4-(methoxyimino)piperidine-2-carboxylate 226f

226d (100 mg, 0.54 mmol) and TEA (160 mg, 1.62 mmol) were dissolved in DCMF (8 mL), and then 226e (150 mg, 059 mmol) was added. The reaction solution was stirred at room temperature for 16 hours. The reaction solution was diluted with 5 mL of water and extracted with dichloromethane (10 mL × 3). The organic phases were combined, dried over anhydrous sodium sulphate, and filtered, and the filtrate was concentrated under reduced pressure. The crude product was separated by silica gel column chromatography (EA:PE=1:3) to afford 226f (100 mg, yield: 45.93%).

MS *m*/*z* (ESI): 406.1 [M+1]⁺

### Step 5

### (S,E)-4'-(2-(Hydroxymethyl)-4-(methoxyimino)piperidine-1-carbonyl)-2-methyl-[1,1'-biphenyl]-3-carbonitrile

### (S,Z)-4'-(2-(Hydroxymethyl)-4-(methoxyimino)piperidine-1-carbonyl)-2-methyl-[1,1'-biphenyl]-3-carbonitrile

226f (100 mg, 0.25 mmol) was dissolved in methanol (2 mL) and THF (2 mL), and then lithium borohydride (16 mg, 0.75 mmol) was added. The reaction solution was stirred at room temperature for 16 hours. The reaction solution was diluted with 5 mL of water and extracted with ethyl acetate (10 mL × 3). The organic phases were combined, washed with sodium chloride aqueous solution (10 mL × 3), dried over anhydrous sodium sulphate, and filtered, and the filtrate was concentrated under reduced pressure. The crude product was separated by silica gel Prep-TLC (EA:PE=3:1) to afford the less polar isomer A0249 (Rf=0.25) and the more polar isomer A0226 (Rf=0.2). Further purification by preparative HPLC and lyophilization yielded the more polar product A0226 (3.91 mg, yield: 4.07%) and the less polar product A0249 (12.50 mg, yield: 13.20%).

A0226: MS *m*/*z* (ESI): 378.0 [M+1]⁺.

¹H NMR (400 MHz, Chloroform-d) δ 7.58 (d, *J =* 6.8 Hz, 1H), 7.49-7.42 (m, 2H), 7.38-7.32 (m, 1H), 7.30-7.22 (m, 3H), 5.10-4.74 (m, 1H), 3.86-3.52 (m, 5H), 3.42-3.12 (m, 2H), 2.75-2.12 (m, 7H).

A0249: MS *m*/*z* (ESI): 378.0 [M+1]⁺.

¹H NMR (400 MHz, Chloroform-d) δ 77.57 (d, *J =* 6.8 Hz, 1H), 7.52-7.41 (m, 2H), 7.37-7.33 (m, 1H), 7.31-7.22 (m, 3H), 5.08-4.72 (m, 1H), 3.86-3.10 (m, 7H), 2.78-2.10 (m,7H).

### Examples A0227&A0256

### 3-(7-[(2S,4Z)-2-(Hydroxymethyl)-4-(methoxyimino)pyrrolidine-1-carbonyl]-1,3-dihydro-2-benzofuran-4-yl)-2-methylbenzonitrile

### 3-(7-[(2S,4E)-2-(Hydroxymethyl)-4-(methoxyimino)pyrrolidine-1-carbonyl]-1,3-dihydro-2-benzofuran-4-yl)-2-methylbenzonitrile

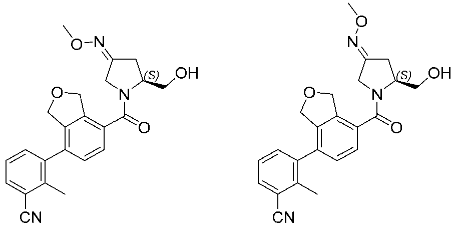

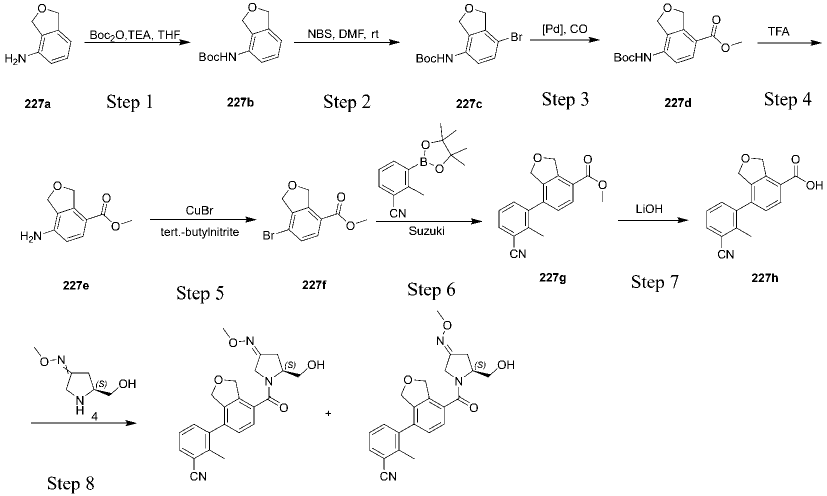

### Step 1

### Tert-butyl N-(1,3-dihydro-2-benzofuran-4-yl)carbamate 227b

227a (1 g, 7.40 mmol) was dissolved in methanol (15 mL), and the mixture was reacted at 50°C for 2 hours. The reaction solution was directly concentrated to afford product 227b (1.7 g, crude) as a yellow oil.

MS *m*/*z* (ESI): 180.1 [M-55+1]⁺.

### Step 2

Tert-butyl N-(7-bromo-1,3-dihydro-2-benzofuran-4-yl)carbamate 227c 227b (0.085 g, 0.63 mmol) was dissolved in DMF (10 mL), NBS (1.29 g, 7.23 mmol) was slowly added at 20°C, and the mixture was reacted for 16 hours. The reaction solution was dried using an oil pump, mixed with adsorbent, purified by silica gel column chromatography (PE/EA=85/15), and concentrated to afford product 227c (2.0 g, yield: 88.10%) as a yellow solid.

MS *m*/*z* (ESI): 258.0 [M-55+1]⁺.

### Step 3

### Methyl 7-{[(tert-butoxy)carbonyl]amino}-1,3-dihydro-2-benzofuran-4-carboxylate 227d

227c (0.860 g, 2.74 mmol), Pd(dppf)Cl₂ (0.20 g, 0.27 mmol), and sodium carbonate (1.16 g, 10.96 mmol) were mixed in methanol (20 mL), and the mixture was reacted at 120°C under a 2M CO atmosphere for 16 hours. The reaction solution was directly concentrated, mixed with adsorbent, purified by silica gel column chromatography (PE/EA=78/22), and concentrated to afford product 227d (480 mg, yield: 59.78%) as a light yellow solid.

MS *m*/*z* (ESI): 238.1 [M-55+1]⁺.

### Step 4

### Methyl 7-amino-1,3-dihydro-2-benzofuran-4-carboxylate 227e

227d (0.450 g, 1.53 mmol) was dissolved in TFA/DCM pre-cooled at 0°C, and the mixture was reacted at 0°C for 5-10 min. The reaction solution was immediately concentrated to dryness, and the residue was treated with dichloromethane once to remove impurities. LCMS analysis indicated complete consumption of the substrate, with a single major peak corresponding to the product. The reaction solution was directly concentrated, and the residue was treated with dichloromethane twice to remove impurities and diluted with 10 mL of ethyl acetate. 10 mL of saturated sodium bicarbonate was added, and the mixture was extracted three times with ethyl acetate (10 mL x 3). The organic phases were combined, dried, and concentrated to afford product 227e (295 mg, yield: 99.53%) as a light yellow solid.

MS *m*/*z* (ESI): 194.2 [M+1]⁺.

### Step 5

### Methyl 7-bromo-1,3-dihydro-2-benzofuran-4-carboxylate 227f

227e (0.280 g, 1.45 mmol), CuBr (0.31 g, 2.17 mmol), and tert-butyl nitrite (0.22 g, 2.17 mmol) were mixed in acetonitrile (10 mL), and the mixture was reacted at 70°C for 6 hours. The reaction solution was filtered, concentrated, mixed with adsorbent, purified by silica gel column chromatography (PE/EA=85/15), and concentrated to afford product 227f (200 mg, yield: 53.68%) as a light yellow solid.

MS *m*/*z* (ESI): 257.0 [M+1]⁺.

### Step 6

### Methyl 7-(3-cyano-2-methylphenyl)-1,3-dihydro-2-benzofuran-4-carboxylate 227g

227f (200 mg, 0.78 mmol), 2-methyl-3-(tetramethyl-1,3,2-dioxaborolan-2-yl)benzonitrile (0.19 g, 0.78 mmol), Pd(dppf)Cl₂ (0.057 g, 0.078 mmol), and potassium carbonate (0.22 g, 1.56 mmol) were mixed in 1,4-dioxane (3 mL) and water (0.6 mL), and the mixture was reacted at 100°C under argon protection for 16 hours. The reaction solution was directly concentrated, mixed with adsorbent, purified by silica gel column chromatography (PE/EA=84/16), and concentrated to afford product 227g (110 mg, yield: 48.20%) as a light yellow solid.

MS *m*/*z* (ESI): 294.1 [M+1]⁺.

### Step 7

### 7-(3-Cyano-2-methylphenyl)-1,3-dihydro-2-benzofuran-4-carboxylic acid 227h

227g (0.110 g, 0.38 mmol) and lithium hydroxide (0.080 g, 1.9 mmol) were dissolved in tetrahydrofuran (4 mL), methanol (4 mL), and water (4 mL), and the mixture was reacted at 40°C for 2 hours. The reaction solution was concentrated to remove a portion of the solvent, adjusted to pH=2-3 with 1M hydrochloric acid, and extracted three times with ethyl acetate. The organic phases were combined, dried, and concentrated to afford product 227h (100 mg, yield: 95.48%) as a yellow solid.

MS *m*/*z* (ESI): 294.1 [M+1]⁺.

### Step 8

### 3-(7-[(2S,4Z)-2-(Hydroxymethyl)-4-(methoxyimino)pyrrolidine-1-carbonyl]-1,3-dihydro-2-benzofuran-4-yl)-2-methylbenzonitrile

### 3-(7-[(2S,4E)-2-(Hydroxymethyl)-4-(methoxyimino)pyrrolidine-1-carbonyl]-1,3-dihydro-2-benzofuran-4-yl)-2-methylbenzonitrile

227h (100 mg, 0.36 mmol) was dissolved in DCM (10 mL), thionyl chloride (0.21 g, 1.80 mmol) was added at 20°C, and the mixture was reacted at 40°C for 2 hours. LCMS/TLC analysis indicated complete consumption of the substrate and the appearance of a new spot (indicating formation of the acyl chloride). The reaction solution was directly concentrated, and the residue was treated with dichloromethane twice to remove impurities. The resulting material was dissolved in anhydrous THF and slowly added dropwise to a pre-prepared alkaline solution of 4 (0.067 g, 0.47 mmol) and sodium bicarbonate (0.24 g, 2.88 mmol) in THF (4 mL) and water (4 mL). After the dropwise addition was completed, the mixture was reacted at 20°C for another 16 hours. The reaction solution was directly extracted three times with ethyl acetate, and the organic phases were combined, dried, and concentrated. The crude product was separated by silica gel Prep-TLC (EA:PE=3:1) to afford the less polar isomer A0256 (Rf=0.30) and the more polar isomer A0227 (Rf=0.25). Further purification by preparative HPLC and lyophilization yielded the more polar product A0227 (8.76 mg, yield: 6.26%) and the less polar product A0256 (5.84 mg, yield: 4.17%).

A0227: MS *m*/*z* (ESI): 406.2 [M+1]⁺.

¹H-NMR (400 MHz, Chloroform-d) *δ* 7.68-7.66 (m, 1H), 7.43 (d, *J =* 8.0 Hz, 1H), 7.39-7.34 (m, 2H), 7.16-7.11 (m, 1H), 5.38-4.78 (m, 4H), 4.39-4.12 (m, 2H), 3.98-3.70 (m, 5H), 3.20-3.12 (m, 1H), 2.98-2.92 (m, 1H), 2.72-2.66 (m, 1H), 2.35 (d, *J* = 6.4 Hz, 3H).

A0256: MS *m*/*z* (ESI): 406.2 [M+1]⁺.

¹H-NMR (400 MHz, Chloroform-*d*) *δ* 7.68-7.66 (m, 1H), 7.42-7.35 (m, 3H), 7.14-7.12 (m, 1H), 5.36-5.33 (m, 1H), 5.17-5.14 (m, 1H), 4.90-4.78 (brs, 2H), 4.38-4.12 (m, 2H), 3.98-3.55 (m, 5H), 3.19-3.12 (m, 1H), 3.00-2.68 (m, 2H), 2.75 (s, 2H), 2.35 (d, J = 6.4 Hz, 3H).

### Examples A0235&A0253

### (S,Z)-3-(9-(2-(Hydroxymethyl)-4-(methoxyimino)pyrrolidine-1-carbonyl)-3,4-dihydro-2H-benzo[b][1,4]dioxepin-6-yl)-2-methylbenzonitrile

### (S,E)-3-(9-(2-(Hydroxymethyl)-4-(methoxyimino)pyrrolidine-1-carbonyl)-3,4-dihydro-2H-benzo[b][1,4]dioxepin-6-yl)-2-methylbenzonitrile

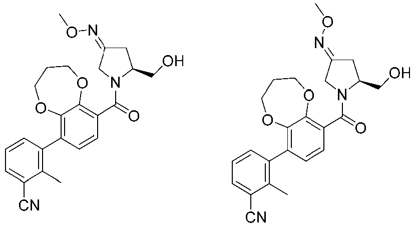

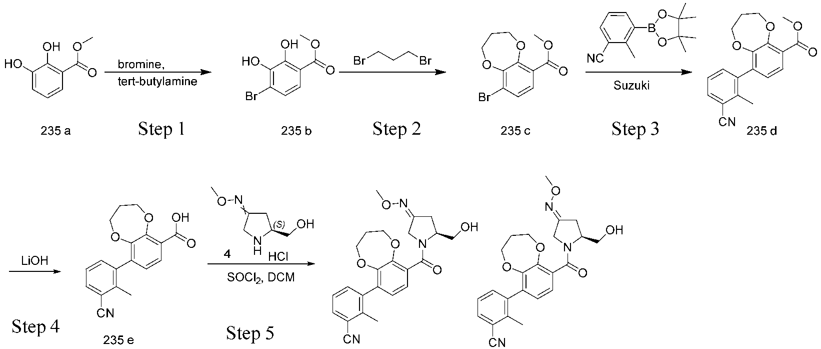

According to Step 1 to Step 4 in the aforementioned synthetic route, with reference to the synthetic method of compound C0061, product 235e (560 mg, yield: 61.12%) was obtained.

MS *m*/*z* (ESI): 310.0 [M+1]⁺

### Step 5

### (S,Z)-3-(9-(2-(Hydroxymethyl)-4-(methoxyimino)pyrrolidine-1-carbonyl)-3,4-dihydro-2H-benzo[b][1,4]dioxepin-6-yl)-2-methylbenzonitrile

(*S*,*E*)-3-(9-(2-(Hydroxymethyl)-4-(methoxyimino)pyrrolidine-1-carbonyl)-3,4-dihydro-2H-benzo[b][1,4]dioxepin-6-yl)-2-methylbenzonitrile

The starting material 235e (150 mg, 0.48 mmol) was dissolved in dichloromethane (2 mL), and then thionyl chloride (4.92 g, 41.35 mmol) was added, and the reaction solution was reacted at 40°C for 2 hours. After the reaction was completed, the reaction solution was concentrated to afford a crude product. The starting material 4 (0.10 g, 0.72 mmol) was dissolved in tetrahydrofuran (2 mL), and then sodium bicarbonate aqueous solution (0.28 g, 3.36 mmol) was added, and the mixture was reacted for 10 minutes. The acyl chloride was added dropwise at 0°C, and then the reaction solution was reacted at room temperature for 2 hours. Then, 10 ml of water was added, and the mixture was extracted with dichloromethane. The organic phases were combined, washed with sodium chloride aqueous solution, dried over anhydrous sodium sulphate, and filtered, and the filtrate was concentrated under reduced pressure. The crude product was separated by silica gel Prep-TLC (EA:PE=2:1) to afford the less polar isomer A0235 (Rf=0.25) and the more polar isomer A0253 (Rf=0.2). The resulting less polar isomer A0235 (Rf=0.4) and more polar isomer A0253 (Rf=0.3) were further purified by prep-HPLC (chromatographic column: Welch Xtimate C18, 21.2*250 mm, 10 um; mobile phase: A: 10 mmol/L ammonium bicarbonate in water, B: acetonitrile, gradient variation of B: 30% to 50% in 17 minutes; column temperature: room temperature, flow rate: 25 ml/min, detection wavelength: 214 nm) and lyophilized to afford the more polar product A0253 (9.42 mg, yield: 4.39%, RT: 11.64 min) and the less polar product A0235 (6.98 mg, yield: 3.06%, RT: 11.64 min).

A0235: MS *m*/*z* (ESI): 436 [M+1]⁺.

¹H NMR (400 MHz, Chloroform-d) δ 7.63 (dd, *J =* 7.2, 1.6 Hz, 1H), 7.33 (d, *J* = 7.6 Hz, 2H), 6.98 (d, *J =* 7.6 Hz, 1H), 6.84 (d, *J =* 7.6 Hz, 1H), 4.83 - 4.70 (m, 1H), 4.45 - 3.92 (m, 7H), 3.84 (s, 3H), 3.78 (d, *J =* 6.0 Hz, 1H), 2.96 (d, *J =* 9.2 Hz, 1H), 2.67-2.60 (m, 1H), 2.36 (s, 3H), 2.19 (brs, 2H).

A0253: MS *m*/*z* (ESI): 436 [M+1]⁺.

¹H NMR (400 MHz, Chloroform-d) δ 7.66 - 7.59 (m, 1H), 7.32 (d, *J =* 5.2 Hz, 2H), 6.98 (d, *J =* 7.6 Hz, 1H), 6.83 (d, *J =* 7.2 Hz, 1H), 4.82 - 4.68 (m, 1H), 4.55 - 3.94 (m, 7H), 3.86 (s, 3H), 3.75 (dd, *J =* 11.2, 6.0 Hz, 1H), 2.97 (dd, *J =* 18.8, 9.6 Hz, 1H), 2.70 (d, *J =* 18.8 Hz, 1H), 2.35 (d, *J =* 3.6 Hz, 3H), 2.19 (brs, 2H).

### Examples A0241&A0244

### 3-(4-[(2S,4Z)-2-(Hydroxymethyl)-4-(methoxyimino)pyrrolidine-1-carbonyl]-3-methylphenyl)-2-methylbenzonitrile

### 3-(4-[(2S,4E)-2-(Hydroxymethyl)-4-(methoxyimino)pyrrolidine-1-carbonyl]-3-methylphenyl)-2-methylbenzonitrile

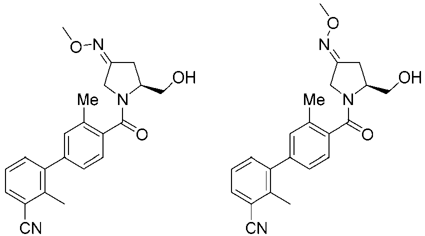

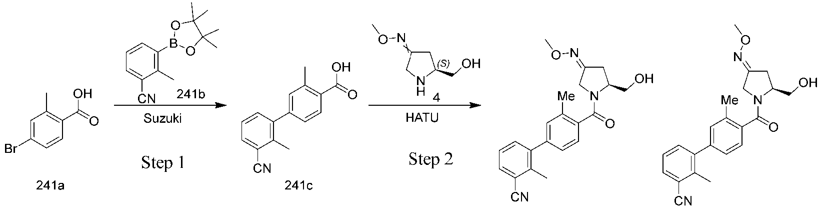

According to the aforementioned synthetic route, with reference to the synthetic method of compound C0102, the more polar product A0241 (12.32 mg, yield: 7.99%) and the less polar product A0244 (9.53 mg, yield: 6.20%) were obtained.

A0241: MS *m*/*z* (ESI): 378.2 [M+1]⁺.

¹H-NMR (400 MHz, Chloroform-d) δ 7.64 (d, *J =* 7.6 Hz, 1H), 7.43 (d, *J =* 6.4 Hz, 1H), 7.36-7.28 (m, 2H), 7.15 (d, *J =* 7.2 Hz, 2H), 4.83 (dd, *J =* 9.6, 6.0 Hz, 1H), 4.12-3.92 (m, 3H), 3.83-3.79 (m, 4H), 3.13 (brs, 1H), 2.99 (d, *J =* 8.8 Hz, 1H), 2.65 (d, *J =* 18.4 Hz, 1H), 2.46 (s, 3H), 2.38 (s, 3H).

A0244 MS *m*/*z* (ESI): 378.2 [M+1]⁺.

¹H-NMR (400 MHz, Chloroform-d) δ 7.64 (d, *J=* 6.4 Hz, 1H), 7.40 (d, *J =* 6.4 Hz, 1H), 7.36-7.29 (m, 2H), 7.15 (d, *J =* 7.2 Hz, 2H), 4.86 (s, 1H), 4.09-3.92 (m, 3H), 3.86-3.80 (m, 4H), 3.26 (brs, 1H), 3.00-2.93 (m, 1H), 2.71 (d, *J =* 18.4 Hz, 1H), 2.44 (s, 3H), 2.39 (s, 3H).

### Examples A0242&A0248

### (S,Z)-4'-(2-(Hydroxymethyl)-4-(methoxyimino)pyrrolidine-1-carbonyl)-2'-methoxy-2-methyl-[1,1'-biphenyl]-3-carbonitrile

### (S,E)-4'-(2-(Hydroxymethyl)-4-(methoxyimino)pyrrolidine-1-carbonyl)-2'-methoxy-2-methyl-[1,1'-biphenyl]-3-carbonitrile

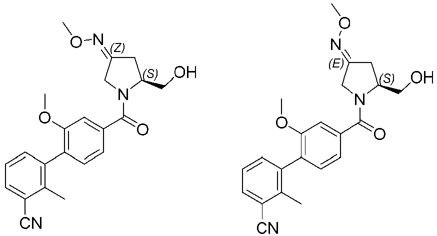

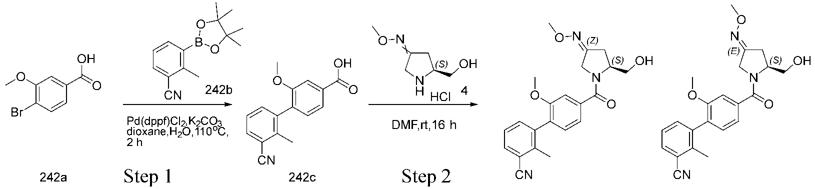

According to the aforementioned synthetic route, with reference to the synthetic method of compound C0102, the less polar isomer A0248 (Rf=0.25) and the more polar isomer A0242 (Rf=0.2) were obtained. Further purification by preparative HPLC and lyophilization yielded the more polar product A0242 (8.95 mg, yield: 2.98%) and the less polar product A0248 (6.96 mg, yield: 2.32%).

A0242: MS *m*/*z* (ESI): 394.0 [M+1]⁺.

¹H NMR (400 MHz, Chloroform-d) δ 7.65 - 7.60 (m, 1H), 7.38 (s, 1H), 7.32 (d, J = 7.8 Hz, 1H), 7.16 - 7.10 (m, 3H), 4.86 (brs, 1H), 4.45 - 4.16 (m, 2H), 3.82 - 3.72 (m, 8H), 2.94 - 2.82 (m, 1H), 2.66 - 2.57 (m, 1H), 2.32 (s, 3H).

A0248: MS *m*/*z* (ESI): 394.0 [M+1]⁺.

¹H NMR (400 MHz, Chloroform-d) δ 7.63-7.61 (m, 1H), 7.33 (dd, *J =* 9.2, 8.8 Hz, 2H), 7.16 - 7.09 (m, 3H), 4.87 (brs,1H), 4.57 - 4.18 (m, 2H), 3.79 - 3.72 (m, 8H), 3.08 - 2.86 (m, 1H), 2.82 - 2.59 (m, 1H), 2.30 (s, 3H).

### Examples A0243&A0245

### 3-(4-[(2S,4Z)-2-(Hydroxymethyl)-4-(methoxyimino)pyrrolidine-1-carbonyl]-3-methylphenyl)-2-methylbenzonitrile

### 3-(4-[(2S,4E)-2-(Hydroxymethyl)-4-(methoxyimino)pyrrolidine-1-carbonyl]-3-methylphenyl)-2-methylbenzonitrile

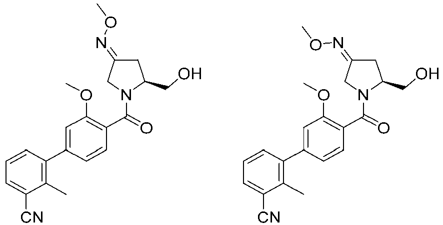

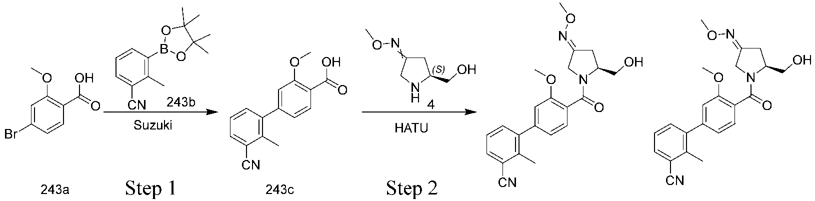

According to the aforementioned synthetic route, with reference to the synthetic method of compound C0102, the more polar product A0243 (6.19 mg, yield: 3.72%) and the less polar product A0245 (4.16 mg, yield: 2.56%) were obtained.

A0243: MS *m*/*z* (ESI): 394.2 [M+1]⁺.

¹H-NMR (400 MHz, Chloroform-d) δ 7.68 (d, *J =* 7.6 Hz, 1H), 7.48 (d, *J =* 6.4 Hz, 1H), 7.38 (dd, *J =* 7.6, 3.6 Hz, 2H), 6.96 (d, *J =* 6.0 Hz, 1H), 6.86 (s, 1H), 4.86-4.73 (m, 1H), 4.18 -3.81 (m, 11H), 3.01-2.97 (m, 1H), 2.68 (d, *J =* 16.8 Hz, 1H), 2.42 (s, 3H).

A0245: MS *m*/*z* (ESI): 394.2 [M+1]⁺.

¹H-NMR (400 MHz, Chloroform-d) δ 7.59 (d, *J =* 7.6 Hz, 1H), 7.36-7.29 (m, 3H), 6.85 (d, *J* = 8.0 Hz, 1H), 6.74 (s, 1H), 4.75-4.71 (m, 1H), 4.64 (d, *J =* 13.6 Hz, 1H), 4.12-3.58 (s, 10H), 2.96-2.87 (m, 1H), 2.65 (dd, *J =* 18.8, 3.6 Hz, 1H), 2.41 (t, *J* = 6.0 Hz, 3H).

### Examples A0246&A0250

### 3-(2-Chloro-4-[(2S,4Z)-2-(hydroxymethyl)-4-(methoxyimino)pyrrolidine-1-carbonyl]phenyl)-2-methylbenzonitrile

### 3-(2-Chloro-4-[(2S,4E)-2-(hydroxymethyl)-4-(methoxyimino)pyrrolidine-1-carbonyl]phenyl)-2-methylbenzonitrile

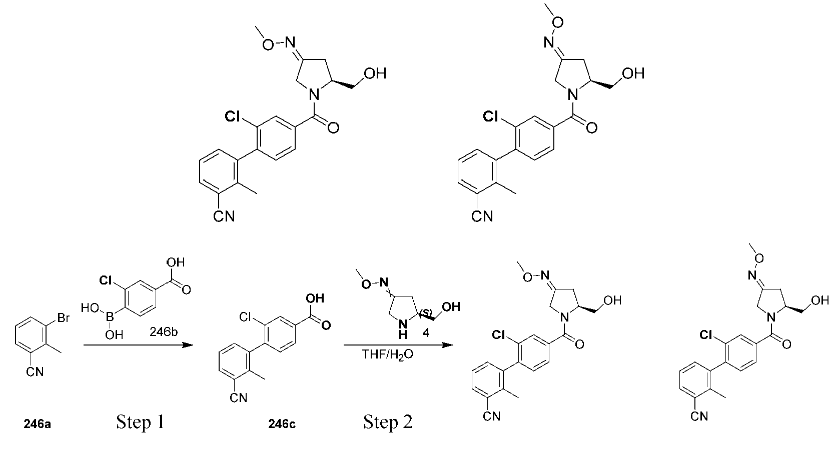

### Step 1

### 3-Chloro-4-(3-cyano-2-methylphenyl)benzoic acid 246c

246a (0.400 g, 2.04 mmol), 246b (0.41 g, 2.04 mmol), Pd(dppf)Cl₂ (0.075 g, 0.10 mmol), and potassium carbonate (0.56 g, 4.08 mmol) were dissolved in 1,4-dioxane (2 mL), and the mixture was reacted at 120°C under argon protection for 16 hours. The reaction solution was added dropwise to 50 mL of 1M hydrochloric acid, extracted with ethyl acetate (50 mL × 3), concentrated, mixed with adsorbent, purified by silica gel column chromatography (DCM/MeOH=85/15), and concentrated to afford yellow product 246c (180 mg, yield: 32.47%).

MS *m*/*z* (ESI): 272.1[M+1]⁺.

### Step 2

### 3-(2-Chloro-4-[(2S,4Z)-2-(hydroxymethyl)-4-(methoxyimino)pyrrolidine-1-carbonyl]phenyl)-2-methylbenzonitrile

### 3-(2-Chloro-4-[(2S,4E)-2-(hydroxymethyl)-4-(methoxyimino)pyrrolidine-1-carbonyl]phenyl)-2-methylbenzonitrile

246c (180 mg, 0.66 mmol) was dissolved in DCM (10 mL), thionyl chloride (0.24 g, 1.98 mmol) was added at 20°C, and the mixture was reacted at 40°C for 2 hours. TLC analysis indicated complete consumption of the substrate and the appearance of a new spot (indicating formation of the acyl chloride). The reaction solution was directly concentrated, and the residue was dissolved in dichloromethane and subjected to rotary evaporation twice. The resulting material was dissolved in anhydrous THF and slowly added dropwise to a pre-prepared alkaline solution of 4 (0.095 g, 0.66 mmol) and sodium bicarbonate (0.44 g, 5.28 mmol) in THF (5 mL) and water (5 mL). After the dropwise addition was completed, the mixture was reacted at 20°C for another 1-2 hours. The reaction solution was directly extracted with ethyl acetate (15 mL × 3), and the organic phases were filtered and concentrated. The crude product was separated by silica gel Prep-TLC (EA:PE=3:1) to afford the less polar isomer A0250 (Rf=0.35) and the more polar isomer A0246 (Rf=0.3). Further purification by preparative HPLC and lyophilization yielded the more polar product A0246 (23.49 mg, yield: 8.61%) and the less polar product A0250 (18.69 mg, yield: 6.88%).

A0246: MS *m*/*z* (ESI): 398.2 [M+1]⁺.

¹H-NMR (400 MHz, Chloroform-*d*) δ 7.71-7.66 (m, 2H), 7.49 (dd, *J =* 8.0, 2.0 Hz, 1H), 7.40-7.37 (m, 2H), 7.29 (d, *J =* 8.0 Hz, 1H), 4.87 (brs, 1H), 4.38-4.23 (m, 2H), 3.91-3.81 (m, 5H), 2.99-2.66 (m, 3H), 2.34 (s, 3H).

A0250: MS *m*/*z* (ESI): 398.2 [M+1]⁺.

¹H-NMR (400 MHz, Chloroform-*d*) δ 7.71-7.66 (m, 2H), 7.50 (d, *J =* 7.6 Hz, 1H), 7.40-7.34 (m, 2H), 7.28 (s, 1H), 4.89 (brs, 1H), 4.40-4.22 (m, 2H), 3.94-3.68 (m, 5H), 3.22 (d, *J =* 34.4 Hz, 1H), 2.97-2.63 (m, 2H), 2.32 (s, 3H).

### Examples A0251&A0252

### (S,Z)-3-(5-(2-(Hydroxymethyl)-4-(methoxyimino)pyrrolidine-1-carbonyl)pyrazin-2-yl)-2-methylbenzonitrile

### (S,E)-3-(5-(2-(Hydroxymethyl)-4-(methoxyimino)pyrrolidine-1-carbonyl)pyrazin-2-yl)-2-methylbenzonitrile

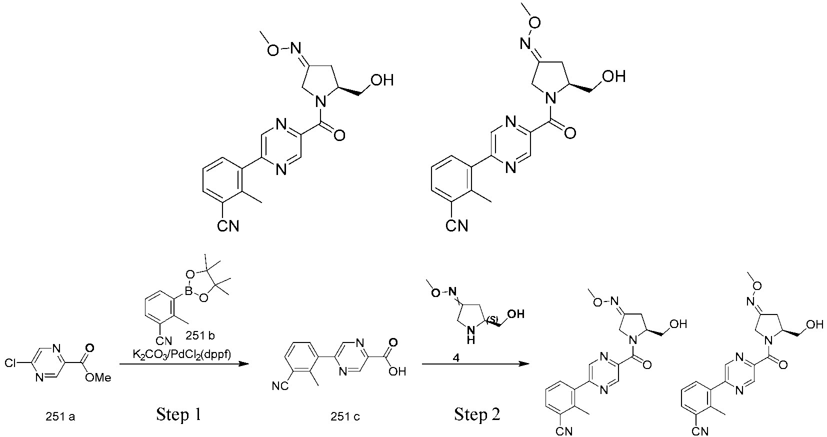

### Step 1

### 5-(3-Cyano-2-methylphenyl)pyrazine-2-carboxylic acid 251c

The starting materials 251a (500 mg, 2.90 mmol) and 251b (0.85 g, 3.48 mmol) were dissolved in 1,4 dioxane (10 mL) and water (2 mL), and then dichloro[1,1-bis(di-tert-butylphosphino)ferrocene]palladium(II) (189.01 mg, 0.29 mmol) and potassium carbonate (1.20 g, 8.7 mmol) were added. After nitrogen purging, the reaction solution was reacted at 100°C for 5 hours. The reaction solution was then extracted with ethyl acetate. The aqueous phase was collected, adjusted to pH=3 with 1M HCl, and extracted with ethyl acetate. The organic phase was collected, washed twice with saturated sodium chloride aqueous solution, dried over anhydrous sodium sulphate, filtered, and concentrated to afford product 251c (330 mg, yield: 47.61%).

MS *m*/*z* (ESI): 240 [M+1]⁺.

### Step 2

### (S,Z)-3-(5-(2-(Hydroxymethyl)-4-(methoxyimino)pyrrolidine-1-carbonyl)pyrazin-2-yl)-2-methylbenzonitrile

### (S,E)-3-(5-(2-(Hydroxymethyl)-4-(methoxyimino)pyrrolidine-1-carbonyl)pyrazin-2-yl)-2-methylbenzonitrile

The starting material 251c (150 mg, 0.63 mmol) was dissolved in dichloromethane (2 mL), and then thionyl chloride (1.5 mL, 20.68 mmol) was added, and the reaction solution was reacted at 40°C for 2 hours. After the reaction was completed, the reaction solution was concentrated to afford a crude product. Subsequently, another starting material 4 (0.14 g, 0.95 mmol) was dissolved in tetrahydrofuran (2 mL), and then a solution of saturated sodium bicarbonate (0.37 g, 4.41 mmol) in water (2 mL) was added, and the mixture was reacted for 10 minutes. The prepared acyl chloride was added dropwise to the reaction solution, and the reaction solution was reacted at room temperature for 2 hours. Then, water was added, and the mixture was extracted with dichloromethane. The organic phases were combined, washed with sodium chloride aqueous solution, dried over anhydrous sodium sulphate, and filtered, and the filtrate was concentrated under reduced pressure to afford a crude product, which was separated and purified by silica gel Prep-TLC (EA:PE=3:1) to afford the less polar isomer A0251 (Rf=0.25) and the more polar isomer A0252 (Rf=0.2). Further purification by preparative HPLC and lyophilization yielded the more polar product A0252 (45.24 mg, yield: 19.08%) and the less polar product A0251 (35.16 mg, yield: 15.08%).

A0251: MS m/z (ESI): 366.2 [M+1]+.

¹H NMR (400 MHz, Chloroform-d) δ 9.30 (d, *J =* 14.8 Hz, 1H), 8.71 - 8.62 (m, 1H), 7.80 - 7.74 (m, 1H), 7.70-7.67 (m, 1H), 7.49-7.46 (m, 1H), 4.95-4.91 (m, 1H), 4.80-4.75 (m, 1H), 3.96 - 3.66 (m, 5H), 2.93-2.67 (m, 2H), 2.62 (s, 3H).

A0252: MS *m*/*z* (ESI): 366.2 [M+1]⁺

¹H NMR (400 MHz, Chloroform-d) δ 9.32 - 9.26 (m, 1H), 8.77 - 8.60 (m, 1H), 7.77 (d, *J =* 9.6 Hz, 1H), 7.70 - 7.64 (m, 1H), 7.46 (t, *J =* 5.6 Hz, 1H), 5.07 -4.75 (m, 2H), 4.01 - 3.64 (m, 5H), 3.28 (s, 1H), 3.23 (s, 1H), 2.93-2.67 (m, 2H), 2.62 (s, 3H).

### Biological test examples

### Test example 1. Determination of the effect of the compound of the present invention on Ca²⁺ mobilization in cells stably expressing OTR

### Experimental objective:

To measure the antagonistic effect of the compound of the present invention on OTR activity expressed in the OTR/HEK293 stably transfected cell line.

### Reagents and consumables:

DMEM medium (Invitrogen, 11965)
F12 medium (Invitrogen, 11765)
Foetal bovine serum (Excell, FSP500)
Geneticin (Invitrogen, 10131)
Penicillin/Streptomycin (Invitrogen, SV30010)
Fluo-4 Direct kit (Invitrogen, F10471)
384-well cell plate (Greiner, 781090)
384-well compound plate (Greiner, 781280)

### Experimental instruments:

FLIPR instrument (Molecular Device)
EHCO instrument (Labcyte)
Cell counter (Beckman, Vi-cell XR)

### Experimental method:

### 1. Cell preparation:

The cultured cells were washed twice with pre-heated DPBS and digested with appropriate amount of trypsin at 37°C for 1 minute, and then appropriate amount of culture medium was added to terminate digestion. The cells were centrifuged at 1000 rpm for 5 minutes. The supernatant was gently discarded without disturbing the cell pellet at the bottom. The cells were resuspended in culture medium and 1 mL was taken out for cell counting and viability assessment using Vi-CELL^{™}. The cells were diluted to 1×10⁶ cells/mL (20,000 cells/20 µL) with culture medium, seeded into a 384-well cell plate and incubated at 37°C with 5% CO₂ for 16-20 h.

### 2. Reagent preparation:

Formulation of 250 mM Probenecid solution: according to the kit instructions, 1 mL of FLIPR buffer salt solution was added to 77 mg of probenecid, prepared fresh just before use. Formulation of Fluo-4 DirectTM (2×, 8 µM) loading buffer: according to the kit instructions, the required amount of Fluo-4 DirectTM was taken out and thawed in advance, 10 mL of FLIPR buffer salt solution and 0.2 mL of 250 mM Probenecid solution were added to each tube, and the mixture was vortexed in the dark for > 5 minutes, prepared fresh just before use.

### 3. Compound testing:

Preparation of agonist reference compound plate: the agonist was diluted 3-fold in 10 points using ECHO, and 750 nL was transferred to the corresponding compound plate using ECHO.

Preparation of reference and test compound plates: the test compound was diluted 3-fold in 10 points with ECHO, and 900 nL was transferred to the corresponding compound plate; the reference compound was diluted 3-fold in 10 points with assay buffer salt solution, and 30 µL was transferred to the compound plate.

30 µL of assay buffer salt solution was added to the corresponding compound plate, and the mixture was centrifuged at 1000 rpm for 1 minute.

The cell plate prepared the day before was taken out of the incubator, and 20 µL of 2×Fluo-4 DirectTM buffer was added to each well, with a total volume of 40 µL.

The plate was incubated in an incubator at 37°C with 5% CO₂ for 50 minutes, and then placed at room temperature for 10 minutes.

The compound plate, cell plate, and pipette tips were placed into the instrument.

For the agonist reference compound plate: the FLIPR instrument software was run, 10 µL of the pre-determined concentration of agonist reference compound was added to the cell plate following the preset protocol, and the fluorescence signal was read. After reading, the data were exported to calculate the EC80 for each cell line, and the agonist at 6 × EC80 concentration was prepared.

For the compound plate: the FLIPR instrument software was run, 10 µL of the pre-determined concentration of test compound and the reference compound were added to the cell plate following the preset protocol, and the fluorescence signal was read. 10 µL of the reference compound agonist at 6 × EC80 concentration was added to the cell plate, and the fluorescence signal was read. For antagonist detection of the compound, the data were exported using the "Max-Min" and "Read 1 to Maximum allowed" methods in the software, and the data were fitted using Prism.

Test example 2. Determination of the effect of the compound of the present invention on Ca²⁺ mobilization in cells stably expressing V1aR

### Experimental objective:

To measure the antagonistic effect of the compound of the present invention on V1aR activity expressed in the V1a-CHO stably transfected cell line.

### Reagents and consumables:

F-12 (Hyclone, SH30026.01)
FLIPR CALCIUM 6 ASSAY KIT (Molecul Device, R8191)
Foetal Bovine Serum (FBS) (AusGeneX, FBS500-S)
Hygromycin B (Solarbio, H8080-1g)
DMSO (Sigma, D4540)
HBSS (Gibco 14025-092)
Probenecid (MCE, HY-BO545)
0.36 mL 96 Round V-bottom Deep Well Plates for Laboratory (Biaosen P-0.36-BSA-96)
384-well conical bottom polypropylene plate (Nunc, 264573)
Corning^{®} 384-well Flat Clear Bottom Black Polystyrene TC-treated Microplates (Corning 3764)

### Experimental instruments:

Biological safety cabinet (ECSO, AC2-6S1-TC)
Carbon dioxide cell incubator (ECSO, CCl-170B-8)
Inverted microscope (Olympus , CKX53)
Microplate low-speed centrifuge (Xiangzhi, TD5B)
FLIPR^{®} Penta (Molecul Device, 5.0)

### Experimental method:

1. Cell plating: V1a-CHO cells were digested and harvested, resuspended and counted, and then inoculated into a 384-well cell plate at a density of: 1.2×10⁴ cells/25 µL/well. The cell plate was then incubated in an incubator at 37°C with 5% CO₂ for about 16-20 h.
2. The next day: after preparing Assay Buffer, 20× Component A was thawed to room temperature, diluted with Assay buffer to 1× loading buffer, and placed at room temperature for later use.
3. The medium was removed from the cell plate, and 40 µL of 1× loading buffer was quickly added to each well. After centrifugation, the cell plate was incubated at 37°C in the dark for 120 minutes.
4. The serially diluted positive compound, serially diluted test compound, and agonist working solution were formulated. After mixing the serially diluted positive compound/serially diluted test compound with the agonist at a 1:1 ratio, 20 µL per well was transferred to a 384-well compound source plate.
5. The cell plate, agonist source plate, and pipette tips were placed into their respective positions in the FLIPR instrument. Using FLIPR Tetra, 10 µL of the diluted agonist from Step 4 was added to each test well, and the data were collected at a wavelength of 515 nm-575 nm.
6. The signal values were plotted against the compound concentrations, and the curve fitting and IC₅₀ calculation were performed using the nonlinear regression method of GraphPad Prism software.

### Experimental results of Test examples 1 and 2:

| Example No. | OTR IC₅₀ (nM) | V1aR IC₅₀ (nM) | Example No. | OTR IC₅₀ (nM) | V1aR IC₅₀ (nM) |
|---|---|---|---|---|---|
| Nolasiban | 70.96 | 389 | C0003 | 2331 | - |
| C0021 | 74.03 | 862 | C0004 | 628.3 | - |
| C0035 | 81.24 | 661 | C0005 | 1793 | - |
| C0036 | 32.14 | 790 | C0006 | 1736 | - |
| C0038 | 13.45 | 1369 | C0007 | 533.9 | - |
| C0041 | 49.31 | 1754 | C0008 | 1008 | - |
| C0044 | 78.48 | 5514 | C0009 | 860.2 | - |
| C0045 | 16.03 | 3498 | C0013 | 853 | - |
| C0046 | 22.13 | 1850 | C0015 | 887.5 | - |
| C0047 | 11.20 | 2399 | C0017 | 140.5 | - |
| A0061 | 99.76 | 2392 | C0018 | 545.4 | - |
| C0056 | 10.17 | 2760 | C0030 | 1825 | - |
| C0058 | 17.69 | 2249 | C0033 | 1900 | - |
| C0060 | 17.91 | 3208 | C0039 | 295.2 | - |
| C0062 | 10.61 | 2742 | C0057 | 82.93 | - |
| C0064 | 11.00 | 584 | C0051 | 114.7 | - |
| C0066 | 72.88 | 2157 | C0071 | 236.9 | - |
| A0134 | 13.12 | 5821 | C0073 | 42.23 | 5026 |
| C0067 | 12.99 | 223 | C0075 | 46.81 | 20028 |
| A0041 | 575.2 | - | C0077 | 672.7 | - |
| A0066 | 197.2 | - | C0079 | 27.73 | 930.2 |
| A0083 | 538.8 | - | C0081 | 129.2 | - |
| A0086 | 237.2 | - | C0083 | 11.13 | 2921 |
| A0087 | 1080 | - | C0085 | 161.2 | - |
| A0112 | 815.3 | - | C0087 | 176.2 | - |
| A0130 | 646.7 | - | C0091 | 279.5 | - |
| A0131 | 171.9 | - | C0093 | 1145 | - |
| A0215 | 58.75 | 11180 | C0095 | 1255 | - |
| A0137 | 152.9 | - | C0097 | 815.4 | - |
| A0144 | 1112 | - | C0098 | 55.89 | 13182 |
| A0147 | 23.5 | - | C0099 | 17.52 | 5285 |
| A0148 | 34.35 | 732.4 | C0103 | 1485 | - |
| A0149 | 17.12 | - | C0109 | 367.1 | - |
| A0153 | 484.6 | - | C0110 | 98.11 | - |
| A0157 | 119 | 6742 | C0111 | 12.58 | 2351 |
| A0158 | 1522 | - | C0112 | 7.515 | 602.4 |
| A0162 | 31.52 | 587.4 | C0113 | 55.32 | 1559 |
| A0163 | 202.4 | - | C0118 | 32.93 | 1140 |
| A0167 | 49.75 | 1481 | C0123 | 16.14 | 9057 |
| A0173 | 36.05 | 8769 | C0127 | 102.2 | - |
| A0179 | 165.3 | - | C0132 | 11.43 | 8371 |
| A0183 | 1484 | - | C0133 | 8.189 | 2350 |
| A0188 | 43.86 | - | C0146 | 228.3 | - |
| A0190 | 1508 | - | A0222 | 111.8 | - |
| A0191 | 16.82 | 1021 | A0249 | 3065 | - |
| A0197 | 402.6 | - | C0162 | 8.349 | 747.2 |
| A0198 | 967.5 | - | A0229 | 122.3 | - |
| A0203 | 20.77 | 1358 | A0232 | 393.7 | - |
| A0204 | 155.8 | - | A0233 | 48.32 | - |
| A0212 | 384.5 | - | A0216 | 198.2 | - |
| A0225 | 483 | - | A0251 | 363.2 | - |
| A0226 | 1046 | - | C0135 | 39.3 | 3907 |
| A0228 | 431.7 | - | C0137 | 7.435 | - |
| A0247 | 25.05 | - | C0139 | 40.55 | - |
| C0163 | 71.97 | - | C0142 | 2940 | - |
| A0248 | 57.76 | - | C0144 | 59.87 | - |
| A0234 | 241.7 | - | C0164 | 20.59 | 1870 |
| A0235 | 23.16 | 1357 | C0148 | 157.7 | - |
| A0240 | 17.32 | - | C0150 | 7.067 | 4049 |
| A0241 | 20.26 | 6132 | C0152 | 6.629 | 9805 |
| A0242 | 10.78 | 1551 | C0154 | 5.315 | 1609 |
| A0243 | 34.07 | 7363 | C0156 | 73.62 | - |
| A0244 | 42.77 | - | C0158 | 17.19 | 519.8 |
| A0245 | 58.11 | - | C0160 | 21.85 | 529.1 |
| A0246 | 24.11 | 1851 | | | |

| | | | | | |
|---|---|---|---|---|---|
| - indicates not determined. | | | | | |

### Experimental conclusion:

As can be seen from the data in the table, the example compounds of the present invention all exhibit antagonistic effects on OTR. For some of the example compounds, both OTR and V1aR activities were tested. These compounds show strong antagonistic effects on OTR and weak antagonistic effects on V1aR, indicating that they have obvious selectivity for OTR.

### Test example 3: Pharmacokinetic determination in SD rats

### Study objective:

In this study, female SD rats were selected as test animals. The LC/MS/MS method was used to quantitatively determine the plasma drug concentrations of the test compounds at different time points after intravenous injection (2 mg/kg) or oral administration (5 mg/kg) in rats, so as to evaluate the pharmacokinetic profile of the test drugs in rats.

### Experimental protocol

### Test drugs

The example compounds of the present invention, made in house.

### Test animals

SD rats (female, 180-220 g, 6-8 weeks old, purchased from Beijing Vital River Laboratory Animal Technology Co., Ltd., with animal production license number SCXK (Beijing) 2021-0006).

### Preparation formulation

Vehicle for intravenous administration: 5% DMSO + 10% Solutol + 85% Saline.

Vehicle for oral administration: 5% DMSO + 95% PEG400.

### Experimental procedure

The test compound, formulated as a clear solution or suspension, was administered to overnight-fasted SD rats via tail vein injection or oral gavage. For intravenous administration, blood samples (0.25 mL) were collected via jugular vein puncture at pre-dose (0 h) and 0.083, 0.25, 0.5, 1, 2, 4, 6, 8, and 24 h post-dose. The samples were placed into sodium heparin anticoagulant tubes, and the mixture was vortexed thoroughly and centrifuged at 6,000 rpm for 3 minutes at 4°C. For oral gavage, blood samples were collected via jugular vein puncture at pre-dose (0 h) and 0.083, 0.25, 1, 2, 4, 6, 8, and 24 h post-dose. The samples were placed into sodium heparin anticoagulant tubes, and the mixture was vortexed thoroughly and centrifuged at 6,000 rpm for 3 minutes. Plasma drug concentrations were determined using the LC-MS/MS method, and the relevant pharmacokinetic parameters were calculated using the non-compartmental model with linear-log trapezoidal method in Phoenix WinNonlin 8.2.0 pharmacokinetic software.

### Experimental results

The results of the pharmacokinetic experiment in SD rats are shown in the table below:

| Example | Mode of administration | Area under the curve AUC₀₋ₜ (h*ng/mL) | Half-life T_{1/2} (h) | Clearance Cl (mL/kg/min) |
|---|---|---|---|---|
| Nolasiban | IV (2 mpk) | 3051 | 2.8 | 11.1 |
| | PO (5 mpk) | 7705 | 2.71 | - |
| C0058 | IV (2 mpk) | 4638 | 3 | 7.4 |
| | PO (5 mpk) | 11426 | 3.11 | - |
| A0203 | IV (2 mpk) | 7156 | 5.23 | 4.66 |
| | PO (5 mpk) | 16035 | 5.97 | - |

| | | | | |
|---|---|---|---|---|
| - indicates not determined. | | | | |

### Experimental conclusion

As can be seen from the experimental results, the example compounds of the present invention exhibit favourable metabolic properties, with good performance in terms of exposure (AUC), half-life (T_{1/2}), and clearance (Cl).

### Test example 4: Blood-brain distribution determination in SD rats

### Study objective:

In this study, female SD rats were selected as test animals. The LC/MS/MS method was used to quantitatively determine the drug concentrations of the test compounds in plasma and brain tissues at three different time points (0.5 h, 2 h, 4 h or 6 h) after oral administration (5 mg/kg or 10 mg/kg) in rats, so as to evaluate the pharmacokinetic profile of the test drugs in rats.

### Experimental protocol

### Test drugs

The example compounds of the present invention, made in house.

### Test animals

SD rats (female, 180-220 g, 6-8 weeks old, purchased from Beijing Vital River Laboratory Animal Technology Co., Ltd., with animal production license number SCXK (Beijing) 2021-0006).

### Preparation formulation

Vehicle for oral administration: 5% DMSO + 95% PEG400 or a mixture of (50% Labrasol+50% Water).

### Experimental procedure

The test compound, formulated as a clear solution or suspension, was administered to overnight-fasted SD rats via oral gavage. For oral gavage, blood samples (0.25 mL) were collected via jugular vein puncture at pre-dose (0 h) and 0.5 h, 2 h, 4 h or 6 h post-dose. The samples were placed into sodium heparin anticoagulant tubes. After collection, the blood samples were placed on wet ice and centrifuged within 1 hour to separate the plasma. The separated plasma was stored frozen in a -80°C freezer. After blood collection, the animals were euthanized by cervical dislocation. The whole brain tissue was dissected out. The collected brain tissue was first rinsed with physiological saline, blotted dry with absorbent paper, and then stored frozen in a -80°C freezer. Prior to analysis, the brain tissue samples and plasma samples were taken out from the -80°C freezer. After the brain tissue samples were completely thawed, a given amount of brain tissue was weighed, and 50% methanol/water was added for homogenization. Drug concentrations in plasma and brain tissues were determined using the LC-MS/MS method, and the relevant pharmacokinetic parameters were calculated using the non-compartmental model with linear-log trapezoidal method in Phoenix WinNonlin 8.2.0 pharmacokinetic software.

### Experimental results

The results of the pharmacokinetic experiment in SD rats are shown in the table below:

| **Example** | **Administration dose** | **Plasma area under the curve AUC₀₋ₜ (h*ng/mL)** | **Brain tissue area under the curve AUC₀₋ₜ (h*ng/g)** | **Brain/plasma exposure ratio** |
|---|---|---|---|---|
| Nolasiban | 10 mg/kg | 5695 | 18727 | 3.29 |
| C0058 | 5 mg/kg | 3516 | 1109 | 0.32 |
| C0099 | 5 mg/kg | 1455 | 150 | 0.10 |
| C0112 | 5 mg/kg | 1566 | 818 | 0.52 |
| C0152 | 5 mg/kg | 2051 | 169 | 0.08 |
| A0203 | 5 mg/kg | 6666 | 2579 | 0.39 |

### Experimental conclusion

As can be seen from the experimental results, the example compounds of the present invention exhibit a low level of drug concentration in brain tissues and a low proportion of distribution in brain tissues after oral administration, thus having a low risk of off-target effects caused by OTR antagonism in the brain.

## Claims

1. A compound as shown in formula I: 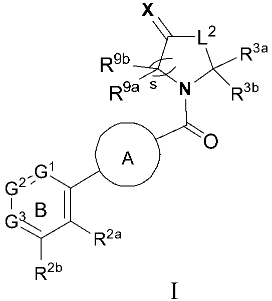
or an isotopic derivative thereof or a pharmaceutically acceptable salt of any of the foregoing;
**characterized in that** X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl, -OR^{5a}, -SR^{5a}, -N(R^{5a})(R^{5b}), -C(O)R^{5a}, -OC(O)R^{5a}, -C(O)OR^{5a}, -C(O)N(R^{5a})(R^{5b}), -N(R^{5c})C(O)R^{5a}, -N(R^{5c})C(O)N(R^{5a})(R^{5b}), -S(O)₂R^{5a}, -N(R^{5c})S(O)₂R^{5a}, - S(O)₂N(R^{5a})(R^{5b}), -Si(R^{5a})₃, -Si(R^{5a})₂(OR^{5b}), -OSi(R^{5a})₃, -Si(R^{5a})(OR^{5b})₂, - OP(O)(OR^{5a})(OR^{5b}), -P(O)(OR^{5a})(OR^{5b}), -OP(O)(OR^{5a})(R^{5b}), -P(O)(OR^{5a})(R^{5b}), _ OP(O)(R^{5a})(R^{5b}) or -P(O)(R^{5a})(R^{5b});
R^{3a} is hydrogen, halogen, cyano, nitro, C₁₋₆ alkyl, C₁₋₆ haloalkyl, C₃₋₈ cycloalkyl, 3- to 8-membered heterocycloalkyl, phenyl, 5- to 6-membered heteroaryl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl or -L¹-R^{3c};
R^{3b} is hydrogen, halogen, cyano, nitro, C₁₋₆ alkyl, C₁₋₆ haloalkyl, C₂₋₆ alkenyl, halogenated C₂₋₆ alkenyl, C₂₋₆ alkynyl, halogenated C₂₋₆ alkynyl, -L¹-R^{3c}, - C(O)OR^{3d}, -C(O)N(R^{3d})(R^{3e}), -S(O)₂R^{3d}, -S(O)₂N(R^{3d})(R^{3e}), -P(O)(OR^{3d})(OR^{3e}), -P(O)(OR^{3d})(R^{3e}), -P(O)(R^{3d})(R^{3e}), C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl, and the C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl optionally forms a fused ring with additional C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl; wherein the C₃₋₈ cycloalkyl, 3- to 8-membered heterocycloalkyl, C₃₋₈ cycloalkenyl, 3- to 8-membered heterocycloalkenyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
alternatively, R^{3a} and R^{3b}, together with the carbon atom to which they are attached, form C₃₋₈ cycloalkyl, 3- to 8-membered heterocycloalkyl, C₃₋₈ cycloalkenyl or 3- to 8-membered heterocycloalkenyl, and the C₃₋₈ cycloalkyl, 3- to 8-membered heterocycloalkyl, C₃₋₈ cycloalkenyl and 3- to 8-membered heterocycloalkenyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
each L¹ is independently -[C(R^{a})(R^{b})]ₙ-; wherein n is 1, 2 or 3; R^{a} and R^{b} are each independently hydrogen, C₁₋₆ alkyl, C₃₋₈ cycloalkyl or 3- to 8-membered heterocycloalkyl; alternatively, R^{a} and R^{b}, together with the carbon atom to which they are attached, form C₃₋₈ cycloalkyl or 3- to 8-membered heterocycloalkyl; wherein the C₁₋₆ alkyl, C₃₋₈ cycloalkyl and 3- to 8-membered heterocycloalkyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
each R^{3c} is independently -OR^{7a}, -SR^{7a}, -N(R^{7a})(R^{7b}), -C(O)R^{7a}, -OC(O)R^{7a}, -C(O)OR^{7a}, -C(O)N(R^{7a})(R^{7b}), -N(R^{7c})C(O)R^{7a}, -N(R^{7c})C(O)N(R^{7a})(R^{7b}), - S(O)₂R^{7a}, -N(R^{7c})S(O)₂R^{7a}, -S(O)₂N(R^{7a})(R^{7b}), -Si(R^{7a})₃, -Si(R^{7a})₂(OR^{7b}), - OSi(R^{7a})₃, -Si(R^{7a})(OR^{7b})₂, -OP(O)(OR^{7a})(OR^{7b}), -P(O)(OR^{7a})(OR^{7b}), - OP(O)(OR^{7a})(R^{7b}), -P(O)(OR^{7a})(R^{7b}), -OP(O)(R^{7a})(R^{7b}) or -P(O)(R^{7a})(R^{7b});
L² is -[C(R^{10a})(R^{10b})]ₜ-, wherein one C(R^{10a})(R^{10b}) moiety is optionally replaced by -O- or -N(R^{10a})-;
s is 1 and t is 1; alternatively, s is 2 and t is 1; alternatively, s is 1 and t is 2;
R^{9a} and R^{9b} are each independently hydrogen, halogen, cyano, nitro, C₁₋₆ alkyl, C₁₋₆ haloalkyl, C₁₋₆ hydroxyalkyl, C₂₋₆ alkenyl, halogenated C₂₋₆ alkenyl, C₂₋₆ alkynyl or halogenated C₂₋₆ alkynyl; alternatively, R^{9a} and R^{9b}, together with the atom to which they are attached, form C₃₋₈ cycloalkyl, 3- to 8-membered heterocycloalkyl, C₃₋₈ cycloalkenyl or 3- to 8-membered heterocycloalkenyl; alternatively, R^{9a} and R^{3a} are joined to form -CH₂- or -CH₂CH₂-;
R^{10a} and R^{10b} are each independently hydrogen, halogen, cyano, C₁₋₆ alkyl, C₁₋₆ haloalkyl, C₁₋₆ hydroxyalkyl, C₂₋₆ alkenyl, halogenated C₂₋₆ alkenyl, C₂₋₆ alkynyl or halogenated C₂₋₆ alkynyl; alternatively, R^{10a} and R^{10b}, together with the carbon atom to which they are attached, form C₃₋₈ cycloalkyl, 3- to 8-membered heterocycloalkyl, C₃₋₈ cycloalkenyl or 3- to 8-membered heterocycloalkenyl; alternatively, R^{10a} and R^{3a}, together with the atom to which they are attached, form C₃₋₈ cycloalkyl, 3- to 8-membered heterocycloalkyl, C₃₋₈ cycloalkenyl or 3- to 8-membered heterocycloalkenyl;
ring A is 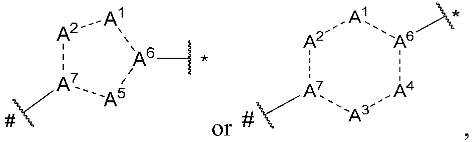
wherein the * end is connected to the carbonyl group in formula I, and the # end is connected to ring B in formula I; each ----bond is independently a single bond or a double bond;
each A¹ is independently CH, C(O), N, NH, O, S, N(R^{4a}) or C(R^{4a});
each A² is independently CH, C(O), N, NH, O, S, N(R^{4b}) or C(R^{4b});
A³ is CH, C(O), N, N(R^{4c}) or C(R^{4c});
each A⁴ is independently CH, C(O), N, N(R^{4d}) or C(R^{4d});
A⁵ is CH, N, O, S, NH, C(R^{4d}) or N(R^{4d});
each A⁶ is independently C or N;
each A⁷ is independently C or N;
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, -N(R^{8c})C(O)N(R^{8a})(R^{8b}), - S(O)₂R^{8a}, -N(R^{8c})S(O)₂R^{8a}, -S(O)₂N(R^{8a})(R^{8b}), -Si(R^{8a})₃, -Si(R^{8a})₂(OR^{8b}), - OSi(R^{8a})₃, -Si(R^{8a})(OR^{8b})₂, -OP(O)(OR^{8a})(OR^{8b}), -P(O)(OR^{8a})(OR^{8b}), - OP(O)(OR^{8a})(R^{8b}), -P(O)(OR^{8a})(R^{8b}), -OP(O)(R^{8a})(R^{8b}) or -P(O)(R^{8a})(R^{8b});
alternatively, R^{4a} and R^{4b}, together with the atom to which they are attached, form C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl; the C₃₋₈ cycloalkyl, 3- to 8-membered heterocycloalkyl, C₃₋₈ cycloalkenyl, 3- to 8-membered heterocycloalkenyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
in ring B, G¹, G² and G³ are each independently N or C(R^{2c});
R^{2a}, R^{2b} and R^{2c} are each independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl, -OR^{6a}, -SR^{6a}, -N(R^{6a})(R^{6b}), -C(O)R^{6a}, -OC(O)R^{6a}, -C(O)OR^{6a}, -C(O)N(R^{6a})(R^{6b}), -N(R^{6c})C(O)R^{6a}, -N(R^{6c})C(O)N(R^{6a})(R^{6b}), - S(O)₂R^{6a}, -N(R^{6c})S(O)₂R^{6a}, -S(O)₂N(R^{6a})(R^{6b}), -Si(R^{6a})₃, -Si(R^{6a})₂(OR^{6b}), - OSi(R^{6a})₃, -Si(R^{6a})(OR^{6b})₂, -OP(O)(OR^{6a})(OR^{6b}), -P(O)(OR^{6a})(OR^{6b}), - OP(O)(OR^{6a})(R^{6b}), -P(O)(OR^{6a})(R^{6b}), -OP(O)(R^{6a})(R^{6b}) or -P(O)(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl; the C₃₋₈ cycloalkyl, 3- to 8-membered heterocycloalkyl, C₃₋₈ cycloalkenyl, 3- to 8-membered heterocycloalkenyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
alternatively, R^{2a} and R^{4b} are joined to form -(CH₂)ₚ-, wherein p is 2, 3 or 4, and wherein one or two CH₂ moieties are optionally replaced by -O- or -NH-;
R^{3d}, R^{3e}, R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl, and the C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl optionally forms a fused ring with additional C₃₋₈ cycloalkyl, 3- to 8-membered heterocycloalkyl, C₃₋₈ cycloalkenyl, 3- to 8-membered heterocycloalkenyl, phenyl or 5- to 6-membered heteroaryl; wherein the C₁₋₆ alkyl, C₃₋₈ cycloalkyl, 3- to 8-membered heterocycloalkyl, C₃₋₈ cycloalkenyl, 3- to 8-membered heterocycloalkenyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₃₋₈ cycloalkyl, 3- to 8-membered heterocycloalkyl, C₂₋₆ alkenyl, halogenated C₂₋₆ alkenyl, C₂₋₆ alkynyl, halogenated C₂₋₆ alkynyl, C₃₋₈ cycloalkenyl, 3- to 8-membered heterocycloalkenyl, phenyl and 5- to 6-membered heteroaryl;
R^{5c}, R^{6c}, R^{7c} and R^{8c} are each independently hydrogen, C₁₋₆ alkyl, C₁₋₆ haloalkyl, C₁₋₆ hydroxyalkyl, C₂₋₆ alkenyl, halogenated C₂₋₆ alkenyl, C₂₋₆ alkynyl or halogenated C₂₋₆ alkynyl;
in the aforementioned heterocycloalkyl, heterocycloalkenyl and heteroaryl, the number of heteroatoms is independently 1, 2, 3 or 4, and each heteroatom is independently N, O, S, Si, P and Se;
provided that at least one of the following conditions (a), (b), (c), (d), and (e) is satisfied:
condition (a): neither R^{2a} nor R^{2b} is hydrogen; moreover, when R^{2a} is C₁₋₆ alkyl, R^{3a} is hydrogen and R^{3b} is a group other than -L¹-R^{3c}, then R^{2b} is not C₁₋₆ alkyl;
condition (b): R^{3a} is C₁₋₆ alkyl or -L¹-R^{3c} and R^{3b} is -L¹-R^{3c}; alternatively, R^{3a} and R^{3b}, together with the carbon atom to which they are attached, form C₃₋₈ cycloalkyl, 3- to 8-membered heterocycloalkyl, C₃₋₈ cycloalkenyl or 3- to 8-membered heterocycloalkenyl, and the C₃₋₈ cycloalkyl, 3- to 8-membered heterocycloalkyl, C₃₋₈ cycloalkenyl and 3- to 8-membered heterocycloalkenyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₂₋₆ alkenyl, halogenated C₂₋₆ alkenyl, C₂₋₆ alkynyl and halogenated C₂₋₆ alkynyl;
condition (c): R^{3a} is hydrogen, C₁₋₆ alkyl or -L¹-R^{3c}; R^{3b} is -L¹-R^{3c}; ring A is 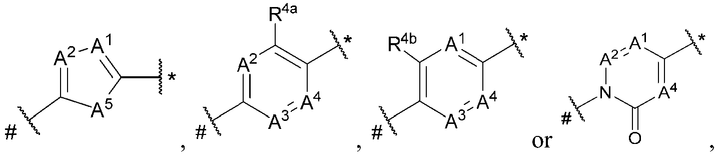 wherein the * end is connected to the carbonyl group in formula I, and the # end is connected to ring B in formula I; moreover, R^{2a} is not hydrogen; wherein each A¹ is independently CH, N or C(R^{4a}); each A² is independently CH, N or C(R^{4b}); each A³ is independently CH, N or C(R^{4c}); each A⁴ is independently CH, N or C(R^{4d}); A⁵ is O, S, NH or N(R^{4d});
condition (d): R^{3b} is -CH(C₁₋₆ alkyl)-OH, 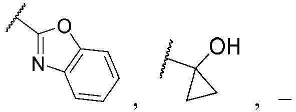 CH₂NHC(O)CH₂OH or -CH₂OCH₂CH₂OH;
condition (e): R^{9a} and R^{3a} are joined to form -CH₂- or -CH₂CH₂-.

2. The compound, or the isotopic derivative or the pharmaceutically acceptable salt according to claim 1, **characterized in that** the compound has a structure as shown in formula I-1: 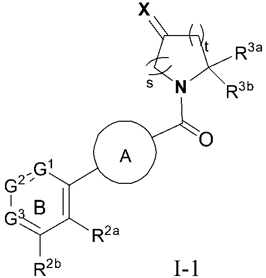
wherein s is 1 and t is 1; alternatively, s is 2 and t is 1; alternatively, s is 1 and t is 2;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{5a}, - C(O)OR^{5a}, -C(O)N(R^{5a})(R^{5b}), -N(R^{5c})C(O)R^{5a}, -N(R^{5c})C(O)N(R^{5a})(R^{5b}), _ S(O)₂R^{5a}, -N(R^{5c})S(O)₂R^{5a} or -S(O)₂N(R^{5a})(R^{5b});
R^{3a} is hydrogen, C₁₋₆ alkyl or -L¹-R^{3c};
R^{3b} is -L¹-R^{3c}, -C(O)OR^{3d}, -C(O)N(R^{3d})(R^{3e}), -S(O)₂R^{3d}, -S(O)₂N(R^{3d})(R^{3e}), C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5-to 6-membered heteroaryl optionally forms a fused ring with additional C₃₋₈ cycloalkyl, 3- to 8-membered heterocycloalkyl, phenyl or 5- to 6-membered heteroaryl; wherein the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
each L¹ is independently -[C(R^{a})(R^{b})]ₙ-; wherein n is 1, 2 or 3; R^{a} and R^{b} are each independently hydrogen, C₁₋₆ alkyl, C₃₋₈ cycloalkyl or 3- to 8-membered heterocycloalkyl; alternatively, R^{a} and R^{b}, together with the carbon atom to which they are attached, form C₃₋₈ cycloalkyl or 3- to 8-membered heterocycloalkyl; wherein the C₁₋₆ alkyl, C₃₋₈ cycloalkyl and 3- to 8-membered heterocycloalkyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
each R^{3c} is independently -OR^{7a}, -SR^{7a}, -N(R^{7a})(R^{7b}), -C(O)R^{7a}, -OC(O)R^{7a}, -C(O)OR^{7a}, -C(O)N(R^{7a})(R^{7b}), -N(R^{7c})C(O)R^{7a}, -N(R^{7c})C(O)N(R^{7a})(R^{7b}), - S(O)₂R^{7a}, -N(R^{7c})S(O)₂R^{7a} or -S(O)₂N(R^{7a})(R^{7b});
ring A is 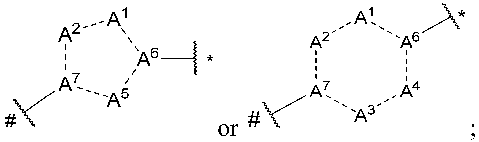
wherein the * end is connected to the carbonyl group in formula I, and the # end is connected to ring B in formula I; each ----bond is independently a single bond or a double bond;
each A¹ is independently CH, C(O), N, NH, O, S, N(R^{4a}) or C(R^{4a});
each A² is independently CH, C(O), N, NH, O, S, N(R^{4b}) or C(R^{4b});
A³ is CH, C(O), N, N(R^{4c}) or C(R^{4c});
each A⁴ is independently CH, C(O), N, N(R^{4d}) or C(R^{4d});
A⁵ is CH, N, O, S, NH, C(R^{4d}) or N(R^{4d});
each A⁶ is independently C or N;
each A⁷ is independently C or N;
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R⁸⁶), -N(R^{8c})C(O)R^{8a}, - N(R^{8c})C(O)N(R^{8a})(R^{8b}), -S(O)₂R^{8a}, -N(R^{8c})S(O)₂R^{8a} or -S(O)₂N(R^{8a})(R^{8b});
alternatively, R^{4a} and R^{4b}, together with the atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
in ring B, G¹, G² and G³ are each independently N or C(R^{2c});
each R^{2c} is independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
R^{2a} and R^{2b} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
alternatively, R^{2a} and R^{4b} are joined to form -(CH₂)ₚ-, wherein p is 2, 3 or 4, and wherein one or two CH₂ moieties are optionally replaced by -O- or -NH-;
provided that, when R^{2a} is C₁₋₆ alkyl, R^{3a} is hydrogen and R^{3b} is a group other than -L¹-R^{3c}, then R^{2b} is not C₁₋₆ alkyl;
R^{3d}, R^{3e}, R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₃₋₈ cycloalkyl, 3- to 8-membered heterocycloalkyl, phenyl and 5- to 6-membered heteroaryl;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S.

3. The compound, or the isotopic derivative or the pharmaceutically acceptable salt according to claim 1 or 2, **characterized in that** the compound has a structure as shown in formula I-1: 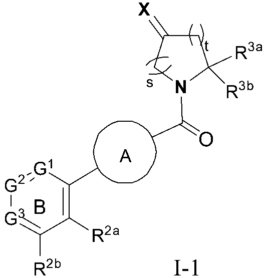
wherein s is 1 and t is 1; alternatively, s is 2 and t is 1; alternatively, s is 1 and t is 2;
X is N-OR¹;
R¹ is hydrogen or C₁₋₆ alkyl, and the C₁₋₆ alkyl is optionally substituted with one or more R^{1a};
each R^{1a} is independently halogen, cyano, nitro, oxo, C₁₋₆ alkyl, C₁₋₆ haloalkyl, C₁₋₆ hydroxyalkyl, C₃₋₈ cycloalkyl, 3- to 8-membered heterocycloalkyl, phenyl, 5-to 6-membered heteroaryl, -OR^{5a}, -SR^{5a}, -N(R^{5a})(R^{5b}), -C(O)R^{5a}, -OC(O)R^{sa}, - C(O)OR^{5a}, -C(O)N(R^{5a})(R^{5b}), -N(R^{5c})C(O)R^{5a}, -N(R^{5c})C(O)N(R^{5a})(R^{5b}), - S(O)₂R^{5a}, -N(R^{5c})S(O)₂R^{5a} or -S(O)₂N(R^{5a})(R^{5b});
R^{3a} is hydrogen, C₁₋₆ alkyl or -L¹-R^{3c};
R^{3b} is -L¹-R^{3c};
each L¹ is independently -[C(R^{a})(R^{b})]ₙ-; wherein n is 1, 2 or 3; R^{a} and R^{b} are each independently hydrogen, C₁₋₆ alkyl, C₃₋₈ cycloalkyl or 3- to 8-membered heterocycloalkyl; alternatively, R^{a} and R^{b}, together with the carbon atom to which they are attached, form C₃₋₈ cycloalkyl or 3- to 8-membered heterocycloalkyl; wherein the C₁₋₆ alkyl, C₃₋₈ cycloalkyl and 3- to 8-membered heterocycloalkyl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
each R^{3c} is independently -OR^{7a}, -SR^{7a}, -N(R^{7a})(R^{7b}), -C(O)R^{7a}, -OC(O)R^{7a}, -C(O)OR^{7a}, -C(O)N(R^{7a})(R^{7b}), -N(R^{7c})C(O)R^{7a}, -N(R^{7c})C(O)N(R^{7a})(R^{7b}), - S(O)₂R^{7a}, -N(R^{7c})S(O)₂R^{7a} or -S(O)₂N(R^{7a})(R^{7b});
ring A is 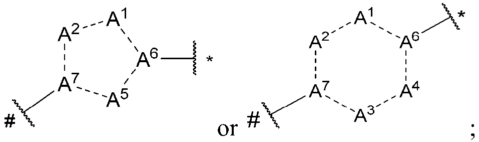
wherein the * end is connected to the carbonyl group in formula I, and the # end is connected to ring B in formula I; each ----bond is independently a single bond or a double bond;
each A¹ is independently CH, C(O), N, NH, O, S, N(R^{4a}) or C(R^{4a});
each A² is independently CH, C(O), N, NH, O, S, N(R^{4b}) or C(R^{4b});
A³ is CH, C(O), N, N(R^{4c}) or C(R^{4c});
each A⁴ is independently CH, C(O), N, N(R^{4d}) or C(R^{4d});
A⁵ is CH, N, O, S, NH, C(R^{4d}) or N(R^{4d});
each A⁶ is independently C or N;
each A⁷ is independently C or N;
R^{4a}, R^{4b}, R^{4c} and R^{4d} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{8a}, -SR^{8a}, -N(R^{8a})(R^{8b}), -C(O)R^{8a}, -OC(O)R^{8a}, -C(O)OR^{8a}, -C(O)N(R^{8a})(R^{8b}), -N(R^{8c})C(O)R^{8a}, - N(R^{8c})C(O)N(R^{8a})(R^{8b}), -S(O)₂R^{8a}, -N(R^{8c})S(O)₂R^{8a} or -S(O)₂N(R^{8a})(R^{8b});
alternatively, R^{4a} and R^{4b}, together with the atom to which they are attached, form C₃₋₈ cycloalkyl, 3- to 8-membered heterocycloalkyl, phenyl or 5- to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
in ring B, G¹, G² and G³ are each independently N or C(R^{2c});
each R^{2c} is independently hydrogen, halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
R^{2a} and R^{2b} are each independently halogen, cyano, nitro, C₁₋₆ alkyl, C₁₋₆ haloalkyl, C₁₋₆ hydroxyalkyl, C₃₋₈ cycloalkyl, 3- to 8-membered heterocycloalkyl, phenyl, 5- to 6-membered heteroaryl, -OR^{6a}, -SR^{6a}, -N(R^{6a})(R^{6b}), -C(O)R^{6a}, - OC(O)R^{6a}, -C(O)OR^{6a}, -C(O)N(R^{6a})(R^{6b}), -N(R^{6c})C(O)R^{6a}, - N(R^{6c})C(O)N(R^{6a})(R^{6b}), -S(O)₂R^{6a}, -N(R^{6c})S(O)₂R^{6a} or -S(O)₂N(R^{6a})(R^{6b});
alternatively, R^{2b} and R^{2a} or R^{2c}, together with the carbon atom to which they are attached, form C₃₋₈ cycloalkyl, 3- to 8-membered heterocycloalkyl, phenyl or 5-to 6-membered heteroaryl; the C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy;
alternatively, R^{2a} and R^{4b} are joined to form -(CH₂)ₚ-, wherein p is 2, 3 or 4, and wherein one or two CH₂ moieties are optionally replaced by -O- or -NH-;
R^{5a}, R^{5b}, R^{6a}, R^{6b}, R^{7a}, R^{7b}, R^{8a} and R^{8b} are each independently hydrogen, C₁₋₆ alkyl, C₃₋₈ cycloalkyl, 3- to 8-membered heterocycloalkyl, phenyl or 5- to 6-membered heteroaryl, and the C₃₋₈ cycloalkyl, 3- to 8-membered heterocycloalkyl, phenyl or 5- to 6-membered heteroaryl optionally forms a fused ring with additional C₃₋₈ cycloalkyl, 3- to 8-membered heterocycloalkyl, phenyl or 5- to 6-membered heteroaryl; wherein the C₁₋₆ alkyl, C₃₋₈ cycloalkyl, 3- to 8-membered heterocycloalkyl, phenyl and 5- to 6-membered heteroaryl are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy, halogenated C₁₋₆ alkoxy, C₃₋₈ cycloalkyl, 3- to 8-membered heterocycloalkyl, phenyl and 5- to 6-membered heteroaryl;
R^{5c}, R^{6c}, R^{7c} and R^{8c} are each independently hydrogen, C₁₋₆ alkyl, C₁₋₆ haloalkyl or C₁₋₆ hydroxyalkyl;
in the aforementioned heterocycloalkyl and heteroaryl, the number of heteroatoms is independently 1, 2, 3 or 4, and each heteroatom is independently N, O and S.

4. The compound, or the isotopic derivative or the pharmaceutically acceptable salt according to any one of claims 1 to 3, **characterized in that** R^{2b} is C₁₋₆ alkyl, cyano, C₁₋₆ haloalkyl, C₁₋₆ alkoxy or halogen; preferably methyl, cyano, monofluoromethyl, difluoromethyl, trifluoromethyl, methoxy or chlorine.

5. The compound, or the isotopic derivative or the pharmaceutically acceptable salt according to any one of claims 1 to 4, **characterized in that** R^{2a} is halogen, cyano, C₁₋₆ alkyl or C₁₋₆ haloalkyl; preferably methyl, monofluoromethyl, difluoromethyl, trifluoromethyl, chlorine or cyano.

6. The compound, or the isotopic derivative or the pharmaceutically acceptable salt according to any one of claims 1 to 3, **characterized in that** R^{2b} and R^{2a}, together with the carbon atom to which they are attached, form C₃₋₈ cycloalkyl, preferably cyclopentyl.

7. The compound, or the isotopic derivative or the pharmaceutically acceptable salt according to any one of claims 1 to 3, **characterized in that** R^{2b} and R^{2a} are defined as any of the following combinations (i) - (x);
(i) R^{2a} is methyl; R^{2b} is methyl;
(ii) R^{2a} is methyl; R^{2b} is chlorine;
(iii) R^{2a} is methyl; R^{2b} is cyano;
(iv) R^{2a} is methyl; R^{2b} is difluoromethyl;
(v) R^{2a} is methyl; R^{2b} is trifluoromethyl;
(vi) R^{2a} is methyl; R^{2b} is methoxy;
(vii) R^{2a} is chlorine; R^{2b} is cyano;
(viii) R^{2a} is cyano; R^{2b} is methyl;
(ix) R^{2a} is difluoromethyl; R^{2b} is cyano;
(x) R^{2a} is trifluoromethyl; R^{2b} is cyano.

8. The compound, or the isotopic derivative or the pharmaceutically acceptable salt according to any one of claims 1 to 7, **characterized in that** G¹ is CH or C(F); G² is CH or C(F); G³ is CH, C(F), C(CN) or N; preferably, G¹ is CH; G² is CH; G³ is CH.

9. The compound, or the isotopic derivative or the pharmaceutically acceptable salt according to any one of claims 1 to 3, **characterized in that** 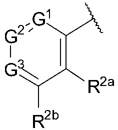 has a structural moiety of

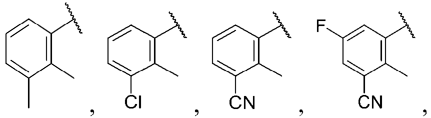

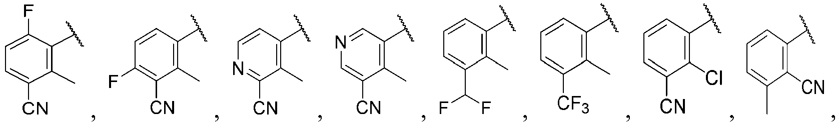

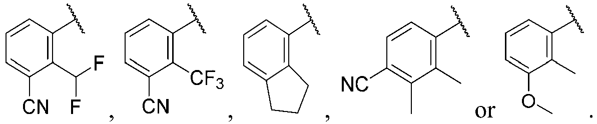

10. The compound, or the isotopic derivative or the pharmaceutically acceptable salt according to any one of claims 1 to 9, **characterized in that** ring A is 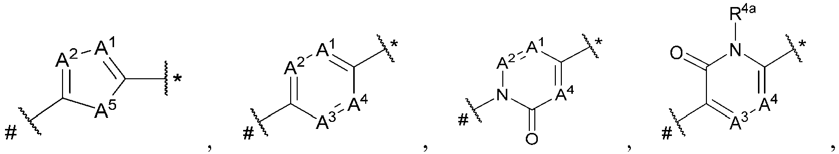 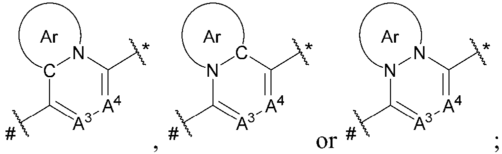 A⁵ is CH, N or C(R^{4a}); A² is CH, N or C(R^{4b}); A³ is CH, N or C(R^{4c}); A⁵ is CH, N or C(R^{4d}); A⁵ is O, S, NH or N(R^{4d}); Ar is 3- to 8-membered heterocycloalkyl or 5- to 6-membered heteroaryl.

11. The compound, or the isotopic derivative or the pharmaceutically acceptable salt according to any one of claims 1 to 9, **characterized in that** ring A is

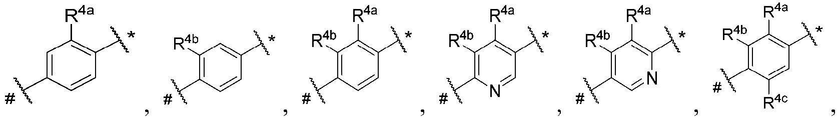

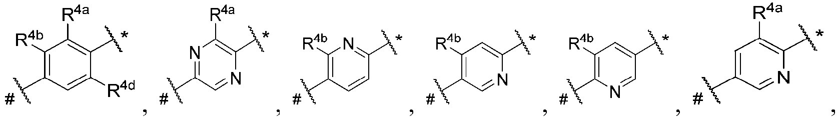

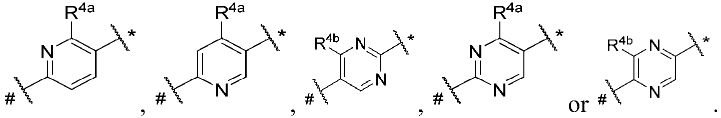

12. The compound, or the isotopic derivative or the pharmaceutically acceptable salt according to any one of claims 1 to 11, **characterized in that** R^{4a} and R^{4b} are each independently halogen, cyano, C₁₋₆ alkyl, C₁₋₆ haloalkyl, C₁₋₆ alkoxy, -OR^{8a}, -SR^{8a}, -N(R^{8a})(R^{8b}), -C(O)R^{8a}, -C(O)OR^{8a} or -C(O)N(R^{8a})(R^{8b}); preferably, R^{4a} and R^{4b} are each independently methyl, monofluoromethyl, difluoromethyl, trifluoromethyl, methoxy, -OCH₂CH₂OH, -OCH₂CH₂OCH₃, -NHCH₂CH₂OCH₃, 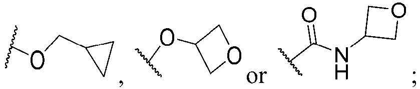 alternatively, R^{4a} and R^{4b}, together with the carbon atom to which they are attached, form 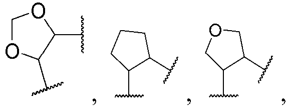 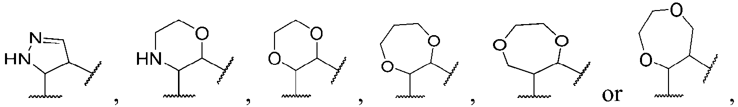 and the aforementioned rings are optionally substituted with one or more substituents independently selected from halogen, hydroxyl, amino, cyano, oxo, C₁₋₆ alkyl, C₁₋₆ haloalkyl, C₁₋₆ hydroxyalkyl, C₁₋₆ alkoxy and halogenated C₁₋₆ alkoxy.

13. The compound, or the isotopic derivative or the pharmaceutically acceptable salt according to any one of claims 1 to 12, **characterized in that** R^{4c} and R^{4d} are each independently halogen, cyano, C₁₋₆ alkyl, C₁₋₆ haloalkyl or C₁₋₆ alkoxy; preferably, R^{4c} and R^{4d} are each independently fluorine, chlorine, methyl, monofluoromethyl, difluoromethyl, trifluoromethyl or methoxy.

14. The compound, or the isotopic derivative or the pharmaceutically acceptable salt according to any one of claims 1 to 12, **characterized in that** ring A is 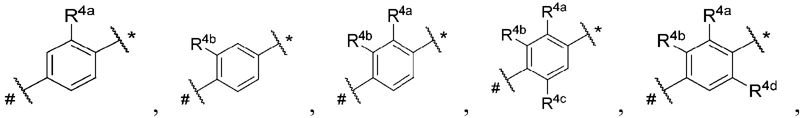 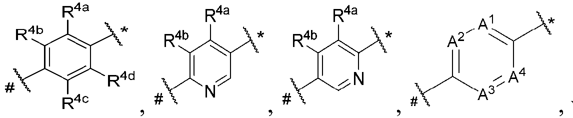 wherein A¹, A², A³ and A⁴ are each independently CH or N, or

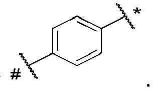

15. The compound, or the isotopic derivative or the pharmaceutically acceptable salt according to any one of claims 1 to 9, **characterized in that** ring A is

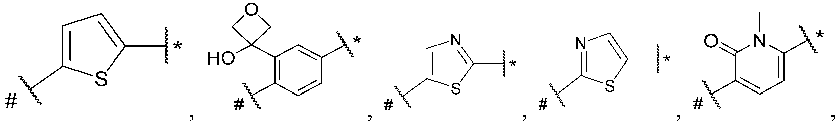

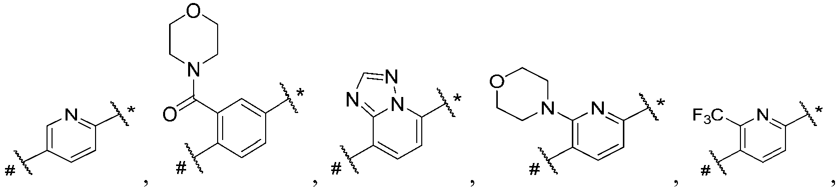

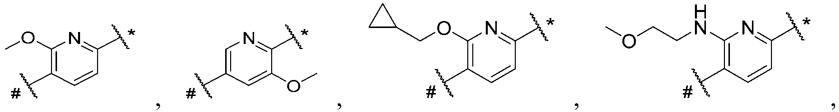

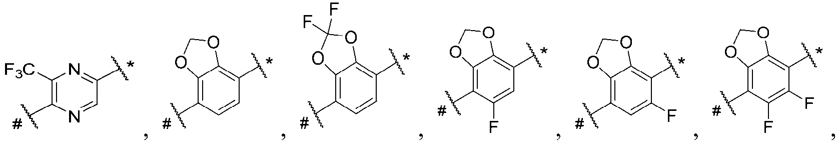

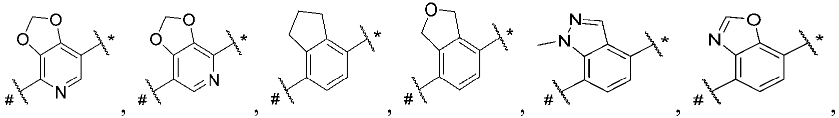

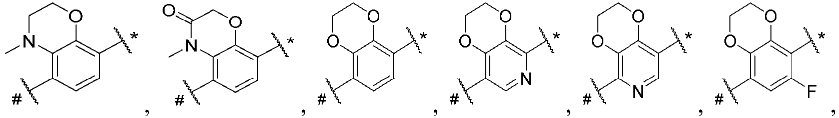

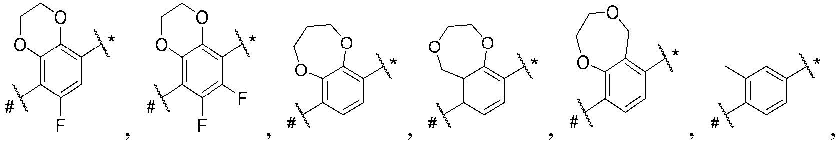

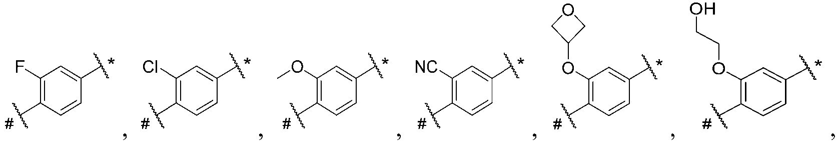

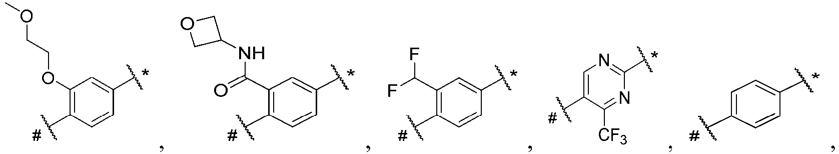

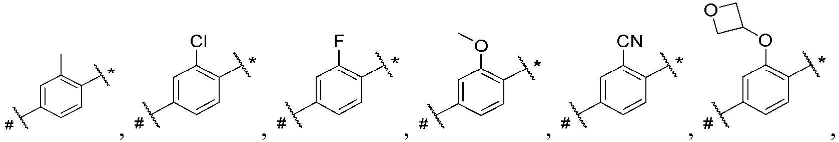

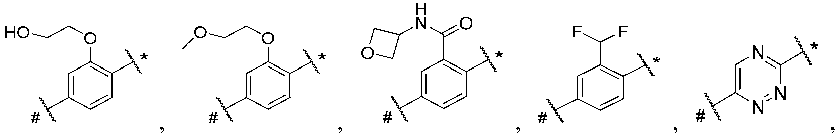

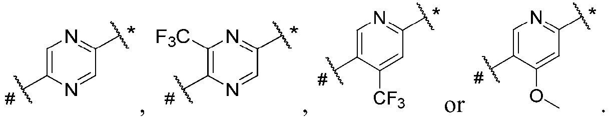

16. The compound, or the isotopic derivative or the pharmaceutically acceptable salt according to any one of claims 1 to 15, **characterized in that** R^{3a} is hydrogen or C₁₋₆ alkyl.

17. The compound, or the isotopic derivative or the pharmaceutically acceptable salt according to any one of claims 1 to 16, **characterized in that** R^{3b} is - L¹-R^{3c}; L¹ is -[CH(R^{a})]ₙ-; wherein n is 1, 2 or 3; R^{3c} is -OH; R^{a} is hydrogen or C₁₋₆ alkyl, and the C₁₋₆ alkyl is optionally substituted with one or more hydroxyl; preferably, R^{3b} is -CH₂OH, -CH(CH₃)OH, -C(CH₃)₂OH, -CH(OH)CH₂OH, - CH₂CH₂OH or

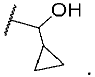

18. The compound, or the isotopic derivative or the pharmaceutically acceptable salt according to any one of claims 1 to 15, **characterized in that** R^{3a} and R^{3b} are joined to form

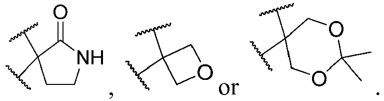

19. The compound, or the isotopic derivative or the pharmaceutically acceptable salt according to any one of claims 1 to 18, **characterized in that** s is 1 and t is 1.

20. The compound, or the isotopic derivative or the pharmaceutically acceptable salt according to any one of claims 1 to 19, **characterized in that** R¹ is C₁₋₆ alkyl; preferably, R¹ is methyl.

21. The compound, or the isotopic derivative or the pharmaceutically acceptable salt according to any one of claims 1, 2 and 4 to 16, **characterized in that** R^{3b} is benzoxazolyl, -C(O)NHCH₃, -C(O)OCH₃ or

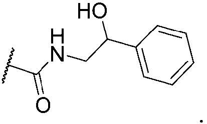

22. The compound as shown in formula I, or the isotopic derivative or the pharmaceutically acceptable salt according to claim 1, 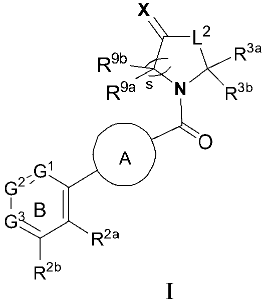 **characterized in that** X is N-OR¹;
R¹ is -CH₃;
R^{3a} is selected from hydrogen or C₁₋₆ alkyl;
R^{3b} is -L¹-R^{3c};
L¹ is independently -[C(R^{a})(R^{b})]ₙ-; wherein n is 1; R^{a} and R^{b} are each independently hydrogen or -CH₃;
R^{3c} is independently -OR^{7a}; the R^{7a} is selected from hydrogen or C₁₋₆ alkyl; L² is -[C(R^{10a})(R^{10b})]ₜ-;
s is 1 and t is 1;
R^{9a}, R^{9b}, R^{10a} and R^{10b} are hydrogen;
ring A is 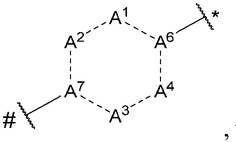
wherein the * end is connected to the carbonyl group in formula I, and the # end is connected to ring B in formula I; each ----bond is independently a single bond or a double bond;
A⁵ is CH or C(R^{4a});
A² is CH or C(R^{4b});
A³ is CH;
A⁴ is CH;
each A⁶ is independently C or N;
each A⁷ is independently C or N;
R^{4a} and R^{4b} are each independently H; alternatively, R^{4a} and R^{4b}, together with the atom to which they are attached, form 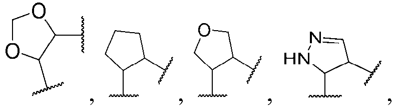 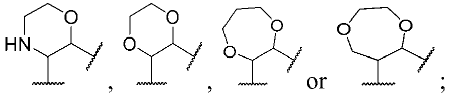
in ring B, G¹, G² and G³ are CH;
R^{2a} is halogen or -CH₃;
R^{2b} is cyano.

23. The compound, or the isotopic derivative or the pharmaceutically acceptable salt according to claim 1, **characterized in that** the compound is a compound as shown in formula Ia, or a stereoisomer thereof, i.e., a compound as shown in formula Ib, or a mixture of formula Ia and formula Ib: 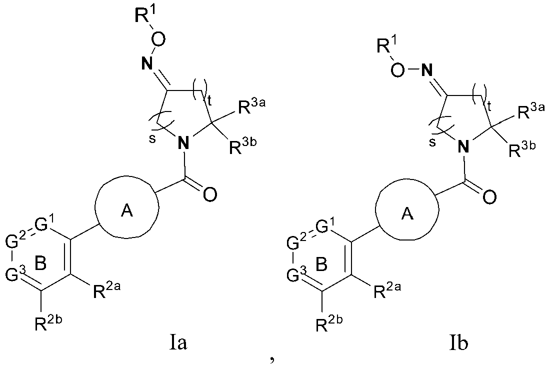 wherein R¹, R^{2a}, R^{2b}, R^{3a}, R^{3b}, s, t, G¹, G², G³ and ring A are as defined in any one of claims 1 to 22.

24. A compound, or an isotopic derivative thereof or a pharmaceutically acceptable salt of any of the foregoing, **characterized in that** the compound is any of the following compounds or a mixture of the compound and a stereoisomer thereof:

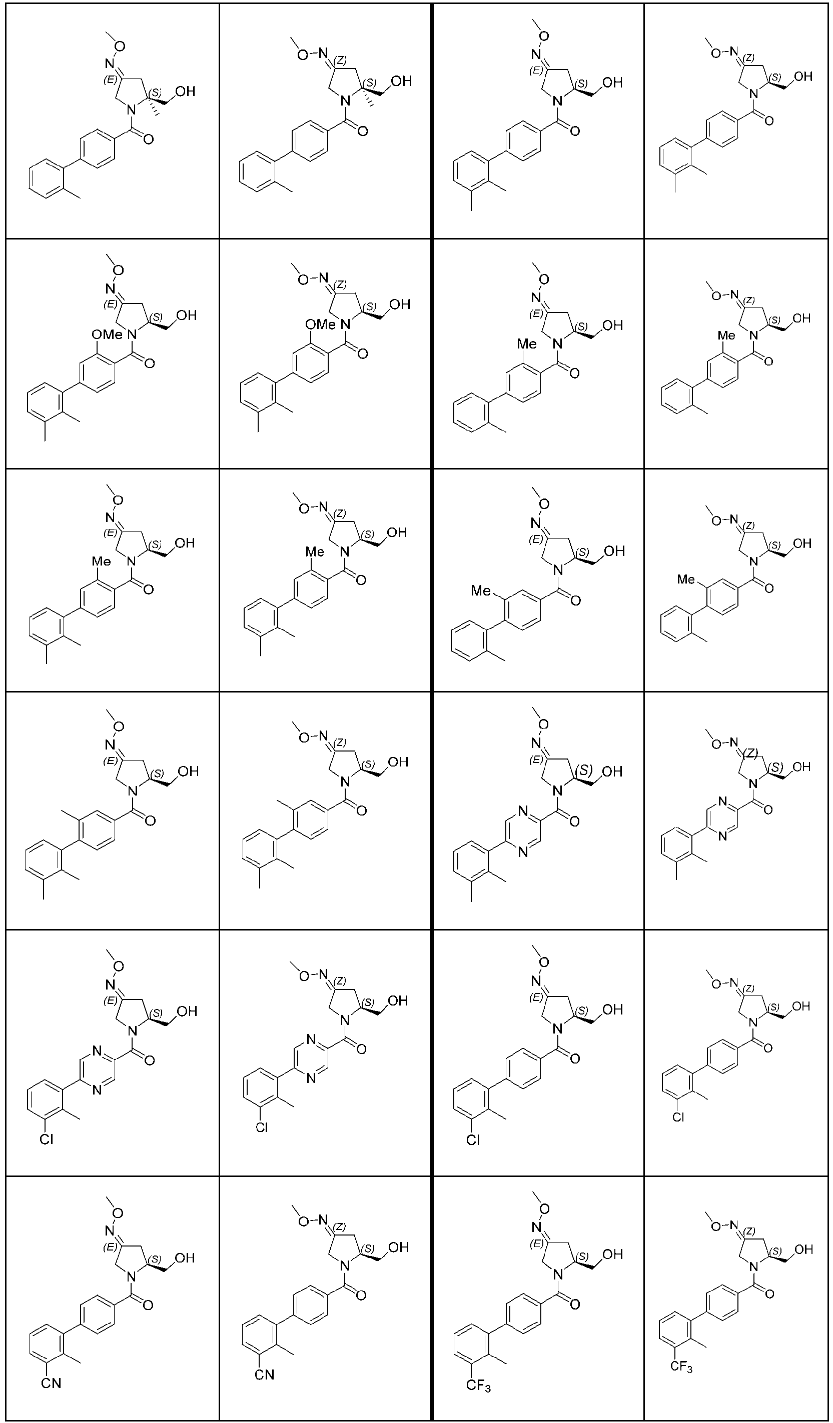

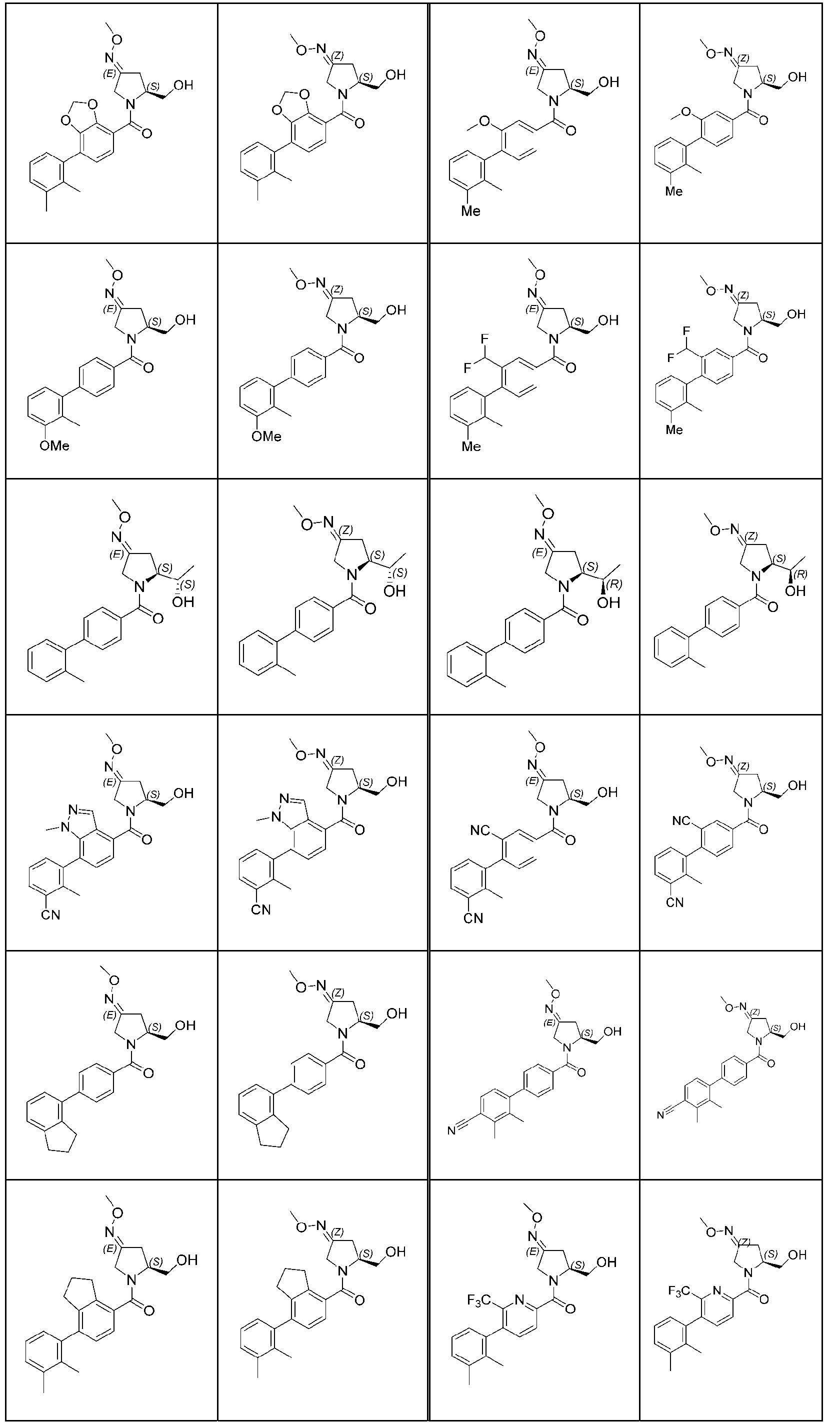

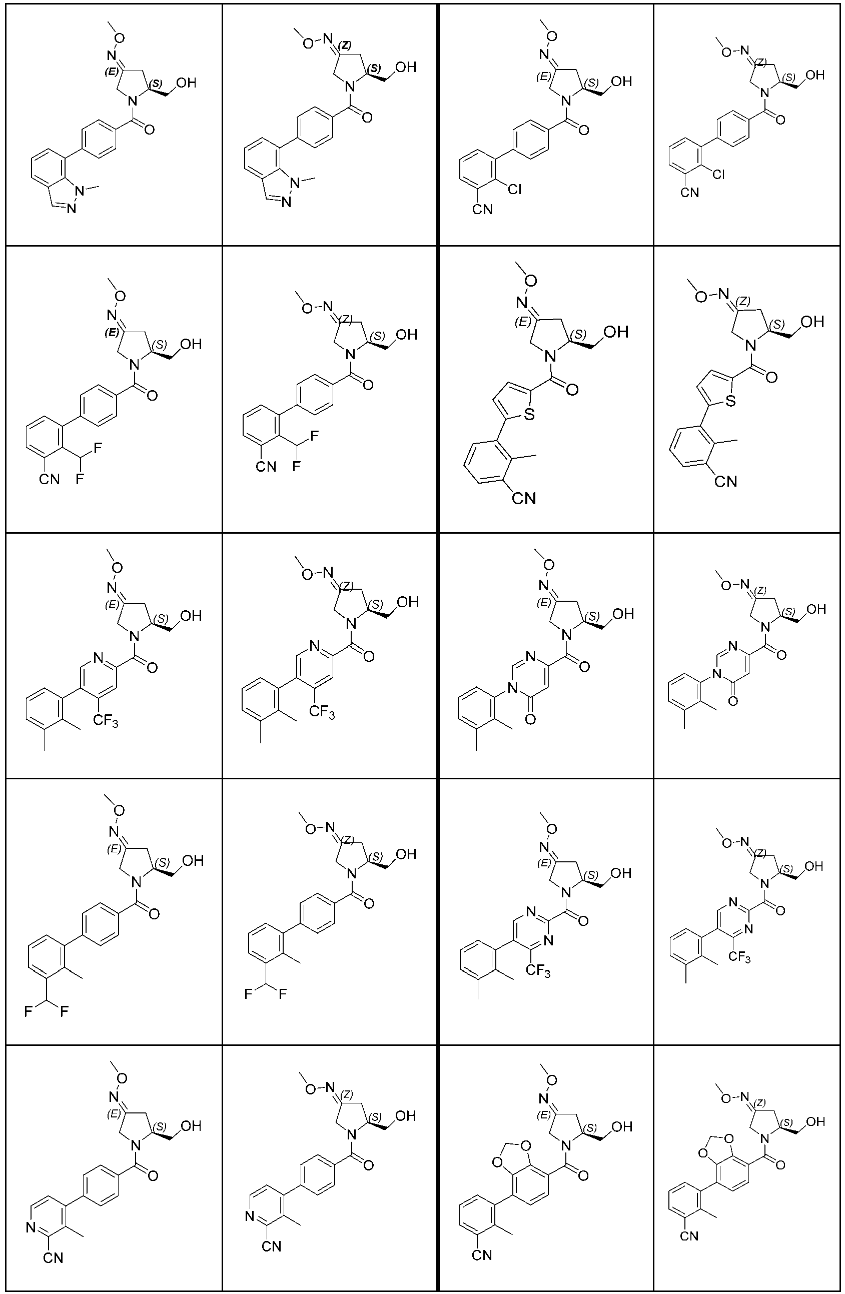

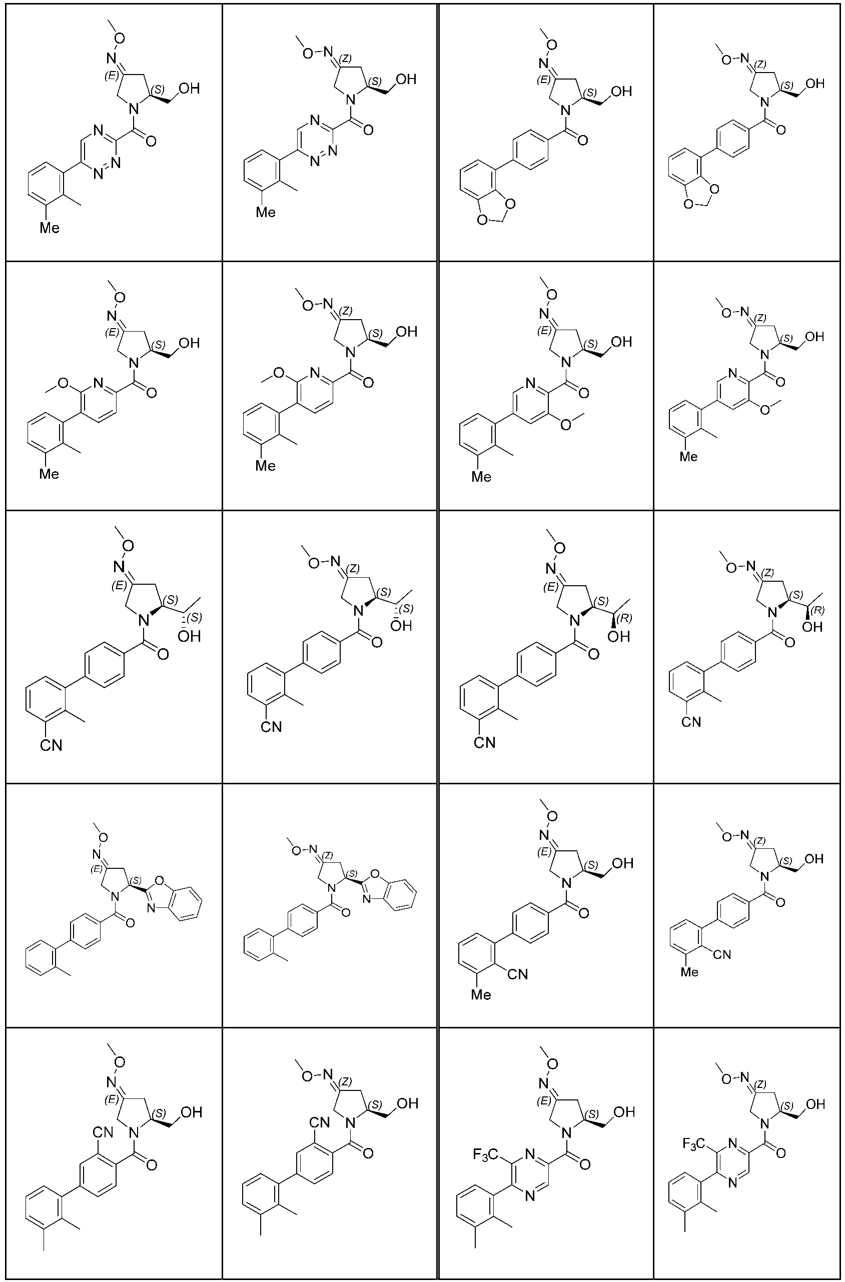

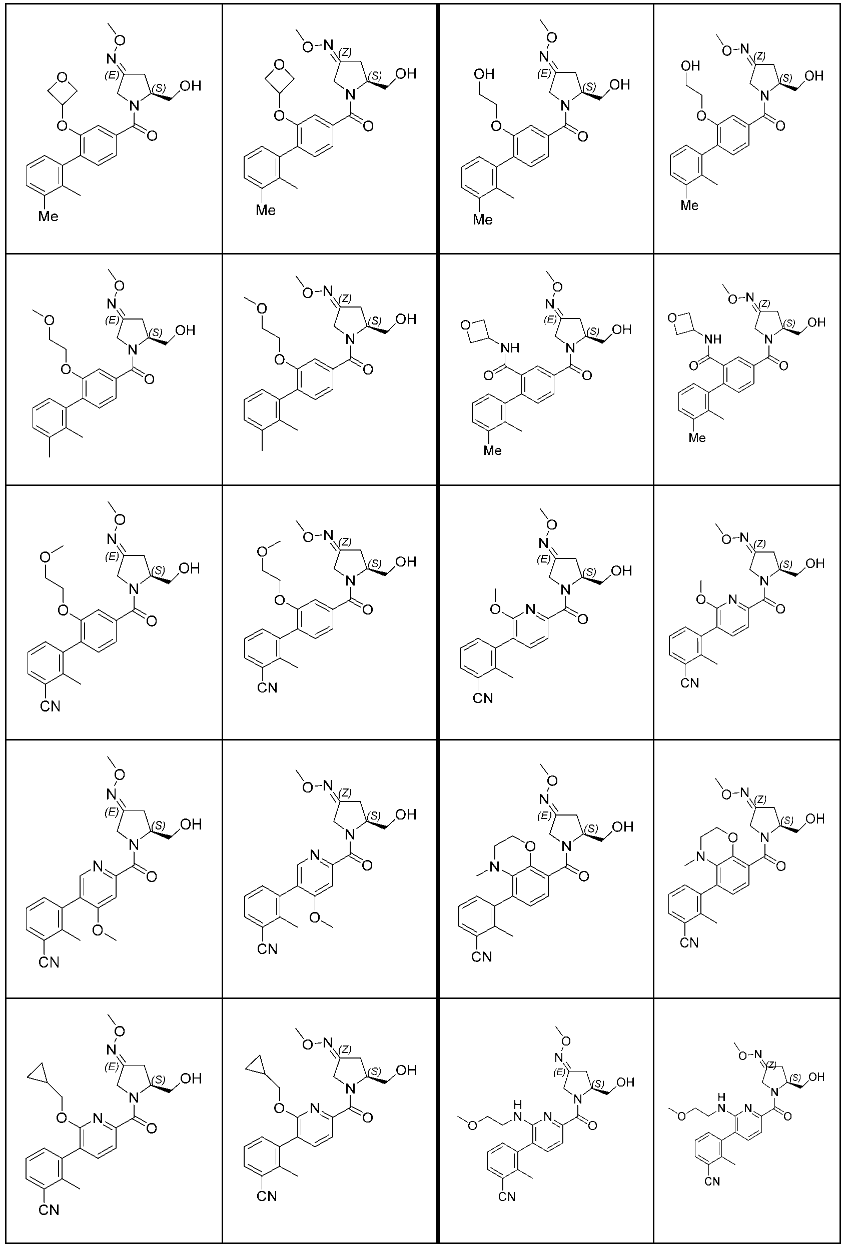

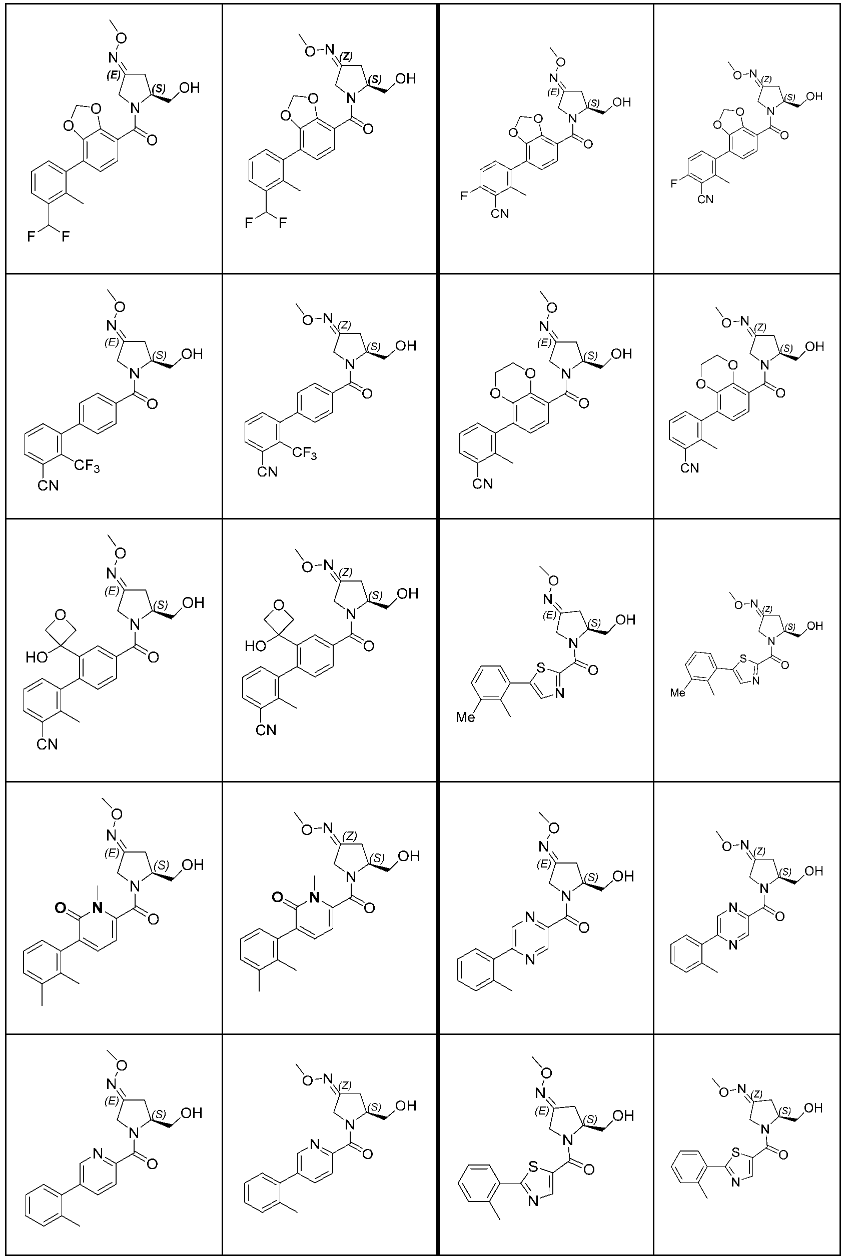

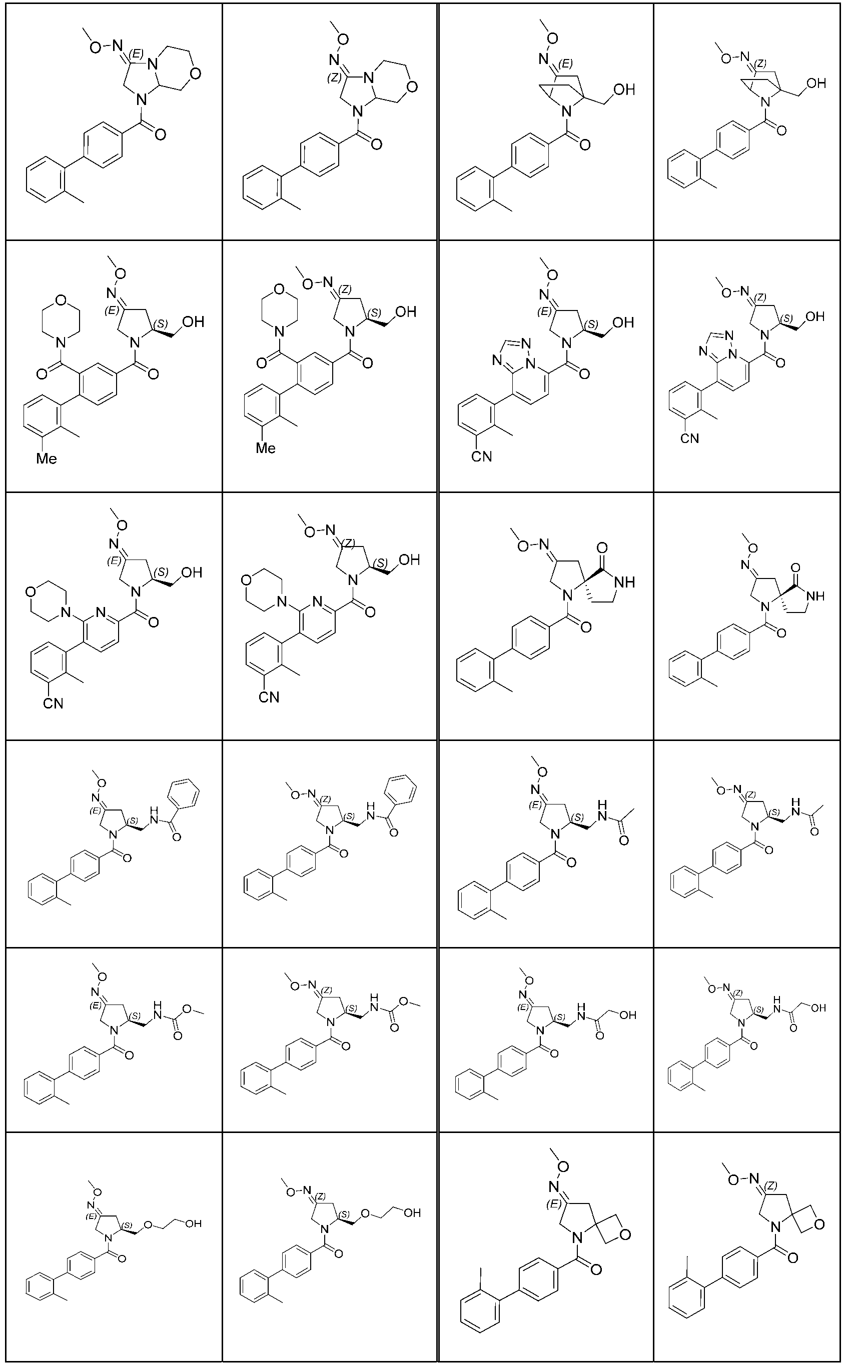

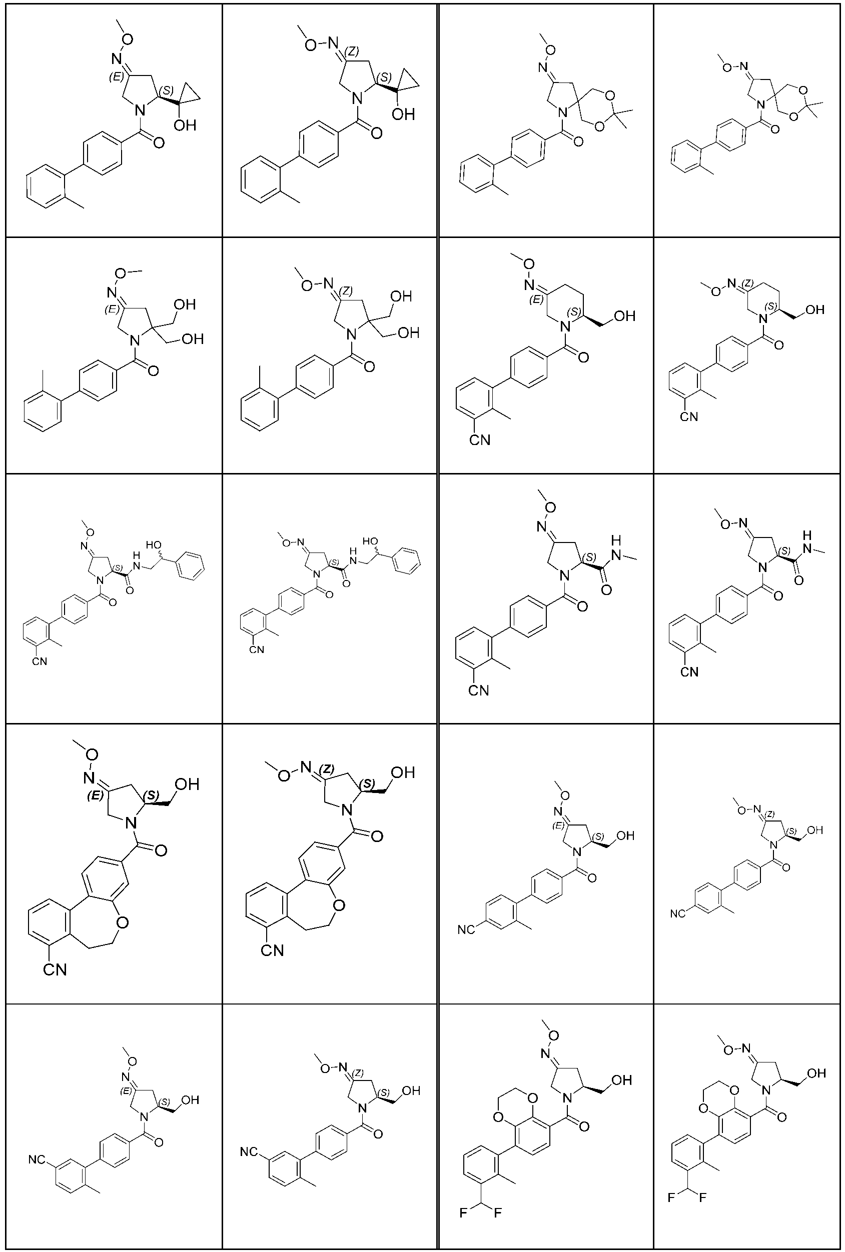

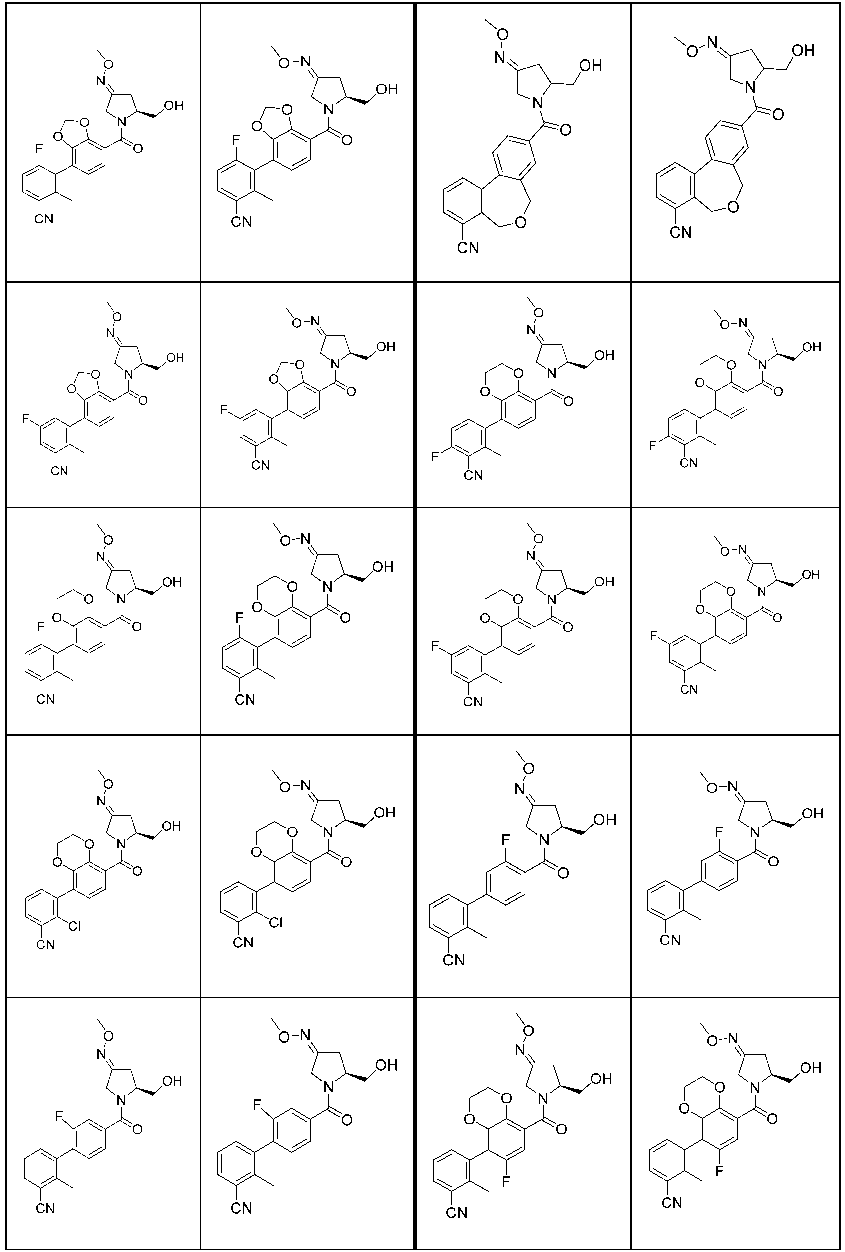

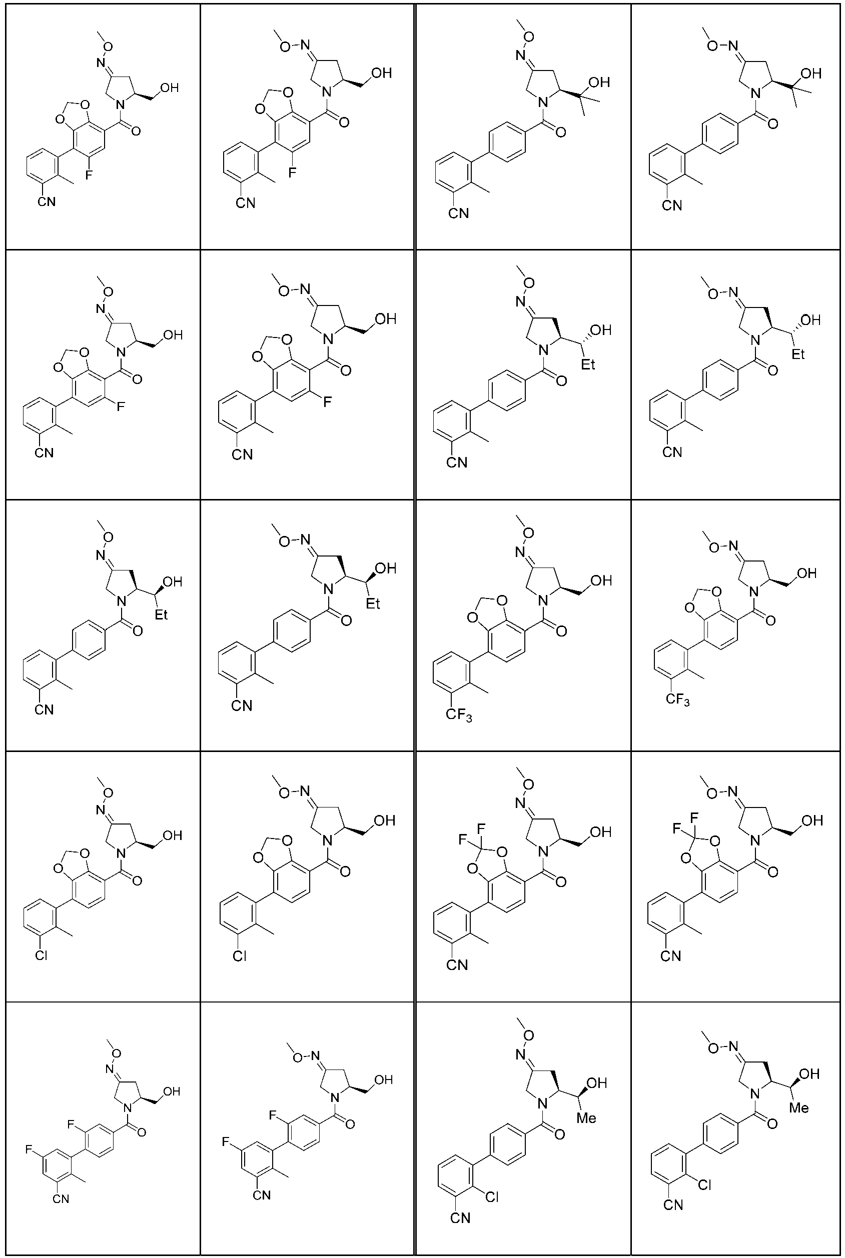

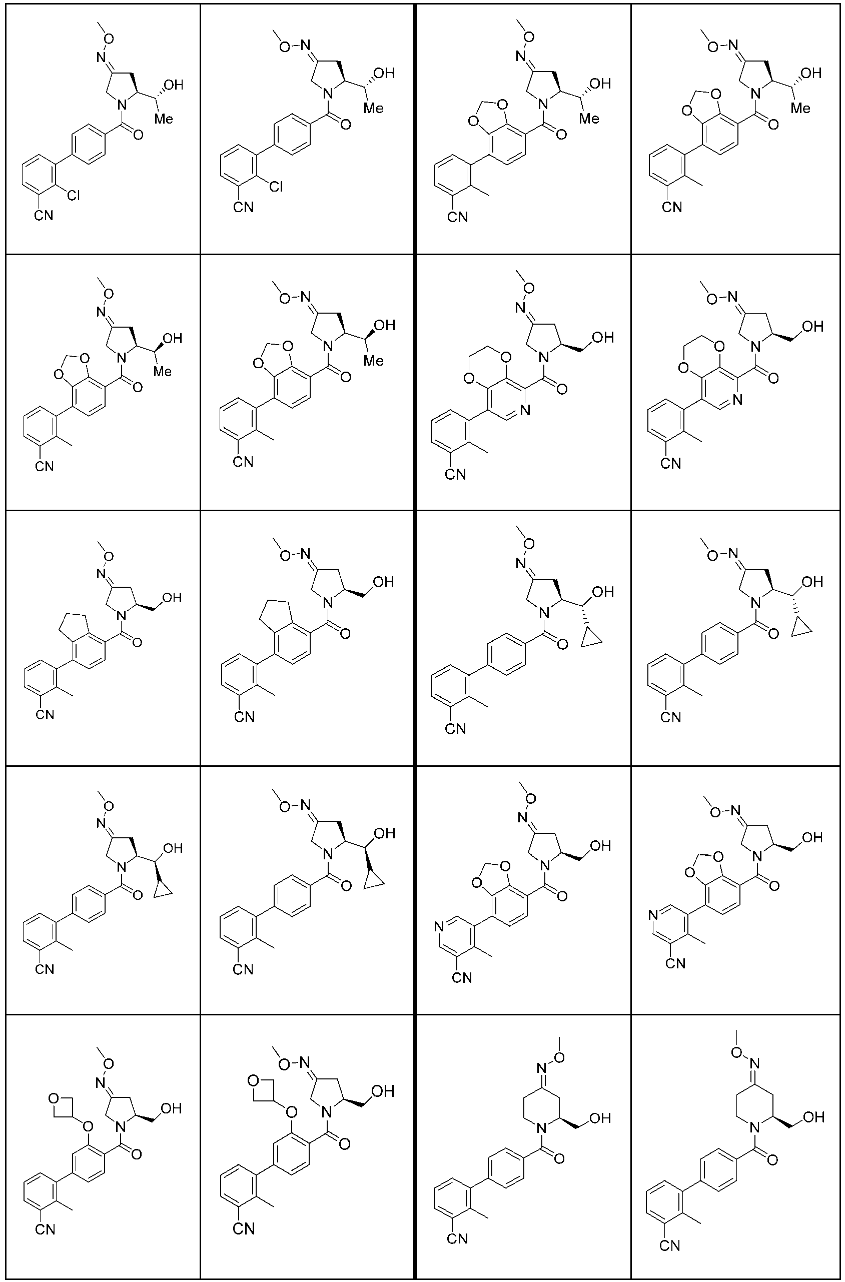

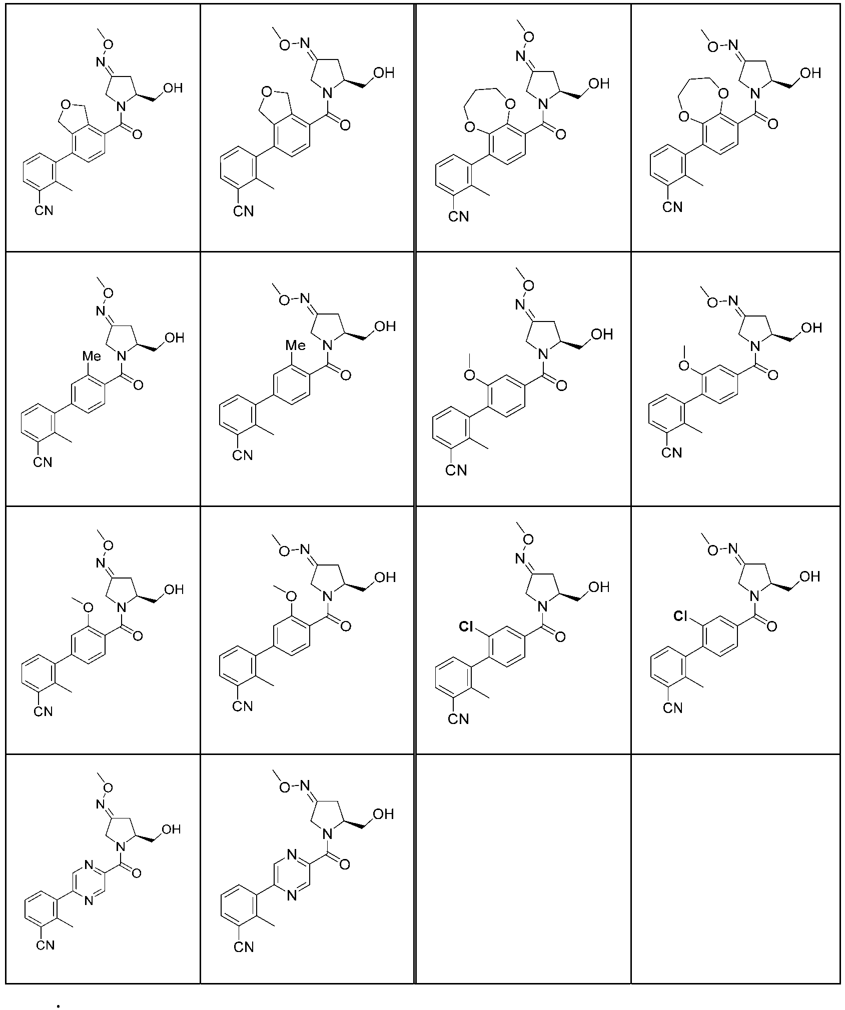

25. A pharmaceutical composition, **characterized by** comprising the compound, or the isotopic derivative or the pharmaceutically acceptable salt according to any one of claims 1 to 24, and a pharmaceutical auxiliary material.

26. Use of the compound, or the isotopic derivative or the pharmaceutically acceptable salt according to any one of claims 1 to 24, or the pharmaceutical composition comprising same in the preparation of an oxytocin receptor antagonist; alternatively, use of the compound, or the isotopic derivative or the pharmaceutically acceptable salt according to any one of claims 1 to 24, or the pharmaceutical composition comprising same in the inhibition of an oxytocin receptor.

27. Use of the compound, or the isotopic derivative or the pharmaceutically acceptable salt according to any one of claims 1 to 24 in the preparation of a medicament for preventing and/or treating a disease or condition for which inhibition of oxytocin is known or demonstrated to produce a beneficial effect; alternatively, the compound, or the isotopic derivative or the pharmaceutically acceptable salt according to any one of claims 1 to 24, for use in preventing and/or treating a disease or condition for which inhibition of oxytocin is known or demonstrated to produce a beneficial effect; alternatively, use of the compound, or the isotopic derivative or the pharmaceutically acceptable salt according to any one of claims 1 to 24 in preventing and/or treating a disease or condition for which inhibition of oxytocin is known or demonstrated to produce a beneficial effect.

28. The use according to claim 27, **characterized in that** the disease or condition is sexual dysfunction, hypoactive sexual desire disorder, sexual arousal disorder, orgasm disorder, dyspareunia, premature ejaculation, preterm delivery, delivery complications, appetite and eating disorders, benign prostatic hyperplasia, premature delivery, dysmenorrhea, congestive heart failure, arterial hypertension, liver cirrhosis, renal hypertension, ocular hypertension, obsessive-compulsive disorder or neuropsychiatric disease; preferably, the disease or condition is premature delivery.
